(19) Europäisches Patentamt
European Patent Office
Office européen des brevets

(11) EP 1 339 840 B1

(12) **EUROPEAN PATENT SPECIFICATION**

(45) Date of publication and mention of the grant of the patent:
**13.09.2006 Bulletin 2006/37**

(21) Application number: **01984902.5**

(22) Date of filing: **04.12.2001**

(51) Int Cl.:
*C12N 15/12* (2006.01)
*C12N 15/63* (2006.01)
*C07K 16/28* (2006.01)
*G01N 33/50* (2006.01)
*C07K 14/705* (2006.01)
*A01K 67/027* (2006.01)
*C12Q 1/68* (2006.01)

(86) International application number:
**PCT/IB2001/002798**

(87) International publication number:
**WO 2002/046404 (13.06.2002 Gazette 2002/24)**

(54) **SCHIZOPHRENIA-RELATED VOLTAGE-GATED ION CHANNEL GENE AND PROTEIN**

MIT SCHIZOPHRENIE IN ZUSAMMENHANG STEHENDES GEN, DAS FÜR EIN POTENTIAL-ABHÄNGIGES IONKANAL KODIERT

GENE ET PROTEINE DE CANAL IONIQUE DEPENDANT DU POTENTIEL, ASSOCIE A LA SCHIZOPHRENIE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **05.12.2000 US 251317 P**

(43) Date of publication of application:
**03.09.2003 Bulletin 2003/36**

(73) Proprietor: **Serono Genetics Institute S.A.**
**91030 Evry Cedex (FR)**

(72) Inventors:
- **COHEN, Daniel**
  **F-92200 Neuilly-sur-Seine (FR)**
- **CHUMAKOV, Ilya**
  **F-77000 Vaux-le-Penil (FR)**
- **SIMON, Anne-Marie**
  **F-75012 Paris (FR)**
- **ABDERRAHIM, Hadi**
  **F-94220 Charenton le Pont (FR)**

(74) Representative: **Brasnett, Adrian Hugh et al**
**Mewburn Ellis LLP**
**York House**
**23 Kingsway**
**London WC2B 6HP (GB)**

(56) References cited:
**WO-A-01/83752**
**WO-A-02/04520**
**WO-A-99/28342**
**WO-A-99/66041**

- **LEE J H ET AL: "Cloning of a novel four repeat protein related to voltage-gated sodium and calcium channels." FEBS LETTERS. NETHERLANDS 26 FEB 1999, vol. 445, no. 2-3, 26 February 1999 (1999-02-26), pages 231-236, XP002212096 ISSN: 0014-5793 cited in the application**
- **DATABASE EMBL [Online] 12 May 2000 (2000-05-12) "Homo sapiens chromosome 12 clone RP11-392G11" retrieved from EBI Database accession no. AC068887 XP002212097**
- **BRZUSTOWICZ L.M. ET AL: 'Linkage of familial schizophrenia to chromosome 13q32' AM.J.HUM.GENET. vol. 65, 1999, pages 1096 - 1103**
- **GOULD R.J. ET AL: 'ANTISCHIZOPHRENIC DRUGS OF THE DIPHENYLBUTYLPIPERIDINE TYPE ACT AS CALCIUM CHANNEL ANTAGONISTS' PROC.NATL.ACAD.SCI.USA vol. 80, 1983, pages 5122 - 5125, XP000867760**
- **JAY GARGUS J. ET AL MOLECULAR MEDICINE TODAY vol. 4, no. 12, December 1998, pages 518 - 524**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention is directed to a voltage-gated ion channel gene and protein and its role in disease. The invention relates to polynucleotides encoding a CanIon polypeptide as well as the regulatory regions located at the 5'- and 3'-end of said coding region. The invention also concerns polypeptides encoded by the CanIon gene. The invention also provides methods for screening for modulators, e.g. antagonists, of the CanIon channel, and methods of using such modulators in the treatment or prevention of various disorders or conditions. The invention also deals with antibodies directed specifically against such polypeptides that are useful as diagnostic reagents. The invention further encompasses biallelic markers of the CanIon gene useful in genetic analysis.

### BACKGROUND OF THE INVENTION

Advances in the technological armamentarium available to basic and clinical investigators have enabled increasingly sophisticated studies of brain and nervous system function in health and disease. Numerous hypotheses both rieurobiological and pharmacological have been advanced with respect to the neurochemical and genetic mechanisms involved in central nervous system (CNS) disorders, including psychiatric disorders and neurodegenerative diseases. However, CNS disorders have complex and poorly understood etiologies, as well as symptoms that are overlapping, poorly characterized, and difficult to measure. As a result, future treatment regimes and drug development efforts will be required to be more sophisticated and focused on multigenic causes, and will need new assays to segment disease populations, and provide more accurate diagnostic and prognostic information on patients suffering from CNS disorders.

CNS disorders can encompass a wide range of disorders, and a correspondingly wide range of genetic factors. Examples of CNS disorders include neurodegenerative disorders, psychotic disorders, mood disorders, autism, substance dependence and alcoholism, pain disorders, epilepsy, mental retardation, and other psychiatric diseases including cognitive, anxiety, eating, impulse-control, and personality disorders. Disorders can be defined using the Diagnosis and Statistical Manual of Mental Disorders fourth edition (DSM-IV) classification.

Even when considering just a small subset of CNS disorders, it is evident from the lack of adequate treatment for and understanding of the molecular basis of the disorders schizophrenia and bipolar disorder that new targets for therapeutic invention and improved methods of treatment are needed. For both schizophrenia and bipolar disorder, all of the currently known molecules used for their treatment have side effects and act only against the symptoms of the disease. There is thus a strong need for new molecules without associated side effects and directed against targets which are involved in the causal mechanisms of schizophrenia and bipolar disorder. Therefore, tools facilitating the discovery and characterization of these targets are necessary and useful.

*Voltage Gated Ion Channels*

Voltage gated ion channels are part of a large family of macromolecules whose functions include the control and maintenance of electric potential across cell mambrances, secretion and signal transduction. These channel proteins are involved in the control of neurotransmitter release from neurons, and play an important role in the regulation of a variety of cellular functions, including membrane excitability, muscle contraction and synaptic transmission. The main alpha-subunits of Na+ channels and the alpha-1 subunits of the Ca+ channels consist of approximately 2000 amino acids and contain the ion conduction pathway. Biochemical analysis has revealed that the physiologically active ion channel is composed of several different subunits. There are two auxillary subunits that copurify with the alpha subunit of Na+ channels, the beta-1 and beta-2 subunit. For Ca+ channels, additional subunits (alpha-2, beta, gamma and sigma) have been identified with modulatory action. The alpha-2 and beta-subunits appear to enhance the functional activity of the alpha-1 subunit of Ca+ channels. The alpha-subunits of K+ channels are associated with beta subunits in a 1:1 fashion resulting in a K+ channel complex exhibiting $(alpha)_4(beta)_4$ stoichiometry (Terlau et al., Naturwissenschaften 85:437-444 (1998)). The basic structure and examples of calcium and sodium ion channels are further discussed, e.g., in Williams, et al. Science 257:389-395 (1992); Mori, et al., Nature 350:398-402 (1991); and Koch, et al., J.Biol.Chem. 265 (29):17786-17791 (1990). A Ca+ and Na+ ion channel nucleic acid sequence from the rat sharing a high level of sequence homology with the CanIon channel is further described in Lee et al., FEBS Lett. 445 231:236 (1999).

The alpha subunit shares sequence characteristics with all voltage-dependent cation channels, and exploits the same structural motif comprising a 6-helix bundle of potential membrane spanning domains. In both sodium and calcium channels, this motif is repeated 4 times within the sequence to give a 24-helix bundle. The amino acid sequences are highly conserved among species (e.g., human and Drosophila), and among different ion channels.

There are several tissue-specific pharmacologically and electrophysiologically distinct isoforms of calcium channels, coded for by separate genes in a multi-gene family. In skeletal muscle, each tightly-bound assembly of alpha, beta and gamma subunits associates with 4 others to form a pentameric macromolecule. For example, neuronal calcium channel alpha-1 subunits are the product of at least seven different genes named alpha-1 A to H. Immunocytochemical sudies have shown differential distribution of alpha-1 calcium channel subunits. Alpha-1A and alpha-1B are expressed mainly in dendrites and presynaptic terminals, and alpha-1A is generally concentrated in a larger number of nerve terminals than is alpha-1B. In the rat and human neuromuscular junction, alpha-1A is localized presynaptically, while alpha-1B and alpha-1A are both present in axon-associated Schwann cells. Alpha-1E is localized mainly in cell bodies, in some cases in proximal dendrites, as well as in the distal branches of Purkinje cells. Alpha-1C and alpha-1D are localized in cell bodies and in proximal dendrites of central neurons.

Native calcium channels have been classified based on their pharmacological and/or electrophysiological propoerties. The classification of voltage dependent cacium channels divides them into high voltage-activated (HVA), including L-, N-, P- and Q- types; intermediate (IVA, R-type); and low voltage-activated (LVA, T-type). (Morena et al., Annals NY Acad. Sci. 102-117 (1999).

The principal subunits (alpha-1) belong to a gene family whose members can form functional channels by themselves when expressed in heterologous expression systems. (Zhang et al., Neuropharmacology 32 (11): 1075-1088 (1993). In native cells, alpha-1 subunits are expressed as multisubunit complexes with ancillary subunits which modify the functional properties of the alpha-1 subunit In many cases, coexpression of auxiliary subunits affects the biophysical properties of the channels. Beta subunits in particular tend to have important effects on the alpha-1 subunits; beta subunits have been shown to alter activation properties, steady state inactivation, inactivation kinetics and peak current.

Much of the molecular diversity of channels is produced by the existence of multiple forms of alpha-1 subunits. For example, it has been shown that differently spliced forms of calcium channels are differentially expressed and have different sensitivities to phosphorylation by serine-threonine kinases (Hell et al., Annals N.Y. Acad. Sci. 747:282-293 (1994)). Mutations in ion channel genes have been shown to be involved in a wide range of diseases, including several central nervous system diseases. Examles of ion channel mutations causing a number of eposodic disorders inclduing periodic paralysis, eposodic ataxia, migraine, long QT syndrome and paroxysmal dyskenesia are reviewed in Bulman et al., Hum. Mol. Gen. 6(10) 1679-1685 (1997). Several Ca+ channel mutation disorders, for example, are shown in Table A (from Moreno, supra).

**Table A**

| Disease | Calcium channel subunit | Model |
|---|---|---|
| Familial hemiplegic migraine | Alpha-1A | Human |
| Episodic ataxia type 2 | Alpha-1A | Human |
| Spinocerebellar ataxia typ 6 | Alpha-1A | Human |
| Tottering and Learner phenotypes | Alpha-1A | Mouse |
| Lambert-Eaton syndrome | Alpha-1A, α-1B | Human |
| Hypokalemic periodic paralysis | Alpha-1S | Human |
| Muscular dysgenesis | Alpha-1S | Mouse |
| Zucker diabetic fatty phenotypes | α-1C; α-1D | Rat |
| Lethargic phenotype | Beta-4 | Mouse |
| Malignant hypothermia | Alpha-2/δ | Human |
| Stargazer | Gamma | Mouse |

Modulators of calcium and sodium channels are also commonly used in the treatment of various diseases and conditions. For example, calcium and/or sodium channel blockers have been shown to be useful for the treatment or prevention of one or more symptoms associated with various diseases or conditions such as various heart diseases and conditions (e.g., angina, arrythmias), hypertension, migraines, neurological effects of strokes, mania, neuroleptic-induced tardive dyskinesia, bipolar disorder, pain, epilepsy, and others.

It has been shown that significant functional differences in the nervous system exist between different ion channels. In addition, functional differences exist between different mutations in the same ion channel gene as well as between splice variants of the same ion channel. Thus, despite the implication of ion channels in CNS disease, it has been difficult to predict which ion channel may be an effective target for therapeutic intervention for a particular disease. One problem has been to provide an ion channel gene implicated in schizophrenia, bipolar disorder or diseases related thereto.

The present invention addresses these and other needs.

## SUMMARY OF THE INVENTION

The present invention is directed to methods for the screening of substances or molecules that modulate the expression or activity of a voltage-gated ion channel protein, called CanIon, as well as with methods for the screening of substances or molecules that interact with the ion channel polypeptide. Methods of using substances identified in these methods are also provided. For example, methods of treating or prevention diseases or conditions including schizophrenia or bipolar disorder using ion channel antagonists are provided.

In one aspect, the present invention provides the use of an antibody or antigen binding domain specifically binding to the polypeptide shown as SEQ ID NO:5 in the preparation of a medicament for the treatment of schizophrenia or bipolar disorder.

In a further aspect, the invention provides in vitro use of an antibody or antigen binding domain specifically binding to the polypeptide shown as SEQ ID NO:5 in the diagnosis of schizophrenia or bipolar disorder.

In one embodiment, any of the herein described antibodies or antigen binding domains is chimeric, human or humanized.

In another aspect, the invention provides the use of an antisense tool that inhibits the expression of the ion channel encoded by a polynucleotide shown as SEQ ID NO:1, 2, 3 or 4 comprising a fragment of the polynucleotide shown as SEQ ID NO:1, 2, 3 or 4 in the preparation of a medicament for the treatment of schizophrenia or bipolar disorder. In one embodiment, the antisense tool is a ribozyme.

In another aspect, the invention provides a pharmaceutical composition comprising any of the presently described antibodies, antigen binding domains, or antisense tools and a suitable carrier or excipient.

In another aspect, the present invention provides a method of identifying a candidate modulator of an ion channel polypeptide, said method comprising: a) contacting a polypeptide comprising a polypeptide as shown as SEQ ID NO:5 with a test compound; and b) determining whether said compound specifically binds to said polypeptide; wherein a detection that said compound specifically binds to said polypeptide indicates that said compound is a candidate modulator of said ion channel polypeptide.

In one embodiment, the method further comprises testing the biological activity of said ion channel polypeptide in the presence of said candidate modulator, wherein an alteration in the biological activity of said ion channel polypeptide in the presence of said candidate modulator in comparison to the activity in the absence of said candidate modulator indicates that the candidate modulator is a modulator of said ion channel polypeptide.

In another aspect, the present invention provides a method of identifying a modulator of an ion channel polypeptide, said method comprising: a) contacting a polypeptide comprising a polypeptide as shown as SEQ ID NO:5 with a test compound; and b) detecting the activity of said polypeptide in the presence and absence of said compound; wherein a detection of a difference in said activity in the presence of said compound in comparison to the activity in the absence of said compound indicates that said compound is a modulator of said ion channel polypeptide.

In one embodiment of the present methods, said polypeptide is present in a cell or cell membrane, and said biological activity comprises voltage gated ion channel activity.

In another aspect, the present invention provides a method for the preparation of a pharmaceutical composition comprising a) identifying a modulator of ion channel polypeptide using any of the herein-described methods; and b) combining said modulator with a physiologically acceptable carrier. Methods of using the pharmaceutical compositions are also provided.

Uses of any of the presently-described ion channel modulators, polypeptides, polynucleotides, or antibodies in the preparation of a medicament, e.g. for the treatment of the human body or for the treatment of any of the herein-described diseases or conditions, are also provided.

Kits for using and detecting the present ion channel polynucleotides and polypeptides in vitro or in vivo are also provided.

In another aspect, the present invention provides the use of biallelic markers of the ion channel gene for determining whether an individual is at risk for or suttering from schizophrenia or bipolar disorder.

## BRIEF DESCRIPTION OF THE DRAWINGS

Figure 1 is a diagram showing a BAC map of the chromosome 13q region containing the CanIon gene.

**Brief Description of the sequences provided in the Sequence** Listing

SEQ ID No 1 contains a genomic sequence of CanIon comprising the 5' regulatory region (upstream untranscribed region) and exons 1 to 7.

SEQ ID No 2 contains a genomic sequence of CanIon comprising exons 8 to 27.

SEQ ID No 3 contains a genomic sequence of CanIon comprising exons 28 to 44, and the 3' regulatory region (downstream untranscribed region).

SEQ ID No 4 contains a cDNA sequence of CanIon.

SEQ ID No 5 contains the amino acid sequence encoded by the cDNA of SEQ ID No 4.

SEQ ID No 6 contains the nucleotide sequence of the amplicon which comprises biallelic marker A18.

SEQ ID No 7 contains a primer containing the additional PU 5' sequence described further in Example 2

SEQ ID No 8 contains a primer containing the additional RP 5' sequence described further in Example 2.

In accordance with the regulations relating to Sequence Listings, the following codes have been used in the Sequence Listing to indicate the locations of biallelic markers within the sequences and to identify each of the alleles present at the polymorphic base. The code "r" in the sequences indicates that one allele of the polymorphic base is a guanine, while the other allele is an adenine. The code "y" in the sequences indicates that one allele of the polymorphic base is a thymine, while the other allele is a cytosine. The code "m" in the sequences indicates that one allele of the polymorphic base is an adenine, while the other allele is an cytosine. The code "k" in the sequences indicates that one allele of the polymorphic base is a guanine, while the other allele is a thymine. The code "s" in the sequences indicates that one allele of the polymorphic base is a guanine, while the other allele is a cytosine. The code "w" in the sequences indicates that one allele of the polymorphic base is an adenine, while the other allele is an thymine. The nucleotide code of the original allele for each biallelic marker is the following:

| Biallelic marker | Original allele |
|---|---|
| 5-124-273 | A (for example) |

In some instances, the polymorphic bases of the biallelic markers alter the identity of an amino acids in the encoded polypeptide. This is indicated in the accompanying Sequence Listing by use of the feature VARIANT, placement of an Xaa at the position of the polymorphic amino acid, and definition of Xaa as the two alternative amino acids. For example if one allele of a biallelic marker is the codon CAC, which encodes histidine, while the other allele of the biallelic marker is CAA, which encodes glutamine, the Sequence Listing for the encoded polypeptide will contain an Xaa at the location of the polymorphic amino acid. In this instance, Xaa would be defined as being histidine or glutamine.

In other instances, Xaa may indicate an amino acid whose identity is unknown because of nucleotide sequence ambiguity. In this instance, the feature UNSURE is used, placement of an Xaa at the position of the unknown amino acid and definition of Xaa as being any of the 20 amino acids or a limited number of amino acids suggested by the genetic code.

**DETAILED DESCRIPTION**

The aggregation of schizophrenia and bipolar disorder in families, the evidence from twin and adoption studies, and the lack of variation in incidence worldwide, indicate that schizophrenia and bipolar disorder are primarily genetic conditions, although environmental risk factors are also involved at some level as necessary, sufficient, or interactive causes. For example, schizophrenia occurs in 1% of the general population. But, if there is one grandparent with schizophrenia, the risk of getting the illness increases to about 3%; if there is one parent with schizophrenia the risk rises to about 10%. When both parents have schizophrenia, the risk rises to approximately 40%.

The identification of genes involved in a particular trait such as a specific central nervous system disorder, like schizophrenia, can be carried out through two main strategies currently used for genetic mapping: linkage analysis and association studies. Linkage analysis requires the study of families with multiple affected individuals and is now useful in the detection of mono- or oligogenic inherited traits. Conversely, association studies examine the frequency of marker alleles in unrelated trait (T+) individuals compared with trait negative (T-) controls, and are generally employed in the detection of polygenic inheritance.

Genetic link or "linkage" is based on an analysis of which of two neighboring sequences on a chromosome contains the least recombinations by crossing-over during meiosis. To do this, chromosomal markers, like microsatellite markers, have been localized with precision on the genome. Genetic linkage analysis calculates the probabilities of recombinations on the target gene with the chromosomal markers used, according to the genealogical tree, the transmission of the disease, and the transmission of the markers. Thus, if a particular allele of a given marker is transmitted with the disease more often than chance would have it (recombination level between 0 and 0.5), it is possible to deduce that the target gene in question is found in the neighborhood of the marker. Using this technique, it has been possible to localize several genes demonstrating a genetic predisposition of familial cancers. In order to be able to be included in a genetic linkage study, the families affected by a hereditary form of the disease must satisfy the "informativeness" criteria: several affected subjects (and whose constitutional DNA is available) per generation, and at best having a large number of siblings.

Results of linkage studies supported the hypothesis that chromosome 13 was likely to harbor a schizophrenia susceptibility locus on 13q32 (Blouin JL et al., 1998, Nature Genetics, 20:70-73; Lin MW et al., Hum. Genet., 99(3) (1997):417-420; Brzustowicz et al., Am. J. Hum. Genet. 65:1096-1103 (1999)). However, while linkage analysis is a powerful method for detecting genes involved in a trait, resolution is often not possible beyond the megabase level and complementary studies are often required to refine the analysis of the regions initially identified through this method.

A BAC contig covering a candidate genomic region of the chromosome 13q-31-q33 locus was constructed using public STSs localised in the chromosome 13q31-q33 region to screen a 7 genome equivalent proprietary BAC library. From these materials, new STSs were generated allowing construction of a dense physical map of the region. BACs were all sized and mapped by in situ chromosomal hybridisation for verification. A minimal set of BACs was identified and fully sequenced which resulted in several contigs leading to the eventual construction of a contig of over 4Mb. The construction of this map led to the identification of the CanIon gene which is located within a genomic region showing significant linkage to schizophrenia.

The CanIon amino acid sequence is characteristic of *CACHANNEL,* a 7-element fingerprint that provides a signature for the alpha-1 subunit of calcium channels (Ref. PR00167, BLOCKs+ database). The fingerprint was derived from an initial alignment of 6 sequences: the motifs were drawn from conserved loop regions capable of distinguishing between these and other cation channels; motifs 1 and 2 encode those between transmembrane segments 4 and 5, and 5 and 6 (first internal repeat); motif 3 corresponds to that between segment 6 of repeat 1 and segment 1 of repeat 2; motif 4 encodes that between segments 5 and 6 of repeat 2; motif 5 corresponds to that between segment 6 of repeat 3 and segment 1 of repeat 4; and motifs 6 and 7 encode those between segments 4 and 5, and 5 and 6 of repeat 4.

Figure 1 shows BAC contigs covering a chromosome 13 region of interest which includes the CanIon gene, and shows the genomic location of the CanIon gene in relation to genetic markers showing the highest significance in linkage studies. In particular, Blouin et al. (1998) conducted a genome wide scan for schizophrenia susceptibility loci using 452 microsatellite markers on 54 complex pedigrees. The most significant linkage between schizophrenia in families was found on chromosome 13q32 near marker D13S174. Brzustowics et al. (1999) evaluated microsatellite markers spanning chromosomes 8 and 13 in 21 extended Canadian families. Markers in the chromosome 13q region produced positive LOD scores in each analysis model used: autosomal dominant and recessive, with narrow or broad definition of schizophrenia. Maximum three point LOD scores were obtained with marker D13S793 under a recessive-broad model: 3.92 at recombinant fraction ($\theta$) .1 under homogeneity and 4.42 with $\alpha$=.65 and $\theta$=0 under heterogeneity. Referring to Figure 1, the CanIon gene is located partly on the contig labelled 'Region E' and partly on the contig labelled C0001A10. The CanIon gene is flanked by the two markers showing highest significance in linkage studies. Marker D13S174 is also on contig C0001A10, while marker D13S793 is located approximately 3.5Mb centromeric to the CanIon gene.

There is a strong need to identify genes involved in schizophrenia, bipolar disorder, and other CNS and cardiovascular diseases and conditions. There is also a need to identify novel ion channels involved in diseases. These genes and proteins may provide new intervention points in the treatment of schizophrenia, bipolar disorder, or other CNS conditions, as well as other conditions such as heart conditions and hypertension, and allow further study and characterization of the CanIon gene and its related biological pathway. The knowledge of these genes and the related biological pathways involved in these diseases and conditions will allow researchers to understand the etiology of, e.g., schizophrenia and bipolar disorder and will lead to drugs and medications which are directed against the cause of the diseases. For example, compounds that block CanIon channels can be used to treat any of a number of diseases or conditions, preferably schizophrenia or bipolar disorder, and also including pain disorders, epilepsy, and various cardiovascular disorders such as heart arrythmias, angina, and hypertension. There is also a great need for new methods of detecting a susceptibility to schizophrenia, bipolar disorder, and other conditions, as well as for preventing or following up the development of any of these diseases. Diagnostic tools could also prove extremely useful. Indeed, to give one example, early identification of subjects at risk of developing schizophrenia would enable early and/or prophylactic treatment to be administered. Moreover, accurate assessments of the efficacy of a medicament as well as the patient's tolerance to it may enable clinicians to enhance the benefit/risk ratio of schizophrenia and bipolar disorder treatment regimes.

The present invention encompasses methods of screening molecules for the ability to modulate the expression or activity of the ion channel gene or protein, as well as methods of using such molecules for the treatment or prevention of schizophrenia, bipolar disorder, or any of a number of other diseases or conditions. The invention also deals with the use of antibodies and antigen binding domains specifically binding to such polypeptides in the preparation it a medicament for the treatment it schizophrenia a bipolar disorder.

The invention encompasses the use of an antisense tool that inhibits the expression of the ion channel polypeptide comprising a fragment of the ion channel gene in the preparation of a medicament for the treatment of schizophrenia or bipolar disorder.

The invention also concerns ion channel related biallelic markers and their use in methods of genetic analysis including linkage studies in families, linkage disequilibrium studies in populations and association studies in case-control populations. An important aspect of the present invention is that biallelic markers allow association studies to be performed to identify the role of genes involved in complex traits.

## Definitions

Before describing the invention in greater detail, the following definitions are set forth to illustrate and define the meaning and scope of the terms used to describe the invention herein.

The terms "CanIon gene", when used herein, encompasses genomic, mRNA and cDNA sequences encoding the CanIon protein, including the untranslated regulatory regions of the genomic DNA.

The term "heterologous protein", when used herein, is intended to designate any protein or polypeptide other than the CanIon protein. For example, the heterologous protein may be a compound which can be used as a marker in further experiments with a CanIon regulatory region.

The term "isolated" requires that the material be removed from its original environment (e.g., the natural environment if it is naturally occurring). For example, a naturally-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

For example, a naturally-occurring polynucleotide present in a living animal is not isolated, but the same polynucleotide, separated from some or all of the coexisting materials in the natural system, is isolated. Specifically excluded from the definition of "isolated" are: naturally-occurring chromosomes (such as chromosome spreads), artificial chromosome libraries, genomic libraries, and cDNA libraries that exist either as an in vitro nucleic acid preparation or as a transfected/transformed host cell preparation, wherein the host cells are either an in vitro heterogeneous preparation or plated as a heterogeneous population of single colonies. Also specifically excluded are the above libraries wherein a specified polynucleotide makes up less than 5% of the number of nucleic acid inserts in the vector molecules. Further specifically excluded are whole cell genomic DNA or whole cell RNA preparations (including said whole cell preparations which are mechanically sheared or enzymatically digested). Further specifically excluded are the above whole cell preparations as either an in vitro preparation or as a heterogeneous mixture separated by electrophoresis (including blot transfers of the same) wherein the polynucleotide of the invention has not further been separated from the heterologous polynucleotides in the electrophoresis medium (e.g., further separating by excising a single band from a heterogeneous band population in an agarose gel or nylon blot).

The term "purified" does not require absolute purity; rather, it is intended as a relative definition. Purification of starting material or natural material to at least one order of magnitude, preferably two or three orders, and more preferably four or five orders of magnitude is expressly contemplated. As an example, purification from 0.1 % concentration to 10 % concentration is two orders of magnitude. To illustrate, individual cDNA clones isolated from a cDNA library have been conventionally purified to electrophoretic homogeneity. The sequences obtained from these clones could not be obtained directly either from the library or from total human DNA. The cDNA clones are not naturally occurring as such, but rather are obtained via manipulation of a partially purified naturally occurring substance (messenger RNA). The conversion of mRNA into a cDNA library involves the creation of a synthetic substance (cDNA) and pure individual cDNA clones can be isolated from the synthetic library by clonal selection. Thus, creating a cDNA library from messenger RNA and subsequently isolating individual clones from that library results in an approximately $10^4$-$10^6$ fold purification of the native message.

The term "purified" is further used herein to describe a polypeptide or polynucleotide of the invention which has been separated from other compounds including, but not limited to, polypeptides or polynucleotides, carbohydrates, lipids, etc. The term "purified" may be used to specify the separation of monomeric polypeptides of the invention from oligomeric forms such as homo- or hetero- dimers, trimers, etc. The term "purified" may also be used to specify the separation of covalently closed polynucleotides from linear polynucleotides. A polynucleotide is substantially pure when at least about 50%, preferably 60 to 75% of a sample exhibits a single polynucleotide sequence and conformation (e.g., linear versus covalently closed). A substantially pure polypeptide or polynucleotide typically comprises about 50%, preferably 60 to 90% weight/weight of a polypeptide or polynucleotide sample, respectively, more usually about 95%, and preferably is over about 99% pure. Polypeptide and polynucleotide purity, or homogeneity, is indicated by a number of means well known in the art, such as agarose or polyacrylamide gel electrophoresis of a sample, followed by visualizing a single band upon staining the gel. For certain purposes higher resolution can be provided by using HPLC or other means well known in the art. As an alternative embodiment, purification of the polypeptides and polynucleotides of the present invention may be expressed as "at least" a percent purity relative to heterologous polypeptides and polynucleotides (DNA, RNA or both). As a preferred embodiment, the polypeptides and polynucleotides of the present invention are at least; 10%, 20%, 30%, 40%, 50%, 60%, 70%, 80%, 90%, 95%, 96%, 96%, 98%, 99%, or 100% pure relative to heterologous polypeptides and polynucleotides, respectively. As a further preferred embodiment the polypeptides and polynucleotides have a purity ranging from any number, to the thousandth position, between 90% and 100% (e.g., a polypeptide or polynucleotide at least 99.995% pure) relative to either heterologous polypeptides or polynucleotides, respectively, or as a weight/weight ratio relative to all compounds and molecules other than those existing in the carrier. Each number representing a percent purity, to the thousandth position, may be claimed as individual species of purity.

The term "polypeptide" refers to a polymer of amino acids without regard to the length of the polymer; thus, peptides, oligopeptides, and proteins are included within the definition of polypeptide. This term also does not specify or exclude post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.

The term "recombinant polypeptide" is used herein to refer to polypeptides that have been artificially designed and which comprise at least two polypeptide sequences that are not found as contiguous polypeptide sequences in their initial natural environment, or to refer to polypeptides which have been expressed from a recombinant polynucleotide.

As used herein, the term "non-human animal" refers to any non-human vertebrate, birds and more usually mammals, preferably primates, farm animals such as swine, goats, sheep, donkeys, and horses, rabbits or rodents, more preferably rats or mice. As used herein, the term "animal" is used to refer to any vertebrate, preferable a mammal. Both the terms "animal" and "mammal" expressly embrace human subjects unless preceded with the term "non-human".

As used herein, the term "antibody" refers to a polypeptide or group of polypeptides which are comprised of at least one binding domain, where an antibody binding domain is formed from the folding of variable domains of an antibody molecule to form three-dimensional binding spaces with an internal surface shape and charge distribution complementary to the features of an antigenic determinant of an antigen, which allows an immunological reaction with the antigen. Antibodies include recombinant proteins comprising the binding domains, as wells as fragments, including Fab, Fab', $F(ab)_2$, and $F(ab')_2$ fragments.

As used herein, an "antigenic determinant" is the portion of an antigen molecule, in this case a CanIon polypeptide, that determines the specificity of the antigen-antibody reaction. An "epitope" refers to an antigenic determinant of a polypeptide. An epitope can comprise as few as 3 amino acids in a spatial conformation which is unique to the epitope. Generally an epitope comprises at least 6 such amino acids, and more usually at least 8-10 such amino acids. Methods for determining the amino acids which make up an epitope include x-ray crystallography, 2-dimensional nuclear magnetic resonance, and epitope mapping e.g. the Pepscan method described by Geysen et al. 1984; PCT Publication No. WO 84/03564; and PCT Publication No. WO 84/03506.

Throughout the present specification, the expression "nucleotide sequence" may be employed to designate indifferently a polynucleotide or a nucleic acid. More precisely, the expression "nucleotide sequence" encompasses the nucleic material itself and is thus not restricted to the sequence information (i.e. the succession of letters chosen among the four base letters) that biochemically characterizes a specific DNA or RNA molecule.

As used interchangeably herein, the terms "nucleic acids", "oligonucleotides", and "polynucleotides" include RNA, DNA, or RNA/DNA hybrid sequences of more than one nucleotide in either single chain or duplex form. The term "nucleotide" as used herein as an adjective to describe molecules comprising RNA, DNA, or RNA/DNA hybrid sequences of any length in single-stranded or duplex form. The term "nucleotide" is also used herein as a noun to refer to individual nucleotides or varieties of nucleotides, meaning a molecule, or individual unit in a larger nucleic acid molecule, comprising a purine or pyrimidine, a ribose or deoxyribose sugar moiety, and a phosphate group, or phosphodiester linkage in the case of nucleotides within an oligonucleotide or polynucleotide. Although the term "nucleotide" is also used herein to encompass "modified nucleotides" which comprise at least one modifications (a) an alternative linking group, (b) an analogous form of purine, (c) an analogous form of pyrimidine, or (d) an analogous sugar, for examples of analogous linking groups, purine, pyrimidines, and sugars see for example PCT publication No. WO 95/04064. The polynucleotide sequences of the invention may be prepared by any known method, including synthetic, recombinant, *ex vivo* generation, or a combination thereof, as well as utilizing any purification methods known in the art.

A sequence which is "operably linked" to a regulatory sequence such as a promoter means that said regulatory element is in the correct location and orientation in relation to the nucleic acid to control RNA polymerase initiation and expression of the nucleic acid of interest. As used herein, the term "operably linked" refers to a linkage of polynucleotide elements in a functional relationship. For instance, a promoter or enhancer is operably linked to a coding sequence if it affects the transcription of the coding sequence.

The terms "trait" and "phenotype" are used interchangeably herein and refer to any visible, detectable or otherwise measurable property of an organism such as symptoms of, or susceptibility to, a disease for example. Typically the terms "trait" or "phenotype" are used herein to refer to symptoms of, or susceptibility to a disease, a beneficial response to or side effects related to a treatment. Preferably, said trait can be, without being limited to, psychiatric disorders such as schizophrenia or bipolar disorder, other CNS or neuronal disorders such as epilepsy or pain disorders, as well as cardiovascular conditions such as anginas, hypertension, and arrythmias, as well as any aspect, feature, or characteristic of any of these diseases or conditions.

The term "allele" is used herein to refer to variants of a nucleotide sequence. A biallelic polymorphism has two forms. Diploid organisms may be homozygous or heterozygous for an allelic form.

The term "heterozygosity rate" is used herein to refer to the incidence of individuals in a population which are heterozygous at a particular allele. In a biallelic system, the heterozygosity rate is on average equal to $2P_a(1-P_a)$, where $P_a$ is the frequency of the least common allele. In order to be useful in genetic studies, a genetic marker should have an adequate level of heterozygosity to allow a reasonable probability that a randomly selected person will be heterozygous.

The term "genotype" as used herein refers to the identity of the alleles present in an individual or a sample. In the context of the present invention, a genotype preferably refers to the description of the biallelic marker alleles present in an individual or a sample, e.g. the alleles of biallelic markers within the CanIon gene or genomic region. The term "genotyping" a sample or an individual for a biallelic marker involves determining the specific allele or the specific nucleotide carried by an individual at a biallelic marker.

The term "mutation" as used herein refers to a difference in DNA sequence between or among different genomes or individuals which has a frequency below 1%.

The term "haplotype" refers to a combination of alleles present in an individual or a sample. In the context of the present invention, a haplotype preferably refers to a combination of biallelic marker alleles found in a given individual and which may be associated with a phenotype.

The term "polymorphism" as used herein refers to the occurrence of two or more alternative genomic sequences or alleles between or among different genomes or individuals. "Polymorphic" refers to the condition in which two or more variants of a specific genomic sequence can be found in a population. A "polymorphic site" is the locus at which the variation occurs. A single nucleotide polymorphism is the replacement of one nucleotide by another nucleotide at the polymorphic site. Deletion of a single nucleotide or insertion of a single nucleotide also gives rise to single nucleotide polymorphisms. In the context of the present invention, "single nucleotide polymorphism" preferably refers to a single nucleotide substitution. Typically, between different individuals, the polymorphic site may be occupied by two different nucleotides.

The term "biallelic polymorphism" and "biallelic marker" are used interchangeably herein to refer to a single nucleotide polymorphism having two alleles at a fairly high frequency in the population. A "biallelic marker allele" refers to the nucleotide variants present at a biallelic marker site. Typically, the frequency of the less common allele of the biallelic markers of the present invention has been validated to be greater than 1%, preferably the frequency is greater than 10%, more preferably the frequency is at least 20% (i.e. heterozygosity rate of at least 0.32), even more preferably the frequency is at least 30% (i.e. heterozygosity rate of at least 0.42). A biallelic marker wherein the frequency of the less common allele is 30% or more is termed a "high quality biallelic marker".

The location of nucleotides in a polynucleotide with respect to the center of the polynucleotide are described herein in the following manner. When a polynucleotide has an odd number of nucleotides, the nucleotide at an equal distance from the 3' and 5' ends of the polynucleotide is considered to be "at the center" of the polynucleotide, and any nucleotide immediately adjacent to the nucleotide at the center, or the nucleotide at the center itself is considered to be "within 1 nucleotide of the center." With an odd number ofnucleotides in a polynucleotide any of the five nucleotides positions in the middle of the polynucleotide would be considered to be within 2 nucleotides of the center, and so on. When a polynucleotide has an even number of nucleotides, there would be a bond and not a nucleotide at the center of the polynucleotide. Thus, either of the two central nucleotides would be considered to be "within 1 nucleotide of the center" and any of the four nucleotides in the middle of the polynucleotide would be considered to be "within 2 nucleotides of the center", and so on. For polymorphisms which involve the substitution, insertion or deletion of 1 or more nucleotides, the polymorphism, allele or biallelic marker is "at the center" of a polynucleotide if the difference between the distance from the substituted, inserted, or deleted polynucleotides of the polymorphism and the 3' end of the polynucleotide, and the distance from the substituted, inserted, or deleted polynucleotides of the polymorphism and the 5' end of the polynucleotide is zero or one nucleotide. If this difference is 0 to 3, then the polymorphism is considered to be "within 1 nucleotide of the center." If the difference is 0 to 5, the polymorphism is considered to be "within 2 nucleotides of the center." If the difference is 0 to 7, the polymorphism is considered to be "within 3 nucleotides of the center," and so on.

The term "upstream" is used herein to refer to a location which is toward the 5' end of the polynucleotide from a specific reference point, or, in the case of a gene, in the direction running from the coding sequence to the promoter.

The terms "base paired" and "Watson & Crick base paired" are used interchangeably herein to refer to nucleotides which can be hydrogen bonded to one another be virtue of their sequence identities in a manner like that found in double-helical DNA with thymine or uracil residues linked to adenine residues by two hydrogen bonds and cytosine and guanine residues linked by three hydrogen bonds (See Stryer, L., *Biochemistry,* 4th edition, 1995).

The terms "complementary" or "complement thereof' are used herein to refer to the sequences of polynucleotides which is capable of forming Watson & Crick base pairing with another specified polynucleotide throughout the entirety of the complementary region. For the purpose of the present invention, a first polynucleotide is deemed to be complementary to a second polynucleotide when each base in the first polynucleotide is paired with its complementary base. Complementary bases are, generally, A and T (or A and U), or C and G. "Complement" is used herein as a synonym from "complementary polynucleotide", "complementary nucleic acid" and "complementary nucleotide sequence". These terms are applied to pairs of polynucleotides based solely upon their sequences and not any particular set of conditions under which the two polynucleotides would actually bind.

**Variants and Fragments**

1-Polynucleotides

Suitable for use in the present invention are variants and fragments of the polynucleotides described herein, particularly of a CanIon gene containing one or more biallelic markers.

Variants of polynucleotides, as the term is used herein, are polynucleotides that differ from a reference polynucleotide. A variant of a polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms. Generally, differences are limited so that the nucleotide sequences of the reference and the variant are closely similar overall and, in many regions, identical.

Variants of polynucleotides suitable for use in the present invention include, without being limited to, nucleotide sequences which are at least 95% identical to a polynucleotide selected from the group consisting of the nucleotide sequences of SEQ ID Nos 1 to 4 or to any polynucleotide fragment of at least 12 consecutive nucleotides of a polynucleotide selected from the group consisting of the nucleotide sequences of SEQ ID Nos I to 4, and preferably at least 99% identical, more particularly at least 99.5% identical, and most preferably at least 99.8% identical to a polynucleotide selected from the group consisting of the nucleotide sequences of SEQ ID Nos 1 to 4 or to any polynucleotide fragment of at least 12 consecutive nucleotides of a polynucleotide selected from the group consisting of the nucleotide sequences of SEQ ID No 1 to 4.

Nucleotide changes present in a variant polynucleotide may be silent, which means that they do not alter the amino acids encoded by the polynucleotide. However, nucleotide changes may also result in amino acid substitutions, additions, deletions, fusions and truncations in the polypeptide encoded by the reference sequence. The substitutions, deletions or additions may involve one or more nucleotides. The variants may be altered in coding or non-coding regions or both. Alterations in the coding regions may produce conservative or non-conservative amino acid substitutions, deletions or additions.

Particularly preferred embodiments suitable for use in the present invention are those in which the polynucleotides encode polypeptides which retain substantially the same biological function or activity as the mature CanIon protein, or those in which the polynucleotides encode polypeptides which maintain or increase a particular biological activity, while reducing a second biological activity

A polynucleotide fragment is a polynucleotide having a sequence that is entirely the same as part but not all of a given nucleotide sequence, preferably the nucleotide sequence of a CanIon gene, and variants thereof. The fragment can be a portion of an intron or an exon of a CanIon gene. It can also be a portion of the regulatory regions of CanIon. Preferably, such fragments comprise at least one of the biallelic markers A1 to A17 or the complements thereto or a biallelic marker in linkage disequilibrium with one or more of the biallelic markers A1 to A17.

Such fragments may be "free-standing", i.e. not part of or fused to other polynucleotides, or they may be comprised within a single larger polynucleotide of which they form a part or region. Indeed, several of these fragments may be present within a single larger polynucleotide.

Optionally, such fragments may consist of, or consist essentially of, a contiguous span of at least 8, 10, 12, 15, 18, 20, 25, 35, 40, 50, 70, 80, 100, 250, 500 or 1000 nucleotides in length. A set of preferred fragments contain at least one of the biallelic markers A1 to A17 of the CanIon gene which are described herein or the complements thereto.

2- Polypeptides

Suitable for use in the present invention are variants, fragments, analogs and derivatives of the polypeptides described herein, including mutated CanIon proteins.

The variant may be 1) one in which one or more of the amino acid residues are substituted with a conserved or non-conserved amino acid residue and such substituted amino acid residue may or may not be one encoded by the genetic code, or 2) one in which one or more of the amino acid residues includes a substituent group, or 3) one in which the mutated CanIon is fused with another compound, such as a compound to increase the half-life of the polypeptide (for example, polyethylene glycol), or 4) one in which the additional amino acids are fused to the mutated CanIon, such as a leader or secretory sequence or a sequence which is employed for purification of the mutated CanIon or a preprotein sequence. Such variants are deemed to be within the scope of those skilled in the art.

A polypeptide fragment is a polypeptide having a sequence that entirely is the same as part but not all of a given polypeptide sequence, preferably a polypeptide encoded by a CanIon gene and variants thereof.

In the case of an amino acid substitution in the amino acid sequence of a polypeptide according to the invention, one or several amino acids can be replaced by "equivalent" amino acids. The expression "equivalent" amino acid is used herein to designate any amino acid that may be substituted for one of the amino acids having similar properties, such that one skilled in the art of peptide chemistry would expect the secondary structure and hydropathic nature of the polypeptide to be substantially unchanged. Generally, the following groups of amino acids represent equivalent changes: (1) Ala, Pro, Gly, Glu, Asp, Gln, Asn, Ser, Thr, (2) Cys, Ser, Tyr, Thr; (3) Val, Ile, Leu, Met, Ala, Phe; (4) Lys, Arg, His; (5) Phe, Tyr, Trp, His.

A specific embodiment of a modified CanIon peptide molecule of interest suitable for use in the present invention includes, but is not limited to, a peptide molecule which is resistant to proteolysis, is a peptide in which the -CONH- peptide bond is modified and replaced by a (CH2NH) reduced bond, a (NHCO) retro inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH- bond. Suitable for use in the present invention is a human CanIon polypeptide or a fragment or a variant thereof in which at least one peptide bond has been modified as described above.

Such fragments may be "free-standing", i.e. not part of or fused to other polypeptides, or they may be comprised within a single larger polypeptide of which they form a part or region. However, several fragments may be comprised within a single larger polypeptide.

As representative examples of polypeptide fragment suitable for use in the invention, there may be mentioned those which have from about 5, 6, 7, 8, 9 or 10 to 15, 10 to 20, 15 to 40, or 30 to 55 amino acids long. Preferred are those fragments containing at least one amino acid mutation in the CanIon protein.

**Identity Between Nucleic Acids Or Polypeptides**

The terms "percentage of sequence identity" and "percentage homology" are used interchangeably herein to refer to comparisons among polynucleotides and polypeptides, and are determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Homology is evaluated using any of the variety of sequence comparison algorithms and programs known in the art. Such algorithms and programs include, but are by no means limited to, TBLASTN, BLASTP, FASTA, TFASTA, and CLUSTALW (Pearson and Lipman, 1988; Altschul et al., 1990; Thompson et al., 1994; Higgins et al., 1996; Altschul et al., 1990; Altschul et al., 1993). In a particularly preferred embodiment, protein and nucleic acid sequence homologies are evaluated using the Basic Local Alignment Search Tool ("BLAST") which is well known in the art (see, e.g., Karlin and Altschul, 1990; Altschul et al., 1990, 1993, 1997). In particular, five specific BLAST programs are used to perform the following task:

(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (i.e., aligned) by means of a scoring matrix, many of which are known in the art. Preferably, the scoring matrix used is the BLOSUM62 matrix (Gonnet et al., 1992; Henikoff and Henikoff, 1993). Less preferably, the PAM or PAM250 matrices may also be used (see, e.g., Schwartz and Dayhoff, eds., 1978). The BLAST programs evaluate the statistical significance of all high-scoring segment pairs identified, and preferably selects those segments which satisfy a user-specified threshold of significance, such as a user-specified percent homology. Preferably, the statistical significance of a high-scoring segment pair is evaluated using the statistical significance formula of Karlin (see, e.g., Karlin and Altschul, 1990).The BLAST programs may be used with the default parameters or with modified parameters provided by the user.

**Stringent Hybridization Conditions**

For the purpose of defining such a hybridizing nucleic acid, the stringent hybridization conditions are the following:

the hybridization step is carried out at 65°C in the presence of 6 x SSC buffer, 5 x Denhardt's solution, 0,5% SDS and 100μg/ml of salmon sperm DNA.

The hybridization step is followed by four washing steps :

- two washings of 5 min, preferably at 65°C in a 2 x SSC and 0.1%SDS buffer;
- one washing of 30 min, preferably at 65°C in a 2 x SSC and 0.1% SDS buffer,
- one washing of 10 min, preferably at 65°C in a 0.1 x SSC and 0.1%SDS buffer,

these hybridization conditions being suitable for a nucleic acid molecule of about 20 nucleotides in length. There is no need to say that the hybridization conditions described above are to be adapted according to the length of the desired nucleic acid, following techniques well known to the one skilled in the art. The suitable hybridization conditions may for example be adapted according to the teachings disclosed in the book of Hames and Higgins (1985).

**Genomic Sequences Of The CanIon Gene**

Suitable for use in the present invention is the CanIon gene, or CanIon genomic sequences consisting of, consisting essentially of, or comprising the sequence of SEQ ID Nos 1 to 3, a sequence complementary thereto, as well as fragments and variants thereof. These polynucleotides may be purified, isolated, or recombinant.

Suitable for use in the present invention is a purified, isolated, or recombinant polynucleotide comprising a nucleotide sequence having at least 70, 75, 80, 85, 90, or 95% nucleotide identity with a nucleotide sequence of SEQ ID Nos 1 to 3 or a complementary sequence thereto or a fragment thereof. The nucleotide differences as regards to the nucleotide sequence of SEQ ID Nos 1 to 3 may be generally randomly distributed throughout the entire nucleic acid. Nevertheless, preferred nucleic acids are those wherein the nucleotide differences as regards to the nucleotide sequence of SEQ ID Nos 1 to 3 are predominantly located outside the coding sequences contained in the exons. These nucleic acids, as well as their fragments and variants, may be used as oligonucleotide primers or probes in order to detect the presence of a copy of the CanIon gene in a test sample, or alternatively in order to amplify a target nucleotide sequence within the CanIon sequences.

Suitable for use in the present invention is a purified, isolated, or recombinant nucleic acid that hybridizes with the nucleotide sequence of SEQ ID Nos 1 to 3 or a complementary sequence thereto or a variant thereof, under the stringent hybridization conditions as defined above. In preferred embodiments, said purified, isolated, or recombinant nucleic acid hybridizes specifically with the polynucleotides of the human CanIon gene, more preferably said nucleic acid is capable of hybridizing to the nucleotides of the human CanIon gene but is substantially incapable of hybridizing to nucleic sequence of the rat CanIon gene.

Particularly preferred nucleic acids suitable for use in the present invention include isolated, purified, or recombinant polynucleotides comprising a contiguous span of at least 12, 15, 18, 20, 25, 30, 35, 40, 50, 60, 70, 80, 90, 100, 150, 200, 500, 1000, 2000, 3000, 4000, 5000 or 10000 nucleotides of SEQ ID No 1 to 3 or the complements thereof. It should be noted that nucleic acid fragments of any size and sequence may also be comprised by the polynucleotides described in this section.

The CanIon genomic nucleic acid comprises 44 exons. The exon positions in SEQ ID No 1 to 3 are detailed below in Table B.

**Table B**

| Exon | Position in SEQ ID No 1 | | Intron | Position in SEQ ID No 1 | |
|---|---|---|---|---|---|
| | Beginning | End | | Beginning | End |
| 1 | 2001 | 2026 | 1 | 2027 | 19187 |
| 2 | 19188 | 19334 | 2 | 19335 | 22995 |
| 3 | 22996 | 23178 | 3 | 23179 | 39730 |
| 4 | 39731 | 39814 | 4 | 39815 | 41336 |
| 5 | 41337 | 41476 | 5 | 41477 | 41564 |
| 6 | 41565 | 41693 | 6 | 41694 | 73012 |
| 7 | 73013 | 73167 | | | |
| | | | | | |
| Exon | Position in SEQ ID No 2 | | Intron | Position in SEQ ID No 2 | |
| | Beginning | End | | Beginning | End |
| 8 | 43726 | 43868 | 7 | 43869 | 43997 |
| 9 | 43998 | 44102 | 8 | 44103 | 52092 |
| 10 | 52093 | 52179 | 9 | 52180 | 77567 |
| 11 | 77568 | 77699 | 10 | 77700 | 98225 |
| 12 | 98226 | 98393 | 11 | 98394 | 106566 |
| 13 | 106567 | 106758 | 12 | 106759 | 144108 |
| 14 | 144109 | 144246 | 13 | 144247 | 159793 |
| 15 | 159794 | 159868 | 14 | 159869 | 191291 |
| 16 | 191292 | 191428 | 15 | 191429 | 192966 |
| 17 | 192967 | 193108 | 16 | 193109 | 211539 |
| 18 | 211540 | 211613 | 17 | 211614 | 225005 |
| 19 | 225006 | 225107 | 18 | 225108 | 225543 |
| 20 | 225544 | 225613 | 19 | 225614 | 228449 |
| 21 | 228450 | 228541 | 20 | 228542 | 228629 |
| 22 | 228630 | 228752 | 21 | 228753 | 231288 |
| 23 | 231289 | 231345 | 22 | 231346 | 231588 |
| 24 | 231589 | 231709 | 23 | 231710 | 231812 |
| 25 | 231813 | 231944 | 24 | 231945 | 232899 |
| 26 | 232900 | 233067 | 25 | 233068 | 235354 |
| 27 | 235355 | 235459 | | | |
| | | | | | |
| Exon | Position in SEQ ID No 3 | | Intron | Position in SEQ ID No 3 | |
| | Beginning | End | | Beginning | End |
| 28 | 3895 | 4001 | 26 | 4002 | 9610 |
| 29 | 9611 | 9731 | 27 | 9732 | 9815 |

(continued)

| Exon | Position in SEQ ID No 3 | | Intron | Position in SEQ ID No 3 | |
|---|---|---|---|---|---|
| | Beginning | End | | Beginning | End |
| 30 | 9816 | 9914 | 28 | 9915 | 15775 |
| 31 | 15776 | 15869 | 29 | 15870 | 16381 |
| 32 | 16382 | 16488 | 30 | 16489 | 16696 |
| 33 | 16697 | 16771 | 31 | 16772 | 17933 |
| 34 | 17934 | 18053 | 32 | 18054 | 23643 |
| 35 | 23644 | 23712 | 33 | 23713 | 24927 |
| 36 | 24928 | 25076 | 34 | 25077 | 25912 |
| 37 | 25913 | 26006 | 35 | 26007 | 30766 |
| 38 | 30767 | 30899 | 36 | 30900 | 31560 |
| 39 | 31561 | 31676 | 37 | 31677 | 34043 |
| 40 | 34044 | 34201 | 38 | 34202 | 37492 |
| 41 | 37493 | 37643 | 39 | 37644 | 39651 |
| 42 | 39652 | 39801 | 40 | 39802 | 41562 |
| 43 | 41563 | 41680 | 41 | 41681 | 44130 |
| 44 | 44131 | 45841 | | | |

Thus, suitable for use in the invention are purified, isolated, or recombinant polynucleotides comprising a nucleotide sequence selected from the group consisting of each of the 44 exons of the CanIon gene and each of the sequences complementary thereto. The invention also provides purified, isolated, or recombinant nucleic acids comprising a combination of at least two exons of the CanIon gene, wherein the polynucleotides are arranged within the nucleic acid, from the 5'-end to the 3'-end of said nucleic acid, in the same order as in SEQ ID No 1 to 3.

Intron 1 refers to the nucleotide sequence located between Exon 1 and Exon 2, and so on. The position of the introns is detailed in Table A. Thus, suitable for use in the invention are purified, isolated, or recombinant polynucleotides comprising a nucleotide sequence selected from the group consisting of the 43 introns of the CanIon gene, or a sequence complementary thereto.

While this section is entitled "Genomic Sequences of CanIon," it should be noted that nucleic acid fragments of any size and sequence may also be comprised by the polynucleotides described in this section, flanking the genomic sequences of CanIon on either side or between two or more such genomic sequences.

**CanIon cDNA Sequences**

The expression of the CanIon gene has been shown to lead to the production of at least one mRNA species, the nucleic acid sequence of which is set forth in SEQ ID No 4.

Suitable for use in the present invention is a purified, isolated, or recombinant nucleic acid comprising the nucleotide sequence of SEQ ID No 4, complementary sequences thereto, as well as allelic variants, and fragments thereof. Moreover, preferred polynucleotides of the invention include purified, isolated, or recombinant CanIon cDNAs consisting of, consisting essentially of, or comprising the sequence of SEQ ID No 4. Particularly preferred nucleic acids of the invention include isolated, purified, or recombinant polynucleotides comprising a contiguous span of at least 12, 15, 18, 20, 25, 30, 35, 40, 50, 60, 70, 80, 90, 100, 150, 200, 500, 1000, 2000, 3000, 4000, 5000 or 6000 nucleotides of SEQ ID No 4 or the complements thereof. In preferred embodiments, said contiguous span comprises a CanIon-related biallelic marker; preferably selected from the group consisting of A12 and A16.

Suitable for use in the present invention is a purified or isolated nucleic acid comprising a polynucleotide having at least 95% nucleotide identity with a polynucleotide of SEQ ID No 4, advantageously 99% nucleotide identity, preferably 99.5% nucleotide identity and most preferably 99.8% nucleotide identity with a polynucleotide of SEQ ID No 4, or a sequence complementary thereto or a biologically active fragment thereof.

Suitable for use in the invention are purified, isolated or recombinant nucleic acids comprising a polynucleotide that hybridizes, under the stringent hybridization conditions defined herein, with a polynucleotide of SEQ ID No 4, or a sequence complementary thereto or a variant thereof or a biologically active fragment thereof.

Suitable for use in the invention is an isolated, purified, or recombinant polynucleotide which encodes a CanIon polypeptide comprising a contiguous span of at least 6 amino acids of SEQ ID No 5, wherein said contiguous span includes at least 1, 2, 3, 5 or 10 of the amino acid positions 277, 338, 574, 678, 680, 683, 691, 692, 695, 696, 697, 894, 1480, 1481, 1483, 1484, 1485, 1630, 1631, 1632, 1636, 1660, 1667, 1707, 1709 of SEQ ID No 5. Also suitable for use in the invention is an isolated, purified, or recombinant polynucleotide which encodes a CanIon polypeptide comprising the amino acid sequence of SEQ ID No 5, or derivatives or biologically active fragments thereof, as well as an isolated, purified, or recombinant polynucleotide which encodes a CanIon polypeptide at least 80, 85, 90, 95, 98, 99, 99.5 or 99.8% identical to the amino acid sequence of SEQ ID No 5.

The cDNA of SEQ ID No 4 includes a 5'-UTR region starting from the nucleotide at position 1 and ending at the nucleotide in position 65 of SEQ ID No 4. The cDNA of SEQ ID No cDNA includes a 3'-UTR region starting from the nucleotide at position 5283 and ending at the nucleotide at position 6799 of SEQ ID No 4.

Consequently, suitable for use in the invention is a purified, isolated, and recombinant nucleic acid comprising a nucleotide sequence of the 5'UTR of the CanIon cDNA, a sequence complementary thereto, or an allelic variant thereof. Also suitable for use in the invention is a purified, isolated, and recombinant nucleic acid comprising a nucleotide sequence of the 3'UTR of the CanIon cDNA, a sequence complementary thereto, or an allelic variant thereof.

While this section is entitled "CanIon cDNA Sequences," it should be noted that nucleic acid fragments of any size and sequence may also be comprised by the polynucleotides described in this section, flanking the genomic sequences of CanIon on either side or between two or more such genomic sequences.

## Coding Regions

The CanIon open reading frame is contained in the corresponding mRNA of SEQ ID No cDNA. More precisely, the effective CanIon coding sequence (CDS) includes the region between nucleotide position 66 (first nucleotide of the ATG codon) and nucleotide position 5282 (end nucleotide of the TGA codon) of SEQ ID No 4. The present invention also embodies isolated, purified, and recombinant polynucleotides which encode a polypeptides comprising a contiguous span of at least 6 amino acids, preferably at least 8 or 10 amino acids, more preferably at least 12, 15, 20, 25, 30, 40, 50, 100, 200, 300, 400, 500, 700 or 1000 amino acids of SEQ ID No 5.

The above disclosed polynucleotide that contains the coding sequence of the CanIon gene may be expressed in a desired host cell or a desired host organism, when this polynucleotide is placed under the control of suitable expression signals. The expression signals may be either the expression signals contained in the regulatory regions in the CanIon gene of the invention or in contrast the signals may be exogenous regulatory nucleic sequences. Such a polynucleotide, when placed under the suitable expression signals, may also be inserted in a vector for its expression and/or amplification.

## Regulatory Sequences Of CanIon

As mentioned, the genomic sequence of the CanIon gene contains regulatory sequences both in the non-coding 5'-flanking region and in the non-coding 3'-flanking region that border the CanIon coding region containing the 44 exons of this gene.

Polynucleotides derived from the 5' and 3' regulatory regions are useful in order to detect the presence of at least a copy of a CanIon nucleotide sequence or a fragment thereof in a test sample.

The promoter activity of the 5' regulatory regions contained in CanIon can be assessed as described as follows. In order to identify the relevant biologically active polynucleotide fragments or variants of SEQ ID No 1, one of skill in the art may refer to Sambrook et al.(1989) which describes the use of a recombinant vector carrying a marker gene (i.e. beta galactosidase, chloramphenicol acetyl transferase, etc.), the expression of which can be detected when placed under the control of a biologically active polynucleotide fragments or variants of SEQ ID No 1. Genomic sequences located upstream of the first exon of the CanIon gene are cloned into a suitable promoter reporter vector, such as the pSEAP-Basic, pSEAP-Enhancer, pβgal-Basic, pβgal-Enhancer, or pEGFP-1 Promoter Reporter vectors available from Clontech, or pGL2-basic or pGL3-basic promoterless luciferase reporter gene vector from Promega. Briefly, each of these promoter reporter vectors include multiple cloning sites positioned upstream of a reporter gene encoding a readily assayable protein such as secreted alkaline phosphatase, luciferase, β galactosidase, or green fluorescent protein. The sequences upstream the CanIon coding region are inserted into the cloning sites upstream of the reporter gene in both orientations and introduced into an appropriate host cell. The level of reporter protein is assayed and compared to the level obtained from a vector which lacks an insert in the cloning site. The presence of an elevated expression level in the vector containing the insert in comparison to the level in the control vector indicates the presence of a promoter in the insert. If necessary, the upstream sequences can be cloned into vectors which contain an enhancer for increasing transcription levels from weak promoter sequences. A significant level of expression above that observed with the vector lacking an insert indicates that a promoter sequence is present in the inserted upstream sequence.

Promoter sequence within the upstream genomic DNA may be further defined by constructing nested 5' and/or 3' deletions in the upstream DNA using conventional techniques such as Exonuclease III or appropriate restriction endonuclease digestion. The resulting deletion fragments can be inserted into the promoter reporter vector to determine whether the deletion has reduced or obliterated promoter activity, such as described, for example, by Coles et al. (1998). In this way, the boundaries of the promoters may be defined If desired, potential individual regulatory sites within the promoter may be identified using site directed mutagenesis or linker scanning to obliterate potential transcription factor binding sites within the promoter individually or in combination. The effects of these mutations on transcription levels may be determined by inserting the mutations into cloning sites in promoter reporter vectors. This type of assay is well-known to those skilled in the art and is described in WO 97/17359, US Patent No. 5,374,544; EP 582 796; US Patent No. 5,698,389; US 5,643,746; US Patent No. 5,502,176; and US Patent 5,266,488.

The strength and the specificity of the promoter of the CanIon gene can be assessed through the expression levels of a detectable polynucleotide operably linked to the CanIon promoter in different types of cells and tissues. The detectable polynucleotide may be either a polynucleotide that specifically hybridizes with a predefined oligonucleotide probe, or a polynucleotide encoding a detectable protein, including a CanIon polypeptide or a fragment or a variant thereof. This type of assay is well-known to those skilled in the art and is described in US Patent No. 5,502,176; and US Patent No. 5,266,488. Some of the methods are discussed in more detail below.

Polynucleotides carrying the regulatory elements located at the 5' end and at the 3' end of the CanIon coding region may be advantageously used to control the transcriptional and translational activity of an heterologous polynucleotide of interest.

Thus, suitable for use in the present invention is a purified or isolated nucleic acid comprising a polynucleotide which is selected from the group consisting of the 5' and 3' regulatory regions, or a sequence complementary thereto or a biologically active fragment or variant thereof. In preferred embodiments, "5' regulatory region" is located in the nucleotide sequence located between positions 1 and 2000 of SEQ ID No 1. The "3' regulatory region" is located in the nucleotide sequence located between positions 45842 and 47841 of SEQ ID No 3.

Suitable for use in the invention is a purified or isolated nucleic acid comprising a polynucleotide having at least 95% nucleotide identity with a polynucleotide selected from the group consisting of the 5' and 3' regulatory regions, advantageously 99% nucleotide identity, preferably 99.5% nucleotide identity and most preferably 99.8% nucleotide identity with a polynucleotide selected from the group consisting of the 5' and 3' regulatory regions, or a sequence complementary thereto or a variant thereof or a biologically active fragment thereof.

Suitable for use in the invention are purified, isolated or recombinant nucleic acids comprising a polynucleotide that hybridizes, under the stringent hybridization conditions defined herein, with a polynucleotide selected from the group consisting of the nucleotide sequences of the 5'- and 3' regulatory regions, or a sequence complementary thereto or a variant thereof or a biologically active fragment thereof.

Preferred fragments of the 5' regulatory region have a length of about 1500 or 1000 nucleotides, preferably of about 500 nucleotides, more preferably about 400 nucleotides, even more preferably 300 nucleotides and most preferably about 200 nucleotides.

Preferred fragments of the 3' regulatory region are at least 50, 100, 150, 200, 300 or 400 bases in length.

"Biologically active" polynucleotide derivatives of SEQ ID Nos 1 and 3 are polynucleotides comprising or alternatively consisting in a fragment of said polynucleotide which is functional as a regulatory region for expressing a recombinant polypeptide or a recombinant polynucleotide in a recombinant cell host. It could act either as an enhancer or as a repressor.

For the purpose of the invention, a nucleic acid or polynucleotide is "functional" as a regulatory region for expressing a recombinant polypeptide or a recombinant polynucleotide if said regulatory polynucleotide contains nucleotide sequences which contain transcriptional and translational regulatory information, and such sequences are "operably linked" to nucleotide sequences which encode the desired polypeptide or the desired polynucleotide.

The regulatory polynucleotides suitable for use in the invention may be prepared from the nucleotide sequence of SEQ ID Nos 1 and 3 by cleavage using suitable restriction enzymes, as described for example in the book of Sambrook et al.(1989). The regulatory polynucleotides may also be prepared by digestion of SEQ ID Nos 1 and 3 by an exonuclease enzyme, such as Bal31 (Wabiko et al., 1996). These regulatory polynucleotides can also be prepared by nucleic acid chemical synthesis, as described elsewhere in the specification.

The regulatory polynucleotides suitable for use in the invention may be part of a recombinant expression vector that may be used to express a coding sequence in a desired host cell or host organism. The recombinant expression vectors according to the invention are described elsewhere in the specification.

A preferred 5'-regulatory polynucleotide suitable for use in the invention includes the 5'-untranslated region (5'-UTR) of the CanIon cDNA, or a biologically active fragment or variant thereof.

A preferred 3'-regulatory polynucleotide suitable for use in the invention includes the 3'-untranslated region (3'-UTR) of the CanIon cDNA, or a biologically active fragment or variant thereof.

A further object of the invention consists of a purified or isolated nucleic acid comprising:

a) a nucleic acid comprising a regulatory nucleotide sequence selected from the group consisting of:

(i) a nucleotide sequence comprising a polynucleotide of the 5' regulatory region or a complementary sequence thereto;
(ii) a nucleotide sequence comprising a polynucleotide having at least 95% of nucleotide identity with the nucleotide sequence of the 5' regulatory region or a complementary sequence thereto;
(iii) a nucleotide sequence comprising a polynucleotide that hybridizes under stringent hybridization conditions with the nucleotide sequence of the 5' regulatory region or a complementary sequence thereto; and
(iv) a biologically active fragment or variant of the polynucleotides in (i), (ii) and (iii);

b) a polynucleotide encoding a desired polypeptide or a nucleic acid of interest, operably linked to the nucleic acid defined in (a) above;
c) Optionally, a nucleic acid comprising a 3'- regulatory polynucleotide, preferably a 3'- regulatory polynucleotide of the CanIon gene.

In a specific embodiment of the nucleic acid defined above, said nucleic acid includes the 5'-untranslated region (5'-UTR) of the CanIon cDNA, or a biologically active fragment or variant thereof.

In a second specific embodiment of the nucleic acid defined above, said nucleic acid includes the 3'-untranslated region (3'-UTR) of the CanIon cDNA, or a biologically active fragment or variant thereof.

The regulatory polynucleotide of the 5' regulatory region, or its biologically active fragments or variants, is operably linked at the 5'-end of the polynucleotide encoding the desired polypeptide or polynucleotide.

The regulatory polynucleotide of the 3' regulatory region, or its biologically active fragments or variants, is advantageously operably linked at the 3'-end of the polynucleotide encoding the desired polypeptide or polynucleotide.

The desired polypeptide encoded by the above-described nucleic acid may be of various nature or origin, encompassing proteins of prokaryotic or eukaryotic origin. Among the polypeptides expressed under the control of a CanIon regulatory region include bacterial, fungal or viral antigens. Also encompassed are eukaryotic proteins such as intracellular proteins, like "house keeping" proteins, membrane-bound proteins, like receptors, and secreted proteins like endogenous mediators such as cytokines. The desired polypeptide may be the CanIon protein, especially the protein of the amino acid sequence of SEQ ID No 5, or a fragment or a variant thereof.

The desired nucleic acids encoded by the above-described polynucleotide, usually an RNA molecule, may be complementary to a desired coding polynucleotide, for example to the CanIon coding sequence, and thus useful as an antisense polynucleotide.

Such a polynucleotide may be included in a recombinant expression vector in order to express the desired polypeptide or the desired nucleic acid in host cell or in a host organism. Suitable recombinant vectors that contain a polynucleotide such as described herein are disclosed elsewhere in the specification.

## Polynucleotide Constructs

The terms "polynucleotide construct" and "recombinant polynucleotide" are used interchangeably herein to refer to linear or circular, purified or isolated polynucleotides that have been artificially designed and which comprise at least two nucleotide sequences that are not found as contiguous nucleotide sequences in their initial natural environment.

### DNA Construct That Enables Directing Temporal And Spatial CanIon Gene Expression In Recombinant Cell Hosts And In Transgenic Animals.

In order to study the physiological and phenotypic consequences of a lack of synthesis of the CanIon protein, both at the cellular level and at the multi cellular organism level, the invention also encompasses DNA constructs and recombinant vectors enabling a conditional expression of a specific allele of the CanIon genomic sequence or cDNA and also of a copy of this genomic sequence or cDNA harboring substitutions, deletions, or additions of one or more bases as regards to the CanIon nucleotide sequence of SEQ ID Nos 1 to 4, or a fragment thereof, these base substitutions, deletions or additions being located either in an exon, an intron or a regulatory sequence, but preferably in the 5'-regulatory sequence or in an exon of the CanIon genomic sequence or within the CanIon cDNA of SEQ ID No 4. In a preferred embodiment, the CanIon sequence comprises a biallelic marker suitable for use in the present invention. In a preferred embodiment, the CanIon sequence comprises a biallelic marker suitable for use in the present invention, preferably one of the biallelic markers A1 to A17.

Suitable for use in the invention are recombinant vectors comprising any one of the polynucleotides described in the present invention. More particularly, the polynucleotide constructs according to the present invention can comprise any of the polynucleotides described in the "Genomic Sequences Of The CanIon Gene" section, the "CanIon cDNA Sequences" section, the "Coding Regions" section, and the "Oligonucleotide Probes And Primers" section.

A first preferred DNA construct is based on the tetracycline resistance operon *tet* from *E. coli* transposon Tn10 for controlling the CanIon gene expression, such as described by Gossen et al. (1992, 1995) and Furth et al. (1994). Such a DNA construct contains seven *tet* operator sequences from Tn10 (*tet*op) that are fused to either a minimal promoter or a 5'-regulatory sequence of the CanIon gene, said minimal promoter or said CanIon regulatory sequence being operably linked to a polynucleotide of interest that codes either for a sense or an antisense oligonucleotide or for a polypeptide, including a CanIon polypeptide or a peptide fragment thereof. This DNA construct is functional as a conditional expression system for the nucleotide sequence of interest when the same cell also comprises a nucleotide sequence coding for either the wild type (tTA) or the mutant (rTA) repressor fused to the activating domain of viral protein VP16 of herpes simplex virus, placed under the control of a promoter, such as the HCMVIE1 enhancer/promoter or the MMTV-LTR- Indeed, a preferred DNA construct of the invention comprise both the polynucleotide containing the *tet* operator sequences and the polynucleotide containing a sequence coding for the tTA or the rTA repressor.

In a specific embodiment, the conditional expression DNA construct contains the sequence encoding the mutant tetracycline repressor rTA, the expression of the polynucleotide of interest is silent in the absence of tetracycline and induced in its presence.

**DNA Constructs Allowing Homologous Recombination: Replacement Vectors**

A second preferred DNA construct will comprise, from 5'-end to 3'-end: (a) a first nucleotide sequence that is comprised in the CanIon genomic sequence; (b) a nucleotide sequence comprising a positive selection marker, such as the marker for neomycine resistance *(neo);* and (c) a second nucleotide sequence that is comprised in the CanIon genomic sequence, and is located on the genome downstream the first CanIon nucleotide sequence (a).

In a preferred embodiment, this DNA construct also comprises a negative selection marker located upstream the nucleotide sequence (a) or downstream the nucleotide sequence (c). Preferably, the negative selection marker comprises the thymidine kinase *(tk)* gene (Thomas et al., 1986), the hygromycine beta gene (Te Riele et al., 1990), the *hprt* gene (Van der Lugt et al., 1991; Reid et al., 1990) or the Diphteria toxin A fragment (*Dt-A*) gene (Nada et al., 1993; Yagi et al. 1990). Preferably, the positive selection marker is located within a CanIon exon sequence so as to interrupt the sequence encoding a CanIon protein. These replacement vectors are described, for example, by Thomas et al. (1986; 1987), Mansour et al. (1988) and Koller et al. (1992).

The first and second nucleotide sequences (a) and (c) may be indifferently located within a CanIon regulatory sequence, an intronic sequence, an exon sequence or a sequence containing both regulatory and/or intronic and/or exon sequences. The size of the nucleotide sequences (a) and (c) ranges from 1 to 50 kb, preferably from 1 to 10 kb, more preferably from 2 to 6 kb and most preferably from 2 to 4 kb.

**DNA Constructs Allowing Homologous Recombination: Cre-LoxP System.**

These new DNA constructs make use of the site specific recombination system of the P1 phage. The P1 phage possesses a recombinase called Cre which interacts specifically with a 34 base pairs *lox*P site. The *lox*P site is composed of two palindromic sequences of 13 bp separated by a 8 bp conserved sequence (Hoess et al., 1986). The recombination by the Cre enzyme between two *lox*P sites having an identical orientation leads to the deletion of the DNA fragment.

The Cre-*lox*P system used in combination with a homologous recombination technique has been first described by Gu et al. (1993, 1994). Briefly, a nucleotide sequence of interest to be inserted in a targeted location of the genome harbors at least two *lox*P sites in the same orientation and located at the respective ends of a nucleotide sequence to be excised from the recombinant genome. The excision event requires the presence of the recombinase (Cre) enzyme within the nucleus of the recombinant cell host. The recombinase enzyme may be brought at the desired time either by (a) incubating the recombinant cell hosts in a culture medium containing this enzyme, by injecting the Cre enzyme directly into the desired cell, such as described by Araki et al. (1995), or by lipofection of the enzyme into the cells, such as described by Baubonis et al. (1993); (b) transfecting the cell host with a vector comprising the *Cre* coding sequence operably linked to a promoter functional in the recombinant cell host, which promoter being optionally inducible, said vector being introduced in the recombinant cell host, such as described by Gu et al. (1993) and Sauer et al. (1988); (c) introducing in the genome of the cell host a polynucleotide comprising the *Cre* coding sequence operably linked to a promoter functional in the recombinant cell host, which promoter is optionally inducible, and said polynucleotide being inserted in the genome of the cell host either by a random insertion event or an homologous recombination event, such as described by Gu et al. (1994).

In a specific embodiment, the vector containing the sequence to be inserted in the CanIon gene by homologous recombination is constructed in such a way that selectable markers are flanked by *lox*P sites of the same orientation, it is possible, by treatment by the Cre enzyme, to eliminate the selectable markers while leaving the CanIon sequences of interest that have been inserted by an homologous recombination event. Again, two selectable markers are needed: a positive selection marker to select for the recombination event and a negative selection marker to select for the homologous recombination event. Vectors and methods using the Cre-*lox*P system are described by Zou et al. (1994).

Thus, a third preferred DNA construct suitable for use in the invention comprises, from 5'-end to 3'-end: (a) a first nucleotide sequence that is comprised in the CanIon genomic sequence; (b) a nucleotide sequence comprising a polynucleotide encoding a positive selection marker, said nucleotide sequence comprising additionally two sequences defining a site recognized by a recombinase, such as a *lox*P site, the two sites being placed in the same orientation; and (c) a second nucleotide sequence that is comprised in the CanIon genomic sequence, and is located on the genome downstream of the first CanIon nucleotide sequence (a).

The sequences defining a site recognized by a recombinase, such as a *lox*P site, are preferably located within the nucleotide sequence (b) at suitable locations bordering the nucleotide sequence for which the conditional excision is sought. In one specific embodiment, two *lox*P sites are located at each side of the positive selection marker sequence, in order to allow its excision at a desired time after the occurrence of the homologous recombination event.

In a preferred embodiment of a method using the third DNA construct described above, the excision of the polynucleotide fragment bordered by the two sites recognized by a recombinase, preferably two loxP sites, is performed at a desired time, due to the presence within the genome of the recombinant host cell of a sequence encoding the Cre enzyme operably linked to a promoter sequence, preferably an inducible promoter, more preferably a tissue-specific promoter sequence and most preferably a promoter sequence which is both inducible and tissue-specific, such as described by Gu et al. (1994).

The presence of the Cre enzyme within the genome of the recombinant cell host may result from the breeding of two transgenic animals, the first transgenic animal bearing the CanIon-derived sequence of interest containing the *lox*P sites as described above and the second transgenic animal bearing the Cre coding sequence operably linked to a suitable promoter sequence, such as described by Gu et al. (1994).

Spatio-temporal control of the Cre enzyme expression may also be achieved with an adenovirus based vector that contains the Cre gene thus allowing infection of cells, or *in vivo* infection of organs, for delivery of the Cre enzyme, such as described by Anton and Graham (1995) and Kanegae et al. (1995).

The DNA constructs described above may be used to introduce a desired nucleotide sequence suitable for use in the invention, preferably a CanIon genomic sequence or a CanIon cDNA sequence, and most preferably an altered copy of a CanIon genomic or cDNA sequence, within a predetermined location of the targeted genome, leading either to the generation of an altered copy of a targeted gene (knockout homologous recombination) or to the replacement of a copy of the targeted gene by another copy sufficiently homologous to allow an homologous recombination event to occur (knock-in homologous recombination). In a specific embodiment, the DNA constructs described above may be used to introduce a CanIon genomic sequence or a CanIon cDNA sequence comprising at least one biallelic marker suitable for use in the present invention, preferably at least one biallelic marker selected from the group consisting of A1 to A17.

### Nuclear Antisense DNA Constructs

Other compositions containing a vector of the invention comprising an oligonucleotide fragment of the nucleic sequence SEQ ID No 4, preferably a fragment including the start codon of the CanIon gene, as an antisense tool that inhibits the expression of the corresponding CanIon gene. Preferred methods using antisense polynucleotide suitable for use in the present invention are the procedures described by Sczakiel et al. (1995) or those described in PCT Application No WO 95/24223.

Preferably, the antisense tools are chosen among the polynucleotides (15-200 bp long) that are complementary to the 5'end of the CanIon mRNA. In one embodiment, a combination of different antisense polynucleotides complementary to different parts of the desired targeted gene are used.

Preferred antisense polynucleotides suitable for use in to the present invention are complementary to a sequence of the mRNAs of CanIon that contains either the translation initiation codon ATG or a splicing site. Further preferred antisense polynucleotides according to the invention are complementary of the splicing site of the CanIon mRNA.

Preferably, the antisense polynucleotides suitable for use in the invention have a 3' polyadenylation signal that has been replaced with a self-cleaving ribozyme sequence, such that RNA polymerase II transcripts are produced without poly(A) at their 3' ends, these antisense polynucleotides being incapable of export from the nucleus, such as described by Liu et al. (1994). In a preferred embodiment, these CanIon antisense polynucleotides also comprise, within the ribozyme cassette, a histone stem-loop structure to stabilize cleaved transcripts against 3'-5' exonucleolytic degradation, such as the structure described by Eckner et al. (1991).

**Oligonucleotide Probes And Primers**

Polynucleotides derived from the CanIon gene are useful in order to detect the presence of at least a copy of a nucleotide sequence of SEQ ID Nos 1 to 4 and 6, or a fragment, complement, or variant thereof in a test sample.

Particularly preferred probes and primers suitable for use in the invention include isolated, purified, or recombinant polynucleotides comprising a contiguous span of at least 12, 15, 18, 20, 25, 30, 35, 40, 50, 60, 70, 80, 90, 100, 150, 200, 500, 1000, 2000, 5000, 10000 or 20000 nucleotides of SEQ ID Nos 1 to 3 or the complements thereof. Further preferred probes and primers suitable for use in the invention include isolated, purified, or recombinant polynucleotides, wherein said contiguous span comprises a biallelic marker selected from the group consisting of A1 to A17.

Suitable for use in the invention is a purified, isolated, or recombinant nucleic acid comprising the nucleotide sequence of SEQ ID No 4, complementary sequences thereto, as well as allelic variants, and fragments thereof. Moreover, preferred probes and primers suitable for use in the invention include purified, isolated, or recombinant CanIon cDNA consisting of, consisting essentially of, or comprising the sequence of SEQ ID No 4. Particularly preferred probes and primers of the invention include isolated, purified, or recombinant polynucleotides comprising a contiguous span of at least 12, 15, 18, 20, 25, 30, 35, 40, 50, 60, 70, 80, 90, 100, 150, 200, 500, 1000, 2000, 3000, 4000, 5000 or 6000 nucleotides of SEQ ID No 4 or the complements thereof. Further preferred probes and primers suitable for use in the invention include isolated, purified, or recombinant polynucleotides comprising a contiguous span of at least 12, 15, 18, 20, 25, 30, 35, 40, S0, 60, 70, 80, 90, 100, 150, 200, 500, 1000, 2000, 3000, 4000, 5000 or 6000 nucleotides of SEQ ID No 4 or the complements thereof, wherein said contiguous span comprises a biallelic marker selected from the group consisting of A12 and A16.

In further embodiments, probes and primers suitable for use in the invention include isolated, purified, or recombinant polynucleotides comprising a contiguous span of at least 12, 15, 18, 20, 25, 30, 35, 40, 50, 60, 70, 80, 90, 100, 150, 200, 300 or 400 nucleotides of SEQ ID No 6 or the complements thereof. In preferred embodiments, said contiguous span of SEQ ID No 6 comprises a biallelic marker A18.

Thus, the invention also relates to nucleic acid probes characterized in that they hybridize specifically, under the stringent hybridization conditions defined above, with a nucleic acid selected from the group consisting of the human CanIon nucleotide sequences of SEQ ID Nos 1 to 3, or a variant thereof or a sequence complementary thereto.

In one embodiment suitable for use in the invention are isolated, purified, and recombinant polynucleotides consisting of, or consisting essentially of a contiguous span of 8 to 50 nucleotides of any one of SEQ ID Nos 1 to 4 and 6, and the complement thereof, wherein said span includes a CanIon-related biallelic marker in said sequence; optionally, wherein said CanIon-related biallelic marker is selected from the group consisting of A1 to A18, and the complements thereof, or optionally the biallelic markers in linkage disequilibrium therewith. Optionally, wherein said contiguous span is 18 to 35 nucleotides in length and said biallelic marker is within 4 nucleotides of the center of said polynucleotide; optionally, wherein said polynucleotide consists of said contiguous span and said contiguous span is 25 nucleotides in length and said biallelic marker is at the center of said polynucleotide; optionally, wherein the 3' end of said contiguous span is present at the 3' end of said polynucleotide; and optionally, wherein the 3' end of said contiguous span is located at the 3' end of said polynucleotide and said biallelic marker is present at the 3' end of said polynucleotide. In a preferred embodiment, said probes comprises, consists of, or consists essentially of a sequence selected from the following sequences: P1 to P18 and the complementary sequences thereto.

In another embodiment suitable for use in the invention are isolated, purified and recombinant polynucleotides comprising, consisting of, or consisting essentially of a contiguous span of 8 to 50 nucleotides of SEQ ID Nos 1 to 4, or the complements thereof, wherein the 3' end of said contiguous span is located at the 3' end of said polynucleotide, and wherein the 3' end of said polynucleotide is located within 20 nucleotides upstream of a CanIon-related biallelic marker in said sequence; optionally, wherein said CanIon-related biallelic marker is selected from the group consisting of A 1 to A18, and the complements thereof, or optionally the biallelic markers in linkage disequilibrium therewith; optionally, wherein the 3' end of said polynucleotide is located 1 nucleotide upstream of said CanIon-related biallelic marker in said sequence; and optionally, wherein said polynucleotide consists essentially of a sequence selected from the following sequences: D1 to D18 and E1 to E18.

In a further embodiment, suitable for use in the invention are isolated, purified, or recombinant polynucleotides comprising, consisting of, or consisting essentially of a sequence selected from the following sequences: B1 to B17 and C1 to C17.

In an additional embodiment, suitable for use in the invention are polynucleotides for use in hybridization assays, sequencing assays, and enzyme-based mismatch detection assays for determining the identity of the nucleotide at a CanIon-related biallelic marker in SEQ ID Nos 1 to 4 and 6, or the complements thereof, as well as polynucleotides for use in amplifying segments of nucleotides comprising a CanIon-related biallelic marker in SEQ ID Nos 1 to 4 and 6, or the complements thereof; optionally, wherein said CanIon-related biallelic marker is selected from the group consisting of A1 to A18, and the complements thereof, or optionally the biallelic markers in linkage disequilibrium therewith.

The invention concerns the use of the polynucleotides suitable for use in the invention for determining the identity of the nucleotide at a CanIon-related biallelic marker, preferably in hybridization assay, sequencing assay, microsequencing assay, or an enzyme-based mismatch detection assay and in amplifying segments of nucleotides comprising a CanIon-related biallelic marker.

A probe or a primer suitable for use in the invention has between 8 and 1000 nucleotides in length, or is specified to be at least 12, 15, 18, 20, 25, 35, 40, 50, 60, 70, 80, 100, 250, 500 or 1000 nucleotides in length. More particularly, the length of these probes and primers can range from 8, 10, 15, 20, or 30 to 100 nucleotides, preferably from 10 to 50, more preferably from 15 to 30 nucleotides. Shorter probes and primers tend to lack specificity for a target nucleic acid sequence and generally require cooler temperatures to form sufficiently stable hybrid complexes with the template. Longer probes and primers are expensive to produce and can sometimes self-hybridize to form hairpin structures. The appropriate length for primers and probes under a particular set of assay conditions may be empirically determined by one of skill in the art. A preferred probe or primer consists of a nucleic acid comprising a polynucleotide selected from the group of the nucleotide sequences of P 1 to P18 and the complementary sequence thereto, B1 to B 17, C1 to C17, D1 to D18, E1 to E18, for which the respective locations in the sequence listing are provided in Tables 1, 2, and 3.

The formation of stable hybrids depends on the melting temperature (Tm) of the DNA. The Tm depends on the length of the primer or probe, the ionic strength of the solution and the G+C content. The higher the G+C content of the primer or probe, the higher is the melting temperature because G:C pairs are held by three H bonds whereas A:T pairs have only two. The GC content in the probes of the invention usually ranges between 10 and 75 %, preferably between 35 and 60 %, and more preferably between 40 and 55 %.

The primers and probes can be prepared by any suitable method, including, for example, cloning and restriction of appropriate sequences and direct chemical synthesis by a method such as the phosphodiester method of Narang et al. (1979), the phosphodiester method of Brown et al. (1979), the diethylphosphoramidite method of Beaucage et al. (1981) and the solid support method described in EP 0 707 592.

Detection probes are generally nucleic acid sequences or uncharged nucleic acid analogs such as, for example peptide nucleic acids which are disclosed in International Patent Application WO 92/20702, morpholino analogs which are described in U.S. Patents Numbered 5,185,444; 5,034,506 and 5,142,047. The probe may have to be rendered "non-extendable" in that additional dNTPs cannot be added to the probe. In and of themselves analogs usually are non-extendable and nucleic acid probes can be rendered non-extendable by modifying the 3' end of the probe such that the hydroxyl group is no longer capable of participating in elongation. For example, the 3' end of the probe can be functionalized with the capture or detection label fo thereby consume or otherwise block the hydroxyl group. Alternatively, the 3' hydroxyl group simply can be cleaved, replaced or modified, U.S. Patent Application Serial No. 07/049,061 filed April 19, 1993 describes modifications, which can be used to render a probe non-extendable.

Any of the polynucleotides suitable for use in the present invention can be labeled, if desired, by incorporating any label known in the art to be detectable by spectroscopic, photochemical, biochemical, immunochemical, or chemical means. For example, useful labels include radioactive substances (including, $^{32}P$, $^{35}S$, $^{3}H$, $^{125}I$), fluorescent dyes (including, 5-bromodesoxyuridin, fluorescein, acetylaminofluorene, digoxigenin) or biotin. Preferably, polynucleotides are labeled at their 3' and 5' ends. Examples of non-radioactive labeling of nucleic acid fragments are described in the French patent No. FR-7810975 or by Urdea et al. (1988) or Sanchez-Pescador et al. (1988). In addition, the probes according to the present invention may have structural characteristics such that they allow the signal amplification, such structural characteristics being, for example, branched DNA probes as those described by Urdea et al. (1991) or in the European patent No. EP 0 225 807 (Chiron).

A label can also be used to capture the primer, so as to facilitate the immobilization of either the primer or a primer extension product, such as amplified DNA, on a solid support. A capture label is attached to the primers or probes and can be a specific binding member which forms a binding pair with the solid's phase reagent's specific binding member (e.g. biotin and streptavidin). Therefore depending upon the type of label carried by a polynucleotide or a probe, it may be employed to capture or to detect the target DNA. Further, it will be understood that the polynucleotides, primers or probes provided herein, may, themselves, serve as the capture label. For example, in the case where a solid phase reagent's binding member is a nucleic acid sequence, it may be selected such that it binds a complementary portion of a primer or probe to thereby immobilize the primer or probe to the solid phase. In cases where a polynucleotide probe itself serves as the binding member, those skilled in the art will recognize that the probe will contain a sequence or "tail" that is not complementary to the target. In the case where a polynucleotide primer itself serves as the capture label, at least a portion of the primer will be free to hybridize with a nucleic acid on a solid phase. DNA Labeling techniques are well known to the skilled technician.

The probes suitable for use in the present invention are useful for a number of purposes. They can be notably used in Southern hybridization to genomic DNA. The probes can also be used to detect PCR amplification products. They may also be used to detect mismatches in the CanIon gene or mRNA using other techniques. They can also be used to detect expression of a CanIon gene, e.g. in a Northern blot.

Any of the polynucleotides, primers and probes suitable for use in the present invention can be conveniently immobilized on a solid support. Solid supports are known to those skilled in the art and include the walls of wells of a reaction tray, test tubes, polystyrene beads, magnetic beads, nitrocellulose strips, membranes, microparticles such as latex particles, sheep (or other animal) red blood cells, duracytes and others. The solid support is not critical and can be selected by one skilled in the art. Thus, latex particles, microparticles, magnetic or non-magnetic beads, membranes, plastic tubes, walls of microtiter wells, glass or silicon chips, sheep (or other suitable animal's) red blood cells and duracytes are all suitable examples. Suitable methods for immobilizing nucleic acids on solid phases include ionic, hydrophobic, covalent interactions and the like. A solid support, as used herein, refers to any material which is insoluble, or can be made insoluble by a subsequent reaction. The solid support can be chosen for its intrinsic ability to attract and immobilize the capture reagent Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon (attached to) the solid support and which has the ability to immobilize the capture reagent through a specific binding reaction. The receptor molecule enables the indirect binding of the capture reagent to a solid support material before the performance of the assay or during the performance of the assay. The solid phase thus can be a plastic, derivatized plastic, magnetic or non-magnetic metal, glass or silicon surface of a test tube, microtiter well, sheet, bead, microparticle, chip, sheep (or other suitable animal's) red blood cells, duracytes® and other configurations known to those of ordinary skill in the art. The polynucleotides of the invention can be attached to or immobilized on a solid support individually or in groups of at least 2, 5, 8, 10, 12, 15, 20, or 25 distinct polynucleotides of the invention to a single solid support. In addition, polynucleotides other than those suitable for use in the invention may be attached to the same solid support as one or more polynucleotides suitable for use in the invention.

Consequently, suitable for use in the invention is a method for detecting the presence of a nucleic acid comprising a nucleotide sequence selected from a group consisting of SEQ ID Nos 1 to 4 and 6, a fragment or a variant thereof and a complementary sequence thereto in a sample, said method comprising the following steps of:

a) bringing into contact a nucleic acid probe or a plurality of nucleic acid probes which can hybridize with a nucleotide sequence included in a nucleic acid selected form the group consisting of the nucleotide sequences of SEQ ID Nos 1 to 4 and 6, a fragment or a variant thereof and a complementary sequence thereto and the sample to be assayed; and
b) detecting the hybrid complex formed between the probe and a nucleic acid in the sample. Suitable for use in the invention is a kit for detecting the presence of a nucleic acid comprising a nucleotide sequence selected from a group consisting of SEQ ID Nos 1 to 4 and 6, a fragment or a variant thereof and a complementary sequence thereto in a sample, said kit comprising:

  a) a nucleic acid probe or a plurality of nucleic acid probes which can hybridize with a nucleotide sequence included in a nucleic acid selected form the group consisting of the nucleotide sequences of SEQ ID Nos 1 to 4 and 6, a fragment or a variant thereof and a complementary sequence thereto; and
  b) optionally, the reagents necessary for performing the hybridization reaction.

In a first preferred embodiment of this detection method and kit, said nucleic acid probe or the plurality of nucleic acid probes are labeled with a detectable molecule. In a second preferred embodiment of said method and kit, said nucleic acid probe or the plurality of nucleic acid probes has been immobilized on a substrate. In a third preferred embodiment, the nucleic acid probe or the plurality of nucleic acid probes comprise either a sequence which is selected from the group consisting of the nucleotide sequences of P1 to P18 and the complementary sequence thereto, B1 to B17, C1 to C17, D1 to D18, E1 to E18 or a biallelic marker selected from the group consisting of A1 to A18 and the complements thereto.

**Oligonucleotide Arrays**

A substrate comprising a plurality of oligonucleotide primers or probes of the invention may be used either for detecting or amplifying targeted sequences in the CanIon gene and may also be used for detecting mutations in the coding or in the non-coding sequences of the CanIon gene.

Any polynucleotide provided herein may be attached in overlapping areas or at random locations on the solid support. Alternatively the polynucleotides suitable for use in the invention may be attached in an ordered array wherein each polynucleotide is attached to a distinct region of the solid support which does not overlap with the attachment site of any other polynucleotide. Preferably, such an ordered array of polynucleotides is designed to be "addressable" where the distinct locations are recorded and can be accessed as part of an assay procedure. Addressable polynucleotide arrays typically comprise a plurality of different oligonucleotide probes that are coupled to a surface of a substrate in different known locations. The knowledge of the precise location of each polynucleotide makes these "addressable" arrays particularly useful in hybridization assays. Any addressable array technology known in the art can be employed with the polynucleotides of the invention. One particular embodiment of these polynucleotide arrays is known as the Genechips™, and has been generally described in US Patent 5,143,854; PCT publications WO 90/15070 and 92/10092. These arrays may generally be produced using mechanical synthesis methods or light directed synthesis methods which incorporate a combination of photolithographic methods and solid phase oligonucleotide synthesis (Fodor et al., 1991). The immobilization of arrays of oligonucleotides on solid supports has been rendered possible by the development of a technology generally identified as "Very Large Scale Immobilized Polymer Synthesis" (VISIPS™) in which, typically, probes are immobilized in a high density array on a solid surface of a chip. Examples of VLSIPS™ technologies are provided in US Patents 5,143,854; and 5,412,087 and in PCT Publications WO 90/15070, WO 92/10092 and WO 95/11995, which describe methods for forming oligonucleotide arrays through techniques such as light-directed synthesis techniques. In designing strategies aimed at providing arrays of nucleotides immobilized on solid supports, further presentation strategies were developed to order and display the oligonucleotide arrays on the chips in an attempt to maximize hybridization patterns and sequence information. Examples of such presentation strategies are disclosed in PCT Publications WO 94/12305, WO 94/11530, WO 97/29212 and WO 97/31256.

In another embodiment of the oligonucleotide arrays suitable for use in the invention, an oligonucleotide probe matrix may advantageously be used to detect mutations occurring in the CanIon gene and preferably in its regulatory region. For this particular purpose, probes are specifically designed to have a nucleotide sequence allowing their hybridization to the genes that carry known mutations (either by deletion, insertion or substitution of one or several nucleotides). By known mutations, it is meant, mutations on the CanIan gene that have been identified according, for example to the technique used by Huang et al. (1996) or Samson et al. (1996).

Another technique that is used to detect mutations in the CanIon gene is the use of a high-density DNA array. Each oligonucleotide probe constituting a unit element of the high density DNA array is designed to match a specific subsequence of the CanIon genomic DNA or cDNA. Thus, an array consisting of oligonucleotides complementary to subsequences of the target gene sequence is used to determine the identity of the target sequence with the wild gene sequence, measure its amount, and detect differences between the target sequence and the reference wild gene sequence of the CanIon gene. In one such design, termed 4L tiled array, is implemented a set of four probes (A, C, G, T), preferably 15-nucleotide oligomers. In each set of four probes, the perfect complement will hybridize more strongly than mismatched probes. Consequently, a nucleic acid target of length L is scanned for mutations with a tiled array containing 4L probes, the whole probe set containing all the possible mutations in the known wild reference sequence. The hybridization signals of the 15-mer probe set tiled array are perturbed by a single base change in the target sequence. As a consequence, there is a characteristic loss of signal or a "footprint" for the probes flanking a mutation position. This technique was described by Chee et al. (1996).

Consequently, suitable for use in the invention is an array of nucleic acid molecules comprising at least one polynucleotide described above as probes and primers. Preferably suitable for use in the invention is an array of nucleic acid comprising at least two polynucleotides described above as probes and primers.

Suitable for use in the invention is an array of nucleic acid sequences comprising either at least one of the sequences selected from the group consisting of P1 to P18, B 1 to B17, C1 to C17, D1 to D18, E1 to E18, the sequences complementary thereto, a fragment thereof of at least 8, 10, 12, 15, 18, 20, 25, 30, or 40 consecutive nucleotides thereof, and at least one sequence comprising a biallelic marker selected from the group consisting of A1 to A18 and the complements thereto.

Suitable for use in the invention is an array of nucleic acid sequences comprising either at least two of the sequences selected from the group consisting of P1 to P18, B1 to B17, C1 to C17, D1 to D18, E1 to E18, the sequences complementary thereto, a fragment thereof of at least 8 consecutive nucleotides thereof, and at least two sequences comprising a biallelic marker selected from the group consisting of A1 to A18 and the complements thereof.

## CanIon Proteins and Polypeptide Fragments:

The term "CanIon polypeptides" is used herein to embrace all of the proteins and polypeptides of the present invention. Also suitable for use in the invention are polypeptides encoded by the polynucleotides of the invention, as well as fusion polypeptides comprising such polypeptides. The invention embodies CanIon proteins from humans, including isolated or purified CanIon proteins consisting of, consisting essentially of, or comprising the sequence of SEQ ID No 5.

Suitable for use in the invention is the polypeptide encoded by a nucleotide sequence selected from the group consisting of SEQ ID No 1 to 4 and 6, a complementary sequence thereof or a fragment thereto.

Suitable for use in the invention are isolated, purified, and recombinant polypeptides comprising a contiguous span of at least 6 amino acids, preferably at least 8 to 10 amino acids, more preferably at least 12, 15, 20, 25, 30, 40, 50, 100, 150, 200, 300, 400, 500, 700,1000, 1200, 1400, 1600 or 1700 amino acids of SEQ ID No 5. In other preferred embodiments the contiguous stretch of amino acids comprises the site of a mutation or functional mutation, including a deletion, addition, swap or truncation of the amino acids in the CanIon protein sequence.

In preferred embodiments, suitable for use in the invention are isolated, purified, and recombinant polypeptides comprising a contiguous span of at least 6 amino acids, preferably at least 8 to 10 amino acids, more preferably at least 12, 15, 20, 25, 30, 40, 50, 100, 150, 200, 300, 400, 500, 700, 1000, 1200, 1400, 1600 or 1700 amino acids of SEQ ID No 5, wherein said contiguous span includes at least 1, 2, 3, 5 or 10 of the amino acid positions 277, 338, 574, 678, 680, 683, 691, 692, 695, 696, 697, 894, 1480, 1481, 1483, 1484, 1485, 1630, 1631, 1632, 1636, 1660, 1667, 1707, 1709 of SEQ ID No 5. Preferably, said contiguous span of SEQ ID No 5 comprises an Alanine residue at position 277; a Serine at position 338; a Valine at position 574; a Leucine at position 678; a Serine at position 680; a Threonine at position 683; a Histidine at position 691; a Serine at position 692; a Serine at position 695; an Alanine at position 696; an Isoleucine at position 697; an Isoleucine at position 894; a Lysine at position 1480; an Arginine at position 1481; a Glycine at position 1483; a Valine at position 1484; an Isoleucine at position 1485; an Asparagine at position 1630; a Serine at position 1631; a Methionine at position 1632; a Threonine at position 1636; an Alanine at position 1660; a Phenylalanine at position 1667; a Threonine at position 1707; and/or an Alanine at position 1709. Polynucleotides encoding any of these polypeptides are also provided.

Suitable for use in the invention are purified, isolated, or recombinant polypeptides comprising an amino acid sequence having at least 70, 75, 80, 85, 90, 95, 98 or 99% amino acid identity with the amino acid sequence of SEQ ID No 5 or a fragment thereof.

CanIon proteins are preferably isolated from human or mammalian tissue samples or expressed from human or mammalian genes. The CanIon polypeptides suitable for use in the invention can be made using routine expression methods known in the art. The polynucleotide encoding the desired polypeptide, is ligated into an expression vector suitable for any convenient host. Both eukaryotic and prokaryotic host systems is used in forming recombinant polypeptides, and a summary of some of the more common systems. The polypeptide is then isolated from lysed cells or from the culture medium and purified to the extent needed for its intended use. Purification is by any technique known in the art, for example, differential extraction, salt fractionation, chromatography, centrifugation, and the like. See, for example, Methods in Enzymology for a variety of methods for purifying proteins.

In addition, shorter protein fragments is produced by chemical synthesis. Alternatively the proteins of the invention is extracted from cells or tissues of humans or non-human animals. Methods for purifying proteins are known in the art, and include the use of detergents or chaotropic agents to disrupt particles followed by differential extraction and separation of the polypeptides by ion exchange chromatography, affinity chromatography, sedimentation according to density, and gel electrophoresis.

Any CanIon cDNA, including SEQ ID No 4, may be used to express CanIon proteins and polypeptides. The nucleic acid encoding the CanIon protein or polypeptide to be expressed is operably linked to a promoter in an expression vector using conventional cloning technology. The CanIon insert in the expression vector may comprise the full coding sequence for the CanIon protein or a portion thereof.

The expression vector is any of the mammalian, yeast, insect or bacterial expression systems known in the art. Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon context and codon pairing of the sequence is optimized for the particular expression organism in which the expression vector is introduced, as explained by Hatfield, et al., U.S. Patent No. 5,082,767.

In one embodiment, the entire coding sequence of the CanIon cDNA through the polyA signal of the cDNA are operably linked to a promoter in the expression vector. Alternatively, if the nucleic acid encoding a portion of the CanIon protein lacks a methionine to serve as the initiation site, an initiating methionine can be introduced next to the first codon of the nucleic acid using conventional techniques. Similarly, if the insert from the CanIon cDNA lacks a polyA signal, this sequence can be added to the construct by, for example, splicing out the PolyA signal from pSG5 (Stratagene) using BglI and SalI restriction endonuclease enzymes and incorporating it into the mammalian expression vector pXT1 (Stratagene). pXT1 contains the LTRs and a portion of the gag gene from Moloney Murine Leukemia Virus. The position of the LTRs in the construct allow efficient stable transfection. The vector includes the Herpes Simplex Thymidine Kinase promoter and the selectable neomycin gene. The nucleic acid encoding the CanIon protein or a portion thereof is obtained by PCR from a bacterial vector containing the CanIon cDNA of SEQ ID No 5 using oligonucleotide primers complementary to the CanIon cDNA or portion thereof and containing restriction endonuclease sequences for Pst I incorporated into the 5' primer and BglII at the 5' end of the corresponding cDNA 3' primer, taking care to ensure that the sequence encoding the CanIon protein or a portion thereof is positioned properly with respect to the polyA signal. The purified fragment obtained from the resulting PCR reaction is digested with PstI, blunt ended with an exonuclease, digested with Bgl II, purified and ligated to pXTl, now containing a poly A signal and digested with BglII.

The ligated product may be transfected into mouse NIH 3T3 cells using Lipofectin (Life Technologies, Inc., Grand Island, New York) under conditions outlined in the product specification. Positive transfectants are selected after growing the transfected cells in 600ug/ml G418 (Sigma, St. Louis, Missouri).

The above procedures may also be used to express a mutant CanIon protein responsible for a detectable phenotype or a portion thereof.

The expressed protein may be purified using conventional purification techniques such as ammonium sulfate precipitation or chromatographic separation based on size or charge. The protein encoded by the nucleic acid insert may also be purified using standard immunochromatography techniques. In such procedures, a solution containing the expressed CanIon protein or portion thereof, such as a cell extract, is applied to a column having antibodies against the CanIon protein or portion thereof is attached to the chromatography matrix. The expressed protein is allowed to bind the immunochromatography column. Thereafter, the column is washed to remove non-specifically bound proteins. The specifically bound expressed protein is then released from the column and recovered using standard techniques.

To confirm expression of the CanIon protein or a portion thereof, the proteins expressed from host cells containing an expression vector containing an insert encoding the CanIon protein or a portion thereof can be compared to the proteins expressed in host cells containing the expression vector without an insert. The presence of a band in samples from cells containing the expression vector with an insert which is absent in samples from cells containing the expression vector without an insert indicates that the CanIon protein or a portion thereof is being expressed. Generally, the band will have the mobility expected for the CanIon protein or portion thereof. However, the band may have a mobility different than that expected as a result of modifications such as glycosylation, ubiquitination, or enzymatic cleavage.

Antibodies capable of specifically recognizing the expressed CanIon protein or a portion thereof are described below.

If antibody production is not possible, the nucleic acids encoding the CanIon protein or a portion thereof is incorporated into expression vectors designed for use in purification schemes employing chimeric polypeptides. In such strategies the nucleic acid encoding the CanIon protein or a portion thereof is inserted in frame with the gene encoding the other half of the chimera. The other half of the chimera is β-globin or a nickel binding polypeptide encoding sequence. A chromatography matrix having antibody to β-globin or nickel attached thereto is then used to purify the chimeric protein. Protease cleavage sites is engineered between the β-globin gene or the nickel binding polypeptide and the CanIon protein or portion thereof Thus, the two polypeptides of the chimera is separated from one another by protease digestion.

One useful expression vector for generating β-globin chimeric proteins is pSG5 (Stratagene), which encodes rabbit β-globin. Intron II of the rabbit β-globin gene facilitates splicing of the expressed transcript, and the polyadenylation signal incorporated into the construct increases the level of expression. These techniques are well known to those skilled in the art of molecular biology. Standard methods are published in methods texts such as Davis et al. (1986) and many of the methods are available from Stratagene, Life Technologies, Inc., or Promega. Polypeptide may additionally be produced from the construct using in vitro translation systems such as the In vitro Express™ Translation Kit (Stratagene).

## Antibodies That Bind CanIon Polypeptides of the Invention

Any CanIon polypeptide or whole protein may be used to generate antibodies capable of specifically binding to an expressed CanIon protein or fragments thereof as described.

One antibody composition suitable for use in the invention is capable of specifically or selectively binding to the variant of the CanIon protein of SEQ ID No 5. For an antibody composition to specifically bind to a first variant of CanIon, it must demonstrate at least a 5%, 10%, 15%, 20%, 25%, 50%, or 100% greater binding affinity for a full length first variant of the CanIon protein than for a full length second variant of the CanIon protein in an ELISA, RIA, or other antibody-based binding assay. In a preferred embodiment an antibody composition is capable of specifically binding a human CanIon protein.

In a preferred embodiment, suitable for use in the invention are antibody compositions, either polyclonal or monoclonal, capable of selectively binding, or selectively bind to an epitope-containing a polypeptide comprising a contiguous span of at least 6 amino acids, preferably at least 8 to 10 amino acids, more preferably at least 12, 15, 20, 25, 30, 40, 50, 100, 200, 300, 400, 500, 700 or 1000 amino acids of SEQ ID No 5. In preferred embodiments, said contiguous span includes at least 1, 2, 3, 5 or 10 of the amino acid positions 277, 33 8, 574, 678, 680, 683, 691, 692, 695, 696, 697, 894, 1480, 1481, 1483, 1484,1485,1630,1631,1632,1636, 1660,1667,1707,1709 of SEQ ID No 5.

Any CanIon polypeptide or whole protein may be used to generate antibodies capable of specifically binding to an expressed CanIon protein or fragments thereof as described.

An epitope can comprise as few as 3 amino acids in a spatial conformation, which is unique to the epitope. Generally an epitope consists of at least 6 such amino acids, and more often at least 8-10 such amino acids. In preferred embodiment, antigenic epitopes comprise a number of amino acids that is any integer between 3 and 50. Fragments which function as epitopes may be produced by any conventional means. Epitopes can be determined by a Jameson-Wolf antigenic analysis, for example, performed using the computer program PROTEAN, using default parameters (Version 4.0 Windows, DNASTAR, Inc., 1228 South Park Street Madison, WI.

Suitable for use in the invention is a purified or isolated antibody capable of specifically binding to a mutated CanIon protein or to a fragment or variant thereof comprising an epitope of the mutated CanIan protein. In another preferred embodiment, the present invention concerns an antibody capable of binding to a polypeptide comprising at least 10 consecutive amino acids of a CanIon protein and including at least one of the amino acids which can be encoded by the trait causing mutations.

Non-human animals or mammals, whether wild-type or transgenic, which express a different species of CanIon than the one to which antibody binding is desired, and animals which do not express CanIon (i.e. a CanIon knock out animal as described herein) are particularly useful for preparing antibodies. CanIon knock out animals will recognize all or most of the exposed regions of a CanIon protein as foreign antigens, and therefore produce antibodies with a wider array of CanIon epitopes. Moreover, smaller polypeptides with only 10 to 30 amino acids may be useful in obtaining specific binding to any one of the CanIon proteins. In addition, the humoral immune system of animals which produce a species of CanIon that resembles the antigenic sequence will preferentially recognize the differences between the animal's native CanIon species and the antigen sequence, and produce antibodies to these unique sites in the antigen sequence. Such a technique will be particularly useful in obtaining antibodies that specifically bind to any one of the CanIon proteins.

Antibody preparations prepared according to either protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

The antibodies suitable for use in the invention may be labeled by any one of the radioactive, fluorescent or enzymatic labels known in the art.

Consequently, suitable for use in the invention is a method for detecting specifically the presence of a CanIon polypeptide according to the invention in a biological sample, said method comprising the following steps:

a) bringing into contact the biological sample with a polyclonal or monoclonal antibody that specifically binds a CanIon polypeptide comprising an amino acid sequence of SEQ ID No 5, or to a peptide fragment or variant thereof; and

b) detecting the antigen-antibody complex formed.

The invention also concerns a diagnostic kit for detecting *in vitro* the presence of an ion channel polypeptide in a biological sample, wherein said kit comprises:

a) a polyclonal or monoclonal antibody that specifically binds an ion channel polypeptide comprising an amino acid sequence of SEQ ID No 5, or to a peptide fragment or variant thereof, optionally labeled;

b) a reagent allowing the detection of the antigen-antibody complexes formed, said reagent carrying optionally a label, or being able to be recognized itself by a labeled reagent, more particularly in the case when the above-mentioned monoclonal or polyclonal antibody is not labeled by itself.

Suitable for use in the invention are antibodies and T-cell antigen receptors (TCR), which specifically bind the polypeptides, and more specifically, the epitopes of the polyepeptides of the present invention, including but not limited to IgG (including IgG1, IgG2, IgG3, and IgG4), IgA (including IgA1 and IgA2), IgD, IgE, or IgM, and IgY. In a preferred embodiment the antibodies are human antigen binding antibody fragments of the present invention include, but are not limited to, Fab, Fab' F(ab)2 and F(ab)2, Fd, single-chain Fvs (scFv), single-chain antibodies, disulfide-linked Fvs (sdFv) and fragments comprising either a $V_L$ or $V_H$ domain. The antibodies may be from any animal origin including birds and mammals. Preferably, the antibodies are human, murine, rabbit, goat, guinea pig, camel, horse, or chicken.

Antigen-binding antibody fragments, including single-chain antibodies, may comprise the variable region(s) alone or in combination with the entire or partial of the following: hinge region, CH1, CH2, and CH3 domains. Also included in the invention are any combinations of variable region(s) and hinge region, CH1, CH2, and CB3 domains. The present invention further includes the use of chimeric, humanized, and human monoclonal and polyclonal antibodies, which specifically bind the polypeptides of the present invention. Suitable for use in the invention are antibodies that are anti-idiotypic to the antibodies described herein.

The antibodies suitable for use in the present invention may be monospecific, bispecific, trispecific or have greater multispecificity. Multispecific antibodies may be specific for different epitopes of a polypeptide of the present invention or may be specific for both a polypeptide of the present invention as well as for heterologous compositions, such as a heterologous polypeptide or solid support material. See, e.g., WO 93/17715; WO 92/08802; WO 91/00360; WO 92/05793; Tutt, et al. (1991) J. Immunol. 147:60-69; US Patent Nos. 5,573,920, 4,474,893, 5,601,819, 4,714,681, 4,925,648; Kostelny, et al. (1992) J. Immunol. 148:1547-1553.

Antibodies suitable for use in the present invention may be described or specified in terms of the epitope(s) or epitope-bearing portion(s) of a polypeptide of the present invention, which are recognized or specifically bound by the antibody. In the case of proteins of the present invention secreted proteins, the antibodies may specifically bind a full-length protein encoded by a nucleic acid of the present invention, a mature protein (i.e., the protein generated by cleavage of the signal peptide) encoded by a nucleic acid of the present invention, a signal peptide encoded by a nucleic acid of the present invention, or any other polypeptide of the present invention. Therefore, the epitope(s) or epitope bearing polypeptide portion(s) may be specified as described herein, e.g., by N-terminal and C-terminal positions, by size in contiguous amino acid residues, or otherwise described herein (including the squence listing). Antibodies which specifically bind any epitope or polypeptide of the present invention may also be excluded as individual species. Therefore, the present invention includes antibodies that specifically bind specified polypeptides of the present invention, and allows for the exclusion of the same.

Antibodies suitable for use in the present invention may also be described or specified in terms of their cross-reactivity. Antibodies that do not specifically bind any other analog, ortholog, or homolog of the polypeptides of the present invention are included. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. Also suitable for use in the present invention are antibodies, which only bind polypeptides encoded by polynucleotides, which hybridize to a polynucleotide of the present invention under stringent hybridization conditions (as described herein). Antibodies suitable for use in the present invention may also be described or specified in terms of their binding affinity. Preferred binding affinities include those with a dissociation constant or Kd less than $5X10^{-6}M$, $10^{-6}M$, $5X10^{-7}M$, $10^{-7}M$, $5X10^{-8}M$, $10^{-8}M$, $5X10^{-9}M$, $10^{-9}M$, $5X10^{-10}M$, $10^{-10}M$, $5X10^{-11}M$, $10^{-11}M$, $5X10^{-12}M$, $10^{-12}M$, $5X10^{-13}M$, $10^{-13}M$, $5X10^{-14}M$, $10^{-14}M$, $5X10^{-15}M$, and $10^{-15}M$.

Antibodies suitable for use in the present invention have uses that include, but are not limited to, methods known in the art to purify, detect, and target the polypeptides of the present invention including both in vitro and in vivo diagnostic and therapeutic methods. For example, the antibodies have use in immunoassays for qualitatively and quantitatively measuring levels of the polypeptides of the present invention in biological samples. See, e.g., Harlow et al., ANTIBODIES: A LABORATORY MANUAL, (Cold Spring Harbor Laboratory Press, 2nd ed. 1988).

The antibodies suitable for use in the present invention may be used either alone or in combination with other compositions. The antibodies may further be recombinantly fused to a heterologous polypeptide at the N- or C-terminus or chemically conjugated (including covalent and non-covalent conjugations) to polypeptides or other compositions. For example, antibodies suitable for use in the present invention may be recombinantly fused or conjugated to molecules useful as labels in detection assays and effector molecules such as heterologous polypeptides, drugs, or toxins. See, e.g., WO 92/08495; WO 91/14438; WO 89/12624; US Patent 5,314,995; and EP 0 396 387.

The antibodies suitable for use in the present invention may be prepared by any suitable method known in the art. For example, a polypeptide of the present invention or an antigenic fragment thereof can be administered to an animal in order to induce the production of sera containing polyclonal antibodies. The term "monoclonal antibody" is not limited to antibodies produced through hybridoma technology. The term "antibody" refers to a polypeptide or group of polypeptides which are comprised of at least one binding domain, where a binding domain is formed from the folding of variable domains of an antibody molecule to form three-dimensional binding spaces with an internal surface shape and charge distribution complementary to the features of an antigenic determinant of an antigen., which allows an immunological reaction with the antigen. The term "monoclonal antibody" refers to an antibody that is derived from a single clone, including eukaryotic, prokaryotic, or phage clone, and not the method by which it is produced. Monoclonal antibodies can be prepared using a wide variety of techniques known in the art including the use of hybridoma, recombinant, and phage display technology.

Hybridoma techniques include those known in the art (see, e.g., Harlow et al. (1998); Hammerling, et al. (1981). Fab and F(ab')2 fragments may be produced, for example, from hybridoma-produced antibodies by proteolytic cleavage, using enzymes such as papain (to produce Fab fragments) or pepsin (to produce F(ab')2 fragments).

Alternatively, antibodies suitable for use in the present invention can be produced through the application of recombinant DNA technology or through synthetic chemistry using methods known in the art. For example, the antibodies suitable for use in the present invention can be prepared using various phage display methods known in the art. In phage display methods, functional antibody domains are displayed on the surface of a phage particle, which carries polynucleotide sequences encoding them. Phage with a desired binding property are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman, et al. (1995); Ames, et al. (1995); Kettleborough, et al. (1994); Persic, et al. (1997); Burton, et al. (1994); PCT/GB91/01134; WO 90/02809; WO 91/10737; WO 92/01047; WO 92/18619; WO 93/11236; WO 95/15982; WO 95/20401; and US Patent Nos. 5,698,426, 5,223,409, 5,403,484; 5,580,717, 5,427,908, 5,750,753, 5,821,047, 5,571,698, 5,427,908, 5,516,637, 5,780,225, 5,658,727 and 5,733,743.

As described in the above references, after phage selection, the antibody coding regions from the phage can be isolated and used to generate whole antibodies, including human antibodies, or any other desired antigen binding fragment, and expressed in any desired host including mammalian cells, insect cells, plant cells, yeast, and bacteria. For example, techniques to recombinantly produce Fab, Fab' F(ab)2 and F(ab')2 fragments can also be employed using methods known in the art such as those disclosed in WO 92/22324; Mullinax, et al. (1992); and Sawai, et al. (1995); and Better, et al. (1988).

Examples of techniques which can be used to produce single-chain Fvs and antibodies include those described in U.S. Patents 4,946,778 and 5,258,498; Huston et al. (1991); Shu, et al. (1993); and Skerra, et al. (1988). For some uses, including in vivo use of antibodies in humans and in vitro detection assays, it may be preferable to use chimeric, humanized, or human antibodies. Methods for producing chimeric antibodies are known in the art. See e.g., Morrison (1985); Oi et al., (1986); Gillies, S.D. et al. (1989); and US Patent 5,807,715. Antibodies can be humanized using a variety of techniques including CDR-grafting (EP 0 239 400; WO 91/09967; US Patent 5,530,101; and 5,585,089), veneering or resurfacing (EP 0 592 106; EP 0 519 596; Padlan E.A., (1991); Studnicka G.M. et al. (1994); Roguska M.A. et al. (1994), and chain shuffling (US Patent 5,565,332). Human antibodies can be made by a variety of methods known in the art including phage display methods described above. See also, US Patents 4,444,887, 4,716,111, 5,545,806, and 5,814,318; WO 98/46645; WO 98/50433; WO 98/24893; WO 96/34096; WO 96/33735; and WO 91/10741.

Also suitable for use in the present invention are antibodies recombinantly fused or chemically conjugated (including both covalently and non-covalendy conjugations) to a polypeptide of the present invention. The antibodies may be specific for antigens other than polypeptides of the present invention. For example, antibodies may be used to target the polypeptides of the present invention to particular cell types, either in vitro or in vivo, by fusing or conjugating the polypeptides of the present invention to antibodies specific for particular cell surface receptors. Antibodies fused or conjugated to the polypeptides of the present invention may also be used in in vitro immunoassays and purification methods using methods known in the art. See e.g., Harbor et al. supra and WO 93/21232; EP 0 439 095; Naramura, M. et al. (1994); US Patent 5,474,981; Gillies, S.O. et al. (1992); Fell, H.P. et al. (1991).

Also suitable for use in the invention are compositions comprising the polypeptides of the present invention fused or conjugated to antibody domains other than the variable regions. For example, the polypeptides suitable for use in the present invention may be fused or conjugated to an antibody Fc region, or portion thereof. The antibody portion fused to a polypeptide suitable for use in the present invention may comprise the hinge region, CH1 domain, CH2 domain, and CH3 domain or any combination of whole domains or portions thereof. The polypeptides suitable for use in the present invention may be fused or conjugated to the above antibody portions to increase the in vivo half-life of the polypeptides or for use in immunoassays using methods known in the art. The polypeptides may also be fused or conjugated to the above antibody portions to form multimers. For example, Fc portions fused to the polypeptides of the present invention can form dimers through disulfide bonding between the Fc portions. Higher multimeric forms can be made by fusing the polypeptides to portions of IgA and IgM. Methods for fusing or conjugating the polypeptides of the present invention to antibody portions are known in the art. See e.g., US Patents 5,336,603, 5,622,929, 5,359,046, 5,349,053, 5,447,851, 5,112,946; EP 0 307 434, EP 0 367 166; WO 96/04388, WO 91/06570; Ashkenazi, A. et al. (1991); Zheng, X.X. et al. (1995); and Vil, H. et al. (1992) antibodies that act as agonists or antagonists of the polypeptides described herein are suitable for use in the present invention.

For example, suitable for use in the invention are antibodies that disrupt the receptor/ligand interactions with the polypeptides described herein either partially or fully. Included are both receptor-specific antibodies and ligand-specific antibodies. Included are receptor-specific antibodies, which do not prevent ligand binding but prevent receptor activation. Receptor activation (i.e., signaling) may be determined by techniques described herein or otherwise known in the art. Also include are receptor-specific antibodies which both prevent ligand binding and receptor activation. Likewise, included are neutralizing antibodies that bind the ligand and prevent binding of the ligand to the receptor, as well as antibodies that bind the ligand, thereby preventing receptor activation, but do not prevent the ligand from binding the receptor. Further included are antibodies that activate the receptor. These antibodies may act as agonists for either all or less than all of the biological activities affected by ligand-mediated receptor activation. The antibodies may be specified as agonists or antagonists for biological activities comprising specific activities disclosed herein. The above antibody agonists can be made using methods known in the art. See e.g., WO 96/40281; US Patent 5,811,097; Deng, B. et al. (1998); Chen, Z. et al. (1998); Harrop, J.A. et al. (1998); Zhu, Z. et al. (1998); Yoon, D.Y. et al. (1998); Prat, M. et al. (1998); Pitard, V. et al. (1997); Liautard, J. et al. (1997); Carlson, N.G. et al. (1997); Taryman, R.E. et al. (1995); Muller, Y.A. et al. (1998); Bartunek, P. et al. (1996).

As discussed above, antibodies of the polypeptides suitable for use in the invention can, in turn, be utilized to generate anti-idiotypic antibodies that "mimic" polypeptides suitable for use in the invention using techniques well known to those skilled in the art. See, e.g. Greenspan and Bona, (1989); Nissinoff, (1991). For example, antibodies which bind to and competitively inhibit polypeptide multimerization or binding of a polypeptide of the invention to ligand can be used to generate anti-idiotypes that "mimic" the polypeptide multimerization or binding domain and, as a consequence, bind to and neutralize polypeptide or its ligand. Such neutralization anti-idiotypic antibodies can be used to bind a polypeptide of the invention or to bind its ligands/receptors, and therby block its biological activity.

**__CanIon -related Biallelic Markers__**

### Advantages Of The Biallelic Markers Of The Present Invention

The Canton-related biallelic markers suitable for use in the present invention offer a number of important advantages over other genetic markers such as RFLP (Restriction fragment length polymorphism) and VNTR (Variable Number of Tandem Repeats) markers.

The first generation of markers, were RFLPs, which are variations that modify the length of a restriction fragment. But methods used to identify and to type RFLPs are relatively wasteful of materials, effort, and time. The second generation of genetic markers were VNTRs, which can be categorized as either minisatellites or microsatellites. Minisatellites are tandemly repeated DNA sequences present in units of 5-50 repeats which are distributed along regions of the human chromosomes ranging from 0.1 to 20 kilobases in length. Since they present many possible alleles, their informative content is very high. Minisatellites are scored by performing Southern blots to identify the number of tandem repeats present in a nucleic acid sample from the individual being tested. However, there are only $10^4$ potential VNTRs that can be typed by Southern blotting. Moreover, both RFLP and VNTR markers are costly and time-consuming to develop and assay in large numbers.

Single nucleotide polymorphism or biallelic markers can be used in the same manner as RFLPs and VNTRs but offer several advantages. SNP are densely spaced in the human genome and represent the most frequent type of variation. An estimated number of more than $10^7$ sites are scattered along the $3x10^9$ base pairs of the human genome. Therefore, SNP occur at a greater frequency and with greater uniformity than RFLP or VNTR markers which means that there is a greater probability that such a marker will be found in close proximity to a genetic locus of interest. SNP are less variable than VNTR markers but are mutationally more stable.

Also, the different forms of a characterized single nucleotide polymorphism, such as the biallelic markers of the present invention, are often easier to distinguish and can therefore be typed easily on a routine basis. Biallelic markers have single nucleotide based alleles and they have only two common alleles, which allows highly parallel detection and automated scoring. The biallelic markers suitable for use in the present invention offer the possibility of rapid, high throughput genotyping of a large number of individuals.

Biallelic markers are densely spaced in the genome, sufficiently informative and can be assayed in large numbers. The combined effects of these advantages make biallelic markers extremely valuable in genetic studies. Biallelic markers can be used in linkage studies in families, in allele sharing methods, in linkage disequilibrium studies in populations, in association studies of case-control populations or of trait positive and trait negative populations. An important aspect of the present invention is that biallelic markers allow association studies to be performed to identify genes involved in complex traits. Association studies examine the frequency of marker alleles in unrelated case- and control-populations and are generally employed in the detection of polygenic or sporadic traits. Association studies may be conducted within the general population and are not limited to studies performed on related individuals in affected families (linkage studies). Biallelic markers in different genes can be screened in parallel for direct association with disease or response to a treatment. This multiple gene approach is a powerful tool for a variety of human genetic studies as it provides the necessary statistical power to examine the synergistic effect of multiple genetic factors on a particular phenotype, drug response, sporadic trait, or disease state with a complex genetic etiology.

### Candidate Gene Of The Present Invention

Different approaches can be employed to perform association studies: genome-wide association studies, candidate region association studies and candidate gene association studies. Genome-wide association studies rely on the screening of genetic markers evenly spaced and covering the entire genome. The candidate gene approach is based on the study of genetic markers specifically located in genes potentially involved in a biological pathway related to the trait of interest. In the present invention, CanIon is the candidate gene. The candidate gene analysis clearly provides a short-cut approach to the identification of genes and gene polymorphisms related to a particular trait when some information concerning the biology of the trait is available. However, it should be noted that all of the biallelic markers disclosed in the instant application can be employed as part of genome-wide association studies or as part of candidate region association studies and such uses are specifically contemplated in the present invention and claims.

### CanIon-Related Biallelic Markers And Polynucleotides Related Thereto

Suitable for use in the invention are CanIon-related biallelic markers. As used herein the term "CanIon-related biallelic marker" relates to a set of biallelic markers in linkage disequilibrium with the CanIon gene. The term CanIon-related biallelic marker includes the biallelic markers designated A1 to A17.

A portion of the biallelic markers suitable for use in the present invention are disclosed in Table 2. They are also described as a single base polymorphism in the features of in the related SEQ ID Nos 1 to 4 and 6. The pairs of primers allowing the amplification of a nucleic acid containing the polymorphic base of one CanIon biallelic marker are listed in Table I of Example 2.

17 CanIon-related biallelic markers, A1 to A17, are located in the genomic sequence of CanIon. Biallelic markers A12 and A16 are located in the exons of CanIon. Biallelic marker A18 is flanking the CanIon gene.

Suitable for use in the invention is a purified and/or isolated nucleotide sequence comprising a polymorphic base of a CanIon-related biallelic marker. In preferred embodiments, the biallelic marker is selected from the group consisting of A1 to A18, and the complements thereof. The sequence has between 8 and 1000 nucleotides in length, and preferably comprises at least 8, 10, 12, 15, 18, 20, 25, 35, 40, 50, 60, 70, 80, 100, 250, 500 or 1000 contiguous nucleotides of a nucleotide sequence selected from the group consisting of SEQ ID Nos 1 to 4 and 6 or a variant thereof or a complementary sequence thereto. These nucleotide sequences comprise the polymorphic base of either allele 1 or allele 2 of the considered biallelic marker. Optionally, said biallelic marker may be within 6, 5, 4, 3, 2, or I nucleotides of the center of said polynucleotide or at the center of said polynucleotide. Optionally, the 3' end of said contiguous span may be present at the 3' end of said polynucleotide. Optionally, biallelic marker may be present at the 3' end of said polynucleotide. Optionally, said polynucleotide may further comprise a label. Optionally, said polynucleotide can be attached to solid support In a further embodiment, the polynucleotides defined above can be used alone or in any combination.

Suitable for use in the invention is a purified and/or isolated nucleotide sequence comprising between 8 and 1000 contiguous nucleotides, and/or preferably at least 8, 10, 12, 15, 18, 20, 25, 35, 40, 50, 60, 70, 80, 100, 250, 500 or 1000 contiguous nucleotides of a nucleotide sequence selected from the group consisting of SEQ ID Nos 1 to 4 or a variant thereof or a complementary sequence thereto. Optionally, the 3' end of said polynucleotide may be located within or at least 2, 4, 6, 8, 10, 12, 15, 18, 20, 25, 50, 100, 250, 500, or 1000 nucleotides upstream of a GanIon-related biallelic marker in said sequence. Optionally, said CanIon-related biallelic marker is selected from the group consisting of A1 to A17; Optionally, the 3' end of said polynucleotide may be located within or at least 2, 4, 6, 8, 10, 12, 15, 18, 20, 25, 50, 100, 250, 500, or 1000 nucleotides upstream of a CanIon-related biallelic marker in said sequence. Optionally, the 3' end of said polynucleotide may be located 1 nucleotide upstream of a CanIon-related biallelic marker in said sequence. Optionally, said polynucleotide may further comprise a label. Optionally, said polynucleotide can be attached to solid support In a further embodiment, the polynucleotides defined above can be used alone or in any combination.

In a preferred embodiment, the sequences comprising a polymorphic base of one of the biallelic markers listed in Table 2 are selected from the group consisting of the nucleotide sequences that have a contiguous span of, that consist of, that are comprised in, or that comprises a polynucleotide selected from the group consisting of the nucleic acids of the sequences set forth as the amplicons listed in Table 1 or a variant thereof or a complementary sequence thereto.

Suitable for use in the invention is a nucleic acid encoding the CanIon protein, wherein said nucleic acid comprises a polymorphic base of a biallelic marker selected from the group consisting of A12 and A16 and the complements thereof.

Suitable for use in the invention are any polynucleotide for, or any polynucleotide for use in, determining the identity of one or more nucleotides at a CanIon-related biallelic marker. In addition, the polynucleotides suitable for use in the invention for use in determining the identity of one or more nucleotides at a CanIon-related biallelic marker encompass polynucleotides with any further limitation described in this disclosure, or those following, specified alone or in any combination. Optionally, said CanIon-related biallelic marker is selected from the group consisting of A1 to A18, and the complements thereof, or optionally the biallelic markers in linkage disequilibrium therewith; optionally, said CanIon-related biallelic marker is selected from the group consisting of A1 to A17, and the complements thereof, or optionally the biallelic markers in linkage disequilibrium therewith; optionally, said CanIon-related biallelic marker is selected from the group consisting of A12 and A16, and the complements thereof, or optionally the biallelic markers in linkage disequilibrium therewith; Optionally, said polynucleotide may comprise a sequence disclosed in the present specification; Optionally, said polynucleotide may consist of, or consist essentially of any polynucleotide described in the present specification; Optionally, said determining may be performed in a hybridization assay, sequencing assay, microsequencing assay, or an enzyme-based mismatch detection assay; Optionally, said polynucleotide may be attached to a solid support, array, or addressable array; Optionally, said polynucleotide may be labeled. A preferred polynucleotide may be used in a hybridization assay for determining the identity of the nucleotide at a CanIon-related biallelic marker. Another preferred polynucleotide may be used in a sequencing or microsequencing assay for determining the identity of the nucleotide at a CanIon-related biallelic marker. A third preferred polynucleotide may be used in an enzyme-based mismatch detection assay for determining the identity of the nucleotide at a CanIon-related biallelic marker. A fourth preferred polynucleotide may be used in amplifying a segment of polynucleotides comprising a CanIon-related biallelic marker. Optionally, any of the polynucleotides described above may be attached to a solid support, array, or addressable array; Optionally, said polynucleotide may be labeled.

Additionally, suitable for use in the invention is any polynucleotide for, or any polynucleotide for use in, amplifying a segment of nucleotides comprising a CanIon-related biallelic marker. In addition, the polynucleotides suitable for use in the invention for use in amplifying a segment of nucleotides comprising a CanIon-related biallelic marker encompass polynucleotides with any further limitation described in this disclosure, or those following, specified alone or in any combination: Optionally, said CanIon-related biallelic marker is selected from the group consisting of A1 to A18, and the complements thereof, or optionally the biallelic markers in linkage disequilibrium therewith; optionally, said CanIon-related biallelic marker is selected from the group consisting of A1 to A17, and the complements thereof, or optionally the biallelic markers in linkage disequilibrium therewith; optionally, said CanIon-related biallelic marker is selected from the group consisting of A12 and A16, and the complements thereof, or optionally the biallelic markers in linkage disequilibrium therewith; Optionally, said polynucleotide may comprise a sequence disclosed in the present specification; Optionally, said polynucleotide may consist of, or consist essentially of any polynucleotide described in the present specification; Optionally, said amplifying may be performed by PCR or LCR. Optionally, said polynucleotide may be attached to a solid support, array, or addressable array. Optionally, said polynucleotide may be labeled.

The primers for amplification or sequencing reaction of a polynucleotide comprising a biallelic marker suitable for use in the invention may be designed from the disclosed sequences for any method known in the art. A preferred set of primers are fashioned such that the 3' end of the contiguous span of identity with a sequence selected from the group consisting of SEQ ID Nos 1 to 4 and 6 or a sequence complementary thereto or a variant thereof is present at the 3' end of the primer. Such a configuration allows the 3' end of the primer to hybridize to a selected nucleic acid sequence and dramatically increases the efficiency of the primer for amplification or sequencing reactions. Allele specific primers may be designed such that a polymorphic base of a biallelic marker is at the 3' end of the contiguous span and the contiguous span is present at the 3' end of the primer. Such allele specific primers tend to selectively prime an amplification or sequencing reaction so long as they are used with a nucleic acid sample that contains one of the two alleles present at a biallelic marker. The 3' end of the primer suitable for use in the invention may be located within or at least 2, 4, 6, 8,10, 12,1 S,18, 20, 25, 50, 100, 250, 500, or 1000 nucleotides upstream of a CanIon-related biallelic marker in said sequence or at any other location which is appropriate for their intended use in sequencing, amplification or the location of novel sequences or markers. Thus, another set of preferred amplification primers comprise an isolated polynucleotide consisting essentially of a contiguous span of 8 to 50 nucleotides in a sequence selected from the group consisting of SEQ ID Nos 1 to 4 and 6 or a sequence complementary thereto or a variant thereof, wherein the 3' end of said contiguous span is located at the 3'end of said polynucleotide, and wherein the 3' end of said polynucleotide is located upstream of a CanIon-related biallelic marker in said sequence. Preferably, those amplification primers comprise a sequence selected from the group consisting of the sequences B1 to B17 and C1 to C17. Primers with their 3' ends located 1 nucleotide upstream of a biallelic marker of CanIon have a special utility as microsequencing assays. Preferred microsequencing primers are described in Table 4. Optionally, said CanIon-related biallelic marker is selected from the group consisting of A1 to A18, and the complements thereof, or optionally the biallelic markers in linkage disequilibrium therewith; optionally, said CanIon-related biallelic marker is selected from the group consisting of A1 to A17, and the complements thereof, or optionally the biallelic markers in linkage disequilibrium therewith; optionally, said CanIon-related biallelic marker is selected from the group consisting of A12 and A16, and the complements thereof, or optionally the biallelic markers in linkage disequilibrium therewith; Optionally, microsequencing primers are selected from the group consisting of the nucleotide sequences D1 to D18 and E1 to E18.

The probes suitable for use in the present invention may be designed from the disclosed sequences for any method known in the art, particularly methods which allow for testing if a marker disclosed herein is present. A preferred set of probes may be designed for use in the hybridization assays of the invention in any manner known in the art such that they selectively bind to one allele of a biallelic marker, but not the other under any particular set of assay conditions. Preferred hybridization probes comprise the polymorphic base of either allele 1 or allele 2 of the considered biallelic marker. Optionally, said biallelic marker may be within 6, 5, 4, 3, 2, or 1 nucleotide(s) of the center of the hybridization probe or at the center of said probe. In a preferred embodiment, the probes are selected from the group consisting of each of the sequences P1 to P18 and each of the complementary sequences thereto.

It should be noted that the polynucleotides suitable for use in the present invention are not limited to having the exact flanking sequences surrounding the polymorphic bases which are enumerated in Sequence Listing. Rather, it will be appreciated that the flanking sequences surrounding the biallelic markers may be lengthened or shortened to any extent compatible with their intended use and the present invention specifically contemplates such sequences. The flanking regions outside of the contiguous span need not be homologous to native flanking sequences which, actually occur in human subjects. The addition of any nucleotide sequence which is compatible with the nucleotides intended use is specifically contemplated.

Primers and probes may be labeled or immobilized on a solid support as described in "Oligonucleotide probes and primers".

The polynucleotides suitable for use in the invention which are attached to a solid support encompass polynucleotides with any further limitation described in this disclosure, or those following, specified alone or in any combination: Optionally, said polynucleotides may be specified as attached individually or in groups of at least 2, 5, 8, 10, 12, 15, 20, or 25 distinct polynucleotides of the invention to a single solid support. Optionally, polynucleotides other than those suitable for use in the invention may attached to the same solid support as polynucleotides of the invention. Optionally, when multiple polynucleotides are attached to a solid support they may be attached at random locations, or in an ordered array. Optionally, said ordered array may be addressable.

The present invention also encompasses diagnostic kits comprising one or more polynucleotides of the invention with a portion or all of the necessary reagents and instructions for genotyping a test subject by determining the identity of a nucleotide at a CanIon-related biallelic marker. The polynucleotides of a kit may optionally be attached to a solid support, or be part of an array or addressable array of polynucleotides. The kit may provide for the determination of the identity of the nucleotide at a marker position by any method known in the art including, but not limited to, a sequencing assay method, a microsequencing assay method, a hybridization assay method, or an enzyme-based mismatch detection assay method.

**<u>Methods For *De Novo* Identification Of Biallelic Markers</u>**

Any of a variety of methods can be used to screen a genomic fragment for single nucleotide polymorphisms such as differential hybridization with oligonucleotide probes, detection of changes in the mobility measured by gel electrophoresis or direct sequencing of the amplified nucleic acid. A preferred method for identifying biallelic markers involves comparative sequencing of genomic DNA fragments from an appropriate number of unrelated individuals.

In a first embodiment, DNA samples from unrelated individuals are pooled together, following which the genomic DNA of interest is amplified and sequenced. The nucleotide sequences thus obtained are then analyzed to identify significant polymorphisms. One of the major advantages of this method resides in the fact that the pooling of the DNA samples substantially reduces the number of DNA amplification reactions and sequencing reactions, which must be carried out. Moreover, this method is sufficiently sensitive so that a biallelic marker obtained thereby usually demonstrates a sufficient frequency of its less common allele to be useful in conducting association studies.

In a second embodiment, the DNA samples are not pooled and are therefore amplified and sequenced individually. This method is usually preferred when biallelic markers need to be identified in order to perform association studies within candidate genes. Preferably, highly relevant gene regions such as promoter regions or exon regions may be screened for biallelic markers. A biallelic marker obtained using this method may show a lower degree of informativeness for conducting association studies, e.g. if the frequency of its less frequent allele may be less than about 10%. Such a biallelic marker will, however, be sufficiently informative to conduct association studies and it will further be appreciated that including less informative biallelic markers in the genetic analysis studies of the present invention, may allow in some cases the direct identification of causal mutations, which may, depending on their penetrance, be rare mutations.

The following is a description of the various parameters of a preferred method used by the inventors for the identification of the biallelic markers suitable for use in the present invention.

**Genomic DNA Samples**

The genomic DNA samples from which the biallelic markets of the present invention are generated are preferably obtained from unrelated individuals corresponding to a heterogeneous population of known ethnic background. The number of individuals from whom DNA samples are obtained can vary substantially, preferably from about 10 to about 1000, preferably from about 50 to about 200 individuals. It is usually preferred to collect DNA samples from at least about 100 individuals in order to have sufficient polymorphic diversity in a given population to identify as many markers as possible and to generate statistically significant results.

As for the source of the genomic DNA to be subjected to analysis, any test sample can be foreseen without any particular limitation. These test samples include biological samples, which can be tested by the methods of the present invention described herein, and include human and animal body fluids such as whole blood, serum, plasma, cerebrospinal fluid, urine, lymph fluids, and various external secretions of the respiratory, intestinal and genitourinary tracts, tears, saliva, milk, white blood cells, myelomas and the like; biological fluids such as cell culture supernatants; fixed tissue specimens including tumor and non-tumor tissue and lymph node tissues; bone marrow aspirates and fixed cell specimens. The preferred source of genomic DNA used in the present invention is from peripheral venous blood of each donor. Techniques to prepare genomic DNA from biological samples are well known to the skilled technician. Details of a preferred embodiment are provided in Example 1. The person skilled in the art can choose to amplify pooled or unpooled DNA samples.

**DNA Amplification**

The identification of biallelic markers in a sample of genomic DNA may be facilitated through the use of DNA amplification methods. DNA samples can be pooled or unpooled for the amplification step. DNA amplification techniques are well known to those skilled in the art.

Amplification techniques that can be used in the context of the present invention include, but are not limited to, the ligase chain reaction (LCR) described in EP-A- 320 308, WO 9320227 and EP-A-439 182, the polymerase chain reaction (PCR, RT-PCR) and techniques such as the nucleic acid sequence based amplification (NASBA) described in Guatelli J.C., et al. (1990) and in Compton J. (1991), Q-beta amplification as described in European Patent Application No 4544610, strand displacement amplification as described in Walker et al. (1996) and EP A 684 315 and, target mediated amplification as described in PCT Publication WO 9322461.

LCR and Gap LCR are exponential amplification techniques, both depend on DNA ligase to join adjacent primers annealed to a DNA molecule. In Ligase Chain Reaction (LCR), probe pairs are used which include two primary (first and second) and two secondary (third and fourth) probes, all of which are employed in molar excess to target. The first probe hybridizes to a first segment of the target strand and the second probe hybridizes to a second segment of the target strand, the first and second segments being contiguous so that the primary probes abut one another in 5' phosphate-3'hydroxyl relationship, and so that a ligase can covalently fuse or ligate the two probes into a fused product. In addition, a third (secondary) probe can hybridize to a portion of the first probe and a fourth (secondary) probe can hybridize to a portion of the second probe in a similar abutting fashion. Of course, if the target is initially double stranded, the secondary probes also will hybridize to the target complement in the first instance. Once the ligated strand of primary probes is separated from the target strand, it will hybridize with the third and fourth probes, which can be ligated to form a complementary, secondary ligated product. It is important to realize that the ligated products are functionally equivalent to either the target or its complement. By repeated cycles of hybridization and ligation, amplification of the target sequence is achieved. A method for multiplex LCR has also been described (WO 9320227). Gap LCR (GLCR) is a version of LCR where the probes are not adjacent but are separated by 2 to 3 bases.

For amplification of mRNAs, it is within the scope of the present invention to reverse transcribe mRNA into cDNA followed by polymerase chain reaction (RT-PCR); or, to use a single enzyme for both steps as described in U.S. Patent No. 5,322,770 or, to use Asymmetric Gap LCR (RT-AGLCR) as described by Marshall et al.(1994). AGLCR is a modification of GLCR that allows the amplification of RNA.

The PCR technology is the preferred amplification technique used in the present invention. A variety of PCR techniques are familiar to those skilled in the art. For a review of PCR technology, see White (1997) and the publication entitled "PCR Methods and Applications" (1991, Cold Spring Harbor Laboratory Press). In each of these PCR procedures, PCR primers on either side of the nucleic acid sequences to be amplified are added to a suitably prepared nucleic acid sample along with dNTPs and a thermostable polymerase such as Taq polymerase, Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites. PCR has further been described in several patents including US Patents 4,683,195; 4,683,202; and 4,965,188.

PCR technology is the preferred amplification technique used to identify new biallelic markers. A typical example of a PCR reaction suitable for the purposes of the present invention is provided in Example 2.

Suitable for use in the present invention is a method for the amplification of the human CanIon gene, particularly of a fragment of the genomic sequence of SEQ ID No 1 to 3 or of the cDNA sequence of SEQ ID No 4, or a fragment or a variant thereof in a test sample, preferably using PCR. This method comprises the steps of:

a) contacting a test sample with amplification reaction reagents comprising a pair of amplification primers as described above and located on either side of the polynucleotide region to be amplified, and
b) optionally, detecting the amplification products.

The invention also concerns a kit for the amplification of a CanIon gene sequence, particularly of a portion of the genomic sequence of SEQ ID No 1 to 3 or of the cDNA sequence of SEQ ID No 4, or a variant thereof in a test sample, wherein said kit comprises:

a) a pair of oligonucleotide primers located on either side of the CanIon region to be amplified;
b) optionally, the reagents necessary for performing the amplification reaction.

In one embodiment of the above amplification method and kit, the amplification product is detected by hybridization with a labeled probe having a sequence which is complementary to the amplified region. In another embodiment of the above amplification method and kit, primers comprise a sequence which is selected from the group consisting of the nucleotide sequences of B1 to B17, C1 to C17, D1 to D18, and E1 to E18.

In a first embodiment suitable for use in the present invention, biallelic markers are identified using genomic sequence information generated by the inventors. Sequenced genomic DNA fragments arc used to design primers for the amplification of 500 bp fragments. These 500 bp fragments are amplified from genomic DNA and are scanned for biallelic markers. Primers may be designed using the OSP software (Hillier L. and Green P., 1991). All primers may contain, upstream of the specific target bases, a common oligonucleotide tail that serves as a sequencing primer. Those skilled in the art are familiar with primer extensions, which can be used for these purposes.

Preferred primers, useful for the amplification of genomic sequences encoding the CanIon gene, focus on promoters, exons and splice sites of the genes. A biallelic marker presents a higher probability to be an eventual causal mutation if it is located in these functional regions of the gene. Preferred amplification primers of the invention include the nucleotide sequences B1 to B17 and C1 to C17, detailed further in Example 2, Table 1.

**Sequencing Of Amplified Genomic DNA And Identification Of Single Nucleotide Polymorphisms**

The amplification products generated as described above, are then sequenced using any method known and available to the skilled technician. Methods for sequencing DNA using either the dideoxy-mediated method (Sanger method) or the Maxam-Gilbert method are widely known to those of ordinary skill in the art. Such methods are for example disclosed in Sambrook et al. (1989). Alternative approaches include hybridization to high-density DNA probe arrays as described in Chee et al. (1996).

Preferably, the amplified DNA is subjected to automated dideoxy terminator sequencing reactions using a dye-primer cycle sequencing protocol. The products of the sequencing reactions are run on sequencing gels and the sequences are determined using gel image analysis. The polymorphism search is based on the presence of superimposed peaks in the electrophoresis pattern resulting from different bases occurring at the same position. Because each dideoxy terminator is labeled with a different fluorescent molecule, the two peaks corresponding to a biallelic site present distinct colors corresponding to two different nucleotides at the same position on the sequence. However, the presence of two peaks can be an artifact due to background noise. To exclude such an artifact, the two DNA strands are sequenced and a comparison between the peaks is carried out. In order to be registered as a polymorphic sequence, the polymorphism has to be detected on both strands.

The above procedure permits those amplification products, which contain biallelic markers to be identified. The detection limit for the frequency of biallelic polymorphisms detected by sequencing pools of 100 individuals is approximately 0.1 for the minor allele, as verified by sequencing pools of known allelic frequencies. However, more than 90% of the biallelic polymorphisms detected by the pooling method have a frequency for the minor allele higher than 0.25. Therefore, the biallelic markers selected by this method have a frequency of at least 0.1 for the minor allele and less than 0.9 for the major allele. Preferably at least 0.2 for the minor allele and less than 0.8 for the major allele, more preferably at least 0.3 for the minor allele and less than 0.7 for the major allele, thus a heterozygosity rate higher than 0.18, preferably higher than 0.32, more preferably higher than 0.42.

In another embodiment, biallelic markers are detected by sequencing individual DNA samples, the frequency of the minor allele of such a biallelic marker may be less than 0.1.

**Validation Of The Biallelic Markers Of The Present Invention**

The polymorphisms are evaluated for their usefulness as genetic markers by validating that both alleles are present in a population. Validation of the biallelic markers is accomplished by genotyping a group of individuals by a method of the invention and demonstrating that both alleles are present. Microsequencing is a preferred method of genotyping alleles. The validation by genotyping step may be performed on individual samples derived from each individual in the group or by genotyping a pooled sample derived from more than one individual. The group can be as small as one individual if that individual is heterozygous for the allele in question. Preferably the group contains at least three individuals, more preferably the group contains five or six individuals, so that a single validation test will be more likely to result in the validation of more of the biallelic markers that are being tested. It should be noted, however, that when the validation test is performed on a small group it may result in a false negative result if as a result of sampling error none of the individuals tested carries one of the two alleles. Thus, the validation process is less useful in demonstrating that a particular initial result is an artifact, than it is at demonstrating that there is a *bona fide* biallelic marker at a particular position in a sequence. All of the genotyping, haplotyping, association, and interaction study methods of the invention may optionally be performed solely with validated biallelic markers.

**Evaluation Of The Frequency Of The Biallelic Markers Of The Present Invention**

The validated biallelic markers are further evaluated for their usefulness as genetic markers by determining the frequency of the least common allele at the biallelic marker site. The higher the frequency of the less common allele the greater the usefulness of the biallelic marker is association and interaction studies. The determination of the least common allele is accomplished by genotyping a group of individuals by a method of the invention and demonstrating that both alleles are present. This determination of frequency by genotyping step may be performed on individual samples derived from each individual in the group or by genotyping a pooled sample derived from more than one individual. The group must be large enough to be representative of the population as a whole. Preferably the group contains at least 20 individuals, more preferably the group contains at least 50 individuals, most preferably the group contains at least 100 individuals. Of course the larger the group the greater the accuracy of the frequency determination because of reduced sampling error. A biallelic marker wherein the frequency of the less common allele is 30% or more is termed a "high quality biallelic marker." All of the genotyping, haplotyping, association, and interaction study methods of the invention may optionally be performed solely with high quality biallelic markers.

## Methods For Genotyping An Individual For Biallelic Markers

Methods are provided to genotype a biological sample for one or more biallelic markers of the present invention, all of which may be performed *in vitro.* Such methods of genotyping comprise determining the identity of a nucleotide at a CanIon biallelic marker site by any method known in the art. These methods find use in genotyping case-control populations in association studies as well as individuals in the context of detection of alleles ofbiallelic markers which are known to be associated with a given trait, in which case both copies of the biallelic marker present in individual's genome are determined so that an individual may be classified as homozygous or heterozygous for a particular allele.

These genotyping methods can be performed on nucleic acid samples derived from a single individual or pooled DNA samples.

Genotyping can be performed using similar methods as those described above for the identification of the biallelic markers, or using other genotyping methods such as those further described below. In preferred embodiments, the comparison of sequences of amplified genomic fragments from different individuals is used to identify new biallelic markers whereas microsequencing is used for genotyping known biallelic markers in diagnostic and association study applications.

Suitable for use in the invention are methods of genotyping comprising determining the identity of a nucleotide at a CanIon-related biallelic marker or the complement thereof in a biological sample; optionally, wherein said CanIon-related biallelic marker is selected from the group consisting of A1 to A18, and the complements thereof, or optionally the biallelic markers in linkage disequilibrium therewith; optionally, wherein said CanIon-related biallelic marker is selected from the group consisting of A1 to A17, and the complements thereof, or optionally the biallelic markers in linkage disequilibrium therewith; optionally, wherein said CanIon-related biallelic marker is selected from the group consisting of A12 and A16, and the complements thereof, or optionally the biallelic markers in linkage disequilibrium therewith; optionally, wherein said biological sample is derived from a single subject; optionally, wherein the identity of the nucleotides at said biallelic marker is determined for both copies of said biallelic marker present in said individual's genome; optionally, wherein said biological sample is derived from multiple subjects; Optionally, the genotyping methods suitable for use in the invention encompass methods with any further limitation described in this disclosure, or those following, specified alone or in any combination; Optionally, said method is performed *in vitro;* optionally, further comprising amplifying a portion of said sequence comprising the biallelic marker prior to said determining step; Optionally, wherein said amplifying is performed by PCR, LCR, or replication of a recombinant vector comprising an origin of replication and said fragment in a host cell; optionally, wherein said determining is performed by a hybridization assay, a sequencing assay, a microsequencing assay, or an enzyme-based mismatch detection assay.

### Source of Nucleic Acids for genotyping

Any source of nucleic acids, in purified or non-purified form, can be utilized as the starting nucleic acid, provided it contains or is suspected of containing the specific nucleic acid sequence desired. DNA or RNA may be extracted from cells, tissues, body fluids and the like as described above. While nucleic acids for use in the genotyping methods suitable for use in the invention can be derived from any mammalian source, the test subjects and individuals from which nucleic acid samples are taken are generally understood to be human.

### Amplification Of DNA Fragments Comprising Biallelic Markers

Methods and polynucleotides are provided to amplify a segment of nucleotides comprising one or more biallelic marker suitable for use in the present invention. It will be appreciated that amplification of DNA fragments comprising biallelic markers may be used in various methods and for various purposes and is not restricted to genotyping. Nevertheless, many genotyping methods, although not all, require the previous amplification of the DNA region carrying the biallelic marker of interest. Such methods specifically increase the concentration or total number of sequences that span the biallelic marker or include that site and sequences located either distal or proximal to it. Diagnostic assays may also rely on amplification of DNA segments carrying a biallelic marker of the present invention. Amplification of DNA may be achieved by any method known in the art. Amplification techniques are described above in the section entitled, "DNA amplification."

Some of these amplification methods are particularly suited for the detection of single nucleotide polymorphisms and allow the simultaneous amplification of a target sequence and the identification of the polymorphic nucleotide as it is further described below.

The identification of biallelic markers as described above allows the design of appropriate oligonucleotides, which can be used as primers to amplify DNA fragments comprising the biallelic markers of the present invention. Amplification can be performed using the primers initially used to discover new biallelic markers which are described herein or any set of primers allowing the amplification of a DNA fragment comprising a biallelic marker of the present invention.

Suitable for use in the present invention are primers for amplifying a DNA fragment containing one or more biallelic markers of the present invention. Preferred amplification primers are listed in Example 2. It will be appreciated that the primers listed are merely exemplary and that any other set of primers which produce amplification products containing one or more biallelic markers of the present invention are also of use.

The spacing of the primers determines the length of the segment to be amplified. In the context of the present invention, amplified segments carrying biallelic markers can range in size from at least about 25 bp to 35 kbp. Amplification fragments from 25-3000 bp are typical, fragments from 50-1000 bp are preferred and fragments from 100-600 bp are highly preferred. It will be appreciated that amplification primers for the biallelic markers may be any sequence which allow the specific amplification of any DNA fragment carrying the markers. Amplification primers may be labeled or immobilized on a solid support as described in "Oligonucleotide probes and primers".

### Methods of Genotyping DNA samples for Biallelic Markers

Any method known in the art can be used to identify the nucleotide present at a biallelic marker site. Since the biallelic marker allele to be detected has been identified and specified in the present invention, detection will prove simple for one of ordinary skill in the art by employing any of a number of techniques. Many genotyping methods require the previous amplification of the DNA region carrying the biallelic marker of interest. While the amplification of target or signal is often preferred at present, ultrasensitive detection methods which do not require amplification are also encompassed by the present genotyping methods. Methods well-known to those skilled in the art that can be used to detect biallelic polymorphisms include methods such as, conventional dot blot analyzes, single strand conformational polymorphism analysis (SSCP) described by Orita et al. (1989), denaturing gradient gel electrophoresis (DGGE), heteroduplex analysis, mismatch cleavage detection, and other conventional techniques as described in Sheffield et al. (1991), White et al. (1992), Grompe et al. (1989 and 1993). Another method for determining the identity of the nucleotide present at a particular polymorphic site employs a specialized exonuclease-resistant nucleotide derivative as described in US patent 4,656,127.

Preferred methods involve directly determining the identity of the nucleotide present at a biallelic marker site by sequencing assay, enzyme-based mismatch detection assay, or hybridization assay. The following is a description of some preferred methods. A highly preferred method is the microsequencing technique. The term "sequencing" is generally used herein to refer to polymerase extension of duplex primer/template complexes and includes both traditional sequencing and microsequencing.

### 1) Sequencing Assays

The nucleotide present at a polymorphic site can be determined by sequencing methods. In a preferred embodiment, DNA samples are subjected to PCR amplification before sequencing as described above. DNA sequencing methods are described in "Sequencing Of Amplified Genomic DNA And Identification Of Single Nucleotide Polymorphisms".

Preferably, the amplified DNA is subjected to automated dideoxy terminator sequencing reactions using a dye-primer cycle sequencing protocol. Sequence analysis allows the identification of the base present at the biallelic marker site.

### 2) Microsequencing Assays

In microsequencing methods, the nucleotide at a polymorphic site in a target DNA is detected by a single nucleotide primer extension reaction. This method involves appropriate microsequencing primers which, hybridize just upstream of the polymorphic base of interest in the target nucleic acid. A polymerase is used to specifically extend the 3' end of the primer with one single ddNTP (chain terminator) complementary to the nucleotide at the polymorphic site.

Next the identity of the incorporated nucleotide is determined in any suitable way.

Typically, microsequencing reactions are carried out using fluorescent ddNTPs and the extended microsequencing primers are analyzed by electrophoresis on ABI 377 sequencing machines to determine the identity of the incorporated nucleotide as described in EP 412 883, the disclosure of which is incorporated herein by reference in its entirety. Alternatively capillary electrophoresis can be used in order to process a higher number of assays simultaneously. An example of a typical microsequencing procedure that are suitable for use in the present invention is provided in Example 4.

Different approaches can be used for the labeling and detection of ddNTPs. A homogeneous phase detection method based on fluorescence resonance energy transfer has been described by Chen and Kwok (1997) and Chen et al. (1997). In this method, amplified genomic DNA fragments containing polymorphic sites are incubated with a 5'-fluorescein-labeled primer in the presence of allelic dye-labeled dideoxyribonucleoside triphosphates and a modified Taq polymerase. The dye-labeled primer is extended one base by the dye-terminator specific for the allele present on the template. At the end of the genotyping reaction, the fluorescence intensities of the two dyes in the reaction mixture are analyzed directly without separation or purification. All these steps can be performed in the same tube and the fluorescence changes can be monitored in real time. Alternatively, the extended primer may be analyzed by MALDI-TOF Mass Spectrometry. The base at the polymorphic site is identified by the mass added onto the microsequencing primer (see Haff and Smirnov, 1997).

Microsequencing may be achieved by the established microsequencing method or by developments or derivatives thereof. Alternative methods include several solid-phase microsequencing techniques. The basic microsequencing protocol is the same as described previously, except that the method is conducted as a heterogeneous phase assay, in which the primer or the target molecule is immobilized or captured onto a solid support. To simplify the primer separation and the terminal nucleotide addition analysis, oligonucleotides are attached to solid supports or are modified in such ways that permit affinity separation as well as polymerase extension. The 5' ends and internal nucleotides of synthetic oligonucleotides can be modified in a number of different ways to permit different affinity separation approaches, e.g., biotinylation. If a single affinity group is used on the oligonucleotides, the oligonucleotides can be separated from the incorporated terminator regent. This eliminates the need of physical or size separation. More than one oligonucleotide can be separated from the terminator reagent and analyzed simultaneously if more than one affinity group is used. This permits the analysis of several nucleic acid species or more nucleic acid sequence information per extension reaction. The affinity group need not be on the priming oligonucleotide but could alternatively be present on the template. For example, immobilization can be carried out via an interaction between biotinylated DNA and streptavidin-coated microtitration wells or avidin-coated polystyrene particles. In the same manner, oligonucleotides or templates may be attached to a solid support in a high-density format In such solid phase microsequencing reactions, incorporated ddNTPs can be radiolabeled (Syvänen, 1994) or linked to fluorescein (Livak and Hainer, 1994). The detection of radiolabeled ddNTPs can be achieved through scintillation-based techniques. The detection of fluorescein-linked ddNTPs can be based on the binding of antifluorescein antibody conjugated with alkaline phosphatase, followed by incubation with a chromogenic substrate (such as *p*-nitrophenyl phosphate). Other possible reporter-detection pairs include: ddNTP linked to dinitrophenyl (DNP) and anti-DNP alkaline phosphatase conjugate (Harju et al., 1993) or biotinylated ddNTP and horseradish peroxidase-conjugated streptavidin with *o*-phenylenediamine as a substrate (WO 92/15712). As yet another alternative solid-phase microsequencing procedure, Nyren et al. (1993) described a method relying on the detection of DNA polymerase activity by an enzymatic luminometric inorganic pyrophosphate detection assay (ELIDA).

Pastinen et al. (1997) describe a method for multiplex detection of single nucleotide polymorphism in which the solid phase minisequencing principle is applied to an oligonucleotide array format. High-density arrays of DNA probes attached to a solid support (DNA chips) are further described below.

Suitable for use in the present invention are poynucleotides and methods to genotype one or more biallelic markers of the present invention by performing a microsequencing assay. Preferred microsequencing primers include the nucleotide sequences D1 to D18 and E1 to E18. It will be appreciated that the microsequencing primers listed in Example 4 are merely exemplary and that, any primer having a 3' end inunediately adjacent to the polymorphic nucleotide may be used. Similarly, it will be appreciated that microsequeacing analysis may be performed for any biallelic marker or any combination of biallelic markers of the present invention. Suitable for use in the present invention is a solid support which includes one or more microsequencing primers listed in Example 4 or fragments comprising at least 8, 12, 15, 20, 25, 30, 40, or 50 consecutive nucleotides thereof, to the extent that such lengths are consistent with the primer described, and having a 3' terminus immediately upstream of the corresponding biallelic marker, for determining the identity of a nucleotide at a biallelic marker site.

### 3) Mismatch detection assays based on polymerases and ligases

Suitable for use in the present invention are polynucleotides and methods to determine the allele of one or more biallelic markers of the present invention in a biological sample, by mismatch detection assays based on polymerases and/or ligases. These assays are based on the specificity of polymerases and ligases. Polymerization reactions places particularly stringent requirements on correct base pairing of the 3' end of the amplification primer and the joining of two oligonucleotides hybridized to a target DNA sequence is quite sensitive to mismatches close to the ligation site, especially at the 3' end. Methods, primers and various parameters to amplify DNA fragments comprising biallelic markers suitable for use in the present invention are further described above in "Amplification Of DNA Fragments Comprising Biallelic Markers".

**Allele Specific Amplification Primers**

Discrimination between the two alleles of a biallelic marker can also be achieved by allele specific amplification, a selective strategy, whereby one of the alleles is amplified without amplification of the other allele. For allele specific amplification, at least one member of the pair of primers is sufficiently complementary with a region of a CanIon gene comprising the polymorphic base of a biallelic marker of the present invention to hybridize therewith and to initiate the amplification. Such primers are able to discriminate between the two alleles of a biallelic marker.

This is accomplished by placing the polymorphic base at the 3' end of one of the amplification primers. Because the extension forms from the 3'end of the primer, a mismatch at or near this position has an inhibitory effect on amplification. Therefore, under appropriate amplification conditions, these primers only direct amplification on their complementary allele. Determining the precise location of the mismatch and the corresponding assay conditions are well within the ordinary skill in the art.

**Ligation/Amplification Based Methods**

The "Oligonucleotide Ligation Assay" (OLA) uses two oligonucleotides which are designed to be capable of hybridizing to abutting sequences of a single strand of a target molecules. One of the oligonucleotides is biotinylated, and the other is detectably labeled. If the precise complementary sequence is found in a target molecule, the oligonucleotides will hybridize such that their termini abut, and create a ligation substrate that can be captured and detected. OLA is capable of detecting single nucleotide polymorphisms and may be advantageously combined with PCR as described by Nickerson et al. (1990). In this method, PCR is used to achieve the exponential amplification of target DNA, which is then detected using OLA.

Other amplification methods which are particularly suited for the detection of single nucleotide polymorphism include LCR (ligase chain reaction), Gap LCR (GLCR) which are described above in "DNA Amplification". LCR uses two pairs of probes to exponentially amplify a specific target. The sequences of each pair of oligonucleotides, is selected to permit the pair to hybridize to abutting sequences of the same strand of the target. Such hybridization forms a substrate for a template-dependant ligase. LCR can be performed with oligonucleotides having the proximal and distal sequences of the same strand of a biallelic marker site. In one embodiment, either oligonucleotide will be designed to include the biallelic marker site. In such an embodiment, the reaction conditions are selected such that the oligonucleotides can be ligated together only if the target molecule either contains or lacks the specific nucleotide that is complementary to the biallelic marker on the oligonucleotide. In an alternative embodiment, the oligonucleotides will not include the biallelic marker, such that when they hybridize to the target molecule, a "gap" is created as described in WO 90/01069. This gap is then "filled" with complementary dNTPs (as mediated by DNA polymerase), or by an additional pair of oligonucleotides. Thus at the end of each cycle, each single strand has a complement capable of serving as a target during the next cycle and exponential allele-specific amplification of the desired sequence is obtained.

Ligase/Polymerase-mediated Genetic Bit Analysis™ is another method for determining the identity of a nucleotide at a preselected site in a nucleic acid molecule (WO 95/21271). This method involves the incorporation of a nucleoside triphosphate that is complementary to the nucleotide present at the preselected site onto the terminus of a primer molecule, and their subsequent ligation to a second oligonucleotide. The reaction is monitored by detecting a specific label attached to the reaction's solid phase or by detection in solution.

**4) Hybridization Assay Methods**

A preferred method of determining the identity of the nucleotide present at a biallelic marker site involves nucleic acid hybridization. The hybridization probes, which can be conveniently used in such reactions, preferably include the probes defined herein. Any hybridization assay may be used including Southern hybridization, Northern hybridization, dot blot hybridization and solid-phase hybridization (see Sambrook et al., 1989).

Hybridization refers to the formation of a duplex structure by two single stranded nucleic acids due to complementary base pairing. Hybridization can occur between exactly complementary nucleic acid strands or between nucleic acid strands that contain minor regions of mismatch. Specific probes can be designed that hybridize to one form of a biallelic marker and not to the other and therefore are able to discriminate between different allelic forms. Allele-specific probes are often used in pairs, one member of a pair showing perfect match to a target sequence containing the original allele and the other showing a perfect match to the target sequence containing the alternative allele. Hybridization conditions should be sufficiently stringent that there is a significant difference in hybridization intensity between alleles, and preferably an essentially binary response, whereby a probe hybridizes to only one of the alleles. Stringent, sequence specific hybridization conditions, under which a probe will hybridize only to the exactly complementary target sequence are well known in the art (Sambrook et al., 1989). Stringent conditions are sequence dependent and will be different in different circumstances. Generally, stringent conditions are selected to be about 5°C lower than the thermal melting point (Tm) for the specific sequence at a defined ionic strength and pH. Although such hybridization can be performed in solution, it is preferred to employ a solid-phase hybridization assay. The target DNA comprising a biallelic marker suitable for use in the present invention may be amplified prior to the hybridization reaction. The presence of a specific allele in the sample is determined by detecting the presence or the absence of stable hybrid duplexes formed between the probe and the target DNA. The detection of hybrid duplexes can be carried out by a number of methods. Various detection assay formats are well known which utilize detectable labels bound to either the target or the probe to enable detection of the hybrid duplexes. Typically, hybridization duplexes are separated from unhybridized nucleic acids and the labels bound to the duplexes are then detected. Those skilled in the art will recognize that wash steps may be employed to wash away excess target DNA or probe as well as unbound conjugate. Further, standard heterogeneous assay formats are suitable for detecting the hybrids using the labels present on the primers and probes.

Two recently developed assays allow hybridization-based allele discrimination with no need for separations or washes (see Landegren U. et al., 1998). The TaqMan assay takes advantage of the 5' nuclease activity of Taq DNA polymerase to digest a DNA probe annealed specifically to the accumulating amplification product. TaqMan probes are labeled with a donor-acceptor dye pair that interacts via fluorescence energy transfer. Cleavage of the TaqMan probe by the advancing polymerase during amplification dissociates the donor dye from the quenching acceptor dye, greatly increasing the donor fluorescence. All reagents necessary to detect two allelic variants can be assembled at the beginning of the reaction and the results are monitored in real time (see Livak et al., 1995). In an alternative homogeneous hybridization based procedure, molecular beacons are used for allele discriminations. Molecular beacons are hairpin-shaped oligonucleotide probes that report the presence of specific nucleic acids in homogeneous solutions. When they bind to their targets they undergo a conformational reorganization that restores the fluorescence of an internally quenched fluorophore (Tyagi et al., 1998).

The polynucleotides provided herein can be used to produce probes which can be used in hybridization assays for the detection of biallelic marker alleles in biological samples. These probes are characterized in that they preferably comprise between 8 and 50 nucleotides, and in that they are sufficiently complementary to a sequence comprising a biallelic marker suitable for use in the present invention to hybridize thereto and preferably sufficiently specific to be able to discriminate the targeted sequence for only one nucleotide variation. A particularly preferred probe is 25 nucleotides in length. Preferably the biallelic marker is within 4 nucleotides of the center of the polynucleotide probe. In particularly preferred probes, the biallelic marker is at the center of said polynucleotide. Preferred probes comprise a nucleotide sequence selected from the group consisting of amplicons listed in Table 1 and the sequences complementary thereto, or a fragment thereof, said fragment comprising at least about 8 consecutive nucleotides, preferably 10, 15, 20, more preferably 25, 30, 40, 47, or 50 consecutive nucleotides and containing a polymorphic base. Preferred probes comprise a nucleotide sequence selected from the group consisting of P1 to P18 and the sequences complementary thereto. In preferred embodiments the polymorphic base(s) are within 5, 4, 3, 2, 1, nucleotides of the center of the said polynucleotide, more preferably at the center of said polynucleotide.

Preferably the probes suitable for use in the present invention are labeled or immobilized on a solid support. Labels and solid supports are further described in "Oligonucleotide Probes and Primers". The probes can be non-extendable as described in "Oligonucleotide Probes and Primers".

By assaying the hybridization to an allele specific probe, one can detect the presence or absence of a biallelic marker allele in a given sample. High-Throughput parallel hybridization in array format is specifically encompassed within "hybridization assays" and are described below.

### 5) Hybridization To Addressable Arrays Of Oligonucleotides

Hybridization assays based on oligonucleotide arrays rely on the differences in hybridization stability of short oligonucleotides to perfectly matched and mismatched target sequence variants. Efficient access to polymorphism information is obtained through a basic structure comprising high-density arrays of oligonucleotide probes attached to a solid support (e.g., the chip) at selected positions. Each DNA chip can contain thousands to millions of individual synthetic DNA probes arranged in a grid-like pattern and miniaturized to the size of a dime.

The chip technology has already been applied with success in numerous cases. For example, the screening of mutations has been undertaken in the BRCA1 gene, in *S. cerevisiae* mutant strains, and in the protease gene of HIV-1 virus (Hacia et al., 1996; Shoemaker et al., 1996; Kozal et al., 1996). Chips of various formats for use in detecting biallelic polymorphisms can be produced on a customized basis by Affymetrix (GeneChip™), Hyseq (HyChip and HyGnostics), and Protogene Laboratories.

In general, these methods employ arrays of oligonucleotide probes that are complementary to target nucleic acid sequence segments from an individual which, target sequences include a polymorphic marker. EP 785280 describes a tiling strategy for the detection of single nucleotide polymorphisms. Briefly, arrays may generally be "tiled" for a large number of specific polymorphisms. By "tiling" is generally meant the synthesis of a defined set of oligonucleotide probes which is made up of a sequence complementary to the target sequence of interest, as well as preselected variations of that sequence, e.g., substitution of one or more given positions with one or more members of the basis set of nucleotides. Tiling strategies are further described in PCT application No. WO 95/11995. In a particular aspect, arrays are tiled for a number of specific, identified biallelic marker sequences. In particular, the array is tiled to include a number of detection blocks, each detection block being specific for a specific biallelic marker or a set of biallelic markers. For example, a detection block may be tiled to include a number of probes, which span the sequence segment that includes a specific polymorphism. To ensure probes that are complementary to each allele, the probes are synthesized in pairs differing at the biallelic marker. In addition to the probes differing at the polymorphic base, monosubstituted probes are also generally tiled within the detection block These monosubstituted probes have bases at and up to a certain number of bases in either direction from the polymorphism, substituted with the remaining nucleotides (selected from A, T, G, C and U). Typically the probes in a tiled detection block will include substitutions of the sequence positions up to and including those that are 5 bases away from the biallelic marker. The monosubstituted probes provide internal controls for the tiled array, to distinguish actual hybridization from artefactual cross-hybridization. Upon completion of hybridization with the target sequence and washing of the array, the array is scanned to determine the position on the array to which the target sequence hybridizes. The hybridization data from the scanned array is then analyzed to identify which allele or alleles of the biallelic marker are present in the sample. Hybridization and scanning may be carried out as described in PCT application No. WO 92/10092 and WO 95/11995 and US patent No. 5,424,186.

Thus, in some embodiments, the chips may comprise an array of nucleic acid sequences of fragments of about 15 nucleotides in length. In further embodiments, the chip may comprise an array including at least one of the sequences selected from the group consisting of amplicons listed in table 1 and the sequences complementary thereto, or a fragment thereof, said fragment comprising at least about 8 consecutive nucleotides, preferably 10, 15, 20, more preferably 25, 30, 40, 47, or 50 consecutive nucleotides and containing a polymorphic base. In preferred embodiments the polymorphic base is within 5, 4, 3, 2, 1, nucleotides of the center of the said polynucleotide, more preferably at the center of said polynucleotide. In some embodiments, the chip may comprise an array of at least 2, 3, 4, 5, 6, 7, 8 or more of these polynucleotides of the invention. Solid supports and polynucleotides suitable for use in the present invention attached to solid supports are further described in "Oligonucleotide Probes And Primers".

**6) Integrated Systems**

Another technique, which may be used to analyze polymorphisms, includes multicomponent integrated systems, which miniaturize and compartmentalize processes such as PCR and capillary electrophoresis reactions in a single functional device. An example of such technique is disclosed in US patent 5,589,136, which describes the integration of PCR amplification and capillary electrophoresis in chips.

Integrated systems can be envisaged mainly when microfluidic systems are used. These systems comprise a pattern of microchannels designed onto a glass, silicon, quartz, or plastic wafer included on a microchip. The movements of the samples are controlled by electric, electroosmotic or hydrostatic forces applied across different areas of the microchip to create functional microscopic valves and pumps with no moving parts.

For genotyping biallelic markers, the microfluidic system may integrate nucleic acid amplification, microsequencing, capillary electrophoresis and a detection method such as laser-induced fluorescence detection.

## Methods Of Genetic Analysis Using The Biallelic Markers Of The Present Invention

Different methods are available for the genetic analysis of complex traits (see Lander and Schork, 1994). The search for disease-susceptibility genes is conducted using two main methods: the linkage approach in which evidence is sought for cosegregation between a locus and a putative trait locus using family studies, and the association approach in which evidence is sought for a statistically significant association between an allele and a trait or a trait causing allele (Khoury et al., 1993). In general, the biallelic markers of the present invention find use in any method known in the art to demonstrate a statistically significant correlation between a genotype and a phenotype. The biallelic markers may be used in parametric and non-parametric linkage analysis methods. Preferably, the biallelic markers of the present invention are used to identify genes associated with detectable traits using association studies, an approach which does not require the use of affected families and which permits the identification of genes associated with complex and sporadic traits.

The genetic analysis using the biallelic markers suitable for use in the present invention may be conducted on any scale. The whole set of biallelic markers suitable for use in the present invention or any subset of biallelic markers suitable for use in the present invention corresponding to the candidate gene may be used Further, any set of genetic markers including a biallelic marker suitable for use in the present invention may be used. A set of biallelic polymorphisms that could be used as genetic markers in combination with the biallelic markers of the present invention has been described in WO 98/20165. As mentioned above, it should be noted that the biallelic markers suitable for use in the present invention may be included in any complete or partial genetic map of the human genome. These different uses are specifically contemplated in the present invention and claims.

## Linkage Analysis

Linkage analysis is based upon establishing a correlation between the transmission of genetic markers and that of a specific trait throughout generations within a family. Thus, the aim of linkage analysis is to detect marker loci that show cosegregation with a trait of interest in pedigrees.

### Parametric Methods

When data are available from successive generations there is the opportunity to study the degree of linkage between pairs of loci. Estimates of the recombination fraction enable loci to be ordered and placed onto a genetic map. With loci that are genetic markers, a genetic map can be established, and then the strength of linkage between markers and traits can be calculated and used to indicate the relative positions of markers and genes affecting those traits (Weir, 1996). The classical method for linkage analysis is the logarithm of odds (lod) score method (see Morton, 1955; Ott, 1991). Calculation of lod scores requires specification of the mode of inheritance for the disease (parametric method). Generally, the length of the candidate region identified using linkage analysis is between 2 and 20Mb. Once a candidate region is identified as described above, analysis of recombinant individuals using additional markers allows further delineation of the candidate region. Linkage analysis studies have generally relied on the use of a maximum of 5,000 microsatellite markers, thus limiting the maximum theoretical attainable resolution of linkage analysis to about 600 kb on average.

Linkage analysis has been successfully applied to map simple genetic traits that show clear Mendelian inheritance patterns and which have a high penetrance (i.e., the ratio between the number of trait positive carriers of allele *a* and the total number of *a* carriers in the population). However, parametric linkage analysis suffers from a variety of drawbacks. First, it is limited by its reliance on the choice of a genetic model suitable for each studied trait Furthermore, as already mentioned, the resolution attainable using linkage analysis is limited, and complementary studies are required to refine the analysis of the typical 2Mb to 20Mb regions initially identified through linkage analysis. In addition, parametric linkage analysis approaches have proven difficult when applied to complex genetic traits, such as those due to the combined action of multiple genes and/or environmental factors. It is very difficult to model these factors adequately in a lod score analysis. In such cases, too large an effort and cost are needed to recruit the adequate number of affected families required for applying linkage analysis to these situations, as recently discussed by Risch and Merikangas (1996).

### Non-Parametric Methods

The advantage of the so-called non-parametric methods for linkage analysis is that they do not require specification of the mode of inheritance for the disease, they tend to be more useful for the analysis of complex traits. In non-parametric methods, one tries to prove that the inheritance pattern of a chromosomal region is not consistent with random Mendelian segregation by showing that affected relatives inherit identical copies of the region more often than expected by chance. Affected relatives should show excess "allele sharing" even in the presence of incomplete penetrance and polygenic inheritance. In non-parametric linkage analysis the degree of agreement at a marker locus in two individuals can be measured either by the number of alleles identical by state (IBS) or by the number of alleles identical by descent (IBD). Affected sib pair analysis is a well-known special case and is the simplest form of these methods.

The biallelic markers suitable for use in the present invention may be used in both parametric and non-parametric linkage analysis. Preferably biallelic markers may be used in non-parametric methods which allow the mapping of genes involved in complex traits. The biallelic markers suitable for use in the present invention may be used in both IBD- and IBS- methods to map genes affecting a complex trait. In such studies, taking advantage of the high density of biallelic markers, several adjacent biallelic marker loci may be pooled to achieve the efficiency attained by multi-allelic markers (Zhao et al., 1998).

**Population Association Studies**

The present invention comprises methods for identifying if the CanIon gene is associated with a detectable trait using the biallelic markers of the present invention. In one embodiment the present invention comprises methods to detect an association between a biallelic marker allele or a biallelic marker haplotype and a trait. Further, the invention comprises methods to identify a trait causing allele in linkage disequilibrium with any biallelic marker allele of the present invention.

As described above, alternative approaches can be employed to perform association studies: genome-wide association studies, candidate region association studies and candidate gene association studies. In a preferred embodiment, the biallelic markers suitable for use in the present invention are used to perform candidate gene association studies. The candidate gene analysis clearly provides a short-cut approach to the identification of genes and gene polymorphisms related to a particular trait when some information concerning the biology of the trait is available. Further, the biallelic markers suitable for use in the present invention may be incorporated in any map of genetic markers of the human genome in order to perform genome-wide association studies. Methods to generate a high-density map of biallelic markers has been described in US Provisional Patent application serial number 60/082,614. The biallelic markers suitable for use in the present invention may further be incorporated in any map of a specific candidate region of the genome (a specific chromosome or a specific chromosomal segment for example).

As mentioned above, association studies may be conducted within the general population and are not limited to studies performed on related individuals in affected families. Association studies are extremely valuable as they permit the analysis of sporadic or multifactor traits. Moreover, association studies represent a powerful method for fine-scale mapping enabling much finer mapping of trait causing alleles than linkage studies. Studies based on pedigrees often only narrow the location of the trait causing allele. Association studies using the biallelic markers of the present invention can therefore be used to refine the location of a trait causing allele in a candidate region identified by Linkage Analysis methods. Moreover, once a chromosome segment of interest has been identified, the presence of a candidate gene such as a candidate gene of the present invention, in the region of interest can provide a shortcut to the identification of the trait causing allele. Biallelic markers suitable for use in the present invention can be used to demonstrate that a candidate gene is associated with a trait. Such uses are specifically contemplated in the present invention.

**Determining The Frequency Of A Biallelic Marker Allele Or Of A Biallelic Marker Haplotype In A Population**

Association studies explore the relationships among frequencies for sets of alleles between loci.

**Determining The Frequency Of An Allele In A Population**

Allelic frequencies of the biallelic markers in a populations can be determined using one of the methods described above under the heading "Methods for genotyping an individual for biallelic markers", or any genotyping procedure suitable for this intended purpose. Genotyping pooled samples or individual samples can determine the frequency of a biallelic marker allele in a population. One way to reduce the number of genotypings required is to use pooled samples. A major obstacle in using pooled samples is in terms of accuracy and reproducibility for determining accurate DNA concentrations in setting up the pools. Genotyping individual samples provides higher sensitivity, reproducibility and accuracy and; is the preferred method used in the present invention. Preferably, each individual is genotyped separately and simple gene counting is applied to determine the frequency of an allele of a biallelic marker or of a genotype in a given population.

Suitable for use in the invention are methods of estimating the frequency of an allele in a population comprising: a) genotyping individuals from said population for said biallelic marker according to the method of the present invention; b) determining the proportional representation of said biallelic marker in said population. In addition, the methods of estimating the frequency of an allele in a population of the invention encompass methods with any further limitation described in this disclosure, or those following, specified alone or in any combination; optionally, wherein said CanIon-related biallelic marker is selected from the group consisting of A1 to A18, and the complements thereof, or optionally the biallelic markers in linkage disequilibrium therewith; optionally, wherein said CanIon-related biallelic marker is selected from the group consisting of A1 to A17, and the complements thereof, or optionally the biallelic markers in linkage disequilibrium therewith; optionally, wherein said CanIon-related biallelic marker is selected from the group consisting of A12 and A16, and the complements thereof, or optionally the biallelic markers in linkage disequilibrium therewith; Optionally, determining the frequency of a biallelic marker allele in a population may be accomplished by determining the identity of the nucleotides for both copies of said biallelic marker present in the genome of each individual in said population and calculating the proportional representation of said nucleotide at said CanIon-related biallelic marker for the population; Optionally, determining the proportional representation may be accomplished by performing a genotyping method of the invention on a pooled biological sample derived from a representative number of individuals, or each individual, in said population, and calculating the proportional amount of said nucleotide compared with the total.

Determining The Frequency Of A Haplotype In A Population

The gametic phase of haplotypes is unknown when diploid individuals are heterozygous at more than one locus. Using genealogical information in families gametic phase can sometimes be inferred (Perlin et al., 1994). When no genealogical information is available different strategies may be used. One possibility is that the multiple-site heterozygous diploids can be eliminated from the analysis, keeping only the homozygotes and the single-site heterozygote individuals, but this approach might lead to a possible bias in the sample composition and the underestimation of low-frequency haplotypes. Another possibility is that single chromosomes can be studied independently, for example, by asymmetric PCR amplification (see Newton et al, 1989; Wu et al., 1989) or by isolation of single chromosome by limit dilution followed by PCR amplification (see Ruano et al., 1990). Further, a sample may be haplotyped for sufficiently close biallelic markers by double PCR amplification of specific alleles (Sarkar, G. and Sommer S. S., 1991). These approaches are not entirely satisfying either because of their technical complexity, the additional cost they entail, their lack of generalization at a large scale, or the possible biases they introduce. To overcome these difficulties, an algorithm to infer the phase of PCR-amplified DNA genotypes introduced by Clark, A.G. (1990) may be used. Briefly, the principle is to start filling a preliminary list of haplotypes present in the sample by examining unambiguous individuals, that is, the complete homozygotes and the single-site heterozygotes. Then other individuals in the same sample are screened for the possible occurrence of previously recognized haplotypes. For each positive identification, the complementary haplotype is added to the list of recognized haplotypes, until the phase information for all individuals is either resolved or identified as unresolved. This method assigns a single haplotype to each multiheterozygous individual, whereas several haplotypes are possible when there are more than one heterozygous site. Alternatively, one can use methods estimating haplotype frequencies in a population without assigning haplotypes to each individual. Preferably, a method based on an expectation-maximization (EM) algorithm (Dempster et al., 1977) leading to maximum-likelihood estimates of haplotype frequencies under the assumption of Hardy-Weinberg proportions (random mating) is used (see Excoffier L. and Slatkin M., 1995). The EM algorithm is a generalized iterative maximum-likelihood approach to estimation that is useful when data are ambiguous and/or incomplete. The EM algorithm is used to resolve heterozygotes into haplotypes. Haplotype estimations are further described below under the heading "Statistical Methods." Any other method known in the art to determine or to estimate the frequency of a haplotype in a population may be used.

Estimating the frequency of a haplotype for a set of biallelic markers in a population, comprises the steps of: a) genotyping at least one CanIon-related biallelic marker according to a method of the invention for each individual in said population; b) genotyping a second biallelic marker by determining the identity of the nucleotides at said second biallelic marker for both copies of said second biallelic marker present in the genome of each individual in said population; and c) applying a haplotype determination method to the identities of the nucleotides determined in steps a) and b) to obtain an estimate of said frequency. In addition, the methods of estimating the frequency of a haplotype of the invention encompass methods with any further limitation described in this disclosure, or those following, specified alone or in any combination: optionally, wherein said CanIon-related biallelic marker is selected from the group consisting of A1 to A18, and the complements thereof, or optionally the biallelic markers in linkage disequilibrium therewith; optionally, wherein said CanIon-related biallelic marker is selected from the group consisting of A1 to A17, and the complements thereof, or optionally the biallelic markers in linkage disequilibrium therewith; optionally, wherein said CanIon-related biallelic marker is selected from the group consisting of A12 and A16, and the complements thereof, or optionally the biallelic markers in linkage disequilibrium therewith; Optionally, said haplotype determination method is performed by asymmetric PCR amplification, double PCR amplification of specific alleles, the Clark algorithm, or an expectation-maatimization algorithm.

**Linkage Disequilibrium Analysis**

Linkage disequilibrium is the non-random association of alleles at two or more loci and represents a powerful tool for mapping genes involved in disease traits (see Ajioka R.S. et al., 1997). Biallelic markers, because they are densely spaced in the human genome and can be genotyped in greater numbers than other types of genetic markers (such as RFLP or VNTR markers), are particularly useful in genetic analysis based on linkage disequilibrium.

When a disease mutation is first introduced into a population (by a new mutation or the immigration of a mutation carrier), it necessarily resides on a single chromosome and thus on a single "background" or "ancestral" haplotype of linked markers. Consequently, mere is complete disequilibrium between these markers and the disease mutation: one finds the disease mutation only in the presence of a specific set of marker alleles. Through subsequent generations recombination events occur between the disease mutation and these marker polymorphisms, and the disequilibrium gradually dissipates. The pace of this dissipation is a function of the recombination frequency, so the markers closest to the disease gene will manifest higher levels of disequilibrium than those that are further away. When not broken up by recombination, "ancestral" haplotypes and linkage disequilibrium between marker alleles at different loci can be tracked not only through pedigrees but also through populations. Linkage disequilibrium is usually seen as an association between one specific allele at one locus and another specific allele at a second locus.

The pattern or curve of disequilibrium between disease and marker loci is expected to exhibit a maximum that occurs at the disease locus. Consequently, the amount of linkage disequilibrium between a disease allele and closely linked genetic markers may yield valuable information regarding the location of the disease gene. For fine-scale mapping of a disease locus, it is useful to have some knowledge of the patterns of linkage disequilibrium that exist between markers in the studied region. As mentioned above the mapping resolution achieved through the analysis of linkage disequilibrium is much higher than that of linkage studies. The high density of biallelic markers combined with linkage disequilibrium analysis provides powerful tools for fine-scale mapping. Different methods to calculate linkage disequilibrium are described below under the heading "Statistical Methods".

**Population-Based Case-Control Studies Of Trait-Marker Associations**

As mentioned above, the occurrence of pairs of specific alleles at different loci on the same chromosome is not random and the deviation from random is called linkage disequilibrium. Association studies focus on population frequencies and rely on the phenomenon of linkage disequilibrium. If a specific allele in a given gene is directly involved in causing a particular trait, its frequency will be statistically increased in an affected (trait positive) population, when compared to the frequency in a trait negative population or in a random control population. As a consequence of the existence of linkage disequilibrium, the frequency of all other alleles present in the haplotype carrying the trait-causing allele will also be increased in trait positive individuals compared to trait negative individuals or random controls. Therefore, association between the trait and any allele (specifically a biallelic marker allele) in linkage disequilibrium with the trait-causing allele will suffice to suggest the presence of a trait-related gene in that particular region. Case-control populations can be genotyped for biallelic markers to identify associations that narrowly locate a trait causing allele. As any marker in linkage disequilibrium with one given marker associated with a trait will be associated with the trait. Linkage disequilibrium allows the relative frequencies in case-control populations of a limited number of genetic polymorphisms (specifically biallelic markers) to be analyzed as an alternative to screening all possible functional polymorphisms in order to find trait-causing alleles. Association studies compare the frequency of marker alleles in unrelated case-control populations, and represent powerful tools for the dissection of complex traits.

Case-Control Populations (Inclusion Criteria)

Population-based association studies do not concern familial inheritance but compare the prevalence of a particular genetic marker, or a set of markers, in case-control populations. They are case-control studies based on comparison of unrelated case (affected or trait positive) individuals and unrelated control (unaffected, trait negative or random) individuals. Preferably the control group is composed of unaffected or trait negative individuals. Further, the control group is ethnically matched to the case population. Moreover, the control group is preferably matched to the case-population for the main known confusion factor for the trait under study (for example age-matched for an age-dependent trait). Ideally, individuals in the two samples are paired in such a way that they are expected to differ only in their disease status. The terms "trait positive population", "case population" and "affected population" are used interchangeably herein.

An important step in the dissection of complex traits using association studies is the choice of case-control populations (see Lander and Schork, 1994). A major step in the choice of case-control populations is the clinical definition of a given trait or phenotype. Any genetic trait may be analyzed by the association method proposed here by carefully selecting the individuals to be included in the trait positive and trait negative phenotypic groups. Four criteria are often useful: clinical phenotype, age at onset, family history and severity. The selection procedure for continuous or quantitative traits (such as blood pressure for example) involves selecting individuals at opposite ends of the phenotype distribution of the trait under study, so as to include in these trait positive and trait negative populations individuals with non-overlapping phenotypes. Preferably, case-control populations comprise phenotypically homogeneous populations. Trait positive and trait negative populations comprise phenotypically uniform populations of individuals representing each between 1 and 98%, preferably between 1 and 80%, more preferably between 1 and 50%, and more preferably between 1 and 30%, most preferably between 1 and 20% of the total population under study, and preferably selected among individuals exhibiting non-overlapping phenotypes. The clearer the difference between the two trait phenotypes, the greater the probability of detecting an association with biallelic markers. The selection of those drastically different but relatively uniform phenotypes enables efficient comparisons in association studies and the possible detection of marked differences at the genetic level, provided that the sample sizes of the populations under study are significant enough.

In preferred embodiments, a first group of between 50 and 300 trait positive individuals, preferably about 100 individuals, are recruited according to their phenotypes. A similar number of control individuals are included in such studies.

Association Analysis

The invention also comprises methods of detecting an association between a genotype and a phenotype, comprising the steps of: a) determining the frequency of at least one CanIon-related biallelic marker in a trait positive population according to a genotyping method of the invention; b) determining the frequency of said CanIon-related biallelic marker in a control population according to a genotyping method of the invention; and c) determining whether a statistically significant association exists between said genotype and said phenotype. In addition, the methods of detecting an association between a genotype and a phenotype of the invention encompass methods with any further limitation described in this disclosure, or those following, specified alone or in any combination: optionally, wherein said CanIon-related biallelic marker is selected from the group consisting of A1 to A18, and the complements thereof, or optionally the biallelic markers in linkage disequilibrium therewith; optionally, wherein said CanIon-related biallelic marker is selected from the group consisting of A1 to A17, and the complements thereof, or optionally the biallelic markers in linkage disequilibrium therewith; optionally, wherein said CanIon-related biallelic marker is selected from the group consisting of A12 and A16, and the complements thereof, or optionally the biallelic markers in linkage disequilibrium therewith; Optionally, said control population may be a trait negative population, or a random population; Optionally, each of said genotyping steps a) and b) may be performed on a pooled biological sample derived from each of said populations; Optionally, each of said genotyping of steps a) and b) is performed separately on biological samples derived from each individual in said population or a subsample thereof.

The general strategy to perform association studies using biallelic markers derived from a region carrying a candidate gene is to scan two groups of individuals (case-control populations) in order to measure and statistically compare the allele frequencies of the biallelic markers of the present invention in both groups.

If a statistically significant association with a trait is identified for at least one or more of the analyzed biallelic markers, one can assume that: either the associated allele is directly responsible for causing the trait (i.e. the associated allele is the trait causing allele), or more likely the associated allele is in linkage disequilibrium with the trait causing allele. The specific characteristics of the associated allele with respect to the candidate gene function usually give further insight into the relationship between the associated allele and the trait (causal or in linkage disequilibrium). If the evidence indicates that the associated allele within the candidate gene is most probably not the trait causing allele but is in linkage disequilibrium with the real trait causing allele, then the trait causing allele can be found by sequencing the vicinity of the associated marker, and performing further association studies with the polymorphisms that are revealed in an iterative manner.

Association studies are usually run in two successive steps. In a first phase, the frequencies of a reduced number of biallelic markers from the candidate gene are determined in the trait positive and control populations. In a second phase of the analysis, the position of the genetic loci responsible for the given trait is further refined using a higher density of markers from the relevant region. However, if the candidate gene under study is relatively small in length, as is the case for CanIon, a single phase may be sufficient to establish significant associations.

Haplotype Analysis

As described above, when a chromosome carrying a disease allele first appears in a population as a result of either mutation or migration, the mutant allele necessarily resides on a chromosome having a set of linked markers: the ancestral haplotype. This haplotype can be tracked through populations and its statistical association with a given trait can be analyzed. Complementing single point (allelic) association studies with multi-point association studies also called haplotype studies increases the statistical power of association studies. Thus, a haplotype association study allows one to define the frequency and the type of the ancestral carrier haplotype. A haplotype analysis is important in that it increases the statistical power of an analysis involving individual markers.

In a first stage of a haplotype frequency analysis, the frequency of the possible haplotypes based on various combinations of the identified biallelic markers of the invention is determined. The haplotype frequency is then compared for distinct populations of trait positive and control individuals. The number of trait positive individuals, which should be, subjected to this analysis to obtain statistically significant results usually ranges between 30 and 300, with a preferred number of individuals ranging between 50 and 150. The same considerations apply to the number of unaffected individuals (or random control) used in the study. The results of this first analysis provide haplotype frequencies in case-control populations, for each evaluated haplotype frequency a p-value and an odd ratio are calculated. If a statistically significant association is found the relative risk for an individual carrying the given haplotype of being affected with the trait under study can be approximated.

Methods of detecting an association between a haplotype and a phenotype, comprise the steps of: a) estimating the frequency of at least one haplotype in a trait positive population, according to a method of the invention for estimating the frequency of a haplotype; b) estimating the frequency of said haplotype in a control population, according to a method of the invention for estimating the frequency of a haplotype; and c) determining whether a statistically significant association exists between said haplotype and said phenotype. In addition, the methods of detecting an association between a haplotype and a phenotype of the invention encompass methods with any further limitation described in this disclosure, or those following: optionally, wherein said CanIon-related biallelic marker is selected from the group consisting of A1 to A18, and the complements thereof, or optionally the biallelic markers in linkage disequilibrium therewith; optionally, wherein said CanIon-relabed biallelic marker is selected from the group consisting of A 1 to 17, and the complements thereof, or optionally the biallelic markers in linkage disequilibrium therewith; optionally, wherein said CanIon-related biallelic marker is selected from the group consisting of A12 and A16, and the complements thereof, or optionally the biallelic markers in linkage disequilibrium therewith; Optionally, said control population is a trait negative population, or a random population. Optionally, said method comprises the additional steps of determining the phenotype in said trait positive and said control populations prior to step c).

Interaction Analysis

The biallelic markers suitable for use in the present invention may also be used to identify patterns of biallelic markers associated with detectable traits resulting from polygenic interactions. The analysis of genetic interaction between alleles at unlinked loci requires individual genotyping using the techniques described herein. The analysis of allelic interaction among a selected set of biallelic markers with appropriate level of statistical significance can be considered as a haplotype analysis. Interaction analysis comprises stratifying the case-control populations with respect to a given haplotype for the first loci and performing a haplotype analysis with the second loci with each subpopulation.

Statistical methods used in association studies are further described below.

**Testing For Linkage In The Presence Of Association**

The biallelic markers suitable for use in the present invention may further be used in TDT (transmission/disequilibrium test). TDT tests for both linkage and association and is not affected by population stratification. TDT requires data for affected individuals and their parents or data from unaffected sibs instead of from parents (see Spielmann S. et al., 1993; Schaid D.J. et al., 1996, Spielmann S. and Ewens W.J., 1998). Such combined tests generally reduce the false-positive errors produced by separate analyses.

**Statistical methods**

In general, any method known in the art to test whether a trait and a genotype show a statistically significant correlation may be used.

**1) Methods In Linkage Analysis**

Statistical methods and computer programs useful for linkage analysis are well-known to those skilled in the art (see Terwilliger J.D. and Ott J., 1994; Ott J., 1991).

**2) Methods To Estimate Haplotype Frequencies In A Population**

As described above, when genotypes are scored, it is often not possible to distinguish heterozygotes so that haplotype frequencies cannot be easily inferred. When the gametic phase is not known, haplotype frequencies can be estimated from the multilocus genotypic data. Any method known to person skilled in the art can be used to estimate haplotype frequencies (see Lange K., 1997; Weir, B.S., 1996). Preferably, maximum-likelihood haplotype frequencies are computed using an Expectation-Maximization (EM) algorithm (see Dempster et al., 1977; Excoffier L. and Slatkin M., 1995). This procedure is an iterative process aiming at obtaining maximum-likelihood estimates of haplotype frequencies from multi-locus genotype data when the gametic phase is unknown. Haplotype estimations are usually performed by applying the EM algorithm using for example the EM-HAPLO program (Hawley M. E. et al., 1994) or the Arlequin program (Schneider et al., 1997). The EM algorithm is a generalized iterative maximum likelihood approach to estimation and is briefly described below.

Please note that in the present section, "Methods To Estimate Haplotype Frequencies In A Population, " phenotypes will refer to multi-locus genotypes with unknown haplotypic phase. Genotypes will refer to multi-locus genotypes with known haplotypic phase.

Suppose one has a sample of N unrelated individuals typed for K markers. The data observed are the unknown-phase K-locus phenotypes that can be categorized with F different phenotypes. Further, suppose that we have *H*-possible haplotypes (in the case of $K$ biallelic markers, we have for the maximum number of possible haplotypes $H = 2^K$).

For phenotype $j$ with $c_j$ possible genotypes, we have:

$$P_j = \sum_{i=1}^{c_j} P(genotype(i)) = \sum_{i=1}^{c_j} P(h_k, h_l). \qquad \text{Equation 1}$$

where $P_j$ is the probability of the $j^{th}$ phenotype, and $P(h_k,h_l)$ is the probability of the $i^{th}$ genotype composed of haplotypes $h_k$ and $h_l$. Under random mating (i.e. Hardy-Weinberg Equilibrium), $P(h_k h_l)$ is expressed as:

$$P(h_k, h_l) = P(h_k)^2 \text{ for } h_k = h_l \text{, and}$$

$$P(h_k, h_l) = 2P(h_k)P(h_l) \text{ for } h_k \neq h_l. \qquad \text{Equation 2}$$

The E-M algorithm is composed of the following steps: First, the genotype frequencies are estimated from a set of initial values of haplotype frequencies. These haplotype frequencies are denoted $P_1^{(0)}, P_2^{(0)}, P_3^{(0)}, \ldots, P_H^{(0)}$. The initial values for the haplotype frequencies may be obtained from a random number generator or in some other way well known in the art. This step is referred to the Expectation step. The next step in the method, called the Maximization step, consists of using the estimates for the genotype frequencies to re-calculate the haplotype frequencies. The first iteration haplotype frequency estimates are denoted by $P_1^{(1)}, P_2^{(1)}, P_3^{(1)}, \ldots, P_H^{(1)}$. In general, the Expectation step at the $s^{th}$ iteration consists of calculating the probability of placing each phenotype into the different possible genotypes based on the haplotype frequencies of the previous iteration:

$$P(h_k, h_l)^{(s)} = \frac{n_j}{N}\left[\frac{P_j(h_k, h_l)^{(s)}}{P_j}\right], \qquad \text{Equation 3}$$

where $n_j$ is the number of individuals with the $j^{th}$ phenotype and $P_j(h_k, h_l)^{(s)}$ is the probability of genotype $h_k,h_l$ in phenotype $j$. In the Maximization step, which is equivalent to the gene-counting method (Smith, *Ann. Hum. Genet.,* 21:254-276, 1957), the haplotype frequencies are re-estimated based on the genotype estimates:

$$P_t^{(s+1)} = \frac{1}{2}\sum_{j=1}^{F}\ \sum_{i=1}^{c_j} \delta_{it} P_j(h_k, h_l)^{(s)}. \qquad \text{Equation 4}$$

Here, $\delta_{it}$ is an indicator variable which counts the number of occurrences that haplotype $t$ is present in $i^{th}$ genotype; it takes on values 0, 1, and 2.

The E-M iterations cease when the following criterion has been reached. Using Maximum Likelihood Estimation (MLE) theory, one assumes that the phenotypes $j$ are distributed multinomially. At each iteration $s$, one can compute the likelihood function L. Convergence is achieved when the difference of the log-likehood between two consecutive iterations is less than some small number, preferably $10^{-7}$.

**3) Methods To Calculate Linkage Disequilibrium Between Markers**

A number of methods can be used to calculate linkage disequilibrium between any two genetic positions, in practice linkage disequilibrium is measured by applying a statistical association test to haplotype data taken from a population.

Linkage disequilibrium between any pair of biallelic markers comprising at least one of the biallelic markers of the present invention ($M_i$, $M_j$) having alleles ($a_i/b_i$) at marker $M_i$ and alleles ($a_j/b_j$) at marker $M_j$ can be calculated for every allele combination ($a_i,a_j$; $a_i,b_j$; $b_i,a_j$ and $b_i,b_j$), according to the Piazza formula:

$\Delta_{aiaj} = \sqrt{\theta 4} - \sqrt{(\theta 4 + \theta 3)(\theta 4 + \theta 2)}$, where:

$\theta 4$ = - - = frequency of genotypes not having allele $a_i$ at $M_i$ and not having allele $a_j$ at $M_j$

$\theta 3$ = - + = frequency of genotypes not having allele $a_i$ at $M_i$ and having allele $a_j$ at $M_j$

$\theta 2$= + - = frequency of genotypes having allele $a_i$ at $M_i$ and not having allele $a_j$ at $M_j$

Linkage disequilibrium (LD) between pairs of biallelic markers ($M_i$, $M_j$) can also be calculated for every allele combination (ai,aj; ai,bj;$b_i$,$a_j$ and $b_i$,$b_j$), according to the maximum-likelihood estimate (MLE) for delta (the composite genotypic disequilibrium coefficient), as described by Weir (Weir B. S., 1996). The MLE for the composite linkage disequilibrium is:

$$D_{aiaj} = (2n_1 + n_2 + n_3 + n_4/2)/N - 2(pr(a_i).\ pr(a_j))$$

Where $n_1 = \Sigma$ phenotype ($a_i/a_i$, $a_j/a_j$), $n_2 = \Sigma$ phenotype ($a_i/a_i$, $a_j/b_j$, $n_3 = \Sigma$ phenotype ($a_i/b_i$, $a_j/a_j$), n4= $\Sigma$ phenotype ($a_i/b_i$, $a_j/b_j$) and N is the number of individuals in the sample.

This formula allows linkage disequilibrium between alleles to be estimated when only genotype, and not haplotype, data are available.

Another means of calculating the linkage disequilibrium between markers is as follows. For a couple of biallelic markers, $M_i$ ($a_i/b_i$) and $M_j(a_j/b_j)$, fitting the Hardy-Weinberg equilibrium, one can estimate the four possible haplotype frequencies in a given population according to the approach described above.

The estimation of gametic disequilibrium between *ai* and *aj* is simply:

$$D_{aiaj} = pr(haplotype(a_i, a_j)) - pr(a_i).pr(a_j).$$

Where $pr(a_i)$ is the probability of allele $a_i$ and pr($a_j$) is the probability of allele $a_j$ and where *pr(haplotype* ($a_i$, $a_j$)) is estimated as in Equation 3 above.

For a couple of biallelic marker only one measure of disequilibrium is necessary to describe the association between $M_i$ and $M_j$.

Then a normalized value of the above is calculated as follows:

$$D'_{aiaj} = D_{aiaj} / \max(-pr(a_i).\ pr(a_j), -pr(b_i).\ pr(b_j)) \quad \text{with } D_{aiaj}<0$$

$$D'_{aiaj} = D_{aiaj} / \max(pr(b_i).\ pr(a_j), pr(a_i).\ pr(b_j)) \quad \text{with } D_{aiaj}>0$$

The skilled person will readily appreciate that other linkage disequilibrium calculation methods can be used.

Linkage disequilibrium among a set of biallelic markers having an adequate heterozygosity rate can be determined by genotyping between 50 and 1000 unrelated individuals, preferably between 75 and 200, more preferably around 100.

**4) Testing For Association**

Methods for determining the statistical significance of a correlation between a phenotype and a genotype, in this case an allele at a biallelic marker or a haplotype made up of such alleles, may be determined by any statistical test known in the art and with any accepted threshold of statistical significance being required. The application of particular methods and thresholds of significance are well with in the skill of the ordinary practitioner of the art.

Testing for association is performed by determining the frequency of a biallelic marker allele in case and control populations and comparing these frequencies with a statistical test to determine if their is a statistically significant difference in frequency which would indicate a correlation between the trait and the biallelic marker allele under study. Similarly, a haplotype analysis is performed by estimating the frequencies of all possible haplotypes for a given set of biallelic markers in case and control populations, and comparing these frequencies with a statistical test to determine if their is a statistically significant correlation between the haplotype and the phenotype (trait) under study. Any statistical tool useful to test for a statistically significant association between a genotype and a phenotype may be used. Preferably the statistical test employed is a chi-square test with one degree of freedom. A P-value is calculated (the P-value is the probability that a statistic as large or larger than the observed one would occur by chance).

Statistical Significance

In preferred embodiments, significance for diagnosis purposes, either as a positive basis for further diagnostic tests or as a preliminary starting point for early preventive therapy, the p value related to a biallelic marker association is preferably about 1 x $10^{-2}$ or less, more preferably about 1 x $10^{-4}$ or less, for a single biallelic marker analysis and about 1 x $10^{-3}$ or less, still more preferably 1 x $19^{-6}$ or less and most preferably of about 1 x $10^{-8}$ or less, for a haplotype analysis involving two or more markers. These values are believed to be applicable to any association studies involving single or multiple marker combinations.

The skilled person can use the range of values set forth above as a starting point in order to carry out association studies with biallelic markers of the present invention. In doing so, significant associations between the biallelic markers of the present invention and a trait can be revealed and used for diagnosis and drug screening purposes.

Phenotypic Permutation

In order to confirm the statistical significance of the first stage haplotype analysis described above, it might be suitable to perform further analyses in which genotyping data from case-control individuals are pooled and randomized with respect to the trait phenotype. Each individual genotyping data is randomly allocated to two groups, which contain the same number of individuals as the case-control populations used to compile the data obtained in the first stage. A second stage haplotype analysis is preferably run on these artificial groups, preferably for the markers included in the haplotype of the first stage analysis showing the highest relative risk coefficient. This experiment is reiterated preferably at least between 100 and 10000 times. The repeated iterations allow the determination of the probability to obtain the tested haplotype by chance.

Assessment Of Statistical Association

To address the problem of false positives similar analysis may be performed with the same case-control populations in random genomic regions. Results in random regions and the candidate region are compared as described in a co-pending US Provisional Patent Application entitled "Methods, Software And Apparati For Identifying Genomic Regions Harboring A Gene Associated With A Detectable Trait," U.S. Serial Number 60/107,986, filed November 10, 1998.

**5) Evaluation Of Risk Factors**

The association between a risk factor (in genetic epidemiology the risk factor is the presence or the absence of a certain allele or haplotype at marker loci) and a disease is measured by the odds ratio (OR) and by the relative risk (RR). If $P(R^+)$ is the probability of developing the disease for individuals with R and $P(R^-)$ is the probability for individuals without the risk factor, then the relative risk is simply the ratio of the two probabilities, that is:

$$RR = P(R^+)/P(R^-)$$

In case-control studies, direct measures of the relative risk cannot be obtained because of the sampling design. However, the odds ratio allows a good approximation of the relative risk for low-incidence diseases and can be calculated:

$$OR = \left[\frac{F^+}{1-F^+}\right] \Big/ \left[\frac{F^-}{(1-F^-)}\right]$$

$$OR = (F^+/(1-F^+))/(F^-/(1-F^-))$$

$F^+$ is the frequency of the exposure to the risk factor in cases and F is the frequency of the exposure to the risk factor in controls. $F^+$ and F are calculated using the allelic or haplotype frequencies of the study and further depend on the underlying genetic model (dominant, recessive, additive...).

One can further estimate the attributable risk (AR) which describes the proportion of individuals in a population exhibiting a trait due to a given risk factor. This measure is important in quantifying the role of a specific factor in disease etiology and in terms of the public health impact of a risk factor. The public health relevance of this measure lies in estimating the proportion of cases of disease in the population that could be prevented if the exposure of interest were absent. AR is determined as follows:

$$AR = P_E (RR-1) / (P_E (RR-1)+1)$$

AR is the risk attributable to a biallelic marker allele or a biallelic marker haplotype. $P_E$ is the frequency of exposure to an allele or a haplotype within the population at large; and RR is the relative risk which, is approximated with the odds ratio when the trait under study has a relatively low incidence in the general population.

**Identification Of Biallelic Markers In Linkage Disequilibrium With The Biallelic Markers of the Invention**

Once a first biallelic marker has been identified in a genomic region of interest, the practitioner of ordinary skill in the art, using the teachings of the present invention, can easily identify additional biallelic markers in linkage disequilibrium with this first marker. As mentioned before any marker in linkage disequilibrium with a first marker associated with a trait will be associated with the trait. Therefore, once an association has been demonstrated between a given biallelic marker and a trait, the discovery of additional biallelic markers associated with this trait is of great interest in order to increase the density of biallelic markers in this particular region. The causal gene or mutation will be found in the vicinity of the marker or set of markers showing the highest correlation with the trait.

Identification of additional markers in linkage disequilibrium with a given marker involves:

(a) amplifying a genomic fragment comprising a first biallelic marker from a plurality of individuals;
(b) identifying of second biallelic markers in the genomic region harboring said first biallelic marker;
(c) conducting a linkage disequilibrium analysis between said first biallelic marker and second biallelic markers; and
(d) selecting said second biallelic markers as being in linkage disequilibrium with said first marker. Subcombinations comprising steps (b) and (c) are also contemplated.

Methods to identify biallelic markers and to conduct linkage disequilibrium analysis are described herein and can be carried out by the skilled person without undue experimentation. The present invention then also concerns biallelic markers which are in linkage disequilibrium with the biallelic markers A1 to A18 and which are expected to present similar characteristics in terms of their respective association with a given trait.

**Identification Of Functional Mutations**

Mutations in the CanIon gene which are responsible for a detectable phenotype or trait may be identified by comparing the sequences of the CanIon gene from trait positive and control individuals. Once a positive association is confirmed with a biallelic marker of the present invention, the identified locus can be scanned for mutations. In a preferred embodiment, functional regions such as exons and splice sites, promoters and other regulatory regions of the CanIon gene are scanned for mutations. In a preferred embodiment the sequence of the CanIon gene is compared in trait positive and control individuals. Preferably, trait positive individuals carry the haplotype shown to be associated with the trait and trait negative individuals do not carry the haplotype or allele associated with the trait. The detectable trait or phenotype may comprise a variety of manifestations of altered CanIon function.

The mutation detection procedure is essentially similar to that used for biallelic marker identification. The method used to detect such mutations generally comprises the following steps:

- amplification of a region of the CanIon gene comprising a biallelic marker or a group of biallelic markers associated with the trait from DNA samples of trait positive patients and trait-negative controls;
- sequencing of the amplified region;
- comparison of DNA sequences from trait positive and control individuals;
- determination of mutations specific to trait-positive patients.

In one embodiment, said biallelic marker is selected from the group consisting of A1 to A18, and the complements thereof. It is preferred that candidate polymorphisms be then verified by screening a larger population of cases and controls by means of any genotyping procedure such as those described herein, preferably using a microsequencing technique in an individual test format. Polymorphisms are considered as candidate mutations when present in cases and controls at frequencies compatible with the expected association results. Polymorphisms are considered as candidate "trait-causing" mutations when they exhibit a statistically significant correlation with the detectable phenotype.

## Biallelic Markers Of The Invention In Methods Of Genetic Diagnostics

The ion channel nucleic acid sequence and biallelic markers suitable for use in the present invention can also be used to develop diagnostic tests capable of identifying individuals who express a detectable trait as the result of a specific genotype or individuals whose genotype places them at risk of developing a detectable trait at a subsequent time. Such a diagnosis can be useful in the staging, monitoring, prognosis and/or prophylactic or curative therapy of numerous diseases or conditions including schizophrenia, bipolar disorder, and other CNS disorders such as epilepsy and pain disorders, cardiovascular conditions such as heart disease, hypertension, arrythmias, and numerous other diseases and conditions.

The diagnostic techniques of the present invention may employ a variety of methodologies to determine whether a test subject has a biallelic marker pattern associated with an increased risk of developing a detectable trait or whether the individual suffers from a detectable trait as a result of a particular mutation, including methods which enable the analysis of individual chromosomes for haplotyping, such as family studies, single sperm DNA analysis or somatic hybrids.

The present invention provides diagnostic methods to determine whether an individual is at risk of developing a disease or suffers from a disease resulting from a mutation or a polymorphism in the ion channel gene. The present invention also provides methods to determine whether an individual has a susceptibility to schizophrenia and bipolar disorder, or to any of the other calcium-channel related conditions known in the art or described herein.

These methods involve obtaining a nucleic acid sample from the individual and, determining, whether the nucleic acid sample contains at least one allele or at least one biallelic marker haplotype, indicative of a risk of developing the trait or indicative that the individual expresses the trait as a result of possessing a particular ion channel polymorphism or mutation (trait-causing allele).

Preferably, in such diagnostic methods, a nucleic acid sample is obtained from the individual and this sample is genotyped using methods described above in "Methods Of Genotyping DNA Samples For Biallelic markers. The diagnostics may be based on a single biallelic marker or a on group of biallelic markers.

In each of these methods, a nucleic acid sample is obtained from the test subject and the biallelic marker pattern of one or more of the biallelic markers A1 to A18 is determined.

In one embodiment, a PCR amplification is conducted on the nucleic acid sample to amplify regions in which polymorphisms associated with a detectable phenotype have been identified. The amplification products are sequenced to determine whether the individual possesses one or more polymorphisms associated with a detectable phenotype. The primers used to generate amplification products may comprise the primers listed in Table 1. Alternatively, the nucleic acid sample is subjected to microsequencing reactions as described above to determine whether the individual possesses one or more ion channel polymorphisms associated with a detectable phenotype resulting from a mutation or a polymorphism in the ion channel gene. The primers used in the microsequencing reactions may include the primers listed in Table 4. In another embodiment, the nucleic acid sample is contacted with one or more allele specific oligonucleotide probes which, specifically hybridize to one or more ion channel alleles associated with a detectable phenotype. The probes used in the hybridization assay may include the probes listed in Table 3. In another embodiment, the nucleic acid sample is contacted with a second ion channel oligonucleotide capable of producing an amplification product when used with the allele specific oligonucleotide in an amplification reaction. The presence of an amplification product in the amplification reaction indicates that the individual possesses one or more ion channel alleles associated with a detectable phenotype.

In a preferred embodiment the identity of the nucleotide present at, at least one, biallelic marker selected from the group consisting of A1 to A18 and the complements thereof, and the complements thereof, is determined and the detectable trait is schizophrenia and bipolar disorder. Diagnostic kits comprise any of the polynucleotides described herein.

These diagnostic methods are extremely valuable as they can, in certain circumstances, be used to initiate preventive treatments or to allow an individual carrying a significant haplotype to foresee warning signs such as minor symptoms.

Diagnostics, which analyze and predict response to a drug or side effects to a drug, may be used to determine whether an individual should be treated with a particular drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the markers suitable for use in the present invention. One or more markers indicative of response to an agent acting against schizophrenia or bipolar disorder or another calcium channel-related condition, or to side effects to an agent acting against schizophrenia or bipolar disorder or another calcium channel-related condition, may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

In particularly preferred embodiments, the trait analyzed using the present diagnostics is schizophrenia or bipolar disorder. However, the present invention also comprises any of the prevention, diagnostic, prognosis and treatment methods described herein using the biallelic markers of the invention in methods of preventing, diagnosing, managing and treating related disorders, particularly related CNS disorders. By way of example, related disorders may comprise psychotic disorders, mood disorders, autism, substance dependence and alcoholism, pain disorders, epilepsy, mental retardation, and other psychiatric diseases including cognitive, anxiety, eating, impulse-control, and personality disorders, as defined with the Diagnosis and Statistical Manual of Mental Disorders fourth edition (DSM-IV) classification. Other disorders include cardiovascular disorders such as angina, hypertension, or arrythmias.

## Recombinant Vectors

The term "vector" is used herein to designate either a circular or a linear DNA or RNA molecule, which is either double-stranded or single-stranded, and which comprise at least one polynucleotide of interest that is sought to be transferred in a cell host or in a unicellular or multicellular host organism.

Suitable for use in the present invention is a family of recombinant vectors that comprise a regulatory polynucleotide derived from the CanIon genomic sequence, and/or a coding polynucleotide from either the CanIon genomic sequence or the cDNA sequence.

Generally, a recombinant vector of the invention may comprise any of the polynucleotides described herein, including regulatory sequences, coding sequences and polynucleotide constructs, as well as any CanIon primer or probe as defined above. More particularly, the recombinant vectors suitable for use in the present invention can comprise any of the polynucleotides described in the "Genomic Sequences Of The CanIon Gene" section, the "CanIon cDNA Sequences" section, the "Coding Regions" section, the "Polynucleotide constructs" section, and the "Oligonucleotide Probes And Primers" section.

In a first preferred embodiment, a recombinant vector suitable for use in the invention is used to amplify the inserted polynucleotide derived from a genomic sequence of SEQ ID No 1 to 3 or 6 or a CanIon cDNA, for example the cDNA of SEQ ID No 4 in a suitable cell host, this polynucleotide being amplified every time that the recombinant vector replicates.

A second preferred embodiment suitable for use in the recombinant vectors according to the invention comprises expression vectors comprising either a regulatory polynucleotide or a coding nucleic acid of the invention, or both. Within certain embodiments, expression vectors are employed to express the CanIon polypeptide which can be then purified and, for example be used in ligand screening assays or as an immunogen in order to raise specific antibodies directed against the CanIon protein. In other embodiments, the expression vectors are used for constructing transgenic animals and also for gene therapy. Expression requires that appropriate signals are provided in the vectors, said signals including various regulatory elements, such as enhancers/promoters from both viral and mammalian sources that drive expression of the genes of interest in host cells. Dominant drug selection markers for establishing permanent, stable cell clones expressing the products are generally included in the expression vectors of the invention, as they are elements that link expression of the drug selection markers to expression of the polypeptide.

More particularly, suitable for use in the present invention are expression vectors which include nucleic acids encoding a CanIon protein, preferably the CanIon protein of the amino acid sequence of SEQ ID No 5 or variants or fragments thereof.

Suitable for use in the invention is a recombinant expression vector useful for the expression of the CanIon coding sequence, wherein said vector comprises a nucleic acid of SEQ ID No 4.

Recombinant vectors comprising a nucleic acid containing a CanIon-related biallelic marker is also part of the invention. In a preferred embodiment, said biallelic marker is selected from the group consisting of A1 to A18, and the complements thereof.

Some of the elements which can be found in the vectors of the present invention are described in further detail in the following sections.

Suitable for use in the present invention are primary, secondary, and immortalized homologously recombinant host cells of vertebrate origin, preferably mammalian origin and particularly human origin, that have been engineered to: a) insert exogenous (heterologous) polynucleotides into the endogenous chromosomal DNA of a targeted gene, b) delete endogenous chromosomal DNA, and/or c) replace endogenous chromosomal DNA with exogenous polynucleotides. Insertions, deletions, and/or replacements of polynucleotide sequences may be to the coding sequences of the targeted gene and/or to regulatory regions, such as promoter and enhancer sequences, operably associated with the targeted gene.

Suitable for use in the present invention is a method of making a homologously recombinant host cell in vitro or in vivo, wherein the expression of a targeted gene not normally expressed in the cell is altered. Preferably the alteration causes expression of the targeted gene under normal growth conditions or under conditions suitable for producing the polypeptide encoded by the targeted gene. The method comprises the steps of: (a) transfecting the cell in vitro or in vivo with a polynucleotide construct, the polynucleotide construct comprising; (i) a targeting sequence; (ii) a regulatory sequence and/or a coding sequence; and (iii) an unpaired splice donor site, if necessary, thereby producing a transfected cell; and (b) maintaining the transfected cell in vitro or in vivo under conditions appropriate for homologous recombination.

Suitable for use in the present invention is a method of altering the expression of a targeted gene in a cell in vitro or in vivo wherein the gene is not normally expressed in the cell, comprising the steps of: (a) transfecting the cell in vitro or in vivo with a a polynucleotide construct, the a polynucleotide construct comprising: (i) a targeting sequence; (ii) a regulatory sequence and/or a coding sequence; and (iii) an unpaired splice donor site, if necessary, thereby producing a transfected cell; and (b) maintaining the transfected cell in vitro or in vivo under conditions appropriate for homologous recombination, thereby producing a homologously recombinant cell; and (c) maintaining the homologously recombinant cell in vitro or in vivo under conditions appropriate for expression of the gene.

Suitable for use in the present invention is a method of making a polypeptide of the present invention by altering the expression of a targeted endogenous gene in a cell in vitro or in vivo wherein the gene is not normally expressed in the cell, comprising the steps of: a) transfecting the cell in vitro with a polynucleotide construct, the a polynucleotide construct comprising: (i) a targeting sequence; (ii) a regulatory sequence and/or a coding sequence; and (iii) an unpaired splice donor site, if necessary, thereby producing a transfected cell; (b) maintaining the transfected cell in vitro or in vivo under conditions appropriate for homologous recombination, thereby producing a homologously recombinant cell; and c) maintaining the homologously recombinant cell in vitro or in vivo under conditions appropriate for expression of the gene, thereby making the polypeptide.

Suitable for use in the present invention is a polynucleotide construct which alters the expression of a targeted gene in a cell type in which the gene is not normally expressed. This occurs when the polynucleotide construct is inserted into the chromosomal DNA of the target cell, wherein the a polynucleotide construct comprises: a) a targeting sequence; b) a regulatory sequence and/or coding sequence; and c) an unpaired splice-donor site, if necessary. Further included are a polynucleotide constructs, as described above, wherein the construct further comprises a polynucleotide which encodes a polypeptide and is in-frame with the targeted endogenous gene after homologous recombination with chromosomal DNA.

The compositions may be produced, and methods performed, by techniques known in the art, such as those described in U.S. Patent Nos: 6,054,288; 6,048,729; 6,048,724; 6,048,524; 5,994,127; 5,968,502; 5,965,125; 5,869,239; 5,817,789; 5,783,385; 5,733,761; 5,641,670; 5,580,734; International Publication Nos:WO96/29411, WO 94/12650; and scientific articles including Koller et al., PNAS 86:8932-8935 (1989).

### 1. General features of the expression vectors of the invention

A recombinant vector suitable for use in the invention comprises, but is not limited to, a YAC (Yeast Artificial Chromosome), a BAC (Bacterial Artificial Chromosome), a phage, a phagemid, a cosmid, a plasmid or even a linear DNA molecule which may comprise a chromosomal, non-chromosomal, semi-synthetic and synthetic DNA. Such a recombinant vector can comprise a transcriptional unit comprising an assembly of:

(1) a genetic element or elements having a regulatory role in gene expression, for example promoters or enhancers. Enhancers are cis-acting elements of DNA, usually from about 10 to 300 bp in length that act on the promoter to increase the transcription.
(2) a structural or coding sequence which is transcribed into mRNA and eventually translated into a polypeptide, said structural or coding sequence being operably linked to the regulatory elements described in (1); and
(3) appropriate transcription initiation and termination sequences. Structural units intended for use in yeast or eukaryotic expression systems preferably include a leader sequence enabling extracellular secretion of translated protein by a host cell. Alternatively, when a recombinant protein is expressed without a leader or transport sequence, it may include a N-terminal residue. This residue may or may not be subsequently cleaved from the expressed recombinant protein to provide a final product.

Generally, recombinant expression vectors will include origins of replication, selectable markers permitting transformation of the host cell, and a promoter derived from a highly expressed gene to direct transcription of a downstream structural sequence. The heterologous structural sequence is assembled in appropriate phase with translation initiation and termination sequences, and preferably a leader sequence capable of directing secretion of the translated protein into the periplasmic space or the extracellular medium. In a specific embodiment wherein the vector is adapted for transfecting and expressing desired sequences in mammalian host cells, preferred vectors will comprise an origin of replication in the desired host, a suitable promoter and enhancer, and also any necessary ribosome binding sites, polyadenylation signal, splice donor and acceptor sites, transcriptional termination sequences, and 5'-flanking non-transcribed sequences. DNA sequences derived from the SV40 viral genome, for example SV40 origin, early promoter, enhancer, splice and polyadenylation signals may be used to provide the required non-transcribed genetic elements.

The *in vivo* expression of a CanIon polypeptide of SEQ ID No 5 or fragments or variants thereof may be useful in order to correct a genetic defect related to the expression of the native gene in a host organism or to the production of a biologically inactive CanIon protein.

Consequently, suitable for use in the present invention are recombinant expression vectors mainly designed for the *in vivo* production of the CanIon polypeptide of SEQ ID No 5 or fragments or variants thereof by the introduction of the appropriate genetic material in the organism of the patient to be treated. This genetic material may be introduced *in vitro* in a cell that has been previously extracted from the organism, the modified cell being subsequently reintroduced in the said organism, directly *in vivo* into the appropriate tissue.

### 2. Regulatory Elements

Promoters

The suitable promoter regions used in the expression vectors suitable for use in the present invention are chosen taking into account the cell host in which the heterologous gene has to be expressed. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell, such as, for example, a human or a viral promoter.

A suitable promoter may be heterologous with respect to the nucleic acid for which it controls the expression or alternatively can be endogenous to the native polynucleotide containing the coding sequence to be expressed. Additionally, the promoter is generally heterologous with respect to the recombinant vector sequences within which the construct promoter/coding sequence has been inserted.

Promoter regions can be selected from any desired gene using, for example, CAT (chloramphenicol transferase) vectors and more preferably pKK232-8 and pCM7 vectors.

Preferred bacterial promoters are the LacI, LacZ, the T3 or T7 bacteriophage RNA polymerase promoters, the gpt, lambda PR, PL and trp promoters (EP 0036776), the polyhedrin promoter, or the p10 protein promoter from baculovirus (Kit Novagen) (Smith et al., 1983; O'Reilly et al., 1992), the lambda PR promoter or also the trc promoter.

Eukaryotic promoters include CMV immediate early, HSV thymidine kinase, early and late SV40, LTRs from retrovirus, and mouse metallothionein-L. Selection of a convenient vector and promoter is well within the level of ordinary skill in the art.

The choice of a promoter is well within the ability of a person skilled in the field of genetic engineering. For example, one may refer to Sambrook et al. (1989) or also to the procedures described by Fuller et al. (1996).

Other regulatory elements

Where a cDNA insert is employed, one will typically desire to include a polyadenylation signal to effect proper polyadenylation of the gene transcript. The nature of the polyadenylation signal is not believed to be crucial to the successful practice of the invention, and any such sequence may be employed such as human growth hormone and SV40 polyadenylation signals. Also contemplated as an element of the expression cassette is a terminator. These elements can serve to enhance message levels and to minimize read through from the cassette into other sequences.

### 3. Selectable Markers

Such markers would confer an identifiable change to the cell permitting easy identification of cells containing the expression construct. The selectable marker genes for selection of transformed host cells are preferably dihydrofolate reductase or neomycin resistance for eukaryotic cell culture, TRP1 for *S. cerevisiae* or tetracycline, rifampicin or ampicillin resistance in *E.coli,* or levan saccharase for mycobacteria, this latter marker being a negative selection marker.

**4. Preferred Vectors.**

Bacterial vectors

As a representative but non-limiting example, useful expression vectors for bacterial use can comprise a selectable marker and a bacterial origin of replication derived from commercially available plasmids comprising genetic elements of pBR322 (ATCC 37017). Such commercial vectors include, for example, pKK223-3 (Pharmacia, Uppsala, Sweden), and GEM1 (Promega Biotec, Madison, WI, USA).

Large numbers of other suitable vectors are known to those of skill in the art, and commercially available, such as the following bacterial vectors: pQE70, pQE60, pQE-9 (Qiagen), pbs, pD10, phagescript, psiX174, pbluescript SK, pbsks, pNH8A, pNH16A, pNH18A, pNH46A (Stratagene); ptrc99a, pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIAexpress).

Bacteriophage vectors

The P1 bacteriophage vector may contain large inserts ranging from about 80 to about 100 kb.

The construction of P 1 bacteriophage vectors such as p158 or p158/neo8 are notably described by Sternberg (1992, 1994). Recombinant P1 clones comprising CanIon nucleotide sequences may be designed for inserting large polynucleotides of more than 40 kb (Linton et al., 1993). To generate P1 DNA for transgenic experiments, a preferred protocol is the protocol described by McCormick et al. (1994). Briefly, *E. coli* (preferably strain NS3529) harboring the P1 plasmid are grown overnight in a suitable broth medium containing 25 μg/ml of kanamycin. The P1 DNA is prepared from the *E. coli* by alkaline lysis using the Qiagen Plasmid Maxi kit (Qiagen, Chatsworth, CA, USA), according to the manufacturer's instructions. The P1 DNA is purified from the bacterial lysate on two Qiagen-tip 500 columns, using the washing and elution buffers contained in the kit. A phenol/chloroform extraction is then performed before precipitating the DNA with 70% ethanol. After solubilizing the DNA in TE (10 mM Tris-HCl, pH 7.4, 1 mM EDTA), the concentration of the DNA is assessed by spectrophotometry.

When the goal is to express a P 1 clone comprising CanIon nucleotide sequences in a transgenic animal, typically in transgenic mice, it is desirable to remove vector sequences from the P1 DNA fragment, for example by cleaving the P1 DNA at rare-cutting sites within the P1 polylinker (*Sfi*I, *Not*I or *Sal*I). The P1 insert is then purified from vector sequences on a pulsed-field agarose gel, using methods similar using methods similar to those originally reported for the isolation of DNA from YACs (Schedl et al., 1993a; Peterson et al., 1993). At this stage, the resulting purified insert DNA can be concentrated, if necessary, on a Millipore Ultrafree-MC Filter Unit (Millipore, Bedford, MA, USA - 30,000 molecular weight limit) and then dialyzed against microinjection buffer (10 mM Tris-HCl, pH 7.4; 250 μM EDTA) containing 100 mM NaCl, 30 μM spermine, 70 μM spermidine on a microdyalisis membrane (type VS, 0.025 μM from Millipore). The intactness of the purified P1 DNA insert is assessed by electrophoresis on 1% agarose (Sea Kem GTG; FMC Bio-products) pulse-field gel and staining with ethidium bromide.

Baculovirus vectors

A suitable vector for the expression of the CanIon polypeptide of SEQ ID No 5 or fragments or variants thereof is a baculovirus vector that can be propagated in insect cells and in insect cell lines. A specific suitable host vector system is the pVL1392/1393 baculovirus transfer vector (Pharmingen) that is used to transfect the SF9 cell line (ATCC N°CRL 1711) which is derived from *Spodoptera frugiperda.*

Other suitable vectors for the expression of the CanIon polypeptide of SEQ ID No 5 or fragments or variants thereof in a baculovirus expression system include those described by Chai et al. (1993), Vlasak et al. (1983) and Lenhard et al. (1996).

Viral vectors

In one specific embodiment, the vector is derived from an adenovirus. Preferred adenovirus vectors according to the invention are those described by Feldman and Steg (1996) or Ohno et al. (1994). Another preferred recombinant adenovirus according to this specific embodiment of the present invention is the human adenovirus type 2 or 5 (Ad 2 or Ad 5) or an adenovirus of animal origin (see, e.g., French patent application N° FR-93.05954).

Retrovirus vectors and adeno-associated virus vectors are generally understood to be the recombinant gene delivery systems of choice for the transfer of exogenous polynucleotides *in vivo* , particularly to mammals, including humans. These vectors provide efficient delivery of genes into cells, and the transferred nucleic acids are stably integrated into the chromosomal DNA of the host.

Particularly preferred retroviruses for the preparation or construction of retroviral *in vitro* or in vitro gene delivery vehicles of the present invention include retroviruses selected from the group consisting of Mink-Cell Focus Inducing Virus, Murine Sarcoma Virus, Reticuloendotheliosis virus and Rous Sarcoma virus. Particularly preferred Murine Leukemia Viruses include the 4070A and the 1504A viruses, Abelson (ATCC No VR-999), Friend (ATCC No VR-245), Gross (ATCC No VR-590), Rauscher (ATCC No VR-998) and Moloney Murine Leukemia Virus (ATCC No VR-190; PCT Application No WO 94/24298). Particularly preferred Rous Sarcoma Viruses include Bryan high titer (ATCC Nos VR-334, VR-657, VR-726, VR-659 and VR-728). Other preferred retroviral vectors are those described in Roth et al. (1996), PCT Application No WO 93/25234, PCT Application No WO 94/ 06920, Roux et al., 1989, Julan et al., 1992 and Neda et al., 1991.

Yet another viral vector system suitable for use in the invention comprises the adeno-associated virus (AAV). The adeno-associated virus is a naturally occurring defective virus that requires another virus, such as an adenovirus or a herpes virus, as a helper virus for efficient replication and a productive life cycle (Muzyczka et al., 1992). It is also one of the few viruses that may integrate its DNA into non-dividing cells, and exhibits a high frequency of stable integration (Flotte et al., 1992; Samulski et al., 1989; McLaughlin et al., 1989). One advantageous feature of AAV derives from its reduced efficacy for transducing primary cells relative to transformed cells.

<u>BAC vectors</u>

The bacterial artificial chromosome (BAC) cloning system (Shizuya et al., 1992) has been developed to stably maintain large fragments of genomic DNA (100-300 kb) in *E. coli.* A preferred BAC vector comprises a pBcloBAC11 vector that has been described by Kim et al. (1996). BAC libraries are prepared with this vector using size-selected genomic DNA that has been partially digested using enzymes that permit ligation into either the *Bam* HI or *Hind*III sites in the vector. Flanking these cloning sites are T7 and SP6 RNA polymerase transcription initiation sites that can be used to generate end probes by either RNA transcription or PCR methods. After the construction of a BAC library in *E. coli,* BAC DNA is purified from the host cell as a supercoiled circle. Converting these circular molecules into a linear form precedes both size determination and introduction of the BACs into recipient cells. The cloning site is flanked by two *Not* I sites, permitting cloned segments to be excised from the vector by *Not* I digestion. Alternatively, the DNA insert contained in the pBeloBAC11 vector may be linearized by treatment of the BAC vector with the commercially available enzyme lambda terminase that leads to the cleavage at the unique *cos*N site, but this cleavage method results in a full length BAC clone containing both the insert DNA and the BAC sequences.

**5. Delivery Of The Recombinant Vectors**

In order to effect expression of the polynucleotides and polynucleotide constructs suitable for use in the invention, these constructs must be delivered into a cell. This delivery may be accomplished in vitro, as in laboratory procedures for transforming cell lines, or in *vivo* or *ex vivo,* as in the treatment of certain diseases states.

One mechanism is viral infection where the expression construct is encapsulated in an infectious viral particle.

Several non-viral methods for the transfer of polynucleotides into cultured mammalian cells are suitable for use in the present invention, and include, without being limited to, calcium phosphate precipitation (Graham et al., 1973; Chen et al., 1987;), DEAE-dextran (Gopal, 1985), electroporation (Tur-Kaspa et al., 1986; Potter et al., 1984), direct microinjection (Harland et al., 1985), DNA-loaded liposomes (Nicolau et al., 1982; Fraley et al., 1979), and receptor-mediated transfection (Wu and Wu, 1987; 1988). Some of these techniques may be successfully adapted for in vivo or *ex vivo* use.

Once the expression polynucleotide has been delivered into the cell, it may be stably integrated into the genome of the recipient cell. This integration may be in the cognate location and orientation via homologous recombination (gene replacement) or it may be integrated in a random, non specific location (gene augmentation). In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle.

One specific embodiment for a method for delivering a protein or peptide to the interior of a cell of a vertebrate *in vivo* comprises the step of introducing a preparation comprising a physiologically acceptable carrier and a naked polynucleotide operatively coding for the polypeptide of interest into the interstitial space of a tissue comprising the cell, whereby the naked polynucleotide is taken up into the interior of the cell and has a physiological effect. This is particularly applicable for transfer *in vitro* but it may be applied to *in vivo* as well.

Compositions for use *in vitro* and *in vivo* comprising a "naked" polynucleotide are described in PCT application No. WO 90/11092 (Vical Inc.) and also in PCT application No. WO 95/11307 (Institut Pasteur, INSERM, Université d'Ottawa) as well as in the articles of Tacson et al. (1996) and of Huygen et al. (1996).

In still another embodiment, the transfer of a naked polynucleotide suitable for use in the invention, including a polynucleotide construct of the invention, into cells may be proceeded with a particle bombardment (biolistic), said particles being DNA-coated microprojectiles accelerated to a high velocity allowing them to pierce cell membranes and enter cells without killing them, such as described by Klein et al. (1987).

In a further embodiment, the polynucleotide suitable for use in the invention may be entrapped in a liposome (Ghosh and Bacchawat, 1991; Wong et al., 1980; Nicolau et al., 1987)

Suitable for use in the invention is a composition for the *in vivo* production of the CanIon protein or polypeptide described herein. It comprises a naked polynucleotide operatively coding for this polypeptide, in solution in a physiologically acceptable carrier, and suitable for introduction into a tissue to cause cells of the tissue to express the said protein or polypeptide.

The amount of vector to be injected to the desired host organism varies according to the site of injection. As an indicative dose, it will be injected between 0.1 and 100 μg of the vector in an animal body, preferably a mammalian body, for example a mouse body.

In another embodiment of the vector suitable for use in the invention, it may be introduced *in vitro* in a host cell, preferably in a host cell previously harvested from the animal to be treated and more preferably a somatic cell such as a muscle cell. In a subsequent step, the cell that has been transformed with the vector coding for the desired CanIon polypeptide or the desired fragment thereof is reintroduced into the animal body in order to deliver the recombinant protein within the body either locally or systemically.

## Cell Hosts

Suitable for use in the present invention is a host cell that has been transformed or transfected with one of the polynucleotides described herein, and in particular a polynucleotide either comprising a CanIon regulatory polynucleotide or the coding sequence of the CanIon polypeptide selected from the group consisting of SEQ ID Nos 1 to 4 or a fragment or a variant thereof. Also suitable are host cells that are transformed (prokaryotic cells) or that are transfected (eukaryotic cells) with a recombinant vector such as one of those described above. More particularly, the cell hosts of the present invention can comprise any of the polynucleotides described in the "Genomic Sequences Of The CanIon Gene" section, the "CanIon cDNA Sequences" section, the "Coding Regions" section, the "Polynucleotide constructs" section, and the "Oligonucleotide Probes And Primers" section.

A further recombinant cell host according to the invention comprises a polynucleotide containing a biallelic marker selected from the group consisting of A1 to A18, and the complements thereof.

An additional recombinant cell host according to the invention comprises any of the vectors described herein, more particularly any of the vectors described in the " Recombinant Vectors" section.

Preferred host cells used as recipients for the expression vectors suitable for use in the invention are the following:

a) Prokaryotic host cells: *Escherichia coli* strains (I.E.DH5-α strain), *Bacillus subtilis, Salmonella typhimurium,* and strains from species like *Pseudomonas, Streptomyces* and *Staphylococcus.*

b) Eukaryotic host cells: HeLa cells (ATCC N°CCL2; N°CCL2.1; N°CCL2.2), Cv 1 cells (ATCC N°CCL70), COS cells (ATCC N°CRL1650; N°CRL1651), Sf-9 cells (ATCC N°CRL1711), C127 cells (ATCC N° CRL-1804), 3T3 (ATCC N° CRL-6361), CHO (ATCC N° CCL-61), human kidney 293. (ATCC N° 45504; N° CRL-1573) and BHK (ECACC N° 84100501; N° 84111301).

c) Other mammalian host cells.

The CanIon gene expression in mammalian, and typically human, cells may be rendered defective, or alternatively it may be preceded with the insertion of a CanIon genomic or cDNA sequence with the replacement of the CanIon gene counterpart in the genome of an animal cell by a CanIon polynucleotide suitable for use in the invention. These genetic alterations may be generated by homologous recombination events using specific DNA constructs that have been previously described.

One kind of cell hosts that may be used are mammal zygotes, such as murine zygotes. For example, murine zygotes may undergo microinjection with a purified DNA molecule of interest, for example a purified DNA molecule that has previously been adjusted to a concentration range from 1 ng/ml -for BAC inserts- 3 ng/μl —for P1 bacteriophage inserts- in 10 mM Tris-HCl, pH 7.4, 250 μM EDTA containing 100 mM NaCl, 30 μM spermine, and70 μM spermidine. When the DNA to be microinjected has a large size, polyamines and high salt concentrations can be used in order to avoid mechanical breakage of this DNA, as described by Schedl et al. (1993b).

Any one of the polynucleotides suitable for use in the invention, including the DNA constructs described herein, may be introduced in an embryonic stem (ES) cell line, preferably a mouse ES cell line. ES cell lines are derived from pluripotent, uncommitted cells of the inner cell mass of pre-implantation blastocysts. Preferred ES cell lines are the following: ES-E14TG2a (ATCC n° CRL-1821), ES-D3 (ATCC n° CRL1934 and n° CRL-11632), YS001 (ATCC n° CRL-11776), 36.5 (ATCC n° CRL-11116). To maintain ES cells in an uncommitted state, they are cultured in the presence of growth inhibited feeder cells which provide the appropriate signals to preserve this embryonic phenotype and serve as a matrix for ES cell adherence. Preferred feeder cells are primary embryonic fibroblasts that are established from tissue of day 13- day 14 embryos of virtually any mouse strain, that are maintained in culture, such as described by Abbondanzo et al.(1993) and are inhibited in growth by irradiation, such as described by Robertson (1987), or by the presence of an inhibitory concentration of LIF, such as described by Pease and Williams (1990).

The constructs in the host cells can be used in a conventional manner to produce the gene product encoded by the recombinant sequence.

Following transformation of a suitable host and growth of the host to an appropriate cell density, the selected promoter is induced by appropriate means, such as temperature shift or chemical induction, and cells are cultivated for an additional period.

Cells are typically harvested by centrifugation, disrupted by physical or chemical means, and the resulting crude extract retained for further purification.

Microbial cells employed in the expression of proteins can be disrupted by any convenient method, including freeze-thaw cycling, sonication, mechanical disruption, or use of cell lysing agents. Such methods are well known by the skill artisan.

## Transgenic Animals

The terms "transgenic animals" or "host animals" are used herein to designate animals that have their genome genetically and artificially manipulated so as to include one of the nucleic acids according to the invention. Preferred animals are non-human mammals and include those belonging to a genus selected from *Mus* (e.g. mice), *Rattus* (e.g. rats) and *Oryctogalus* (e.g. rabbits) which have their genome artificially and genetically altered by the insertion of a nucleic acid according to the invention. Suitable for use in the invention are non-human host mammals and animals comprising a recombinant vector of the invention or a CanIon gene disrupted by homologous recombination with a knock out vector.

The transgenic animals suitable for use in the invention all include within a plurality of their cells a cloned recombinant or synthetic DNA sequence, more specifically one of the purified or isolated nucleic acids comprising a CanIon coding sequence, a CanIon regulatory polynucleotide, a polynucleotide construct, or a DNA sequence encoding an antisense polynucleotide such as described in the present specification.

Generally, a transgenic animal suitable for use in the present invention comprises any one of the polynucleotides, the recombinant vectors and the cell hosts described in the present invention. More particularly, the transgenic animals of the present invention can comprise any of the polynucleotides described in the "Genomic Sequences Of tThe CanIon Gene" section, the "CanIon cDNA Sequences" section, the "Coding Regions" section, the "Polynucleotide constructs" section, the "Oligonucleotide Probes And Primers" section, the "Recombinant Vectors" section and the "Cell Hosts" section.

Further transgenic animals suitable for use in the invention contain in their somatic cells and/or in their germ line cells a polynucleotide comprising a biallelic marker selected from the group consisting of A1 to A18, and the complements thereof.

In a first preferred embodiment, these transgenic animals may be good experimental models in order to study the diverse pathologies related to cell differentiation, in particular concerning the transgenic animals within the genome of which has been inserted one or several copies of a polynucleotide encoding a native CanIon protein, or alternatively a mutant CanIon protein.

In a second preferred embodiment, these transgenic animals may express a desired polypeptide of interest under the control of the regulatory polynucleotides of the CanIon gene, leading to good yields in the synthesis of this protein of interest, and eventually a tissue specific expression of this protein of interest.

The design of the transgenic animals suitable for use in the invention may be made according to the conventional techniques well known to those skilled in the art. Additional details regarding the production of transgenic animals, and specifically transgenic mice, can be found, e.g., in US Patent Nos 4,873,191; 5,464,764; and 5,789,215.

Transgenic animals suitable for use in the present invention are produced by the application of procedures which result in an animal with a genome that has incorporated exogenous genetic material. The procedure involves obtaining the genetic material, or a portion thereof, which encodes either a CanIon coding sequence, a CanIon regulatory polynucleotide or a DNA sequence encoding a CanIon antisense polynucleotide such as described in the present specification.

A recombinant polynucleotide suitable for use in the invention is inserted into an embryonic or ES stem cell line. The insertion is preferably made using electroporation, such as described by Thomas et al. (1987). The cells subjected to electroporation are screened (e.g. by selection via selectable markers, by PCR or by Southern blot analysis) to find positive cells which have integrated the exogenous recombinant polynucleotide into their genome, preferably via an homologous recombination event. An illustrative positive-negative selection procedure that may be used is described by Mansour et al. (1988).

Then, the positive cells are isolated, cloned and injected into 3.5 days old blastocysts from mice, such as described by Bradley (1987). The blastocysts are then inserted into a female host animal and allowed to grow to term.

Alternatively, the positive ES cells are brought into contact with embryos at the 2.5 days old 8-16 cell stage (morulae) such as described by Wood et al. (1993) or by Nagy et al. (1993), the ES cells being internalized to colonize extensively the blastocyst including the cells which will give rise to the germ line.

The offspring of the female host are tested to determine which animals are transgenic e.g. include the inserted exogenous DNA sequence and which are wild type.

Suitable for use in the invention is a transgenic animal containing a nucleic acid, a recombinant expression vector or a recombinant host cell described herein.

**Recombinant Cell Lines Derived From The Transgenic Animals Of The Invention.**

Suitable for use in the present invention are recombinant host cells obtained from a transgenic animal described herein. In one embodiment the invention encompasses cells derived from non-human host mammals and animals comprising a recombinant vector of the invention or a CanIon gene disrupted by homologous recombination with a knock out vector.

Recombinant cell lines may be established in vitro from cells obtained from any tissue of a transgenic animal according to the invention, for example by transfection of primary cell cultures with vectors expressing *onc*-genes such as SV40 large T antigen, as described by Chou (1989) and Shay et aL (1991).

**Methods of screening for CanIon modulators and interacting compounds**

Suitable for use in the present invention are compounds that interact with, bind to, or activate or inhibit the expression or activity of CanIon polypeptides, channels, and polynucleotides. Such compounds may be any organic or inorganic compound, including, but not limited to, polypeptides, polynucleotides, lipids, carbohydrates, nucleotides, amino acids, or small molecule inhibitors or activators. As described elsewhere in this application, such compounds arc useful for the treatment or prevention of any of a large number of diseases or conditions. Preferably, inhibitors of CanIon activity or expression are used in the treatment or prevention of a psychiatric disorder such as schizophrenia or bipolar disorder.

**Methods of screening for CanIon channel modulators**

Compounds capable of binding to CanIon and compounds capable of modulating CanIon function have important applications in the treatment of disease. Voltage-gated ion channels are generally well established as drug targets because they are pharmacologically accessible, encoded by a variety of genes and usually operate as multimeric protein assemblies, resulting in a high degree of functional and anatomical specificity. Furthermore, because ion channel opening and closing involving the movement of charged voltage sensitive amino acids leads to changes in conformation states, ion channels allow the design of state dependent molecules that, for example, bind only to channels that are in conducting (activated) or non-conducting (inactivated) state.

In addition, numerous calcium channel modulators have been demonstrated to be efficacious in the treatment or prevention of numerous diseases and conditions. For example, calcium channel inhibitors have been shown to be effective against various cardiovascular diseases and conditions (e.g., angina, arrythmias, hypertension), as well as CNS and neuronal disorders (e.g., migraines, neurological effects of strokes, mania, neuroleptic-induced tardive dyskinesia, schizophrenia, bipolar disorder, pain, epilepsy, and others). In addition, calcium channel agonists have been shown to be effective for various applications, such as in reducing the duration of and otherwise attenuating the effects of local anesthesia. Antagonists and agonists of CanIon channels are similarly useful in the treatment or prevention of these and other diseases and conditions. For example, CanIon antagonists are useful in the treatment or prevention of schizophrenia and bipolar disorder.

Because voltage gated ion channels do not require agonist binding for activation, compounds are preferably screened against functional CanIon channels. Assays may include functional and radioligand binding approaches applied to cells (vesicles or membranes) expressing native or cloned channels, or to whole cell assays. Functional whole cell assays may use electrophysiological techniques, such as patch clamping. Assays may involve any voltage-gated channel type, preferably L, N, and T type channels. Kinetic ion flux through the channel may also be measured, e.g., using fluorescence, end-point radiotracer or cell viability techniques.

Assays may also make use of various toxins, venoms or compounds that bind to and open or close channels (Denyer et al., Drug Disc. Today 3(7): 323-332 (1998). In one embodiment, the present assays involve the use of any of the large number of known calcium channel agonists and antagonists, e.g., as positive or negative controls. Examples of suitable known calcium channel antagonists include phenylalkylamines (e.g., verapamil), benzothiazepines (e.g., diltiazem), and dihydropyridines (e.g., nifedipine); calcium channel agonists include FPL-64176 and BAY K 8644; sodium channel agonists include Batrachotoxin; and sodium channel antagonists include spiradoline, mexiletine, U-54494A ((+/-) -cis-3,4-Dichloro-N-methyl-N-[2-(1-pyrrolidinyl)-cyclohexyl]-benzamide). Such compounds may also be used as "lead" compounds, i.e. to serve as starting molecules for the design or discovery of derivative molecules that specifically bind to or modulate CanIon channels.

In preferred embodiments, assays of the invention comprise a method for the screening of a candidate substance comprises the following steps:

a) providing (i) a sample or a host cell containing a polypeptide comprising, consisting essentially of, or consisting of a CanIon protein or a fragment thereof, or (ii) a recombinant host cell expressing a polynucleotide encoding a polypeptide comprising, consisting essentially of, or consisting of a CanIon protein or a fragment thereof;
b) obtaining a candidate substance;
c) bringing into contact said host cell with said candidate substance;
d) determining the effect of said candidate substance on CanIon activity.

Determining the effect of the candidate substance on CanIon activity can be accomplished according to well known methods. Preferably, the effect of the candidate substance on CanIon activity is an agonist or an antagonist effect. Generally, a compound inhibits CanIon if the ability to transport ions (eg. Ca2+ or Na+) is decreased. A compound stimulates CanIon if the ability to transport ions is increased.

CanIon activity can be detected using any suitable means. In preferred examples, CanIon activity is detected by measuring a signaling event. While a signaling event may comprise any suitable change of a molecular characteristic or parameter of the cell, nonlimiting examples of a signaling event include changes in ion fluxes, such as changes in or generation of a Ca2+ or Na+, or K+ flux or enzyme activation.

In one aspect, ion flux can be monitored by measuring electrophysiological properties of the CanIon channel, using for example techniques for measuring whole cell current from a single cell or in membrane patches. In other examples, fluorescent or radioactive labels can be used to detect displacement of a known CanIon-binding compound, or to detect ion flux in a across a cell (eg labelled Ca2+ or Na+). An indicator for the physiological parameters of a cell can be used, such as a fluorescent indicator for cell viability. In other examples, change in the physical location of an indicator can be detected, such as the use of fluorescence activated cell sorting to identify exclusion or uptake of a physiological indicator.

The sample used in the assay of the invention contains a polypeptide or a host cell expressing a polypeptide comprising, consisting essentially of, or consisting of a CanIon protein or a fragment thereof, or (ii) a recombinant host cell expressing a polynucleotide encoding a polypeptide comprising, consisting essentially of, or consisting of a CanIon protein or a fragment thereof. Preferably, CanIon assays of the invention involve the use of a recombinant host cell expressing a functional CanIon polypeptide. Host cells may express or comprise a functional alpha subunit of CanIon channel, or may express or comprise one or more additional ion channel subunits, or a ion channel complex comprising CanIon. Preferably, a host cell is used which has low endogenous ion channel expression or have low background ion, particularly Ca2+ and/or Na+, conductance.

*Radioligand Binding*

In one aspect, a CanIon channel may be screened by identifying a high affinity ligand that binds to a site of interest of CanIon and preferably has a desired modulatory effect, and detecting the ability of a test compound to displace said labelled ligand. Lists of toxicological/pharmacological agents used in voltage gated (Ca2+, Na+ and K+) channels assays are provided in Denyer et al. (supra). This method is generally suitable for detecting compounds which bind to the same site, or are allosterically coupled to the site, as the labelled ligand, but does not provide information as to agonist or antagonist properties of the compound.

*Cell based fluorescence and radiotracer assays*

In another assay, CanIon function can be monitored by measuring changes in intracellular concentration of permeant ion using fluorescent-ion indicators or radiolabelled ions.

Typically, ion channels such as Na+ channels inactivate in miliseconds after voltage stimulation. Ca2+ channels exhibit no or a lesser degree of inactivation and can be opened by high K+ depolarization. In cell based fluorescence and radiotracer assays, the CanIon channel can be generally activated by a toxin or any test compound, or high K+ depolarization, such that the channel is opened for prolonged periods (up to many minutes).

In fluorescence based assays, fluorescent Ca2+ dyes are available for use (e.g., Fluo-3, Calcium green-1, Molecular Probes, OR, U.S.A). Ca2+ channels can be activated by depolarizing the membrane with an isotonic solution or Na+ channels with a toxin or other compound, and the resulting transient movement of fluoresence in the cell can be measured over 20 to 60s. Fluorescence measurement systems and devices are further described in Denyer et al. (supra). Radiotracers 22Na+ and 14C-guanidine are commonly used for Na+ channel analysis and 45Ca2+ for Ca2+ channel analysis. In a preferred embodiment described in Denyer et al. (supra), *Cytostar-T* scintillating microplates (Amersham International, U.K.) are used to perform high throughput CanIon cell based assays.

In further assays, Ca2+ function of an ion channel is monitored by measuring membrane potential with a membrane potential indicator. High electrical resistance of biological membranes allow small ionic currents across the plasma membrane to cause large changes in membrane potential. Voltage assays can thus be conveniently used to detect generic ion flux across membranes. Cell lines are generally chosen so that effects from endogenous ion channels are minimized. A range of dyes are available as membrane potential indicator dyes, divided into fast and slow response dyes, as well as FRET-based voltage sensor dyes. (Aurora Biosciences, CA, USA; reviewed in Gonzalez et al., Drug Disc. Today 4(9): 431:439 (1999)

*Cell viability*

In cell viability assays, ion channel activity and ion flux are directly related to cell viability. Both yeast and mammalian cell systems are available for testing an ion channel target. For example, a yeast system employing an ion-specific K+ uptake deficient Saccharomyces cerevisiae cell line has be used, in which a functional K+ channel of interest is expressed in the cell line thereby restoring K+ uptake and promoting cell survival. (Anderson et al., Symp. Soc. Exp. Biol. 48: 85-97 (1994)) Such an assay for Ca2+ or Na+ channels may be used to identify compounds capable of blocking CanIon function. Mammalian cell systems are also available, such as a Na+ channel assay using mammalian neuroblastoma cells with a colorimetric cell viability readout. Cells treated with a Na+ channel opener and a Na+/K+ pump inhibitor to promote a lethal intracellular Na+ overload. Treatment with a test compound capable of blocking the channel will improve cell viability, which compounds which enhance channel opening will further promote cell death. (Manger et al., Anal. Biochem. 214: 190-194 (1993).

*Eletrophysiology*

Electrophysiological voltage-clamping techniques involve the measurement of ionic current flowing through one or many channels. A single microelectrode to control the membrane voltage while the current flow is measured through a single cell or membrane patch. (Hamill, Pfugers Arch. 391, 85-100 (1981). Ionic current can thus be measured in the presence or absence of a test compound of interest. A large scale compound screening system has been designed (Neurosearch A/S, Glostrup, Denmark; Olesen et al., *Voltage gated ion channel modulators,* 7-8 December, Philadelphia PA, USA (1995); Denyer et al., supra).

**Methods for screening for substances interacting with a CanIon polypeptide**

For the purpose of the present invention, a ligand means a molecule, such as a protein, a peptide, an antibody or any synthetic chemical compound capable of binding to the CanIon protein or one of its fragments or variants or to modulate the expression of the polynucleotide coding for CanIon or a fragment or variant thereof.

In the ligand screening method according to the present invention, abiological sample or a defined molecule to be tested as a putative ligand of the CanIon protein is brought into contact with the corresponding purified CanIon protein, for example the corresponding purified recombinant CanIon protein produced by a recombinant cell host as described hereinbefore, in order to form a complex between this protein and the putative ligand molecule to be tested.

As an illustrative example, to study the interaction of a polypeptide comprising a polypeptide as shown as SEQ ID No 5, with drugs or small molecules, such as molecules generated through combinatorial chemistry approaches, the microdialysis coupled to HPLC method described by Wang et al. (1997) or the affinity capillary electrophoresis method described by Bush et al. (1997).

In further methods, peptides, drugs, fatty acids, lipoproteins, or small molecules which interact with a polypeptide comprising a polypeptide as shown as SEQ ID No 5, may be identified using assays such as the following. The molecule to be tested for binding is labeled with a detectable label, such as a fluorescent, radioactive, or enzymatic tag and placed in contact with immobilized polypeptide under conditions which permit specific binding to occur. After removal of non-specifically bound molecules, bound molecules are detected using appropriate means.

Another object of the present invention comprises methods and kits for the screening of candidate substances that interact with an ion channel polypeptide.

The present invention pertains to methods for screening substances of interest that interact with a CanIon protein or one fragment or variant thereof. By their capacity to bind covalently or non-covalently to a CanIon protein or to a fragment or variant thereof, these substances or molecules may be advantageously used both *in vitro* and *in vivo*.

*In vitro,* said interacting molecules may be used as detection means in order to identify the presence of a ion channel polypeptide in a sample, preferably a biological sample.

A method for the screening of a candidate substance comprises the following steps:

a) providing a polypeptide comprising a polypeptide as shown as SEQ ID No 5;
b) obtaining a candidate substance;
c) bringing into contact said polypeptide with said candidate substance;
d) detecting the complexes formed between said polypeptide and said candidate substance.

The invention further concerns a kit for the screening of a candidate substance interacting with the ion channel polypeptide, wherein said kit comprises :

a) an ion channel polypeptide comprising an amino acid sequence of SEQ ID No 5 of a peptide fragment or variant thereof;

b) optionally means useful to detect the complex formed between the ion channel polypeptide or a peptide fragment or a variant thereof and the candidate substance.

In a preferred embodiment of the kit described above, the detection means comprises a monoclonal or polyclonal antibodies directed against the ion channel polypeptide or a peptide fragment or a variant thereof.

Various candidate substances or molecules can be assayed for interaction with a CanIon polypeptide. These substances or molecules include, without being limited to, natural or synthetic organic compounds or molecules of biological origin such as polypeptides. When the candidate substance or molecule comprises a polypeptide, this polypeptide may be the resulting expression product of a phage clone belonging to a phage-based random peptide library, or alternatively the polypeptide may be the resulting expression product of a cDNA library cloned in a vector suitable for performing a two-hybrid screening assay.

The invention also pertains to kits useful for performing the hereinbefore described screening method. Preferably, such kits comprise an ion channel or a fragment or a variant thereof, and optionally means useful to detect the complex formed between the CanIon polypeptide or its fragment or variant and the candidate substance. In a preferred embodiment the detection means comprise a monoclonal or polyclonal antibodies directed against the corresponding ion channel polypeptide or a fragment or a variant thereof.

### A. Candidate ligands obtained from random peptide libraries

In a particular embodiment of the screening method, the putative ligand is the expression product of a DNA insert contained in a phage vector (Parmley and Smith, 1988). Specifically, random peptide phage libraries are used. The random DNA inserts encode for peptides of 8 to 20 amino acids in length (Oldenburg K.R. et al., 1992; Valadon P., et al., 1996; Lucas A.H., 1994; Westerink M.A.J., 1995; Felici F. et al., 1991). According to this particular embodiment, the recombinant phages expressing a protein that binds to the immobilized CanIon protein is retained and the complex formed between the CanIon protein and the recombinant phage may be subsequently immunoprecipitated by a polyclonal or a monoclonal antibody directed against the CanIon protein.

Once the ligand library in recombinant phages has been constructed, the phage population is brought into contact with the immobilized CanIon protein. Then the preparation of complexes is washed in order to remove the non-specifically bound recombinant phages. The phages that bind specifically to the CanIon protein are then eluted by a buffer (acid pH) or immunoprecipitated by the monoclonal antibody produced by the hybridoma anti-CanIon, and this phage population is subsequently amplified by an over-infection of bacteria (for example E. coli). The selection step may be repeated several times, preferably 2-4 times, in order to select the more specific recombinant phage clones. The last step comprises characterizing the peptide produced by the selected recombinant phage clones either by expression in infected bacteria and isolation, expressing the phage insert in another host-vector system, or sequencing the insert contained in the selected recombinant phages.

### B. Candidate ligands obtained by competition experiments.

Alternatively, peptides, drugs or small molecules which bind to the ion channel polypeptide comprising an amino acid sequence of SEQ ID No 5 or to a peptide fragment or variant thereof. In such assays, the ion channel polypeptide, or a fragment thereof, is immobilized to a surface, such as a plastic plate. Increasing amounts of the peptides, drugs or small molecules are placed in contact with the immobilized ion channel polypeptide, or a fragment thereof, in the presence of a detectable labeled known ion channel polypeptide ligand. For example, the ion channel polypeptide ligand may be detectably labeled with a fluorescent, radioactive, or enzymatic tag. The ability of the test molecule to bind the ion channel polypeptide or a fragment thereof, is determined by measuring the amount of detectably labeled known ligand bound in the presence of the test molecule. A decrease in the amount of known ligand bound to the ion channel polypeptide, or a fragment thereof, when the test molecule is present indicated that the test molecule is able to bind to the ion channel polypeptide, or a fragment thereof.

C. Candidate ligands obtained by affinity chromatography.

Proteins or other molecules interacting with the ion channel polypeptide comprising an amino acid sequence of SEQ ID No 5 or to a peptide fragment or variant thereof. The ion channel polypeptide or a fragment thereof, may be attached to the column using conventional techniques including chemical coupling to a suitable column matrix such as agarose, Affi Gel® , or other matrices familiar to those of skill in art. In some embodiments of this method, the affinity column contains chimeric proteins in which the GanIon protein, or a fragment thereof, is fused to glutathion S transferase (GST). A mixture of cellular proteins or pool of expressed proteins as described above is applied to the affinity column. Proteins or other molecules interacting with the ion channel polypeptide, or a fragment thereof, attached to the column can then be isolated and analyzed on 2-D electrophoresis gel as described in Ramunsen et al. (1997). Alternatively, the proteins retained on the affinity column can be purified by electrophoresis based methods and sequenced. The same method can be used to isolate antibodies, to screen phage display products, or to screen phage display human antibodies.

**D. Candidate ligands obtained by optical biosensor methods**

Proteins interacting with the ion channel polypeptide comprising an amino acid sequence of SEQ ID No 5 or to a peptide fragment or variant thereof can also be screened by using an Optical Biosensor as described in Edwards and Leatherbarrow (1997) and also in Szabo et al. (1995). This technique permits the detection of interactions between molecules in real time, without the need of labeled molecules. This technique is based on the surface plasmon resonance (SPR) phenomenon. Briefly, the candidate ligand molecule to be tested is attached to a surface (such as a carboxymethyl dextran matrix). A light beam is directed towards the side of the surface that does not contain the sample to be tested and is reflected by said surface. The SPR phenomenon causes a decrease in the intensity of the reflected light with a specific association of angle and wavelength. The binding of candidate ligand molecules cause a change in the refraction index on the surface, which change is detected as a change in the SPR signal. For screening of candidate ligand molecules or substances that are able to interact with the ion channel polypeptide or a fragment thereof, the ion channel polypeptide, or a fragment thereof, is immobilized onto a surface. This surface comprises one side of a cell through which flows the candidate molecule to be assayed. The binding of the candidate molecule on the ion channel polypeptide or a fragment thereof, is detected as a change of the SPR signal. The candidate molecules tested may be proteins, peptides, carbohydrates, lipids, or small molecules generated by combinatorial chemistry. This technique may also be performed by immobilizing eukaryotic or prokaryotic cells or lipid vesicles exhibiting an endogenous or a recombinantly expressed ion channel polypeptide at their surface.

The main advantage of the method is that it allows the determination of the association rate between the ion channel polypeptide and molecules interacting with the ion channel polypeptide. It is thus possible to select specifically ligand molecules interacting with the ion channel polypeptide or a fragment thereof, through strong or conversely weak association constants.

**E. Candidate ligands obtained through a two-hybrid screening assay.**

The yeast two-hybrid system is designed to study protein-protein interaction *in vivo* (fields and Song, 1989), and relies upon the fusion of a bait protein to the DNA binding domain of the yeast Gal4 protein. This technique is also described in the US Patent Nos. US 5,667,973 and 5,283,173 (Fields et al.).

The general procedure of library screening by the two-hybrid assay may be performed as described by Harper ct al. (1993), Cho et al. (1998), or Fromont-Racine et al. (1997).

The bait protein or polypeptide comprises, consists essentially of, or consists of an ion channel polypeptide comprising an amino acid sequence of SEQ ID No 5 or to a peptide fragment or variant thereof.

More precisely, the nucleotide sequence encoding the ion channel polypeptide or a fragment or variant thereof is fused to a polynucleotide encoding the DNA binding domain of the GAL4 protein, the fused nucleotide sequence being insertion in a suitable expression vector, for example pAS2 or pM3.

Then, a human cDNA library is constructed in a specially designed vector, such that the human cDNA insert is fused to a nucleotide sequence in the vector that encodes the transcriptional domain of the GAL4 protein. Preferably, the vector used is the pACT vector. The polypeptides encoded by the nucleotide inserts of the cDNA library are termed '"prey" polypeptides.

A third vector contains a detectable marker gene, such as beta galactosidase gene or CAT gene that is placed under the control of a regulation sequence that is responsive to the binding of a complete Gal4 protein containing both the transcriptional activation domain and the DNA binding For example, the vector pG5EC may be used.

Two different yeast strains are also used. As an illustrative but non limiting example the two different yeast strains may be the followings :

- Y190, the phenotype of which is (*MATa, Leu2-3, 112 ura3-12, trp1-901, his3-D200, ade2-101, gal4Dgal180D URA3 GAL-LacZ, LYS GAL-HIS3, cyh')*
- Y187, the phenotype of which is *(MATa gal4 gal80 his3 trp1-901 ade2-101 ura3-52 leu2-3, -112 URA3 GAL-lacZmet$^-$),* which is the opposite mating type of Y190.

Briefly, 20 μg of pAS2/CanIon and 20 μg ofpACT-cDNA library are co-transformed into yeast strain Y190. The transformants are selected for growth on minimal media lacking histidine, leucine and tryptophan, but containing the histidine synthesis inhibitor 3-AT (50 mM). Positive colonies are screened for beta galactosidase by filter lift assay. The double positive colonies (*His*$^+$, *beta-gal*$^+$)are then grown on plates Jacking histidine, leucine, but containing tryptophan and cycloheximide (10 mg/ml) to select for loss of pAS2/CanIon plasmids bu retention of pACT-cDNA library plasmids. The resulting Y190 strains are mated with Y187 strains expressing CanIon or non-related control proteins; such as cyclophilin B, lamin, or SNF1, as *Gal4* fusions as described by Harper et al. (1993) and by Bram et al. (Bram RJ et al., 1993), and screened for beta galactosidase by filter lift assay. Yeast clones that are *beta gal-* after mating with the control *Gal4* fusions are considered false positives.

In another embodiment of the two-hybrid method according to the invention, interaction between the CanIon or a fragment or variant thereof with cellular proteins may be assessed using the Matchmaker Two Hybrid System 2 (Catalog No. K1604-1, Clontech). As described in the manual accompanying the Matchmaker Two Hybrid System 2 (Catalog No. K1604-1, Clontech), nucleic acids encoding the CanIon protein or a portion thereof, are inserted into an expression vector such that they are in frame with DNA encoding the DNA binding domain of the yeast transcriptional activator GAL4. A desired cDNA, preferably human cDNA, is inserted into a second expression vector such that they are in frame with DNA encoding the activation domain of GAL4. The two expression plasmids are transformed into yeast and the yeast are plated on selection medium which selects for expression of selectable markers on each of the expression vectors as well as GAL4 dependent expression of the HIS3 gene. Transformants capable of growing on medium lacking histidine are screened for GAL4 dependent lacZ expression. Those cells which are positive in both the histidine selection and the lacZ assay contain interaction between CanIon and the protein or peptide encoded by the initially selected cDNA insert.

## Method For Screening Substances Interacting With The Regulatory Sequences Of The CanIon Gene.

The present invention also concerns a method for screening substances or molecules that are able to interact with the regulatory sequences of the CanIon gene, such as for example promoter or enhancer sequences.

Nucleic acids encoding proteins which are able to interact with the regulatory sequences of the CanIon gene, more particularly a nucleotide sequence selected from the group consisting of the polynucleotides of the 5' and 3' regulatory region or a fragment or variant thereof, and preferably a variant comprising one of the biallelic markers suitable for use in the invention, may be identified by using a one-hybrid system, such as that described in the booklet enclosed in the Matchmaker One-Hybrid System kit from Clontech (Catalog Ref. n° K1603-1). Briefly, the target nucleotide sequence is cloned upstream of a selectable reporter sequence and the resulting DNA construct is integrated in the yeast genome *(Saccharomyces cerevisiae).* The yeast cells containing the reporter sequence in their genome are then transformed with a library comprising fusion molecules between cDNAs encoding candidate proteins for binding onto the regulatory sequences of the CanIon gene and sequences encoding the activator domain of a yeast transcription factor such as GAL4. The recombinant yeast cells are plated in a culture broth for selecting cells expressing the reporter sequence. The recombinant yeast cells thus selected contain a fusion protein that is able to bind onto the target regulatory sequence of the CanIon gene. Then, the cDNAs encoding the fusion proteins are sequenced and may be cloned into expression or transcription vectors *in vitro*. The binding of the encoded polypeptides to the target regulatory sequences of the CanIon gene may be confirmed by techniques familiar to the one skilled in the art, such as gel retardation assays or DNAse protection assays.

Gel retardation assays may also be performed independently in order to screen candidate molecules that are able to interact with the regulatory sequences of the CanIon gene, such as described by Fried and Crothers (1981), Garner and Revzin (1981) and Dent and Latchman (1993). These techniques are based on the principle according to which a DNA fragment which is bound to a protein migrates slower than the same unbound DNA fragment. Briefly, the target nucleotide sequence is labeled. Then the labeled target nucleotide sequence is brought into contact with either a total nuclear extract from cells containing transcription factors, or with different candidate molecules to be tested. The interaction between the target regulatory sequence of the CanIon gene and the candidate molecule or the transcription factor is detected after gel or capillary electrophoresis through a retardation in the migration.

**Method For Screening Ligands That Modulate The Expression Of The CanIon Gene.**

Suitable for use in the invention is a method for screening molecules that modulate the expression of the CanIon protein. Such a screening method comprises the steps of:

a) cultivating a prokaryotic or an eukaryotic cell that has been transfected with a nucleotide sequence encoding the CanIon protein or a variant or a fragment thereof, placed under the control of its own promoter,
b) bringing into contact the cultivated cell with a molecule to be tested;
c) quantifying the expression of the CanIon protein or a variant or a fragment thereof.

In an embodiment, the nucleotide sequence encoding the CanIon protein or a variant or a fragment thereof comprises an allele of at least one of the biallelic markers A12 or A16, and the complements thereof.
Using DNA recombination techniques well known by the one skill in the art, the CanIon protein encoding DNA sequence is inserted into an expression vector, downstream from its promoter sequence. As an illustrative example, the promoter sequence of the CanIon gene is contained in the nucleic acid of the 5' regulatory region.
The quantification of the expression of the CanIon protein may be realized either at the mRNA level or at the protein level. In the latter case, polyclonal or monoclonal antibodies may be used to quantify the amounts of the CanIon protein that have been produced, for example in an ELISA or a RIA assay.
In a preferred embodiment, the quantification of the CanIon mRNA is realized by a quantitative PCR amplification of the cDNA obtained by a reverse transcription of the total mRNA of the cultivated CanIon -transfected host cell, using a pair of primers specific for CanIon.
Suitable for use in the present invention is a method for screening substances or molecules that are able to increase or decrease the level of expression of the CanIon gene. Such a method may allow one skilled in the art to select substances exerting a regulating effect on the expression level of the CanIon gene and which may be useful as active ingredients included in pharmaceutical compositions for treating patients suffering from any of the herein-described diseases.
Thus, suitable for use in the present invention is a method for screening of a candidate substance or molecule that modulated the expression of the CanIon gene, this method comprises the following steps:

- providing a recombinant cell host containing a nucleic acid, wherein said nucleic acid comprises a nucleotide sequence of the 5' regulatory region or a biologically active fragment or variant thereof located upstream a polynucleotide encoding a detectable protein;
- obtaining a candidate substance; and
- determining the ability of the candidate substance to modulate the expression levels of the polynucleotide encoding the detectable protein.

In a further embodiment, the nucleic acid comprising the nucleotide sequence of the 5' regulatory region or a biologically active fragment or variant thereof also includes a 5'UTR region of the CanIon cDNA of SEQ ID No 4, or one of its biologically active fragments or variants thereof.
Among the preferred polynucleotides encoding a detectable protein, there may be cited polynucleotides encoding beta galactosidase, green fluorescent protein (GFP) and chloramphenicol acetyl transferase (CAT).
Suitable for use in the present invention are kits useful for performing the herein described screening method. Preferably, such kits comprise a recombinant vector that allows the expression of a nucleotide sequence of the 5' regulatory region or a biologically active fragment or variant thereof located upstream and operably linked to a polynucleotide encoding a detectable protein or the CanIon protein or a fragment or a variant thereof.
In another method for the screening of a candidate substance or molecule that modulates the expression of the CanIon gene, the method comprises the following steps:

a) providing a recombinant host cell containing a nucleic acid, wherein said nucleic acid comprises a 5'UTR sequence of the CanIon cDNA of SEQ ID No 4, or one of its biologically active fragments or variants, the 5'UTR sequence or its biologically active fragment or variant being operably linked to a polynucleotide encoding a detectable protein;
b) obtaining a candidate substance; and
c) determining the ability of the candidate substance to modulate the expression levels of the polynucleotide encoding the detectable protein.

In a specific embodiment of the above screening method, the nucleic acid that comprises a nucleotide sequence selected from the group consisting of the 5'UTR sequence of the CanIon cDNA of SEQ ID No 4 or one of its biologically active fragments or variants, includes a promoter sequence which is endogenous with respect to the CanIon 5'UTR sequence.

In another specific embodiment of the above screening method, the nucleic acid that comprises a nucleotide sequence selected from the group consisting of the 5'UTR sequence of the CanIon cDNA of SEQ ID No 4 or one of its biologically active fragments or variants, includes a promoter sequence which is exogenous with respect to the CanIon 5'UTR sequence defined therein.

In a further preferred embodiment, the nucleic acid comprising the 5'-UTR sequence of the CanIon cDNA or SEQ ID No 4 or the biologically active fragments thereof includes a biallelic marker selected from the group consisting of A12 or A16 or the complements thereof.

Suitable for use in the invention is a kit for the screening of a candidate substance modulating the expression of the CanIon gene, wherein said kit comprises a recombinant vector that comprises a nucleic acid including a 5'UTR sequence of the CanIon cDNA of SEQ ID No 4, or one of their biologically active fragments or variants, the 5'UTR sequence or its biologically active fragment or variant being operably linked to a polynucleotide encoding a detectable protein.

Expression levels and patterns of CanIon may be analyzed by solution hybridization with long probes as described in International Patent Application No. WO 97/05277. Briefly, the CanIon cDNA or the CanIon genomic DNA described above, or fragments thereof, is inserted at a cloning site immediately downstream of a bacteriophage (T3, T7 or SP6) RNA polymerase promoter to produce antisense RNA. Preferably, the . CanIon insert comprises at least 100 or more consecutive nucleotides of the genomic DNA sequence or the cDNA sequences. The plasmid is linearized and transcribed in the presence of ribonucleotides comprising modified ribonucleotides (i.e. biotin-UTP and DIG-UTP). An excess of this doubly labeled RNA is hybridized in solution with mRNA isolated from cells or tissues of interest. The hybridization is performed under standard stringent conditions (40-50°C for 16 hours in an 80% formamide, 0. 4 M NaCl buffer, pH 7-8). The unhybridized probe is removed by digestion with ribonucleases specific for single-stranded RNA (i.e. RNases CL3, T1, Phy M, U2 or A). The presence of the biotin-UTP modification enables capture of the hybrid on a microtitration plate coated with streptavidin. The presence of the DIG modification enables the hybrid to be detected and quantified by ELISA using an anti-DIG antibody coupled to alkaline phosphatase.

Quantitative analysis of CanIon gene expression may also be performed using arrays. As used herein, the term array means a one dimensional, two dimensional, or multidimensional arrangement of a plurality of nucleic acids of sufficient length to permit specific detection of expression of mRNAs capable of hybridizing thereto. For example, the arrays may contain a plurality of nucleic acids derived from genes whose expression levels are to be assessed. The arrays may include the CanIon genomic DNA, the CanIon cDNA sequences or the sequences complementary thereto or fragments thereof, particularly those comprising at least one of the biallelic markers according the present invention, preferably at least one of the biallelic markers A1 to A17. Preferably, the fragments are at least 15 nucleotides in length. In other embodiments, the fragments are at least 25 nucleotides in length. In some embodiments, the fragments are at least 50 nucleotides in length. More preferably, the fragments are at least 100 nucleotides in length. In another preferred embodiment, the fragments are more than 100 nucleotides in length. In some embodiments the fragments may be more than 500 nucleotides in length.

For example, quantitative analysis of CanIon gene expression may be performed with a complementary DNA microarray as described by Schena et al. (1995 and 1996). Full length CanIon cDNAs or fragments thereof are amplified by PCR and arrayed from a 96-well microtiter plate onto silylated microscope slides using high-speed robotics. Printed arrays are incubated in a humid chamber to allow rehydration of the array elements and rinsed, once in 0.2% SDS for 1 min, twice in water for 1 min and once for 5 min in sodium borohydride solution. The arrays are submerged in water for 2 min at 95°C, transferred into 0.2% SDS for 1 min, rinsed twice with water, air dried and stored in the dark at 25°C.

Cell or tissue mRNA is isolated or commercially obtained and probes are prepared by a single round of reverse transcription. Probes are hybridized to 1 $cm^2$ microarrays under a 14 x 14 mm glass coverslip for 6-12 hours at 60°C. Arrays are washed for 5 min at 25°C in low stringency wash buffer (1 x SSC/0.2% SDS), then for 10 min at room temperature in high stringency wash buffer (0.1 x SSC/0.2% SDS). Arrays are scanned in 0.1 x SSC using a fluorescence laser scanning device fitted with a custom filter set. Accurate differential expression measurements are obtained by taking the average of the ratios of two independent hybridizations.

Quantitative analysis of CanIon gene expression may also be performed with full length CanIon cDNAs or fragments thereof in complementary DNA arrays as described by Pietu et al. (1996). The full length CanIon cDNA or fragments thereof is PCR amplified and spotted on membranes. Then, mRNAs originating from various tissues or cells are labeled with radioactive nucleotides. After hybridization and washing in controlled conditions, the hybridized mRNAs are detected by phospho-imaging or autoradiography. Duplicate experiments are performed and a quantitative analysis of differentially expressed mRNAs is then performed.

Alternatively, expression analysis using the CanIon genomic DNA, the CanIon cDNA, or fragments thereof can be done through high density nucleotide arrays as described by Lockhart et al. (1996) and Sosnowsky et al. (1997). Oligonucleotides of 15-50 nucleotides from the sequences of the CanIon genomic DNA or the CanIon cDNA sequences, particularly those comprising at least one of biallelic markers according the present invention, preferably at least one biallelic marker selected from the group consisting of A1 to A 17, or the sequences complementary thereto, are synthesized directly on the chip (Lockhart et al., supra) or synthesized and then addressed to the chip (Sosnowski et al., supra). Preferably, the oligonucleotides are about 20 nucleotides in length.

CanIon cDNA probes labeled with an appropriate compound, such as biotin, digoxigenin or fluorescent dye, are synthesized from the appropriate mRNA population and then randomly fragmented to an average size of 50 to 100 nucleotides. The probes are then hybridized to the chip. After washing as described in Lockhart et al., supra and application of different electric fields (Sosnowsky et al., 1997), the dyes or labeling compounds are detected and quantified. Duplicate hybridizations are performed. Comparative analysis of the intensity of the signal originating from cDNA probes on the same target oligonucleotide in different cDNA samples indicates a differential expression of CanIon mRNA.

## Methods For Inhibiting The Expression Of A CanIon Gene

Other therapeutic compositions according to the present invention comprise advantageously an oligonucleotide fragment of the nucleic sequence of CanIon as an antisense tool or a triple helix tool that inhibits the expression of the corresponding CanIon gene. A preferred fragment of the nucleic sequence of CanIon comprises an allele of at least one of the biallelic markers A1 to A17.

### Antisense Approach

Preferred methods for using antisense polynucleotides according to the present invention are the procedures described by Sczakiel et al. (1995).

Preferably, the antisense tools are chosen among polynucleotides (15-200 bp long) that are complementary to the 5'end of the CanIon mRNA. In another embodiment, a combination of different antisense polynucleotides complementary to different parts of the desired targeted gene are used.

Preferred antisense polynucleotides according to the present invention are complementary to a sequence of the mRNAs of CanIon that contains either the translation initiation codon ATG or a splicing donor or acceptor site.

The antisense nucleic acids should have a length and melting temperature sufficient to permit formation of an intracellular duplex having sufficient stability to inhibit the expression of the CanIon mRNA in the duplex. Strategies for designing antisense nucleic acids suitable for use in gene therapy are disclosed in Green et aL (1986) and Izant and Weintraub (1984).

In some strategies, antisense molecules are obtained by reversing the orientation of the CanIon coding region with respect to a promoter so as to transcribe the opposite strand from that which is normally transcribed in the cell. The antisense molecules may be transcribed using in vitro transcription systems such as those which employ T7 or SP6 polymerase to generate the transcript. Another approach involves transcription of CanIon antisense nucleic acids in vivo by operably linking DNA containing the antisense sequence to a promoter in a suitable expression vector.

Alternatively, suitable antisense strategies are those described by Rossi et al. (1991), in International Application Nos. WO 94/23026, WO 95/04141, WO 92/18522 and in European Patent Application No. EP 0 572 287 A2

An alternative to the antisense technology that is used according to the present invention comprises using ribozymes that will bind to a target sequence via their complementary polynucleotide tail and that will cleave the corresponding RNA by hydrolyzing its target site (namely "hammerhead ribozymes"). Briefly, the simplified cycle of a hammerhead ribozyme comprises (1) sequence specific binding to the target RNA via complementary antisense sequences; (2) site-specific hydrolysis of the cleavable motif of the target strand; and (3) release of cleavage products, which gives rise to another catalytic cycle. Indeed, the use of long-chain antisense polynucleotide (at least 30 bases long) or ribozymes with long antisense arms are advantageous. A preferred delivery system for antisense ribozyme is achieved by covalently linking these antisense ribozymes to lipophilic groups or to use liposomes as a convenient vector. Preferred antisense ribozymes according to the present invention are prepared as described by Sczakiel et al.(1995).

### Triple Helix Approach

The CanIon genomic DNA may also be used to inhibit the expression of the CanIon gene based on intracellular triple helix formation.

Triple helix oligonucleotides are used to inhibit transcription from a genome. They are particularly useful for studying alterations in cell activity when it is associated with a particular gene.

Similarly, a portion of the CanIon genomic DNA can be used to study the effect of inhibiting CanIon transcription within a cell. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the CanIon genomic DNA are contemplated within the scope of this invention.

To carry out gene therapy strategies using the triple helix approach, the sequences of the CanIon genomic DNA are first scanned to identify 10-mer to 20-mer homopyrimidine or homopurine stretches which could be used in triple-helix based strategies for inhibiting CanIon expression. Following identification of candidate homopyrimidine or homopurine stretches, their efficiency in inhibiting CanIon expression is assessed by introducing varying amounts of oligonucleotides containing the candidate sequences into tissue culture cells which express the CanIon gene.

The oligonucleotides can be introduced into the cells using a variety of methods known to those skilled in the art, including but not limited to calcium phosphate precipitation, DEAE-Dextran, electroporation, liposome-mediated transfection or native uptake.

Treated cells are monitored for altered cell function or reduced CanIon expression using techniques such as Northern blotting, RNase protection assays, or PCR based strategies to monitor the transcription levels of the CanIon gene in cells which have been treated with the oligonucleotide.

The oligonucleotides which are effective in inhibiting gene expression in tissue culture cells may then be introduced in vivo using the techniques described above in the antisense approach at a dosage calculated based on the in vitro results, as described in antisense approach.

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix. For information on the generation of oligonucleotides suitable for triple helix formation see Griffin et al. (1989).

## Pharmaceutical Compositions And Formulations

### CanIon-modulating Compounds

Using the methods disclosed herein, CanIon agonist or antagonist compounds that selectively modulate CanIon activity *in vitro* and *in vivo* may be identified. The invention thus encompasses methods of treating schizophrenia, bipolar disorder, or any of the other herein-described diseases or conditions in a patient comprising administering an effective amount of a CanIon-modulating compound. Preferably, said compound is a selective CanIon modulating compound. The compounds identified by the process of the invention include, for example, antibodies having binding specificity for a human CanIon polypeptide. It is also expected that homologues of CanIon may be useful for modulating CanIon -mediated activity and the related physiological condition associated with schizophrenia or bipolar disorder. Generally, it is further expected that assay methods of the present invention based on the role of CanIon in central nervous system disorder may be used to identify compounds capable of intervening in the assay cascade of the invention. In a preferred embodiment, a patient suffering from schizophrenia or bipolar disorder is treated by administering to the patient a pharmaceutical composition comprising a therapeutically effective amount of a CanIon antagonist.

#### Indications

While CanIon is linked to a genomic region associated with schizophrenia and bipolar disorder, indications involving CanIon may include various central nervous system disorders. Nervous system disorders are expected to have complex genetic bases and often share certain symptoms. In particular, as described herein, indications may include schizophrenia and other psychotic disorders, mood disorders, autism, substance dependence and alcoholism, epilepsy, pain disorders, mental retardation, and other psychiatric diseases including cognitive, anxiety, eating, impulse-control, and personality disorders, as defined with the Diagnosis and Statistical Manual of Mental Disorders fourth edition (DSM-IV) classification. In addition, numerous cardiovascular disorders including angina, hypertension, and arrythmias may also be treated using CanIon modulators, preferably antagonists.

#### Pharmaceutical Formulations and Routes of Administration

The compounds identified using the methods of the present invention can be administered to a mammal, including a human patient, alone or in pharmaceutical compositions where they are mixed with suitable carriers or excipient(s) at therapeutically effective doses to treat or ameliorate schizophrenia or bipolar disorder related disorders. A therapeutically effective dose further refers to that amount of the compound sufficient to result in amelioration of symptoms as determined by the methods described herein. Preferably, a therapeutically effective dosage is suitable for continued periodic use or administration. Techniques for formulation and administration of the compounds of the instant application may be found in "Remington's Pharmaceutical Sciences," Mack Publishing Co., Easton, PA, latest edition.

Routes of Administration

Suitable routes of administration include oral, rectal, transmucosal, or intestinal administration, parenteral delivery, including intramuscular, subcutaneous, intramedullary injections, as well as intrathecal, direct intraventricular, intravenous, intraperitoneal, intranasal or intraocular injections. A particularly useful method of administering compounds for treating central nervous system disease involves surgical implantation of a device for delivering the compound over an extended period of time. Sustained release formulations of the invented medicaments particularly are contemplated.

Composition/Formulation

Pharmaceutical compositions and medicaments for use in accordance with the present invention may be formulated in a conventional manner using one or more physiologically acceptable carriers comprising excipients and auxiliaries. Proper formulation is dependent upon the route of administration chosen.

For injection, the agents of the invention may be formulated in aqueous solutions, preferably in physiologically compatible buffers such as Hanks's solution, Ringer's solution, or physiological saline buffer such as a phosphate or bicarbonate buffer. For transmucosal administration, penetrants appropriate to the barrier to be permeated are used in the formulation. Such penetrants are generally known in the art.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration,the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable gaseous propellant, *e.g.*, carbon dioxide. In the case of a pressurized aerosol the dosage unit may be determined by providing a valve to deliver a metered amount. Capsules and cartridges of, *e.g.*, gelatin, for use in an inhaler or insufflator, may be formulated containing a powder mix of the compound and a suitable powder base such as lactose or starch.

The compounds may be formulated for parenteral administration by injection, *e.g.*, by bolus injection or continuous infusion. Formulations for injection may be presented in unit dosage form, *e.g.*, in ampoules or in multi-dose containers, with an added preservative. The compositions may take such forms as suspensions, solutions or emulsions in aqueous vehicles, and may contain formulatory agents such as suspending, stabilizing and/or dispersing agents.

Pharmaceutical formulations for parenteral administration include aqueous solutions of the active compounds in water-soluble form. Aqueous suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder or lyophilized form for constitution with a suitable vehicle, such as sterile pyrogen-free water, before use.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Additionally, the compounds may be delivered using a sustained-release system, such as semipermeable matrices of solid hydrophobic polymers containing the therapeutic agent. Various sustained release materials have been established and are well known by those skilled in the art. Sustained-release capsules may, depending on their chemical nature, release the compounds for a few weeks up to over 100 days.

Depending on the chemical nature and the biological stability of the therapeutic reagent, additional strategies for protein stabilization may be employed.

The pharmaceutical compositions may also comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Effective Dosage.

Pharmaceutical compositions suitable for use in the present invention include compositions wherein the active ingredients are contained in an effective amount to achieve their intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used in the method of the invention, the therapeutically effective dose can be estimated initially from cell culture assays, and a dose can be formulated in animal models. Such information can be used to more accurately determine useful doses in humans.

A therapeutically effective dose refers to that amount of the compound that results in amelioration of symptoms in a patient. Toxicity and therapeutic efficacy of such compounds can be determined by standard pharmaceutical procedures in cell cultures or experimental animals, e.g., for determining the LD50, (the dose lethal to 50% of the test population) and the ED50 (the dose therapeutically effective in 50% of the population). The dose ratio between toxic and therapeutic effects is the therapeutic index and it can be expressed as the ratio between LD50 and ED50. Compounds which exhibit high therapeutic indices are preferred.

The data obtained from these cell culture assays and animal studies can be used in formulating a range of dosage for use in human. The dosage of such compounds lies preferably within a range of circulating concentrations that include the ED50, with little or no toxicity. The dosage may vary within this range depending upon the dosage form employed and the route of administration utilized. The exact formulation, route of administration and dosage can be chosen by the individual physician in view of the patient's condition. *(See, e.g.,* Fing1 et al., 1975, in "The Pharmacological Basis of Therapeutics", Ch. 1).

## EXAMPLES

### Example 1

### Identification Of Biallelic Markers - DNA Extraction

Donors were unrelated and healthy. They presented a sufficient diversity for being representative of a French heterogeneous population. The DNA from 100 individuals was extracted and tested for the detection of the biallelic markers.

30 ml of peripheral venous blood were taken from each donor in the presence of EDTA. Cells (pellet) were collected after centrifugation for 10 minutes at 2000 rpm. Red cells were lysed by a lysis solution (50 ml final volume: 10 mM Tris pH7.6; 5 mM $MgCl_2$; 10 mM NaCl). The solution was centrifuged (10 minutes, 2000 rpm) as many times as necessary to eliminate the residual red cells present in the supernatant, after resuspension of the pellet in the lysis solution.

The pellet of white cells was lysed overnight at 42°C with 3.7 ml of lysis solution composed of:

- 3 ml TE 10-2 (Tris-HCl 10 mM, EDTA 2 mM) / NaCl 0 4 M
- 200 µl SDS 10%
- 500 µl K-proteinase (2 mg K-proteinase in TE 10-2 /NaCl 0.4 M).

For the extraction of proteins, 1 ml saturated NaCl (6M) (1/3.5 v/v) was added. After vigorous agitation, the solution was centrifuged for 20 minutes at 10000 rpm.

For the precipitation of DNA, 2 to 3 volumes of 100% ethanol were added to the previous supernatant, and the solution was centrifuged for 30 minutes at 2000 rpm. The DNA solution was rinsed three times with 70% ethanol to eliminate salts, and centrifuged for 20 minutes at 2000 rpm. The pellet was dried at 37°C, and resuspended in 1 ml TE 10-1 or 1 ml water. The DNA concentration was evaluated by measuring the OD at 260 nm (1 unit OD = 50 µg/ml DNA).

To determine the presence of proteins in the DNA solution, the OD 260 / OD 280 ratio was determined. Only DNA preparations having a OD 260 / OD 280 ratio between 1.8 and 2 were used in the subsequent examples described below.

The pool was constituted by mixing equivalent quantities of DNA from each individual.

### Example 2

### Identification Of Biallelic Markers: Amplification Of Genomic DNA By PCR

The amplification of specific genomic sequences of the DNA samples of example 1 was carried out on the pool of DNA obtained previously. In addition, 50 individual samples were similarly amplified.

PCR assays were performed using the following protocol:

| | |
|---|---|
| Final volume | 25 μl |
| DNA | 2ng/μl |
| $MgCl_2$ | 2 mM |
| dNTP (each) | 200 μM |
| primer (each) | 2.9 ng/μl |
| Ampli Taq Gold DNA polymerase | 0.05 unit/μl |
| PCR buffer (10x = 0.1 M TrisHCl pH8.3 0.5M KCl) | 1x |

Each pair of first primers was designed using the sequence information of the CanIon gene disclosed herein and the OSP software (Hillier & Green, 1991). This first pair of primers was about 20 nucleotides in length and had the sequences disclosed in Table 1 in the columns labeled PU and RP.

**Table 1**

| **Amplicon** | **Position range of the amplicon in SEQ ID 1** | | **Primer name** | **Position range of amplification primer in SEQ ID No 1** | | **Primer name** | **Complementary position range of amplification primer SEQ ID No 1** | |
|---|---|---|---|---|---|---|---|---|
| 99-62626 | 12343 | 12810 | B1 | 12343 | 12363 | C1 | 12793 | 12810 |
| 99-62632 | 13814 | 14296 | B2 | 13814 | 13832 | C2 | 14279 | 14296 |
| 99-62633 | 24863 | 25396 | B3 | 24863 | 24881 | C3 | 25378 | 25396 |
| 99-62611 | 69198 | 69650 | B4 | 69198 | 69218 | C4 | 69632 | 69650 |
| 99-62605 | 73005 | 73483 | B5 | 73005 | 73022 | C5 | 73466 | 73483 |
| 99-62635 | 79808 | 80334 | B6 | 79808 | 79826 | C6 | 80314 | 80334 |
| **Amplicon** | **Position range of the amplicon in SEQ ID 2** | | **rimer name** | **Position range of amplification primer in SEQ ID No 2** | | **Primer name** | **Complementary position range of amplification primer in SEQ ID No 2** | |
| 99-79335 | 51031 | 51559 | B7 | 51031 | 51051 | C7 | 51539 | 51559 |
| 99-79336 | 60925 | 61374 | B8 | 60925 | 60945 | C8 | 61354 | 61374 |
| 99-79338 | 80271 | 80720 | B9 | 80271 | 80290 | C9 | 80700 | 80720 |
| 99-79339 | 91037 | 91486 | B10 | 91037 | 91056 | C10 | 91466 | 91486 |
| 99-79314 | 100285 | 100784 | B11 | 100285 | 100305 | C11 | 100764 | 100784 |
| 99-79316 | 106568 | 107020 | B12 | 106568 | 106585 | C12 | 107000 | 107020 |
| 99-79322 | 165864 | 166401 | B13 | 165864 | 165884 | C13 | 166381 | 166401 |
| 99-79306 | 235713 | 236210 | B14 | 235713 | 235732 | C14 | 236190 | 236210 |
| **Amplicon** | **Position range of the amplicon in SEQ ID 3** | | **Primer name** | **Position range of amplification primer in SEQ ID No 3** | | **Primer name** | **Complementary position range of amplification primer in** SEQ ID No 3 | |
| 99-79310 | 31618 | 32100 | B15 | 31618 | 31635 | C15 | 32080 | 32100 |
| 99-79311 | 42324 | 42723 | B16 | 42324 | 42344 | C16 | 42704 | 42723 |

(continued)

| Amplicon | | | Primer name | Position range of amplification primer in SEQ ID No 6 | | Primer name | Complementary position range of amplification primer in SEQ ID No 6 | |
|---|---|---|---|---|---|---|---|---|
| 99-62617 | | | B17 | | 20 | C17 | 435 | 453 |

Preferably, the primers contained a common oligonucleotide tail upstream of the specific bases targeted for amplification which was useful for sequencing.

Primers PU contain the following additional PU 5' sequence: TGTAAAACGACGGCCAGT; primers RP contain the following RP 5' sequence: CAGGAAACAGCTATGACC. The primer containing the additional PU 5' sequence is listed in SEQ ID No 7. The primer containing the additional RP 5' sequence is listed in SEQ ID No 8.

The synthesis of these primers was performed following the phosphoramidite method, on a GENSET UFPS 24.1 synthesizer.

DNA amplification was performed on a Genius II thermocycler. After heating at 95°C for 10 min, 40 cycles were performed. Each cycle comprised: 30 sec at 95°C, 54°C for 1 min, and 30 sec at 72°C. For final elongation, 10 min at 72°C ended the amplification. The quantities of the amplification products obtained were determined on 96-well microtiter plates, using a fluorometer and Picogreen as intercalant agent (Molecular Probes).

## Example 3

### Identification Of Biallelic Markers - Sequencing Of Amplified Genomic DNA And Identification Of Polymorphisms

The sequencing of the amplified DNA obtained in example 2 was carried out on ABI 377 sequencers. The sequences of the amplification products were determined using automated dideoxy terminator sequencing reactions with a dye terminator cycle sequencing protocol. The products of the sequencing reactions were run on sequencing gels and the sequences were determined using gel image analysis (ABI Prism DNA Sequencing Analysis software (2.1.2 version)).

The sequence data were further evaluated to detect the presence of biallelic markers within the amplified fragments. The polymorphism search was based on the presence of superimposed peaks in the electrophoresis pattern resulting from different bases occurring at the same position as described previously.

In the 17 fragments of amplification, 18 biallelic markers were detected. The localization of these biallelic markers are as shown in Table 2.

**Table 2**

| Amplicon | BM | Marker Name | Polymorphism | | BM position in SEQ ID | |
|---|---|---|---|---|---|---|
| | | | all1 | all2 | No 1 | No 4 |
| 99-62626 | A1 | 99-62626-168 | | | 12642 | |
| 99-62632 | A2 | 99-62632-275 | | | 14088 | |
| 99-62633 | A3 | 99-62633-409 | | | 24981 | |
| 99-62611 | A4 | 99-62611-51 | | | 69248 | |
| 99-62605 | A5 | 99-62605-56 | | | 73428 | |
| 99-62635 | A6 | 99-62635-443 | | | 80250 | |
| **Amplicon** | **BM** | **Marker Name** | **Polymophism** | | **BM position in SEQ ID** | |
| | | | **all1** | **all2** | **No 2** | **No 4** |
| 99-79335 | A7 | 99-79335-60 | | | 51090 | |
| 99-79336 | A8 | 99-79336-369 | | | 61293 | |
| 99-79338 | A9 | 99-79338-332 | | | 80602 | |

(continued)

| Amplicon | BM | Marker Name | Polymophism | | BM position in SEQ ID | |
|---|---|---|---|---|---|---|
| | | | all1 | all2 | No 2 | No 4 |
| 99-79314 | A10 | 99-79314-201 | | | 100485 | |
| 99-79314 | A11 | 99-79314-225 | | | 100509 | |
| 99-79316 | A12 | 99-79316-158 | | | 106725 | 1658 |
| 99-7932 | A13 | 99-79322-224 | | | 166087 | |
| 99-79322 | A14 | 99-79322-473 | | | 166336 | |
| 99-79306 | A15 | 99-79306-182 | | | 235894 | |
| | | | **all1** | **all2** | **No 3** | **No 4** |
| 99-79310 | A16 | 99-79310-29 | | | 31646 | 4481 |
| 99-79311 | A17 | 99-79311-50 | | | 42373 | |
| **Amplicon** | **BM** | **Marker Name** | **Polymophism** | | **BM position in SEQ ID** | |
| | | | **all1** | **all2** | **No 6** | |
| 99-62617 | A18 | 99-62617-105 | | | 105 | |

BM refers to "biallelic marker". All1 and all2 refer respectively to allele 1 and allele 2 of the biallelic marker.

**Table 3**

| BM | Marker Name | Position range of probes in SEQ ID No 1 | | Probes |
|---|---|---|---|---|
| A1 | 99-62626-168 | 12630 | 12654 | P1 |
| A2 | 99-62632-275 | 14076 | 14100 | P2 |
| A3 | 99-62633-409 | 24969 | 24993 | P3 |
| A4 | 99-62611-51 | 69236 | 69260 | P4 |
| A5 | 99-62605-56 | 73416 | 73440 | P5 |
| A6 | 99-62635-443 | 80238 | 80262 | P6 |
| **BM** | **Marker Name** | **Position range of probes in SEQ ID No 2** | | **Probes** |
| A7 | 99-79335-60 | 51078 | 51102 | P7 |
| A8 | 99-79336-369 | 61281 | 61305 | P8 |
| A9 | 99-79338-332 | 80590 | 80614 | P9 |
| A10 | 99-79314-201 | 100473 | 100497 | P10 |
| A11 | 99-79314-225 | 100497 | 100521 | P11 |
| A12 | 99-79316-158 | 106713 | 106737 | P12 |
| A13 | 99-79322-224 | 166075 | 166099 | P13 |
| A14 | 99-79322-473 | 166324 | 166348 | P14 |
| A15 | 99-79306-182 | 235882 | 235906 | P15 |
| **BM** | **Marker Name** | **Position range of probes in SEQ ID No 3** | | **Probes** |
| A16 | 99-79310-29 | 31634 | 31658 | P16 |
| A17 | 99-79311-50 | 42361 | 42385 | P17 |

(continued)

| BM | Marker Name | Position range of probes in SEQ ID No 6 | | Probes |
|---|---|---|---|---|
| A18 | 99-62617-105 | 93 | 117 | P18 |

## Example 4

### Validation Of The Polymorphisms Through Microsequencing

The biallelic markers identified in example 3 were further confirmed and their respective frequencies were determined through microsequencing. Microsequencing was carried out for each individual DNA sample described in Example 1.

Amplification from genomic DNA of individuals was performed by PCR as described above for the detection of the biallelic markers with the same set of PCR primers (Table 1).

The preferred primers used in microsequencing were about 19 nucleotides in length and hybridized just upstream of the considered polymorphic base. According to the invention, the primers used in microsequencing are detailed in Table 4.

Table 4

| Marker Name | Biallelic Marker | Mis. 1 | Position range of microsequencing primer mis 1 in SEQ ID No 1 | | Mis. 2 | Complementary position range of microsequencing primer mis. 2 in SEQ ID No 1 | |
|---|---|---|---|---|---|---|---|
| 99-62626-168 | A1 | D1 | 12623 12641 | | E1 | 12643 12661 | |
| 99-62632-275 | A2 | D2 | 14069 14087 | | E2 | 14089 14107 | |
| 99-62633-409 | A3 | D3 | 24962 24980 | | E3 | 24982 25000 | |
| 99-62611-51 | A4 | D4 | 69229 69247 | | E4 | 69249 69267 | |
| 99-62605-56 | A5 | D5 | 73409 73427 | | E5 | 73429 73447 | |
| 99-62635-443 | A6 | D6 | 80231 80249 | | E6 | 80251 80269 | |
| Marker Name | Biallelic Marker | Mis. 1 | Position range of microsequencing primer mis 1 in SEQ ID No 2 | | Mis. 2 | Complementary position range of microsequencing primer mis. 2 in SEQ ID No 2 | |
| 99-79335-60 | A7 | D7 | 51071 | 51089 | E7 | 51091 | 51109 |
| 99-79336-369 | A8 | D8 | 61274 | 61292 | E8 | 61294 | 61312 |
| 99-79338-332 | A9 | D9 | 80583 | 80601 | E9 | 80603 | 80621 |
| 99-79314-201 | A10 | D10 | 100466 | 100484 | E10 | 100486 | 100504 |
| 99-79314-225 | A11 | D11 | 100490 | 100508 | E11 | 100510 | 100528 |
| 99-79316-158 | A12 | D12 | 106706 | 106724 | E12 | 106726 | 106744 |
| 99-79322-224 | A13 | D13 | 166068 | 166086 | E13 | 166088 | 166106 |
| 99-79322-473 | A14 | D14 | 166317 | 166335 | E14 | 166337 | 166355 |
| 99-79306-182 | A15 | D15 | 235875 | 235893 | E15 | 235895 | 235913 |
| Marker Name | Biallelic Marker | Mis. 1 | Position range of microsequencing primer mis 1 in SEQ ID No 3 | | Mis. 2 | Complementary position range of microsequencing primer mis. 2 in SEQ ID No 3 | |
| 99-79310-29 | A16 | D16 | 31627 | 31645 | E16 | 31647 | 31665 |
| 99-79311-50 | A17 | D17 | 42354 | 42372 | E17 | 42374 | 42392 |

(continued)

| Marker Name | Biallelic Marker | Mis.1 | Position range of microsequencing primer mis 1 in SEQ ID No 6 | | Mis. 2 | Complementary position range of microsequencing primer mis. 2 in SEQ ID No 6 | |
|---|---|---|---|---|---|---|---|
| 99-62617-105 | A18 | D18 | 86 | 104 | E18 | 106 | 124 |

Mis 1 and Mis 2 respectively refer to microsequencing primers which hybridiz with the non-coding strand of the CanIon gene or with the coding strand of the CanIon gene.

The microsequencing reaction was performed as follows :

After purification of the amplification products, the microsequencing reaction mixture was prepared by adding, in a 20μl final volume: 10 pmol microsequencing oligonucleotide, 1 U Thermosequenase (Amersham E79000G), 1.25 μl Thermosequenase buffer (260 mM Tris HCl pH 9.5, 65 mM $MgCl_2$), and the two appropriate fluorescent ddNTPs (Perkin Elmer, Dye Terminator Set 401095) complementary to the nucleotides at the polymorphic site of each biallelic marker tested, following the manufacturer's recommendations. After 4 minutes at 94°C, 20 PCR cycles of 15 sec at 55°C, 5 sec at 72°C, and 10 sec at 94°C were carried out in a Tetrad PTC-225 thermocycler (MJ Research). The unincorporated dye terminators were then removed by ethanol precipitation. Samples were finally resuspended in formamide-EDTA loading buffer and heated for 2 min at 95°C before being loaded on a polyacrylamide sequencing gel. The data were collected by an ABI PRISM 377 DNA sequencer and processed using the CanIonSCAN software (Perkin Elmer).

Following gel analysis, data were automatically processed with software that allows the determination of the alleles of biallelic markers present in each amplified fragment.

The software evaluates such factors as whether the intensities of the signals resulting from the above microsequencing procedures are weak, normal, or saturated, or whether the signals are ambiguous. In addition, the software identifies significant peaks (according to shape and height criteria). Among the significant peaks, peaks corresponding to the targeted site are identified based on their position. When two significant peaks are detected for the same position, each sample is categorized classification as homozygous or heterozygous type based on the height ratio.

## Example 5

## Preparation of Antibody Compositions to the CanIon protein

Substantially pure protein or polypeptide is isolated from transfected or transformed cells containing an expression vector encoding the CanIon protein or a portion thereof. The concentration of protein in the final preparation is adjusted, for example, by concentration on an Amicon filter device, to the level of a few micrograms/ml. Monoclonal or polyclonal antibody to the protein can then be prepared as follows:

### A. Monoclonal Antibody Production by Hybridoma Fusion

Monoclonal antibody to epitopes in the CanIon protein or a portion thereof can be prepared from murine hybridomas according to the classical method of Kohler, G. and Milstein, C., (1975) or derivative methods thereof. Also see Harlow, E., and D. Lane. 1988..

Briefly, a mouse is repetitively inoculated with a few micrograms of the CanIon protein or a portion thereof over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall (1980), and derivative methods thereof. Selected positive clones can be expanded and their monoclonal antibody product harvested for use. Detailed procedures for monoclonal antibody production are described in Davis, L. et al. Basic Methods in Molecular Biology Elsevier, New York. Section 21-2.

### B. Polyclonal Antibody Production by Immunization

Polyclonal antiserum containing antibodies to heterogeneous epitopes in the CanIon protein or a portion thereof can be prepared by immunizing suitable non-human animal with the CanIon protein or a portion thereof, which can be unmodified or modified to enhance immunogenicity. A suitable non-human animal is preferably a non-human mammal is selected, usually a mouse, rat, rabbit, goat, or horse. Alternatively, a crude preparation which has been enriched for CanIon concentration can be used to generate antibodies. Such proteins, fragments or preparations are introduced into the non-human mammal in the presence of an appropriate adjuvant (e.g. aluminum hydroxide, RIBI, etc.) which is known in the art. In addition the protein, fragment or preparation can be pretreated with an agent which will increase antigenicity, such agents are known in the art and include, for example, methylated bovine serum albumin (mBSA), bovine serum albumin (BSA), Hepatitis B surface antigen, and keyhole limpet hemocyanin (KLH). Serum from the immunized animal is collected, treated and tested according to known procedures. If the serum contains polyclonal antibodies to undesired epitopes, the polyclonal antibodies can be purified by immunoaffinity chromatography.

Effective polyclonal antibody production is affected by many factors related both to the antigen and the host species. Also, host animals vary in response to site of inoculations and dose, with both inadequate or excessive doses of antigen resulting in low titer antisera. Small doses (ng level) of antigen administered at multiple intradermal sites appears to be most reliable. Techniques for producing and processing polyclonal antisera are known in the art, see for example, Mayer and Walker (1987). An effective immunization protocol for rabbits can be found in Vaitukaitis, J. et al. (1971).

Booster injections can be given at regular intervals, and antiserum harvested when antibody titer thereof, as determined semi-quantitatively, for example, by double immunodiffusion in agar against known concentrations of the antigen, begins to fall. See, for example, Ouchterlony, O. et al. (1973). Plateau concentration of antibody is usually in the range of 0.1 to 0.2 mg/ml of serum (about 12 μM). Affinity of the antisera for the antigen is determined by preparing competitive binding curves, as described, for example, by Fisher, D. (1980).

Antibody preparations prepared according to either the monoclonal or the polyclonal protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

## References

Abbondanzo SJ et al., 1993, Methods in Enzymology, Academic Press, New York, pp 803-823

Ajioka R.S. et al., *Am. J. Hum. Genet.,* 60:1439-1447, 1997

Altschul et al., 1990, J. Mol. Biol. 215(3):403-410

Altschul et al., 1993, Nature Genetics 3:266-272

Altschul et al., 1997, Nuc. Acids Res. 25:3389-3402

Ames, R.S. et al. J. Immunol. Methods 184:177-186(1995)

Anderson et al., Symp. Soc. Exp. Biol. 48: 85-97 (1994)

Anton M. et al., 1995, J. Virol., 69 : 4600-4606

Araki K et al. (1995) *Proc. Natl. Acad. Sci. USA.* 92(1):160-4.

Ashkenazi, A. et al. PNAS 88:10535-10539(1991)

Aszódi et al., Proteins:Structure, Function, and Genetics, Supplement 1:38-42 (1997)

Ausubel et al. (1989)Current Protocols in Molecular Biology, Green Publishing Associates and Wiley Interscience, N.Y.

Bartunek, P. et al. Cytokine 8(1):14-20 (1996)

Baubonis W. (1993) *Nucleic Acids Res.* 21 (9):2025-9.

Beaucage et al., *Tetrahedron Lett* 1981, 22: 1859-1862

Better, M. et al. Science 240:1041-1043(1988)

Blouin JL et al. Nature Genetics, 20: 70-73 (1998)

Bradley A., 1987, Production and analysis of chimaeric mice. *In:* EJ. Robertson (Ed.), Teratocarcinomas and embryonic stem cells: A practical approach. IRL Press, Oxford, pp.113.

Bram RJ et al., 1993, Mol. Cell Biol., 13 : 4760-4769

Brinkman U. et al. J. Immunol. Methods 182:41-50(1995)

Brown EL, Belagaje R, Ryan MJ, Khorana HG, *Methods Enzymol* 1979;68:109-151

Brutlag et al. Comp. App. Biosci. 6:237-245, 1990

Brzustowicz et al., Am. J. Hum. Genet. 65:1096-1103 (1999)

Bulman et al., Hum. Mol. Gen. 6(10) 1679-1685 (1997)

Burton, D.R. et al. Advances in Immunology 57:191-280(1994)

Bush et al., 1997, J. Chromatogr., 777 : 311-328.

Carlson, N.G. et al. J. Biol. Chem. 272(17):11295-11301(1997)

Chai H. et al. (1993) *Biotechnol. Appl. Biochem.*18:259-273.
Chee et al. (1996) *Science.* 274:610-614.
Chen and Kwok *Nucleic Acids Research* 25:347-353 1997
Chen et al. (1987) *Mol. Cell. Biol.* 7:2745-2752.
Chen et al. *Proc. Natl. Acad. Sci. USA* 94/20 10756-10761,1997
Chen, Z. et al. Cancer Res. 58(16): 3668-3678(1998)
Cho RJ et al., 1998, Proc. Natl. Acad. Sci. USA, 95(7) : 3752-3757.
Chou J.Y., 1989, Mol. Endocrinol., 3: 1511-1514.
ClarkA.G. (1990) *Mol. Biol. Evol.* 7:111-122.
Coles R, Caswell R, Rubinsztein DC, *Hum Mol Genet* 1998;7:791-800
Compton J. (1991) *Nature*. 350(6313):91-92.
Davis L.G., M.D. Dibner, and J.F. Battey, Basic Methods in Molecular Biology, ed., Elsevier Press, NY, 1986
Dempster et al., (1977) *J. R. Stat. Soc.,* 39B:1-38.
Deng, B. et al. Blood 92(6): 1981-1988(1998)
Dent DS & Latchman DS (1993) The DNA mobility shift assay. In: *Transcription Factors: A Practical Approach* (Latchman DS, ed.) pp1-26. Oxford: IRL Press
Denyer et al.,Drug Disc. Today 3(7): 323-332 (1998
Eckner R. et al. (1991) *EMBO J.* 10:3513-3522.
Edwards et Leatherbarrow, *Analytical Biochemistry,* 246, 1-6 (1997)
Engvall, E., Meth. Enzymol. 70:419 (1980)
Excoffier L. and Slatkin M. (1995) *Mol. Biol. Evol.,* 12(5): 921-927.
Feldman and Steg, 1996, Medecine/Sciences, synthese, 12:47-55
Felici F., 1991, J. Mol. Biol., Vol. 222:301-310
Fell, H.P. et al. J. Immunol. 146:2446-2452(1991)
Fields and Song, 1989, Nature, 340 : 245-246
Fisher, D., Chap. 42 in: Manual of Clinical Immunology, 2d Ed. (Rose and Friedman, Eds.) Amer. Soc. For Microbiol., Washington, D.C. (1980)
Flotte et al. (1992) *Am. J. Respir. Cell Mol. Biol.* 7:349-356.
Fodor et al. (1991) *Science* 251:767-777.
Fraley et al. (1979) *Proc. Natl. Acad. Sci. USA.* 76:3348-3352.
Fried M, Crothers DM, *Nucleic Acids Res* 1981;9:6505-6525
Fromont-Racine M. et al., 1997, Nature Genetics, 16(3) : 277-282.
Fuller S. A. et al. (1996) *Immunology in Current Protocols in Molecular Biology,* Ausubel et al.Eds, John Wiley & Sons, Inc., USA.
Furth P.A. et al. (1994) *Proc. Natl. Acad. Sci USA.* 91:9302-9306.
Gamer MM, Revzin A, *Nucleic Acids Res* 1981;9:3047-3060
Geysen H. Mario et al. 1984. Proc. Natl. Acad. Sci. U.S.A. 81:3998-4002
Ghosh and Bacchawat, 1991, *Targeting of liposomes to hepatocytes,* IN: *Liver Diseases, Targeted diagnosis and therapy using specific rceptors and ligands.* Wu et al. Eds., Marcel Dekeker, New York, pp. 87-104.
Gillies, S.D. et al. J. Immunol. Methods 125:191-202(1989)
Gillies, S.O. et al. PNAS 89:1428-1432(1992)
Gonnet et al., 1992, Science 256:1443-1445
Gonzalez et al., Drug Disc. Today 4(9): 431:439 (1999)
Gopal (1985) *Mol. Cell. Biol*., 5:1188-1190.
Gossen M. et al. (1992) *Proc. Natl. Acad. Sci. USA.* 89:5547-5551.
Gossen M. et al. (1995) *Science.* 268:1766-1769.
Graham et al. (1973) *Virology* 52:456-457.
Green et al., *Ann. Rev. Biochem.* 55:569-597 (1986)
Greenspan and Bona, FASEB J. 7(5):437-444 (1989)
Griffin et al. *Science* 245:967-971 (1989)
Grompe, M. (1993) *Nature Genetics.* 5:111-117.
Grompe, M. et al. (1989) *Proc. Natl. Acad. Sci. U.S.A.* 86:5855-5892.
Gu H. et al. (1993) *Cell* 73:1155-1164.
Gu H. et al. (1994) *Science* 265:103-106.
Guatelli J C et al. *Proc. Natl. Acad. Sci. USA.* 35:273-286.
Hacia JG, Brody LC, Chee MS, Fodor SP, Collins FS, *Nat Genet* 1996;14(4):441-447
Hall L. A. and Smirnov I. P. (1997) *Genome Research,* 7:378-388.
Hames B.D. and Higgins S.J. (1985) *Nucleic Acid Hybridization: A Practical Approach.* Hames and Hamill, Pfugers Arch. 391, 85-100 (1981).
Hammerling, et al., in: MONOCLONAL ANTIBODIES AND T-CELL HYBRIDOMAS 563-681 (Elsevier, N.Y., 1981)
Harju L, Weber T, Alexandrova L, Lukin M, Ranki M, Jalanko A, *Clin Chem* 1993;39(11Pt 1):2282-2287
Harland et al. (1985) *J. Cell. Biol.* 101:1094-1095.
Harlow, E., and D. Lane. 1988. Antibodies A Laboratory Manual. Cold Spring Harbor Laboratory. pp. 53-242
Harper JW et al., 1993, Cell, 75 : 805-816
Harrop, J.A. et al. J. Immunol. 161(4): 1786-1794(1998)
Hawley M.E. et al. (1994) *Am. J. Phys. Anthropol.* 18:104.
Hell et al., Annals N.Y. Acad. Sci. 747:282-293 (1994)
Henikoff and Henikoff, 1993, Proteins 17:49-61
Higgins et al., 1996, Methods Enzymol. 266:383-402
Higgins Ed., IRL Press, Oxford.
Hillier L. and Green P. *Methods Appl.,* 1991, 1: 124-8.
Hoess et al. (1986) *Nucleic Acids Res.* 14:2287-2300.
Huang L. et al. (1996) *Cancer Res* 56(5):1137-1141.
Huston et al. Methods in Enzymology 203:46-88(1991)
Huygenetal. (1996) *Nature Medicine.* 2(8):893-898.
Izant JG, Weintraub H, *Cell* 1984 Apr;36(4):1007-15
Julan et al. (1992) *J. Gen. Virol.* 73:3251-3255.
Kanegae Y. et al., *Nucl. Acids Res.* 23:3816-3821 (1995).
Karlin and Altschul, 1990, Proc. Natl. Acad. Sci. USA 87:2267-2268
Kettleborough, C.A. et al. Eur. J. Immunol. 24:952-958(1994)
Khoury J. et al., *Fundamentals of Genetic Epidemiology, Oxford University Press, NY,* 1993
Kim U-J. et al. (1996) *Genomics* 34:213-218.
Klein et al. (1987) *Nature.* 327:70-73.
Koch, et al., J.Biol.Chem. 265 (29): 17786-17791 (1990)
Kohler, G. and Milstein, C., Nature 256:495 (1975)
Koller et al. (1992) *Annu. Rev. Immunol.* 10:705-730.
Kozal MJ, Shah N, Shen N, Yang R, Fucini R, Merigan TC, Richman DD, Morris D, Hubbell E, Chee M, Gingeras TR, Nat Med 1996;2(7):753-759
Lander and Schork, *Science,* 265, 2037-2048, 1994
Landegren U. et al. (1998) *Genome Research,* 8:769-776.
Lange K. (1997) *Mathematical and Statistical Methods for Genetic Analysis.* Springer, New York.
Lee et al., FEBS Lett. 445 231:236 (1999).
Lenhard T. et al. (1996) *Gene.* 169:187-190.
Liautard, J. et al. Cytokinde 9(4):233-241(1997)
Lin MW et al. Hum. Genet., 99(3): 417-420 (1997)
Linton M.F. et al. (1993) *J. Clin. Invest.* 92:3029-3037.
Liu Z. et al. (1994) *Proc. Natl. Acad. Sci. USA.* 91:4528-4262.
Livak et al., *Nature Genetics,* 9:341-342, 1995
Livak KJ, Hainer JW, *Hum Mutat* 1994;3(4):379-385
Lockhart et al. *Nature Biotechnology* 14: 1675-1680, 1996
Lucas A.H., 1994, In : Development and Clinical Uses of Haempophilus b Conjugate;
Manger et al., Anal. Biochem. 214: 190-194 (1993).
Mansour S.L. et al. (1988) *Nature.* 336:348-352.
Marshall R. L. et al. (1994) *PCR Methods and Applications.* 4:80-84.
McCormick et al. (1994) *Genet. Anal. Tech. Appl.* 11:158-164.
McLaughlin B.A. et al. (1996) *Am. J. Hum. Genet.* 59:561-569.
Morena et al., Annals NY Acad. Sci. 102-117 (1999)
Mori, et al., Nature 350: 398-402 (1991)
Morrison, Science 229:1202 (1985)
Morton N.E., *Am.J. Hum.Genet.,* 7:277-318, 1955
Muller, Y.A. et al. Structure 6(9):1153-1167(1998)
Mullinax, R.L. et al. BioTechniques 12(6):864-869(1992)
Muzyczka et al. (1992) *Curr. Topics in Micro. and Immunol.* 158:97-129.
Nada S. et al. (1993) *Cell* 73:1125-1135.
Nagy A. et al., 1993, Proc. Natl. Acad. Sci. USA, 90: 8424-8428.
Naramura, M. et al. Immunol. Lett. 39:91-99(1994)

Narang SA, Hsiung HM, Brousseau R, *Methods Enzymol* 1979;68:90-98
Neda et al. (1991)J. *Biol. Chem.* 266:14143-14146.
Newton et al. (1989) *Nucleic Acids Res.* 17:2503-2516.
Nickerson D.A. et al. (1990) *Proc. Natl. Acad. Sci. U.S.A.* 87:8923-8927.
Nicolau C. et al., 1987, Methods Enzymol., 149:157-76.
Nicolau et al. (1982) *Biochim. Biophys. Acta.* 721:185-190.
Nissinoff, J. Immunol. 147(8): 2429-2438 (1991).
Nyren P, Pettersson B, Uhlen M, *Anal Biochem* 1993;208(1):171-175
O'Reilly et al. (1992) *Baculovirus Expression Vectors: A Laboratory Manual.* W. H. Freeman and Co., New York.
Ohno et al. (1994) *Science.* 265:781-784.
Oi et al., BioTechniques 4:214 (1986)
Oldenburg K.R. et al., 1992, Proc. Natl. Acad. Sci., 89:5393-5397.
Olesen et al., *Voltage gated ion channel modulators,* 7-8 December , Philadelphia PA, USA (1995)
Orita et al. (1989) *Proc. Natl. Acad. Sci. U.S.A.*86: 2776-2770.
Ott J., *Analysis o fHuman Genetic Linkage, John Hopkins University Press, Baltimore,* 1991
Ouchterlony, O. et al., Chap. 19 in: Handbook of Experimental Immunology D. Wier (ed) Blackwell (1973)
Padlan E.A., Molecular Immunology 28(4/5): 489-498(1991)
Parmley and Smith, Gene, 1988, 73:305-318
Pastinen et al., *Genome Research* 1997; 7:606-614
Pearson and Lipman, 1988, Proc. Natl. Acad. Sci. USA 85(8):2444-2448
Pease S. ans William R.S., 1990, Exp. Cell. Res., 190: 209-211.
Perlin et al. (1994) *Am. J. Hum. Genet.* 55:777-787.
Persic, L. et al. Gene 187 9-18(1997)
Peterson et al., 1993, Proc. Natl. Acad. Sci. USA, 90 : 7593-7597.
Pietu et al. *Genome Research* 6:492-503, 1996
Pitard, V. et al. J. Immunol. Methods 205(2): 177-190(1997)
Potter et al. (1984) *Proc. Natl. Acad. Sci. U.S.A.* 81(22):7161-7165.
Prat, M. et al. J. Cell. Sci. 111(Pt2): 237-247(1998)
Ramunsen et al., 1997, Electrophoresis, 18 : 588-598.
Reid L.H. et al. (1990) *Proc. Natl. Acad. Sci. U.S.A.* 87:4299-4303.
Risch, N. and Merikangas, K. (*Science,* 273:1516-1517, 1996
Robertson E., 1987, Embryo-derived stem cell lines. In: E.J. Robertson Ed. *Teratocarcinomas and embrionic stem cells: a practical approach.* IRL Press, Oxford, pp. 71.
Roguska M.A. et al. PNAS 91:969-973), (1994)
Rossi et al., *Pharmacol. Ther.* 50:245-254, (1991)
Roth J.A. et al. (1996) *Nature Medicine.* 2(9):985-991.
Roux et al. (1989) *Proc. Natl. Acad. Sci. U.S.A.* 86:9079-9083.
Ruano et al. (1990) *Proc. Natl. Acad. Sci. U.S.A.* 87:6296-6300.
Sambrook, J., Fritsch, E.F., and T. Maniatis. (1989) *Molecular Cloning: A Laboratory Manual.* 2ed. Cold Spring Harbor Laboratory, Cold Spring Harbor, New York.
Samson M, et al. (1996) *Nature,* 382(6593):722-725.
Samulski et al. (1989) *J. Virol.* 63:3822-3828.
Sanchez-Pescador R. (1988) *J. Clin. Microbiol.* 26(10):1934-1938.
Sarkar, G. and Sommer S.S. (1991) *Biotechniques.*
Sauer B. et al. (1988) *Proc. Natl. Acad. Sci. U.S.A.* 85:5166-5170.
Sawai, H. et al. AJRI 34:26-34(1995)
Schedl A. et al., 1993a, Nature, 362: 258-261.
Schedl et al., 1993b, Nucleic Acids Res., 21: 4783-4787.
Schena et al. *Science* 270:467-470, 1995
Schena et al., 1996, Proc Natl Acad Sci U S A,.93(20):10614-10619.
Schneider et al.(1997) *Arlequin: A Software For Population Genetics Data Analysis.* University of Geneva.
Schwartz and Dayhoff, eds., 1978, Matrices for Detecting Distance Relationships: Atlas of Protein Sequence and Structure, Washington: National Biomedical Research Foundation
Sczakiel G. et al. (1995) *Trends Microbiol.* 3(6):213-217.
Shay J.W. et al., 1991, Biochem. Biophys. Acta, 1072: 1-7.
Sheffield, V.C. et al. (1991) *Proc. Natl. Acad. Sci. U.S.A.* 49:699-706.
Shizuya et al. (1992) *Proc. Natl. Acad Sci. U.S.A.* 89:8794-8797.
Shoemaker DD, et al., *Nat Genet* 1996;14(4):450-456

Shu, L. et al. PNAS 90:7995-7999(1993)
Skerra, A. et al. Science 240:1038-1040. (1988)
Smith (1957) *Ann. Hum. Genet.* 21:254-276.
Smith et al. (1983) *Mol. Cell. Biol.* 3:2156-2165.
Sosnowski RG, et al., *Proc Natl Acad Sci USA* 1997;94:1119-1123
Sowdhamini et al., Protein Engineering 10:207, 215 (1997)
Spielmann S. and Ewens W.J., *Am. J. Hum. Genet.,* 62:450-458, 1998
Spielmann S. et al., *Am. J. Hum. Genet.,* 52:506-516, 1993
Sternberg N.L. (1992) Trends Genet. 8:1-16.
Sternberg N.L. (1994) *Mamm. Genome.* 5:397-404.
Stryer, L., *Biochemistry,* 4th edition, 1995
Studnicka G.M. et al. Protein Engineering 7(6):805-814(1994)
Syvanen AC, *Clin Chim Acta* 1994;226(2):225-236
Szabo A. et al. *Curr Opin Struct Biol* 5, 699-705 (1995)
Tacson et al. (1996) *Nature Medicine.* 2(8):888-892.
Taryman, R.E. et al. Neuron 14(4):755-762(1995)Schaid D.J. et al., *Genet. Epidemiol.*,13:423-450, 1996
Te Riele et al. (1990) Nature. 348:649-651.
Terlau et al., Naturwissenschaften 85: 437-444 (1998)
Terwilliger J.D. and Ott J., *Handbook of Human Genetic Linkage, John Hopkins University Press, London,* 1994
Thomas K.R. et al. (1986) *Cell.* 44:419-428.
Thomas K.R. et al. (1987) *Cell.* 51:503-512.
Thompson et al., 1994, Nucleic Acids Res. 22(2):4673-4680
Tur-Kaspa et al. (1986) *Mol. Cell. Biol.* 6:716-718.
Tyagi et al. (1998) *Nature Biotechnology.* 16:49-53.
Urdea M.S. (1988) *Nucleic Acids Research.* 11:4937-4957.
Urdea M.S. et al.(1991) *Nucleic Acids Symp. Ser.* 24:197-200.
Vaitukaitis, J. et al. J. Clin. Endocrinol. Metab. 33:988-991 (1971)
Valadon P., et al., 1996, J. Mol. Biol., 261:11-22.
Van der Lugt et al. (1991) *Gene.* 105:263-267.
Vil, H. et al. PNAS 89:11337-11341(1992)
Vlasak R. et al. (1983) *Eur. J. Biochem.* 135:123-126.
Wabiko et al. (1986) *DNA.5*(4):305-314.
Walker et al. (1996) *Clin. Chem.* 42:9-13.
Wang et al., 1997, Chromatographia, 44 : 205-208.
Weir, B.S. (1996) *Genetic data Analysis II: Methods for Discrete population genetic Data, Sinauer Assoc., Inc., Sunderland, MA, U.S.A.*
Westerink M.A.J., 1995, Proc. Natl. Acad. Sci., 92:4021-4025
White, M.B. et al. (1992) *Genomics.* 12:301-306.
White, M.B. et al. (1997) *Genomics.* 12:301-306.
Williams, et al. Science 257: 389-395 (1992)
Wong et al. *Gene.* 10:87-94 (1980)
Wood S.A. et al., 1993, Proc. Natl. Acad. Sci. USA, 90: 4582-4585.
Wu and Wu (1987) *J. Biol. Chem.* 262:4429-4432.
Wu and Wu (1988) *Biochemistry.* 27:887-892.
Wu et al. (1989) *Proc. Natl. Acad. Sci. U.S.A.* 86:2757.
Yagi T. et al. (1990) *Proc. Natl. Acad. Sci. U.S.A.* 87:9918-9922.
Yoon, D.Y. et al. J. Immunol. 160(7): 3170-3179(1998)
Zhang et al., Neuropharmacology 32 (11): 1075-1088 (1993)
Zhao et al., *Am. J Hum. Genet.,* 63:225-240, 1998
Zheng, X.X. et al. J. Immunol. 154:5590-5600(1995)
Zhu, Z. et al. Cancer Res. 58(15): 3209-3214(1998)
Zou Y. R. et al. (1994) *Curr. Biol.* 4:1099-1103.

## Claims

1. The use of an antibody or antigen binding domain specifically binding to the polypeptide shown as SEQ ID NO:5 in the preparation of a medicament for the treatment of schizophrenia or bipolar disorder.

2. The in vitro use of an antibody or antigen binding domain specifically binding to the polypeptide shown as SEQ ID NO:5 in the diagnosis of schizophrenia or bipolar disorder.

3. The use according to claims 1 or 2, wherein the antibody or antigen binding domain is chimeric, humanized or human.

4. The use according to any of claims 1 to 3, wherein the antibody or antigen binding domain is a single-chain antibody or antigen binding domain.

5. The use according to any of claims 1 to 4, wherein the antibody or antigen binding domain is monospecific, bispecific, trispecific or multispecific.

6. The use of an antisense tool that inhibits the expression of the ion channel encoded by a polynucleotide shown as SEQ ID NO:1, 2, 3 or 4 comprising a fragment of the polynucleotide shown as SEQ ID NO:1, 2, 3 or 4 in the preparation of a medicament for the treatment of schizophrenia or bipolar disorder.

7. The use according to claim 6, wherein the antisense tool is a ribozyme.

8. A pharmaceutical composition comprising an antibody or antigen binding fragment as described in any one of the claims 1 to 5 or an antisense tool as described in the claims 6 and 7, and a suitable carrier or excipient.

**9.** A method of identifying a candidate modulator of an ion channel polypeptide for the treatment of schizophrenia or bipolar disorder, said method comprising:

a) contacting a polypeptide comprising a polypeptide as shown as SEQ ID NO:5 with a test compound; and
b) determining whether said compound specifically binds to said polypeptide; wherein a detection that said compound specifically binds to said polypeptide indicates that said compound is a candidate modulator of said ion channel polypeptide.

**10.** The method of claim 9 further comprising testing the ion channel activity of said ion channel polypeptide in the presence of said candidate modulator, wherein a difference in the activity of said ion channel polypeptide in the presence of said candidate modulator in comparison to the activity in the absence of said candidate modulator indicates that the candidate modulator is a modulator of said ion channel polypeptide.

**11.** A method of identifying a modulator of an ion channel polypeptide for the treatment of schizophrenia or bipolar disorder, said method comprising:

a) contacting a polypeptide comprising a polypeptide as shown as SEQ ID NO:5 with a test compound; and
b) detecting the ion channel activity of said polypeptide in the presence and absence of said compound;

wherein a detection of a difference in said activity in the presence of said compound in comparison to the activity in the absence of said compound indicates that said compound is a modulator of said ion channel polypeptide,

**12.** A diagnostic kit for diagnosing schizophrenia or bipolar disorder comprising:

a) a polyclonal or monoclonal antibody that specifically binds to an ion channel polypeptide comprising an amino acid sequence of SEQ ID NO:5, or to a peptide fragment, optionally labelled;
b) a reagent allowing the detection of the antigen-antibody complexes formed, said reagent carrying optionally a label, or being able to be recognized itself by a labelled reagent.

**Patentansprüche**

**1.** Verwendung einer Antikörper- oder Antigenbindungsdomäne, die spezifisch an das in Seq.-ID Nr. 5 dargestellte Polypeptid bindet, bei der Herstellung eines Medikaments zur Behandlung von Schizophrenie oder einer bipolaren Störung.

**2.** In-vitro-Verwendung einer Antikörper- oder Antigenbindungsdomäne, die spezifisch and das in Seq.-ID Nr. 5 dargestellte Polypeptid bindet, bei der Diagnose von Schizophrenie oder einer bipolaren Störung.

**3.** Verwendung nach Anspruch 1 oder 2, worin die Antikörper- oder Antigenbindungsdomäne chimär, humanisiert oder menschlich ist.

**4.** Verwendung nach einem der Ansprüche 1 bis 3, worin die Antikörper- oder Antigenbindungsdomäne eine einkettige Antikörper- oder Antigenbindungsdomäne ist.

**5.** Verwendung nach einem der Ansprüche 1 bis 4, worin die Antikörper- oder Antigenbindungsdomäne monospezifisch, bispezifisch, trispezifisch oder multispezifisch ist.

**6.** Verwendung eines Antisense-Werkzeugs, das die Expression des Ionenkanals hemmt, für den ein als Seq.-ID Nr. 1, 2, 3 oder 4 angeführtes Polynucleotid kodiert, und das ein Fragment des als Seq.-ID Nr. 1, 2, 3 oder 4 angeführten Polynucleotids umfasst, bei der Herstellung eines Medikaments zur Behandlung von Schizophrenie oder einer bipolaren Störung.

**7.** Verwendung nach Anspruch 6, worin das Antisense-Werkzeug ein Ribozym ist.

**8.** Pharmazeutische Zusammensetzung, die eine in einem der Ansprüche 1 bis 5 beschriebene Antikörper- oder Antigenbindungsdomäne oder ein in Anspruch 6 oder 7 beschriebenes Antisense-Werkzeug sowie einen geeigneten Träger oder Exzipienten umfasst.

**9.** Verfahren zur Identifikation eines vermutlichen Modulators eines Ionenkanalpolypeptids für die Behandlung von Schizophrenie oder einer bipolaren Störung, wobei das Verfahren Folgendes umfasst:

a) das Kontaktieren eines Polypeptids, das ein Polypeptid umfasst, wie es in Seq.-ID Nr. 5 dargestellt ist, mit einer Testverbindung; und
b) das Bestimmen, ob die Verbindung spezifisch an das Polypeptid bindet;

worin die Detektion, dass die Verbindung spezifisch an das Polypeptid bindet, anzeigt, dass die Verbindung ein vermutlicher Modulator des Ionenkanalpolypeptids ist.

**10.** Verfahren nach Anspruch 9, das weiters das Testen der Ionenkanalaktivität des Ionenkanalpolypeptids in Gegenwart des vermutlichen Modulators umfasst, worin ein Unterschied zwischen der Aktivität des Ionenkanalpolypeptids in Gegenwart des vermutlichen Modulators und seiner Aktivität in Abwesenheit des vermutlichen Modulators zeigt, dass der vermutliche Modulator ein Modulator des Ionenkanalpolypeptids ist.

**11.** Verfahren zur Identifikation eines Modulators eines Ionenkanalpolypeptids zur Behandlung von Schizophrenie oder einer bipolaren Störung, wobei das Verfahren Folgendes umfasst:

a) das Kontaktieren eines Polypeptids, das ein Polypeptid umfasst, wie es in Seq.-ID Nr. 5 dargestellt ist, mit einer Testverbindung; und
b) das Detektieren der Ionenkanalaktivität des Polypeptids in Gegenwart und Abwesenheit der Verbindung;

worin die Detektion eines Unterschieds zwischen der Aktivität in Gegenwart der Verbindung und der Aktivität in Abwesenheit der Verbindung zeigt, dass die Verbindung ein Modulator des Ionenkanalpolypeptids ist.

**12.** Diagnoseset zur Diagnose von Schizophrenie oder einer bipolaren Störung, Folgendes umfassend:

a) einen polyklonalen oder monoklonalen Antikörper, der spezifisch an ein Ionenkanalpolypeptid, das eine Aminosäuresequenz der Seq.-ID Nr. 5 umfasst, oder an ein Peptidfragment bindet und gegebenenfalls markiert ist;
b) ein Reagens, das die Detektion der gebildeten Antigen-Antikörper-Komplexe ermöglicht, wobei das Reagens gegebenenfalls eine Markierung aufweist oder selbst von einem markierten Reagens erkannt werden kann.

## Revendications

**1.** Utilisation d'un domaine liant l'antigène ou l'anticorps se liant spécifiquement au polypeptide montré par SEQ ID NO :5 dans la préparation d'un médicament pour le traitement de la schizophrénie ou d'un trouble bipolaire.

**2.** Utilisation in vitro d'un domaine liant l'antigène ou l'anticorps se liant spécifiquement au polypeptide montré par SEQ ID NO :5 dans le diagnostic de la schizophrénie ou d'un trouble bipolaire.

**3.** Utilisation selon les revendications 1 ou 2, où le domaine liant l'antigène ou l'anticorps est chimérique, humanisé ou humain.

**4.** Utilisation selon l'une quelconque des revendications 1 à 3, où le domaine liant l'antigène ou l'anticorps est un domaine liant l'antigène ou l'anticorps mono-caténaire.

**5.** Utilisation selon l'une quelconque des revendications 1 à 4, où le domaine liant l'antigène ou l'anticorps est monospécifique, bispécifique, trispécifique ou multispécifique.

**6.** Utilisation d'un outil antisens qui inhibe l'expression du canal d'ions codé par un polynucléotide montré par SEQ ID NO :1, 2, 3 ou 4 , comprenant un fragment du polynucléotide montré par SEQ ID NO :1, 2, 3 ou 4 , dans la préparation d'un médicament pour le traitement de la schizophrénie ou d'un trouble bipolaire.

**7.** Utilisation selon la revendicati on 6, où l'outil antisens est un ribozyme.

**8.** Composition pharmaceutique comprenant un fragment liant l'anticorps ou l'antigène tel que décrit dans l'une quelconque des revendications 1 à 5, ou un outil antisens tel que décrit aux revendications 6 et 7 et un support ou excipient approprié.

**9.** Méthode d'identification d'un modulateur candidat d'un polypeptide d'un canal d'ions pour le traitement de la schizophrénie ou d'un trouble bipolaire, ladite méthode comprenant :

a) mise en contact d'un polypeptide comprenant un polypeptide tel que montré par SEQ ID NO :5 avec un composé de test ; et
b) détermination du fait que le composé se lie spécifiquement audit polypeptide ; où une détection que ledit composé se lie spécifiquement audit polypeptide indique que ledit composé est un modulateur candidat dudit polypeptide de canal d'ions.

**10.** Méthode de la revendication 9 comprenant de plus le test de l'activité de canal d'ions dudit polypeptide de canal d'ions en présence dudit modulateur candidat, où une différence de l'activité dudit polypeptide du canal d'ions en présence dudit modulateur candidat en comparaison avec l'activité en l'absence dudit modulateur candidat indique que le modulateur candidat est un modulateur dudit polypeptide du canal d'ions.

**11.** Méthode d'identification d'un modulateur d'un polypeptide de canal d'ions pour le traitement de la schizophrénie ou d'un trouble bipolaire, ladite méthode comprenant :

a) mise en contact d'un polypeptide comprenant un polypeptide tel que montré par SEQ ID NO :5 avec un composé de test ; et
b) détection de l'activité de canal d'ions dudit polypeptide en présence et en l'absence dudit composé,

où une détection d'une différence de ladite activité en présence dudit composé en comparaison avec l'activité en l'absence dudit composé indique que ledit composé est un modulateur dudit polypeptide du canal d'ions.

**12.** Kit de diagnostic pour le diagnostic de la schizophrénie ou d'un trouble bipolaire comprenant :

a) un anticorps polyclonal ou monoclonal qui se lie spécifiquement à un polypeptide de canal d'ions comprenant une séquence d'acides aminés de SEQ ID NO :5 ou un fragment peptidique facultativement marqué ;
b) un réactif permettant la détection des complexes antigène-anticorps formés, ledit réactif portant facultativement un marqueur ou étant capable d'être reconnu lui-même par un réactif marqué.

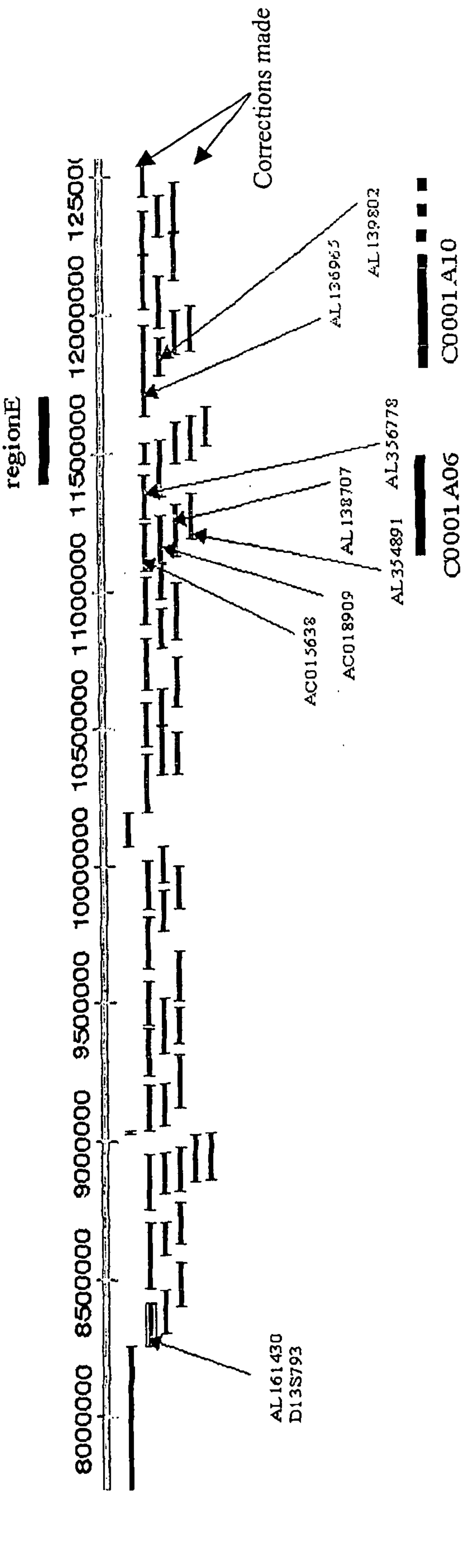
regionE
Corrections made
8000000
8500000
9000000
9500000
10000000
10500000
11000000
11500000
12000000
12500000
AL161430
D13S793
AC015638
AC018909
AL354891
AL138707
AL356778
AL136965
AL139802
C0001A06
C0001A10

Fig. 1

SEQUENCE LISTING

<110> GENSET

<120> SCHIZOPHRENIA RELATED VOLTAGE-GATED ION CHANNEL GENE AND PROTEIN

<130> 93.WO1

<141> 2001-12-04

<150> US 60/251,317
<151> 2000-12-05

<160> 8

<170> Patent.pm

<210> 1
<211> 82293
<212> DNA
<213> Homo sapiens

<220>
<221> misc_feature
<222> 1..2000
<223> 5'regulatory region

<220>
<221> exon
<222> 2001..2026
<223> exon 1

<220>
<221> exon
<222> 19188..19334
<223> exon 2

<220>
<221> exon
<222> 22996..23178
<223> exon 3

<220>
<221> exon
<222> 39731..39814
<223> exon 4

<220>
<221> exon
<222> 41337..41476
<223> exon 5

<220>
<221> exon
<222> 41565..41693
<223> exon 6

<220>
<221> exon
<222> 73013..73167
<223> exon 7

<220>
<221> allele
<222> 12642
<223> 99-62626-168 : polymorphic base A or T

<220>
<221> allele
<222> 14088
<223> 99-62632-275 : polymorphic base A or G

<220>
<221> allele
<222> 24981
<223> 99-62633-409 : polymorphic base A or T

<220>
<221> allele
<222> 69248
<223> 99-62611-51 : polymorphic base A or C

<220>
<221> allele
<222> 73428
<223> 99-62605-56 : polymorphic base A or G

<220>
<221> allele
<222> 80250
<223> 99-62635-443 : polymorphic base A or G

<220>
<221> primer_bind
<222> 12343..12363
<223> 99-62626.rp

<220>
<221> primer_bind
<222> 12793..12810
<223> 99-62626.pu complement

<220>
<221> primer_bind
<222> 13814..13832
<223> 99-62632.pu

<220>
<221> primer_bind
<222> 14279..14296
<223> 99-62632.rp complement

<220>
<221> primer_bind
<222> 24863..24881
<223> 99-62633.rp

<220>
<221> primer_bind
<222> 25378..25396

<223> 99-62633.pu complement

<220>
<221> primer_bind
<222> 69198..69218
<223> 99-62611.pu

<220>
<221> primer_bind
<222> 69632..69650
<223> 99-62611.rp complement

<220>
<221> primer_bind
<222> 73005..73022
<223> 99-62605.rp

<220>
<221> primer_bind
<222> 73466..73483
<223> 99-62605.pu complement

<220>
<221> primer_bind
<222> 79808..79826
<223> 99-62635.pu

<220>
<221> primer_bind
<222> 80314..80334
<223> 99-62635.rp complement

<220>
<221> primer_bind
<222> 12623..12641
<223> 99-62626-168.mis

<220>
<221> primer_bind
<222> 12643..12661
<223> 99-62626-168.mis complement

<220>
<221> primer_bind
<222> 14069..14087
<223> 99-62632-275.mis

<220>
<221> primer_bind
<222> 14089..14107
<223> 99-62632-275.mis complement

<220>
<221> primer_bind
<222> 24962..24980
<223> 99-62633-409.mis
<220>
<221> primer_bind

<222> 24982..25000
<223> 99-62633-409.mis complement

<220>
<221> primer_bind
<222> 69229..69247
<223> 99-62611-51.mis

<220>
<221> primer_bind
<222> 69249..69267
<223> 99-62611-51.mis complement

<220>
<221> primer_bind
<222> 73409..73427
<223> 99-62605-56.mis

<220>
<221> primer_bind
<222> 73429..73447
<223> 99-62605-56.mis complement

<220>
<221> primer_bind
<222> 80231..80249
<223> 99-62635-443.mis

<220>
<221> primer_bind
<222> 80251..80269
<223> 99-62635-443.mis complement

<220>
<221> misc_binding
<222> 12630..12654
<223> 99-62626-168.probe

<220>
<221> misc_binding
<222> 14076..14100
<223> 99-62632-275.probe

<220>
<221> misc_binding
<222> 24969..24993
<223> 99-62633-409.probe

<220>
<221> misc_binding
<222> 69236..69260
<223> 99-62611-51.probe

<220>
<221> misc_binding
<222> 73416..73440
<223> 99-62605-56.probe

<220>
<221> misc_binding
<222> 80238..80262
<223> 99-62635-443.probe

<220>
<221> misc_feature
<222>

2375,2377,4041..4042,5180,7138,7174,10118,12577,12637,12648,13556
15322,15341,15357,15383,15409,15994,16064..16065,16077,16237
16264,17751,19182,19424,20735,20840,21865,21885,21900,22814,23954
24969,24985,25001,25005..25006,25017,25040,25057,26646,27478
27496,29278,30793,31122,31125,34515,34541,34563,34572,34884,34915
36809,36834,36944,37011,39862,39881,39895,39903,39921,39948
39954..39955,41108,42572,43423,44122,53546,53634,55777,56140
56160,56182,56185,56200,56206,56219,56234,56258,56268,56280,56290
56324,56561,57259,58256,58297,58910,59321,60724,60743,68789,68803

68871,69936,77190,77484,78780,78891,79343,80394,81773,81795,82232

<223> n=a, g, c or t

<400> 1

```
catcaatgca ctccaggcta ggcaacggag ataggccttg tctctaaaaa taacaaagaa     60
aacaaaagaa gagagatact gagcaggtag aaggggtact atgatatttt ggatgtacct    120
gtttttaaa atgattgtct actccaatac acagtgaatc cctatgcatt ataggatgag    180
tgtgctggat tccatgactt ccttgaagcc catgaattct ctgagactgt ctccatcatc    240
attgggattg tttggctcat gagcatgaat tatagagact taacagaggt agttcaacca    300
aggacagaga aaggtctgaa atgcctgttg ggtgaggagt ggcactgaga ttagtaaaaa    360
atcactgcct tgaccacttc aaaacattat aaataaatgt ttaaatattt ccttaatata    420
atttactaac agataattcg aacgaatgtc aaggaataac acactatcat tcagctacca    480
tatatatata gctatatatt tcagtttgtg aaataaacta gtatttagaa caagcaattg    540
aaagccgcaa ataaagatga tgaaacaagt tggtaaatac tttttctaag caaactggtt    600
gtagatttag cattgtttaa attactgtca atttttttctt atgaggagtg atttcatcat    660
ttgtttaaaa cctggctttt gacacagttt ttctgaagtc tttagaaatt tgaaaaatta    720
tctcgcatga ctctgtattt catgaaatac caatatttta acatttccct gtaaacaata    780
aaacgtacta atatggatta tgaatgtgaa caaattgtga atggactttt taagaaagta    840
gattaattaa atatctgtaa ccagaaaata catttttaaa atgtaggttt gtgtagtttt    900
acttctatac cttgccacat aacattttgt gatagacaaa tgctctttgt ttagaatttc    960
tcttgtagag aaagcttcat tcagttgttt ctttcccttt atttatatgc ctgtgtgcat   1020
gatgtctaaa tcactggtca ggagttccct ggggattttc gtatctgact acatggcaag   1080
attccagacc caaagtaaac ctttcagaga gtgggaatga gtgggaggat gtcgccctgg   1140
gcatggtgct taggaggcca gatttcccta aacctgctct gctgggagcc ctaggaagcc   1200
tcagatgcag tctgctgtcc aggtatactc acagggtatg ggtgggggga ctgtcaattt   1260
ctgaatagac tttgtctcat ccacacgaca acatatttta attttaaagg cgaacgtcaa   1320
cctatttaga aaactataag tgtgactgat gcattcatta tttaataact ccggggtgaa   1380
acttgaggtg aacggtttca ccctttcata ctcaggaatg gaactagtcc tcggtaccgt   1440
gcacagtctg acctcacgct cgcacgctgg gctcccggcc tcctggcagt gcacttaagt   1500
gtaccccgcg tgtgcccggc ggactcccgt gcacaccgcc gccgtgctgt tgagtacgaa   1560
agacccccga gaggagctcg cagatgagag cctagctgtt gctgtgagac tgactgcggg   1620
tggcagccgg caagaaggaa ctgccagtac cgaccctggc gctccccacc aactgcggcg   1680
cagcctcctc tgcggtgccc gcccttcggc atcactgcag tccctgtagc aaagctaact   1740
ttaaaaattt aaataaaata ataatttttt aaaggcccga aaactccggc tcccacccct   1800
cccccagcct gcccggcggc ggcggcgctc agtgagtgac acggcgagcg tgagccgcgg   1860
ccccagccgg gccgagcgcg ctgcctgagc tgagccgccg taggtgaggg gcccgcgtcc   1920
ccgcccgccc tgggcgccgc gcctggcact gatcctgccg gtcgcccact gtcgccgccg   1980
ccgccgcccg cgggcaccat gacagctctg agcgctgggg ttacaggttg gtcaccgccg   2040
ccggtgcccg ggaggctgcg gcgcggccgg ctccgtgccc tgcgcccgag gcccgcgatg   2100
cgcgctcgtt agttccttgg ccggtccggg ccaggacggc tcaggggcgc tggggctgcg   2160
agaggggctg ctagaggggc tgcgggcggg ggccccgcgc gggggagggg tggggagggg   2220
cgcctccacc cgagagcggg gcggtggggt ccttgccggc ctggccgggg cgcgggagcc   2280
gcgatgcgcc ctccctattc ctgcgcgtgg gtgtcggggg cgcctgccgc gggatgcccc   2340
tgggctggct tgggaagccc agggccggcg acacntnggg cctgccccgc ctgggcatct   2400
agcaggtgtt ggggggcagg tggctgagga cgaggccact gggcatcact acagcgctgc   2460
acctgccccc tcggcaccag ggccgctgcc tgggtaaacc tctgggccaa cgcgcgggtg   2520
gggaggtggg ggtgggcagc ctggctccag caggtctccg cgggggctcc gcggccttgc   2580
cccaccccca tgctcctgtg gattgctcct tgctaaatgt cccttatgat attcattggc   2640
cttgttaagc agccgtcatt agggtggcta caccgaacgc aaaacagtgt cctgccaagc   2700
aacaccattt tgtcactgaa tctttaagac accagcaagg atggcctggc tgggatccct   2760
ctgggggccc agatcgcagt cattttgctg acttaatgtt cctctttcag gatggtgtga   2820
gaatgctgaa ctcagaccct tagtgcttga aataccttag aataaatctt tcagtttcag   2880
gtacaatttt tcagacactg tattattaag gatgtgtgaa tacacgctca ctttcaggcc   2940
tcagaaggtt ttactcttaa tctggtgttc gtgtggtcat cagctaggcc cgactgccct   3000
ggtggtgaaa tgtgggttga ggaccagtga ccttcgtgtt agtagttggt ggataacacc   3060
tctaataggg gatcaaaaat taacaggtgg cgattttgaa atatatatgt atgtatatat   3120
atatatatat gtgtgtgatt tgtatgttca taactacaac tgtgatttga tatggcctgg   3180
gagaggagcg gtttcactat ggtagttacc cctgaggcag atgcatctca ggactggctt   3240
tttaaattca aagataaggt ttccacagtt ctaaataaaa atcatggctt cccaaacatc   3300
tgcaacgaga aacatttact ccctaactgg ttggctgtca ttctgcgtgc ttttctagag   3360
tcgatttttc gttcttctgt agtatacttt gacctagcca ctgctaaaac cagacagtgt   3420
gaagacaaaa tccagctttg gagaaaaact taaaaagtga aaaggttcag ttggatcctc   3480
ttctgtgttc atagaagaca taatctgttt gcagaataaa tgtgccgtca ctgataagga   3540
```

```
ctatgctaaa atctctacag aactctagga gaaagcatgt cttataaaat agtggttgta 3600
cacttataac tttggatagg acttgcaaga tattcaaagg agaaatgtac ttaaaaaatg 3660
tacttaaaaa aagtctactc aaaatagtgt catttagttg caatttagtg aactgaagat 3720
ttgagtagca ttttcttctt cttgggtatg gcatttcttt gaaatggttt ccaggtgatt 3780
ttatcattcc ggcatgcttt gtgtgtgcat gtatgtgtat acacaaatgt acacatttgt 3840
tttatcattt atcgatgatg ttcagataat ttcagaattt ccatagctgt ataaatttat 3900
cccaggtcag agtcacatgt ggatttcaag tttgtgaatg aaaattcaca tctacacctc 3960
tatatcctaa gataagctaa gcactgggcc agaagctggg tggtgagtga gaaaaagaga 4020
taaactgaga gcctaagaat nnttcaggaa agacaaatat gtaagcaggt ggatatacag 4080
tgagatgtct tattttctag ttttgctgtg gaaactatga tagtgagctt aaacatatta 4140
agaatggtcc ccaaggccac acacggtggc tcatgcctat aatcccagca ctttgggagg 4200
ccaaggcagg cctattgctt gaacccaaga gtccagacca gcctgggcaa catggtgaaa 4260
ccccgtctct acaaaaaaaa aaaaaattca ccaagtgtag tggtgtacac ctgtagcccc 4320
agttacttgg gaggctgagg tggaaggatc gcgtgagccc agagatgtca aggctgcagc 4380
aggctatgat ggcaccactg tactctagaa tgggcgactg ggcacagagt gagactctgt 4440
ttaaaaataa aaaaagaatg cccctgtccc aaaatgttat caacatttta gagcttcact 4500
catgttatac aattctcgcc tgtctcaagg accagcacag attttcatac cttcaaactc 4560
tatgcaaatg cttaaatttt tttaattgat tttttgaatt ttaaagtaac caaagttgat 4620
agagatgata gaatgataga gaaaaaagag taaacagcta gttttaataa ccgaagacag 4680
atgaccacaa tatctgtgtg ttttttgtag tctttttaag ggcttgactt caggtagttg 4740
tcatcgtggc attggacaac ttttatactg tttttaccac tgaatattta ttataaatat 4800
ttaaaatatt tctatataat tttcttactt aaaaattgta aatggctgta taacaccata 4860
cccagtaaat acagcctcat atatttaacc atacctctat cattggacat ttgctgtttc 4920
cagtttttca tcattataaa cagtgtcctg atagttataa tgtagagatt tatttctatt 4980
ttatgttttt cagtataaat atccaaatat gtccatatct gtcacctatt tttccatatc 5040
ttcaaattgg tttcctgaaa aattcagatt ataatttatt tatgataaat ttccagtaat 5100
tatgtctttg aaaaaagaag tatctgaatt attatagaga tatattgtat gtctttaaat 5160
tgatttccta aagaattcan gattataatt ttgaataaat tgcttaatct gcttttgagg 5220
taaatctacc cttttacaaa aagtgttggt ggatcacatt ccctctttta cagaaataaa 5280
agaaggtaac ataataattt ggatgtttga cctttgtgct atataaaaac ttgaccttta 5340
caaaaatcac ctgaaaacca gatgctaaaa tatacctctt agagtaaagc caaaatagga 5400
atactataaa agttcaaagc tggaaaaaga aaaaaatcaa tgcaagttcc tttagaaaat 5460
tggctgttgt attcaggagt ctaattctgc tttttgcttt cctactcaga cttcaagtaa 5520
atgatcaaaa gggcttacat agtgagaaat ctgtaccccc acaggtaaag gaagaagagg 5580
atctttcttt cctaggctgc agaatccaag gcttttctgg tagattcagt atagatggaa 5640
caggactgat aggaaaagct aaagttcagt tcacaatttc ctttctaaga tcagtgtaca 5700
aaaaatacgg aagagagacc ctggaagagc cccttagtat gaaggagcga agttgaaagt 5760
tggaagcagg cttttgtaaa acagaattgc ttgtttctcc atgagatccc agggcaaggc 5820
agccataccg gaatatgttc tgaagacagg gactgacccc acaggtccag ggtgggggca 5880
ggggtaggcc tccgcagcag gagtggaaga attagaagta ggatagacca gaacactgaa 5940
gccaagagaa tcatttatga agagatcaag agtgagaatt atttccaatt atgcaaagaa 6000
aagagatgaa aagaggatgt tgtttggaga gagaatgata tagatggaag acagccaaca 6060
gagacacacc aataaatgtt tttcctgaca gagaggatca cagtgcatgg aacacagcca 6120
tgttttaaag tgagataaga ttcttttttt cttttagatt aaaaacaatc caaatcttca 6180
gatgaaaata agttatcatg taccaaaaca attaataaaa cttagccaac atctacatat 6240
agcctgataa aaactttgga tttgaaaacc aataaaaaga aaaatatttc accagcattg 6300
tgatcaaatg cttcagaaat tacttatcca caaagagaaa atttagatta atgttatact 6360
tcctctctaa aattatcagc aaacataaaa taacgtcctg aaaatctgta gtcctgaaaa 6420
atatcagtag acaattctct tacgttgtaa atgagtcaaa agtgaaaaat aaataggtga 6480
agaaatacat actaggcaag tgatatcagt aaaaaaaaat gaggtagaag tgtgtaaata 6540
tcagaaaaca gttcaaaaaa ttgtttgatt aaatgagaaa aaagaccgta aagtcataaa 6600
agttgctatc tttaatacat ttataactac aggcttccca tatataccaa cgatcagagc 6660
attatagtat gcacagtcaa aagcataatg ctggccgggc gcggtggctc acgcctgtaa 6720
tcccagcact ttgggaggcc gagatgggtg gatcacgagg tcaggagttt gagaccagcg 6780
tggccaacat ggtgaaaccc cgtccctgct aaaaatacaa aaaaattagc taggtgtgat 6840
ggtgggcacc tgtaatctca gctactcggg aggggaggct gaggcaggcg aatcatttga 6900
acccaggagg cagaggttgc agcgagccga gatcacgcat tacactctag cctgggcgac 6960
agggcaagac tccgtctcaa aaaaaaaaaa aaagtctgat gcttatttta ggggaaatgg 7020
aagcggaagc atgcccacag actcaatatc tttttaaaga gttttgtgac ctaaaatggt 7080
attgggagtc agcatttcat cttaagaagt acaaaaaaaa aagaaaaaaa aaagaaanga 7140
ggataaagtt ttaagaatga aaacatgaat gaanttcact atgacataga taaactctaa 7200
atctgatagc aaaaactgag agctacagca ttccaatctg ggaggaatat tgatctacag 7260
gtatatttaa ggtaaattag aatgccaaca aattggaatg atgacaagga ggttagtgaa 7320
```

```
gaaatgcact ggagaaattg gatatacaat tccaagattg aagaactgga atcagattgc   7380
agtaatggaa atgaagaggt ggatggtaaa gatatatcca caaaaactaa tgctaccacg   7440
tcttagtgat ggaaggaaga gaatgaaagg gagtcctagg ttaaaaataa tttccgatca   7500
tcgagcttgg gtgattgtgc tattgtattc tattaagtct agttaaaaga aattgttacg   7560
gctttatctt tcagttaata gtatctacag ggtaaatcct ttgcttcatt ccctttcta    7620
gttgttttat tgttattttg aaattcacat gagattactt ccagaggcaa tatcgttttg   7680
cttcctttct ttgaaggcat tgaatggcaa tttaaaggtg tagtctagac tagaaaatca   7740
taacagaaac cccaacaaaa actcaaatat cttagttatc cttttataaa acaaaaattt   7800
aattcctgta tacaatgcaa gttagttatt ttaaaagtaa attctgaaga tttctgagga   7860
aagaatagaa tatcctgtat atacatacaa ttataaatca aaatttttgt ctcagacata   7920
ttttgcctgt gttgcttata attagactct cttattcttt tccacacaga gccatgacta   7980
tgcaatgtga actctctaag tgttctcttc tgagcatcta ggctagtgat gcctcattaa   8040
tttggaaaca gtgatgggag aggtgtgcag ggatcacctg aagatgtttt taaaaaaata   8100
caatgtcctg ttttccaccc ttaaagattc tgatttagta tgtctgaggt gaaggaggcc   8160
tcagcttgag tgtcttagtt agcttgggct gctgtaacaa atagcacaga ctaggtggct   8220
taaactccag actttggttt tatacagtct ggagactaag aagtccaaga tcaaggtgct   8280
ggctgattcc tgtgagggct gtcttcctgg tccacagacc attcagctgc ctccgttctt   8340
cttatagatt cgctagtccc attcatgggg tctccacctt catggcctca tgacctccca   8400
aaggccccac ctccaaatac attagagata agagcttcaa catatgactt gaggaaggca   8460
gaaccattca gtttatagtg ttgagaatgt ttctgaacct cccttgataa ttcatatgtt   8520
aagcagtctt taggaattac tattttaaac catcccettt tgagcaaatt tgtggattca   8580
tattgcaaaa tttaacaaac acaaaaacat aatctccctt tgatcttctg ttatgtttaa   8640
atatagtagc aaattttgaa aaccactgtt ttaaaccatc caccttcttt atctaacctt   8700
tacagattca tattataaga ttaaacaacc acaatcaaaa taagtatccc ctttctggtt   8760
tttcataata tataaatgat agtagcagct catcaaaata aaatatccaa cactggataa   8820
ttaatataga gtaggatata tactcagcac agggtaggac ggtggtggcc aggggctggg   8880
aggaggggaa aatggggagt tgttaattac tgggtagaac gtttcagtta tgcaagatga   8940
gtgagctcga gagatctgtg ttatgacatt gtgcctgtag ttaacatttt atagtgaatg   9000
taaaaatctg ttaagacagt tctcgtgtta aatgctccta ctacaatgaa acaaaaataa   9060
aaattactgt gatgtaggtg acaaaaatac gacccagttc cctctcatac tgcttgacag   9120
ttttagtttg ggcatgtagg tgcaatgaca gttttagttt gggcgtgcag ggtgcaatga   9180
caaaagggca ttggggcatt gtggtgtgta ctgaatccct ttagcaaacc caatcacttt   9240
aaaggccaga gaaaattcaa ttttcacaat taaaaaataa aacaaaacag aatccttgga   9300
gaagtaatga cagttttgga gtttttctag aaataaccag agaaaaataa cctgctgttt   9360
tactttggga tttaagaaat agacttataa aaagtgggga accatttttt atttctaatc   9420
agtaccttga caaaaggaaa acagaagagt ctgtttttgg agatggttct ttgaaacatt   9480
tgccttcccc tatgtggtca ccaccttggg tagcccttga agtctgctgg gactggacca   9540
tgcatgcaaa attctgtttt ttgcagagct acattattta gtgagggcca agacacagat   9600
gaatgagtac ccaaaagaa aagtggcagg tggcatttgg aggagagaga gagaaggaga   9660
gaggcagaaa gaaaaagaga aggaagaaca aagaaaacca ggcagccaaa tcagggtctt   9720
gacttttgta caaaattaga ttgaaaatca accacaacaa tccactacct gcaggggcag   9780
gggagtgggc cgctgttctg ttgtggagac acgcattgcc gctgtgatgc ccagggtgtc   9840
agttttttga tgctgtctta gttctcctgc taacatgttc tctgctgtgt tgctggggga   9900
gacgcaggac tccaggctaa gtttaagaga gaaaacatag agctgctgcc tttcctctga   9960
gcagaggcca gattacaggg aatgatgcat ccagctcatt gaagttcaca agctaaggat  10020
acgcggagga gaggcttgct tgagagggcc cggaaggact ggcattgtaa gtctagtggc  10080
aggcatgctc taatttggtt ttattactta gacgtggnaa taggcaggaa cttggagatt  10140
cccgtctctg ttctctggga aagaagactg ttctatttac aaagacttga aagggtcaga  10200
ctgaaagaag acatggacag caaagtattc ctggggtggc aatcagaaag acctggctcc  10260
ctcgtggttt atcacagcta accatgtgac ctcagacaag gcacttatct tctccaaaac  10320
tcagtttcac ctgtaaaatc agaataacag agagagagac acttacctca ttgaggcatg  10380
gtgagaaata ggtaacaatt agcctcacac ttaataaacc ctcagcaaat gttaggtgta  10440
acttgtttaa actgaaaatt tttttaatgt tttacttgtt tcaaagaaat tataaataaa  10500
acacatcatc tttgtgaaca tgtacagaag ttcgtgtttg tgaaatgtac aaaagttctt  10560
aaggttaaaa agtgaaagtc aaatgcttca ccatttctct ccctccctct gttctcccaa  10620
caaatagact acttggattc taaacttgct tccaattcat gtgggtctgg gcatgtttgc  10680
ctccaatttt taatctctgt agtgggaata atactaaaaa ctacttcaga gaggtgtcaa  10740
atgcttagaa tagtgcctgg catggagtaa gctttcaata atatttgttc tcatctttca  10800
agttaaaaaa atgagtacat aatgtctaag cttgcttcta ttctataatt atgcacagta  10860
gtttatggac tttcattcta ccctgagaaa tgatcaaatg cataaaaatc tgagcagtgt  10920
caagagtatt aatcactttt atcattggaa gtcactttgg atatcatcta ttgcaaagtc  10980
ccagacagag gagaaatctt ttctgcaaac tctaaaatgt gtagtcattc cctctgagag  11040
tatccataaa ggagtttacc accttttggg atattccaac atattatgat tggaaactta  11100
```

```
tagagtagat gttcttttta tcaaataaac agtcacctct tgataaactt ttgttagaga 11160
atttctattt tgtctgcatt ttccttgcta gaagaacaga gagcactaca aaacaaatca 11220
taacaactgt gttctgggct ttggattttg ggtatgacag aaaaactttc tcaatacata 11280
ccttttaatt gctcttgcac aatctctgta gaaaattaca tacattcttt tagccatgta 11340
tcataggtgg aatctatttt ttagttttca tattcttagc ctagtcagtc aagaaattac 11400
gagactaaaa ctaactctag gaagtgaaga aatctgtcat tttaggaaat ggagagatct 11460
gtctcttcac tacatggggt gagattatca aatgggagtt agtaaatatg tttttctcat 11520
tctttaccaa atttagagaa aaaaataatg aaggtataat agaatatttc taggattcat 11580
cctcaaggaa aaaggaataa taaatggata ataccttcag aaacttgaaa gggttattta 11640
gatagattta atgagaggtt tgatgatttc taccagaaaa aaattgacat ttttataagt 11700
gtgtggtgag aaaaatcata aagtgagtca gataataaaa atggattcaa tttcaggaga 11760
tgagaaatgt ctgtactatt tgcacaaaaa ataattgatg ccaagagttt gtaaacacca 11820
ggctatatga tttaaatata ttgaaaaagt cattttctca tttcctgtcc attctgtaat 11880
atacttaatt tttgattcaa tagttctgtc aggcacattg tcccgtcttg tctctgtgcc 11940
taagtttcct catctgtaaa atggttcttg tgaaaatgga atgacatagt gcatattctt 12000
ttttcttttt ttctttttga gacagagtct cactctgtca cccaggctga ggggcagtag 12060
tatgatcttg gctcattgca gcctctactt cccaggctca agtgagcctc ccacctcaga 12120
ctcctgagta gctgggacta caggtgcatg ccagtactcc cagctaattt tttgtatttt 12180
tttttttttt tgtagagatg ggttttctcc atgttgccca ggctggtctc gaactcctgg 12240
actcaagcga tccactgcct cagcttccct aaatgctagg attacaagtg ggagccacca 12300
cacatggcct gaaaatgtat gatattatta aacagactgg cacataataa cccctcaacc 12360
aacattttttg ttagaaggac tctgtttttg catgacagta aacgtaattt gggttgaaat 12420
caaccagtga gctcactatt ttaacatgga aactctcatg tatcccaaag cactttttca 12480
tggggtatat gtgtgtgtgc aagtgtgtgt gagctactga aggctatttt cctttggaga 12540
aagcattctg agtaggagat aacattcatt tttacangca acttctgttt ttaacacttt 12600
cccttaaatt gtcgtgatga ttgaaatctc aaggcangtc awtgagcnag cccctaaatg 12660
ggagacagca gagttgctgg attctggtct ggaaagtgcc cagtgcgtgt gagaggctgt 12720
ggaggccgtg agaggtgacc tcatggaggg agaggaagag cgtgagcatg caggacacag 12780
atgatctggg ctcagagaaa gaggggaagt tcatacactc aagatgcaca gagaggacac 12840
tcagctgggc agagggaggt gcaggtatca gtagctctga ggctcaaatg ggccctggga 12900
attggaggac ggcagagtgg gcagggatgg tgacacatga ggcaggtgga tggatgttgg 12960
ataacagata ataagacaaa aatatggggt atttttcct agttgaaacc atagcataaa 13020
atgtatgatg ctacattcat ttgctctgaa tggacatgtc ttctgtctga gaaggaactt 13080
agcaagcaaa attgacagtc aaactttaca gaaacaaggc catcttgctg ttaagcatcg 13140
ccttttgtcg tcaagttttc actgtggtgt attgaggtaa aactgaagat gtaaatgtgc 13200
atatagggaa aggacaggaa gctcttgttc aaatgtagat tttagtggtt tgttgttttg 13260
agttctgtgg ctattggaac taaaaagagg aagaaataaa aacatttgaa ataagaatta 13320
atattgtaat gcctacatta acaaattggt aacatcaaat acatgtgtct tcaaaatatg 13380
gggtaatgcc tttctcagat acaaatttaa cactgggaac ctaatctgga aagctgtgct 13440
tatatcagct caccttgtaa ggccttgcac gtataagcat gtaaatgagc tatgggacaa 13500
gaaagacaag gaaggggcac agatgtttga aggtagtgtt tacttacagt tagganttttt 13560
ctgcacatgt gtatttattg acagaggatg atgttcacag aatatggcta gatttaaaaa 13620
atctggttac aaaaccatgt gtaagcagcc atttaaaaat aaatgtatgc atggaaaaat 13680
atatgtggca aagaaagtcc taaaagagat acaattcata tcctcttgga gagtaagcac 13740
cctaaagaaa attccacaag agaccataac taattacttc atctgcagcc ccttgttcgg 13800
tggcagacac aatgtatgtg ctcaacaaat tctttgcaaa aatgattgag tctctagaag 13860
tggaatccct gatggctttc atttttgttt cttattttgt ttatatagtt tttctaattt 13920
ttctacaaca aacctgtatt acttgtgtta atgaaaataa aataagaata gaattatttt 13980
cttctaactt aatttaaggt ttaagtattt gagatctagg caaagaaaga ccagtcagct 14040
ctcctggatt tttgcatgaa tctcattatg cccgatagca cagaaacrtg ttgggaatcc 14100
actgctcaat attccttgcc tagggaggta gtatggagcc agcagtaagg tactgaagga 14160
ataagaaaac cactagatgg acgttgaaga gaaagtgcgc cacactagag gctctctttt 14220
aattaatcag ctactctgcc tttaaataca gtatgttgtc ttccatgtca ggaacatgga 14280
gggaaaagac ctagtatgtt ttgttcaatg ttaaatccac gtcacttaac aaaatggtca 14340
ggatagtcaa aaaacaaaac aattatgcaa acatgaatga atgagaaact tctgctagca 14400
atgctggttg gtttcccaaa gacatgaaag tcagagtttg aggtgaagag tcccggagat 14460
gttattgaac attttctttt cttaatttcc ttctcaaact gtgccaggga tactaagcaa 14520
agagataaaa acccaccatt aagtgtgtat gtgtgtgtct gtgtctgcaa tagcgcggtt 14580
gataaacaat ttcacttgca ctcctaaaga ccccacagtt tatgcacaaa agttatcaga 14640
agagtggttg ttggtattga tgtattgaca accttaataa cacctaatga ttaaaaatga 14700
gtcaatgact tctgtcttct ttggggcttt ttagaatgaa atagatgctt tcattttata 14760
caagttgtat gtttatcttc tcacatcttt attcccaaat attaagtttt tcagttataa 14820
caaatcacac acatagacaa agtcttcatg agttaagtga gggaattagg aaagactgat 14880
```

gtgggattgg cattggataa caaagaggac atggcttgga taaagggttt gaattagttg 14940
agtaaaggtt gcaggatggt gaataatggt atgtttagcc aacctgagtg cagcatgtgg 15000
tatgagtgtg aaaggaaggg caggtacaaa tcagtgggac tcagggtcct ggaaacataa 15060
ggttaaataa agtaggcagt tcgtaagctt caagaatgcc ttcatattca gtgctgtata 15120
cccagcacat accgcagtgc ctgataccac agtggatgca taatatttgc tgtgtgaatg 15180
aatgaatgaa caaatgagtg agtgaatgag aaataagaag atacactgag gttttctggg 15240
ctacaaagga gacctacagt gtgtttggaa gctgattttt ttttatataa aagagggaac 15300
attttgaaag gcatcccaga cntgagggcc catggcctac ntagaaagga gtctagnaaa 15360
tggggatagg tgaaaaaggc cgnttgtaca tatatggaat gaatgagcna agaaaacaat 15420
ccactgctga gagtgggtgc aaagagaacc tgctgggcct tcccgctgca catggggaac 15480
ctgaagagtt tagagcagta gggtctggga catgagagga aggagaaaag caggatgcct 15540
ggctcatgtc gctctctcag gctgaatctt cattcatgat ggtgttggca ccgtcttcct 15600
acatctgcgg gggaagcagc accgatacct ctctaaatta gtagctggac acagatggta 15660
atggaaactg taacaacagg tgatcactta aggggttaag tagagaagac aggcaagaag 15720
ggcatgctgt gaaccataat ttggaagaca ggagaagatt caaaagactt taatactgaa 15780
cccaagagag ctgcaaggga aagaaatgac ccatagtgtt gcaagggcgc tgcagttaaa 15840
gaaaatggaa tattttaatt ttaatactgt gaagacgggg ctggaatgaa gtagggaaag 15900
tatataccaa tagaaatgaa attgctatca cagaatacaa aaactgggtc ttaaccattt 15960
aaaacagcaa tgaacatggg atgaatgagt ttcntgagtt gctataagct gtttctgagt 16020
aaaagattta ttaatggata cattgtgaac tgtgtagcct ctcnnccgat attgaantat 16080
ttaaatacca actactttta ataaatgtcg gaaacctttt gaaagccatg tttttcaaac 16140
tttgtgtact gtcttatgaa ggacactttg gtaaacagaa aaaagagata ttggcttata 16200
aaaacataat aatgataagg ctcagataca caggaancaa tttcctatca aaataataaa 16260
cttnacctag gatacttaaa tatccctact ggtttttatc agagaaataa taggtctgct 16320
gtatgcagtt ttattgattc aacatgaatg aaggcataaa acaatatttc tgtttgcaaa 16380
ggaaaattat tacaatcaac cacctcaaat taattttgaa ttcctcatgt gtttgtaact 16440
taaatttatt ttactacttg aatgctttat ataatattta atagtattaa attaaatcta 16500
gagatccagg agctagttgt tgagtttttta tgtatgtgtg gattagccag gtatctttgg 16560
agcttagatc atttcttggg cactgtgaga tttagtggca cctgggagca ggtaaagtga 16620
aggtaaatgt gagtgacagc taatgtcatt cagttataat tgcactattt tggttggtca 16680
gatataacca aagtgggaat tttttaaaaa acaataaatt ctggacatgt atttttctag 16740
ataaacaaat ccataatcaa aaatgatttt tgctttaggt ggcgtattga taataaaggt 16800
gcatggaatt catatgccca ttgtctccac tgctcagtga aagaggaatt gtggacagat 16860
ctctaaaatt ccttatataa tctttgaagt gaaaccactc ttttacttgc tcctttgtat 16920
gtgtcatctt gaaggatttc taaaagattg tatctacact cagtatttgg gtggatagga 16980
gactgtcatt gaagaggcat ctcaaattga ggccaccaga tttccttccc cactaccagt 17040
acatcagaga aaaaaaaaac actttaaata ctctgtatac attaacatgg gatatcaaag 17100
agtcccaaac tccaattata taacataaat taattagtgc agcctactgc aaagcccata 17160
ttcaaactca atattcagag caataacagc ctggaaatac ccagcatgtc ttcaagccag 17220
ttagcttgtt ttcttctggg aataggaggt gaatccgtcc gttttcacac ttctataaag 17280
aaatacctga cgacgctggc taatttataa aggaaagagg tttaattgac tccattctgc 17340
atggctgggg aggcctcagg aaacttacaa tcatggtcga agacaccttc ttcacaaggc 17400
ggcaggagaa agagtgtgtg aagtaggaac tgtcaaacac ttataaaacc atcagatctt 17460
gtgagaactc actccctatc atgagaacag catgggggaa accgccacca taattcaacc 17520
cctctctcca cacgtgggga ttgtggggat tataattcaa gatgaaattt tggggggtgg 17580
gaggacacag ccaacccata tcaggagagt taggcagaga tacctgccca ggtagataaa 17640
ggagcacata gcgatgggga taggtgctca atacaggcaa tcagattcgg agctgctttg 17700
ttctgagttt ctgtgtttta ttctcttttc cagtaaaaac tgcatctgtg ngcgcgcgca 17760
cacacgcgcg cgcacacaca cacacacaca cacacacaca cacacactgc aaacatgttc 17820
ttgtactcca gtgaggacta agtggcaccc attgtgcttg gttaggtttc tttctctaga 17880
cccatggagc accagatagg agagccagtg aaccccgcctg atgttaaaac tgcagtgcta 17940
cgttttcctg actcagcctc tttttttgc ctgaagcgct ctttttttc tgttacccgc 18000
cttgagaaat tctcctaata gtctgcagct tcagcatggt caactctaaa ccccttacac 18060
attgtagtca aacagagctt attttaaatt tcaagtttgc ttggatggcc ttggtcaagt 18120
tattacctgc cttgaggcta gcttttctca tttttctcat gcatgtgtaa gaagatgagt 18180
tttttgtttt ttgtttttttt ttggttaggg ctgttgtaaa gatcaaataa aatatcattg 18240
ctaagtgtcc caaacatagt atatgcccat agctgttagt tcctcacctt cctttaagac 18300
tcaatccagg tgctaatcaa atgggaagtc tcacagtcga ctcagggatc atcaggtacc 18360
acctgccttt ataacaattc ttctcacatt gtattgcact tatttgctat ttgtgtcctg 18420
agggctgaca tataatagcc ccccttcaag tgactgctga gttgtggaag gcagtagcaa 18480
tggccatagg gaggtaacgt ctatcacagg atgtagatgt ttcagaattg tggaaattta 18540
agcagttagc aggcatctca tctcccacct caatgtaatt tcatgaacaa tttacttcag 18600
agtagctgga agaatttaga taatcactgc attcataaaa caacatctcc atttagggga 18660

ggttcttccc ccattctttc tgacctccct cccctcccaa gttgggagat caacttgggg 18720
ctactgttgg aagtaagaga cgtctgtgaa tgtcgtatta aattaacact ggggaaacca 18780
tggggaagat ctgtgcattt catctgtatt actcagcagt gttctagcac aaagcacctc 18840
tatgtggaga cagctttagg tataagagca tacatctaaa aagattttcc caagaatatg 18900
tctgccctaa gaagcaaccc attttactct agctattcta aatagctagc ctctaaactg 18960
cttttgaagt acataaaata atccattgct ttgggagaaa aaaatcaagc ttctgttggt 19020
attaatttat ctcatccttt taaagtttct attttcatat gtttattata cactatggag 19080
tagaacacat atatgcctta caaataagta gatatatgtg gggaactcaa tttttttgatg 19140
agggcaagat ggtttagatg acaaatacaa cttttccttt tncccagact gtggttttgt 19200
gcttgctcac caaagctaac ctcagcatgc tcaaaaggaa gcagagttcc agggtggaag 19260
cccagccagt cactgacttt ggtcctgatg agtctctgtc ggataatgct gacatcctct 19320
ggattaacaa accagtaagt ttcttctttg agtatggaga agcatatgtg gatgtgagat 19380
aaatgtgatt gacaaattca aatatcatgt acctttggct tcgnaaatat ctgagctgca 19440
gtagaatatg gatgaggttc agagtctgga agtagagaac aatccaatga aatagtgaga 19500
tgtcgctatg agttactcac ataggaagat gcactgggta gtctgatccc ctcccctccc 19560
cacgctgtgc accctgtcct cagagtcaca tggtgtagag atgataccac ttctcaggct 19620
gatccaaagc tggggtccaa gatgagcaac ctgttctgaa tgaacctcct ttacttgttg 19680
tcaacgttcc tgaatccgtg agacaatatg cacatgtttg gaattaaagg tctccttctg 19740
atttgcaaaa tttgagtaaa aggtcaatga gataatggat ggtatctctg aagaactttg 19800
tcaccttcac cgcctaatta agttgatgag gatttgagaa gaactgctag gccaggcacg 19860
actgtgtgcc agaaagcata gtaaccagaa ctaagcctga caggcatgca tatccatgag 19920
tgctggaatc tcagctctgc aaaaccaagc aggatcaact tcttactcat ctcatggttg 19980
atgctgaagt aacaaaagga aatggcctgt tccctcttgc agtgcttgtc cttcccccaa 20040
ccatgataca cacatgatct gcttggactc tttgctgtcc ttcccagtct ctcttcctct 20100
aatctgccct ccaggttact ttccctgcct gctcacattt ctttctatat ttctattttt 20160
attcacatca cgcttctgct ttataatcat ggaagcatcc ctattacctt cattgcaaat 20220
taaaatacgt tagtgtgaca ttcctgcttc ttttcattca ggacctaacc tgtccagcaa 20280
ctgctccttt cctgtgtcag cacactgtcc acttgagccc catcagcagc ctcctactcc 20340
tctaggctct ctggattgtt cacattgctt tccctgctcg tactgctccc tggccccagt 20400
gacccttttcc cagctcctct gatgagtcaa atcctcctca tccattagga ctggatgaga 20460
tgtcacctct ggcatcctcc tctgaaaggc ttatttccgc cacagacaga atgtacctct 20520
cttctctttc cacttagcct gttttatatt tgtatgtaaa tataaagtga tctgtaaata 20580
actgtatttt tatatgtatc atatattatg tgtatattta cattatatac attgtaggtc 20640
gtgtgtatat ttatataata tacatatatt tatggatcta ttgtcttagg tatatataca 20700
tgttatatac acatacatat aaacatataa caggnttata tgtatatcac atatatacac 20760
attacacata cacatatatg ttatatgtgt taacatatat gcatattacc cacacatata 20820
aaatatcatg tgtatataan catatataca tgtcgcatat gcatatacat atatattata 20880
tgtatgtata atgtgtacat atatttatat gtgcatgcat ataacatgta tatgtaatat 20940
gtatatatgt tatatacgtg agactataca tgatatatgt atattatgtc ttatctagaa 21000
catgttttat aatgttatat aatgtgacat gtttttgcct cttgcagtgc ttgtctttcc 21060
cctaaccatg atacatgcag gatctgcatg gactctttgt tgtccttccc agtctgtctt 21120
tctttctcta atctgccttc cagactacat gtgtttacat gtatgtgtat ttacatgaaa 21180
cataatatat acatacatat aacatgctat gtgtatatgc acacattata tatatgtatg 21240
tgtgtgtata tatatatata tatatatata tatatatata tatatatata atacattcat 21300
aggtgtttta cttcttggac tgcaaagcag gagtcagaag tgtgttgctt ccatattttg 21360
ttctccattc ccagcattct gacacatagt aggccttcat tcatacttag agaattgaat 21420
ggaatagaga atgaagcacc cctaactttt tcttagtgtc catcttatta cagagctttc 21480
tacaaactta tgaagaaggt agtataaaag atgtggcctt ggaggcactt actaaaatta 21540
ataattcttc attaaaatta tgtctttaaa ctaaaaacac ttttgttgtt gaagaactta 21600
tatttggaag ctcttttgta taattagatt ctttatgttg aaagtccaga tggggctaaa 21660
ttcacacacc aatgtttatt gaagtgcttc caaattgcat gaaaaataca tcaaccttta 21720
aagtaaactg aatcatcagt ggcttatgtg gtatggaatt agcagcaaaa tatcttcatg 21780
taaatattat aactgtcaga taaatattat aactcctcag ctgcaagtgt ctgagtctcc 21840
tgaaagttct gacagtcttt tctantttt catcttgagt gtttngctag tatatatagn 21900
ctgcagaatc catctactac aagactaaaa tttttttcag agtattatat taaagaatta 21960
tcataatttc atatatgtcc cagatagtca ataaaaacgt atttggatta tgtttaagaa 22020
ttgtatacta attgattaac tggtaatatt aattgtggtg ggtttagcat ggagatgcag 22080
ttaagtattt ttttaatcct agatactagt tttcataaaa aaatattgag tatgagtgat 22140
tgtatgtcct gtatatatat tcatgttaac acacacttat ctttaattct ttcttcttat 22200
tttggggtta aagaaaatct tcagacagca gacatgcctg tagtgttgca cagaaagtta 22260
ttttgtggaa ttttgaaaga tgtctgggct aaatttgtat aaaatgtgtt ctccaaaata 22320
aagagaaaaa tctatgcatt tttttcaaat gtaggatgag accagatttt taaaaagttt 22380
ctttgtattt ttatacaaag gcattatata tcagatacaa atagaaagga aattctaaat 22440

atgggatgtg ttatatatca attcatttaa aaagcatttc ttaactacta tgataggccc 22500
ttgtgctaga tgctagggaa gttatggtaa atatagtatg cccctaattc taggtactta 22560
tagtacaaaa atgaaaaaaa agtaatggca catcaagttc ctatgtgact aaattacaaa 22620
cacatatttt aattgaacca gaatcttcct atattgtagt ctgatgccca ggaggttatc 22680
agatgtttgg gagagcctga tcaggttccg tctatgcttt gggtctttgt aacgcatggc 22740
cacagaaatg cctccacctt tctgtcttag tgtgtttatg tggaaaatag gtgccttgag 22800
aattaaatga tatnaaaaac gtgaaaatat ttgtagcaca ttatatgcac ttagatgtta 22860
gatttcttct tcttcacctc catattagta aactatttta acctcagtat tatgttttcc 22920
acttagtgtg gttcataata caagtttgag atatcagtta tggtgccgtg tagtaactct 22980
ggaccatcat tgcagtgggt tcactctttg ctgcgcatct gtgccatcat cagcgtcatt 23040
tctgtttgta tgaatacgcc aatgaccttc gagcactatc ctccacttca gtatgtgacc 23100
ttcactttgg atacattatt gatgtttctc tacacggcag agatgatagc aaaaatgcac 23160
atccggggca ttgtcaaggt gagcacttcc atgtcattta aactaagaac ctaaatgtgt 23220
tgaaagtctt aatcccttta atcatatttt cttgttcatt tcagtaaaac aaaacatcat 23280
tttatcttat acatatggaa gtccttttta aaaaggcata actccagaga ctgggagaaa 23340
ttgttgcatg agactatttt aaaagttaaa taaattttgt aagtcacaaa ctcttggaat 23400
atctgagaag gaacaagatg gtttgactaa tgactgtgtg aagtctcgag tagggttatt 23460
catgccactt agggctgagc ccacaaccca gcacaggggc tggcccagag aagcacctta 23520
ttatttgttg acttagggag tgcagaatca gagtcccatg atagagttag agcatgatat 23580
tattgcagca cgatgtcatt tacttgatgt gttttgaaaa tacaaaagga taaaaaaaaa 23640
aggacatgag atggaaaaaa tgtctggggc caaaaggaaa attatgtgtc atgtgaaatt 23700
agtaccaatt ggaaacaaaa tagatataaa atgttttaaa agggtaaaga aagggaaaga 23760
aaaggaagaa aatttctgtc actggatatc ttttccttcc atagcactaa tctttacata 23820
ggttaaacat atgtagatga tgctgtccct agagtattta aattgtacta tttggggaag 23880
cagtaatagg agtgcagatt ttagagtttg acaaatgaca gacgactcag atcttagttc 23940
tttcacttac tggnttgtat cagcatctca agttagctaa ttttggtgag aaagtgtttt 24000
attttgtaca aggattttga gaatctactg agacaatata taacacataa ttatttgtca 24060
atttcattgc ttccctttta tacataatta attgtgtgcc atccttgaaa gacagagatc 24120
acagacaatg cattaatagg ttcttgttta actttttatt gtggacttta aaaaatttac 24180
cccaaagtat attacagtta taatgtgaat ccctgtaact acacatcaac aattatcaat 24240
atttagccaa ttctgtttca ttttaaagca aatctgagaa accaagtcat ttcagaaata 24300
actccttttg tattggtgac tgataaaatt ttctcttttt ataaccacca taccattatc 24360
aaatataata atattttacg tatgataaaa tatttgtaac aagacttagc aataattcct 24420
taatgtaggc taatacttaa cccaccgttc atatttccta atattgtcac aaattttttt 24480
tatggttggt ttcttcaact caagactcag acaaggttta catagtatac ttgattgttt 24540
ttgttttcgt ttttgggagg gaggttcact tttgttgccc aggctggagt gcaatggcac 24600
gatctcagct cactgcaacc tctgcctcct gcattcaagc aattctcctg cctcagcctc 24660
ccaagtagct gggattacaa ggcctaagcc accaccccgg gctaattttg tattttcagt 24720
agagatgggg cttctccatg ttggtcttga actcccgacc tcaggtgatc tgcccgcctc 24780
ggcctcccac ttatgtttct taagtctcct ttaagtctct gtaagtccac taagtttcag 24840
cttttcacat cctgcaaaaa attttcctat gtcattgatc cattggaaga catgcgtctt 24900
ttgtcttgtg gaagattcta catttgagat ttggctgatt gcttccagat ggtgctcttg 24960
aatagtttnc tgtgtactcc wtgtncctct gaactgcttt ngtanntttt gaccagncaa 25020
tgcacacagg tgcttctttn cccacatggt cacctcnaaa atatgtcaga cttctggata 25080
tttgccagtc tgaagggtgg gaaacggtaa tccactgtag ttttaatttg catttccctt 25140
ttgagagtga ggttagatgg gattttatgt atcaagagcc atttttaagt tactattttt 25200
tcattcaatt ttggccactc tgtgtgtgtt agaagtatca gctgtttgtg atataagttg 25260
caattgtttt tcccagtttg ccattgtcat tggctttgtt tgtggttatt tgtaccatac 25320
acaattctta tccaaatcat ggacaaactt tctattgtct gcattttatg tcatacagga 25380
gagttttgta ggtttcctca tacagatttt gcacattctt ggttatattt atgctttggt 25440
atttatttta atcattttgt tatttgtatg aggtcttcta ttacatgttc taacttgttc 25500
ttttaaatga aggctgttgg tttctataca ttaatattat attctgctac tttactaaat 25560
gttctagatt cttgtattag tcataatagt tttcctgcta attcttttga tgcttccata 25620
tgatcatatc atattcaaat aaagaaagat ttgtcttttt ctttgtaatt tttatgccct 25680
agttgctttt acttacttaa agtaaaataa atgaattgac taacactgcc ggtaaaattt 25740
taaatactag tagaggtagt gaacattttt attttgtttt gtttttggtt tatctagaac 25800
atctctagtg tttcttcatt aactagttca aaaaattggg atttgaagta tgtgtacatt 25860
attggattaa gtgaataaac tttaatttca cattttgaga gttgtagcac aactagcttt 25920
ttaatttgtc aaatgtttat ttttaatttg tcaaattatt atttttttcct tagtgcttgt 25980
aagagaagag atttttaaat attgagttat ccttccattc cgataaagaa aaatccctac 26040
tgggtcaaca tacattttta ttctatttta atgtgttgtt ttgttatata tgttattatt 26100
ttccttattt taaatttggt ctaatctttg tcaggttttg atattagatt tttcatgtgt 26160
gtgggtggcg ggcatgtatg cacactataa aatagtctga gcactctcag aattgtttc 26220

actttaaaag tttggtatca gttcccggtg aaatcatctg gtcctgttgc gtttttgtgg 26280
gggttccttt gcagtatttc ctatttcttc tatggaaatt gtctgtttta tttttatttt 26340
tatttttat tttttgtttt ttttttgttt ttgtttttgt tttttcgaga tggagtctca 26400
ctcttgtcac ccaggctgga gtgcagtggt tcattttcgg ctcactgcaa cctctgtccc 26460
ccaggttcaa gcgattctcc tgccttagcc tcccgagtag ctgggactac aagcgtgtgc 26520
caccacgcct ggctaacttt ttgtgtgttt agtagagatg cggtttcact gtgttggcca 26580
ggatggtctc aatctcctga ccttgtgatc cacctgtctt ggcttcccaa agtgctggga 26640
ttacanggcg tgagccactg tggcagacct atgtcttcat tttttagtag agacaagatc 26700
ttgctctgtt gcccaggctg gagtgtagtg gtgtgatcat agctcactgc aacctccagc 26760
tcttgggatc aagagatctc ccacctcatc ctcccatgta gctgggacta caggtgtgtg 26820
ccaccatgtg tggctgattc ttaaaacttt ttttagagac agggtcttgc tatgttgccc 26880
aggctggtct ccaactccca gactcaagcg atcctcccac ctcggcctcc caaagtgctg 26940
ggattacagg cgtgagtcac agtgttcagt caaaactgtc tgtttcagac ttgcttggtc 27000
tattggggtc tatttggtga agtatatttt cttaggaaat tatcccctat ttattttgtt 27060
taattatttg gtaacataga cctgtgtaaa tagcttttct ttcctctgtt tgattttctc 27120
ctaattttgt atttttttgt tattttggtt agcattttgt tgtcatcagg ttggctagca 27180
gtatttttttg tttgttccat tttgttttag gttagccaat tggtttgctc atttgttgga 27240
tttttttttaa aaagaatcag cttaattaac ttattaattt ttgtttaatt tttttccttt 27300
aaaaatattt ttattatact ttaagttcta gggtacatgt gcataacatg caggtttgtt 27360
acatatgtat acatgtgcca tgttggtgtg ctgcacccat taacttgtca tttacattag 27420
gtatatttcc taatgctatc cctcctccct cccccgaccc catgacaggc cccggttntg 27480
tgatgttccc cttccntgtg tccaagtgca tttattagtt tctttctttg ctttatttgg 27540
tttactttat gggtctgttt ctaacttttc gaaggtgttt gtctcacatt ttcatttctt 27600
gagactctta aatctgctgc ttccttgcct cgtggttaaa atgttaacca caaggataat 27660
gttaaccagc agcataaaat gttactgctg agaatgtttt ttttccctcg aagtggcttg 27720
ctcttttctc ctgaatacgg aaagagactt tccctttatt gttcaagtca gcagctctat 27780
gagaatgtat gttatctttt tgttgatagt tacggatata ttttcctagt ttcatcatgt 27840
gatatttatt ttattgcatt gttttcagga ttccttttgt attctaatta taccaggatc 27900
tatggtttgg tctttccttt cctttgtttt ctttccatgt atttccctct gtttttatgt 27960
tttctacatt ctctttccct tgcttcactc ctcatgctgt cccctcactc ctctgtcccc 28020
tgtatttcac tcctctttct caaatacttt tttcatttct ttttcatcat ttcctgaaca 28080
gtttttgttc tttctttttt cagtctctta tcatttagac atcatttctg tgttttctta 28140
tttctagttt tgtgattctt ttggagcttc tatttgttta aatttctttt ggtcatgttg 28200
tcatattcga ttatagtctt catctgcttt gagtgtttat tttctgttta tacgtttatg 28260
tattatttta tttttacttt tattattttt tgagacagaa cctcgctctg tcacccaggc 28320
cggagtacag tggtgcaatc ttggctcact gcaacctcca ccttctgggt tcaagcaatt 28380
ctcctgcctc agcctcctga gtagctgaga ttacaggcac gcaccaccac acccggctaa 28440
tttttgtatt tttagtagag atggggtttc accacgttgg ccaggccagt ctcagactcc 28500
tgacctcagg tgattcgcct gccttggcct cccaaggtgc tgggattcca ggcgtgagcc 28560
accatgcccg gcctatgtat tatttcattc atcttattct gtaatgtttt tacaagggct 28620
tcatcatgat ccgtttgttt ggttgttctt cttgttgttc acatgaaatg ggagtttttc 28680
ccacacattg ggtagagtag cctctgcata ttgtcagtac gaggggtccc tccactgttg 28740
ttatcctgat gggtaaacat ggagcttttc tgtgtaccga ccgtcaggtt ctgagctctg 28800
ctgcctctgg actgctggcc caattatcga atatttctta cccactggcc ccagtgtccc 28860
tgccctactg aattgccatt ctaaacatgg ggctgtggtc tggaaagggt ttttgttggg 28920
tgtcctttgg agtttctaat attccattgc ctctgatctt tctgcagttt cttggggaca 28980
ttggtagtca tttgtttttg ttttgttcct ttgttttaca ttaacctgca acttgcagcc 29040
taccagttta catttctaca aatgttgcca tttattttaa ggttcacctt ctgagatatc 29100
tgctcagcat tccacacgta tttattagtg attttggggt tttggaaatc tactggttta 29160
tggaagaggt gggcatcctc catagcttca atgaacagat ttcaggatga ttccaaaatt 29220
gtctgtatct tccacaatgt ggctaggcta gtttttataa gcactaggtt tagataangc 29280
ataggttcag aaaactggaa cctgtctcag gaggccatct tgggagaaag ttggtcttgg 29340
gagaatgcta acatctctgt tattcacagt actcccctgg acgttgcccg gccctagctc 29400
acattctgtg tagttgcccg gccctagctc actagagggc catgaatggc cctgtcactt 29460
gtgtgtagtg gttattgggg ccatggcaac ctttgcccat gcattgaaag atctgaatat 29520
tctgggtgaa gcattttatg attgaaattg ccatgtgttg acttcttaag ggttccattt 29580
gaatgaaaca aataagtgac atgttcctac aagaaaactt ttgttttttt aagtcattaa 29640
ataaagactt agatttcaca tcactaaaat gatagtaggc attctgctag ttgtcacagt 29700
ctaatgataa aaagaacaga ggcatactta atatataaat tatatactca ttccaagtga 29760
acagagcagt gagagaacac aagttttaat aatgtaaatg ttagaagttg aatcaatagt 29820
taaaagagaa agaaatgact taggaccaaa tcgttttcaa aatgctgtaa attagccatt 29880
tccagtattt tcacatttcc atcaacattt gggtgatgag ctatttacta caaccaaaaa 29940
ttttttttt tattttcaat ctgtaagagg tgaaatttaa aatgaaaatc aaataaggtt 30000

ttctggtttt cattcagaaa taattggatc aaatacaact gtaatgcaat tttagtaata 30060
ttgcactgtt aacattatct tgaagtagtc agctcaactt catagctcag taaaactggt 30120
attctatgtt taaatgctag ctaacgtttt agttaaatat agttaaatat gaaatctgtt 30180
tatcctgaac caagctttaa atgtatcaac tcactaaatt tttaccatca tggcctctat 30240
tattgttaga gggtaataat tatatcagta agtactgtca ttttcatgat attaaatcta 30300
agccacagtt ttattatttt tatactacag acttcttcaa acatgcatgt cataacagat 30360
acagctcaaa gagtatattg ggcttgatat tattttctta tattcaaaac ttaaactttt 30420
aataagactt tattttagca aaatgtcctt tgtcacatta aatgtatgtt cctaatttat 30480
attttattag ctttgcattt ttgtatttgt aaatttgttt tgcttttata cttacatgag 30540
aaatttgtac atattgtagg tgtttaagtt acatcataat aaaaacacac tgtcacatcg 30600
aagttctata cctttcaggg gccctctcat attactcaag tttgagttac tatgctttct 30660
tcaactctac cataattttt aaaacttttg ttaagcactt gtctgctgaa aacctcatca 30720
tttttcatac tttttctacc ttaatacatt tgccaatagc actgcagtta tctctcaatt 30780
ctttagtccc ttncacccag aataatttgc aaatatgtgc tgcattttct cactaacatt 30840
taagcttgta ttttttcata cataaatagg aacgttatga atatacatgt tctattttaa 30900
tcttcaggtt acacttttca tagtaagttt tcacattgtt tggaacagag ttgtgcaaat 30960
atgaaatgtt taatattcac tgatttccaa ctaaaatatt tctaaggaat agtcaaattt 31020
agttattgag tgagcagtac ctgttagcca tgtgttctgg gtggaaaagt gtgtgtgagt 31080
tgatgtacac atatgccagt gatttgcgaa atgctaacct gntanttttt tttttttttt 31140
ttttttttga gacggagtct cgctctgtcg cccaggccgg actgcggact gcagtggcgc 31200
aatctcggct cactgcaagc tccgcttccc gggttcacgc cattctcctg cctcagcctc 31260
ccgagtagct gggactacag gcgcccgcca ccgcgcccgg ctaatttttt gtatttttag 31320
tagagacggg gtttcacctt gttagccagg atggtctcga tctcctgacc tcatgatcca 31380
cccgcctcgg cctcccaaag tgctgggatt acaggcgtga gccaccgcgc ctggccaacc 31440
tgttattttt taaagagtct tttccatgca taaaactatg ttagtccata agaaaatatt 31500
tatagaagca aaccaatgta ttttttgccc agctgtcatg cacagttctt taaaattata 31560
cctagccaat tttggcaatg ttatggtgta cctaagagtc atacctaatc gttaaagtag 31620
tgttatccaa tgatacctag tttataaaat tatgcacgac aaaccaaacc cagaaacttt 31680
attttttcaa aacaaacgtg tcaaagctgt ggcttcctag cattgcctac taagtttatt 31740
gacctagtaa tttgccaaat atattaaaat atgtataaat ataaatataa aatatgtaag 31800
atatgaaaat gtttaagata tttaagatat aaatgtaata taagaatgag ttatattctt 31860
aaacccaagt acagtttcca agttttcctt tagtcatcca gcaaccattt attgaacact 31920
tacaatgagc tcatctctgt ccttggagaa gggaaacaga caaaaccaag cagagctcct 31980
gtcctcagag aaaacctggt tcactctgtg ggacagactt agcacataca ggcactcttc 32040
attttcaaca tccaatatat ttctgaaaac atggtggata gcatattttc agaaaatagg 32100
cacttctctt tcactaattt atatgggaaa actaataatg tattagggga gctcgactga 32160
tttctccaaa tatcactgca ttaccatcta atacctatac aagtcaacct gcatatttct 32220
gcaaaataaa aagcatttaa aacatccctt acccaattat ttgcactaat caaaccacag 32280
tggatgtttt atatgtgccc aacaataaca ggaagtttct gttattaagt aaatttaaaa 32340
aatacattaa agtaccatcc accatatttt attgaattgt tgatatcatc aattgtaaaa 32400
aaaaatggca tcacttcaca gattcacggg tctctaagaa ataataaagg cttctaatta 32460
tactttgact gtaagatgta agattttaat tgaagcatca attagaagat gcatgctgaa 32520
tccagaattg tgcaactgtg ttggagcagg gtagggtgtg ttttagaatt ggtgaaatat 32580
gataaaatgg gcctgggttg gtaaactcaa tttttggatc ttttgaaagg ctaactttca 32640
ggggaaaggt aagacagagg aagatatcaa tgcactagta ttaaatagtt aagtaggtaa 32700
acaatgctgg tacaagattg ggcccaaaag gtgaacaaca gagatacacc tgcattgcac 32760
actatacctt tggcagaatc cactggcaac tttaatgtct ccattggcac acgtgcaaag 32820
tcattatggt ggttcttcta ggttcatagt gattctgggg caaacatttt ttgcagatat 32880
aaatagcaaa tattagtata atgagaatct acatagagag ggaccttctt ttttttttatg 32940
aaagggcttt aatagtggtc tgtaaaagct atgggggagg caggcataaa aaagaaaaac 33000
accaaaactg tctgcttggg gatatcagag aagtcttgaa gaaagaggaa cgtgaaacaa 33060
tgccttgaag gatgagtaag tgtggtctgt ggcaggggtg atggaagtaa gatagaagta 33120
agggatctca cggagaaaga aaaggaatat gaggcacaaa ggagtggaac agcaaggtac 33180
atgccgagct caaagtagtc agaagtggca gatgggctgt gcacaggagg cagctggaga 33240
ggagtttgtg aaactagacc tggctgcatc atggagggcc cagcaggctc tgcaaggagt 33300
tctgactttg ttctgtggtc agtaaagatg catggaggat tttcatctga gagtggtaca 33360
atctgatttg tgtttgaata gttcactctg gaagttacat gctggatgca cagctgggga 33420
gagaaactga agcagaaagt ccttccagca agaaatgtct ttaacaattt ttgttggagg 33480
aatgcatttg ttttaagtgt tttccatctt ttataatatt cccaaagctt gaaggtgctt 33540
gcccatcaat tcccactcct caacagcttt cttctaattg cctccctaat tcctcttgct 33600
ggaagcattt tcactcacca aatccctaga ggtgaaaaga aactatttgg atgatacatg 33660
tctgtgttgt tgcagagcac ttcatgcatt gcagactgtg agttctttag accattctca 33720
gttgtctatt tcacatctgg caatagatac aagtaaaagt agcatgcatc ctttaagcac 33780

```
tgttgaagta atatataatg atgctaagac atagtgacag atgggccctg gaaacttggg 33840
acaataaatc ctgttctcat gaaactgtac cagtgcatat cattttgtca ctgcaaaata 33900
tatccctata aatgtgtttc ctttttctgc ttgttaaata cttgcatgat atgttgttac 33960
tttcttacct tatcgttgat tggcagtctt caagtgttta ccgttataaa aattgacata 34020
tacagctctt gaagcactcc tctcctcagc caccaaagtc ctgatttttg cgcatagctc 34080
aagcactctc tgccctccat ttcttgttga tggaacaaac aaattaaaac attttttcat 34140
atttaaggtg ttaaaatatc ttgagtacca ctgggcattc atatgtgtta gcttctggtt 34200
gcatatttta gaattgggtt gcaaatatta taacttgagc ctcaatacag tagaatttta 34260
aaggatctgg ccatgcttat aaatggaatc aaagcaactt tgactactat ggctagggct 34320
tctgacgata atttgataaa tgtgccagat acaaaacata atccaagatt gtattgcaaa 34380
gtgtcagatt tcaaggtctg cattctactt atttaaaagt attataaact tattgtgcct 34440
tagcaattat tgataatgat taatatgata gtaattttaa ttcctaccag ttcaattgta 34500
tttacaatca tggtnaatgt ataaaatatg taaaccgtaa nctgtatttg catggcatgt 34560
ttnagttctg cnttatgcaa actatgctaa tccaggaagt atccataagt catctcataa 34620
tctcatctgt gatctttcat ggttacaaag gagccagaaa ggaataattt ctttttagaa 34680
gcatataaat aatttcagtg aatagatgga tggatggatg cattcattaa gaaatattaa 34740
ctgacatctt gctgtataca ttgtatatta actgacatct tgctgtatac actgtatcta 34800
agacatggtt catgtaccta tctcatcatt catttaatat ctgctatatg tcacccactg 34860
tactgggtgc tgcaaacaca accnttaaat tatccgactt aaagtctcag ggtgnttact 34920
catcttcaga atacactcaa acatttacca tgagagatag acttcttttt tttttctaa 34980
tgtaactcca gagaatacgt tcagagaaga tttaagtagg aaactcaaac ttcaccatca 35040
gatccaataa gttggagaga ccaaatataa attcatgaaa taaccaatat ggattacaaa 35100
ggcatattct gcgagtaaca caagatgcac attatttata tacatgagct gagttgtcag 35160
ttgttgcatg acgcctgagt taaggtgatc agaggaggct gtagtcagat cactctggaa 35220
ggaaaggttg gcacagactt ggcagaattt ttcgggaaga tggacagcat gaaaaacaat 35280
gaacattaaa aagccaaaga ttttttacaa atggaaaaat gctgagggtt gctggccttt 35340
gtatcttggg tcataactct gcttataaaa gatgtttatg aatattgaat gactcttgga 35400
aacatgagcc aaaaagtaat acatgaaact tatgcccaga ggattatttc catacttcac 35460
tgggaaaata aaaagaggtc aattctgatt gaccactgca taccatctag catgtgctga 35520
gcttgttggt gacacaggaa tgccactata tgtttctgaa aagtccaagg cagaagacat 35580
tccaaacaaa acaattaaat tacttcctat ggaaaagaaa gtttctctaca ttacatggta 35640
gaatttttgc taagccagtt tttataactg aggtcgttcg aaattaagat gtttgtatgc 35700
ttctaatttc tgatcaccac tggtgcttct aaaatgattt aaaataattc attattttat 35760
atagtttgaa atgtagtgtg ggccagtttt acaacattaa tttccttagc aaagaaatca 35820
gaaacaaaaa ttcagtcaca tctgttattg atagcttttc attctgaatt ggtgaggccc 35880
caatttttaa atttaaattt atttttgcca actggagaag tgattttgtt aaagctaaaa 35940
tgtccaactt aaaaaaaaaa ttgaaaagct atttttaaaa atttaacatg taagcattgg 36000
tccaaaaaca tatcagaata cattgtgaaa agcatgtctc tcctccacct ttggctcctg 36060
gccattcact tgcccgcccc cctccccata acacaaaggg aagtatcact gtttatcctc 36120
atttgttaac attgtaaatg tacctcattg taatggtgaa ctctcattca atgttccacc 36180
gagtttaatt catttctaaa gcaaattatg agcaattaaa aagagggtaa ttgtagtgct 36240
gctacatata acattgttaa tgacaaaaat gaaataaaat agtaatactg ttttgaaaaa 36300
gtacaatata aattcatcct atttgggtgc ccgtgtactc aacgatctat aattactctt 36360
ttcctgccta atatgtactc attcattgct cccaatttta acatagttct ctgctgagtc 36420
cccataaaat ttcagttttc gtttttggaa ttctttgcct ttttaaaaac aagtgacaac 36480
tctgcctagt tcttaaaata aagatgaagt gatacttttc tttttttttc cctaacacac 36540
aagtaaaaaa catttgttca tttaataaaa gtccatttga aaatatacac ttttattttt 36600
cttcatgaca cttgtcaaca tataacataa tgaatatttt atattttatt tcattcagaa 36660
tgtaaattgc ttaaagacag aagcgtttgt tttgttcacc ctcgtatccc tagttcttga 36720
acactgccta ggttaaaggg atgcttatga gctgagggaa agaaggaagt aagggaggga 36780
gagagggagg aaggaatgaa gggcacaanc tttaaataat tcatcttcta ttanccttct 36840
tgggtattaa tcaaatttag gctcagaaaa agtcaatttt atatgggcta tgttatctgt 36900
atttagatta taattaattt acattttcaa ctgtgcaata tacnaggcaa gtatgatgct 36960
tatagttaaa gatcctctgt gagatcttta acacaactat taaaaaatgc nactataaaa 37020
aaagttgaag atgactataa aaaatcctta cgttatttgt tatatatgta acctttgtga 37080
tgcgtttatg tgtagcacat aaacaccagt agcatcggta gcatcctaag atgcagcttt 37140
tctcttcctt gggtataaat cttcatatga tgataatcac acaatttaat aaggccattg 37200
tccttcttgt cacccagagc caagcttcag agtttgcttt catatctctt ttgtgagtct 37260
tcccaaaccc aggagtcacc aagtttggtt gattaccttg cccctgaaaa cattatttgt 37320
ccgttcattt gggaattaac attggtttct ctaaactccc catagaatta atcttaccat 37380
ctttcccacc atgtttatag ctttaagtta ttttgcttta tatactagct ggatgtgttc 37440
acatctgttt ttctacaaga ctgttctttt ggttgttggt ggtggtgatg gtggtgtgtg 37500
tgtgtgtgtg tgtgtgtgtg tgttagctag gcattatata tcctctaatg taaaacaggc 37560
```

acttacccag tgttcattaa gttgagttta tacttcattt ttgggtcaaa taacttccat 37620
taagtgtttt atcatagtgt tagttttgca tacaccataa actatttcca gaaacctctc 37680
aaggagatat ttggatactt atttgttact aaaggtagag gcttatgtca atatcctgca 37740
acatgtttta tcatttgatt tgaaatcttg acattttatt tctattttca tttccctagc 37800
aggtttaatc agaggaggta atgatatatg tgactgaatt cagaattgac ttacatggat 37860
gattacagaa ttcatgattt aatgggatag tcctaaaaaa gcaaaggctt ttctgtttaa 37920
agtttctaat ttttaacctc atgaagtttt ctgttttatt attccaaagg catttttttaa 37980
attcccatgt tattaaagca ttctaatgtc agaatagata ccctattaaa aaatgaaagt 38040
ggtacatctt tatctttctt ttacatatgc aatcccaatt aaaattaaag tcaatatgtg 38100
ttttccttgc aaattaagac aagtttcttc ttcacttttt tttgttagtc ttgaattcta 38160
tttttattac actgtggtcc aagagaatgg ttgttttgat ttcagttatt ttgcattttc 38220
tgagagatgt tttatgtctg attaagtgtt gatgttagaa tatgtgctat gtgacaatga 38280
gaaaaaatgt atatatatat atgtgtatat atacatccac atatatacag tggaaagttc 38340
tgtagatgtc tatcaggtgc atttgatctt tggggggtttt tgccttaatg atctaatact 38400
gtcaaggtgt tgaaatctcc caatattatg cagaactgta agtctctttg aacatctcta 38460
agaacttgct ttataaattt gggtgttcct gtgtaaagtg catatatagt taggatagtt 38520
gggtcttttt ttattattat aaaaaatttg cagaatgtgc agttttgtta cacaggtata 38580
cacgtgccat ggtggtttgc tgcaccatca acctgtcatc tacgttaggt gtttctccta 38640
atgctatccc tctcctagcc ccccacctct tgacaggccc gtgtgtgatg ttcccctccc 38700
tgtgtccata tgttctcatt gttcgactcc cacttatgag tgagaacatg cagtgtttgg 38760
ttttctgttc ctgtgttagt ttggctgaga gtgatggttt ccagcttcat ccatgtccct 38820
tcaagaaaca tgaactcatc ctttttttatg gctgcatagt attccatagt atattgggcc 38880
acattttctt taaccagttt atcattgatg ggcatttgga ttgattccaa gtctttgcta 38940
ttgtgaatag tgctgcaata aacatacgtg tgcatgtgtc cttgtactag aatgatttgt 39000
aatcctttgg gtatatatcc agtaatggga ttgctaggtc aaatggtatt tctggttcta 39060
gatccttgag gaatcgccac actgtctttc acaatggttg aactaattta cactcccacc 39120
aacagtgtaa aagtgttcct agttctctac atcctctcca gcatctgttg tttcctgact 39180
ttttaatgat caccattaaa ctggcgtgag atggtatctc attgtggttt tgatttgcat 39240
ttctctaatg accagtgatg atgagctttt tttcttcttc actttttaat cagcttgaag 39300
tgaatgcctt gttagttttc ctactaacgg tgctaaacct agcagtttta aagaccttcc 39360
tgttgaccac agtgcactct tggttcttgt aggcagggag ctggcttagg acctacatag 39420
catatttgga acaatggctt ctggttcttt aagtgttaga atttgtgtgg ctgagaaaaa 39480
aaaaatagtg atattttccc tttgcatgaa ttaaatattt tacctgcaaa gttgcttgtc 39540
ttttataaat tatttttctt ataagtaatg ctacttaaaa tgctaaactt attccttgtg 39600
aaagtctgtt ctattatgat acaaaaattg tagatagctt ttattgaaat aatatttgac 39660
attttcctcc acagattgtt ctttaaaata tttgctttgc ctaataatgc catgtaaaat 39720
ttatttttag gggggatagtt cctatgtgaa agatcgctgg tgtgtttttg atggatttat 39780
ggtcttttgc ctttgggttt ctttggtgct acaggtaatt aaatattttt taaattacaa 39840
gcaggtattc tccaaaatgg gnaattattt tgcattaaaa ngtctaaata gaaantatta 39900
canaaatata tatcaaatgt ngattgtgta attgcctaca acattttnat tttnnatttt 39960
tattttaaca gatgtgatta gtctgtttac tctgacatat ttgaaatttg aaaaattaat 40020
tttaattgat acgtaatact tgtatgtatt tatgggggac tgagtgatat ttgatgtata 40080
caaggtgtaa tgatcaaatc tgcataatta gcaattttat atcacctcaa acattttatt 40140
gttagaaaag tcaatagctg taatatctta attattgtca gttatgattg tgaattattt 40200
ctcaatcaga aacgaaaatc aatactatag aaaattatta gctgttaatc agatataaaa 40260
tatattccct gaaatgaagg aaacaatgtg gagcaatatt tctgtatttg taaataaaaa 40320
attgaactta aaaatttaaa gtctagtctc cccatatttg gataggtatt agccaacata 40380
aaatactata ttgcaaatat taaatgcttt attataagtg atggggaacg tacattaaaa 40440
agacttacag gacagatttg attggaaaat aagtagaatt aatttctgtg cattcattca 40500
gacatgattt tgccttttaa ttcaactaaa gttactatat tctattgatt tgatttgatc 40560
tgttttgatt tggttgtggt aagaatgcct aacatgaaat ctactgtttt aatgaatttt 40620
aattgtacaa taaattattg ttaactgtag gtacaatgtt gtacaggtct ctagagttca 40680
atcatctatt ctttatttaa aaattcagaa ttcaaaactt cacagcccat tctgccaact 40740
catcatagtg tttgttgata agctttcctt tcatattttt ataaagttca cctctcccac 40800
aatttaattt catcactcat atattttttt aatcagcttt gtcaggttga attgtttctc 40860
acattaatgt gttcttttcc atttaataac tattatgtga ttgacaaaca tttcaaatta 40920
ttattttgcc tgttagtaaa tagtgcaatt cttttttaat attttaaccc tttctctatt 40980
actttatttt ctagatattc aaaagcctcc actaaagaac gttattaaca agaaagacag 41040
catacttact atatattcta agttggcact tcttgtaact aaaaactgtc ttaacgaacg 41100
agacaggnaa atgaataaga ttaatgttgt tcttattagt ttatgtattt gtttctgtta 41160
agatagctat agcttcaaaa tatactcaaa agaaagtgga ttttggtgga aaataatgta 41220
tggctaaatt cagagtagag gtgcacctac ttatgctgct aacatctgta tgttccatga 41280
ctataagcaa tgctaaggga aaatccaacc tcatttaccc atcttttaat ttgtaggtgt 41340

```
ttgaaattgc tgatatagtt gatcagatgt caccttgggg catgttgcgg attccacggc 41400
cactgattat gatccgagca ttccggattt atttccgatt tgaactgcca aggaccagaa 41460
ttacaaatat tttaaagtga gcgctgcttt acaataagtt aagaggaaaa gtttatgaag 41520
tttttgtaa gttttatgta ctttacctac ttttgtttct ctaggcgatc gggagaacaa 41580
atatggagtg tttccatttt tctacttttc tttctacttc tttatggaat tttaggagtt 41640
cagatgtttg gaacatttac ttatcactgt gttgtaaatg acacaaagcc agggtaagtt 41700
tatctattaa ctgcatttta atctagttga ttaacaaaga tatctctgta attttcataa 41760
gaataacctc tatgtcagat tttttaaaca gtgcttatgt cctctttgtt ctgtgtctct 41820
gcttcctcgg tattgttttt gtccaatttt tattttattt tattttattt tatttttgag 41880
atggagtctc gctctgtcat gaggctgggg tgcagtggcg cgatctcggt tcactgcaac 41940
ctccgcatcc cgggttcaag cgtttctcct tcctcagcct gccaagtagc tgggactaca 42000
ggcgtgcacc accatgcccg actaattttt gttttgtcca atttttaaag acaagtattc 42060
aagacaggac agattggaaa atgtaacctg gtggaaaagt agattaagca aatgcaaagt 42120
gtacatttag aaaccttagt gtggcaaaat ggggagtgtg ctaacataat aacataaacc 42180
tagagcttca ctgtggaagc ttttcatatt agaatgagtt acatgttcgg gaaaaaaagc 42240
tcatttttat tattatttac ttttgtcatc attccctaaa atagtttcct aaatgattat 42300
aacatcattt ctagactaaa cactattatc tgttgcattt gactgttgta gggttggtgg 42360
gtaagataga atgtatgtac tttaattacc tattatgtat tatgcaatag agcacctgcc 42420
tcttctactt ggctgatcat tgttaatata caactccagg gatgtgatgg caggagagag 42480
cagagtggga tgttggcccc tggatggtcc agaagtgtcc cattaggtgt tagctagata 42540
tagagtttgg ggttccaagc acaacccaga angtgcaaca gatatgacta tgccagattt 42600
cagggcagca gggattgggg ataatacaat tgattttata ccttatattc aaatcttcac 42660
ataggaattt gtctatttat aaatgaagaa ctcatccata aattaccgat tgaaaaatct 42720
tgattcaaat cttagaaatt agatttaaag cactttgtaa aattcaaaaa gagattacaa 42780
gcagccattc atcttcaaaa tatttttaat atatcaatat ctacttacaa accaattggg 42840
tatataagga tcattgagtt taaaaacatt gaaagggtta tgttgtgttt tctgcagttg 42900
gaggatttct ttttcataaa gtctatatac ttgtatatat acaaagtaaa taaaccacaa 42960
ataagtgaat atctctgtga gtatagacat catgataagc ttggctcaat tccaacactt 43020
tattttatga gtttgcataa attatttagg ttactcctca atgaagtaga ttaataacag 43080
gacatacttt ctagaggcat caggaagagc aaatgaggga atgaatgtaa agacaagcaa 43140
agtaaccagc acatagtaaa ggcattcact gtatgccagc tgtcattatt ttgttgtcat 43200
aaagagatac gtaaacacgc ttacatgctg aggtggtagg cataagtgta tcctccaaaa 43260
tagagataac atggttaaaa aaaaggaaag agcaataaag aagaccaaaa tttaaacttg 43320
taagagtgtg aaaggaaaac atctatgagt aactcatggc tatgttattt tactgacaaa 43380
gtaactaaat taattctgag tttatttcac atttataatg gtncatttaa ttattagtct 43440
ctcacagaaa taaatttaat aattgaattt ctaattcatg cacaaacagt atatgtatat 43500
tatgcaactc ctaataatta tataatgttc taaaaactaa aagtgaattt agttgtttgc 43560
tgttgcacca ccccaaagaa tgtcaactcc aaaaaggtag agattttgtt tccacgtgtt 43620
ttcagaccag gaggcatgtg caagtttcta ggaaaatatg aatgtattca gtaacttcaa 43680
aacatcagta taatttcact ggttaaatgt taaacagtaa agcataatct attagtcaag 43740
gccttaatta attttatttt tatttagaag gcatgcatct catacctact tagaaattta 43800
ccagaggggg ctgtgcacag tggctcacac ctgtaatctc agcactttag gaggctgagg 43860
agggtagata acctgaggtc aggagttcca gaccagcctg accaatacgg tgaaaccccc 43920
tctctactaa aaatatgaaa attagccggg catggtggca caggcacctg taatgccagg 43980
aggcacctgt gatcgggagg ctgagacaag agaattgctt gaatccagaa ggcggaggtt 44040
gcagtgagct gagatcgcgc cactgcactc cagcctggac cacagagcaa gactccgtct 44100
caaaaaaaaa aaaaaaaaaa angaaagaaa tttaccttag gttcacagaa gtgtggacag 44160
ataccatgcc ttccttgatt atccatagta ttatgttgaa gtaaagatct cattttttct 44220
tgttttttaa tataactttt atttttgcat gatgtgagtg aataatctat tcaaaattat 44280
ttttaaaaga aaaatactgt tgctactaac ccactcccta atcttcatta ttattgttca 44340
aaatagcctt ggaaaataac tttttgataa ccattttctg gacatattat tattttcccc 44400
tattattgaa attatgtctc tactttaacc actttattga gattttggtg aatataaaaa 44460
tctttacata tttattatat atgtcttgag ggaactattt cctttttctg tcagaattaa 44520
atccagagag taagttattg catttattat tctacttcct gaaaattatg atccttctca 44580
aattaacttg tatatttctt atgaggtact tcctgaaaat aatgatcctc ctcaaattaa 44640
tttgtgtatt tcttatgagg tacgggtcaa gatgtagtct taacttagtg gttggaatgg 44700
acatcggtat cttaggagaa gctctcagta tttccgttaa gtgggatgtg agctgtagag 44760
ttttcaaagt gaggaagtta cctttaattc ctatagtgcc aagagttttt gatcattgat 44820
gtatgctaaa ttttgtccag tgcttttccg ttttgttcag ttaatgtaat gaattacatg 44880
attgactttt taaatagaaa ccaatcttgc atttctggta tattgctaat ttggtcatat 44940
tgtattcctc tttatacatt ggaagatttg atttgctaat gtttttgtta caaattattg 45000
catctttgtt cacaaggagc attattttta ggttttttaa attgaaatat ctttaccggg 45060
tcttgaaatc cgggtaatgc tgtcctcata aaacaagtgg aacatgtttc caagggttaa 45120
```

tataagattc atattctttc tttcttaaat gtttgataga attcatgagt aaaactcagg 45180
gcctgaaatt ttctttgtga gaacgttttt cattacaaat taaatgtatt caacttacag 45240
agctatggag ttgttatctt tcttgttctg ttaagttggt aaattttgct tttcaaagag 45300
cttacctatt tttataattt gtcaatattg cataatgttt atacaaatgc cctctgatta 45360
tcttttgaat gtctgattat ctttttaaca tctgtagaat agtggtaata agccattttt 45420
tcctgacatt gataatttgt acctttctct ccattttttt atgtcttctc aattgtatta 45480
ttcttttcac acacacacaa gaatctttgc cctatttaat gttctctatt gttagtctgt 45540
ttttcatgtt gatttttttat cttcattatt tccttatttc tgctttttag tttttgtttt 45600
ctttttcatc tttcataaaa tgaaggtata tagacatctt ttaacatttt tctaatacaa 45660
taatttaaaa atataatttt cctctaagca ctgttttagc ctctaagcac tgttttagct 45720
gcatttccaa aatgttgata tgttgtattt ttattattat tcaattcaaa tatcttctaa 45780
tatatgtgat ttctcctttg acctgtaggt tatttagaag tgcattttta gaaggatgag 45840
atttctaaa aatgttattt gggtgcataa ttttattctg ttgtggtcag acaatagtcc 45900
ctgtgaaatt tcagcctttt gaaattcatt aggaatcatt ttaaggacca gtatatggtc 45960
tgaattggtg aaaattccat gagaatttga aaagaaaaaa gtgaaatctg cagtttttga 46020
gtataatatc tataaatgtc accaaagtca agttggctga taatttgttc tgggtatttc 46080
tatccttctt ggtttttaaa atcagggttg ttctagcaat tgctgagaga gtactattca 46140
attttctaga catgacagaa attttcaatc tctcttattt ttgtcagttt ttgctttcta 46200
taattttcaa ctttaattag gcgcataacg tgtcattgta tctttcagat gggctgacct 46260
ttttatcgtc atgaattttg aattttttttc ctaattagct tcgggatgcc agggtatatg 46320
atgggtcaag aacatgacgt ttcaaatttt tcagtaatca taactctaaa gtaatgtgta 46380
actctaaaat aatttatttt atttaatcat cttttatctt attaatcaat tgatttgttc 46440
ccacaattac ttacgtgtac tttagaccat tcctggattg acagtaaaag ggagcacatg 46500
acaaattctt agttttagag catgggctgc acaatcctga gcccagcctg gtggtgatga 46560
aattaagccc agtactaaga gcgtaaatga agaagaaact cagtagtaag agagtgtgga 46620
ccaactggtc agatgctcta atcagcaact ctcattccac tgtcaaaacc ttggagattt 46680
tgtatgtttt ttaaataggt gaagtgaatc aagtggtgcc taccaaattt attttgtcct 46740
ctgcatcagt gcgtggcata caacattaac aatgagaagc aactaactcc tataagctat 46800
tttgggggaa tagaggatgg tacaaatatc acaactttac agcaaatatt acaactcctt 46860
caaaacacag tttggcagtt tttaaaaaaa taaaaataaa agagttgatc aagctgggta 46920
cagtggcaca cacctgtaat ctcagcactt tgggagactg aggtgggatg atcacttgag 46980
cccaggagtt tgagactaga gtgagctatg attgcgtcac tgcactccag cctgggtgat 47040
agagtgagac ccagtctcta agaagtaaaa atgaaagaaa gaaaaaaagt tggacaggca 47100
cctgccacat ctcatttatt ccactccagg tatttaacca aaataaataa aaatgtatgt 47160
ccatagaaag atttacacat gaatgcccac agcaacttta ttcatagtac tccaaactgg 47220
tgacaaccca aatttccatc aacagataga taaactaatg atggtatatc catataataa 47280
atactgttta ataataataa ataatgaact attgatgtat acagcatctt ggataaatct 47340
caaaataatt atgatgactg aataaagcca gatttaaaaa gaggacatgt tctattattc 47400
catttacata aaattctaga aaatgcaaac ttatctttaa tgatagaaaa gaaatcagca 47460
gttgcctagt gataaaggtt gtggtgggaa ttaagggatt acaaaggggc ctcaagaaac 47520
ctttggggtt tattgacatg ttcattatct tgactgtgct tctgtacatg tgccaaaact 47580
tatcaaactg tacactttcg tatgtgcagt ttatgttatg tcagttacac ccactgaaac 47640
tgcttaaaat gtctcagtgt gaagtagaaa tctaattttt catatggtta agcaattgtg 47700
cctgcactat ttattgtgtg ctattaccca ctgagtttag tttcacctgt ctcattcact 47760
aattccctgt ctatacgtgg gtctgttcct gtggggctct gttgtgcttc actggtctat 47820
tggactacct ctttgccaat atcacactct gaaagtcttt cagattacaa tctggcttta 47880
cacatggggt catatttcca gctttcagct ttacatgggc tcaagacctg gtcttccgcc 47940
tcacatgggc attaaaaccc aggcattagc tcccattcat aacactatgt caactcatgt 48000
tccttctgtt cctactcatt tactgctttc cttcttatgt tttgacattt tggaatgcag 48060
gttttgtatt gtgagcctaa atgcggagtt tttaatgcta tctatttgtt tgtagtggaa 48120
ggagttgaat ttggtgtagt ccatcttgcc acaaccagga ctcacatcca tagctatttt 48180
tttttctatt tttgtctaag gtccttgagg acacgtatga gaaaggtaaa gaggatattg 48240
tgttatattc atctttttat ccttactacc tagtatccta acacagaata aataattagt 48300
aactatttat tgaaggcatg gttgagaaca tattctattt agacagataa tattaacatg 48360
atgacaaatt gataaagaag atgaaataaa aaagaaacag agaaaagtaa gttgctaaaa 48420
gcaggaaagg agaaatggct aaaaaataga aaaacaaagt ttaagcagaa agaagcagtg 48480
aattagttca tatacggagt aagaaaattg aagcaagtgg tgagtaaggg agttgactgt 48540
gtgggtgagg gaagagacaa atgcatggaa gaaaatctgt aggtagttgg atttattttt 48600
ttagtgtatg taggctgtag aatatgccta gattgttcta tgagtgaata tgaaaatgga 48660
aaaagatatt gtgtgggttg gagacaatca atatggagat gaagaacagg gaaatcttct 48720
ggataagaaa gcagctgtgt agtactgcag tgggtacaga ggctggcttc aaattaccgt 48780
tttattaaaa ccaagtcaga atgtgagagt tataaaactt gtaaatgcag ctagaagatc 48840
aggcattatg ctggcaaacg aaagctcata ttggcacata aatgatcgca gaaattgaga 48900

acagatacac gaatgtcttc tgaactcatg accctagagt tgcttcagta agtattcttt 48960
gctagttcca cgctgtattt ggtaccaaga aaagaatgat actgaaaaaa tgcatgaaaa 49020
catgccttgt gatctaaata ttagctccag ctcattactt tattctaaaa atgaagttca 49080
ccatgaaaaa tttcataaat gttgacagaa tgcttagtta cctgagacat agccattatg 49140
gtgaaatggc tgcattttc tctttcccat atgtgtaaaa cctgcctagt agcatacaca 49200
cacacacaca cacacacaca cacacacatt tattttgtct tctttttatg tgactctttg 49260
tttactattt aagaagaaaa tgcttaacta gccacatggt agtaatgttg ctcataatca 49320
caactgttat agctttgata attcatttca agaatctatc aataatatct tttttgttgt 49380
tgttttttttg agacagactc tcactctgac acccaggctg gagtgcagtg gcatgatctc 49440
agcttactga aacatccatc tcccaggttc aagagattct cctgcttcag cccccccaagt 49500
agctgggact acaggcgtgt gccaccacac ccgggtaatt tttgtatttt tagcagagac 49560
agggcttcat catgttggcc aggctggtct cgaactcctg acctcaagag atctgcctgc 49620
ctcagcctcc caaagtgctg ggattaaagg cctgagccac catgtccagc cttattttta 49680
aaaaaaaatt tcagttcaat attactgcat tttaaagccc cacacataag cagaaaaact 49740
gtcagcacca ttttaccgtt actattagcc cagacataaa tgtagtttta ctcttagaga 49800
ctcaagaaat atccaaactc aaaataaaac agaaaatgtt aattgatttg aaaattttta 49860
aacttttctg tttccttttg tagcctctaa actatgactt tttttgctgc tataaagaca 49920
catgcacacg tatgtttatt gcagcactat tcacaatagc aaagacttgg aaccaaccca 49980
aatgtccaac aatgatagac tggattaaga aaatgtggca catatacacc atggaatact 50040
atgcagccat aaaaaatgat gagttcatgt ccttggtagg gacgtggatg aaattggaaa 50100
tcatcattct cagtaaacta tcgcaagaac aaaaaaccaa acaccgcata ttctcactca 50160
taggtgggaa ttgaacaatg agaacacatg gatacaggaa ggagaacatc acactctggg 50220
gactgttgtg gggtgggggg agggggggagg gatagcttta ggagatatac ctaatgctaa 50280
atgatgagtt aatgggtaca gcacaccagc atggcacatg tatacatatg taactaacct 50340
gcacattgtg cacatgtacc ctaaaactta aagtataata ataataaaat aaaataaaat 50400
aaaataaaat aaatttcaaa aaagtcaaaa aaagaattcc ctgtgtaaga acattcaaag 50460
tgaaaggagc atcataggga aaagacaaac cgtgaaagca gagaaatagg agacgccaca 50520
tcaagacacc accatgctta caagttggca ttttattctg aagaccataa tgaactgcca 50580
aagaagctga aacctaaaat aaaattattg tttttacata taaagatgat ttttcacatc 50640
tatatttatg atctcacctt ttaaatcagt atctactttt gaaaatgtac atgtaacctt 50700
caacatatac acagaaaata agataaacta atttatatag ttttgtttca atcctacaca 50760
attagagttg atttttactc catatttaga tttccagaat cccagctcat taatttaata 50820
tttattaagc ataaaagctt taagacctag aatgcatggg acgtggaatt aagcactagg 50880
gataaaagag gaagaaaaaa ataggtcatg tcctggagga gctcacactt gaggcagcca 50940
aagattaatt accttaggca ttgattagta ataataataa taattagtta gaaagtgtta 51000
tcagatgtca aaaaagagag aaaatgtgag ttttatagtc tttgtaaaga atatgacctt 51060
tgagcaaact ctagaattag gaatggagtg tttgaattgg aaattaaggc aggataatac 51120
ctttcagatg aaacaatgta atagagtact aggatgtata atagaatgct ggccccaaac 51180
tgggatggta gatttaagtt gtgagtttct ttctacaact gcagagtcca ttgaataaat 51240
acaactttat atattaaaat taaaaaaata aaaaaaaata aaaaaagaaa ttctgtcatt 51300
tggaataaca ggaatgaatc tagaggacat tatgctaagt gaaataagcc aggcacagaa 51360
agatcaatac tgcatgatct cacgcatatg tggaatccta accagttaat tcattgaagt 51420
agagaggaca atggtggtta tcaggggctg cggcgagaag gagatggagc tgcaagggaa 51480
tgaggagctg ctgatcaaag ggtacaaagt ttcagctagg cagtaagagt aagttttgag 51540
atccatgcac aacagggtga caatggtcaa taataatgcg ttatatatgt caacataact 51600
aagaaagtaa atttcaaatg tatcatcaca aaaaagttaa gtgatatgat ggatatatta 51660
attggcccga tgtaaccatt atgtcttgta tacatacatc aaaacatcac atggtgctgc 51720
attaaagtaa tacaattatg atttgtcaat taaaatacta ttatttaaaa aatgtattat 51780
acacatctcc gagtcacata ttaagctaag gtatgatggc tgtgttttca gcaatcagtg 51840
ttaccagcta ctgccgtgac ctcatttctc ttaatcattt aagagaaacg attaagagaa 51900
gttgttaaca attgttccaa gttctgccta attgatttgc atgtattata gcctaatcat 51960
gacagagatt tcagaccaga aacttgcata tcttgggttt catagttgac ctccagaact 52020
gattgtgttt aatatgtgag gcaaacaaga taaagaagca acacaatata tacagttttg 52080
cttttaaatg gaacttagtt cctgtaaggc ccaatagcag gaaatcttag ttatgtcttc 52140
agtagcagta ctagtaaagc tttagattct tctctgttaa tgaatggaga cctaaggcaa 52200
ttatgaaaag agtaatgaga tttgactaag caaagcagct catttcctaa agaaagttga 52260
ggagagcaat ggcttgtagc agaaacaggt gttgtgaaat accaactgtg atggcagaaa 52320
tctccccgaa gctggaacag gaatgtttaa aaatgagctg gcaggttaaa tagaaaagat 52380
gaagacttac ctgacacctt attatcattc ctgaaaaaga agggaaacat attcatagct 52440
cggtatcaaa taatgaaatc ttatttccaa gagtgagata attagaaaaa ctttaaggta 52500
agtaagtgta accattaaat atatgaaagt ttaatataga gagagcagga ttcaaacagg 52560
aactcaggaa catattcgcg atggctggac cactagcttt aagcccagag gttgggtgat 52620
cacacatcac agatgcccag gacaatcttg gcttatccct actgtttcaa agtaatcact 52680

aacagcctgt tattcgattt taaggaatct caatgtagac aatgcatttt atggtcacct 52740
tgcccagggg aagtttatag aacttggtat tttataaaat atcaccaaga gttgaattag 52800
agataatata aagttggatt tttttgcagt ttgctcttgc tggtccaaat acattttaca 52860
tttattaaaa taaatacatt aaaatttatc aactaaatta aaatttattt gtcacgtatt 52920
ctttagggta cttggcaaat aagtgaagta ttgtcttata aattttgttg acaaaataaa 52980
gaggtttttt catggaatgc tatgcagcca tgagaaagaa tgagactatg tcctttgtag 53040
caacatgaat gaagctggag gccgttatcc taagcaaact aacacaggaa gagaaaacca 53100
aataccgctt gttctcactt atgaatgaga gctaaacact gagtacatac agacacaaag 53160
aaggcaacaa cagacactgg agcctacctg agggtggagg atgagaggaa ggtgaggact 53220
gaaaaactac ctatcgggtg ctatgcttat tagctgagtg gcgaaacaat ctgtacgcca 53280
cgctcgcatg acacgcagtt tacctatata acaaacctgc acatgtatcc ctgaatctaa 53340
aataaaagtt aaaagaaaaa gaaatacaaa tggaaacata ctggaattta gagcattaat 53400
ttttaatttc taaggacttt atcaagagac agttttgtag aatatgtaaa atcataattg 53460
ccccaaattc atgcaacggt ctatgacgtc ttcttgattc ctatgtattt atttatttac 53520
atatgtttat tgacacaata gatgtnaagt atttttggag tacatatgat atttcgatac 53580
attgatgtaa ggcataataa tcaaatcagg gcaattggca tatccatcac ctgnaaacat 53640
ttttcttaat actgggaaca tttgaattat tctctactag ctatttgaa atatgtaata 53700
aattactgtt tactatagtc accctactga tttatcaaac gcttaggtct tatttatttt 53760
atctgactgt atttttgtac ccattcacca acctttcttc atccaaccct ggcctttacc 53820
cttccccttc tctggtaaca accaatctac tttctatctt cataagatcc acttacttaa 53880
ggcctaaata tgagtgagat catgtgatat ttgtctttct gtgcttggct tatttctttt 53940
aacataatga cctccagttc tgtccatgca gctgcaaacg tcaggatttc actcattttt 54000
atggctgaat agtattaaca ctgtgtgtgt gtatatatac atatcacatt ttctttatcc 54060
atttatccat tggtgggcag ttaggttgat tccgtatttg gctatttgga atagtgctgc 54120
agtcaacatg tgagtgctgc tatctcttaa atatatagat ttcctccctt ttggatatat 54180
acccaatagt gaaattgctg gatcatagag ttgttccatg tttagttttt ttcagtccgt 54240
actgtttttc acagtggctg tcagaatttg cattaccacc aacagtgtat gagtgttcct 54300
gtttctctac attctcacca gcactcactg tctttttat aaaagccatt ttaacttggg 54360
gtgacatgat atttcactgt agcttttatt tgcatttccc tgatgattag tgatgttaac 54420
catttttata tacctacttg ccatttgtat gtcttctttt gagaaatgtc tattcagatt 54480
ttttgcccat ttcttaatca gattattaga tttttcccat tgagttattt gagttcttta 54540
tatattctgg ttattaatcc tttgtcagtt gaatcatttg cagacatttt cccttattct 54600
gtgggctgtc acttcaattt gttgattgtt tcctttgctg tgccgaagtt ttgagcttaa 54660
tgcaattcca tttatctatt tttgctttag ttgcctgtgc ttttcagatt ttgccccccc 54720
aaaaatttgc ccagtccagt gtcctggagc atttacccaa tgttttcttc taggagtttc 54780
atagattcag ttcttagatt taagtcttta atcctttatt atttgatttt tatatgatga 54840
gaggtaggag cataatttta ttcttctgca catggatatc catttttccc agcaccattt 54900
gttgaagaga ctgtcctttc cttaatgttc ttgatacctt tgtcaagaat gaattggctg 54960
taaaagcatg tatttatttc tgggttttct attccattcc attgggatat tttctgtttt 55020
tttaccagta ccatgctggt ttggttgcta tagctttgta gcatattttg aagtcagtta 55080
gtgtgatgcc tccagctttg ttctttttgc acgagattgc tttggctatt cagaatcttt 55140
tgtgttttaa tataaatttt aggatagttt tttatattcc tgtgaagaat gtcattggta 55200
ttttgataga gattgcattg aaactgtaga ttgcttcggg tagtgttgac attttaacaa 55260
tattgattct tccaattcat gagcatcgaa tatctttcca ttttttgtgt ccacttcaat 55320
ttctttcatc agagtgttat agttttcatt gtagagatct ttcacttctt tagtcacaat 55380
tattcctagg cattttaat tttttaataa atattgtaaa tggcattgct ttcttgattt 55440
atttttcaga ttgttcactg ttggtgtata taaatgctat taatttttgt atgttggttt 55500
tgtatcctgc aatttactga gtttgcttat catttctaac agtttttggt ggagtcttta 55560
ggattttcta agtgtaagat catgtcgtct gcaaatgagg ataattttat ttcttccttc 55620
ccaatttgga tgtcgtttat ttctttctct tatctgattg ctctggctag aacttccagt 55680
agtatgttga ataaatctga tgaaaatggg tgtcctgatc ttgttccaga tcttagcaga 55740
aaggctttca atttttccgc ctttggtatg atgttangcc atgggtttgt caaatatggt 55800
ctttattgtt ttgaggtata cccctttat actcaatttt ttagtgtttt gataataaag 55860
acatgttgaa ttttaccaaa tgctttttct gcatctattg aaataataac atggtttttg 55920
ttcctggttt atttatgtaa tgtatcatgt ttattgattt ccatatgtta agccatcctt 55980
tcatttctgg gaggttaaat aatatgtcca gatggtaagt aagaaagcca ggattttaac 56040
tcaagtctaa cttccaagcc tatgtacgtt ttaccatttg agaaacttct gtattttaat 56100
tctacattca taaacctata gacatataga gatccacatn ccatatgtat aaactgtttn 56160
ataatatatg tagtataatt tnctnacatt aaagtttttn attaangtct ggtttctant 56220
ccctatgtat atgnttatgt ttcatgggat tcgacttnca tctaaatnct ataaactagn 56280
tgatatattn atatattttt agtactatct atatatctct tttnaaaatt gttctgcaca 56340
ttgatctcca agatgtgaac cacctgaggt gccagaaacc agcctttcat atgataataa 56400
tattaataaa gtaaatataa gaatatattt cagaataaat tgaactttca gatgaaaatt 56460

aataccagca tgatttttac cagaatagca aaaatgtttg tgagagtctt cagtatacca 56520
aaagaaaacc atgcagtttg aatgggggtc taaaaggaaa ngtaacttat tttgatccat 56580
cttttgatca tatgtttact tcccaaaaag aagataatgt aatattatag gttataagat 56640
tcttattttt agtggtaaat aacttttatt gaacaattgc tgtatgttat tttctcactt 56700
aatacttata acaacccgat aagttatgtt ccatttttac actgtatatt acagccaagg 56760
aaattgaagc tgagtaaagt taaacaaagt ggctgagatg aaacagcttg tatcagaagc 56820
aagactagac ccatttctcc ctcacactgc tttctatgct ttcagttgta acttgttcag 56880
tgtatgagat aaccttcctt caacatgtgt gtccaaaatg tttgaataaa atatttagtt 56940
ttcctttagt tacaaggagc ctattcttaa aagacaagtt ctcagtggga cgcatgagta 57000
agtggttaat tacgattgct tattttaaga ctgaattcta aaggcatgca ctggaacttc 57060
tgaacatcct ctgtgagtgt ggaatgagga gctctaccta ttctgtccct cacaaatagt 57120
gtttactgta gccgcctcca cagaatcgat atttactatc acttttgtat ctcaggttcc 57180
acccaaccag cttggttttc cccatgatag aaaccataat aagtatgcat aactacagca 57240
gcaccaagag taaattttng aaaattaatt tttaggatga aatctctttg gtaaagaaaa 57300
tcacctggca aaagttgtaa aaaatactga ttccaagcca catttgtgat tcgtattttt 57360
aggcaaggct gagattctca gaattggcca ggttagaaat caatcgttta aaatttagt 57420
tttgcaatta aactccaaaa gatatgttat agagtagaac acaaaattac atggcttttt 57480
aaaaaaatag accttgagtc ttcaaagaaa tgttagtctt ctagctagct gcttactgcc 57540
ctgcccaagg gtagcatgcc tgtcctcttc ccttaagact atttccccag aaatgtccaa 57600
ggagcaccag aggtaattag agcctcctgt ctttcctgca aagaggcagg tatgtctttg 57660
ttttctggga cggacaacca tctcaatttt atttatttag tgactcttac atgttcttat 57720
ttctagagat tgaattgggc tggtgtactt tgtggttgag tgtggttgga gtcgtcaggg 57780
gcattggcac ttttaaaatc cctcactgac tttacttttc gtgttcacat cattggggag 57840
tttgtaaatc cattctttca ttgctataaa gaaatacctg aaactggcag ggcacggtgg 57900
ctcatgcctg taatcccagc actttgagag gtcaagacgg gcagatgact tgagactagg 57960
agttcaagac caacctgggc aatatggtgg aaccccatct ctactgaaaa tacaagaaat 58020
agctgggaat ggtactgtgc acctgtgatc ccagctactt gggaggctga ggcacaagaa 58080
tcgttggagc cgggaggtga gggaggttgc agtgagccga gatcatgcca ctgcattcta 58140
gcctgggtca cagagtgaaa ctccatcgaa ggaaggaagg aaggaaggag agagagagag 58200
agagagagag agagagagag aggaagagag gaagagagga agaaagagaa agaaangaaa 58260
gaaaaaaaga gagaaagaaa gaaaaaagaa aggaaangaa aaagagaaag agaaagaaaa 58320
gaaagaaaga gaaagaaaga aagaaaaaga aagagaaaaa gaaaaactga gactgggtat 58380
cttatttctt ttattacatt tttgggtcct atatttgtat catacagaat gagttctgta 58440
tatttttcag atggaaaaga aatattgact taaaaggtac ctctcaaatc ttaatgtgta 58500
caataaactg gagatcgtgt taatgtagat tattttttca gaagatctgg aagaaagcct 58560
cagtttctga atttttaaca agctcaccag taatgtcaat gtttttggcc aaagaagaat 58620
attttgtcaa aaattcagta agtggaagag cctgtgtttc tttttctagt ttttctggct 58680
tgtaaacaaa gatgagagtt ttcatttacc aaagacagat atatgcaaag aaaacctaag 58740
aaagaagggc agctgccaga ttaaatgtga tggtttatgc acatcagctg cataaagata 58800
cctaataatt aagagaaaaa taattaactc tgtagtgcga cagtctgtca gagagctctt 58860
taaccaagtg aatcataaac attaactgca ctaggacaaa tttttatgan tgtgctgatg 58920
cacacagaag gacgcagcat cactgctgtg atattgcccc ctcaaaagta cattatagtc 58980
taaatttaac cataaagaga catcagtttt atgcgaagct caagatacac gtatctttca 59040
tgttctgtaa tttgtaatgt tgattttaag tagtctttag caccctaggg agcaagcatc 59100
tcctaataat ttttttcaga actttctggg taataaatgc catcctgtta aataagtatt 59160
ttctactttt tttcctgctt tgtatccatt tattcacttg tttgcttttt aaaaaatttt 59220
tactttaagt tgtgggatac atgggcagaa tgtgcaggtt tgctacctag gtatacatgt 59280
gccatggtgg tttgctgcac ctgtcaaccc atcatctatg nttttcagcc ccgcatgcat 59340
tagctatttg tcctaatgct ctccctcccc ctgcccctca ccccgcgaca gaccccagta 59400
tgtgttgtat gttctcattg ttcaactccc acttatgagt gagaacatgc gctgtttggt 59460
tttctgatcc tgtgttacgt ttgctgaagg tgatggcttc cagcttcatc catgtccctg 59520
caaaggatat gatctcattt ctttttatgg ctgcatagta ttccatggtg tatatgtccc 59580
acattttctt agtccagtct atccttgatg ggtatttggg tggttccgtg tctttgctat 59640
tgtaaatagt gttgcaagaa acatacgtgt acatgtatct ttatagtaga atgatgtcta 59700
ttcttttggg tgtataccca gtaatgggat tgctgtgtca aattttattt ctggttctag 59760
atccttgatg aatcatcaca ttgtcttcca cagtggttga actaatttac attcctacca 59820
acagtgtaaa agcgttccta tttctccaca tcctcgccag catttattgt ttcttgactt 59880
tttaataatc gccgttctga ctggtgtgag atggtactgc attgtggttt tggtttgcat 59940
ttccctagtg atcagtggtg ttgagctttt tttcatgttt tttggctgca taaatatctt 60000
cttttgagaa gagtctgttc atatcctttg ttcacttttt gatggggttg tttctttttt 60060
tcttgcaaat ttgtttacat ttcttatagg ttgtggatat tagacctttg tcagatgggt 60120
agattgcaaa aattttctcc tattctattc tgtaggttgc ctgttcactc tgatgatagt 60180
ttcttttgct gtgcataagc tctttagtac ttctagttta attagatccc atttgtctgt 60240

```
tttggctttt cttgccattg cttttagcat tttcatcatg aaatcactgt ccatgtctat   60300
gtcctgaata gtattgccta agttttcttc tagggttttt atggttatgg gtttaacatt   60360
taagtcttta atccatctcg atttaatttt tgtctaaggt ataaggggtc cagttttagt   60420
tttctgcaca tggctagcaa gttttctcag caccatttat taaataggga atcctttccc   60480
cactgcttgt ttttgtcagg tttgtcaaag atcagatggt tgtagatgcg tgatgtgatt   60540
tctgaggtct ctgttctgtt ccattggtct atatggctgt tttggtacca gtgccatgct   60600
gttttggtta ctctagcctt gtagtatagt ttgaagtcag gtagcatgat gcctccagct   60660
ttgttctttt tcctaaggat tgtcttggtt atacaggctc tttttggtt ccatatgaaa   60720
tttnagggta gtttttttct aanttctgtg aagaatgtca atggtagttt gatgggaata   60780
gcattgaatc tataatttat tttgggcagt atggccattt tcatgatatt gattcttcct   60840
atccatgagg atggaatgtt tttccatttg tttgtgtcgt ctcttatttc cttgagcagt   60900
gatttgtagt tctccttgaa gaggtccttc acgtcctttg taagttgtat tcctaagtat   60960
tttattctct ttgtagcaat tgtgaatggg agttcattca tgatttggct ctctgcttgt   61020
gtatttttgg tgtataggaa tacttgtgat ttttgcacat ccatttgta tcctgagatt   61080
tgctgaagtt gcttatcagg ttaaggagtt ttcgggctga gatgatgggg ttttctaact   61140
atagaatcac gacatctgga aacagaaaat ttcacttcct ctcttcctat ttaaataccc   61200
tttatttctt tctcttgact gattgctctg gccagaacat ccaatactat gttgaatagg   61260
agtggtgaga gagggcatcc ttgtcttgtg ccagttttca aaaagaatgc ttccagcttt   61320
ttcccattca gtatgatact ggctgtgggt ttgtcacaaa tagcacttat tattttgaga   61380
tatgttccat caatacctag tttattgaaa gcttttaaca tgaagagttg ttgaatttta   61440
tcaaaggcct tttctgcatc tattgagaat catgtggctt ttgtctctgg ttctgtttat   61500
gtgatgaatt acgtttattg atttgtgtat gctgaaccag ccttacactt cagggatgaa   61560
gccgacttga ttgtggtggg taagcttttt gatgtgctgt tggattcggt ttgccagtat   61620
tttattgagg atttttgcat tgatgttcat cagggatatt ggcctgaagt tttattttg   61680
ttgttgttgt gtctctgcca ggttttcgta tcgggatgat gctgacctca taaaatgagt   61740
taggaaggag tccctccttt tcaattcttt tggaatagtt tcagaaggaa tgataccagc   61800
tcctctttgt acctctggta gaatttggct gtgaatctgt ctggtcctgg gctttttttg   61860
gttggtaggc tattaattac tacctcaatt tcagaacttg ttattggttt atttagggag   61920
ccaacttctt gggagggtgc gtgtgtccaa gaatttatcc atttcttcta gattttctag   61980
tttatttagg tagacatgtt tatagtattc tgatggtatt ttgtatttct gtggggtcag   62040
tggtgatatc cccgttatca ttttttattg tgtctatttg attcttctct cttttcttct   62100
ttatcagttt agctagcagt ctgtctatct attttgttaa ttttttcaaa aactagctcc   62160
tgaattcatt aattttttga atggcttctt gtgtcactgt ctccttcaat tctgctctga   62220
tcttagttat ttcttgtctt ctgctagctt ttagattttt ttgctcttgc ttctctagtt   62280
cttttcattg tgatgttagg gtatccattt gagatatttc tagctttctg atgtgggcat   62340
ttagtactat aaatttcctt cttaacactg ctttagttcc cagatattct ggtacgttgt   62400
ctctttgttc tcattggttt caaagaactt cttgatttgt gccttaactt cattatttac   62460
tcaggagtca ttcagcagca ggttgttcaa tttccttgta attgtgttgt tttgagtgag   62520
tttcttaatc ctgagttcta atttgattgc actgtggtct gagagactgt ttgttatgag   62580
ttcagttctt ttgcatttgc tgagcagtgt tttacttcca attatgtggt tgattttaga   62640
ataagtgcca tgtcgcactg agaagaatgt atattctgtt gatttggggt tgagagttct   62700
atagatgtcc attaggtcca cttgatccag agctgagttc aagtcctgaa tatccttgtt   62760
aattttctgt ctcgttaatc tgtctaatat tgacagtggg gtgttaaatt ctcccactat   62820
tattttgtgg gagtctaagt ctctttgtgg atctctaaga acttgtttta tgaatctggg   62880
tgctcctgta ttgggtgcat atatatttag gatagttagc tcttcttgtt gaattgatcc   62940
ctttaccttt aagtaatgcc attctttgtc tttttttatc tttgtttgtt taaagtccgt   63000
tttctcagag gctaggattg caacccctga tttttttttt ttctttccat ttgcttggta   63060
ggtcttcctc catcccttta ttttgagcct atgtgtgtct ttgcacatga gattggtccc   63120
ctgaatacag cataccaatg gatcttaact ctttatccaa gttggagact gggtatttta   63180
taaagaagaa aggtttaatt ggctcatggt tctccaggct gtacaggaag catggcacca   63240
ggcacctgct cagcttctga tgaggcctca ggaagcttac aatcatggca gaaggcagac   63300
ggggagcagg cacatcacgt gacaagaatg aacacgagag agagatagtg gaggggaggt   63360
gccctacact tgtaaatgac cagatcccct gtgaactcag agcaggagct cattgatcac   63420
ctaggagttg gctcaagcca ttcatgaggg ttctgcccct gtgatccaat cacctcccac   63480
caggccccac ctccagcgct gaggagtata cttcgatgag atttgcgcaa ggacaaatat   63540
ccaaactata tcagggagtc gttagtatgt ctggccacct tgaaaggatg aaagcttatt   63600
ggagtggaaa aaaaattgcc tctctccagc catacaactc agtcaaacca cattcaatta   63660
acataaatat attttgacct ccaagtatgt aagaggtgat ttatattcac aatttctaat   63720
ttataataca gtttcataaa tttgatcttc acaagaaaga tgagcaaatt gctccttgcc   63780
aaagttaatt ggctgaatta ccaactgaag ttctgttatg cttggcccca aattctggtg   63840
gcccctttcc attatgtcaa ctggctctct ttacatatag attctcaccc tgtagtttcc   63900
agttcctaag gcaaaaggag aaaagttgct gcgtgaggat ctcttggccc agatttaaa   63960
agtcaaagac agtggactgc cttataacct caaacggttt cacatttcag tttggagctg   64020
```

atcagtgcat cctctgtgtc ctgatgctta acttttcttt tcttttcttt ttgaggtgga 64080
atctcactct gttgcccagg ctgtagttca gtggcatgat gttggctcag tgcaacctct 64140
acccgctggg ttcaagtgat tctcctgcct cagcctccca agtagctggg attacaggca 64200
tacgctacca aacctggcta atttttgtat ttttagtaga gatgggtttt actgtgttgg 64260
ccaggctggt ctcgaattcc tgaccttagg tgatccgccc gcctcggcct cccaaagtgc 64320
tgggattgca ggcgtgagcc actgcacccg accaactttt ctgatattat tttgaatatg 64380
ttcatgccct ctcattgcct tatcctaacc caactgcagt cttctggctt ctgtcaggtg 64440
cactgtccct tatccatact ggcaaagtag ttgagggagt caacttgggg aacatatatt 64500
attttaaaaa gctattcaga tataacaaga taactattta aaatattcct ttaaaataaa 64560
atcatgtact gcattgtcac ttaaatttca tagtcacaat atttgaaaaa atacttaatt 64620
ttaccatttt tatgcatgat ggctacaatt ctttacaact ttgcctacca accctttcag 64680
gaatgggtga aaggagtggg aaaatcatat taaaatactc agttttacaa cttatttcct 64740
agaaaaaatg tgccaggtta acttgtttat taaaagaggt gtgatgtcaa tattatttcc 64800
tgcttcagaa tatttatctt gttttgcagt tccactaaaa gtccaggcgg agccattgaa 64860
agttttccaa aatcaataaa taatttatct ctgcaaataa gactcttgct ttccagggtg 64920
ggattagtat tccaaagcat ccagcattag aattagctag accaattcat aggagtttat 64980
aattaatcct gaccagagga agctaaataa gagtgaaaat gaagtcagat gattgtctga 65040
agtgaagctg gaaggcggta acagccagac taagttttct gtaagtacat gcaatgacca 65100
ggaaaacctc tcaatctagg ggtcagcttt tagcaagata atgtgtcttg ctaaattttt 65160
atatctgcca caaaaggcaa gacaagcacc acgtaaggta tcaaaggcta tattcattat 65220
tctcttgagt gcaaaagagt ctgtgtctgt gtctatgtat gattgtgtgt gggtgtaatt 65280
ttctccatgg gtttatttag aattgtgttc ctctattttt gatgtatttc tagaacatac 65340
tatataaata aaaatgaagc cttacagatg caatcaaaaa caaaatgctc aagttgtata 65400
gtgttatctg ttgcatgtgg atttgttggg ctacttattt atttacatca ctgacaattt 65460
cttatggaat gccgacttca gaaatgtatt gtagcttgga ctctgcaaat gtcttttaca 65520
acaaatgggt caaatttcga taccaagaag tcatccttag cctgtttgtc tttagctttt 65580
taacaaaaaa aggtaataac aatgtaaaca aaattgctat gtataaattc ctatcatagg 65640
tatatgtttt gaaaatctag caagtaaatt tagtaataat ttatttgtg agtatctttt 65700
gaccttttttt gtcctttctc acctactagg aacagtttat aatcagctca tgctgcatca 65760
gtcacagtat ccatttaggg tagcacgaat ccttgtcttt taccctaatc tataaatctc 65820
taacagtgta gtttaaaaat tgcttttgta tgataacaac tgcttgttaa caatccttgt 65880
atctcactaa acagctacac tcaccagaga aaaggtgaaa aggtctctct ctttctctct 65940
ctctttcttt ctgtcactct ctctctctca tatgcaaatg caggctctga cattttcact 66000
ttccagaatg ttatgctgtt tattccatcc ctagaactag tacgtgtgca tcctttaata 66060
atagtcactc caagcaacac cctattgcaa ggttttccca gtacccacca tctgccaccc 66120
gtagtcatag ttgatgtttc cgctttgaac ttcagttaac atcaggagca tatagatagc 66180
tcttaaagca ttcatcacac tctcctgtgg tcattccttt tttattctct ctcatactaa 66240
gcagtaaagt ccctgagtcc attttattcc ttttattcat ctccattcct agcatgctgt 66300
gtggcataga gtaggcttgt caaaaataat tattatgaat taataaacag acattcaaat 66360
acaattaagt gcccccctac gaatacagga aaatttttct gaaaagaggc taagtcatca 66420
taagcatagt catattcatt aatgacagtg attacagaag gccaacacta agtgcagtgc 66480
cagaagccat gatggtagtt ccatatctgt agagaaagac gatattgtga ataacgaaga 66540
gaggaatctt aacatgaagt cttaagctat tgcctttgtc gttggtgagt cttgtggtga 66600
gaggagaact atgcacagtg aagcatatgt tgaattatgg ggcacatgct tttgcttaag 66660
tatatccctt ttcttttata tgaaagcgtt atgaggtcct tctgggaatt gttttttaaa 66720
cagctggaaa gtttctcatt tttcagatga ggaaactgaa gctcaaatgt ttcaagtgaa 66780
tctcccaagg taattaagtt ggttaatcac cgaactgaga tgatcatctt tgtgttgtga 66840
tatacagctc agtgctctgc accgctagga gtggtgaata acttaaaacc acaggactct 66900
gaggtcctga ggtattgttt gtggccatta tcaacaattt tagtaacaag ttcagaacac 66960
attttcttag atctcaaact cctacaaaat gttatttgta aatagaatgt tttgttaaga 67020
attaaaaaca aacaagcaaa catatgctag gaggataatt gaaggctttt aaaaaaacaa 67080
ttagctgctc ccagggattc tagtactcat tttgatacat ggaagctact aaagccagaa 67140
atttaaaaca ttgaaaatca gtgccaattt gtggattatt acacttaaaa gtgcataaaa 67200
caacaggatc cacttattaa agagtttctg tagtttacaa ataaataatt caccatagat 67260
ttaaataatg gtagaaagac acattcattc attcattcat ttattcagaa aataattatt 67320
aaacatctac tatatattag gtttctccag agagaatata gaatagaata tattatatat 67380
atgtatgtgt atatacatat atgtatgttg gcttagctca aaagatatgt ctatagatac 67440
agatacagat ataaagagat ttactttaag gaattggctc atgtaatttt gtaggctgtt 67500
aagaccaaaa tcttcagggc agcctggaaa tttcagcaga agctgatgtg gcaggccaaa 67560
ggctctgcca aagatgtggt aggccagtga cctctccctg cagagaagtc aaaatctttc 67620
ctattaataa gatgagtccc actcacatta tggagggtaa catgctttac tcaaagtcta 67680
tcaatttaag tgttaatcac aactgtaaaa taccttcaca gcaacatcta gactggtgtt 67740
caaccaaaca gccaggcacc atactttagt caagttgata cataaattta actatcatag 67800

```
acgctgtgtc agggacttca tggaattgga aattacacta aggaacagtt gagtttggaa   67860
gacatcccaat gaacagatga ccacacaaac atgaattgcc tttgtctcaa gtgccagaaa   67920
caaaaagtga gggtaccttg agaagttata gcagggaaac ctggcccagt ggaaggcact   67980
tggagtggct cccccgagaa aatgatagtt aagctggggc ctgaaggcta caaaaatggg   68040
gttaaccaaa gaaaacagga gagacttcca agtggaggag aagaccctgc attaggcagg   68100
agctgggaga aggccaacgt ggtcagacac tgggatactt gggggaaagg gggtacaaag   68160
gggtgtttga agtgtgggag gagccagatc cttcagtatc ttgcagacca tgttacaggt   68220
ttgccactgt taaggtagga cagtgattat gaagggtctc tccatatgct ttgtgccaaa   68280
tgtatcagaa aggctaagag tgggaggcag ggtggccagt gaggaggcca gataagagtg   68340
tgtgtagaga gggagatgga gagaatggaa ttagctcaaa agatatttg agaatatgtt   68400
ggagaacgtg acgattactt atgagggttg agggagaagt aggtatcaag catgattcta   68460
agacttcaag tttgctccac ggtttggaag ctggtcctgt ttgttatgac agggaactct   68520
ggtttaggat ttcaaattga ataagggacc tattgagctg gaggtacttg tggtgtagcc   68580
aagtggagaa atctaataag cagttggaca tgtagctcta agactctaca acgatgtaaa   68640
ggatgacaga gaattgagaa atcatgaaag tggatgagat tgcctgggag agttatcatg   68700
aaaagagaaa ggtcaaagac agagctcaga gcagctcatg actaaaggtc agctcacgac   68760
tagaggagat ggaaaagcag aatggcctna agaaatatgg acnaaaccag caccttcaaa   68820
gccagcagag ttgcatctgt gtcttcctcc catactcaca tctctctcca naccacagcc   68880
atgatcactg ttagtgattc atggagttag gttgggtcca cctggataat tcaggatcat   68940
ctcccaaatt taatgtcttt agccttaatc acacctataa ctttagttcc cttttgctat   69000
gcaacattat ctactcacag gttccatatg ttagaatggg gacatcttta ggggaccagt   69060
tttttctcta ccacaaaaac aacaattcag agcactgctg agaggtcaca gaagataaaa   69120
ctaaaatttt tctgattggg tatatcatat ggaagtgaac aaaagtaatt tcattgtctt   69180
ggagtcacag aaatgggcta aaaagttaaa agagggagat tacaagtaga tactttattc   69240
aagaaatmtg tgagtaagaa atgctggtct tgaaacccgt ttaaacatga gtggatattt   69300
attaccactg aaagaatcat tgtctatgtc caggattttc ctggtggggc aagaattcca   69360
tccaccccac ccccaactcc agagagtcac ttgttctcta tccaaagtac ctgagggaaa   69420
atgactacgg aaataattaa ggttaatgaa taaaaataaa ctctggaaga ttcttgttaa   69480
ctgaaaaaaa tgaaacaaag attaacaaat atatattctg aaactcttga cattcatttc   69540
aatttttaaa aacagtaatt accttgtggt tttagataca tgtgttccag ggcaattaat   69600
cttctaataa atgtgtttct aataaacatt tgctaataac ttgatgttgg taaaaatgtt   69660
tcaagcctgc tgcctctgaa gccaaagact tcaaaataat atctagtgag agttgtgctt   69720
aaaatgagta tcagcactaa tatgaaagaa aaagcaaata taataggaag aaaaaaggaa   69780
aaaatactct aatatttata aattaattta aaaaatcata atcattaaca gttgagtgat   69840
taacttattt ttggcaccat tctgagtgtt gcacattatc taatttaatc attaaaatgc   69900
ccctacaaag aatgcaataa ctatcctcat tttggnaagt gggataactg aggctataca   69960
cacagggctg gagtttcaat ctaggaaatc tacaactttc aaactgtcaa taacagtagg   70020
taacatctag cattgattgt ctatcaagta cctgttttat acaatttaca gatgtaaata   70080
cttacgtctc accccaggct tatgaggtag acgttttcac accattatgg attggaaaac   70140
taaagcatgc ttaagtcaca catgtaataa gtaggtcaga actcacagcc aagaagattg   70200
gctccaaagt ccaatttctt ctccaaatat ctgaaatatg atttttttgg aaactttttc   70260
atgtagacag agtgttatct tagacatttc tgctattgta tttttaaaag tatttggaat   70320
taaaaatata tgtaattttg atgggctagt agacccttcc ctctctgaac ttcaccttgc   70380
cttcaagagt aaatgaagaa ctaggtgatc catatagtat ctcttagctg catagttcta   70440
tgaacatgct aaagtttctt ctcctctttt aacaagtctt tattgttctg tttttacatg   70500
ctgggattta tgctacattg tattattgta atgattaata atcatttaga tcaagcatta   70560
tttgagtaca gtacttgcaa cacattaggt gctatgcaaa gtgcattact tggtttactt   70620
tacttaaccc tcacatgagc tttgctttta attatgatta ctctctgtac cttacagatg   70680
aggaagctga gcttagaggc ccttagagcc ttatttaagt cacacaacca gtaaatggca   70740
gagctgggat caaacccaga aagcctcact ctgaaactca gaagcccagt ctccttacca   70800
tcttactgtt atattgtagg taccaaatca ttgatgacaa gcatcgttga tgaggttaat   70860
agtatttgta ccacacattc tgaaacagag tcagatcaga ctgaagcaaa gatgaatcat   70920
ttggcctaca tgtaagaatg agaaatgggg agatatacac gatgtgtctg tctaaaataa   70980
tatctttctg acccacccaa ttgtggtaat ttccacaatt agagcctttc ccttgggttt   71040
tacagatggg gtccaaagga ccagcttaaa atatgactag agggagcaat gatttaacca   71100
cttatttcct gagaccagca gacagatgag ctcatctgct gctgacaaag ggcagaaccc   71160
taaaggaata gcttagggtg tttgaatcat gggtgaagtt tctggagctt tgtttatggc   71220
tgagaagtgg gcaaagtatc accagccatg gggtgtgtgt gtgtgtgtgt gtgtgtgtgt   71280
gtgtgtaaaa ctatgtacgt gtgtgtatgc gtggtggcag caaatagcta agggattgca   71340
caattaggtg tcagacttta gagaaagcat attataaaag ggatggaagc agcaactgaa   71400
cataagaagg tattaaagaa aaagagtcat ctggtaagtc tacctcaatt tggtcagaaa   71460
ctcacgtgac tgaggctgtc ttagggttgt tctccattta agaaattcct agaaacattg   71520
atgtagtggg aaattagtgt aacacaatta tggatttaga aggcttctga aatcatcaag   71580
```

cccagacttt ggcgtctgct caatctgtgt gggatacctc agatagcagc tatttggggt 71640
ccatttttaa ggtcaaccca gaatgagagt ctacagtcag cagtcctatg ttatctatac 71700
ttgctgtcag gaaagccatt acatatctaa atcctttata tcaagttgat atccaaccta 71760
ttcttaagaa ggataaaaat gggttatgtc attatccacc taaaggattc ttatattcct 71820
agagctatca ctaaatcacc tcataatctc tctttcctat gggatacctt gagttccttc 71880
catttctcct tatttgtctg gttatttcta aactagacac cttccaacac tcagaaggac 71940
aagtatcatt gatgagtttc gtagtattcg tactgcaaag aaaatgattt tcatcatgca 72000
acattgctta gaagtgggga agtaaaatgt gtgcccacag tgagacaaga attttgtgaa 72060
tcacaatgac tcctcagaca agttaacgtt ctttgcaaat ctgtagaaaa attccttcct 72120
ttgcttatct gggtctcagt ctgtaatggc aaaaaccgta atggtgatag tattcttata 72180
aagagctatt gtggggatga atcagatatc atttgagaat gtttaatatg taattaatgt 72240
tatgaatatc atttatggat tatgaatctt aaaactatat tcagatacac atacacaatt 72300
ttgcaaataa catcatatat atatatatat atatatatag agagagagag agagagagag 72360
agagagagag agagagagag agagagaaag tctctcaagg tctcatatat atattaccaa 72420
ttaccacagt tgggtgggcc agaaagatat gattttagac agacacgtca cgtatatctc 72480
ctcatttctc attcttatat gtaagccaaa tgatccatct tcgcttcagt cttaactgac 72540
tctatatttc aaagtgtgtg gtacaaatat tattaacctc atcaacaatg cttgtcatca 72600
gtgatttggt atctacaaca taagagcaag aaggtctctc aaggtctctc tttctctctc 72660
tctccctctc taaatatata tatatatata tatatatatt ctctttgctg tactcttaga 72720
tagatagata gatagataga tagatagata gatagaaaga gcaaaggtct ctcaaggtct 72780
tgctctgttg cccaggctag aatgcagtgg catgatcata gttcactgca acctcgaacc 72840
cctgggctca agggattctc ctgcctatgc ctcccaaagt gcaacgatta caggcatgag 72900
caacctcact tggccagata catagatatt aatgaagggt aattacatta ttcattgtaa 72960
tcaacattta tgattgttga aattgtctaa cactttcatg tgttgttcac aggaatgtaa 73020
cctggaatag tttagctatt ccagacacac actgctcacc agagctagaa gaaggctacc 73080
agtgcccacc tggatttaaa tgcatggacc ttgaagatct gggacttagc aggcaagagc 73140
tgggctacag tggctttaat gagataggtc tgtcttactg gtaaaatacg ttttaagttt 73200
ctattttgtg atgtttatga agtggacaga ataattaaat aattgatact aatattttga 73260
ctggctgtag tatagaagaa atacaaatat aaattttaat tctatgccta ttttataata 73320
tattctaccc gacagtgcgt agcatacaag ctgtgtatgt atatgggcag cagtgtttgt 73380
atggagttac agatctctca tttttgacta tttcgattct caataacrgc atttaagcat 73440
ttaaaaatat ttgtttgttg aacagggttc tgggttgttg actcaaagat aaacaaaatt 73500
tggtttcctt tcatatgtaa ctatgtaact ataccctaat aggcaaggta agtcttaaaa 73560
actgtaatac taggtattga tgagtgcatt agagctgaaa agtgctcctt gattagaata 73620
caattaatga actacttagt gaatatgcat caactaagta ataaatgtat gggaaatgct 73680
tgtgcatgag aactatgtgc tcaaatgtgt acattcttct tggcttgcct tattaagtcc 73740
taaaatgtgc cattacatac ataatgaaag aatttagcat ctgccattta gttgatatca 73800
ttgtattttt ataattccat gcaagttgcg catattatat tattaaagca aaaaatgtat 73860
aattccaaac aagtatttta tgccttgaga attgttatca gagtaatttt ttaaactaaa 73920
atttcttcaa gtgtcaggcc tgcgtgctaa accataaagt tacagctttt atatagaata 73980
tagtactgaa cgtgggctga tggtatcttt ttaaagtcag ttttgcaaat aacatctcag 74040
gttttcatct ttatgtttta gtatttcctc atattttatt tcaagcatta tagttagatg 74100
gcattcttcc atctcttttg accagtaata ttgtctccta tttcacaaaa atccagaagc 74160
caactgatgt gaagcaattt caattttcta tactctctta aacttatcca actttgtaat 74220
tagtcttaaa tccttccctc tgtctcaaaa gtacagaggc atcatgaatt ataccaataa 74280
cttttaggca tgaggaagaa atcacagatg tggtatggga ggcgagatgt atataggctg 74340
ataggctgtg cggaataagc ttcaggagtt ccaacagctg ggcatgacta gaatgcatgc 74400
cttatcagag tctgcagctc tttcttttag ggtctgagtg tatccatgtc ttctatggag 74460
agtggtcacc attccagggt taaggttgcc cttacagaaa ataacctcca attttttaaa 74520
atcatgtagc taaagcacct cataccctca ccgtttttaa tgcaacaatt gtacccaaat 74580
tcaaaatgca gcattatatt caagttgatt catctttctt cactctgact tacaatggct 74640
ttgcttaata tagggtagaa tctctttttg agagaaaaag tctccttgta ctgtgctgac 74700
ttgagaggac cagttctagg aggaaattgc cttggaggca gtcgcccccct actaaagcaa 74760
ttatcttact atagctgtac tgttaattta tctcccaagg atttaagctc cttgaaaaca 74820
ggaagtgcat cttttcgtct tcaagtctcc agggcacagg gctatgcctg gctcacaggg 74880
agccctccat aaatatttac taataggctc aacaaagcgt atgtcttttt aattcattgg 74940
taaccaggca tgtttgtatg aggaggcaga gccacaatag agaatttagt tgttttcttg 75000
ttgttctttt gcctgatttt gtctgttttt gttggcaata ttcattattc ctcatgactc 75060
cttcctttct atacaggctt aatttcatcc gtttgcttat ttttcccaaa ttcatcatca 75120
ctgtatgact aattgttaga taatgaaatg ttgatcttgt accataacca aaacattttt 75180
aaatggtcgt tataatttag atcgaaattg atagctgctt cctgatatta tgtatctctg 75240
tagatacaca aactactata tttaaaagtt cttatggaaa caaaagacaa tttttgccc 75300
aaaatgagga aaataaaata ttttattaga cacatgtgat catctcttca tgcatgcatt 75360

```
tgaatttaaa acataattag gtgcctaatt atgcatatga atttggctaa tatacttgaa   75420
tatgtaagta attatggtta gttaaaaagg agcccatttc cttttcttac taatcaaatt   75480
accattcaaa acatgaattt caattcactt ataaaatttc tggcagaagt agctttatgt   75540
ctcattaaga tttaacactg atgatgttga aaaattcaaa ttttaaaata tattaaaagc   75600
cagagtcatg gagtagaaaa aattacaagg aacccctgtt gtaagattga agagtaaaga   75660
catctttgcc tctttgtgtc cccaaaatca tccaaatatc aagaagattg agaaacagaa   75720
atgaaatttt catgtttaag aactctgaaa agctgtaact ataaaccaca tcacaggact   75780
gggcatctaa cagaaaactt cctgagaaga ttctgcctac tgtggatctc agacagcacc   75840
agcaggatag caatattcta agataatgca tgttcctgga ggaacacaac tgtctcatat   75900
ttgagtcagg gaatcagagc aaaggctgat tgtggaggct tactaggacc ccatgcagca   75960
tcacaaggca gaggccctca tttctgcagg cagttggtgg ggagatagag tggaggggac   76020
acaactgctt tgttgctgct gatctgggtt ggctagaatg ggataaaagc taaacggaag   76080
gttttcatgg ccactataac ttaggatgac ctggggaacc ttttaaaaca aacagattct   76140
caagcaccat cctcatgtca attaaatcac aaccttagag tgatccaagg aatagatggc   76200
ttttaaaggc tctccaggtg attaatttat gtgcatgcag agttatgaac cattggacta   76260
aagaaataca cacatagaca cacacacacc tgtattataa aaaccttcaa ttccatcttg   76320
atccattttg gttgttgtaa caaaatgtca cagaatcagt gtctcataac caagagaaat   76380
ttatttctca cagttctaga gtctgggaag tccaagagca agacagcagc agatttcatg   76440
cctggtgaag gctcacttcc tggttcatag aatggcacct tttcactgtg tcctcacgta   76500
gtagaagcag taagggttct ctcttgaacc tccttcataa ggttactaat accattaatg   76560
tgggctccac cttcatgatc taatcacacc caaaggtcct accttctggt agcatcaccc   76620
ccatgggggt tagaaattca acaaatgaat ttgggggaaa catcagcatt ctgtccattg   76680
cactatccta gaacattacc ctgttcctcc ccaaagtgaa acagtgacag acatctggtc   76740
taacaaaaat ttatttcaaa gatgagtcat aattgaaaga atgaggacca catctataaa   76800
aatgtacttt gaggtacttt taaaactgaa aaaaaatcat gatatataac tcataaaagc   76860
ttagtaggaa acagagcact gaattacaga ttcgaaggat gcagcttttc tcagtaagtg   76920
aacatagaac agtcaacact gagacacatc ttagtaaaat tgttggaaat caaagctaaa   76980
gaaaacattc attaggcatc cagaaaaaat aaggtcaagt tagctataag gaggtacatc   77040
agagaggcct gaaacttcat gtcaatatgg aactctagaa tgtaatggca caatgcttac   77100
aaattcttta gggaaagaat gggtcaagct ctttctgaat atttttgaag aggcaggaac   77160
atagagacta tattttgcat gaaatcttcn ttgaaaacat tcagttaatc aagaatttct   77220
tatcatgagc tagtctctag tatctatttg ttttaagaac aaagctaata taactgtagg   77280
atttacaaaa tacaatgtaa atattataaa ttatgaatat ctagaagaga taagatgaaa   77340
accgggggag atagtgtgat tatgttgata ttcttaacat tcacatttta gcagttaaaa   77400
aagtaacatt tatacaatat atgaaatgta taatagagat atgtgcatac ttgtcatgag   77460
atgagaatgc atgataaact cagnaaatta ctttctttag aaataactca aaaatccaaa   77520
taatattatc tatgaccacg tcccaaaaaa tcatttcaat atgagtaata atattggaat   77580
caattctttc taaatcttga taatagttct caaaagaaaa gtaacaaaca gtatattttt   77640
taacctctga atagagttat gctatttgct gtagcatgtt taaaattatt aggggcacat   77700
gactcctttg tggtaaagtt tgcttgaagc acagttgttc aaaagaatgg ccctggggtt   77760
tagatcatga ctccacttta tattagtgtg acagccttag gaaagtaatt aactctcttg   77820
cctcaatttc cccatctagg aaatggggta agtgatagac ctaccttata cagttaatgt   77880
aaagagcaac tgagaaacta cacttagagt acgtgtagcg tagaacaaac gcagaatgaa   77940
ctagaaattg ggatgcacgg gcaaaatatg aatgtatctc tgtgttattt gagggaaaat   78000
ggtgtaataa taagggaggg caggacatag atattttcac actcacacac tgcatctgat   78060
atgattccct ttgtttacag acattaacta tgcaggtttt ccagtatctt attcaaggga   78120
gaggcattgg ggcaggggca cggtgttttt tgtttgtttg tttgtttgtt tgagatggag   78180
tctcggtctg tcgcccaagc tggagtgcag tggcacaatt ttggctcact gcaaactttg   78240
cctcctgggt tcaagcagtt ctcctgcctc tgcctcccag ttagctggga ctacaggcgc   78300
ccatcaccat gcctggctaa tttttttgta tttttagtag agacggagtt tcaccgtgtt   78360
agccaggagg gtctcgatct cctgacctcg tgatccaccc gcctcggcct ccaaaagtgc   78420
tgggattaca ggcgtgagcc actgcacccg ccggggcacg gtgttcttaa ccttgcatct   78480
tgcctatgtt aactcatatc tcccatttca ttttattcca atcaatgact agcattctag   78540
aaggcaagtt tttggtgatg tttggtcttt attcatcaat tattcccctc ccctctcaca   78600
aatgtcctga cttctgtgct tgtaccaatc tctctagaat ctgatcaaca tttagcccgt   78660
gtcattgaat tttctagttt gatgaagcat catgcatatg tttaatttta tttatttatc   78720
catatacatt cgaatctgtg acccctggat tggagcctct ccctttcagc gcctttcaan   78780
caggtgctcc ttccacagag cctaagtaaa gcaccagctg gggcattgag cagaaggcac   78840
agccatgctg tgatgtgcag gggctccagc actaagtgat ttattttccg nacctcctct   78900
aattccctga gccacgtgac aactgagata ctagttattt ctgtctcttt cttccattta   78960
tgcacctttc catctttatt tctggatagc tttcctgtgg ctccttccta aggacccccca   79020
actgactcat ccctgctaag acattagccc gtgcactcaa gatatcacac actccccatg   79080
ttgcaagctt cctgagtagt agacataatc taaaggacat cttgcaaaat cgcaaaattt   79140
```

```
atttagcaga cattgagatt ccgtgattgc agtatgggtg gcaaggtggg taggaatcat 79200
acagtggtaa ggtttgggga ttgacaacat cctaaaagcc acactgtcga agtgacacaa 79260
aatcacagca acttacccta tttagaaaat gtgtcttccc ttttctgaat acttacccca 79320
ctttcagttc ctacttccgc tgnttcccac ttgctcataa acatatgaga cataaaagct 79380
gagacataaa agaggcgaca ctgtgatttt ctgtaataac actggaaatg aaattacaga 79440
agtttagagc tggaacagat aatgcagtca tacattttac ccacacaggt ggggatctga 79500
gagtcagagt gagtgggatt agaattacag agaactccgg gcagaggcag gctgaactca 79560
cttctccaga tttgcagatg tccaccatac ttgctgtttc caccaaaccg tcttacaact 79620
caaaatagtg tttaagaaga aaattcaaaa acaatttgca aagcttagtt ttattcctaa 79680
ttttctagtg gaatgttagc attatttccc tttagtgaaa tgctgatggg gaagaccgtt 79740
atccaaacag aggtgatgtg ccagttagaa agaaagcaac acaaaatata ccatatatgc 79800
gaaataaatc acagcaaaaa aacaccatcg aactggtgat tccttgtcat gacctttttaa 79860
aagaaagtaa aacatttggt acaagagatg agaacaaaac tattcaggtg gttatttgaa 79920
cccttcattc attgtacagt caggtaaaaa ataaaaaatg ttcagtttgg actaaacaac 79980
ttaatgaatg tcattggatg ataaaactca tcctgaaaag tcacttagga agtaaatcgc 80040
tatcatgcat tttttattag tctttagaat catcagaata aatgacaaaa gcccattttc 80100
aaaaaaatcc agaataaatg aaattcaact gaaacagctt atagcagagg aagcccacaa 80160
gcaagataaa ctatgaatca agacattctg aaaataacta atagaaaaga catacaacat 80220
atgcccttaa cactacacga gctgtatatr gtggatcact ttgcctgttc tatatattca 80280
aaagtcgaag gtaaagccag gaaataataa acactgtatt cttatggtga ctaattgtcc 80340
atcagacagt cgttactttt gtaattactg tcattactta ggacaactgt tggntatcat 80400
ttatcgattg catattatgt gcctggcacc agtgaaaagg ttttgcatcc attgtgtttt 80460
taatcctcaa aatcagccca tggggtatca ttatccctca gctgatgagt ggggagaatg 80520
agataagcaa gaatgaactc tcccagagcc tcttaccagc cgcatttgga agccatgtct 80580
tgctgccccg tgtgctgagg ctgaagcctg tgctcttgat ctacaaacta ctacgttacc 80640
agagtagcca gccatgagta accttcattc ctgtggcttg tcaggacacc tggtccatta 80700
taaagtctca ttatgatgat ggaagaaaca catcttccaa aagagtgacc tgccatagtg 80760
gcatatcctg caacatggtg acctccctct tgtcctgaag tctttagaca agtcttagaa 80820
aaaagaataa atgtggctg ggcgtggtgg ctcaagcctg taatccaagc actttgggag 80880
gctgaggcag gcagatcacc tgaggtcagg aattcaagac cagcctgggc aacatagtga 80940
aatcctgtct ctacagaaat acaaaaatta gctgggcatg atggcgggtg ccagtaatcc 81000
cagctactcg ggaggctgag gcaggagaat cgcttgaatc tgggaggcag aggttacagt 81060
aagccgagat tgagccactg aactccagcc tgggcaacag agcaagactc tgtctcaaaa 81120
aataaataaa taaataaata aataaataaa taaataaata aataataaag agtaaatgtg 81180
aaggaatgtg acgtccactg caaactgatg gccccttaag ggttggtct ctggctttaa 81240
gtctagtccc ttgtattctg tattctgtga gacagagagt tggtgcataa tatatgttag 81300
ttgaatgaaa gaatataaat attaaacaat gtacctggga ggcaatgcag ttttataagc 81360
tactgagctg taaacatctg ggcaaggatt agagataatg ccagaaaaaa ttagtaaata 81420
ctacttttga tgtaaaatta ggtggtagac acatcatggc tagagatgtg ggagagaact 81480
tagcaagcaa aagaaaatat gctatttggt atattttagt aagttttgtg tgtatttgaa 81540
ctaaattcaa aatgccgtga taaggcatgt gtttgctata tgtaatgtat agctacatgc 81600
attaatttct gttgtgtatt tgaatttact ttctttgaga tatgaatatt taaatttgct 81660
ttctaaatta tcacaaatta accaacaaat ttatacaagg tgattttgga aatccacatt 81720
aattaatttc tttattagaa tgtgacataa acagtattta ccttattagt ttntctttac 81780
gaaaaaatta taaangcttt tctatatata tcctccttaa aatattagat aagaaaacta 81840
caagtcgtca gctccacagg agttaacatg tggattttgg atgctaagaa caaatttaag 81900
atgatgactt gtaatcttca tgaattgctt ttggcaacaa taaattaatt gggcctttca 81960
agaaaatgca gaaaactaag gtaaagggtg attgagtctt ccagatttcc tatattccca 82020
actcacttct gaagaacgta attatgagta ttgaaatttg ggactggtat tttttacaag 82080
aagaaatata ctgtagctgt tatttacttt tgtggtattt ttcttctttg aatttgggta 82140
gtattgacta taagctatgg actcagctcc ctgtgtgggt cataaatcag ttttattaaa 82200
tattgattta gaaatatcag ttattatttt cntcactggt ggagttctgt gcttcggact 82260
acccctgtat cttgctgaaa taggattgac aat 82293
```

<210> 2
<211> 237961
<212> DNA
<213> Homo sapiens

<220>
<221> exon

<222> 43726..43868
<223> exon 8

<220>
<221> exon
<222> 43998..44102
<223> exon 9

<220>
<221> exon
<222> 52093..52179
<223> exon 10

<220>
<221> exon
<222> 77568..77699
<223> exon 11

<220>
<221> exon
<222> 98226..98393
<223> exon 12

<220>
<221> exon
<222> 106567..106758
<223> exon 13

<220>
<221> exon
<222> 144109..144246
<223> exon 14

<220>
<221> exon
<222> 159794..159868
<223> exon 15

<220>
<221> exon
<222> 191292..191428
<223> exon 16

<220>
<221> exon
<222> 192967..193108
<223> exon 17

<220>
<221> exon
<222> 211540..211613
<223> exon 18

<220>
<221> exon
<222> 225006..225107
<223> exon 19

<220>
<221> exon
<222> 225544..225613
<223> exon 20

<220>
<221> exon
<222> 228450..228541
<223> exon 21

<220>
<221> exon
<222> 228630..228752
<223> exon 22

<220>
<221> exon
<222> 231289..231345
<223> exon 23

<220>
<221> exon
<222> 231589..231709
<223> exon 24

<220>
<221> exon
<222> 231813..231944
<223> exon 25

<220>
<221> exon
<222> 232900..233067
<223> exon 26

<220>
<221> exon
<222> 235355..235459
<223> exon 27

<220>
<221> allele
<222> 51090
<223> 99-79335-60 : polymorphic base C or T

<220>
<221> allele
<222> 61293
<223> 99-79336-369 : polymorphic base A or G

<220>
<221> allele
<222> 80602
<223> 99-79338-332 : polymorphic base C or T

<220>
<221> allele
<222> 100485

<223> 99-79314-201 : polymorphic base G or T

<220>
<221> allele
<222> 100509
<223> 99-79314-225 : polymorphic base A or G

<220>
<221> allele
<222> 106725
<223> 99-79316-158 : polymorphic base C or T

<220>
<221> allele
<222> 166087
<223> 99-79322-224 : polymorphic base G or T

<220>
<221> allele
<222> 166336
<223> 99-79322-473 : polymorphic base A or G

<220>
<221> allele
<222> 235894
<223> 99-79306-182 : polymorphic base C or T

<220>
<221> primer_bind
<222> 51031..51051
<223> 99-79335.pu

<220>
<221> primer_bind
<222> 51539..51559
<223> 99-79335.rp complement

<220>
<221> primer_bind
<222> 60925..60945
<223> 99-79336.pu

<220>
<221> primer_bind
<222> 61354..61374
<223> 99-79336.rp complement

<220>
<221> primer_bind
<222> 80271..80290
<223> 99-79338.pu

<220>
<221> primer_bind
<222> 80700..80720
<223> 99-79338.rp complement

<220>

<221> primer_bind
<222> 91037..91056
<223> 99-79339.pu

<220>
<221> primer_bind
<222> 91466..91486
<223> 99-79339.rp complement

<220>
<221> primer_bind
<222> 100285..100305
<223> 99-79314.pu

<220>
<221> primer_bind
<222> 100764..100784
<223> 99-79314.rp complement

<220>
<221> primer_bind
<222> 106568..106585
<223> 99-79316.pu

<220>
<221> primer_bind
<222> 107000..107020
<223> 99-79316.rp complement

<220>
<221> primer_bind
<222> 165864..165884
<223> 99-79322.pu

<220>
<221> primer_bind
<222> 166381..166401
<223> 99-79322.rp complement

<220>
<221> primer_bind
<222> 235713..235732
<223> 99-79306.pu

<220>
<221> primer_bind
<222> 236190..236210
<223> 99-79306.rp complement

<220>
<221> primer_bind
<222> 51071..51089
<223> 99-79335-60.mis

<220>
<221> primer_bind
<222> 51091..51109
<223> 99-79335-60.mis complement

<220>
<221> primer_bind
<222> 61274..61292
<223> 99-79336-369.mis

<220>
<221> primer_bind
<222> 61294..61312
<223> 99-79336-369.mis complement

<220>
<221> primer_bind
<222> 80583..80601
<223> 99-79338-332.mis

<220>
<221> primer_bind
<222> 80603..80621
<223> 99-79338-332.mis complement

<220>
<221> primer_bind
<222> 100466..100484
<223> 99-79314-201.mis

<220>
<221> primer_bind
<222> 100486..100504
<223> 99-79314-201.mis complement

<220>
<221> primer_bind
<222> 100490..100508
<223> 99-79314-225.mis

<220>
<221> primer_bind
<222> 100510..100528
<223> 99-79314-225.mis complement

<220>
<221> primer_bind
<222> 106706..106724
<223> 99-79316-158.mis

<220>
<221> primer_bind
<222> 106726..106744
<223> 99-79316-158.mis complement

<220>
<221> primer_bind
<222> 166068..166086
<223> 99-79322-224.mis

<220>
<221> primer_bind
<222> 166088..166106

<223> 99-79322-224.mis complement

<220>
<221> primer_bind
<222> 166317..166335
<223> 99-79322-473.mis

<220>
<221> primer_bind
<222> 166337..166355
<223> 99-79322-473.mis complement

<220>
<221> primer_bind
<222> 235875..235893
<223> 99-79306-182.mis

<220>
<221> primer_bind
<222> 235895..235913
<223> 99-79306-182.mis complement

<220>
<221> misc_binding
<222> 51078..51102
<223> 99-79335-60.probe

<220>
<221> misc_binding
<222> 61281..61305
<223> 99-79336-369.probe

<220>
<221> misc_binding
<222> 80590..80614
<223> 99-79338-332.probe

<220>
<221 > misc_binding
<222> 100473..100497
<223> 99-79314-201.probe

<220>
<221> misc_binding
<222> 100497..100521
<223> 99-79314-225.probe

<220>
<221> misc_binding
<222> 106713..106737
<223> 99-79316-158.probe

<220>
<221> misc_binding
<222> 166075..166099
<223> 99-79322-224.probe

<220>

<221> misc_binding
<222> 166324..166348
<223> 99-79322-473.probe

<220>
<221> misc_binding
<222> 235882..235906
<223> 99-79306-182.probe

<220>
<221> misc_feature
<222>

6485,14246,14248,14250,14252..14254,14256..14262,14969..14980
17712,18786,19477,19496,21043..21064,21547,24607,24946,24974
25072,25124,25136,25142,28843,31239,31372,37750..37751,40488
46542..46557,49309,49326,49357,49395..49398,49400..49402
49408..49409,56275..56280,61048,61050,61059,61856..61857
61860..61861,64842,66307,66326..66327,69835..69840,70893,70905
73279,76154,76354..76355,76371..76372,76407..76408,77095
78933..78934,79363,89491..89492,95836,95838..95839,95841..95842
95958,96966,97049,97067,97078,97292,98642,98712..98713,98716
98740..98741,101368,101373..101374,101376,101378,101401..101403
101406,110014,129061,129066,129973..129974,135336,135345,135359
139924,147368..147369,147469,147507..147510,147767,151694,151729
163111,164480,169764,173340,173390..173391,173398..173401,173407
173414,173419,173426,174800,174894,175041,175486,176256,177240
178864,181149,181154,181230,181287,183433,186606,186648,191065
191267,193117,193203,193223,193380,194051,194053,194057,196577
198388,198655,198828,198849,198862,199667,199693,199701,199704
199747..199748,201117,201224,201226,201314,201410,202524,203887
204396,204939,205891,205930,205998,206908,207363..207364,210938
212018,214253..214254,217022,217333,219027,219909..219912,220924

222620,222882,223870,224902..224903,224911,224914,226899,226915
227232..227233,227415,228441,228446,229373,229471,233305,233405
233512,233601,233629,236096,237468,237471..237472,237478..237482
237486,237488..237490,237492,237495..237496,237498..237499
237504..237507,237509

<223> n=a, g, c or t

<400> 2

```
gatgttaaat aaggcgataa aatatgagga agaaaaatac ttttattcaa attataatgg     60
gatgtaaatt gaattttgtt gatgctgctg ctaaaaattg attcatcaga gttaaatgat    120
attaaagcat atttcatgct accatatgtg atatcagatg agaactttac aaataaatag    180
aaaacagaaa gttatatatc tacaaccatg atttattaca gaacgtatat ttttactgca    240
gtcataaaag aaaaagccca gtaaagacta atttgtaaaa tatgtaattg agatttataa    300
tgctttcaaa aaaaaaaaag caccaagggg tttttctgat gcatttatct tgatgtttac    360
tgttgccata ggaattactt attgattttt atgaaaacac atatggaaga gagaactaag    420
gcaatagaat tcaactaaaa gggcttcatc tatgataatt ctgtttgctc tgataaaaat    480
tcctttatca tggaacaagg aaaacctgac atatttttgt tgagtcgagg acaagatcaa    540
gaaacatcct atgaggaata tttgtgaaaa ctcttagcca aatgcaagga gttttatcct    600
tgtatcttga atcataagtg cccctaggtt acatgaaatg tgtgcttgag gatttttaaa    660
gggactgtct ttaggtttct caggttctca aatagggcca cagacttact taaactgaaa    720
ttaggtgtcc agatattctt cagaatgtta ggaagaaagg agtttaaagg cagaaattgc    780
aggcatgact ttttaaaaat aaggttgtcc attgtctgct gaaagatgtt taggagttct    840
tgttgaatgg atgagaaaag atagtgacta gaggagtttt tcatcagaca gtgaaaggaa    900
attttaaaag taaatttggg agagcataaa gtgatgattt atttcatgtt atatgtagaa    960
gacagtgttc attggaaata tctagtggat tccaagaaat tagcattttt atggttgtat   1020
tttaaacatt tataagttaa aaaagtaata cttcaaataa tgtaagagat ttaatgattt   1080
aagaaagtaa taaattctgc catatataat tccctccctc cttccctctc ttcctttctt   1140
ttctcatttc ttctcccctc tctgtaagtt tcatggtatg ttataacatg tttttcattc   1200
ttcccttact gatagacatt taagtggttt tcagtctttc cctgcaataa gtaatgctgt   1260
gttgaataca ggcatatata cattctgtgc tcatgtgtga ttatgcttta gaacagattc   1320
ctagaaacaa tttctgggtg aaatgatatg cacattttta atatgagata gctggcggtt   1380
tacttttttta aaagataagg attcatagta ctgcctttat tgcgtgagtg aatttttata   1440
aatacaaggt attcttttttc aatatgcata aatattgaag aaaaaaaata atgtaggcat   1500
ttcgtattcc gtactctgta ctcttctgaa cactggcagt ccattctctt cttgaatgta   1560
tgtattcatc ccattcttcc tggaatgcct ctctccattc tcttcttgaa tgtatgtgtt   1620
catcccattc ttcctggatt gcctctctcc taccgctccc cctacccccca tctcctgtga   1680
ctcacctgtc aggacctact caggtaagtt tcccctaatc tgccagcagc actcatctcc   1740
cttctgtctg aactgtgata gcaaggtttg tgcatcaaag tggacacttc gttacttcgt   1800
tctacatgtg tcccaccgtc tgggagtgtg tgtgtgtgtg tgcacacgtg cgtgcctgtg   1860
tgtgttttac ccttatgttc tggattcgtt cattagaccc agttgtctca ggtgggagca   1920
gagcctcagg cacttctttt gatgtctttt ctcgctctca ttgaactcgg tatatacttg   1980
aggactggct gatcacattt atgagaattt tatatttgat agattgcctt ttgaaaatca   2040
tcattctgca gaaatttagt aattgaaaat ttaaatcaaa ttcaaataca actctaggct   2100
ctaatttcac tgttcgtttg aatgtatgac ttctctgtta agccagatac tcacgtgaaa   2160
caaatgtgaa tagaatagca tggaaatcgt gtatggacag tcaaggaaac tttctctgct   2220
ggcgacagca gcctgcagaa cataattccc tggacactct atgaaatcaa atccgtttta   2280
tcaactaatc agttggagca aaaaataata atagtgttat ttaggaaagc cgtaacacag   2340
ctcttacctc aaggctaatg aagacctgac ccttgaactc taggggcttc catgttccag   2400
cttgtgagcc ctgctttgcc aaaaccctgc tgaagaggta agagcctcct tctctggaga   2460
gaaagccccc agcctttgac aaagtgcttc tagcctgagc catgtgggct atgaaaaaga   2520
agttggtcat cccgaggagt cactggacag tggagagagc ccatccattt ttctccttta   2580
ttcttcccta cccctcagct ctcactccct taggacttgg gattctggaa tcataaaagc   2640
aaaatattct aaatatacta aaacatgcta aataacttaa atatgctaaa taaaattaaa   2700
actaaaataa acgtattaga ataaatcatg aatggctaat aaatataagc tgccaccatt   2760
aaaacaaaaa taacaaccac taccacaatt gctactataa agattattat tgatcagctt   2820
tatttttagg taagtagaaa agtgagatgt atttctattt ttctgagaat gtaaccattc   2880
tgaaagaata ggagatactt tctcacagta agcagaagaa tgagatgtac tcataaataa   2940
tatattcaac aaacagttat tttgcactta ctctatatag gtactgaaaa tagaatgatg   3000
ttaataaata tgaacagccc tcaaacagag atattaaaat aaaacaagaa atgaaggtga   3060
aataaattta gaagaaacga tggatcatta acttgtagta gttcttacag ccatgtgctg   3120
gatgcaggca tccctgtctg agtgtgtgcg tgtgtacaga cattgtttcc aaactttttt   3180
actataactt tgttaaagtg aagcgtgagc actccacatt tgctgcaagt ctcacttccc   3240
ctatttcctt tatttctcac atttacgtaa cttgtctaac ctctgaaaca aatgttttta   3300
```

```
ttagttctgg taatgcaaaa taatctttag agaattacgt ttttattttt gcttttgtta 3360
aataagatct acagtagcat aagttaagtg tgccaccttc attttgtagc tggctaaata 3420
tttctgcttc ctgtgtactt ttctgaaaat ggctttaatt tgtcttattt ttgataatta 3480
gcttcaatta gtgcagaatt ctcctttggt attgattgga cataaaacct gagatatgtt 3540
cgttgtattc tggaatctat tactgccaat aacaagccta ccgtgagtcg aattttaagg 3600
ctttcctttt tttttttttt tttttttttt tggctttgtg aatttgggtt ttttttaaca 3660
tcgtttttct ctggtagttt ttaagattta ccctttgtct ttgctgtctt atggtcttag 3720
tatcctgtgc ctaggcatgg atttttattt tttatctact gtgaactcat tctctgcata 3780
tgagactcat gcctttcttc aattctggaa ggtttttagg tattatcttc tcaaaacagc 3840
tctcttctcc attctctcta ttctttgttt tctggaatac tgattaatat ttgtttttat 3900
catatttttt attctatcct ccatgtcttt aaatcgtgct ttctaacttc cctctatttt 3960
cttgacttct cttgttacca cacaatgggc gggttcagtt gcttggcagg tgggagtcca 4020
atgaccacaa ccaaggagga tttaaccaag ggatttcatt actggcaaca agtaagtaga 4080
acactgggga tagttcccaa agcagtgcct ccccagacac aggtggaaac agggctttgt 4140
gggggctgct tagctgagtc actgtgtgca gaggtggagt gcaggcaggc acagctgttg 4200
ttcatgcttc ttcatatgtt gcccatacag aaaatggcac ataagctctt ccctgggcag 4260
ggcatttagt atggtaaggt gaagaggtct ccaaagttca tctccagctc aggcatcccc 4320
ggatgcctgg gaccagtttt ttgtgtttgt tctttgtttt gcaggggctc ggcttcttcc 4380
tagaacactt tttaaacaacg gaaactcaag gtgcaacagt taaaagtgga tagttcttca 4440
cagtgtgtac ccaaaaaccc agggaccctg ggttacactc ttgatccttg attagttgca 4500
caaggaatct tctatcattc tccgtccctc caagagttgt ttacgtattt tcaagctcaa 4560
attctttgct gaactcttaa ttttctctca gtgtgtctag actgctattt atttatcgaa 4620
ttttcaattc caacatctat aatttttata cctaatattt ctatctaatt ctgtttcaaa 4680
cctgcctgtt cttttatctt agattcctgt tcttgctgtt ttctttctaa tcttttcttc 4740
atctcaccaa acattttaaa catgcttatt tttagaatttg tttctaatgc ttccatattt 4800
tctaactctc ctttacaata gttcattttc ttgtgtggtt tataatattg gctatgagct 4860
catttccact ggctatgagc tcatttttca ggggtactct ggcctgaact gtgactgggg 4920
ttgtgtttgc tgcagtttca tggatatcaa ccgttcttac tgatactcgt gttacttttg 4980
tggcttaaag aatatattaa tgtgttctca tgctgctaat aaagacatac ttgagactgg 5040
gtaatttata aagaaaaaga tttaatagat tcacagttcc acatggctgg ggaggcctca 5100
caatcatggc acaaggtgaa gaagtaaagg tatgtcttac ttggtggcag gcacatctta 5160
catgggggca ggcaagagag cctgtgcagg gaaactgccc tttataaaac cgtcagatct 5220
cgtgaggctt attcaccatc atgagaacag cacaggaaag actcgccccc atgattcaat 5280
tacctcccac tgaatccctc ccaggacaca tggggattat tatgggagct acaattcaag 5340
atgacatttg ggtggaaaca cagctaaacc atatcaggga aggagaggtc cttgaactat 5400
ttgcattatg gggcactgt aaaattagac tacacacttc agttggccca ggtctggatt 5460
ctgatttctc tgaggagact cttttgcccc gcatggatct ggacagaagt caagcaacat 5520
tctgcttttt tctgagctgg aggccagggt tcttccagtc cagataaaat gcagaacaat 5580
cctttgtaac acccagcttt gtagagacac ctcagctcca acttactgtc ttatattagg 5640
tgcctcccaa ctcctctctc tattgggacc cagaagccct gaagatttat attgactcca 5700
agtacatctg tgaaccattt ggcttcagct gctgctcaca gctctgattt tatattctcc 5760
caaactctct gaccgctgga tagtttgctc atgtagcttt gagataattt atacattcaa 5820
aacttactct cttttatcta tttccttgtg ttgatttggc atagggtgag gagggtaagg 5880
gaaaaaggtg tctaaataag catagtccaa cacattgacc taaagcccat atatatatat 5940
gctgctaata acattatata tatgtatata tatattatat ataatttttt ttgaaacagt 6000
ctctctctgt cacccaggct ggagtgcagt ggcatgatct cagctcactg cagcctcctc 6060
ctcccgggtt caagcaattc tcatgcctca gcctcccaag tagctgggac tacaggcgtg 6120
agccaccacg cccggctaat ttttatattt ttagtagaga cagggtttca ccatttcggc 6180
caggctggtt tagaactcct gacctcaggt gatctgccca cctcagcctc ccaaagtgca 6240
gagattacag gtgtgagcca ctgtgtccag cctatgtttt tatattttta ggattcccat 6300
gtaatgatgc aattttcctt tctttgatat gatgcatcta tgattttata ttctaaatgt 6360
atttttatac ttatatataa aatgcatttt attaattaag cattagttct taaaagttgg 6420
tatttccttg aacttttaat tttacaaata ttccatttcg gtgttattat ttttagtatt 6480
ttttngctgc tgaatagtta tgccaacctc tgtgcagcct gggaagacaa cacatcagca 6540
gtcaatttgt gtaaagacaa aataatatta gagagcaaga gaatgagaca agactgcaaa 6600
tcagcaatga taattattga gtactgagaa caattccagt ggattaagat ttgggatgct 6660
taagcttctt aatattaatt gcagcaaata ttcaaatgct ttcactttcc atataagtac 6720
ttcagcaaat aagcaaaata tgaaataaaa accctaattc agaattacct aaatataaaa 6780
caagtaaatt gtcctaggca attcttacca tcttatgtcc agtaaaacaa taacctatat 6840
ttgtgcatac agatatatta tctcatttaa aaactagcgg tgattgtaaa tcttgacacc 6900
aaattgaagt tgaactgaaa gaggctttcc tgtaaagata cttaggatcc ttacattgta 6960
tgtaaggaaa gtcacctagt cctcacatca accccaggtg tgacatgtta ttaacttatt 7020
ctaatggtac agatgttgag atgcagagaa cagaagcagc tcttagggga cagagacagg 7080
```

```
gctcagtgat gattatattc acctgtcatg gggtagagct acaatagtga gtattctgtg 7140
gatggtccta tccagataaa agaaggaatt ggtttcagga catagtctgc atcttactag 7200
gtggggaatg ttggacaagt tacttaacct cttctgccct gtaaaatagg gataaaaatg 7260
gtacatatca cagagtgtca taggaagact aaagtagact attctcatcc tcacacctaa 7320
tgagttctca atatttaact gattataatt atctttacta gatacataat taattaaata 7380
aattaaggaa ttatagagca aatagtcata gggtcacaca gatagaaagt gggaaaaata 7440
ggagacaaac ctatgtctat attcattcca tggcattaga tttagattac ctccttttag 7500
tctggcaaaa cactcatgat tatacaatgt aatttaaaaa tatacagtac tttgctgggt 7560
gtggtggtgc atgcctgtgg tcccagctac ttgggaggct gaggtgggag gatggcttaa 7620
gcctgggaag cagaggttgc aatgagccga gatcacgcca ccgcactcta gcctgggctg 7680
tagaacgaga tactgtctca aaaaagaaaa aaaaatacag tactcacatt ttactttgct 7740
agtatatgct gtattcactg gtgtatgctc tcttgcatgg agactttcat tgggaataaa 7800
atgtttgaaa ctattttact atttcttgca ctgctttgag atactaccag caaagctatt 7860
tctaagctaa gacgctatgt cctggcagga cttctaaaga cttaaagcaa tgctgtagct 7920
gtgtatttac aactgactag agcagggatc agcaaaccat ggccagtggg tcaaatcctg 7980
cttactgcca gtttttgtaa acaaagtttt atcgcaactc cctgcccatt tgtttagaca 8040
tggtctgtgg ctgcttttct ccaacagcag aaaagttcag tagtttcaac agaaaccctg 8100
aggcccataa agcctaaaat gtttaccatc tggccattta cggaaagtta gccaaccccc 8160
agactagaat aaaggtagga aaaaccactc agcccaagct tctttttga ttctatcttc 8220
ccacagctgc ctagaatcct ttgcagtctt tatcctagga ggctcagagg ctataacaca 8280
tctctaattt tgtctagatt tgtgagaatc catttctcac aaatccagtt ctggccctct 8340
ttccctgact cgatgttcct tgaactctaa acatgaattt ttaaaagaag gattacctgg 8400
gatgggtata tccctgaaag tcacctcctt tctgctgcct ctcacaaata cacaggctat 8460
gtgccttaaa cctgtcattc actttaagga atttttgaaa agtggataat aggttagaca 8520
taaaattcac tgaatctggg ttaaatttaa tcagtgagta ttctttagaa tggaagcatg 8580
agcctgaatg attaaagaaa ttgcttaaaa aattagaatc aaaatactaa atatcatccc 8640
ccccccaatg ttttagctag aaaaattact tgaaaatgag ccagagaagg agaaaaataa 8700
ttatcttaca gaaaactaat aaaaatacaa taaactatca tttatttaaa tacaataaac 8760
tatcatttgt taaaatacaa taaacatgca aaagcgctat cattgatagc acttttaatg 8820
tttaaataac ccactgatta ctttctctag tggattattt gcaagggtag agtaagaatc 8880
gttgcttctc tggacacata acccatttgc catgtgacat tgcctccctt ccaccaagaa 8940
gtggaatgta tttttcctcc ccttaaatct gatgcctata actttctttc cccttgagat 9000
aatggcagag tggcattgtg caagttttgg aactaaggtc ccacgaggcc ctgcggctct 9060
ctctcttgtt tatctgagta ctgccctaag atgaccatgg agagaagccc agtctagcct 9120
agaaaaggag aagaggccac atggagaact gaggtcagcc agccccagct gcttgacttc 9180
tgaacaaagc cttctccgac tatttggccc tgttagaccc agctgagatc agcagaagaa 9240
tcacccatct gaccctagcc caaattgcct tctgcagaac cacgggccaa taaatagcag 9300
atgctttaac ccaccatatt ttgcagtgat ttgttatatg gcaacatatc attgaaaaac 9360
cctcatttta ccaaagaagg ggggtcatga cattaagcaa ctacctaaac ccacaatgta 9420
aatggcaagt cagggattta gatgcaaatc attccaacta aaaatgtata ctctaaatca 9480
atgccctgta ctatactctg tattttccaa aaactaatta gattgtgtca tcagcatttc 9540
tgaagacaga ggtagtcgtc tgggaagtag agttttctca accagtattt tgcacttgtt 9600
gggatttttg agatcaggaa tgttttggaa aacctgagat gtgcagtcac atctctgagc 9660
agtgttttaa gtttattcat tcactcattc attcattcag caaatattta ttgagatcta 9720
ctatatgtta ggcactatgt ttaccactgg aaggcaggca ccaggtaagt acaaacacag 9780
aaactgagat ggatggcatt gcattttcat ttgattacaa atttattctt gcttataagt 9840
tgattaccta ccttattaag agctgatttg tttcaagttt tagaactcca attgtgtcct 9900
taaattttg tctagcttaa aacatagtgc tagatttcaa gaagtaaatt gtcaaacagg 9960
tttgctgttt agtttttgta aaaaaggaaa cttgtaggca gaaacaaaaa ccaaaataaa 10020
taaaatctaa attatggtaa taactaaaga tagttgaggc attaacagag tttgactgaa 10080
ctctcaagtc tcaagcaaat ttctaaacta aactgttttc ttctgttaat cagtttaaaa 10140
gggaatacct tgtgctaatt tttcttggtg ttggtaacat taataaagct ctgtgaaatg 10200
aggccagaag gaacagaaat tgagtcaaaa taatgataac acctcttaca tattcttacc 10260
atcttgtctc ctagccacac acaccatgtt taaacctaaa aggagagaca cctggagggg 10320
ttaattagct tgcagctgtc tgtgactagt ctgcagagga taaaacggat atcagccgta 10380
agaaagcagc tagcactagc ctggatgatg aacaaagaaa agaagcagct gctgaggaaa 10440
agcacatttt gctgaccttt gaggagggct taagaggccc tttgccccct gaaaatggga 10500
cctgggaaga aagtcttgat ggcagacagt acaaaatcaa gggacagcag ctggctgttt 10560
tgtgtaaggg gcaaaattca agaggatttg tgaatcattg tgatttacag tgaccattaa 10620
ctattgtaat aaggagagaa aaatcaacat ccattcagtg cccacattgc aaattgccaa 10680
gcactggagg ggtactttca cctaggccaa caaacttaat aagcagagaa acccttcagt 10740
gttttggctc ctacagctca agtgtactag gttggcaagt ggaagttcct ttggtcatct 10800
ctgtttatac acttgtttga gatatttcat aaagtcagcc catttgttac tttatctgta 10860
```

```
gtatgtgctg tttcatgtgg aagatgcttg ccttttgata gtattccaaa ctgaccaacc 10920
ctgtacggat ttaagccact gagaagctat gaacagacag gtgaagtttt ggtattgcca 10980
ggaaccagct tgtccagaag tttaatcctt taaggaaaag gatagcaagc ccaatgcaac 11040
agcccttcat gctaaaaact ctcaataaat taagtattga tgggacgtat ctcaaaataa 11100
taagagctat ttgtgacaaa accacagcca atatcatact gaatgggcaa aaactggaag 11160
cattcgcttt gaaaactggc acaagacagg gatggcctct ctcaccactc ctattcaaca 11220
tagtgttgga agttctggcc agggcagtca ggcaggagaa agaaataaag ggtattcagt 11280
taggaagaga ggaagtcaaa ttgtccctgt ttgcagatga catgattgta tatctagaaa 11340
accccatcgt ctcagcccaa aatctcctta agctgatagg caacttcagc aaagtctcag 11400
gatacaaaat caatgtgcaa aaatcacaag cattcctata caccaataac agacagagag 11460
ccaaatcatg agtgaactcc cattcacaat tgcttcaaag agagtaaaat acctaggaat 11520
ctaacttaca agggatgtga aggagctctt caaggagaac tacaaaccac tgctagacaa 11580
ttgttaaaaa ctaaagttgt tggggaacaa attgtactta aaatatattt attgtgtaat 11640
ctgataaaat attttaaatt ctggtgttac aaagatttag tgtttctttt gttcttttta 11700
caaaacagaa acccactaaa atgttaactg attgacatta tattttttgc tttcgtcagt 11760
gttaaatact tgttgcacag tagtatgagt tgtttctatc atttactctt ctacaagcca 11820
gttgagacgt gttacgagga ctcaaaatat tctaattggc ggtgaccaaa tgtcatacaa 11880
ttatgaagta tacaagccat tctaaccttc acagtaaatt tttttaccct ctggttgcat 11940
acttaaatag aattatacta ataatgcata ttttctgagt gttaatgtca tgtgaaattg 12000
ctactaagtt aaacgttgct tcccatggtt ttctgggtga tgggtgttta ttctgcttat 12060
tcagtgcctc tctacataga ggaacctatc tcagtaactc ctatactact ctacaacaaa 12120
atggcagaag aaaagttacc catttgaaaa aataccttat tttccaaaca gaagtcttca 12180
gacaggcagt gcacaatatt tgccttccag aaaaggagat tgcataaatg cataacttac 12240
atgttagtac atgtgactgt ttccctgtct tatcctgaat gcatggaaag tcatgagact 12300
tatttttttc ttcctactgg attccattat aaaacctcta tttgtatagg ttataaattc 12360
taaccaataa ttgagcttac tatgttgagg ctgtaggcag aggataagat ttacatgtca 12420
gactttatgt tctgctcttt ggattaggct aattttcaaa agttattgac tgtaaaataa 12480
taatcatgca ctgaggaaat acaatattaa aataaatttt gaagaagatg gaattatagc 12540
aggggacctg tggaaataga atctaagaac atattgtaca tatgctgaat taatagagtt 12600
gcattgtgtc gttcacatag caaattcagt acagattgtt tgttaatgtc aacagattat 12660
gccgtaaaat ttgtaatttg tttggcaaat cttgacaatg ctgagacatt tattctagtt 12720
aatacaaatg gctaaggtct gtaattatga gttatttaag ctaagaaatt tggcaagttc 12780
attattcatc agtctctacc tactattcca aattaattga ctgttttata tactgataca 12840
attaatagta aacttttat attaagtata catatgtgaa ataaaatcat attattattg 12900
gattataaga tgttaataat ggtaagtcat gactgttatt ttgttgaagt gattttttag 12960
aatttttaaa ggtttattca acaaaataga gttatcttac ttaaaaatgg atgcttaggg 13020
ccaggcgcag tggctcacac ctgtaatccc agcactttgg gaggccaagg cgggcagtat 13080
cacctgaggt cacaagtttg agaccagcct ggccaacatg gtgaaacccct gtctctacta 13140
aaaatacaaa attagccggg catggtggtg ggcacctgta attccagcta cttgggaggc 13200
tgaggtagga gaattgtttg aacccgggag gcggaggttg cagtgagccg agatcatgcc 13260
actaaatttc aacctgggcg acagagtgag actctgtctc aaaaaaataa aaaataaaaa 13320
aacaacgctt agagattttt aaattaaatt agtttcaata cttctataat agttaaaaca 13380
agtggtttct atttaaatca caaatgatag aggttagaga tgaaactaaa catatgagat 13440
catcaagtcc aaccttctgc tacgatatta gacttcaaat ggaaagcaca tcaaacgtta 13500
accagatgat ttctttgata gtctgtttaa aaatattgaa ttaataaact ggaaacattt 13560
taaaaattgc tgttcttttg gaattaatat aaaaacatat actttaaagt aaggaatttt 13620
atactgatta agttatacac tgcttagcct agcatgtgta tgttatactt aaaaatgtgc 13680
attgtacatt gtcaaataat ttgcacacga tcatttacac atctgtattg ttcacattga 13740
cgtactttgt gtcatatata tttgtgcatt atcagttgat ttgcacattg cacaccagag 13800
tcacacagtt atagatctgt gtgaattccc tatattccac atgcatggac atgttcatat 13860
atacccacac aaatacaccc catgaaatga tatccagtta atgaatacaa aggtttttaa 13920
aggagaaatt cattagaaaa aaatcctata agcctctagt tttcccaatg aattaatcta 13980
agaatggata ttttattttt tccatgtttg acttgtaaag taaaacttta gtaataaaga 14040
gtgtctgcct tacagttcat atatttcttg aatttaaaaa actcaattac ataagaaata 14100
cattaattct tttctttaaa ctattaagac agcaaagtct tttttatgat tatctacaat 14160
tccggtcctc tctccggtat ctccaagata acatgattat gagtttaatg tatgacattt 14220
aaaactttga aactcacttt catatngntn tnnncnnnnn nntacataga atatatggag 14280
tgttgtttac atattattta tatgaataaa tatacaccta ctccaggtaa tttagtggtg 14340
actatcacgt attttatttg gtcattctac aattgattga tgccttttgt gtgtgtgtga 14400
ttactaatac tggagtatta atatacttgc atgagtctcc ttgtgtacca tgggaatagt 14460
tctgtatttg aaaatagaat tactgggtat gcacattttt aatttttaat atatacttcc 14520
aaattgttcc tgcatactcc caccagtact aagaatgtat ctgttttcct cttagcatgt 14580
caatatttga tataatgaaa cttgtaaatt ttagccaact aaatgagtgt gatttctatt 14640
```

cctttatagt aactgatgag gttataatca agttaagcat ttttttatat gtttattggc 14700
cactcaggtt tttttcttct acgaattgct ggttcacatg cttcgcccat tttccttctg 14760
gacttgtgtg tgtgtctttt tcttacatat attggtatat ttgacttttg tctattattt 14820
attttaaggt tataggattt tagagttaat gaaaggataa aatgctggag gcctagaatt 14880
cagaatcgta cataggattc taaacagaag ccatgttcta aataaggatt tggcttgtgg 14940
gacaaaagaa aaatgtggaa tttcacagnn nnnnnnnnnn acatttatgg gcaatagaga 15000
atcaagaatg actttgtcat aacatcggca tcattgaatg gtattgtcac ctataccatg 15060
atcaagaaat taacggtgtt gctctgcaga ctggcaaatc tcattgccct ttcttctaac 15120
cctgggaaag tattattaca atggaaaaag atatgcgaca agatattgta ttaatagggt 15180
gttagaatca aataaaagga gggatagatg tagtaagaga agcagatttt atagctatcc 15240
ccattccaag aataaaaaac aaaaaaataa taggcgagtt cattgtgggc atctgtcagg 15300
aacaaggaag gatagagctc tgaaagctga ctgcagtcac taacaaaaac ccttttgaga 15360
catattccaa gaacctgaaa aactggtaat ggacccttcc ttatactcgg tgtcttgaaa 15420
ggcagtaaac tgtctaatgg gatataaaaa tagagtcttt ttttcctccc ccactctaat 15480
attctcctat tttaaaaacc ttctttatat ggcactgcaa ataatagcac acagcaagaa 15540
ctgtactttg cagtattatt gagcatctca agtgtctgaa aggtacaaag ctaacaacca 15600
aatgacttca gaatggaaaa tgtcccagaa tttttaattt tccttctaaa acagaaatat 15660
aggggaagca aaaacaacct taatctaaaa tccagcttaa aatgacaacc agaggtttc 15720
tagcttatat tttatttcag aatgcttagc aaatttcttg actttgtatt aattttagtg 15780
agttgtaatt tgaggaaact catggttttc taagatcctt tatacctgaa aagttgtcgc 15840
atacgtgtaa gacttgcttt gttattgttg ttgtaaattg aatacctgaa ggtagatttt 15900
agtacactaa ataatcatga tgttcattct gtgctaatag gcaggaaaga tggacaatag 15960
aacttactgg gatgccaggà agtgctctga tcacccagga gtgagacatg agatgggcag 16020
aaagaacaag attagttaac tatcatggga aacataagaa gaatttggct ttagggattg 16080
tgtgtttgag attgattttt aaaagaaaaa gacctattta aaaatccaca gatgcttagg 16140
gaatagaata gctatttttt taattcataa attttatatt ggttagtttt cacagcagtg 16200
aattatcaac tttatataaa agtaaaatga taatttgata tatttcctga tatgagtcca 16260
cattttcaga gccatctaaa ttcagccttg tttgttcatt atacatatat aagaaaatcc 16320
ttcttctatg caacaaatgg ctgtatataa aatatacatg acttgttgat ttaaaatctt 16380
ttaccttttt ttctcatcat aaaggtaaaa cacatgcatt tcataaatgt ttgaaaatgg 16440
gaacgtatgt accaaaaaaa tcacttcaaa atcatggttt acgtatcttc taaaaacatg 16500
taatcaaata taacaaagta tatttaggcc aatattttga ctatgataaa atcgctttga 16560
ttcagaaggt attgtgtttt ctgagtattt cattatttaa agcagaaggt ttcatctttc 16620
agaagtgtca ctccagcctg ggtgtcctgt gtggttgagt caaatcagtg cttccactat 16680
cagtaggttt ggcacaggat gaagcaagaa catcaaacta aaggggccaa gaacaatagg 16740
tgggagaggc tgcaggagga gcagagaggt cctgataatg aggttccctg actatagcag 16800
gttaggacta gacaagccct gtgaccaaca gaggaaagag tttctgagca ctttctatat 16860
ctggctgtac cttgggggac ttcctccatt ttgtctgggc tcatcccacag tcctgctaag 16920
agattctgca aaatatactg cagttctcat tcatgtgccc cttgagatgc atttgagctg 16980
caggtatttt ccctccagtg gtacccagtt tattatttct agtccattca actcataaca 17040
acatccagca gcactttacc cacaggtggg tgtttattag gtttttattt cttttgtcca 17100
ggtttattga gtttgaagag taggcaggga tttctgtttt tatgagtcac ttgtcagctg 17160
taatgaagtc ttcttcacta aaaaaaagat gtgaggacaa tcttgcgcag tgtcataatt 17220
attctaaaaa tacatcatgc ttaattgtat ccacaaaggt tttaaaaatt taaacatcag 17280
ttttgagttt tagggctttc ttattggtga attatcaata attgtagtgc tttcttgaaa 17340
agttccctgg ggtggatttc cttagttgca tataattcta gcctcttcag cctactaagg 17400
ataaaaggga catgaagtac acatatctgg gaattaatta catttttctg ctcaagagcc 17460
aagtcatttt cagaaattga tgtcaaatag gtcttccata gctgaaagtg ctaggtgtct 17520
cttcacatag ttaagatttc cccctattca gaaatattag tttctgatga caatggaaaa 17580
ttccaaatgc agcagcttca ctgtgttcta ataatgttca tgcttaggga tcctgaaacc 17640
acttgcagta tgcctggtct agccacagca gggagccggc gcccatgcca gttcccagag 17700
ctgaccaccc cnaccgcagc cagcgtgtct ggctgtgcac catggccaga ctccatgctc 17760
actcactcat gcaccccgca ctgctcccca cctggctcaa ccttggtggg catgggatcc 17820
aggctggtag tgaaagtcaa atgctgcctg ccaggccggg tgggcagaac gagcccagcg 17880
ggtgagaaaa acttgggcaa acgtgccact ggctacagag gtttccggct ggtgaagtga 17940
caccctaagg attctgtgac aatgggaaaa ggggtaatga agggatagag cagattaagg 18000
aatttaaaac ccagttgagc aagtatatct gtaaataatg tacttttgta tactacaaaa 18060
ccacaaagaa aggatctccc tcctctatta tcacagggtc atcccggaag aactgacatt 18120
tcaatacgtt aaaaaataag tagataatgt ttttagcttt aaaaaaaagt ggaataatct 18180
atgcatatat aaagaataaa tgcacataac atttatgtgg gttatgtaag cagaggtaag 18240
cattataata aagcaaacac tgatgagcca ctggtgagga aacataacct tcctaaatct 18300
ctgaggcttt cagagtggct cttggcccat gtagaggtaa gggtgtgaaa gaacaggaag 18360
agagggcgtc catgtttgag aaaccacaaa atcacaggag tcgctaaagg agtagtttaa 18420

ttggttatat ggaggaaatc tggagtgatg ttagaagatg caattgacca gatcaggtgg 18480
atcacagagg ccatcctgag cagtacaata ggaaattaat cagggatccc ctatggttac 18540
cataccttat gggtttaaac caatgatgct tacgagggag aagttagctt ttctggacaa 18600
tgtccttggt gtgcactttt cagatggaga ttggcaagcc aatggctaag gagggtgaat 18660
cagatcaaaa ttcttccttg actttcatag catttccaga ggatgtctag tttatgagta 18720
aaatgtcttg ccttcatctc ataaaattat ctctatgtcc tcacctgtaa agatgttcta 18780
tatcanaaca ttattattta tacaaataat tatattttct taaaaagact tttttgtaca 18840
gtaaaataag tggaagcaaa cttaaatgat tcagaacaac tttctaaata tttgactaaa 18900
caatttcagg aaatagccat ttaatccctc gaaaacaaaa tttggcttcg gttaattttt 18960
agaaaatcct aagatgagga gatgaagcta aaaaaaatat attgaacact gtgcatttca 19020
ataaagaatt tacagaatag ctggaaaatt aaggttaagt atacaaggta attatatatt 19080
tatttttaaa atgaaaaggc atttaacaat ctaaaaacta catcaaatac attatattta 19140
gcatcatgaa actacttaat tatctgcacc tatcagtgaa gcattttttt tctcataaat 19200
ctgttctatg ataatctaca ggaagacata attttaaaat ttgtaaggat tagagtggat 19260
ggtcacctgg tcccaacctt tggaacattt gaaaagcaag gaacatataa actgaacatt 19320
aattttctcc ttgcaatgta tacaagaaat ttaaaaaaga attttccaga gtacaggtat 19380
aaaagggtat attcttgaca gggaatcaga gtgctgacag atggctaaga tttccatcca 19440
agaaaaattt gatgtttgaa cttaagaaaa tggaaanttt ctttgctgga cctcanataa 19500
tattttcagt aatgacagaa agtcaggaaa gatgaatgtg gaaacaaaat gttcttgaac 19560
acatgaaata aataggtaat ttagatacag tgggtggtgc cagtatgctc tgtaggtgaa 19620
gaataaatct ggaatcctag gaggtcgccc taaccagtga ggtctctctc acttatgaac 19680
ataaaatgtc tgggtactta ggactcctct aggcaataaa tgaccttcag ttagattgag 19740
ggctgatcga actgagaggg attagtacat aatttaatct atttgctaat tagcatgatg 19800
aattagacca ttgtcatctg ttgctgtgtg ggagaggaag aggacaaggc atttaagttt 19860
tgatgagaag gacatttatt gaatagcatt gtacacttta agagtggatt ctcatatatg 19920
atctaagtat cttagtctat agactaagtt tgattctttc ctttcattag tgacagagct 19980
acaactgtca cttgagtatt gggtgacttg acttctcttc caagtcgaac acactgcata 20040
acatggaaag aaggcaagga agaggagaat atatcttgtc ccatcactgg tcatttcagg 20100
ggagattttg gcactgaaga cagatcagga gcatatcaga taggaacaaa aaaaacaggc 20160
ttggtagctt aaaaggagga gtcaagtctt accaactctg aacactcacc tcagtagtga 20220
tgtttcattt ttaccctctt agggtaaaag agtctggtat ttctaagtat atgctttcta 20280
gtgtttgtgt ttactatatt taagaaacac cattagatca attcttatta atgattactt 20340
gatattaatc attatgatta gacactagtt taaaagcata gattagtttc attaaagact 20400
tttcttgaga gattatatat taaatgggtg gtgtacacat tcttactaag aaagttttct 20460
gctgtttata tcttaaatta atgtagaatt tctcccttaa tttctacata gaaacttagt 20520
atagataagc ttgtgcagtc agctgtgaat tcatctaggc ctactaccat ttcttcatct 20580
cctctaaaga ataaaatatg aactatcttg ttgaaattaa gtgctactag atctatactg 20640
ctagtgatcc tgtaatatca atacatgcat gcatacatac ataaatattt taagaaggaa 20700
aagaaatctg gttggaactg atgccttccc agtgagcctt tgctggctct tgacagtttt 20760
cgctctcttc tttaagggtg ttcagattat ctagactttc tagaattaat gattatttta 20820
tcgttttgac aatttttctt tctaagttaa aaccacattg gcatttgtg aatatgatca 20880
tatttctcat tttgaccaat ttttccctag cattacagtt cgttaattta ctggtaatta 20940
aataatgatt aaacatatta attaaacaaa taatgattat ttatttttgt attacgtttt 21000
attccttaaa gcctctctcc atcgttaacc cttttccttt gtnnnnnnnn nnnnnnnnnn 21060
nnnnaagccc ttccttagca tctcactgga ggtacgttca ggcagcatca ctttagtatc 21120
tcgccagcat tgtcctcttc ccgccagtgg gaaccaacta acaagtacac tctcaggtat 21180
gaacttaagt acaggcaagt caacagattt catatgcatc caaatgacac gttaaataca 21240
aagctttgct cacctgcctt tgtacaggag tctgctgctg ctgggtttta atgaaactta 21300
aaggctgaat caaagcctta ttagtgggag gatctttgac atgaaattct ttttgttcag 21360
tataatttgc attttgtatt catttgaaaa attgtcctgt gcatttgttc gaatatcgag 21420
aatagagaac tgagtgattc ctgggtttat gtgggtgata ttgtggaaat aagaggtgct 21480
gtggtatgta cagctagtcc ccaaatatga ttgtatttta ctagagatag agaattcaca 21540
gtgactngaa ctattcaggc tgtaaaatat aggaaccatc tgccatagaa taagcatatt 21600
aggaaaatgc cacctattcc tacattagtc tggcccttgc tccaaaattt cacatgtact 21660
catatgtttg ttttccgtgt ctctgattga tgcagctagc taatgcctaa tgacagtttt 21720
tttaatcatt ccctatatac attttttat tttattatta tactttaagt tttagggtac 21780
atgtgcacaa tgtgcaggtt tgttacatat gtatacatgt gccatgttag tgtgctgcac 21840
ccattaactc gtcatttagc attaggccta tctccaaatg ctatccctcc cccctccccc 21900
caccccacaa cagtccccag tgtgtgatgt tcgccttcct gtgtccatgt gttctcattg 21960
ttcaattccc acctatgagt gagaacatgc ggtgtttgat tttttgtcct tgcgatagtt 22020
tgctgagaat gatggtttcc agcttcatcc atgtccctac aaaggacatg aactcttcat 22080
tttttatggc tgcatagtat tccatggtgt atatgtgcca cattttccta atccagtctc 22140
tcgttgttgg acatttgggt tggttccaag tctttgctat tgtgaatagt gccacaataa 22200

acatacatgt gcatgtgtct ttatagcagc atgatttata atcctttggg tatataccca 22260
gtaatgggat ggctgggtca aatggtattt ctagttctag atccctgagg aatcgccaca 22320
ccaacttcca caagggttga actagtttat agtcctacca acagtgtaaa agtgttcctg 22380
tttctccaca tcctctccag cacctgttct ttcctgacat tttaatgatt ggtgtgagat 22440
ggtatctcat tgtggttttg atttgcagtt ctctgatggc cagtgatgat gagcattttt 22500
tcatgtgttt tttggctgca taaatgtctt cttttgagaa gtgtctgttc atatccttca 22560
cccacttttt gatggggttg tttgtttttt tcttgtaaat ttgtttgagt tcattgtaga 22620
ttctggatat tagccgtttg tcagatgaaa aattttctcc cattttatag gttccctatt 22680
cactctgatg acggtttctt ttgctgtgca gaagctcttt agtttaatta aatcccattt 22740
gtcaattttg gcttttgttg ccattgcttt tggtgtttta gacatgaagt ctttgcccat 22800
gcctatgtcc tgaatggtat tgcctaggtt ttcctctagg gtttttatgg ttttaggtct 22860
aacacgtaag tctttaatcc atcttgaatt aatttttgta taaggtgtaa ggaagggatc 22920
cagtttcagc tttctacata tggctagcca gttttcccag caccatttat taaataggga 22980
atcctttccc catttcttgt ttttgtcagg tttgtcaaag aacagatggt tgtagatatg 23040
tggcattatt tctgagggct ctgttctgtt ccattgatct atatctctgt tttggtacca 23100
gtaccatgct gttttggtta ctgtagcctt gtagtatagt ttgaagtcag gtagcttgat 23160
gcctgcagct ttgctctttt ggcttaggat tgacttggtg atgcgggctc tttttggtt 23220
ccatatgaac tttaaagtag ttttttccag ttctgtgaag aaagtcattg gtagcttgac 23280
cacatagttg gaagtaaagc actcctcagc aaatgtaaaa gaacagaaat tataacaaac 23340
tgtctctcag accacagtgc aatcaaacta gaactcagga ttaagaaact cactcaaaac 23400
cgctcaacta catggaaact gaacaacctg ctcctgaatg aatactgggt acataatgaa 23460
atgaaggcag aaataaagat gttctttgaa accaacgaga acaaagacac aacataccag 23520
aatctctggg acacattcaa agcagtgtgt agagggaaat ttatagcact aaatgcccac 23580
aagagaaagc aggaaagatc caaaattgac accctaacat cacaattaaa agaactagaa 23640
aagcaagagc aaacacattc aaaagctagc agaaggcaag aaatagctaa aatcagagca 23700
gaactgaagg aaatagagac acaaaaaacc tttcaaaaaa ttaatgaatc caggagctgg 23760
ttttttgaaa agatcaacaa aattgataga cctctaacaa gactaataaa gaagaaaaga 23820
gagaagaatc aaatagacgc aataaaaaat gataaagggg atatcaccac cgatcccaca 23880
gaaatgcaaa ctaccatcag agaatattac aaacacctct acgcaaataa actagaaaat 23940
ctagaagaaa tggataaatt cctcgacacg tacaccctcc caagactaaa ccaggaagaa 24000
gttgaatctc tgaatagacc aataacaggc tctgaaattg tggcaataat caatagctta 24060
ccaaccaaaa aaagtccggg accagatgga ttcacagccg aattctacca gaggtacaag 24120
gaggaactgg taccattcct tctgaaacta ttccaatcaa tagaaaaaga gggaatcctc 24180
cctaactcat tttatgaggc cagcatcatc ctgataccaa agccgggcag agacacaacc 24240
aaaaaaagaga attttagacc aatatctttg atgaacattg atgcaaaaat cctcaataaa 24300
atactggcaa accgaatcca gcagcacatc aaaaagctta tccaccatga tcaggtgggc 24360
ttcatccctg ggatgcaagg ctggttcaac atacgcaaat caataaatgt aatccagcat 24420
ataaacagaa ccaaagacaa aaaccacatg attatctcaa tacatgcaga aaaggccttt 24480
gacaaaattc aacagccctt catgctaaaa actctcaata aattaggtat tgatgggacg 24540
tatctcaaaa taataagagc tatctatgac aaacccacag ccaatatcat actgaatggg 24600
caaaaantgg aagcattccc tttgaaaact ggcacaagac agggatgccc tctctcacca 24660
ctcctattca acataatgtt ggaagtcctg gccagggcaa ttaggcagga gaaggaaata 24720
aagggtattc aattaggaaa agaggaagtc aaattgtccc tgtttgcaga tgacatgatt 24780
atatatctag aaaaccccat tgtctcagcc caaaatctcc ttaagctgat aagcaacttc 24840
agcaaagtct caggatacaa aatcaatgtg caaaaatcac aagcattctt atacaccaat 24900
aacagacaaa cagagagcca aatcatgagt gaactcccat tcacanttgc ttcaaagaga 24960
ataaaatacc tagnaatcca acttacgagg gatgtgaagg acctcttcaa ggagaactac 25020
aaaccactgc tcaatgaaat aaaagaggat acaaacaaat ggaagaacat tncatgctca 25080
tgggtaggaa gaatcaatat cgtgaaaatg gccacactgc ccanggtaat ttacanattc 25140
antgccatcc ccatcaagag attttttttac acattccata tgtgaatatc ctattttctg 25200
attcagttct gactttattt ccattatatg tgaatgtgtt tctttactat ttttctatca 25260
gtttccttcc tggtcaattg actatatggg aaaatatttt atccattcaa tattcaaaaa 25320
tagtttattg aggacccact atgtgctagg catttgatca caacagtgaa aaacagagaa 25380
aaaatccttg ctttcatgga gtttacaaat tctaccaaag gaaacaggaa ataatgaaaa 25440
taagtaagta aaataaatag catgctacat agtaataagt gtcaaggaga aaaggaaagc 25500
agacaaagag aggggaaggt gtgagcagga attgcagttt cagataaggt ggccatgaag 25560
gtgtcccttc aaggaaaatg tagtagttgg gaaggggcaa gtcacatggc tctctgggga 25620
agagtgtgca gggcatcggg agcagccctt gcaaaggcct tgagaaggaa gcatgcccca 25680
tgctctcctc ctcatatgag gaggccaaca ggtcttgaat gaagctatgg agaaagagaa 25740
agggaatacg attggaaagg taacaaggaa tggatcacgt agagccttcc agggcaacgt 25800
aatgaccttg gctattcctt ttaaatgaga tggaaagccc gtggagcatt ttgagtagag 25860
gcgagacaaa actgatcagg acaactctgc ttgctgggca gagaatagaa tcaagggaag 25920
caatgacaga agagactgtc ctaagagtcc aggcgagaga tggcagtggc ttggacaaag 25980

gtggtagcag tggaggtcac agaagatgct gaatatagtg ttcatgttta ctttatggga 26040
tttactaaga gattggctgt gtgttaaaag gaaaagatca ttctaaagct tctggcctca 26100
gtgattagat agacttgcca ttaacttcca gaagagcatg tttgaggga aatatgagga 26160
tctcggtttc attacatgtt aagttccagt aatgtgatta gattgaaaat gctatagctg 26220
gtagaatttt ttcacttttt atgtcaaaac agaaaacatg attgaataat gaaaactaca 26280
catgctcaat tttttaaaa ttgttttctt tgacacagta aaggatcaat gagacttgct 26340
taattcttgt caagaataca aagtcaacaa tggctgagat gaaaatattt tccataaagt 26400
aaaatgatca aatttgcaaa agcttggtta catgatgtga atttcactca aaccacaagg 26460
cattacacta gagtcttgct gaaatttaag acagactgtc ttaggaatta tttgccaata 26520
gacaaaatga atatgggtg gttgatgtgc tgaagtggcc aatgagcatg ctttgattga 26580
cagatgagga aatgtatccc tacattttat caaactatgt ggtgtctgat ttatatcact 26640
gaggtggcca ttcattatgg tcatgaactg tgaggtcaaa gcacttgcaa aacttgcca 26700
aaatacttcc tattccagta ctaaatattt ctcatatttg tttgcctaga ataacaagat 26760
gcaggcttgg tagtcctttc aagaatgtga atttctttgg tatgtctgag tttagtaatg 26820
ttgaattgtg tgtgtgtgtg tattattctt aagactttgc ctgtatttta acattttatt 26880
tggtaaaaat agcattattt tggcaagatt tttaaagcct tgaaatggga caaattaaag 26940
tataatgctt tttcttgaga aataatttat tgctccatgt tgttgaatca taccattagt 27000
catgaaacca aaaccctata tttaaaaagt aacaatctca tattcaaagt atctcctttg 27060
aacatttcaa agaatcttaa gcaaatcttt atgtgacatt aaattgtaca ttttaccttg 27120
ttattttaga tctatgggtg gagttcattc ttcgggctaa gtgtaggtat gaagtgtgaa 27180
tttctgtgtc ttggtatgcc agtaacttag ggcaaggtta gtagagtggc ttttgtgaga 27240
aaaaaagagc tgttttatca gacatcagtt ggaactttgc cttgttttgt ttccctgaac 27300
cgtccaatct gcttcatata cttgtgtgaa atcctaaaat gtctgtatgt caataatttt 27360
tagttagcat atttttcca aacagacatc cttgtgaaca tcagccgttt ctgagaactg 27420
ggcccacgga ccatagctac tttgagtctc agtctatgta atagttgggg taaagtaagc 27480
tttaaagaga gaactcacaa cacaatggct caagcaagac acaagttgat ttctttctga 27540
agctgccatc ctgggtatgt gggtatttgg atgagctggg ccccatgaag tcatcccaaa 27600
gtgcaggcct ctgtggcatc tctgccaaac tcaacactta ctctccaagt ctgtccttgg 27660
cttctctatt tctagctggt ggaagggaag ggaaaaatga gtccagggaa ggagtttttg 27720
tagatggctt gggagttgca tacatcagga agaactttgt cacaaggcca caactccttg 27780
caggaaattc taggaaacat attccctcac tgagcagcca tgcgcccagg aagaagagaa 27840
gaatgagtct agagtcgcaa gcagcaatat ctactacagg cttaaataac caaaggacaa 27900
aatgcccagt ccattataaa tatattccaa aaatgaagcc atgaaatttg acttcattta 27960
ttgaagtcaa aacaaattta ttgttatgaa ctaatttctc tattttatat ttctcaaatg 28020
attacatttt taacacagaa caaagccata gttagaattt cagtaatcgt atggaatatc 28080
caaaaggtac acccaataca cccaaaagct aaaccagagt cactgctaaa ttttatattg 28140
ttctgattta cctaacagat tagtgaaaat aattaaaatc ttgctgcttt ttaaatttta 28200
aatatcggat tttggttaga actctaagaa tatttaaaac tatctttacc tactcttgtt 28260
ggacaatcat ttgaggagct aagagtgcag agaaggcaga gaaatgttct cctcaaccca 28320
agcaaaagca cagaagaact attgctaatc caaaatgcag gaaggaaatt tcacccctcc 28380
tcccttccct tttcttgggc tagtctgttt aactttaaaa gcagaatttt tgctctcaca 28440
aagtatacca gacaggaatt tgttcaaatt cctcaaaacc aagccaagaa aagtttcaga 28500
aagttcagac atgcatggca gagtggaaag aacatagtct gtggaggcga gcacggattc 28560
attggaatgc cgctccagac atccactctg ggtgaactca gggatgtgtt tagtctcatt 28620
aagccacagt cttttcattt ttaatgtaag agtgataaga acgacttgcc agtgtgttgg 28680
tggagaactg aaatagagaa aggcctggtg tgtaacagga tgtttctctc catctgtgta 28740
atctgtgaca gtcctaggaa gttcaaagtt atgactgttc tgggtttgtg gccaacagtt 28800
gtctgtggct tccatgggtt ggagctgtct aatggggccc agnccaccat cgctctgctc 28860
agaagctaat cataccaaaa catgaggttt ttctccaagc gacaaagagg ggctaagcac 28920
ccaaggatct ccataatagt agcaggaaaa tgaagctgag accttaccct gacttagaag 28980
ttcataacaa taatggcccc aaacaagatg ctcatgtcta ttggttgtgg ttaataatca 29040
tgcaacaaca tgaacttcaa acccagccta tgcttggctc ttccagggac tgaaggcatc 29100
tcagagcccc accccttacc tcagaaaccg caggaaggag cctgggttct cagagtccaa 29160
gaaaatgcac ctgataatag cacctgaact tcctccacac aaagaaagcc tgatagggac 29220
aggggattaa gcttgcatgg agggcaggat ccaacccaca gtagcacttc cttgccatgc 29280
gaccttagca aatggcttaa ctcgctttct gtatcagtaa aatggtgttt gtagtactaa 29340
ggtgtcagtg aggattaaat gagccaagcc tgacccacag caattcatga caagtctcag 29400
ccactttaat gaatgtgatg atattctaac gtttgggatg ctgaggtgga ggacgatggt 29460
gtcccacctc ctgcaaatgc catgtgaaca ctgaagctac acaagcaatg gctctaaagt 29520
caaagccagc atcaggactg gtataaaaga atcaccagga caagggcagg gcagagggga 29580
ggccaaagca gctttgtcag tgcctgcacg gctggaccta gaggaatttc tcgacataga 29640
gggtaaaaca acccattggc ccacatttct gaggaccgac gtgggagaag ggtcacatga 29700
ggccatgaaa tgggcatctg cagaggaaat ggaagccagt gtctggagac aagtggagcc 29760

attgagctag caatgcgtga tagatactgt gggaagggat gtggggaatg cagcacctct 29820
caagggaggt ggctgagtct ggcaatgagg gcctggggca tccttagtca gcaaagcagg 29880
tctgcagcct cgggagctct ggcagctggt gtgggtcacc aactggcctc gggcaccatt 29940
catggaaatt aatctggata gtcccctcct ggagaccctc caggatggca gatgagcaga 30000
ctggccggct ttcaaagtct gctgacctct ataattgtgg ctcttcacat ggtccaacac 30060
actcttccct tttacatgtg tttcctcttt gaatatttcc accagcccct ctccctgacc 30120
tctctctgct ttgttacttt cctttgccca tttaatctac attttatgtt ttgtccagat 30180
taacatacct gtgtttcatc tgccaggcac tggcccaggc cctaggaata tggtggtgac 30240
caacacagag tggctgcttt catggagttt acaatctaca aagggacaaa aaggataaac 30300
aactttaaac tttaaaattt ggacaaactt ttataaatac tgcatttttc caataaaatg 30360
agataaacaa tggtataacc tacattgtac agaatctgtg tctccctaaa attaaagctt 30420
tgagataaaa ggagacaaga ggtattgttg gacttgaatt tgtcgtgcaa ataagattaa 30480
aaccttgaat attagccttt tgattgttgt ctctttttgc agtatgaggt agttgtgaat 30540
tctaggtcaa aacataaggg ttaaaaggct ggaaaaatta aataggccaa atgaaagaat 30600
tgtcttgcta aaatgtctat gtagatgata aaatgtcctt gtgcctcttt aacttcaatg 30660
atttgtgctg gatattttat aagcgtccat taggcaatag caaatttatt caactataag 30720
agaattacga aacacaacta tcataggaga tacttatttc cttccaacca ataagtaacc 30780
accagtgatg agaaggcaga aagtgacaag attaaccaag taagagagtt ggatacaaca 30840
ttcaactcca tttcttcatg tcccaataag agacaacgtt tgacctgccc tctcattcct 30900
aatcttgttg aatgaatcta gtacagaggt tcataaaaac cgtcaggtat ataatgccgg 30960
attttgtcaa caaacaataa caataatttt ttctttaaaa attattccag gccaggccgg 31020
gtgcggtggc tcatgcctgt aatcccagca ctttgggagg ctgaggcggg cggatcacga 31080
ggtcaggaga tcaagaccag tctggccaac atggtgaaac cccatctct actaaaaata 31140
caaaaatcag ctgggcgtgg tggcagggac ctgtagtcgc agctactcgg gaggctgagg 31200
caggagaatc actttaacac gggaggcgga gattgcagng agctgagatc cagccaccgc 31260
accctagcct ggcaacagag caagattccg tctcaaaaaa aaaaaaaaaa gaaaagaaaa 31320
aaattattcc aggccaggtg tggtggctca cacctgtaat cccaacactt tnggtggcca 31380
aggtgggtgg accgcttgag ctcaggactt caaggccagc ctgagcaaca tggcgaaacc 31440
tggtctctac aagaaataca aaaattaacc aggcgtggta gtgagtgcct gtagtcccag 31500
ctcctcatcc gcctgtaatc ccaggcggag attgaagtga gccgagatgg caccactgca 31560
ttccatcctg ggtgacagag ccaaaccctg tctcaaaaaa taacaaaata cacaccaggg 31620
cctgtcaggg ggtgggaggg aaggggaggg agagcatgag gacaaatacc tagtgcatgt 31680
ggggcttaaa acctagatga tgggtcaata ggtgcagcaa accatcatgc cacatgcata 31740
cctatgtaac aaacctgcac attctgcaca tgtatcccag aacttaaatt taaaaataaa 31800
ataaaaataa taacataata aaataacaaa aaagtcttta catctgaaat aagtgtactt 31860
aaaattaaaa ataaaattaa agtacaaaaa cttaaaataa aaatcatttt agatatttcc 31920
tcagatttaa atggaggtca ttgaagctca aacacctggc tgcaaagtga tttgtaagac 31980
gaacacctac actctacaaa aagacttatg aataccctgc attggttgat gaacaagaac 32040
agtgagttat accttctgaa tagtgaatta aataaaatac tattaaagtt ttaaataatt 32100
gccaattgct ccatatttct ctgcatgtta gagtttagct agtgtttcca gggatgattt 32160
ttctgtattc ctcccagtca ggaatagcca gtcagtatta ctccatcagc cacagcacaa 32220
atgtctgatt gctctcttga ggtcgttagc ccagatactt gctagaaagg tatgattgct 32280
ggccaaatga gctctggcct ggtgcagccg tcttgccaat gtgcctaatg agagcgcgga 32340
aagacatttt attccaggca taagcaggcc agatcacagc tgcccaaagc tctctggctt 32400
tgattaagtt tgttgtgcta ttatgtaatg ggtacttgaa ccatattccg tgaatgaccg 32460
ttttccttcc tctttcgcca ctattctaag tatgttgtct gtatctaatg ttgccttgca 32520
gttagttgtt aaaatcaagc agaaaagaaa acaggcaaat attaaaacgt aaatagaaaa 32580
agttgttggt ggggaaaatg acttataatg cattattctg acataatgtc ttggggtcca 32640
ccaggagaat cgccccatca caattcagcc tctctctgtg ctaatgacgg ccttttcatc 32700
ctgtgctgtt agtgatgcat aagccaaatt gtgaccaggc agactagacc acatgtaagc 32760
agagaaccac gtctttgtag acattaattt aaaactcgtt ctaatcaaat atgttcattt 32820
atctgtacta tttttgatct atatcttaaa tcaaaacgaa gctttttgga cttctggttt 32880
ctgtttctgc ttgtaaagag cttggaagtt atcaatcctg tcttcataac aagaaaaatg 32940
ctgagcacac tgaaagtcag taactggaaa tcaacagtgt tttaaaagtt aattggttac 33000
aattttctca tattcttctc tcacctctgt agagttctgg tctacattgg aaatgctgga 33060
aaagtaggaa tatacagtac agagctttct tggcagcata acttaataca tgaaaaatga 33120
tttttaattt tagtttaacc tttaaactgg aatctgtagc acattggaaa aaaaaaacct 33180
aaaccagttg aacaaatttc aattttaaac atgttttcag gctttggaag cttactgaag 33240
aatgtggata gcaaatacag gaaaaattct gtgaatattt ttattcacat atgtaaacgt 33300
gaagttataa gacagaacaa tttccccatg tacaagcttc aaattttag cagttagtta 33360
acattttcta ttcagtttta accattaagt tgtaatatta ttacaaatga tattagaaca 33420
catctttgct tttagtgctg tattataaat atttaaatta atgacattca gtgtaaattt 33480
ggtatttatc gtaatacaaa taagaacttt atttttagtt aattccatgt gttatttgta 33540

tttcttatct cacatttatt taaaattaca tttggtccct atcactcagg tatgtttctt 33600
ccctcccacg tagacctaat cggatttttt aaaattggat tttaatctgc ctttataaac 33660
aaacgtcaat tatttatttc caatcttggt tgatcttttt attccgagat tatttactag 33720
ttcattcagc cttcctatta aaaataaata aataaataaa taattgctct tgtagctctt 33780
ctgaggtaga ctgaccaggt ctaccttatt gttaattttt gcattttcat ttttattgaa 33840
tatgtatgat gatgcagata tgaaccatgt gcatatctca gaataggagg aaaaatatct 33900
tagtactttt gccaaatgct tggttctaat ttttggacta ttatgaattt tttatgtcat 33960
aactattatc ttaacattat gcgaacttct agccctttttg cttcaatttc ctttgaaaag 34020
atttccttcc aataaatctt taaaatatgg gtgacaaaac taaaagaccc tcagaggaaa 34080
catatttctg gtatgacata ttttgtgtac tctttaggac aattgaaaag cttctaatta 34140
acatttcttt agcgccttag tggtgatcag cagtatcatc actgccctag tgcatccatg 34200
gaagttgagg tccagggtgt actgactcac acaggttacc taggtaagaa gtaatggatg 34260
actggaatgt taggaactgt aaatgacagc taactgtggc aaagcgtagg gttttgtttt 34320
tttttttctt gtcttgtctt tttttttga gacagagtct tgctctgtcc ccgaggctgg 34380
agtgcggtgg tgtgatctcg gctcactgca ccctctgcct cccaggttca agcaattctc 34440
ctgcctcagc ctcccaagta gctgggatta caggtacatg ccaccaggcc tggataattt 34500
ttgtattttt agtagagaca aggtttcacc atgttggcca ggttggtctc aagctcctga 34560
cctcatgatc cacctgcctt ggcctcccga agtgctggaa ttacaggcat gagccactgc 34620
gcccggcctt tcttttcttc ttaagacaac taatggaaaa taaacttcca agaagctctt 34680
ttgacctgac ctcactgctc ctcaaaggca gaatgctgat tcacaggctg aggggtatct 34740
gaccaggttt ctttgctacc cttactctca gcctacctgg aaattgccga gcgcaggggg 34800
cgcagggagt gctgtagttg gcgtgtggac tgagcgtcct gggaactgtt ctgctgtagt 34860
acctacactt tcctgccaat gtcagcatcc ggcttgtcaa cacttctttt ccatttgtga 34920
atactatagt gaaagccttc acctctcctt tctttgaagt attctctgtt cagaacttgg 34980
aactttagaa attttctaca tttaaggaaa catatttcaa ctatgattct ttattactta 35040
taaaaaatta acttagaaga tatatgtaaa catgtgtatg gtttaaggta acaaagtgag 35100
tttaccaata tggcaagttg tgccaaaaga gtgcttaagt gtaaaaaaca aacaaagaaa 35160
ccaaaatcaa aattaaagta gctcaaaaac ataaagttta taaatatttt taaggcattt 35220
ataattttaa aaaagtcctt ttatgtttgt cccatgttct ggaatagcaa ataaaaaatg 35280
tggtttttgc tttattttgt tttattgcct tgatatgcca cgtggcaagc ctcaggctgg 35340
gtgtgtggag cacatgactt gagtgtgtgg acaagttgtg ttgaagatta ttattacagg 35400
cacaatgtta cagcacagtc tatcagggaa aaatacttaa gtttattttt ttgaggctga 35460
gcgtggtggc ttatgcctgt aatcccagct ccttgggagg ctgaggcaga aaattggttg 35520
aacccaggag gtgaaggctg cagtgagcca agactgtacc actgcactcc agcctgggtg 35580
acagggcgag actccatctc agaaaaagaa aaaaaggaaa ggaagaaaga aagagagaga 35640
aaaaaaagag aaagaaagga aggaaggagg gaaggaagga aggaaggaag gaaggaagga 35700
aggaaggaag gaaggaagga aggaaagaaa gaaagaagaa aaagaaaaag aaggaaggaa 35760
ggaaaagaag aggaagaaga agaaggaggg aaggaaggaa ggaggatcgg agggagaaaa 35820
catggtttac agaggtttta ttcactagga tggtaacatc ctcccaagta atttagttct 35880
agctgcttta tcgtttagta aatctctgca attcaggatg cacctttctt gctgttgctg 35940
gtctgctgat ccacatgatc ttatttcatg tatgtggatc atccagccac cgtgacaaat 36000
gttcattatg ttgagtccag atgtccacct taagagtaca cacccctcgc tgctgctttt 36060
tttttttaat tatgtcacac taaatacatt tactttatga taactactgt tggggtacaa 36120
tttactctta ccacagaacc agctctcagt taagaagaaa gtgctgagat ggtgtgggga 36180
aatttggtga agaaaaacat tatagtaagt tacagtcaat ctcactactg acctttggct 36240
aaaatttttt ttaatatgtg gctaaaatct gcataacaga tggtatatct gagtgaagag 36300
attattagag gatgagagaa actgagagtt caccaaaaat aaaaactaat aattgcaata 36360
tagattttgc catcaaaagt gacctcaaat gcaagtttgt agctgaattg aagaggtggc 36420
cactttgttc ttaatctggt ggctgctgct taggtttcat tgtagaggct actgagctgt 36480
tttgcaagtt tgtattatct gtctcatgat ccagacagaa ccagagtcta ccaacgtgga 36540
ttaagaatta gtctgttttc ttctgtgttt ttctatgaat atactgaatg tctcatcttg 36600
attcagaaga aaaacctgaa agcttaccca aaaacaaaca aacaaacaaa caaacaaaca 36660
aaaaaaaccc tgtgtgtggt ttgtcttggg acagttcaga aggcagtcat ttaatttcgt 36720
ttgggaacat tttgcactat ttatttcatt caaaatatgt taatttattt tcgattttaa 36780
ttaatgtact tttgtaatgg cttattccgc aagcagagat agcctccctt cccccaaatt 36840
ctaagatatg catgtataca gtcatcttac tacatagcag gaattttaaa aagaaataag 36900
cttttctttc cagtgcctag ttgttaaaac agcaaaatga taaaaattta tgtaatggta 36960
ctattgagct gtctactgtg tggcaaatac tttatacaaa taagcaatgt cttaggatct 37020
aggcttttag ccttcaaaaa tctttaaaga agaatgaaat gtcattttta agtcttgtat 37080
ttgatctcaa gtgaagagat tcaattcatt tcaaaaatac ttaatataaa ttatatgcta 37140
gaccctgtgc taggcactgc agggcaaaag ctgaagaatc agagaggctg tcaagttaga 37200
aaagtgtttc tcaattggca atttttccaa caagggccac tggtgaatgt ctacaagtat 37260
ttttggttgg gggttgcagg gactgttgga atttagtggg tagaggtcag gggtgtggct 37320

acacatcttg caatgcatag gacagacctt cccattaaag catcatccac ctgcagatgt 37380
cactagtacc aaacttacat tttgtgtagc ttcagcaagt tctctcttaa actattcatc 37440
gctctctttt ttcattagtt ctgcagttta ataagaatat tgccctatgg agcagttcta 37500
gcactttggg aggccgaggt gagtggatca cctgaggtca ggagttcgag gccagcctgg 37560
ccaacatgac gaaaccctgt ctctactaaa aatacaaaac ttagccaggc gtggtggcat 37620
gtgcctgtag tcccagctac ttggggaggc tgaggcagga gaatcacttg aatctggagg 37680
cagaggttgc agtgcgttga gatcaagcca ctgcactcca gcctgggtga cagagcaaga 37740
caccgtctcn naaaaaaaaa aaatagctgg agattaggca agttgtatat atgtaaacat 37800
aaaaagacag aaacaaataa aaaccaatat agcacaaccc accccatctt gaacaagttg 37860
tacttctaat catgcacatg tgaaaacatc aagctcttat ttgaccacca ttcaggattg 37920
aatatttagt cattgcaaat gtagttgagc aaataagtat ctatttggag tttaggatta 37980
ggatcataca cttccacagt taaatctact gactgacagt agacagttca ttgagacaca 38040
gatacagcat agtcatccat gtatagtggt tgagtaacgt gtaagtttct tttcccttcc 38100
aactgaaggg gatgctcaaa ctgagattta accaggacaa agtcaatcag cttctctctg 38160
aaaagggtga tgtctgatca gagtcagctg gaggatcaga aagagtcatt tagaaaagtc 38220
tgagggaaac tgatgatctt gagtaacaag aaagttgcca ttttgttcac cacagatgaa 38280
agtctattcc cagacagtaa atgaagccgg ataatcaggc caatttcata aacagtagat 38340
cagattggaa gagttaaagg accagataag aaaacaagca cctgtagcag gatgacccga 38400
gtaacacaaa ggatgattca cgcaaagggg gtgcaaggac taattctgca gactcctata 38460
gcttgttgtg ccttgcaagc ccaagagagg ttcccaaagg gactgaagcc tggcagcatc 38520
cagtgaatgg gctctagcat acctggtaag gagctgtctc agaagatggg gtgttccata 38580
tccctggagg ggttaaagtt caagaacatt gctgaggatc caaacactgg gagagagaaa 38640
gaaatggatg gtcataaaat aaatcctctt tgttctttta aaaattctga gtgtgggagt 38700
gaaggtgatt tttcagacca agcccttggg tactgactca ttcttattcc actgtctcgg 38760
gcttacatct ccaattctaa cttcccaact tctgtcatgg cagatcaggt attaccattg 38820
ttagaatagt actataggta ttttgtgttt ggagagaggt ttagtggtag gaccccaggt 38880
ttgtagtgtg ttgctagtta tcatcacaat tttatgaccc tctcacttga gagtggaatg 38940
acaatgtggt tatgtaacct aagactagaa gaaacaaatg ggagtactcc caaaacacaa 39000
atatctcacc tcttttttca tgggaaaaga gtcagcagaa gagggcaaag cggtaaataa 39060
aatcctaaag gtaatcaaaa cagatgggaa tataatttg ttcagagcta taaaaaatat 39120
cctctactat tctcaataca ggcagtgtgt agaaagtttc aaggttagta tttatcttct 39180
aaacatagac ttgtggtctt cttatttaat caattcaaac caccagtgga agaggtcaga 39240
aagaatgaat gaaatcagga gaactgatta tcatgagtat cattgttgtg attgcatttc 39300
ttgcacagct ttaatttgtg ccttgagagt ttggggtctg agaaagtagt gctattccaa 39360
gagctctcca gggatatttt tggaacaggc cttattgttc tctaaaaaat taatgcgtta 39420
tattcccagg tgctgttgta gcatcatttt tcatttaaca aaaatatggt cgtttagcca 39480
gaaattttag ttctataatt aaaatggact aaaaatattc tgtagcacat ctagaaaaaa 39540
tattttaaat gtatcaatgt tcttcctaga aattttccat gttggatcaa acagtagatg 39600
aagacttttc tgtgtgtata gcatgggttt ttatgtttat atatgagtgt gcaggtgttt 39660
taagagaatt ttgaaaatca gcaaagcaaa gtcaattcac tacaaaatag tttccaaatt 39720
atctgtgcag tattaatatt gaggaagaat taatcaaaaa gcaaatattt gagaaatgct 39780
ttcctatgtc tgtaggctac aaaatcattg tttaatttat caacaatctt tgagtgctcc 39840
tcagtgtctg gtacagaaat gaaagataca gttttaactt tagagtgctc acacctactg 39900
agggaaacaa acaaggtaga gaattcagta tgacgagtga catgagacag ttgaatggga 39960
ggctctctgg gagtactaag ggccattgcc tagtgtagct gagggcacag aggagctgag 40020
ctgggcagcc caggagtctg tcaagagggc cagagggaag cagtgcaggg ccagcagcga 40080
gagcgggact gtgcaaaaga agtttcagta gctacagttt ctttctcagg gtcctccact 40140
tcccaaagaa aatgaaagga acatacattt gatttttgtc agtttaaata ttggtcgacc 40200
attggaaaac catttttgaa aagtgatttc ttttgctatg tttggcaaag ccaggagtag 40260
taaagagatg aggaaggacc atcttcgtcc attgcctttg cctttgtaac tgttacttct 40320
tcacagcctt tctacccagt gtagttcacg tatgggaaac atttctttcc atcgtcagcg 40380
tcttcctggg gagtgttttg gttatacttt cacgaatgtc tcacctcttt gtgtcaacaa 40440
acccttctct ttgagctgta ctgtgggttt ctgtttcttt cctttttntc ctttgtccaa 40500
ttagaaaaaa aaatggagta gaagtttcaa ggctactagt tcaatgtgct ctttttttact 40560
tgactccaaa tgacatcttg cttgcagatg tgattaagaa agatctggac agggagaggg 40620
aggataattt tcaatgttcc tctcaaattt aatcttatag tgacaagctc catattctac 40680
atgaaaatgc tcagttaata cagctattaa acttgaatgg tagataatag tggatttctg 40740
tagccactgt ttggtttcat tcaaaaacct agaggaaaat aagggatata cattataaat 40800
tgtttgttta atgttaaaaa tatgtttcat attcagtgca cataaagcaa aggtattgtc 40860
cagtgttagt acctatgaat catcaataaa aacagttgct tctgtttgct gttgacattt 40920
tatgtatgtg ttgttcctct ctgaaccttt ttcttaaaag tcaactttta agaaacttat 40980
aagtttactg tggagagcct atgaacagat gcatgagggg gcgcctgttc atatggataa 41040
gatagggcta taaatgccct tatcttgcca gggctcttct aggcctcttt agggttaagg 41100

```
catactccct tctgagaatt tgtgttgtaa ccggttgtct agcttcacgt cctgtttcta 41160
tggattgttt gtaaccagct tttgctgcaa ctgttactgc tgattaatac cttgctaatc 41220
ataggttatg gaaagactgt tttctgtttt aaggctctgt tagaaattgc tgatgcacac 41280
aatattgtaa attcttatct ctgtatactg tacttctgca tacagatatt atgttaaaga 41340
attacttcat ccccatgtga ccatctcact tcataatcaa atgactctaa atccctcact 41400
aaactacccc caccctcact aaacttaaca ataaatgctg gtatatccag tacgttggcg 41460
gcatcacagg accagaaggc agtgatcccc ctggacccag ctttcactaa aaaaaaaaaa 41520
aaaaaaaaaa agaaacaata aacttagcca ttataatccc tcagaaaaac attaactata 41580
tttaaggata ataagagaaa ataatctctt tttaacatac ttattgatac ataataattg 41640
tacatattta tggggtacct gtgatatttt gttttatgga taaaatgtgt aattatcaaa 41700
tcaagatatt taggatatcc ataacctcaa gcatttatca tttctatgtg ttgggaacat 41760
ttcaaggcct ctctgctagc tattttgaaa tacacaatac attgttgtta actataatca 41820
ccctcgtctg ccatggaaca ttacaactta ttccttctat ctaactctat gtttgtaccc 41880
attaataatt tctttttaaa gccccagtct caatcaagag aaatataaat agcatggtaa 41940
aaaaaaattt tttttgacct gatgatatgg gttcaatttc tggttctacc actttccacc 42000
ttggagacct tcattaacca gtgaccttca cttttctttt ctgtaagatg gtattgataa 42060
tatctgccct catccccact tcgccatttg cctagctaat aagacagaga tttccttagc 42120
atgttgtgtt aaagaggcta gaaactcaac atgctttaca aataccagct gtatgacaag 42180
gagttacagt accaatgtac aaaatatgat ttttaaaatg aaaggcagga catttatgaa 42240
gttatattct atagaaagta tattagcaca cattaatgca taataggcga taggactgca 42300
ctttgcttct ttctcttagc ttttctttcc agcctctttt tctcccttgt catcaagtct 42360
gcggctggac ttgccttctg ttctgcatgc cttggtgttt gctgttgctc tgattctgct 42420
ttctcatgca caggctcaaa gaaacagtga ggctgcccca acctgtgcaa ctctcctctt 42480
tgtttggata cttggtcata tgcacattct agtttaaatt tctgtgtaac taaaacatgt 42540
atgagtggag attggtttta tattctgtaa taataaaact tatttataga agtagaattt 42600
attgaggtac aaatgtttac cagttttatg aataacaact tatatttatt ttataaacaa 42660
cttcaatggt atatgtgtgt gtgttttctc acaaaagcat attgagcaat tattttcaat 42720
tttatagtca taaatgatac ctgaaatatc ttaagaacct accctgtgat ttacaggaat 42780
taagtcatta aatccttaca acacagtaaa gtctgtgtta tttcccattt tacagatgga 42840
gaaactaact acagaaagac tattaaagag gcaagatatc atcacagttg gatgtgtggg 42900
gctttgtacc agaatgactg agttaattcc agttccctta attattatct ttgcatcctc 42960
agacaagtca ctttatcttt atatgcttta gtttccctgt ctgtaaaaca tcttatagtg 43020
ttgttaggat taaacacatc ttttccctac atggcatttt aggttctctt aaccaatgtt 43080
aaaaaatcta tccacatatt tttagctttc ctttctttcc attactagaa gtactttcta 43140
tgaggtaatt ctcccagaaa atacaaagtc ctatttggtg attcactgcc ttacattctg 43200
gtgttcggta acaccatgga atccaaagac aagctccatc atatcaattg tggagcctga 43260
gatactttac tctccctgtg agaaagtgat ttcaaatgaa actcagttct cccatttatt 43320
tcattttggt taaaactata gcaggtgctt ttcagcttat tgaatgtgtt aggctccaag 43380
tggcaagctt aaatggtaca tatccttttt ttaattatac atatatatac ttggtttgac 43440
tgtttatttc cttttccaca ttattatttg catttaacag ctaaaggtat aatatcaaat 43500
tattttattg ttagatgttg aaattaccct ggcttgaagt cagaaacaac tttattaaaa 43560
ttttaagaga acagtaaaat gtgttgcatg tgattctaat caatttttga agcacttttg 43620
tcattgagaa aataagtatg gataaatatt gattgttttt ctggaaaatg ctttcttatt 43680
tcaaaagtca gctgtgactc agtctgtcct cctctgtcat gacaggaact agtatattca 43740
ccgtctatga ggccgcctca caggaaggct gggtgttcct catgtacaga gcaattgaca 43800
gctttccccg ttggcgttcc tacttctatt tcatcactct cattttcttc ctcgcctggc 43860
ttgtgaaggt actgcgaaga attgtactaa gaaagtctat gttctgtgat agttctgcag 43920
atcttttgtt tggttttggt tgccctctgc tttgtcattc ttagactttg gtaaatgtgg 43980
tttgtgattt ttttcagaac gtgtttattg ctgttatcat tgaaacattt gcagaaatca 44040
gagtacagtt tcaacaaatg tggggatcga gaagcagcac tacctcaaca gccaccaccc 44100
aggtacgcta tcagcaagag gatgttataa cccattcgag catgcacatg tctcaccctc 44160
accatcagtg gaccttgggg aagctcttgc atgatgggct tctatagtct gactcaggaa 44220
gctgtttaaa aatacagttg ccagtcaggc acagtggctc atgcttgtaa ttccagcact 44280
ttgggaggct gagggagagg gattgcttga gcccaggagt ttgagatcag cctaggtaat 44340
gtagcaagac cctgtctcta caaaaaaatt ttaaaaatta gccaagagta gtggcatgag 44400
cttgtagtcc cagctactca gtaggctgag atgggaggat cacttgagct caggaggtca 44460
aaggtacagt gagctgcaat cacaacactg caccccagcc tgggtgacag agcaagatcc 44520
tatccccaaa aaacataaaa gaaaataaaa taaaacaggt tcaaaggccc aaacacagaa 44580
caatcaaatc agagcttcca ggcagtgagg taatgacacc tatattttgt aacagctccg 44640
tgggtggttt tgatacaaaa ccaagatcgt caaccacatt tattacatac ctttgattct 44700
gacatcacaa atcctcaaat ggcttgtgga taaaatgcaa ccactatgga atttcttact 44760
tttatgggta tttattcatg tacttgcaag cctatgtaat gtctatgtgt aggattagaa 44820
ttttgtaatg gaaaatatat ttcattcaga acaaaccaca gtacaataaa ctatgaaaaa 44880
```

gatccctcaa ccccaaatgc atgtttgtga tttttaaag aatccctata tattcagaag 44940
gattcccata agttccatag caatattgtt tccataaaac cataaggctc ctgagaattt 45000
gaaggtgggc tacagatttg ctaaaagcag cagggcagtt attacaaaag agggcaattc 45060
agatccaatg cctttgtttt tatttttggt tttgtttcac gtgtgtgttt ttgtggtgtt 45120
aaaaaaagga agggcagatt tgtgtatctt cttctagcgt gaatctttat aaaagagaga 45180
gaaaatataa attccagaat caggaataaa atgatataat gccttgtaat tattataata 45240
ttaaattcta aaggatattt aacaactttg taaacactta caaatactgt taagacaaag 45300
gtatctatca aggagccgag gtgtacaaat tttacttttg aaaaccctca agttgagctt 45360
ttaataatta tttgttaaaa tgcaattaaa cacactaatt tgggaaaagc ctggtggtgt 45420
tgaggcatct ttatgatgct gtaaaagtct gaattgtcca gtcaattcat tatagatgaa 45480
atagtgtcag tcttattcag tttttacaaa caactgttca aacttccatg aaaggttttt 45540
tgaatatgct atccaggtta tatcagaaat gccatgtaga aaagccttca agaaatcaac 45600
catagcaagt tctcatctcc tctctcctct ctttttgcaa actatgagag ataagaaagt 45660
tcccggtcca ttccgaggtg gcagcctcta gaaggacatg ttttcagtta tgactttgag 45720
acctaaatgg aatatggaac tatgatctca gcccttaact cacatgcagt gttcctggag 45780
atgacctaag attcctttaa aaccatagta tgtttatgga gtaactctca cgtataaaat 45840
cacagctgga aaaaatagtg tttggaagca ccaccaagtc aggcaatggt tctaatagaa 45900
agggaaatgt gaagagactt ggaacaaagt tgtacagaat cattttgatg caaaattgta 45960
cttaatcaag aggaataaat tggtaattca gaggtaatgc gtctagatat tcagcagtgg 46020
taatcataca gagaattgtg aaaaaccttc attaagaaaa atgctatagc tgaggacctg 46080
gttgctaagc aaccaaagct cctgctgtca gcctctgggt gaaacaccac tcaggcagcc 46140
tagcaacgag cttcaggccc cgcccaccct ggctagccac ttgcgagtgg gccctctgat 46200
gcttttatgt gtagagaagg gagagacata atttgccatg atgctattat gtttgtaaaa 46260
tatgaccaaa ttccgtgaac aggacttctc cattcagtgg tttaaaacac tagatcacaa 46320
gttactaatt tgaaggaaat aaaataaaga tgattattat aatgcacaca gcttttcaaa 46380
catggaagta cattcttgcc tttctctagc atatggaaaa tttgcagttg ttaaatatag 46440
gctattctca tcaaaggact aaagggtcct gggcttgttg gacttgttga tgtctaagtc 46500
tgcagacttc ccataacagc catgtgtata gaaaatatgt gnnnnnnnnn nnnnnnntat 46560
atatatatat atatatatat atgcacattt tcagaacaaa gaagggcttt tctgttatat 46620
taggcagctc actaggaact ggctctgaca gggaccattt caagggaggc gaaaggaaat 46680
ctgtgatgag aactgtttct ttaaaataac gaagcaatat ttttcaattg aatggtcacc 46740
accaaatgcc tccaagacac agcataggaa caaattacag agacattaat gccaagggtc 46800
tttaggagac cataatataa catgtaactg gaaaacagaa ggaaaatgat gtagaaaatt 46860
tcaaattact aaatattgac tctggcctct gagagccttg tgatttcata cctgcctgat 46920
tcttagacgt gggtaactat tgccttgtgc tcagttgtgg attgggttga aggataagtg 46980
attcatacac ccatgatata ataccctgct ttgttaatcc actatagggt tacccaggcc 47040
acaacttttt agtgatgtcc catgactgga tgttgggatg tgcaggtgag ctgaaagacg 47100
aagtcctgag taccgtatct cactaaccca gacctttact cccattattt agatgcttta 47160
cttcagatac ctgttatgag ccccatggtc ctctgatcta gactagctca tgatggagat 47220
gagcttatcc caactttgcc ttgagtagaa ctcaaagaac tgctaaattt tagagccaca 47280
caaggctact tttctgttaa aatggtagga gacagaaagc aggcttctga cagtcaatac 47340
atatctaact tttataaatg aatcataatc caataggatc atattgaagg tttggtaaat 47400
aattaaatat tctgattgca caaagacagc aatcatttct gggtctgtta ttatctctga 47460
ttgagcactc ttgctgacat gtgttttctg aattaatatc ccctaattaa cagagaagac 47520
tgataaaggt agaactggtg tgggaagaat gggcactttt gagtttttct cctttgggtt 47580
tccatcaaca ttcttagaat atgggttata caatgcctaa agcaacttgt gcataaatgt 47640
tcagtgctgg gttaaaaagt agagaaaaaa tggatgaaaa gacacatgat ccaaaatgga 47700
aagaatttct cctccaaaaa gaaagctaca tataacttaa tgctcaaaga aaatacttta 47760
gcaaaaatgt gtttactgtg ttccaagacc atttatttcc tcatgaatat acatcattaa 47820
aatatgtgag aaaacaattc aattaatgca tttcagggaa gggtgcttct agcatggtgt 47880
aataatgagt gccttctaat aagaaaccat aataatagaa tagtacaatc atcataggaa 47940
accataaatg agaattaaat tttgtgaggt tgcatcattg aatatattat ttcgtaatga 48000
aatcaagcca ggtatccact ttcactattt tctgtcatag tgactattag ctattttttg 48060
aaaaaccata aattttcaaa gaatccaaag agcatgcaaa ttgcaaagta aattttagat 48120
tttctctgca ttactgtgta tgtggatgtg tgtgtgcctg tcttgagttt ggaattctaa 48180
ttcagctgct agtgattctc cattaccaag attctttctc ttaccactat ccattagata 48240
acctgttatt catgttgttg atgatgatga tattatgcct ataatggaac aatcccagaa 48300
gcatttttag ctactttaca aaccaaccga ccagttataa aactgtagca gttgcaaatg 48360
gacaggcctg agagaattct caaagttgaa atctccatgc gcttccaaat gattgtgcta 48420
agaggagagt gccatggaaa ttcatttgtg ttattggtga gattctacta attcagaaca 48480
tcttcttaca gtcactccca tatattgaga tctgcactag tctaatggct ttacaaactg 48540
tcttatttaa ttttcataat aatactgtga agtgggcact attaatgtct ccactttgca 48600
tgtaaaaaat gacaatgatt ggaaagttaa ttatctaacc caatgttata taactggcta 48660

gtggtaaaac tgggactcaa aaccaggttg ctcagctgca agagcctttt tattcagcat 48720
gagatgctac cacctttcaa tttagccatt acaagccctg ctgtcaacat gctgtctttg 48780
ttctggcctc atgttaactt ttcttttctt aaaaaagaca gtatttattg ctggctgaaa 48840
gggacattcg catacattgt tgccaagagt atgaattaaa acatcttttc cagaaaaccg 48900
tttggcaata tgtatccaaa agtaaaacat acatactatc tgaccaagaa agtccgcttt 48960
tagaaatgta tcctaagaaa aattgggggc agtagccaag ggcatatgtt tgaggatgtt 49020
tatgaggcat tatggcaatt ttttaaaaat ggaaacaatc ttagtctcta gcaataagtt 49080
atcgattata aattatgaaa ttggaataaa tgcaaaccat taaaataaga attgaacttt 49140
attgatgtga agatatgctg atgacatatt agtacaaaaa aagatcctat gtttgtctgt 49200
gggatgcagg tgtgtgtgtg tgtgtgtgtg tgtgtgtgtg tgtataatac ctggaatgtt 49260
aactgtaaaa tgttagtggt ggtgtctctg gggaatgggt ttaagctgnt tcttttgcaa 49320
ctcatncctc attttctaag cctttaatag taaaatnatt taattataga atgagaaagg 49380
aaaattacat cagtnnnntn nnaaaaanna aattaatgga cattctaaca gaaaccacgt 49440
atactagtta agttacattc agttgcaagg gacaaaaaag cccaaaataa gttgcttagg 49500
tgacgtatgc gatgaggaat cccattctct ctcatataaa gttggaggtg ctcattcagc 49560
atggtatggt tgtctgtgtt gccacggatc cagacacctt ccttcctgtt ccactttgca 49620
cagccccccat taccacagtc accttgtaga ctagcatggc tgctgtagct ctaaccctca 49680
catccatatc ccagagagca ggaagaagaa ggaaatacag gaaaagaggg tttctgcttg 49740
catcctatag tcctgtatgt agttacatgg tcaagctcag cttcaaaaaa agggcaagaa 49800
ctggcctttt ttcttggtag cctagtggcc ccatacagat tctctttgta aaacagggaa 49860
aagagataca aaggaaagac tggcagtccc tgccacgctc cccgcaagga tttgagtctc 49920
tgaggattct tggcttctgc tagctagccc agcttctgtc tcaaagatct actgtaatat 49980
taaccaatct aaatgttgta taattcaggg gattaaagag aaattgaacg tttttgcatt 50040
tcctttactt atttcataca aataagtgac cttgggagaa cacttttact gaaaattaaa 50100
ttgattcttt tgattttggt ctattgaaaa aaaaatttaa gagagaaaat tagtctgcta 50160
ctgatcaaag tcaatatatg ccccagggat cctttatgcc acaattagag agtataggat 50220
gtatcaacag agggttttta ttaataactg gtgactggca atgctggttt tgtcttttat 50280
ccttaaaatg aggcttccag aattttgaca gaaactttac aaggtcctat aacaggtttg 50340
catctccact gatgtctata ttctcagaat gagctattca ggaagaatat gcttgattca 50400
ccccaaaaag agtttttgca gtttattcta aagaaaatca gaatcctgta aatggacagc 50460
tgagatgagc aatttgcagt tggcatcttt gtcaaaggag ctttatgtaa atgtgcacct 50520
taattttgtc atttaaaaat acatatagga ggcagaagct gggggattgc ctgagctcag 50580
gagttcaaaa ccaccctgga caacaagctg aaaccctctg tctagtaaaa aaaaaaatac 50640
aaaaattagt tgggcattgt ggcgggtgcc tgtagtccca gctactcggg aggctgagac 50700
aggaaaattg cttgaacccg ggaggtggag gttggagtga gtggagatca cgcccctaca 50760
ctccagcctg ggcgacagag caggactcta tctcccccca aaaataaata aataataaaa 50820
atacatatag ggactgtgat ttttggcaac tgtctctcac cacctcactc atttataga 50880
aggcaagttt attttccaca aaggatctac caccactact accagggatt tgaaactcta 50940
attacaaatc ctaggtaatg ggatctttgc atcatctcat tcgtatttcc atcagcagtt 51000
acccccttcga atacagggtc agactaacag gaggacataa ataagggtga caaaggaaac 51060
atttatttct gtaaaacagc gacctcaggy aggaatgaaa cagtgagcac ttgtagttag 51120
tatcattatt tagaaaaaca aaaatgttta attttgccct attcctacca tacacacata 51180
cacacacact cgctcctaag aactagaaag aaaaatatgt tagtgggaaa atacgtgaaa 51240
gtgggaaatg tcttccagct gggtgtaata ttgacacata ggtaatatgt gatgatacct 51300
aggagataat aggtacctag gagataataa cctaggtatc taattcaatg gatttcagat 51360
ttagtgggcc tgtagcatct tgaatttcat cacttgggtc tttaatccaa aagaaactac 51420
ttgttcccctt cataatacgt acataaaatt tctgctgagc aggttaattg ctgcctagtt 51480
aaaataatta cacaaagtgt attaagcaat gtggcaatct tctaccatta aaataacacc 51540
agttctttcc ctatgccaca cgcacacttc ttattttatg gatcctgaga atatttttat 51600
taggtcagtg ctcctgttca gccttgaggg taagatacca tggtagtcca agccagggag 51660
tggaagactg tccactaaat aaaaataaaa atttgcaacc tatagtaaaa acacaccaga 51720
cacaatgctt ttaaattctg attttcattt tctcccagat ctaatagata tttatcaata 51780
taatggggat aaaatactaa agtatttctc ttgtggagaa gaatatttga gcaattatat 51840
gcaaagcata tgcaagacag aaagtgcctg acatagcaag tagtcaataa actcaagtta 51900
ttatcattgt tcttttttca ttcatttaag ctttcaatgt taaagaacta taattgaaat 51960
gacttcattc caaatataaa tccataagaa gacaaatttt tgataacata aaaatataca 52020
aagctggaga catagagttc tcaatgttcc atattaaatg tctctgactc atctcccttc 52080
tttgtcttga agatgtttca tgaagatgct gctggaggtt ggcagctggt agctgtggat 52140
gtcaacaagc cccagggacg cgccccagcc tgcctccagg tgcagtacaa tgacattttt 52200
aaaaatcgcc cagcaaaggt ctttgaattt tatttcatcc aagaaaatcc acagctcttt 52260
aagctctaga tttgtccaaa tttaaaatcc tgaagttaga gatggtattt cactccttcc 52320
tctattccca ggacctagct tttttttttt taacatacac aatagggatt tgataagttt 52380
ctgatggctg caggcatgta agagcatttc agtggtattg aatcaatgaa gaattttgtt 52440

gacatgtgaa atcttataaa aatattcttt accgaaggac tgagttatgt ggcagtgggc 52500
aaattcattg tttcatacct ccccgagtaa ctgggaaaaa tatgttaata catagtctct 52560
ctgtttttct gcatttggaa gctttcagag gaacataatg tagaggtgtt tctttagcaa 52620
agtgcactga tagcaaacat aaggattgca ggtggggcct gagagtcctc atgagataga 52680
ttctcacagt gattagaaga tggagtctca cgtccctgcc tgtgaacttt ctggaaaaac 52740
catcttctcc aagctgccat tgacaacaat atggataaca ataataacaa taaggcccaa 52800
taaactcctt tatctcttct tcaggggggcc atactgacat cttctcttcc ttggtttccc 52860
ctccttgccc cctaaatatc agtaactcat tcaaaataat gtcaccttac caagagcagc 52920
acccctaact ttccataata ttttcacttt cattttccct ccaagcagcc cactcgtagg 52980
accggagaat tgattcttcc acctggagaa ttttattttc tttagccttt ttggttttca 53040
gtgacaaatc ctcttctcgc aaggggtggt ttccatagtt gtttatatcc tgccctcata 53100
atttggagaa gtgttcacat ctgccgtggg atgagactgt atctcttttc tttcttttgg 53160
gtctttctcc agatagggac ttcttatgca actcaaggat gggtacatga aaaataaaat 53220
tgtactctga gccattactg tgggctatgt ttatatggcc attttaccat agagttattt 53280
acttcttttt gtttctattt gtattgaggt gtgattaaca aataaaattg taaatactta 53340
aggtatacaa tgtgatgatt tgatatatgt acacagtgta aaacgattgt tgcaaccaag 53400
ctaattaaca cacccatcac cgcatatatt cagtagttac ctctgtgtgt gtggggtggg 53460
gggtaacact taagatctaa tctcctagca aatctcatgt atacaataca gtattattaa 53520
ccattaatga atcactttgt ttattagatc ctgagaactt ggccatcttt tgactgataa 53580
ctaatatctc cccatttcta ccccctgtcc ccagcccctg gtaaccacaa tcctactctc 53640
tgcttcaatg aatttgactt tttgagattc catatataaa tgagattatg cggtgtttgt 53700
ctttctgtgt ctagttcatt tcacttagca taacgtcctc caggcttatc catatagttg 53760
tcaatggcag gatttttttc tttattatgg ctgagtaata tttcattgta tgtctatacc 53820
acattttttt tatccattca tccatcagta gacacttagg ttgttccctg tcttgaccat 53880
tgtgaataac gctgcagtaa acacggggct gcagatgtct ctttgagata ctgatttcat 53940
ttcctttggg catatatcca gaagtgggat tgtgggatca tatggtagtt ctatttttaa 54000
ttttttggga aacctccata ctgcgtttca gaatggctct accaatttaa atccccacca 54060
acagtgtaaa agggttatct tttctttatg cctttgccaa atagttatct tttgtctttt 54120
tgatggtgac cattgtaaca gatatgaggt gatatctcaa tgtggtttaa atttgtattt 54180
ccctgataat tagggatcgt atgcactttt tcatatacct attggccatt catatgtctt 54240
ctttggaaaa aaatctactc agaatttgct catttataat cagattattt gcattttgtg 54300
tttgtttttg tttttagttt tgggggcttt ttgttattga gttgtatgag ttctttgcat 54360
cttttgaata ttatctcctt ttcagaaata tggtgttcca atttcccct gttccataga 54420
ctgccttttc attttgttta ttgttagtta tttatttctg tcagtttaaa aaaatggagg 54480
gcaataagta gaaaagctta caatgaatat tgataagtat tattttgtgg tcctccttcc 54540
atcctatatt tgatgttgac tatgctgcta atataaaact actacaacta cctttactcc 54600
tactatcact actaatagta ataacaataa tgacaaagat tatagtacta gcttcttacc 54660
taaaaagcac ttttattgac attatttttt aatccatcaa ctgatattta ctgagtacca 54720
actatgtgct agacactaag atagtcttga aagaaacatg cacagtctcc acactcatag 54780
agcttacagt atgcttgaga gtgggaggga aaaaggcagc attgagctag caatcacaag 54840
agtgatgtgg gttttgaaag aagtaatgca agatgcaatg agaatatata aattggggtc 54900
cgctttgaga ggtcagggaa ggttttagtg cataatctta aaatcaaaac cttgagtaat 54960
tccagcattt tgggaggctg aggcaggcag atcacctgag gtcatgagtt tgagaccagc 55020
ctggccaaca tggtgaaacc ccatctctac taaaaatcca aatattaccc aggtgttgtg 55080
gtgcacgcct gtaatcccag ctacttggga ggctaaggca caagaatcac ttgaacccgg 55140
gaggtggagg ttgcagtgag ctgagattgt gccactgcaa tgtagcctgg gcaacagagt 55200
gagactctgt ctcaaaaaaa aaaaaaaaga gagagagaga atgaggaaaa gaatgactaa 55260
ggatgaggtt tactacaacc ctgtgatgta gaaagaccta catgcctggt cagccattta 55320
taggagaaag tgctgattca gagaacatag ggatgtgaat tacattcaac cagagggaga 55380
acccaggact aatggctggg tcttatggct cctagcccca tgctttatcc agctgaaaaa 55440
aatatgcaag taatcataag ttcgtgatag agaaataaac tatcagggct actctgagtg 55500
ccaaatcaga gtcgtatggt atccagagct ttaaaatcta ttaaatcctt gttaatagat 55560
gcctcaaggt ggccatctga aattaggagg caagtcagcc tccttatcac aaagatttga 55620
gaataacaag ggattaatga cagggtgaag tgtgatgaca aaatcgagca aggagatagt 55680
aaaataaaga ttggaatggt gtggggatag atgttgggct tgaagtgtat agatgtggca 55740
tctcagagac ccctatatga gatatgtgtt atgaatatag cagcatgctt atgacatctt 55800
atgaatgtga ctcatatgct tagaaactgg ggggaaaggg gtcaggtaat tcagtgtgac 55860
acaagtttca ttccactcaa cttcatgtgt ttggccctga ataaagcttt ccagaggcag 55920
caatgaagag tgcatggaag aaaagtggga tggccaagaa gttatctgtt tgtaccgggc 55980
aggagctgtg attagagaga gaatacattt ctatgtctca gcaagaaaat aattaattaa 56040
ttaaaagaaa agaattaagt aaaaatatat tgaagataaa atggtcctga aatacttgat 56100
ggctttcact aatgattaat gccatctaaa actgggtaac aggaaaactt tcatctcagt 56160
cttccactcc ctggcttgga ctaccatggt atcttaccct caaggctgaa caggagcata 56220

taccacttct ttttatttat ttatttattt atttatttat ttatttattt atttnnnnnn 56280
tttatttatt tattttttga gacggagtct cgctctgtcg cccaggctgg agtgcagtgg 56340
cgggatctcg gctcactgca agctccgcct cccgggttca cgccattctc ctgcctcagc 56400
ctcccaagta gctgggacta caggcgcccg ccactacgcc cggctaattt ttttgtattt 56460
ttagtagaga cggggtttca ccgttttagc cgggatggtc tcgatctcct gacctcgtga 56520
tccgcccgcc tcggcctccc aaagtgctgg gattacaggc gtgagccacc gcgcccggcc 56580
ctttttattt ttaaaacaag caaaatgtga acatgttctc ataaaaaatt aatatggaaa 56640
taaaattaaa aggaagagtt tacttgaaat cttctttcaa tgtatccttc ctcattcttc 56700
ttccaatgag taacttggag gttaatgttg agtcgattct tctcagactc ctgcatttat 56760
gcattttatc tcacatgaaa aagcatttct gcctccagga agcactgaca ttttcacaga 56820
atgtttcctc ttcctacacc acttccttct cactcatcct tcagaactca gctcaaacat 56880
cactttctta tcctcaaaaa ataagccagg tacctctgtt acatgttttc atatcatcct 56940
acacttttct tttatgtctg tcataatttt aataaaataa attaaggata taattagttg 57000
ttgatgtctg cttctccaac caggggaaat tccattactt tagagaccat accagtcatg 57060
gtcacttctg caactgtgct ttagacatga gagatgatca attatgttta ttaaccgaga 57120
acagtccagt gaaagaagga ggcagtacct tcttggctta tgaaagtact tatttgacct 57180
ctcgcttaga agctgctctg tttgtcctcc tggtcttgtc tgtaagaact agaaattgaa 57240
tagactgtgg caataacacc cttttccata cagaacactt atttctatat gcatgtcgtt 57300
ggagtctaaa gcaagcagta cacttggagt ttgcttccag aaagtgcctc gcagaggttg 57360
taggactttg gatatctctg gatagttttg ccaggatgtc tcctctaata tgtggtggca 57420
gagccttggc aggacataac cctgacttct gttatgtttc acctctttca gaactctttt 57480
catcttgtaa gcatataaga taaatttgtc ttatgtttag aaatatagtg ttaggaatag 57540
catcattgaa aagaacccaa actcattctt tttttttttt tttttttttt ttgagacaga 57600
gtctcactct cctgcccagg ctggaatcca gtggcacaat cttggctcat tgcaacctcc 57660
gcctcccagg ttcaagtgat tctcctgcct cagcctccag agtagctggg actacaggca 57720
tgcgccacca cgcctggctg atttttgtat ttttagtaga aaccgggttt caccatgttg 57780
gtcaggctgg tcttgaactc ctgacctcat gatccgccca cctcggcctc ccaaagtgct 57840
gggattacag gtgtgagcca ctgtgcccag ctgaacccaa actaattgta tgttaatgag 57900
aaaacagagt ttattggaat gaaacatttg aacggctgta ctttgtaatt ttgaaactgt 57960
tgaaataaat tagaaaaaat gaggtatcaa taatatctga gagacagcaa actgatgttt 58020
gagttcccat ttaactactg ggaactgttg attttgttca aagtgggact ttgtttttaa 58080
atttgaccca aagtttaaaa accagagtat tttacataaa aacccacacg tcctacccat 58140
cttgtaaaag agaatgatct ggcaataaag agctctcatt tccacctggc tagagttggc 58200
cagagctgag tggtggctgc cacatgagca gaggcagaga ggctttattg tgcctcagtt 58260
tcccaactct ccatattgtc tccacagcac tggaggtgag tgttttacct acccaagaat 58320
tcatacattt atttttctaa gagtagagaa agatttctct ttgtccgtga ctctatcgtg 58380
tggaaataaa taaacaagta ataaattcat aaatatataa atgtataaag atacacaaat 58440
gcatggatga atgaataaag aataaataca aggcatttta aaagcaccct gtgattacag 58500
aaaacaatga gaaatgaacc tagtatttta agaaaaaagt tattccgatg tgttaaatat 58560
gtaaccctaa gcctagtttt ctcattatgt tacttgctag gtctcaggag gcacttggtt 58620
gggtgactca cggaataaat gaaaataatg taactttaga tcacaaagat tatataataa 58680
caattagtcc acacatttca gtgattccaa aatagtattt tcatatattt tatgttttta 58740
acattttaaa atgttaaaat cataactcag cataattgga agctggctca agttctctta 58800
taaatgaaat gtactaaaaa atcaaaactg tctccattgg tattatgtat tatacttttc 58860
accatatttt ctactctgta tcctcatcca gttactataa gtaattaggt aatttaataa 58920
aacaagattg attaaggtgg cttaattttc ctcaccatta ttaaattaaa tacatgaagt 58980
aaccaatgct tctgtgatgc tgagtttttt acgagtaata atttattctc tctttctccc 59040
tctctctcta tgtgtgtgta tatatacaca aacatatata tacacacgtt tttatatata 59100
catatataag tgcatatgtg tgtatatgta tatgtgtata tacatatata tagaaacatt 59160
gctggcataa aactattagg attttatata tttcaatggg cagtgattct gatcacacat 59220
tagcaactaa ttttaaattg ttttcctaca ttcccacatg tgcacataca tattttacct 59280
tatatcaaat atttccatta ttatggtatt tgtttcctct tttcaaatat tgcttatgga 59340
gatagtcaat gaattagggc acataaaaga tcttcttact cttctacaga aaaaataggc 59400
tctattgagc catttgtgga ggagaatata ggcatctttt gtctgcagtt tgaaaccaca 59460
aatttatttt gtgttctgaa acacaaatta caattacaat catagctata attataatta 59520
gaataggaac catatgcagg catgcaagga aacgattcaa tctggtgccc ttgaagtgat 59580
ctttctgttt ttataataaa tgaaaatgca catacttgta aatttaactg caagaccaca 59640
agtttggagc aggaggacta gcagaaaggt attgttagct tggtaagtta tatgtggtga 59700
gctctttaac gccacttgtt aaaatttttt gttaatcaac ttaattatat taatttgtct 59760
aggacatttc tcatgtttat ccagttatta atgaaattta taccatgctt aataaggctg 59820
agtttaataa gatactgagc atggtaaatg tgtgcaattc ctaataaatt tatcctcacg 59880
ttaaaacgag taagcgaatg tcctagaacc cctgaaaaac cccctcccat cctctgtcat 59940
gcctgcaatg tctttctggc tctttctcat ctctgtgcat tgctgatgtg cttgtcactt 60000

aggggcccct cgtgcctgtt cctttttttt tttttttttt tttttttttt ttgagaagga 60060
gtctcgctct gtcatcaggc tggagtgcat tggtgcgatc tcggctcact gcaacctcta 60120
cctcccaggt tcaagcgatt ctcctgcctc agcctcctga gtagctgaga ctacaggcgc 60180
ccgccaccat gcccagctaa tttttgtat tttagtagag acggggtttc accacgttgg 60240
ccaggatgat ctcgatcttc tgacctcgtg atctgcctgc cttttcctgt ttttggtcca 60300
tactgtgggg acaagatgac tcgtggtgaa aaagaaaagc ctaggaacat agataagcca 60360
agccccatac ctggtttctt ttcctttttg tgctacaact gcctaaatgc gcatctccca 60420
acatgacctc ctcacctctc ccttcttcct ccccagctac agatctcctt atcttcacct 60480
gatgcataga cacccaacac aacatcttgt tttgagacta cacgctattg tggtctgtta 60540
gagataagac tctggaaaca tatttcagct acactaccta ccagtggtac gcgacaggac 60600
acgtttatgg aaaatgggga tgagaatgca gttttcctgg gtttgagaga attattgagt 60660
atttatgact tagtaaagaa ttatttggaa ggaacagacc atctgctcaa agggaacatt 60720
gattgcaagt gtcattcggc ctttctctgc ctgtctctca ctctccgttt gagccacatg 60780
atttctgtct gtgagcctgc ctctgttttc tcaccttgaa cactggcttt attctgtttt 60840
gttttggttt gtttctctcc ttgcatatta cccaagtatg gctgattgtg actgggtgta 60900
atgacctccc aatcaaaagc cctaacgaga ctcccccagaa aacacccaaa ggtgaaggtg 60960
cactgggaaa aaatgaagtt gcgtgggggc atcagaaaca ggaggcgtgg ccgggagcag 61020
caggtcagga gagtgcaaat ggaagganan gagcaggtnc tgctgaagcc aagtattgga 61080
tttccctgca gagcaaaccc aaagtcgttt gaaaccgccc tttttaatcc tctctgaaag 61140
ccttgcccca tctctgctgg aaccactact caaccaccct ccttagcttc atggaatggg 61200
aggagagagg acacaactct ttggccatac taagtggtga gacttgactg cagtacttac 61260
aactcaggga tcatttttcc tgtcaaactt ctraatctta taggacttga gaagtttatt 61320
aagagctaaa aaaataggct attacagtct cacctgaagc tcaaatcatg ctgtataaga 61380
tagtttcttt agagaaacac cacgtagtta ctataaactt acaaatggac aatttgtaaa 61440
ttggtgctat tttacaacat aatattaagt cgatttcctt gcatttaaaa aatacatatt 61500
ctgaaataat ttagaaacac agataaaaaa tacctatgtg tctgtcagaa ataataatag 61560
ctaatattta tggaccactc actgtgtgtt ttattaggta ttaaattatt taatgctcac 61620
agcaccataa aggtaggtac taatacgatc tgttttacaa aagaggaggt tgaggcacag 61680
acagcataag gaatttgatg aaggtcacat agttactgaa tggggatttc atctcaggca 61740
gtccacgtta tacttttaac tacttcattt tcacccctgt gtattaccct actgtgaaaa 61800
taacccagtt tcttcattca tttattcatt ccactatcga tggacattga gcttgnnttn 61860
nattgttttg ctattgcaga ccttctaaga cctttcttat ccgtgccttt gtatgcacac 61920
gtgagagagg tgttctaaca tatatagcta ggacagcaat ttctgggtta aaggatatga 61980
gcacctttga ctctctgaga tatggcagat ggctctccgg atgatagggc tattttgtat 62040
gccttctaga aacgtgtaag ggtgctcgtt gtctacatct tcatctgcac ttgatgttat 62100
caggctttta caccagatga cacgaaatgg tgtcctgtaa tgtaaatttg catctccctg 62160
atctccctga ttaatggatg ttgaacattt ttaggttcaa gggccacaca ttttcttttt 62220
tttttttttt ttttttttga gatggagtct tgctctgtcg cccaggctgg agtgcggtgg 62280
cgtgatcttg gctcactgaa agctccgcct cctgggttca cgccattctc ctgccccagc 62340
ctcccgagtt gctgggtcta caggtgcctg ccaccacgcc cggctaattt tttgtatttt 62400
tagaagagac ggggtttcac tgtgttagcc aggatggtct cgatctcctg actgtagatt 62460
gcttgtttat ctcatttgcc catttatctg ttgatttttc taagtgatgt tcaggaattc 62520
tgagtgtttc taaagggtat attgtttctt tctaaataaa aatttaagat attagatata 62580
aagacaaatc taaaatatta aatgcttgac acatgtgcct ttttcatctc tcccatgttt 62640
agtcaataag gcaattcttt tatgtggtat taaagaaacc gattagaaag ttgcttcgtt 62700
gtgtgcttaa gtgtgagtct tagaaaggtg aaaagttata attgactctt ttagcaccaa 62760
accaagcagg tatatagctg tttaaatact attttagcat aagccatagc tgtgggaagg 62820
gtcactccag tgcttgataa atagaagtct attcaattgg accctcattc ctcagcggct 62880
gctgtgacaa gccgttttcc cgctgctgct caggtaggag ttttcatctc ttgacacttt 62940
gacagatgtg gctgcacact tttgaaccat cttccaacac acccccgtgc tctattacca 63000
tgaggaaagt ctctgacaag agaatgaaag tacatatgca tgagacgtgg tacaatggat 63060
gtcagtttcc ctctggtagc atattccacc gcgtgcctcc tgtgaggaac agagggaggt 63120
aacaggtgct tgctgaaagt aggtcaaacg gtggcgaaat tattccttca gtgttttaat 63180
gctctttgaa aacatttcca ttgaaggcta agattgaggg gaggtggcct gtgtgatgtg 63240
agcatttcaa gtgacaaagt gacatcctag gcactcctga aactgtcttc ttcgtgaacc 63300
ttcccacaaa ctggcagatc atttcaagaa ttgatggagc ccatgagaat gtaggttccc 63360
tccagaacgc agctctctcc ccagcccatt cccacaaaga aactaggttt tccctgcatt 63420
tccagaaacc tccaaaccaa cccattatgc ttctcctttt gtcctctcca aattcaatct 63480
gatccttctt caattctgtc attacttcag tattttgata tttttgact cattctctcc 63540
actccatctt catattcctg ccctcaaaat caggtcagtg cttcctcaac agagtgatct 63600
ctgattcctt gattatattt ggtcattttc ttttttgttt ctctttcttc tctattgctt 63660
cattcacgat aggcatttgc caaaaatttt ctgaattata cgtgattgta catatccagc 63720
tgcgttcacg tgcagaaaac atagcctcac acacagcaga cccaaattct tactctcccc 63780

ttaggcaggt gtcaaacgat acttcccccca aatgctcacg catgcacgca aacagagaca 63840
cacagtcaaa aacacagcat gcatgtgtac acacacatac acactcatac aggctcacac 63900
atgcatgcag agacacacag tcaaaaacac agcatgcatg tgtacacaca cacactcata 63960
caggctcaca catgctcaca cgcacacaca cctcaaacac acacagtgac acagacacac 64020
acatgcacac tcgtgcacat acacatgcct acacacttat acatgctcat gcacgctcac 64080
acaactcaca tgtacataca cacacagtga cacacaatcg cagacacaca catgcacaca 64140
ggcacacaca catatctaca cgcacatgct catgcacaca cacttcacac gtacatacac 64200
acagtgacac acaatcacac gcacacacat atgcacacat gcatatggct acacacacgt 64260
gcatgctcac acacacctca caatgcacac tcacaaacgg tgacacactg acacacacac 64320
acatgcatgc acacagacat gcatacacac gcttatacac acacacacct cacgcatact 64380
cagtgacaca gtgacacaca gacatttaca tgcttatgca cacatgcaca cacacatgca 64440
caaacacact tgtacataat catgcatgct cacacataca cacgcacata cctcacacac 64500
acacatacac tcacacacag gcgcacacac attcattttc aatttctcag gggaatataa 64560
aacaactgct tggaggaaaa gtttgtagga aaaagtgaat ataaaaatgg ttatgcagta 64620
attacctgga ataaatatta aaaccaggaa aaataaaata gctacatgta gaaaggggta 64680
gctaaaatgc tacttgaaaa aggcctattg taattttgaa gtcagtggcc aaataaacta 64740
aacagagagg ccaattgtat aaatcctcag ttctaaatat cccatatata tttatttaaa 64800
tatatattca atatgtatct aatatatcct gattatattt cnaaataata attatattta 64860
gaatagacac cagatgacca ggcataggat aataaaaaat aagattaaaa tatcagtgtt 64920
atttttattt atttgtttat ctgtcttact ggcaattaaa cagttttgct tctttgattt 64980
ttaaacctca ctggaagaaa tatgcagaac attttaaaat tatttgcatg ccacattatc 65040
tcaaactaaa ttcctgtctc ctattatatt ttcatcctcc agtttgaatc agtcctataa 65100
ctcttgaatg tttacaatta tattaattag gagacatgtg ctaacttatg ccaagcattt 65160
ttaaaatgtc cattggagac aaatttccat caccaaagat ttgttgcatt gtcacctttg 65220
gcaaaaatga aggatgagtt tttctgtttg ttcgtttggt ttggttttgc aagtcttgat 65280
ctccaggtta aatacacagc cttctttaag acttaaaccc agggtttgaa agtgctgtgt 65340
attttctcag tgttcaacac aatctgcagt agaagggttt ggagcagcaa tatgatgcct 65400
tatgcactct aaagctgctg acaggtttag acataatcta aaatgtaagt cactttgtgt 65460
atcaggcagt tcagtccttc gcatttgttt taatgttttt cctaaatact ctaaatattt 65520
caaaatgagt ctttccttaa agattctaca tagctcaaaa ttattttatt tatgtatgca 65580
tttattaaat atcacaagtg aggtcctgca ttgcattaga aatgcagtgt attagaaagg 65640
acagctagct tcaattttga attcataatt tgtctcttga gtttaaaaca aaagttttgt 65700
gttcaactgc ccaccgttta atactaaaaa taaaagatga acataaaagt caatttgaag 65760
tgattctaga tgtaggaatt taattagctg tgggaatgtc aatgacatct caaaaccatc 65820
atggcgtcgt gtcacctgtc tagcagccct attgtagtcc ttcctgactt ctcactaact 65880
tacccatgaa gacaaagata aagaaaaact cacaacaata caggtaacta agaaagcacc 65940
ttttgtcaaa gtctgttgag attattcagt aactatgtgg ttggcggagt actagtgaga 66000
aaaatataaa acatcatgta tattcaaatt gtttaatcta atgcgtccat ccttttagga 66060
actcagtact gaagtctagg aacatcaggc taaaaaaaat aatggttgga tttttctact 66120
ctttctagac accagggaac atgagagaaa attattgtgg aaggactaat attcttattt 66180
ttattattac ctatgtccag ccaagtaagc aaggtcaaca cttcaccgtt gaaaagccaa 66240
gattttagca ttttcattct taattttttac tctagtattt cactgacact tgagtgtatg 66300
tcagaantca gagggtcatg aagaanngga cagccctggt gactgccaca ggctgagact 66360
tactgctgga acatgtatag tgaggcctcc tttgttgacc atcaccctgg gtcttcttgt 66420
gcccaaagca gaatggtgtg gcttcctgga ataggagatc atattacttg aagcaacaag 66480
attgtattgc ttgaactcta gtgtcctaat ttgttttact cactttgtat taatacatgc 66540
caaagatagg gaaggggatg aaagggtctt gggaccacca tctaggttac agcatccctc 66600
ttttaggagt ccttgctaag acacctcagg ggaaactgcc tttctgaagc ctggctcatg 66660
gtccagttaa ctatgtcaac agtaccaaca ttactttcat ttaatacagg agatagagaa 66720
atgtttcacc cccgaggcta ctttcctcag aaggaggcag aagatgctcc atggccaatt 66780
gagtacatgg aggaaattaa gttatatcaa aaattattgt tgaatctact atgaatccag 66840
ccttgcagga actgagtact gaagcttagg aacaccaggc taaaatatat atatatatat 66900
atatatatat atatgagctt tttctactct ttctggacac caaggaacat gaaagaaaag 66960
tgttgcggaa taactaatat tttttatttt tcttacccat gtccagccaa gtaagcaaag 67020
tgaacacttc attgttgaaa agccaagatt ttagcatgtt tattcttaat ttttcttcca 67080
agtcaataat gaagtcatgc aggggtattg agttcttaat ccataaatta taggttagtt 67140
aattgaaagg tttttgtaca tgtcatcatt tacttttcct ttctcctact aatccataaa 67200
tgtaggccac aaagtgtgag gaaaccagga gattttaagt agtgttctat aagatagttt 67260
aaattgtgaa tatccattat ggcattgtac tctaggaaat acatgttgtc ctaaattgaa 67320
gctttcataa agaaaatttc ccatgtttta aatcaattca agacagatga ttttattggg 67380
caggatatag gaaaagttct gttttaaata tttcttactt taactgatat atttgtatct 67440
ctgtagtctt agtggcagta tcccaatgcc cagaaatggc ttttgtgtgc tcctagtgct 67500
tagtaaggca tcatagctat gtgactgcca tggcattatg attgataagg agtctctaaa 67560

cagcagaaaa ggactatatg taatatatat gacccagact agagtaacaa agctaaacac 67620
cttagaaaag cccagaaaat agtctcccct tcccacaagg gttattattt atgatcaatg 67680
tatgagtttt caaataccct acagcagact cagagatgcc ataagagctg ggggagctgg 67740
gtggtgaaca atcaagttga agtccaggag tcctgactgt tagcattttc tgctttccat 67800
aatgtaaatg ctcccaccat ggccaatttc aagtcaccaa catgacatca gtaaacatga 67860
aggtggagag agatgtgatt gcagctccag cacaccacag ctggggcaag ctgcactccc 67920
actgcagtca gagatggcat atacatacat acatgtcata catgtctatg taaacacgtt 67980
gcaaaatttt gtttgcagaa agtggaattt ttgatgcctc catccaaagt acctagtttc 68040
caacatcact gtagagggaa agagagaggg agactttcga gaaggcacac cctttctcaa 68100
agtgacacat ccaaagtgat gcatcacttc catttctgtt ctggttgtga gaaccaatca 68160
cattgctcca cttcagtaat agtaagctga gtgatgtaga tgagctatat gccaggaaga 68220
ggaaaccagc ttggtaggta taatagcttt tgctaaacca caaggcatcc taaaacttaa 68280
tggcctggac agacactaac tcttgctcat gaatttgcgg atcagttgaa cagccctgct 68340
agtctgagcc aatctcagct gactacagct gagctcaccc ctgcctctgc ggtcagccac 68400
tcgtcagctg gcatctctga ttttgggtgt ggctcgccat taattggaat gaagagaatg 68460
gcagggccca tggccacctg gccaccatgc aacaggctag gctgcagggt tgatgtggtc 68520
gctcggggtt ccaagagcag caagagtaat ttctaataca caagtatttt ccaagtctct 68580
gcttacttca cctttgctac catctcactg accaaaataa gtcacatggc tgagtctgga 68640
gttggtgtgg aggatactgc caaggatgtg tggatacagg gaggtgaatt caattggagg 68700
ccactgggtt ttttcacaca ggctaagagg aatttgaaaa tcactggctt ggcatcataa 68760
tcaatcctgc ttagaggtga tttctttcac accagccatt actggtgctt tttccttctt 68820
gttaaaatct agtttgtgtt gtaaagtcaa gaggagctgg tgattgaaaa aaataacatc 68880
cacatgtgtt tgttgatttg tgatttcaca tctaacctta ctcttacaga gtagagtgaa 68940
agctatactc acatggccta gagataaagg gtctgtccag gattccctta ctcggctctc 69000
cattccaata aagagagcca gccactgata gggcgtggca gctggcaggg tgtccactga 69060
aatcataggc tcttggtacc tttgtggaga tggacatgtg gattcccttc tgtcctgact 69120
ctctaaggac gcatcaggaa cagacagatg ggaaaggcag tctctaagga tatccactgg 69180
ttatgttttc agtgttgata taagtgttac ctttttcata taaattcttt gtattggaca 69240
gaataattgt gtacattgat agtagaagta atgaatatcc agatgtcttc taaataaggg 69300
aagcaaaaga tgtcccactt gcaaatggga gatttatttt aggttttatg catttggtct 69360
tttacattgt gtccttgttc ttatgttcat ggctcatatg caagatggtt tctgagaatt 69420
tcatcatgta attgcattgt tctccaaata agtttatttc aaaatccaat taaaacacaa 69480
gttttagtac atggctgatg cagtattcag tggaggagac tttgttttac taagttgagt 69540
tgttttactc actcaagcag caatttaaag acagatttca tttctgagct gtactattca 69600
ttatcctaat ttatatttat tgcaacctct gagaccacta ttttgtctct ttgcctacag 69660
tcttcagaat ctttaagtta ctgtttcata ggaaaaatta ttggagaaat ttaatagaaa 69720
gagcttttat gaagtactta agcatttata ccacaaaaaa agataagata gctttagatc 69780
attttgaaag tttataaatg ttcacggtcc atttcaagat tactaatctc ttccnnnnnn 69840
acctgatttc ttatgttaca ccttcaggcc ttccctgtaa gcaactgact gcatctctac 69900
tttcaaatcc ttagagtgct ttccaagact gaaataatga gagttacaga gcacacggtg 69960
tccactgcag gctagactct acactgaagt ctcatttatt ttcgttttac agccaaaaga 70020
tgtagatgtc atcatactca tttcctctgt gagaaaactg agtcacagaa aggtgaaatc 70080
cagcacagag acaagattta aactcagttc atcctgtctt caaaattgat attccattaa 70140
accatggcaa tcttaaatct ttctgtgaaa aacagattgt cttcagcttt cagatttagg 70200
gttcatgtac ttataccttc tatccaacca caaatacttg gtgaggaagc tgctgtcact 70260
ctttttaat atatagagca atgttgactg atgaattttg actgtctgat acaaggcaag 70320
agactacctg gtttgggctc acaatgtcta agatgtttct atctctgtgg gcctagaaat 70380
ctacttagac tttctgagcc tgtttctcca tatgtaaaag gggatgcaaa agtatctacc 70440
tcacagagtt gttgaggatt aaagatgtta ttacatgagc aagcaacatc tttggaatga 70500
atcttggcac aaaccaagca ctccagaaaa cagtagctct tatttcaata atccatcctg 70560
ttggtgtcca ttttaattgc gtgagaagcc ctccccgtgt ttggcttaga cttccacagt 70620
ctcctgggag gtttgctttc atggcaggac tcagaatcaa tgcgggaaaa agtgaccttt 70680
cttgttgttc tgcttgaggt tggactcagt cccattgttc cagactcatt gtctcattac 70740
acttttggac acaaagcaag atgaaagcgt tggttgctgt tatgagtttt atatctgtag 70800
ttgtcaaagt tacagagacc ccacgttggc aagtcacctt gtgtggaacc ctatggtcag 70860
gtaccatttt gtagccctgg gcacttttat acnaatcatg tttcnataga aggtgatttg 70920
agctggtata caagacagtt tgagctggtg tctgtcaaaa tcgttgacaa tgctgtgtta 70980
gtttcctgtt gctgctgtaa caaataacca caaattcagt gacttaaaac cacacacatg 71040
tattctctta gaactctgca gttagaagtc caaaatggac ttccttaggc taaacacaag 71100
gtatcaacaa ggctgcattg ctttgggaga ctctggagaa gaaatggctt ccttgctttt 71160
ttcacatcct ggaggccacc tgcatttgca tttcttaact ttatgagagt ctgtaatgat 71220
gtctttataa tgcctccaaa tggttgcaaa actaacacat gcttttgttt ttatgaagcc 71280
aaacagattt agttgatttt tgaccatgga gttggggtat ggggacaagg ttggcagcca 71340

tttttatgtt agtgtagtcc aacatattac attccagcca gcatctgtgg tattctattc 71400
tagtcattat agacttttag aatcatgaaa ctgtgaagtt gcaaataatc ttataggtca 71460
cccagtgcag cctgctatta aggagtatcc cctacaacag tgcttctcaa aattcaacat 71520
gctcacgaat cacctgggaa ccgtgttaaa gatacagatt ctgattcagt aggcctggga 71580
tggggcctga gatcctgcat tcttaataat tgcttccagg ttgctgctgg tccagggacc 71640
acactttgag taacaatgct cattcctgac agataatgct gccttgggga ttaatctagt 71700
atagactctt tgtattttga aaattccagc tgtcatatac acagataagt aatttttgga 71760
tcaaagccaa actaaaaaca tatccaaaac tcctctgctt ttaaaaaatg taaaccaaaa 71820
tgacattgca attaaaggaa actttaaaaa atcaaatgtg tatgtggagt ctgaaggacc 71880
ttatattaaa atatataaat aaaaaagaat atgcacacac atatgtgaat ccacatatgt 71940
atgtactcac atacacatat actagagatg tatgtgttca tctgtctaca tagataggta 72000
gagggataga tcatgcatcc atattaaata atgcaattgt gaagacctca agtcatataa 72060
tattttgtgg tactgactca gctttaacat tttcctttat attaattttt attagaaaat 72120
aaaaaattga aaagtataaa tttggacttt cctgtagata caaggatgaa tctaaatgta 72180
atgtcatcag ccttttttaca ttaaagaaac tgatagccag tttcaagcac tggctgaagt 72240
gaattgagtt gaagtgggcc atgccactag ctctaacttt aaagctggag ttactgattt 72300
taatatttaa atgtaggtac tgcacatatt tatcacctta attcttattt aatatttgaa 72360
agttactgct ttggagaaag tcagctaagc aatggtgatt atagtgtatc aaaatccatt 72420
gtaagtgatt attttaataa ctctgaacca acagggacat gtagacattc agacaaaaaa 72480
caacctaatt ataatgacag caccaaagtg aagctgtgac agttacaaaa ccaatttaaa 72540
agtttcctga cagccatggc taaaagcttt gtttggatct ttcagtaata acagtttact 72600
taagacataa atccacagct aaggaaaata gaaaaagaaa gttgtggctt ttctgccatg 72660
cagatgaaat ggaaatcagc agaaatattt ctaatagaac attatgctga aaggagtata 72720
gaaaaggaat aatagtaatg gaatttaact ctcacaatgc aagaaagact aaataacaat 72780
aaaaatttta atgagcatag aaatgtgcca gatgatttgc ctatctgtgt tatgaaggtc 72840
agtcatttct gcaagtaaat tgatgtgcat ataaacatgt tgattggcag ctctggaatt 72900
tgatttttgt attacctgta aaataaagtc aaagggaaac ggatatgcat caatagccac 72960
ggtgacagga agccttaata aaagtctact ttcccgatgt cactggcatg tctcaaatta 73020
ttagatcctt atcaatgtta cttacctcac tagactatgg gctgagtctg atatgcaaca 73080
taattaattc ttcaacctcg ggtaattaat ttctcattcc cgaacagaaa aatgcttgat 73140
tcacaaaatt gcattccttt tttctgtaaa cctttgtaga atatttgtaa gtgtaatcac 73200
tgttctaagc cctttacata tattgactca tttaaaatta ataacaagtc tttggagtag 73260
gtactatttt ctccacttna tcaatgagaa aacagaggtt taagtaactc ccctggggcc 73320
acagtgccag taagtggtgg agttacggct ccaggcagtt tggcccagag cacctgatgt 73380
aggataagga agacccccatg aaacacatac atagagtcat ggagacattg gcagtgctca 73440
gaagaggcag ttcttgttct cttaccccat tttagtaata acagtttgct taagacataa 73500
atccacagct aaggaaaata gctctttgag ctgcaaggac taaaggattc tcaggtcaga 73560
ccagacagta agggcatgtg ttaataaata cccagaagaa atgaaacctc agaaagagga 73620
tgtgtggtgg cgctagtccc tgggcagatg tggctcagtg gtgtgttgtt tgtgctcagt 73680
tcctttccat aggtctgctt ctctctcctc tttctgctgc ccagggcctt cagcctccac 73740
tctctccatt taagtattct ctaccccaca gtgggcagcg ggccccagaa tggactttaa 73800
atagactagg gtaaaatgac ttagtctctc tgaactgtgt gaaatgtgag taatatatct 73860
caccatatag agctatggtg gatattaaag tagataaaac aaataacatt tgaataatgt 73920
ggattatgac caagtcggat ttatccctgg gatacaagga tgattcaaca tatgcaaata 73980
aatgaatgtg atacatccta tcaacagaat gaaggataaa agctatatga tcatttcaat 74040
tcatgctaaa aaagcatttg gcaaaattca acatctcttc atggtaaaac ccctcagcaa 74100
aactggggat agaaggaaca tacatcaaca taataaaagc tatatatgac agacccacag 74160
ctagtatcat actgaatggg gaaaatctga aaagcctttc ctctaatacc tgcaacatga 74220
caaggatgcc cactatcacc tctgttattc aacatagtac tggaagtccc agctagaaaa 74280
ataggacaag agaaagatta aagggcatcc aaattggaac ggaagaagtc aaattatcct 74340
tgtttgcaga tgatatgatc ttatatttgg aaaaacctaa agactccaca aggaaactat 74400
aagaactgat gaacaaacct agtaatgttg caggatacaa aatcaacata caaaaattag 74460
tagcatttct ataagccaac agcaaacagt gtgaaaaaga aatcaagaaa gtaatcccat 74520
ttacggtagc tacaaataaa ataaaattcc ttgcaattaa ccaaaaaatt gaaggatctc 74580
cataatgaac actatcaaac agtgatgaaa gaaatcgaag aggacaccaa aaatggaaaa 74640
atattccata ttcatggatt ggaagaatca atattgttaa aatgttcata ctacccaaag 74700
cagtctacag atgcaattga atccttatca aaataccaat gatattcttc acagaaatag 74760
aaaaaaattc taaaattata tgaaaccaca gaagagccaa atctatccta aacaaattga 74820
acaaagctgg gggaatcaca ttacctgact tcaatttata attcaaagct acagtaatca 74880
aaacgccgtg gtactgccat aaaaacagac acatagacca acgaaacaga atagagaacc 74940
cagaaggaaa tccacacacc tacagtgaac tcatttttaa caaaggtgcc aagaacatac 75000
actggggaaa agagagttcc ttcaataaat ggtcctggac atccatatgc agaagaatga 75060
aactaacccc tatctctgta aggtacaaaa atcacatcaa aatggattaa agacttaaag 75120

ctatgacctc aaactatgaa attactacaa gaaaacactg gggaaaatct ccaggacatt 75180
ggactgggca aagattttat gagcaatacc cccataagca ccggcaacca aagcaaaaat 75240
ggacaaatgg gattacatca agttacaaag cttctgcaca gcaaaagata caattaacaa 75300
agtgaagaga caacacacag aattagagaa aatatttgca aactacccct ctgacaaagg 75360
attaataacc agaatatata aggacctgaa acaactctat aggaaaaatc taataatctg 75420
atcaaaaaat gggcaaaaga tttgaataga catttctcaa aagaagacat acaaatgaca 75480
caaacaggca tatgaaaagg tgttcaacat cattgatcat cagagaaatg caaatgaaaa 75540
ctacaatgag acatcatatc accccagtta aaatggttta tattcaaaag tcaggcaata 75600
ataaatgctg gtgaggatgc gaagaaaagg gaacccttct acactgttgg tgggaaggta 75660
aattagtaca atcactatgg agaacagttt gaacattcct caaaaaacta aaaattgagc 75720
tgctgtaatt tagcaatccc actgctgggc atatacccaa aagaaaggaa ataagtatat 75780
ctaagagaaa tcttcactcc catgtttgct gcagcactgt ttgcaataag atttggaagc 75840
aacctaagtg tccatcaaca gacgaataga taaagaaaat acagtacata tgcgtaatgg 75900
agcactattc aaccatagta aagaacaaga tccagtcatt tgtaacaaca tggatggaac 75960
tggagatcat tatgttaagc gaaataagcc aggcacagaa agacaaacat cacatgttct 76020
cacttatttg tgggatcaca aaatcaagat aatcaaactc atggacatag agagtagaga 76080
ttggttgcca gaggctgaga tgggtactgg ggggacgggg ggaggcaggg atggttaatg 76140
ggaacaaaaa aaantagaaa gaatagataa gacctactat ttgctagcat aatacagtga 76200
ctgcagtcaa taataataac tgtacatttt aaaataactt aaagagtgta acataatcat 76260
ttgtaactca aaggataccc cattctccat gatatgctta ttccccattg catgcctgta 76320
tcaaaacatc ttgtgtactc cataaatata tatnngtgta tatatatata nntttatgtg 76380
tgtgtgtata tatatatata tatatannca cttactatgt acccacaaat atttttaatg 76440
tgaataatat ggcctggaac atagtaaccg tttaatacat gataaccttt ataataatac 76500
tcattatggc caggcatggt ggctcatgcc tgtaatccca acactttggg aggccgaggc 76560
gggcggatca tgaggtcaga agatcgagac catcctggct aacgtggtga aaccctgtct 76620
ctactaaaaa tacaaaaaaa ttagccagac gtggtggcgg gcgcctgtag tcccagctac 76680
tcgggaggct gaagccggag aatgacgtga atccaggaag cagagcttgc agtgagccaa 76740
gatcacgcca ctgcactcta gcctgggcga gagagcgaga ctctttctca aaataataat 76800
aataataata aataatactc attatttttt aatgtttaat tatctttatc ttaatgtagg 76860
cagtagactt ggaccagact tgggttccag tattgagtcc atcttcaatc cctgtttgac 76920
agtgggaaag ttacttaacc tctttgagcc tcagtttcct tattagaata ggttcctagg 76980
gctgttatga gttaatttat gtaaagcacc tagaaaaact ccaggtagag agcaagtatg 77040
atgtacatgt cacttactag aagttgtagt aaaattttca acaaatattt atggnaagta 77100
ataagtaaaa aataaatctc aggtttctct tacttttcca gttgtataat aatcatctct 77160
aaaatatttt catctgcagt attctcccta aacacagaat aaagaaatac ttgttttgtg 77220
ttactagctt tttaaaagcc atttatccat acagtcagaa ttaaggataa atactaccca 77280
accaaccaat taaaaattaa gatgcaagcc atacacaaat gatatatgtt ttccattttt 77340
agtcaaaagt ctctgaaacc atcttcctag tcactgttta gctgcctgaa agaaaggttc 77400
ccttgcctgc ccagttcagc agtgtgggac atggatggct ttatcttgga agccctgttt 77460
tgcatagcag cacatcacaa aggtgctgtc agccaaggac taagtcaatt acacttaatc 77520
atgaggttta tttctttgtt aagagtctgt ctgtttcgcc ccctcagaaa atgatgcggt 77580
catccgtttt ccacatgttc atcctgagca tggtgaccgt ggacgtgatc gtggcggcta 77640
gcaactacta caaaggagaa aacttcagga ggcagtacga cgagttctac ctggcggagg 77700
taagcagctc tccaccgtgc agatcgctgg gcaaggagca ggtgaaccgc ggacagtgcc 77760
tcttagagat gcaaaagtga agtgtagcct gttcattact tgactttcac caaagctgaa 77820
tcattgtctg cttcttaagt ggttcctctg gtcacagtgt ggcctgattc atttcctgtc 77880
accgcgggcc tgagtcatct tcctcacagt atgtcacgtt gccagggtaa ccctttttccc 77940
ctgggtctta ctctagaatc ttgctgtttt cctatccacc tcttattatc tctccctttc 78000
tcagcgtaca ttagtttgtt tcctactaag tacaagcagg cacagattca gaagatcata 78060
aataagctgg tcagctcagc aatacgtaga agagggctgg aaggacatat agagagatgg 78120
taatgataaa ttactcctct agaaacgggt gttacaacct acattaaaga caccaattaa 78180
aggtaggttt catgattaaa taagcatctc cctagcactc tacatatcat attacagttt 78240
tggaatagta cttcctattg ataaggtaat tttgttaata cctgattctt aaaatatttt 78300
aatttttatt tctttcaata agcattttgc atttatatta aagcatttta ataattttcc 78360
aagatctttg tttttttaat gtaaaaactt acctaaaata tatctcagaa ttcaatggtt 78420
acatatttac aaaaaaattg attgacaacg tagaaaccca ttcattgtgt gagtgaggta 78480
tacatacaca gttgtacaac gcttcaataa atccaaccta tgaaaaagta aactgaaaag 78540
acagaaaaaa gtcacaattt tattattttt accaaagcat ttttcacata aaaaaaatca 78600
atttgccata taattaagtg ttattacatt cctagaattc tctcagtaca tttaaaaatt 78660
gatttggtta ataaaaattt agtctaccca cgcacaagct tttctgaaaa agaattgatt 78720
gcatgtacct aacgagcata gacatctacg tggatatcaa gaaactttta agtagtcgct 78780
tctgaattct cctcttcatg gaagtatgtc tcctgaaaca aaataccgtt aatacaaaac 78840
ttttactggg ctacagtctc tgtgatgcaa catttgccca atggccacct ggtatatgga 78900

```
gcttacaatc tagcaaaaaa aaaaaaaaaa aanncaataa acaataagag ttttgtgcta 78960
tggagaacta atgacagggg ctgatgaaga aattatatcc agcccagcct ctgaaaaata 79020
ggtataattt tgctgaaaat ccagcacacg atgaatggat tgtcaataaa agcagagaga 79080
aggtcgggta tggtggctta cgcccgtaat cccagaactt tgggaggcca aggcgggtgg 79140
atcacgaggt caggagttcg agacaagcct ggccaacatg gtgaaacccc atctatacta 79200
aaaatacaaa aaattagctg ggtgtagtgt cgggcacctg taatcccagc tactctggag 79260
gctgaggcag gagaatcact tgaacctggg aggcggaggt tgcagtgagc caagagcgca 79320
ccactgaatt ccagccaggg tgacagagtg agactccatc tcnaaaaaaa aaaaaaaaag 79380
cagaaagaag cctagactga tactaatatt tgcaggtaaa tttctaacac agggaaagaa 79440
caatgaagga agcctcatca agttatttct gtttttccca ggaagtagga agcaaggtct 79500
gcatccagcc agatgatgag acagtgaagt gggaagttgg caaagaaagg ggagagggaa 79560
gaggtgcctg gggttaggca aaaaattaac aatacagcaa atgcaacatt aaagatccat 79620
ttaggactat ggatttacag tgataccaat tcagatagct ctgcgacttt ttagccctgt 79680
ataagaagag ggaaaagcaa catttaaatc aatccacctt tagggatttt tttcaggtta 79740
ggggaactga gaaacaaggg gaaaagatgg attgctggca aaggagttga aatgatgacc 79800
ctgtggtttc tgttggatgt ggaaatggga ggagaatgga ggagtgaagg tgaaggatca 79860
atggtctcta tatggttaaa ggacgggtac caccaaagtg atggaataat agaaccagaa 79920
gaggttatga tcagagagtg gagtccagga ttttacaatt ccagggtaat tctaagtaat 79980
gaaatataaa atctagggtg tcacgtgaat gtgggtggct taaacatatt gatggagtca 80040
aggcagaaat ggaaacgact gttgaagcta aggcgctggc tgagtcttca catggacatt 80100
gaagtcactc agagtgaggc cagttcttgg agagaagagt ggagtcaggg agcaaagtca 80160
cggggaatga gcgagtgact gatgaacaga ttgacagaag gacagaagac agctacaggg 80220
agagtgggtg acagcatggt ttaacctcat ggaatacgtt gcaaaggagt atcaaggagt 80280
aatggatggg cacctcttcc tcgtctacat gttctactag aaagggctgg aaagggctgc 80340
aggggtaact catgtatttg agagagaatc agttgaggca cagacagaaa gtagattctg 80400
ggaacagaga ttgataatgt agaggacatt atttaacgta ggagagactc aaagattagt 80460
tcaaggctct gaggagggag aagtgggaag atcgatgaga cagaagaatg atacaagagt 80520
aatagcatca gtaggtaaag atttgctggc aaaaatgcca ggaaacaaaa gcagaaatga 80580
ccacaagaga ttacagttat gyttatctct ctgggaggag gaatctgcat ctgctcctga 80640
gggaatcttg acattagaga ttacagtaac attgcttttg tgatctagca cagggtgggc 80700
aggtggtcac actaaatcag tcctggagca taagtgaata ggccctccac actggcaaca 80760
gcagggaagg gaattcttta gtaaatttta cctaactttt tcatgtacag aattgatcct 80820
ttcctaatgt gtttatgtat ggtagatgat ttatcctcat accttaagaa attccaacaa 80880
aatgtcaaat aattgaatgt tgtagagttg actggttact atttaagtgt attgtagagt 80940
ccaggggtag ttggtaaatc cttgtctttt ttcttttta tataatatta aaaataaaaa 81000
caacctcaac aacagacaga caggattgga tggtggaatt cagtatatat tatgtgtcca 81060
ataattgttg tcccctgaca ataataataa gaaagggtgt gggaggaaac tttgagaggt 81120
ggtggatatg tcaatggcat tgatggtaat ggtttcacag gggtacactt attcccaagt 81180
tcatcacatt ggtacattac atgaatgcag cttttaacat gtcattcatt cttttttttt 81240
tttttttttt ttttgagaca gagtctcgct ctgtcgccca ggctggagtg cagtggcggg 81300
atctcggctc actgcaagct ccgcctcccg ggttcacgcc attctcctgc ctcagcctcc 81360
caagtagctg ggactacagg cgcccgccac tacgcccggc taattttttg tatttttagt 81420
agagacgggg tttcaccgtt ttagccggga tggtctcgat ctcctgacct cgtgatccgc 81480
ccgcctcggc ctcccaaagt gctgggatta caggcgtgag ccaccgcgcc cggccacatg 81540
tcattcattc ttaacaaagc ggcaaatcag ttaaccagct aaaaactaac ttgaaatcct 81600
ggaaaaataa ataaataaat aaactaggta gtggtgattt tgttgactta aacaaccaga 81660
tagaccccat cagtgtactc ttgaactact taagcattct gaatttgttc cactctaggt 81720
tttggggttt ttttgttgtt gtttttgtct cctttttttt tttttttttt ttttgagaca 81780
gtcccactct gttgctcagg ctagagtgca gtggcaccat ctcggctccc tgcaacctct 81840
gcctcccaag ttccagtggt tctcttgcct tagcatccca agaagctggg ataacaggca 81900
cacccagcta atttttgtat ttctagtaga gatggggttt cgccatgtta gccaggctgg 81960
tctcaaactc ctgggctcga gcgatccatc catcttgacc tcccagagtg ctggaattac 82020
aggcgtgagc catcactcct ggccgttcca ctctgttttt gattttgtgt gtttgacttt 82080
ccccaagtat gtttcgtttt tattcagatt acattgccat ttctctttgt atcagaaact 82140
tatacatgag acaaagtgat ttgtttcaaa tgaatgtgaa aaagtcactt taattaaact 82200
tactttttca taatatattc tatttttata aagtttataa agatgtcttg agtttaggcc 82260
agggtttggc aaactctttc ttgcagaccc agatagtaaa cattttgcct tttgtagacc 82320
accctctctc tgtctcaact actcaactct gaaattgtac aaaagtgagt gtgtctcaga 82380
atggttgttt tccaataaaa ctttatttac aaaaatggat cttggtttaa gctatagaaa 82440
cagagagcaa tgtcaaataa ttgaatattg gattatattc aattatttaa aatacaaata 82500
aataataaca ttttgtgtta aagtcaaata ttttaatact gacaacaata aagctatgat 82560
ttttgggggg cccattatgt tctaagtgct ttaaaagaat tcattgtctc atttaattct 82620
caaaatagtt ataataacag caattcaagt ttatagacta taatgatgca gtatgtaagg 82680
```

caatagcatt atgtaatatt ttgaaaagtt tattttaaaa atggatcaca acaggtcatg 82740
agcttttaaa gaagttattt tttaagttcc cacatattcc agaataaatt atttagaaaa 82800
tctaaaataa agcactttac ccattaagag ccaactcaca gtctaaaaat caccctttaa 82860
gaataaatta agatacttag cttctgttgt gaagctgggg acttgttttg ttactttgga 82920
ttaattaacc tacatttaga attagttaag aaacaggaaa cagtttacct cttttgtagc 82980
ctatgcataa acagaaaaag gtaaagttta ttttcctctg taagaatttt aacttttcat 83040
tgtgatctaa gagaatatat tattattgca cattaaatca tatgttataa tgaacatgat 83100
tacattttaa aatatatatt attttcttaa gaaataaaat ataagcaata tttttcttat 83160
tattatatga atgtatttgt atccattaga ctgtacattt aacaaaattt atcagcacag 83220
cacaggagat taaaagcttt atcttctttt atctcaattc ttcttttgag aaactcacaa 83280
acagatggga gagatacaac aaggaaggca acggacctac agagaggccc aaagttagct 83340
ctgtgtcacc ccctagcatt atccccagct atgttctctg atttcctcta gacctatctg 83400
aatggggagc tcaatcctct tccacctcct ggtgttaagt attaatgagg acagtcatgt 83460
tttccaatga agccagaggg gagaaagaag tcctaggact ctgtgctaca taccccacag 83520
taaattaact agaactcctc ttaccctcaa ctctgtctat ccctcctacc cagcctactc 83580
ccatatctcc ccaacttgtc atcagccagg caggctcttc ccctgctact catgcacagc 83640
aattccactc cacccccaga cttgcatctt cacttgcttt ttgttcagca aaacaaaaag 83700
ctctgctatt aagaggatct agattcctca gaaaagaaag tatcatctat tacttaagcc 83760
ctcttagtcc cccacaggaa atatttattt taaatacatt cagagtttct ctgcaaagat 83820
ttcacatgga tataaaattt ttcatctcat ttcaattata aagtcagaga acagagagga 83880
agaagggcct agtatccttt cttacatggc agttgtgctt ggtgatgggg aatcagaggt 83940
aaataaagta aataagatat ggccatcgat gtgtgactct aaggacctct ttctactctt 84000
agaagctata gttctgtgag attatctggt gtaatcttga gtgaggtgta tacctaaaat 84060
ttgtggcaat aacatgattt atcaaagaaa ttttagtgac tctacaagaa gaaattaaga 84120
acaatttcca agtcagtgtt tgtctcaaca catcatatcc atatgagcag ttttagttat 84180
taaatccgtg tttccaatag atctaaaaat cattctctat tgtggtagac taaatatgta 84240
ttaagttgtc ttgtcggagg gaatgagtca taaactgcca aggtgctagt tcgctgagag 84300
aagttacagc ttgagagaat gttgtttcta aaacagatta ttgatgaagc aaaacgtgct 84360
gagactaaat taattttaca caaataagat ttgggcagga ttagacttat ttatgtcaga 84420
tacctatgat gtcagaagca accattagcc tttgtgtatt aatatgatta ttttgcagga 84480
ttctttttct atggttattt ttccttcttt ttgaaatttc tatcctttga aaatctgtga 84540
ctttctttta tttccttctt aattagaaag tatttcataa attcaacaca tagagaaaat 84600
attataacca atatttgtgt gtccgtcacc cagattttta aaaaattaat agtttttcat 84660
gtttgggtca ggtttttcaa tcagaaataa aacatcatta gtagagtaga agatccctgt 84720
gtaccacttc ttgatctcct ccttctccct aatccacaga gaactatatt catcatatca 84780
atgcatgctt tataatagta tgaagtatta aggtgccact ttttcaatcc tcattatatt 84840
tgggaaattg attcatgtcg attgaagcat gaatttattt gttctgcatg taagggcaat 84900
gggtatttca attgcttcca actttttgct gttgaaaaca atacttcagt aaacattgtc 84960
attagaattt ctgtgccaca tttgggagag ctctttctaa atatatacct agacttaaaa 85020
ttgtgaattt gtcagatatg ctcactttca actttgctaa atattgttaa attgaacttc 85080
aaagtgcttg tgtcaattta cattcctacc aggatatatg agcagctcta cttcatacta 85140
cttttttttg ttcaggattt ttaagtttct tacaatctca tataatcatt atagtcttaa 85200
tatacatttt cttggttacc atagagattt gacctttttt tcgtattctg tcagccctga 85260
atttcctctt ctataatttt gcctgtttaa tctgtaccct aattgatttt tcagttatct 85320
agtagtatgc agtgcagaca tctcccagcc ttcagcttgt cttttaactt ttatttgctg 85380
tcttttcttc aatagaagtt attaatttta acactgtaag atatagtcat ctttaatttt 85440
tgattcagtg ctttttgtgt cttatctata ccagagtcat taaagacatt ctcttatagt 85500
aaaggctaga gttttcaagt tgtgcttttt caattttaga ctccaataca tttggaatga 85560
gataaggact tgattttttc cccatatata aacaattgtc cttcgaagat tcattgaaaa 85620
ttcattcttt cctcactcct gtagtgcaat ttctgtcata tatcaggttt acacatgtgc 85680
acaattgtgt ttctggacac ctcagtctgt gtctatacat caaagcttta gttgataaca 85740
tgttgtcata agtgctatcc tttataataa accttgagat ctattagggc aagctactga 85800
tgttgatatt cttcttcaga tttgttttat ctgttcttgg tcttgctatt cttgctctac 85860
attatgaaaa tttaattcat tttagtaatg agattttaat tgatgttaag aacatctcta 85920
ttcagttatt ctaaccttcc ttagtgtttt ccaacaatgc tttataattt tctccaaagg 85980
tcttccacat tttttgttac attcatgcct acatatccta cagtttcttt tgtgaatgaa 86040
ataatttcaa ttacattttt attgatgtag gaataatatt aattttttat attgacctaa 86100
ttcatagcca gcaactttac tacaattggt tgtttgtgat catccataga cttgattgga 86160
atttctatgt agataattgt atcttctgaa attaaggttt tgtctttcca cgtctaatta 86220
tcatatctct gatttttttc ttcttttatt atccgggtgt gggtctctaa taaaatgttg 86280
aatagaggca gtgatagtgg acatgctggg tgtttttcta tattttgata cgaatgctta 86340
tgatttcatc attaatgtca tttgtaggtt tttatagatg cctttcatca tgttaataaa 86400
gctcacttct atttctaggt tgccaagagc atttctcatt atgagttgag tattcaatct 86460

```
tcttctacct atactgagct gactacacag actttccttt ttaatttctt agtgcagtat 86520
attagaataa tagatatttt gatactgaaa ctattttaga ttcctgaaac acttgatttt 86580
atagcattac ttttaataaa caaatccaat aagctgacat tatattagga attttcaatc 86640
ataaggatag tagttctgtg attttatttt cttttacatt gctagtctga ttttgttagg 86700
gaagttacac ttcctttcac aaatggaata gacatatttt tctctttttc tcttctgtga 86760
aagaatgaat aaagtccctc tggaacacca tctagggcta ttgtttgcag tgggatccct 86820
ggtggagaaa gggagaagag agatcttgat taatgactta ttttatggtt tgattattat 86880
ggtttgtcag tttcatggtt tattgcttgc tatttataga gtcattaatt ttaactaatg 86940
tctcacattt ccacagtttt ctctcaggat ttttataatc tctattgaaa ctgaatgtat 87000
aatccttttt gctgccaata ttgtttattt ataactttct tttttataga agtaaatgac 87060
ttctgatgct aactacctgg attttagacc aaacttcaca gcacagaggc acagtcctct 87120
acaagattac ccttactcga gatactaact gtaagctcag gggttccag ggcctccctg 87180
acatatgaca tatcatatgg ctaatatggt tgatatgaca tatgacatat catatgacat 87240
atcaacaggc taaaaactcg gggtttcca ctagccttct caagtttgat aatttgctag 87300
aatgactcac agaaatcagg aaagtgctat acttctgatt acatttttat taaagcaaaa 87360
aggttgcaaa tcagagccag ccaaaagaag agatgcagag agaaaggtct ggagagtcct 87420
acatgtgaaa cttgctgttt cctcttttca tggagtgagg acatatcacc ctctcagcac 87480
atggatgtga aacaatattc agagcgctga gaaccaggga agctcacctg agctttgtgc 87540
tgaggcttct gattgggatt tccttaggta ggcatgatcg ctggaatcac tggccatgca 87600
cacaaggttg gagtttctga catggccagt gctcatgaat gatctcattg ccataaacta 87660
ccagggtcac cataaataac aaagatacct atcaattggc aaattccaag gatgtagagg 87720
ctacttccca ggaacttgag gcaaaagcca gtaaaattct ttatttttca ttcaaagaac 87780
cagcttttag tttggttgac actaacagtt tctaatgtac ctgaaattat tggttcgttc 87840
ccaatatttt gaactattaa gtccctttat ttctttgttt tttaaatttc tcctctcacc 87900
tttattattt tctcttttc atttatctct ttggtcatct taaacttctt tgaaaattct 87960
tactggggtt ctctgggtgg aatataaatc attctattat aaagatacat gcatgcatat 88020
gttcatcgca gcactgttca caatagcaaa gacatggaat caacccaaat gtccatcagg 88080
gatagactgg ataaagaaaa tgtggtacgt atacactatg gaatactagg cagcgataaa 88140
aaggaacgag atcacatcct ttgcagggac atggatagat cgggaagcca tcatcctcag 88200
caaactaaca caggaacaga aaaccgaaca ctgcatgttc tcacttataa gtgggagctg 88260
gataatgaga acactgaata gctgaacatg gacacaggga gaacaacaac acacactggg 88320
gcctatcagg aggtgggggg aggggtagca tcaggataaa tagctaatgc atgtggggct 88380
taatacctag gtcatgggtt tataggtgca gcaaaccgcc atggcacaca tttacttatg 88440
taacaaacct gcatatcctg cacatatatc ctggaacttt aaatttaatt gaatttaatt 88500
taaaaaaata aaataatgaa aacaactaaa tgatggtgaa aaaaaagttt gacaaaatta 88560
attttactga gagtaaattc atagtttgaa ttttagaatt ttgttttctt tctcattaga 88620
tttatttaaa atctttgcat gctgcctcat ttggcttggg agattttctt atttctctct 88680
ccctccctct atgatcactt ctactttctt agggatattg cagttgcctt aaccttgacc 88740
ctggacccta ccatccagag ccaggttttt aagtgtcatt tggagaatgc tagccccttg 88800
atatgggtga tatcacagtt cttgtcacta gtctatattc cataaaatat cttgttctga 88860
tttttcagat aagctgataa tttctcctcc tgcctctgga ggcctgcagc tgcctataag 88920
gtcttagcct tgggtagttg gtctctgtcc tttccttagc agagaaaccc attctagtcc 88980
ctgtcttttc actatgaagg ttctcatctt taccttatct aaggtgcctt agtctcctca 89040
ccctatagga agcaattgag tccaacattg cctgattctt gacctagcat atgtttctgt 89100
tgcatttctg gtcaacaaag aggttaacct cgtttagaat tccttcctgc ctctttggtt 89160
tcctgttttt tcctttgatc acagtatgtt tgaaacagaa ggggtttcat aaatcatgaa 89220
ctcattttgc cgtcttgcag ggagtcctct tttctagaaa agtgatttct gcattcctct 89280
gaagacattt ggttttatga tatttgttaa aatgaagagt catttatttt ggccccaaca 89340
aaatctgaca ccccacgtttg agttcattaa tcataaaaat tggccaaatg aagaaaataa 89400
tcaatcttgc catagcagca gtctttgaca ttattggtga gtttaatttc attttctggg 89460
aaacttcaag taaataggtt tcttctgaca nntacttttt ggggggtgtt tctaaaagat 89520
ctggacatta tcttgttagg taaaaatata atacaataat gacaatctgg acttctcaac 89580
catccatctc aaaatttgct ctttataaca tactgatata cactataagc taagagtagg 89640
tgatttacag acagctccag actgatacag gtaatcagta atcacttttt gttgttattg 89700
aatctgaatt tcatgtaaga gaatgggatt gcctgaactg attttcttgt gagctttgtc 89760
ctgtaatctt cctttgtgat aaatatgcat ttttttctca tgatatccat catgattctt 89820
tgaaattaga aaacagagac tgcaacacaa atattagata acacatttca aatatctttc 89880
cactcatggg caatgtccat ctctaccatt ttatctcata ggcctgtgtt tacgagatta 89940
gttaaactct gctgttgtta ctggtagagg gtctcgactg caagttgtcc aggttcttga 90000
cgttttgaat aaagaactgg acaaaacgca cagcaaagca aggaaagaaa gaagcaagga 90060
aagcagagat ttattgaaag tgaaagtaca cttcacagtg aggaaagaaa gaagcaagga 90060
agctcaaggg ccccagatac agaatcttct caggtccaaa tgggagtgag cccaagcagc 90120
ttggccacct acttggtgtt caccccatgt aaatgaagtg gttgcccgca atcagaggct 90240
```

gaggtgaagt tacgaagtta cattcctagg caagtgtctg gttgggaaaa gcaaccgatc 90300
agaggtactt tcaattatcc atctgccgca cagaaaaggt gggggtttgc aaagggagta 90360
gcccctggtc cttttgctac tcaggcatgg aaagttaggg tttttctttc aatttagttc 90420
taagaagtca gtgtgaaatg gacttaggtt ccctgcctcc agaccctatt ctcctgcctc 90480
attatggcga catcaaacca ggtatatcgg ttgagcatgc attctcaaca gggagtaaaa 90540
tcacctccaa aggggtgaaa atatgttctt agttgggggg tagaaaattc tctcttttat 90600
aatgcagatt gccttgtaga ccaaataacc aaagtagagt atgtaaaaag atatataata 90660
tggtagaggt atcaaaactc catgaggtgg tgattaagaa caaaatctct aaaagacttc 90720
ttagaagagg tgatttaaaa aaagattggg aaacactgct ttggggtatt tgtaagaaga 90780
aataaatatt tctcttcttt tctatgcatt gtttctaagt tttcttaaag taatgcaaaa 90840
aaaaagaaaa gtttattata ctcttacaaa ttttgtctta actcttaaaa agttaagttt 90900
taacttgtag gtactcacaa aggtggaatg cactgcagaa tatgaaacag aagaagggtc 90960
ccaatgagcc cttcttggtg caggggctgc agactgagga gggccaaagc agtggagggc 91020
tgccaggcta agagtgacaa atggaagcca ccgttccact tttccacagg ctgcctgctg 91080
aatgcctggg atttgcagaa agccttgaga ttggacacct gcacaggtga ctttggtggg 91140
agccctccag gcaactaggt tctgcagaga ccccagttgc cttctccctt ttagatggct 91200
cccagcatgc ccctggcaaa ttccaccctg tccagctacc atcaaggttg agttcactgc 91260
aataatctgg agctgcacat tgagaagtgg ttactagagc ttgcctccac tgcttctatt 91320
ttggttcttc tgaactatta tagtcctctg aatttaagtc ctgtaccaaa ccccataacc 91380
tacactgaaa tacttagtga tcatttaaac taatttgatt ttcctaatca ccttctttaa 91440
atcctgacac agttcttctc tatggcagga taacccatac cttcaagtta agttaggcat 91500
atttttatgt gcattgattt tccataaaat gttatcactt tggcatgaat atttcagaga 91560
gctagagtgt tggcttgtaa aagttaacaa attatgttca aagcaagcca tttgattcca 91620
agagaatctg gaatcaggaa ttctagggga aaatcagtga gactatagat atttaaatat 91680
aatcacataa caatttgcta aatcttttag tgccttttag tgtctttcaa aagaatatca 91740
ttttcccaca gagaaaattg taatgcaaac agatccagct gcatatttta ctataaatta 91800
tgctcattta aacagttcat tccatctgtc cttttgataa tatatgcagt tagtaaggtg 91860
attaattgta tgtatacata attagacaat ctattggaat atctttcagg cattcattag 91920
tagttttctt ttaaaaaaac actttaaaat ggatcaccac tcttttttttt ttggaatgaaa 91980
atctcagtct gaaaatacag gtgaagggag agatgatgta atgaatgcct attactgctt 92040
cctctgctaa tacagcaacc aggtcaggca tgtggatggg accttcatga cacatcaatt 92100
tcagtttctc ctgagattcc ctagtatcca taggagagaa aacaggttca agtcttcccc 92160
atatcattgc aaggccagaa ctaagaagcc tgtgctagag aggaagtacc tgtcgcagga 92220
cttgcagcaa agtctgagtc ctctggtccc ctgaggccac tgctcagggc agagagcctt 92280
tcatttcccg cagaggagct ggacctgcct gtatgctgct tgtggtccag gagcagtcca 92340
cagagtcctg tgggtctcag tgggccagga agggaagctc actagagact gaattactcc 92400
aggtggagaa gggggtcaat aaatgactcc actcatttct gggaaatcct gtcaccaaaa 92460
agccttcttc agatgttcag caaaggagct atggcttttc aggtctttcg cctccgtaaa 92520
ctatggtgcc ctctactacc cctcatttgc cactgcttaa gatagcataa aagacaaacg 92580
acaattacat caaaaacatg tatccatggt gaaggaaaga aaagttaact cacacacaaa 92640
gggagactca gaaactgagg atgaaactca gagaatcaag acaaatgtct agtctagaga 92700
tttcccaagc aagtagaatt gatgggcgag agaatccgtt gttgttgttg ttgttgttgt 92760
ctcaagaaaa agtaagcagg gcacaaaata caatttctgt aacttcaact aaaaatgtcc 92820
aaaaaatgag ttaaaaaagg gcatagtcct tgagagctag tttttaatat gggagatgaa 92880
gcaggaaaaa ataactcaaa aagtggtaga gatataataa taagggcaaa gtaaaatagc 92940
ctatccacac agctatgaat aataggagtc cctgaaggaa aaagcgaaca gatagaagaa 93000
aagttgtcat aaacaacgga agaaagctta cataaatgaa aaaaaaaaaa aaacataaat 93060
ctgcaaactg aaagagctca ctaaatttta gtgagattaa atgaaaatag atgcttactc 93120
atgtcctggt gaccttccta ggttgcaagc ataaagaagt aaccttgttc agcccctaaa 93180
caggctgaac ctaaacctga aaaagtaaaa taaatgtatt gtgcaatgga tgactcatct 93240
acaatactgg aaagtggaag gtaatgaatc aatatacata gactactgag agaatagaac 93300
tgcaactaca gaatctgaac acagacaaca tgttcatctg tcagtgttaa aaatgcattt 93360
tatatacaaa aggactaaga gagcataaaa ccctttgtat ttatttgaga aaactacttt 93420
aaagtgtgct aatcacaaga taagtaaatt gcaatctcaa tgactagaca aaagaagaga 93480
gaattgatgg gcattggatc tcaatagata ataataaata tgtcaaatag ggatctttga 93540
aaaaagagaa atgataacat taaagaaact agtaataaat ttgtaaatga tttcagcaaa 93600
accaggactt aggaaagggt gtgatatcaa aggagagtgg gatggtagaa gtgtactatg 93660
aagtttgcag atgtgtgggg tggagggatg agtgagccag caatttagtt gtgaagcaat 93720
aagaaaaaag cattggtttg atgtctaacc attgagaatt aagccaggag attagaaaaa 93780
ccaagtcgag cgtccaaatt aatgaatagg gaaagaaatt agcaaatagt tgaccaagtt 93840
aaaaaaagac aagataaggt taaaaatgaa acagcaatga cataaaatac acaggaagcg 93900
taagaccaag tataactttg gcacattttg tttgagcagg ttgaattttc ttatcaaaaa 93960
ccctagaaaa tctgagtatg tttaaacaca aaataaaaat tgtacactgc ttataggaag 94020

cacatacaaa gtggccctgc aaggtaacta aaaatcattt ctaccaaaca cttaaaatat 94080
taagacgttt taaatgttca gcagagtgtg caaaaaatta aggaaaattc ttgaggccaa 94140
aaattaaatg tgacgtgagt ccagaatacc aggtgggccc caaggcagtg gctgccctga 94200
ggacatttgc aaatccttgt gaccagaact tgcagttttc aggagtgtgt ccaggatgga 94260
agtgactaga ggcctgtgca aagtgaagag gcagaagaga ggccccacat aacatcagga 94320
aggaaaaatc ccatccctca gagggaaagg atgggatcat cattctggac cttgacccct 94380
ggcagatcaa aaatgttatc tctggccggg catggtggct cacgcctgta atcccagcac 94440
tttgggaggc cgaggtgggt ggatcacttg aggtcaggag ttcgagacca acctggccaa 94500
catggtgaaa ccccatctct actaaacata caaaaattag ccaggcatgg tggcatgtgc 94560
ctgtaattcc agctactcgg gaggctgagg caggagaatc acttgaacct gggaggcaga 94620
ggtcccagtg agctaagact gatcatgcca ctgcactcca gactgggtga cagagtaaga 94680
ctctgtctca gaaaaagaaa aaaaaaggtt atcatgttat ctgtactgaa ttgtgtaaga 94740
cagcctgccc actcgtgggg ttggagctca aaatcccatt acgtccagaa tctgagaaac 94800
agcagccaaa agttgattta agggggaccc aaatttctaa gatgacccag gttccctgga 94860
agaaccaatt ctttctggaa aaatatgtac cctaaatgca ggtcttgaaa atctagagac 94920
aatcaatgat ttcctggcaa aatacaaact gccaaattga tggatgagat aagaaacctg 94980
atctgaacag cactggacag ctttgctcca ccatcaccaa aaggcctgtg cccatgtggg 95040
tttcctggtg ctttctgtct acttttaaag gtgtgctaat tcttatttta tttcagaaat 95100
tttcatacaa aagataaaaa ctgtacagaa tgaatgataa agctagcata atcttagcca 95160
aaacctgata atgatataca agtaaaacta taggccaatt tcacataaaa cagtaactgt 95220
gaaaattata aataaaatat attacccaat aaaatccaac atgttagcat tatcatctag 95280
agttttttc caaaaataaa taaatggtta acatcaggag cttttgtgtt atatttcatt 95340
gcctcagtga attaaaggtt aaatcctttg aaggagaata ggaagaaaag gaaagtcttt 95400
aaacatgata aaaattaatc accaagactc atttcaacca ctatatatga acgctgaaga 95460
aactttcatt gaaaacaagg agcaagttat ggattcctat tattaccatt ttgtatttag 95520
caaccattat ataactgtac ctttataact aatgcattca tatgattaac tccaaaataa 95580
atcttaaaaa tgaaataatc ttattttatt tccttatgaa attgtgcaca tttctataaa 95640
aatcgtgaga ctttatgaaa aatattagaa ttaatagaag aatctagtaa agtgggtaca 95700
gaaggtcaat atagagaaat caatagcttt tcttcccact ggaactaact gggtagaaat 95760
agtaaaaaag aaaaaataca tagatagata gagagagaga gagagaaaga aagataggat 95820
atttaggata tttatntnna nnaaacttat taaagaacgt aaaatccagt tcaaataaat 95880
aggaaaagag tgcattcctg gatatgaaga ctagtattaa tcttcttaaa gttctatttt 95940
atatgtaaaa ggtaaagnaa aaaaatcata aagttgtaga catatgtgaa catgtaaatt 96000
ttatgtttgt agcactaaca atatcaaagt tagaaaccag actgttggct agggaaaaaa 96060
tgtttgcagt atatatagca aagattattg tcctcaatac caacaactcc caaaacgcat 96120
aaggaacaca acataacacc atataatagt gtcaaggtta cggacagtaa catttaatta 96180
ctttaaaaag aaattaaagt gattaataaa catataaaca gatgagcaac aataagtttg 96240
attacactga tactggcatg gatttgagac aagataactt tcctgtattg ctgaagggca 96300
aatgatttct tataatattt tgaaagtaat cactttaata ttttctaaat ttaaacacaa 96360
ctaatctgtc tttgccccag caataccaac tttggaaatc tatttataga tatgaaagcc 96420
tcaataaata ctggtattga gggaggatgc ttagtgcagc ctttttattg aagtagcaat 96480
gtgcttaacc accttcctga tgattcctgc aaataccacc catcaatgaa ttagtagaga 96540
aaatgttgag ttacataaac attgaacact atgggaaatt atgcagctga taaataaaac 96600
ataggaccta tatcccttaa tctggaggga tatccacgat tgttagtgac tcagaaaaca 96660
agttgcagag tgatatataa tattcctgca tacccaaatc tttatcattc acagttcacg 96720
taactttagg taataccaag aatgcctatt tgtctttaga attttttttc tttccagagt 96780
aaatacctgt cacattattt ttaacaactt catagaactg tatgtaatat ttatacataa 96840
taagtgtata gaaagtatga acttgacaga ggacaatgac atctttaaaa ttagaaatag 96900
tgctttcata aattgtaaca attgttttta catctcatgt aaaaacatgt aattgcaagc 96960
ctgttnctgt gattgcatat gtgtggatga agaagcagat gatcaaggaa agaatataca 97020
tgacattaac atgaaatacc tgagaaacnt gggcaaagga gttcatncca cgtgtttngt 97080
ggacttaaga tcatttcaaa tttaaaatat attaccttgt tatgtttttt aaggagggaa 97140
atggcaatta aaattaaaaa gcaactagaa tctgcagaaa tggtagattt gagaagtgat 97200
gctaatttcc tcttcacatc taaattaact atctgcagtg cctttaatgt ttcatttctt 97260
aaaacactat tattaaaact gcattttccc cncatgttcc atatatgagt ggaagaaaat 97320
cccttctgat actccacatt agtctagaag aagagaagca aggtctgcta tattcctatc 97380
ttcaataaat catgtagtgt cttctcagag ggacatatta tgactaatcc cagtagtcac 97440
cctcatagtc tttaattaga agttaatctt ctattgcaaa ttttaattcc agctgcaatc 97500
tcctttacaa aatgaaaatt cttgttcata ggacatgtaa aggaaataac atgctgtctt 97560
tatgtgtgtg ttttataata ttgttatttg gaatagtaaa ttcatagaat gcatgtgaga 97620
gattaaaaga tcaaataaat gtgttaatat actaatattg atcattaatt ttattaatat 97680
taaagtaaca tattgattta tttgcataag cacacataga tcaattgctt ggtatgaaat 97740
ctcaatttct cttttgccat gtggcctaaa gcataaagga ataaatgata ttttaacttt 97800

taaattttag tttaattagg ttaataatat gtaagttgtt attttataat atgccatgta 97860
cattaaataa attttatctt aaaaaataca acatacaaaa aaattcagcc ttttgtgtac 97920
atagaaagtc tttaaataat aatggtttta aaataatatc aaatatagat aataaagaaa 97980
taatgccact cacaaacatt caaaatcata acatgttttt ataattgttt aagattgctt 98040
tggtctactg tgttctagtt ggtatgtttc aaatgcattc ttaaagtaaa atgtcattct 98100
tgaccttatg gggcacctta gtgtatatga tagtggcaaa aattcataca ctgacaaata 98160
tgtgaaataa tttagaataa attatagttc tgaatttcaa tttcaatgtt tctttgtttc 98220
tctaggtggc ttttacagta ctttttgatt tggaagcact tctgaagata tggtgtttgg 98280
gatttactgg atatattagc tcatctctcc acaaattcga actactactc gtaattggaa 98340
ctactcttca tgtataccca gatctttatc attcacaatt cacgtacttt caggtaataa 98400
aaataatgcc tatttgtctt tagaaattta tttatttcca gagtaaatac ctgtcacatt 98460
attttaacaa ccacatagaa ttatatataa tatttatata taataactat tatggtaaat 98520
atgaacttga gaaaggcaca atgacatctt acaaattaga aaagtgcatt cacaaattgt 98580
aacaatcgct ttttaaaact cctgaaaatt cacagaaaca atgtagatgt tttacttact 98640
tnttcctaaa ttagcagtat atagatacac atatttttat atatggataa catatatcca 98700
tacatgcgta tnnaantttt aaactacata agatgttggn ncaaacttta aagactagga 98760
aacattaatt taatcccaag tgtttctctt ccttgtcgat aaggctaagt gtttgctcaa 98820
caacggttta taatgaaaat attaaaggtt tataaaactt acctttacag attgtaatta 98880
ctcatacagt attatttata aatgcctgtt acttcatgat ggcacttata attcttattt 98940
aacttgtaat aattggatat ctttttttaa atgtgatata ttggatcaca ttgctttcta 99000
attatgcaat aaacagtaat tattagttac attctttgtt attattgaat atagcaacac 99060
gctcactgtc ctggtaaata cttagtcaaa atgataaaat atgtctacat ggtattctag 99120
tcaaaaacac ctcaaatgta gtcattttcc tgattcaaac ttggtcattt atttttttatt 99180
ttattttatt ttattttata ttattttatt ttttatttta ttttatttta ttattattat 99240
actttaagtt ttagggtaca tgtgcacaat gtgcaggtta gttacatatg tatacatgtg 99300
ccatgctggt gtgctgcacc cactaactcg tcatctagca ttaggtatat ctcctaaagc 99360
tatccctgcc ccctcccccc accccacaac aggccccaga gtgtgatgtt ccccttcctg 99420
tgtccatgtg ttctcattgt tcaattccca cctatgagtg agaatatgcg gtgtttggtt 99480
ttttgttctt gcgatagttt actgagaatg atgatttcca atttcatcca cgtcccctac 99540
aaaggacatg aactcatcat tttttatggc tgcatagtat tccatggtgt atatgcgcca 99600
cattttctta atccagtcta tcattgttgg acatttgggt tggttccaag tctttcctat 99660
tgtgaagagt gccgcaataa acatacgtgt gcatgtgtct ttatagcagc atgatttata 99720
gtcctttggg tatataccca gtaatgggat ggctgggtca aatggtattt ctagttctag 99780
atccctgagg aatcgccaca ctgacttcca caagagttga actagtttac agtcccacca 99840
acagtgtaaa agtgttccta tttctccaca tcctctccag cacctgttgt ttcctgactt 99900
tttaatgatt gccattctaa ctggtgtgag atggtatctc attgtggttt tcatttgcgt 99960
ttctctgatg gccagtgatg gtgagcattt ttccatgtgt tttttggctg cataaatgtc 100020
ttcttttgag aagtgtctgt tcatgtcctt cgcatttata taatttatca tggaaacact 100080
agaatgtgca ttgattgttt tctaaataga tattgttaaa tatttgggaa ataaaatgac 100140
agtagaattt taaagaagta attttgagaa acaggactct aacccctag gaaaaagaac 100200
atgcattgca actttattga ttttatttat tagtaatacc aaaatattat tttcagaaca 100260
atattcactt taactgccag atcacaggta actagacact tttagtacct gggggaaagg 100320
acatgcaaat ataattcaca aaaagtaaaa agcaaaaccc cctacaaacc caggcaaaaa 100380
gctgagccct tgtaatcaca gctaagcata ttgaaaccac agagggtttt catagttcaa 100440
gcgctcactt ccctcagcat ccgtggatgt aaatctctcc tcatktgttg agttatatcc 100500
tgaagcttrg ctatcataag tgacatggct ggggcaagag atatacatgt ttaaaactct 100560
tgatacaaaa tgtgctttcc aaacattcct gtgattataa gctcttgtgc actgctctct 100620
agattcacaa agttatcatg gccattgagt acctacctta gggcatcatt gcacataatt 100680
atagagaata tcctttatat caaaacaaca gcatacettt tttacaaagc tattaaagta 100740
gccaaattaa gaaaattggt gttgcctatt tcagcaaagg tacagtataa aatatattca 100800
gggccttaaa attgctatag tctctgacat gccattttttc agtaaaaagt tagaaaataa 100860
catttgtgtg caaaatattt attatagaat taatgagaga aaaaagaaaa ctgtttaaat 100920
gacaaacatt tgaggaatag cttaataagt cataacgcga atttaaccat aaaattataa 100980
ttgtgatgag ttttttataa cttagaaata tgactgtttt atttaaacga taaacagcat 101040
aaattatatt atatacataa ctacatatat gtatgtatat atttattata tacatatgta 101100
aaatacctat aaaatggaaa aaaatgacta gaaagaaacc aatttattac aaattattaa 101160
tcatctctag tttcctactc ttgtcatcat gtctgatttt cctactagtc tgttttttta 101220
caaaatatct aatattagcc tgcaaggctg taaccatttt atagatagac agatgataga 101280
tagatagata ggtagatgat agatagatag atagtagata gatagatagt agatagatag 101340
tcaaatcaca gtgaagtttg caatttcnag ttnngncntt gattttggat tattctgtaa 101400
nnngtnctgt ttaatttatg ttaatgatta ggtgatttc atttgtatct ctctatacat 101460
ggccatgctt ctctttttct aatgtattaa taatgaataa aaatgatctc agttaatttg 101520
tggctagaaa gtaaatctct tgagaaactt aggtgtcaca agggtttttg caaatttgca 101580

tgtggagaat ccactgtacc agacagagcg gctctttgta taaaggccta cattggtctt 101640
ccctattggt cctcacactt ctcatttcaa tcaacctttc tttactgtgt tggcaacaag 101700
tctttcctta gacaatttca tatttctcac atttaaatga agtaatgttt agtgcaaagt 101760
ggcttccttt aaaatattag ttatctgtta gactaactac tgaagttatt catgtattta 101820
ttcaagtgat atttctgagc ttctactatg tgtcagccac tgttccagat actgaagatg 101880
gagcagtgga caaagcagag aggattccca ttttatgaag ctgaaattct aaaggcaaac 101940
agttcacaaa gaagtacaca aaggaatgtg aagtacaagt actggaaatg tcttaaggaa 102000
gaagtgtgca gggtaaaaag catagagatt gtgtagtagg ggctatttta gattagttgt 102060
tcatagaagc cctctctgaa gaggtgaggt ttgagcagag actgaatgaa gtgaaggagc 102120
aaactgtaag gaggtctgag gggaagcatt ccaggtggag agaatcacac tgagcatgtc 102180
ctgaggtggg tgcatgattg atgtgttcaa ggaatagcac aaaggccagc gtggctgggc 102240
tgcaaccaat gcagggcagc atggcctgtg ggaaatcatc aggtcttgca aaacgctgtg 102300
gttcttacat atcggtatca tcctgatgat attgactggt gcgtcttgtg aatttcctgt 102360
gggtcgggca tcttgctgag tgcattgtct catttgtttc tcataacaag ccctaagata 102420
aggactgtat gtgtaaattg aggtctcaag aaatctgttg acttgcccaa gaccgcatac 102480
ctagcagatg atggagctaa atcttcttcc cggctgggcg cagtggctca cacctgtaat 102540
cccagcactt tgggaggccg aggcgggtgg atcacgaggt caggagatca agactatccc 102600
ggctaacatg gtgaaacccc gtctctacta aaaatacaaa aaattagcca ggcgtggtgg 102660
tgggcgcctg tagtcccagc tacttgggag gctgaggcag gagaatggtg tgaacccggg 102720
aggtggagct tgcagtgagc tgagattgcg ccactgctct ccagcctggg ccacagaggg 102780
agactccgtc tcacaaaaaa aaaaaaaaaa attcttccca agaacatgaa tttcagagtt 102840
catattctta actatgcaat ttctacagca tctcactaca aatcaacaga gtgaccttta 102900
agagaaggac tcagaaatgt gaccaggaaa atgtgtttca gagacaaaat gttctgaagt 102960
gcttatcttt gagagtatgg tttacttcat gttgtatctg taggaacaca aaatcaaaaa 103020
tctttatagg gtagatagag tgaaaaacct gctgatccct gaataaatga aaaggcacaa 103080
ttagactcac tgacagggat ttttcctttc taggaatgca ggtttaaccc tttgaaaatg 103140
ctgatcaaat caccaatgtg gaagtggtat taaatagttc catttttttc aagctgttta 103200
cagttaggta ctgaattatt tatgtccgaa aatagacaaa tctgataaaa atgtgaaatc 103260
tttccagatt attattctgg aaattaatac gaatgttaat actttaatag ttcttatgat 103320
aaagctgttt gcttgttctt tgtaaagatt atctgagagt aatatgtcct gatttgggaa 103380
tgtatgttaa gaattacagc aggatggccg ggtgcagtgg ctcacacctg taatcccagc 103440
actttgggag gccgaggcag gcggatcacg aggtcaggag atcgagacca tcctggctaa 103500
catggtgaaa ccccgtctct actaaaatta caaaaaaatt agctgggtgt ggtggtgggc 103560
gcctgtagtc ccagctactc gggaggctga ggcaggagaa tggtgtgaac ccgggaggtg 103620
gagcttgcag tgagccgaga tcgctccact acactccagc ctgggcgaca gagtgagact 103680
ccgtcacaaa aaaaaaaaaa gaaaagaaaa gaattacagc aggaaaaatt ggtagctgca 103740
ccatgtggta tgatgcttaa tttgaacaac ataatttctt acatatgttt aaaaattgca 103800
aattagcatg aaggaaacat gctttagtca ttttttttca attggtttac ttacatgctg 103860
taaaccatat catttgcatt ctgcagacaa taggaataag actgaccact gatacttcct 103920
gttaaatcaa agtaaatgca gtaaacgtgg tttcaggtat ggcaaaattc agaggccgat 103980
atggtgtcag gggctcagtg tatgtcttca cctacctttc ttctgttggt gtgtcggtgt 104040
cattcttaag ctggttttct ctatgtgaag gcaaagatgg ctcccaggag ctctgtaaga 104100
gtttgtgtga ctcttacagc agtacccgtt ggggattaaa aaaaaaaaaa aaagaaatag 104160
aacatttctt attaaaggca tccagcaaaa gccctgagga tgactcagat gactgtgaat 104220
actctggcca gatctggatc atgacgggtg gggagtcctt cttgcacctt tgtactaagg 104280
ctggggtagg aatgatttgc taaaggaaaa ccaggcagag ttgaagaagc cagaggcact 104340
ggaaagccaa aaagaaacaa atgttatttt agacatgtaa aaattaaaat ctaattttta 104400
aaaatgaatt ttctgtttct tactgtgttc taaatacact attcatcttt aacagacaac 104460
tcaatctctt attgacatca aaaaatcttg gatgatcatt gtataaacta tatttcattt 104520
atgccaagaa tgcaattttc ctatgttaat atctctgaat ttgggagctg ttataatcag 104580
tggtgtgtta tactttaatt ggcagcattt ttttatttct tagagctaca tacaaaattg 104640
gtgacatctt agacttaatg aaaagttgta tttttcacac tatatacttc agcttttaat 104700
tatgtactaa tttatacata ctaatcatat aaatatgtcc ttaaaagctg tgtctttgtg 104760
ttatactcgc tttgtaatag tagccattga atcctatttg tccaacacaa agtttggcac 104820
atagtaatca ataaaggttt gttaaacaaa tgagtgaata aatttgtcaa ttaattactt 104880
attttttttt gcaaatgtga gtatgcaaaa catctaaata tatctgtata tatttagatg 104940
tgtgtgtgtg tgtgtataca cagtcatgca tcatttaaca gcagagatac attttgagaa 105000
atgcattgtt aggcaatttt gttgtcgtgt gatcatagag tttacttaca caaacctaga 105060
tgtatagccc actatatacc taggctatat ggtatggcct attgctccta ggctacaaac 105120
ctgtacagtg tatgactaca ttgaatactg taggcaattg tcacacaaag gtaagtattt 105180
gtatatctaa acatagctaa atatagaaaa ggtaagtctt ataggaccac catcctatac 105240
gtggcccata cttgactcaa aagttatgcg atgcatgact aaaatatatg taaatatata 105300
cacacacaca tatacatgcg tgtatatata gagagagaat agttgagtac atataacaaa 105360

gatccaaaaa tacaaaactt acataagaca gaagtttatt tctctgcagc agttggtaag 105420
tatgcaggga aagcaggtgg ctgtgccccc catggtcacc cagcctgtcg ggttggacag 105480
gacctgtcgt attctatgca agccttctat ttctggtcca aaggggctac actatttgtt 105540
tccatttcca ggcaagagaa aggtatttta aaaaggattc agagaaagtt taacttaatg 105600
caccaaaact tgtacaaatc atttcctctc acattccgta ggtctgaacc cagtccactg 105660
gagacagcta gctgcaaggg agactggaat gtgtaggcta tagccccagg caaccacttg 105720
ctaggttaga attcggaaag ttctgttttt taaaggagga aagagaaaat ggatactggg 105780
ctgataatct caataagaag taaagattct tggcaaaagt ttgcttctct ttccatttcc 105840
ttgtgatttt ctaataatac ctaggaaaat attttatgag acagtgaata taactaatac 105900
aggttgagta tctgtatccc aaatgcttgg gaacagaagt gttttggctt tcgatgtttg 105960
tcagattttg gaatatttgt atgtatgtgc ataatgagat atcctgggaa tgggacccaa 106020
gtctaaacac aaaaattgtt tatgttttat aaacacctca aatacattcc ctgaaggtag 106080
ttgtatgcaa tattcttaat agttttttta tgcatgaaac aaagtgtgta tacattgaac 106140
catcataaag caaaggtgtc aggtgtagaa tttttcactt atggcctcat atcagcactc 106200
aaaaacttta aatttttgag cattttatgt tttaaattag gaatgttcaa cctgtattga 106260
attaaaggtt tccttgcata tttgaacgtt agaaaaatat taattttatt tcaacaatac 106320
gtactagtga atatcatttt gtataatcat ataatatgtt aaaacactta gttcatccat 106380
tcatttggga tttattgagc tagaccaatg gttattctat tgttaatttt agtcactaga 106440
tattcaaata gagaggtatg attttcataa agcactataa aataatattt tagtatgaat 106500
atatttaaga ttcagtgtaa ataaatgatc atatgtgtaa ataaatgttt ctctctccct 106560
tgataggttc tccgagtagt tcggctgatt aagatttcac ctgcattaga agactttgtg 106620
tacaagatat ttggtcctgg aaaaaagctt gggagtttgg ttgtatttac tgccagcctc 106680
ttgattgtta tgtcagcaat tagtttgcag atgttctgct ttgtygaaga actggacaga 106740
tttactacgt ttccgagggt aagagtttta aaaatgcagt aagttaaatt cattgttctt 106800
atttttagtaa taatgattaa catcaaagta atttcacttt agtcattcag aagtattatc 106860
ctatttttga taacttggga taattagatt ttaatattta aaattgtctc ttctgcaggg 106920
tgcaggtgtt tgagaccatt ttcagcattg tcagattata tatcaattct aaaattctgg 106980
aaataatcta atagagttag atgctgaaac ctgaatgcta gggttcttag cacctataaa 107040
ttaaaataca gcagaatttt aaaataaagc ttgcattttt gtgttcaaga gagttaacat 107100
atttttaatg ttttataatt tatatattgt tatgttagtc agaatctaga aggaagcttt 107160
tgattttcaa taaaataaaa ttcataggct cttcagattt taaatgtctc tcagctttct 107220
cagtagaatt cacttaagct aaaaaggaag ttagaaatca atggatccat aaacaaaagt 107280
catgtaagag gtacagctct taaccagaag gaccttgaaa tttaactcta tgtggactta 107340
gaaacataaa aaaatacaat gaccagacaa ataatgtata tatttttacc ctaagttgaa 107400
atagtaaggc ctattatatg gtgtattagt ccattcttac actgatgata aagacatact 107460
cgatgaaact gggtaattta taaagaaaaa gaggtttaat ggactcacag ttccacgtgg 107520
ctggagaggc ctcacagtca tggtggaagg tgaaaggcac atcttacatg gtggcaggca 107580
agagagaatg agagccaagc taaaggggga aacctcttat aaaaccatca gatctcgtga 107640
gacttattca ctaccacaag agcagcaggg gggaaaccgc ccccatgatt caattatctc 107700
acacctggtc ccttccacaa cacatgggaa ttatgggagc tacaattcaa gatgacattt 107760
gggtggggac acagccaaac catatcatat gggaagaata atattggggt aaaaacaaca 107820
tatattttta aaagttaagc tttgacagca aaagggtctg tacttctaat ggatagctta 107880
gctttatttt agggattttt ttaaaaatcc tatgctaaat ttattatttt agctaccagt 107940
gttggaattc atccttttat aatgcagatt cttcattcat gtgccgccaa ggatttgtcg 108000
agtgccttta aagcatctgg tcatttaggt aatcatgggg acataagtcc tcaagaaatt 108060
ctcagttgag gggaggaacc agtaggtaag cacagggcac cacaggcctt gatgaaggca 108120
gacaccaggt gctctgggga cacagaggca aggccgattc ccaggcatgg aaaagtccag 108180
aaaggcttat gggaacaggt gactccaatg gctggatgta gccaaaggaa gtgcaaggga 108240
acggattccc atcacaggaa cagcatgtac taaatgtctg aggtgagcaa atccttcttc 108300
cagaacttag caagaaccat caatggtaag aggaggacaa aaggccaagg aactctgaag 108360
tcggttgaaa gtgtgatcgt atctgtcatg ccaaactcct atcaactcta gtagggaagg 108420
caccaggttc aagaggctga agaagagacc cagagccagc agatgagaca tggggtttca 108480
ttgagggctt acatacaggg gagggagtcc aatgctggct ggctggtcag gagaacaaga 108540
cctgcttgca aaatgcatgc aatttacata gcatttttac ttagcacact cccccagcaa 108600
cctccacatg gaaatcttaa ttcaccccag acttggggcc tcaattccct gtgtatagcc 108660
agtgttccac gggaggagat ggggactcag atgttcctca tcaacaagga gtcagtctct 108720
gggttggcca ctcctagatt ccctagctca gaacacacat tcaggtgtgt ctggcaagaa 108780
gggtcattct cggggtatgc ttgagttatt gctttcaggt gcacttacat acgatgttta 108840
agtgctgatg agagagggca agtagctagt gaggttgcag atgggtgaga taaggaggat 108900
gatggcaaag gttcctgcag aggtgggaga aggcagtatc cacagcaaga ggacaaagat 108960
tggccttaga caggtgttga gctgacaggt gtcgaggtga tcctcaggac agcaagccaa 109020
gctgaaagtt acaatcaaga ctttgttccc tcactgcaac agtacaggca ggaggcaaag 109080
gtaggcaccg ctctccagct ctccagcgtc cggaggtgtt ggttaagggt caggtggatc 109140

agcaggcatg ggggagggag ggctcttttc cttgctaagc agtcctgaac aaaaggttcc 109200
agccattcct gcacctaggg aggggaagca taaggaagga agggatagga gaagaaagga 109260
acggagccag catggaggaa agtgcctcat ctgggccctg atcaggaggc ctctgcacag 109320
aggcacctgg tctgggactg cagctatggc catgatccca gctggcctgg cagggcagag 109380
ctgagtcata aattgcaagt ccctccagaa atggcagtgc cctggtcatg caccatgtct 109440
agggtgggga tggcagtggt ccccgggagg tctcccaggc aaggccttgc catcaggtct 109500
gaatggatca cacataggat tttggccagg agcaggactc ctggaacgga ggctggaggg 109560
aggcaagcca aaatgatagc cacatcatta ggcccacctt ctctgggtct gccccttcac 109620
tgggtaaata aaaagatcag ctggccatct tttccatggg aacatgggga tggttattgt 109680
aagagttaaa gaggtaagaa acacaaaaag cagctcaaca gtaaaagaca gatttatttt 109740
ggagaataaa ctttagaggg gcttctggcc aattttgctc aggagcattc tctcttacag 109800
actaagggta tttaagggtg taggaagtgg ggagcttatc gcaggttcgg aatgtttcta 109860
tgtgagggaa agtttattgt ggggttggga ttgggatgtc tggttggagg ggaggctgtc 109920
tcagggttgg cgtgtttctg gtcagagggg gtttatctta gggttggaat gtttctggtg 109980
atgctgacat taggcattag gctgatgttt gggngcttga tttgggtgtt tttttaatca 110040
aagggaactt aaaatggtgg tgtttctcca agacgacgat ctcctgctct gtcaatccag 110100
tccctacagt tatagaaagg aagaggggtg gtatattctt tctggctact tcctgctgag 110160
tggagggtca gagggttctt tggtcttggg ttgattgtgg gagtaatgcc atctgtagat 110220
gtttttaggt aattgtgaga tgtccatgat cctgtcagtt aaaaaatctt tgaaaaaggt 110280
gaattaagcc aggtaagcac attagtccta ggcatattat taggagaggg cccaagaatg 110340
ggatggtcca tgttaggatt ttgtttctaa accaagaatg tatttggttg ttttggtatt 110400
cctttcacgt tttagccctt tcttcaagtt tttcagcagc atctcttact aggcctgatt 110460
ggttgagata gaagcaacat tcctcactca gtgagaggca gagacccccct ttttcagcca 110520
ttgtaagatc taatcccagt ctattttgga ggactgctcc aggcaaggag tctaattggt 110580
cttcgactct tataaggctt tgggctatat cttttaataa atcctgtagt tctgttgaaa 110640
gaactttaaa gtatgttaag gagttggcca gtctgcctgc tcccaatcca agcccggagg 110700
ttatacataa ggcagccatt aaaggaatga cttggatgct cctccttttt ctaacatact 110760
ggatgaatgg aacaggtgaa gattgattag gaggaactag tccaacggag ggagaaagat 110820
aaactagggt acaggttctg gtctagttgg tggggagaaa aagatatgtg ttggtaccgc 110880
ataaaaagaa cgagcctttg tcataatgca ggcggagata gggaaagaag ataagcaaat 110940
taaagatggg gtgttttttc tttcctgcgg ttttttactc caggtggaca aggaggaggc 111000
cagggagaca cccactatag tagagatata ggaagagtta ttttttacac atttaatgca 111060
atcggatgtt gcaccattga tattgagcca tggaaaaagt tgggcaccag gggcagcgca 111120
agttaggcag gaggcattgg ttgacggaga ggctgtaaac tagctggttg cagaaatgct 111180
ccatttgttt caggtgatac ttggtagtca acgtggctac tttgatggtc agaggggtgt 111240
ttaacaatga tagtgtgatt gcattggtta gatgttaagg accctacagg gaaatttccc 111300
tccgaggctg aaaagcaaag tgaggcttgt tgtaaaaggg gggtgtgtgt agttatgggc 111360
ccttcaatgg gagggtcttg gtgcttaagg cattgtagag agttgtaata ggtttgaaat 111420
attttgttgg cccgattggc cctggaagtg aaataatctc tgaacagggt gtcggctctc 111480
tcaaaaaagg aagctccttt tgggagttta taagttaggg ttatatttcc tgttcaaagg 111540
tcatgaagag gtgtaggaag ggctgtgtaa gctgaggaag acagcgataa gcatatccag 111600
cactctggag caaaggaaga gttagctttg cagcaagagg gattgtgtaa ggtttataga 111660
gcattctagt ttgagagcag tgaggagtgg ttttattggt gaggttgata tgattaggga 111720
atttatattg aaagaaacaa tcaaaaagag aaaaaagtta tttgggtagt agtaaagtcc 111780
tggaaagttc cttgaagcca taggtaccat agaacagtca gaaactgatt agcatgtata 111840
atgggagctt tgtaagggta ccactgaagg tctgtagcaa ggttggttag gaagcagtga 111900
aagtttggga gagaaagaga cataagcagc ttatgtggat ttctcttcct tttcctccag 111960
gatttaggtg aggcgaaggg aagtaggtcc tgtggagaca caagagaagg ctgaaggggt 112020
tttagatttg gttgtttgtg tatgtggtga agggaagtct gttttcttga gagaggtata 112080
atgaaaccag ttggggagtc cctggagttt tgctgccatg ggtgtagtaa ggatgatctg 112140
gtaaggtcct ttccatttag gtgtaaggag ggaattgggg ctaggactgg gatcttttat 112200
cagaacccag tctcctggct gtaggaaggg attagaggag ttggtgacaa gttgtggcag 112260
gtgtttgtca gcatattccc aaatgaaatg gcagatggta tgtacaagag gggaaatgag 112320
aggggttggc agaggtgggg cttgaccctg aggtggacca aaagggggcaa gtggtctccc 112380
atatgtgagt tcaaagggggc tgagcattaa aggtttatgt gagagcacct gaatttttag 112440
aagggctaaa ggcaaaagtg taaccgagtc tttatgtgtc tggagtgagt acctggtgag 112500
ggtgtttttt agaatgccat tcattttttc aacctttttct gaagattgag gtcgataggg 112560
gatgtgtagc ttccaggtaa tttgtagggc ttgtgaaagt gtttgagtaa tctgagaaat 112620
gaattcaggg ccattatcag attgaaaaga aagaggcacc ccaaacctgg ggatgatttc 112680
tgttattaat ttggaggtga cattagaggc tcgtttgttg gttgtgggaa aagcctcaag 112740
ccattccaaa aaggtatcaa ccagaactaa aagaaatcaa accctttttta ctgggggtat 112800
atgggtaaaa tcaatttgcc aatcctgtcc tggaaggtgt cccctggctt gatggattgg 112860
gaaagaagag tgtctagtgt tggaatgggg tgaagctttc tggcaaatag agcattgatg 112920

ggaaatggct tttaactgtt cctttatatc tggggttatg tgtttatggg aatttaagaa 112980
gtgttgtaga ggtgaatggc tagcatggaa gaggttgtga atgtcccgta aaagagttgt 113040
ttttttcagg gtcaggtagg actaatttgt tttgtatgaa tcagtttggg gggtttgaat 113100
tgtgcccctg ccatgattag ttgttgtgtt tggtgttctg gataaaagaa ggggatatgt 113160
tgtatgaagg gaaataagta ttggggaatt gggtgattgg ttgagacatg ttttgcccag 113220
tagtcagcct catggttccc taaagaaatg tggcttttgt ctgatgttct ttgccatgaa 113280
taactgcaac cttttctgga agtagaagtg cctttaatag aggatgaatg agctttccat 113340
taatgatagg ggttcctata actgtgagat agccccgctc cctccaaatt taggcattgg 113400
aatggatgat gttataagca tatttagaat tggtgtatta ttaacttgtg tgtttttttgg 113460
tagggttagt gctcttatta gggcaactaa ttctgcttgt tgggcggatg tgcccaaagg 113520
caagggggca gcctctatca ctcttctaga tgggagagag tgggtatcat aacgctatcc 113580
ctcaatgatg gcatatcctg cttggagagg agggtttttt gatgcactgc catctataaa 113640
ccagtctggg gctcccttta tgtgagtgga agtaagatgg tgaaacatgg taggaggact 113700
ttcaattaga tcagagcatg agtattggtc aggatctaaa atcggtattg agggtaaaag 113760
agaggcggga ttaggtgggg agcatctatg aagagagata gcgggttgaa ggagggttaa 113820
atgtaaggct tgcatatgag aggatgagat gggggtgagc gcctatggct gaccatatct 113880
tgtagactgt gagaagaaaa tacctgaagg ggtttgtaga acatgagttt ttgtgtctca 113940
gagataatta gagaggctgt ggccaaaatt tttaagaaaa gggaccagat tttataaatg 114000
gggtctagtt gttttgaaaa atatgccact gttgagaagg agtctcccat aagttgggct 114060
aatagtccaa gggcctgatt atgagaactg tgtaaatata gatgaaaagg tcttaggagg 114120
tttggaatgt ctaaagcatg ggcctgcaat aaggcacgtt ttagatgaga aaaagcatga 114180
taaaggtctg gggtgggagt gagtggttgg tcaagatttc cttttgtgtg ttcataaagg 114240
ggtttagcaa taatggcaaa atttgctacc cataatcgga aatatcccac taatccaagg 114300
aaggaaagta agtccctttt cgttttagga aatgggattt gttgaacacc ttgctttcat 114360
gccaacagca tttctcgggt atttggagtt atgatcagtc ctaggtatga aactttcggt 114420
gaggttaatt gggccttcct tttggacacc aggtacccac attcagccaa aacatttaaa 114480
atcttggtgg tgtgttgaat acaatggtca agaaaggggc tgcaaaggag taaactgtcc 114540
acatattgca gcaaaatcct gggttgtaga ggtagttggg agaggtctaa ttgaagtgac 114600
tgaaatagtg atggctgtct ctaaaccccc aggggaggac agtccagatg agttgttggg 114660
agtagcctgt gtcagggttg gtccaagtga aagcataaag gttttgagag gagggatgta 114720
gagggatagt aaaaaaggtg tctttggcca ggtgcagggc tcatgcctgt aatcccagca 114780
ctttgagagg ctgaggtggg cggatcatga ggtcaggaga tggagaccat cctggccaac 114840
atggtggaac cctgtctcta ctaaaaacac aaaaattagc tgggtgtggt ggcgtgtgcc 114900
tgtaatccca gctactcagg aggctgaggc aggagaatca tttgaaccag ggagtcagag 114960
gttgcagtag attttgctac taaatgtact ataagaaaaa aaaaaaattc aaaaggacct 115020
tcaagtattt tcagaaattt ttaaattttg gagttgtgaa taaggactgt gggcctctat 115080
ttttaaaaaa tgcatttaag agtctgtact tctgtcccta caagtccgat tgtgcatcaa 115140
tgccccattg ctttttagct atgtgatact gggcaaatca ttctttacat ttttattctt 115200
cacaaccaag atgtaagccg aaatgtgttt ttttttcttc tgcattttgt gtattaaata 115260
atatacaaag atgacacaac tgggcttaga aagtatttct taaaagacta gattacagcc 115320
tatgtaatat atttgaaatt gataatatta tctgtataaa tggcaaggct gctttttaga 115380
tccattcctg acttagcccc tctatacttg ctctattttc cagctataat agtaatcaag 115440
tagtattaca ttttcagatc tccaattact caattgcacc atttgcctg attattttga 115500
ccatcataat gaaagcagct cggcctctcc caggccccct aatcagttat tcagcagctg 115560
acaatggtga cagcctgaac gtgtgaagtg actagaagga gtatattttt cctttcacat 115620
aaatgttact aaaggtgaaa tccatgaaga gtaataaaat tttacaagga aatgttaaaa 115680
aatattacaa ttacaaaagg ggtcattatc atccctaatc aaataaagca tcaccaattg 115740
gtcttacttg ccagatagaa aatcattcag gagagatgat taaaaacata ttttactcaa 115800
ggctagattt gtcgtcagac gcacatccct gggtttcttt tccctcccct ctctagacat 115860
tatccagtgg ttgagtgctt aaggtaggtt ggttaattac agtattagcc aaggcacagg 115920
acatgaccag aacaaatttt ctgcaagaaa aatcacatga tgtatcgcaa agtgaaatac 115980
agactttaaa aatcagcaat tgtctatgtt atcttgactt gggtccaggc cgtcttggag 116040
gctgcctccc agcaatgttt atagggctgc agaatggtgt gggaaaataa agtgtggaag 116100
cacatgggag gcttggggag ggcactcacc ctgaatctgc ttcagcacta tttaccaggt 116160
gtgtagtctt aggtgacact taatttgtct gagcttcatt catttttaaa atggtaatga 116220
tgtctatcaa acaggcatga taactaagag ccctacttac acatagtaag actttgaagc 116280
ttggagagct tgcataaccc acacaaagtt acacagctgg acagctggat ttgaagttgt 116340
ctactgctcc taccaactat tatccattag gtcacttcta gccaagcaaa cacactagaa 116400
agtgttcagt taggaaatac ttattgagtg ccaagaatat aaatattttg tcaatctgta 116460
cctggaagga aaggcctctg gaatagttca tcacaaactt cccagaggac ttcaggaaat 116520
caagaaaggt atagaggtat taacattttg gggcacagcc tgtgagtgtt aggaatgatc 116580
atgtcaggat cccaaaccag cacctgatag tctctgtgcc acagcagaaa cagcatttgt 116640
ctaagaagat cccacacatg agacaggtga aaggatgttc caagtgacag cttgtgaaga 116700

ggtgtttgaa tggagaatga gcaacaatgc tgaaccaaca caaacatctg tcatccttac 116760
atactgggtg aggctcagca tgccggattc cttttcccag tattgttcat tcctccagga 116820
tcctcattac cctccatcat tccccttacc ctatccaatc agaagcgtct gcataattcc 116880
gttgcaaaca ctttggctgc aagtaacaga aaacttgatt ccagttgact ttaagaataa 116940
ggaaatttat tccataaaat tcaagagatc aggtagcttt caggaagtca ggactggatc 117000
tttcactgaa attctcttaa ttgttgtttg aattgtcagt gttcccccaa aaatgtatgt 117060
tgatatccca actcccagaa cctcaaaata taactatatt tgaaatacag gtaatcaagt 117120
taaaatgaag taatcagggt ggaccctaat ccagtatgcc tgatgctttt atgaaaaggg 117180
aaagaacaga cacaaatatg cataagagga agatgatata gacccacagt atattagcct 117240
gttctcacat tgctataaag aaatacctga gactgggtaa ttacaaagaa gagaggttta 117300
attgctcccg gttctgcagg ttgtatagga agcatggcag catctgcttg gctcctgggg 117360
aaaccacaag aaacttacaa tcacagcaga aggcaaaggg gaagcaggca agtcttatat 117420
ggctggagca ggaggaaaag aaagagagag gaggtgctac acacttttaa gcaacctgat 117480
ttcatgatga ctcactcagt gtcatgagaa cagcactgag aagatggtgc tcaaccattc 117540
ctgagaaacc acccctataa tctaatcact cccatcaggc cccacctcca atattgggga 117600
ttacaattga acatgagatt tgggtgggga cacagatcta aactgtatga cacggggata 117660
tggccatggg actggaatgg catctacaag ccaaggaatg tcaaagaatg ctggcaaaca 117720
ccagaagccg taagatacaa gggaagattt ttttccctag agctgtcaga gcacagccct 117780
gctaacacct taattttaga cttctggcct ccatacctgt gagacaatac atttctgttg 117840
ttttaagcca ccaggtttgt ggagcttttt taccacagcc ctagcaaact catacagtga 117900
ccttctccat gggttgtctt cctatgttag caaaatggat gtgacatttc caggcttcct 117960
gatgacatct agaggaatat gtgggtcctc tgttcccagg cctcctcctt aggaccagga 118020
aaccttttct atcatccctt tccttccagc aaacttgctg tcatctctca ttggcaggat 118080
tgagtaacag ggtcattcct agaattaaaa ttactggctt tggttaatca catgggaagg 118140
agtagatgtt gtgaagacaa ctacttgggt cagacccaaa acctggacaa gatttctctc 118200
agctctgtct ccaaagctgc tcctatattc cccatgcaaa caaggtttgt aaatagacgt 118260
ggtagaaaga aagctggtgt ttaattcctt tcaccagaaa catctggacc ccagatcctt 118320
cccaagatgc ccaaataggt aggtacctgc aaagtagcta gcatagggct ggacacagag 118380
tagatcagtt atggattcct gctctccctg tttttatggt actggtcagt ttctatcaca 118440
taccattgac attacacatg ggtcttactg cctttgctag tctacaggta ccttgaggtt 118500
gagagcatgt gcattcctta cacctccaag agcacttaac actgtgcagt gggatctgta 118560
agcatcagta agtagagtca gaaaggaaga cagctaggac tctatcagta tataagccca 118620
agagatggag aagggatgct cgaattctac tcgatggaaa aacaaagaat gctatattga 118680
tagaatttaa taaaacaact ttgtcatctc aagccaataa ctttctgttc tagtttggac 118740
caaacagcta cactgctaaa tagaaataac aagatgaaat cctgatgtaa ggcaatatgc 118800
aacagttcct gttttgttgg gatgaatata ttgtatgagt acaatttaga tgaactattc 118860
attctggaac agcctgtaca ggtcctaaga cagattgcca taagcaagaa caaccacagt 118920
ctttctgtat caaaaacaaa ttgcatgttt ttccataatt gcctaacaaa agctcataag 118980
cagcttcagt aagattttac tcagtttttc ctccatttat tatagaaagc aactgcctct 119040
aaattactga aataaatgca gtaaacattc actgtccatc tttaaccagc tctgtatctt 119100
tattccttct gatttcccac taaaataata aacagacctc atatgtttag cggttgtata 119160
aatggggata gtttttattt ctaagtaata tattatcctc tataacacaa agtacttaaa 119220
ttttagtatg atttatttga tataggtttt cttaattttc attctctaaa tgttaattac 119280
ttgctctatt gagttttcac ttataaaata tgtccaaaac agttattgca cgatagaagc 119340
aacaaatgct ataaaacaca taattaaaaa ttaactttcc tttcctcttg ccaccaaaat 119400
gactagtcgt gcagttagtt gtattaaagg aattaccaaa aaatttgaag aaacaatttg 119460
ctccttaatt ttttaaatta tttgtcagat gtagtgagag tgtgtgacat ttttaataga 119520
ttcaaggttt attttaatat ttaaaactat tagttcaatt aatattgctg aaatctttaa 119580
tttttgttct gtctttcatg tatttttaat tttatcatga aatatttgat gtgcaaaaag 119640
atatgaataa taataaaaca tactcctctg tggccatact taacaaacac taaattgcaa 119700
tagacctgaa gtccctgggt gcctcttcct gatctctgtc ctctcactct cccatggaga 119760
atcacttttc tgattttgat gtgtgttttg ttaataaatt tctttatact tttaatgcat 119820
atatggacat caactgaaaa tcatatatct tttgtgtaat ttaaacttgc cttatatggt 119880
ctcattctgt actatttctc ctgctaattg cttttattgc tcaatattat gtttatgaga 119940
gttacctaaa tgagtacatg gagctgtatt ttatttattg tcattactgt ataaaaagca 120000
actacatatt atattgcata tataatacat tctttataca gacagaacac aatttatttt 120060
ttcattttcc tgtgcagtag gcagattcta agctgtcccc catgatatct ccgcacgagt 120120
gtccataccc tgcatcatcc cctacccttc gatactggta ggatttgtga atatgatggg 120180
ctgtcattcc tgtgaatagg ttgctaagca tttaatgttg agtcagtcat aaatagagat 120240
tgtcatgtgt gggcctgtcc taatcagagg tactggcctt ttcttagatt taaagcaaga 120300
aagggtcttc tgttggcttg cattaggaga tatacctaat gtaaatgatg agttaacggg 120360
tgcagcacac caacgtggca catgtataca tatgtaacaa acctgcacgt tgtgcacatg 120420
taccctagaa cttaaagtat aataaattaa taaataaatt aataaatagg aaaaaaaaag 120480

taagcctcca tgttgtgaag tgctgtgtgg ccaagaccct gggcaacagc catcaagaaa 120540
gcaggactct cagtcctgca accctaagga acaaccctct gataataacc caaatgaagt 120600
tagaagaaga ccgcaagctc cagatgagac caaagcccag ccagcatctt gatttccacc 120660
ttttgagact ctgagctgag agcacagtta tattacacca tgcccacatt ctgaccttca 120720
aaaactgtga agtaacaaat gagtgttaag ctgcttcgtt tgtggtaatt tgttacacat 120780
caatagaaaa gtaatgcatc caggtgttgg atgtttctaa atattgtaaa tccaaacagt 120840
actgctctga acatttttgt acattgattt gtgcacatgt gaaataattc tttttagggt 120900
atgtatctgg gaatagagtt gctgggtctt agaataaaca tatcctcatt tctcattggt 120960
attgataaac tttcattcca aagtagtttt accaatttat cctaagctat cagcagtata 121020
tgagagtatt ctctacttta ccactcagga acacttggtc tggtcaggct ttattttgct 121080
tgaaacactt agctcttcat ttagcctgaa ttgattttg tggctgctgt gagagaggga 121140
acaattttat ttccatttgg ataaacaatt gccacagtac catttattga ccaatctatt 121200
catgcccccct agttctgcat catatgccag ctgtgtcata acgtggttcc atatgtgagt 121260
gcccaagtca agaggcacag gctcccttgt catccccttt tgccattgaa ttttttttac 121320
tggatctgga tcaacctcct ctctgaggtt taaaaccaat aactatatag ctttgctcaa 121380
gtatctcagc agtctcctcc tttcaaaggt tcatgacttt gtaccttatc tctgacacaa 121440
cattaaaaac caaagtctag cagagatcgg aaattctcta gagaaactga cttttcttga 121500
ttcaagctac ttcttagctt ttggtccctg gagtttttaa ttattttctt ggactattca 121560
cctttaaaag gatgcttttt gcattttatc cagaatttcc tggggttttt gaatcatttt 121620
tttccataca ggatgagaaa ctgaggttca ctaataaatt tttgagaaga agaaacagta 121680
ctccatggac tctcagatgg atagactttt ttaaaaatta tactttaagt tctagggtac 121740
atgtgcataa cgtgcaggtt tgttacatat gtatacatgt gccatgttgg tgtgctgcac 121800
ccattaactc atcatttaca ttaggtatat ctcctaatgc tatccctccc acctcccccc 121860
accccatgac aggccctgga gtgtgatatt ccccaccctg tgtccaaatg ttctcattgt 121920
tcaattccca cctatcagtg agaacatgca gtgtttggtt ttctgtcctt gcgatagttt 121980
gctcagaatg atggtttcca gcttcatcca tgtccctaca aaggacatga actcatccat 122040
ttttatggct gcatagtatt ccatggtgta tacatgtgcc acattttctt aatccagtct 122100
atcattgttg gacacttggg ttggttccaa gtctttgcta ttgtgaatag tgccacaata 122160
aacatacgtg tgcatgtgtc tttatagcag catgatttat aatcctttgg gtatataccc 122220
agtaatggga gggctgggtc aaatggtatt tctagttcta gatccttgag gaatcgccac 122280
actgtcttcc acaatgattg aactagttta cagtcccacc aacagtgtaa aagtgttcct 122340
atttctctat atgcctccag cacctgttgt ttcctgactt tttaatgatc gccattctaa 122400
ctggtgtgag atggcatctc attgtggttt tgatttgcat ttctctgatg gccagtgatg 122460
atgagcattt tttcatgtgt cttttggctg cataaatgtc ttcttttgag aagtgtctgt 122520
tcatatcctt cgcccacttt ttgaaggagt tgtttgattt tttcttgtaa atttaagttc 122580
tttgtagatt ctggatatta gccctttgtc agatgggtag attgtaaaaa ttttctctca 122640
ttctgtaggt tgcctgttca ctctgatggt agtttctttt gctgtgcaga ggctctttag 122700
cttaattaga tcccatttgt ctattttggc tcctgttgcc attgcgttgg gtgttttact 122760
catgaagtcc ttgcccatgc ctatatcctg aatggtattg cctagatttt cttctagggt 122820
ttttatggtt ttaggtctaa catttaagtt tttaatccat cttgaattaa tttttgtata 122880
aggtgtaagg aagggatcca gtttcagctt tctacatatg gctagccagt tttcccagca 122940
ccatttatta aatagggaat cctttcccca tttcttgttt ttgtcaggtt tgtcaaagat 123000
cagatggttg tagatgtgtg gtattatttc tgagggctct gttctattcc attggtctat 123060
atctctgttt tggtaccagt accatgctgt tttggttact gtagccttat agtatagttt 123120
gaagtcaggt agcgtgatgc ctccagcttt gttcttttgg cttaggattg tcttggcaat 123180
gcgggctctt ttttggttcc atatgaactt taaagtagtt ttttccaatt ctgtgaagaa 123240
agtcattggt agcttgatgg cgatggcatt gaatctataa attaccttgg gcagtatggc 123300
cattttcatg gtattgatcc ttcctatcag atggataaga cttcagatgg atagactttt 123360
taaaatgagc atgatctagg gcaggccatc ccagtgtttg cctttactgt ccgcagcctc 123420
acagtctttt gcctttgttt gcattgagga tattgtgtcc tctttgaatg tcatagcctc 123480
agctttaccc tccctcagtc tgccacacca ctgaaaatca tctttcacaa aatatctgat 123540
atacccattt atgatgaaga taaataaatc atgttcttgg tttttcattc ataattctat 123600
caacattctt tatgcaacac agctaggaag catactgaca tgaacaaatg tactcactgc 123660
cctctttaac tatagcattg tcaagaaccc aaggaaggac tgaaacaact catcccatag 123720
tagaacacat aagaaagatc tcaagtaagc caaaggtgac atatgctaca atagatgtac 123780
tcagttctaa agtaatagca aagacatttg ggagagtatt ttaaaattct gggaatttac 123840
tgtgtgccta gcacttccaa atatcagctc atttaatgag agaaaaatat tgcttccaga 123900
gaaggaggtg tgcagagctt tataaagatt tgtctttagg ggtgaatata atttcaacag 123960
gtagagattt gggtcatggc attagatgaa ggcattccat ataagaaaaa ccaaatgagt 124020
aaattagaga catggagaat cacaggcatg gagggacata tgaccaaagg atggcagtag 124080
caatgggaaa ccagacatgg agggtcttca gtggttggaa ctgtgacaca agggagcttt 124140
tgaagatttt tactttattt tttttaattt tttttttctt ggagaatagc agaagagcta 124200
ttttgaggaa agttacactg gaagaaatgt tatttctatg aggaggtatt ttcattgaac 124260

aaaattcatt tttattgatg aaagacaaga atttatgatc agttgataaa tttcaggcat 124320
ctttggagat attttatatt tttttctgtg aggctgttac tgtgtcaaat ctttcacagt 124380
ttcataaaat tgagtctcat ctgaagcttt aggccagatt gttatccatg gttgagcatg 124440
actctgcaat agaaactaac aatttggctt attttaaagt gataccatga gatatatata 124500
tatatatata tagtttattg tcattgtctc cccccccaaa aaaaactgta aaattaattt 124560
tcattatttc ctttctacta taacattagg gttttttttt ccttgctcct gttggaattt 124620
atttttgtgg tgactcatct gtggcataat gatgaaatga taaaataata tcctgtgtac 124680
ttccttttag aggatgattc acataaaatt ggccatcttt tcctgctgtt gtatattaat 124740
atcattggtc cactgaactg caatgatcag aattatgact ttccttgaat aaagcatcct 124800
gtttcctacc cctaacccta gaagagtact ctttcaggga gaacctattt ttgcagctca 124860
tcttattttg gtagtttttc accaatcatc ttatttgcaa tttcccaact atttaattgt 124920
catcttacat taatattatt ttaataatca gactgttcca ttgttcaaat tttaaaaata 124980
aatgaaagtc attaatgaaa agtattgtct cattccatta taatgagctt cctcaagtat 125040
gttttcagtg atgaaaacat gaccgtgtta ttaaatattt tgtggattct tgcctacaaa 125100
aagaggacag ttcatacatt tgtgtctgta tgccttttag ctctactgca taaagaatgt 125160
cggtggaatt ttgaacaaca aaacaggcac atgatttatg tagtcttgcc tacaaaaaat 125220
acagtctgta atgagactct attgtcagtc tctcatggct acccacaatt tcctatcaat 125280
aaaacaggat aataatacat aacttagagg attggtagga agattaaagg aagtaacagg 125340
tgcaaagtag ttagcactgc gcctgccatg taaacaggca cttaacaagt agtagcagtt 125400
gtccttctta aatgcatgga aagcccatgt cttagcatgg catgctgaat tcctagcagc 125460
actgaggtag cccagcatcc aagggtaaat gactggagaa ttaatggaat ggtgggcact 125520
cagttttctg tgttgtatag aaagtttaaa actttaaaaa tagtccccaa ttccagttta 125580
ggagattatt tttaatcagt cttttgcgac tttccagaca aatgcaaaag gtaaaaaaaa 125640
atcatattac agaaaaataa gacaaaccga caagttattt ccagacgcgg aaatagctta 125700
tcattaacta ttacatatac ggaactaaac agagaccatt cacccaatga tatgtatatt 125760
cattctttct cttgggtata cccttcttgg atgtaacacc cagagtctta gtgtgccaga 125820
agactaaaat gcctacttct gcagaggact gtctctaaca ttttttctag gaatcaatgt 125880
tgaaatctta atttccagga agcatgatgg gataaagcat aatgtgtgta cgtgtgtgag 125940
catgcatctg gctgagcaca tgtgcatgca gatgggggtg gggaaggaga gagggacccc 126000
ccctaagcaa atggaagcta atgggctgca tcctataatc ttaaattgtg caatacaaaa 126060
ggaaaagcag agcaggtggg accagatgca gaaagaacct tagtagctag tttagatgga 126120
ggtctgcagt attgctgctg tggcttttgc tgttgcttct ttgtgttgtt atttagttt 126180
tcagaattcc ggagaaaaag gcaacaggca aaagataaaa atcctgcctc aatccaacag 126240
aaatgtactt gctaaattcc tgtgagaact ctgcaaagtt ggcaataaag aatgcatatt 126300
aatatgaacc accaaaaagt gaattctgtt aacattctag acaaatgtca catttgctgt 126360
ctcttgggat aaaccaagtc atttcaagtt gagtaaaatg aagtaaagcc agtgggatta 126420
tgcaaaggta cagcaaatga aaggaaaggc aacactctct tgaagagcga gggagaactt 126480
tcctgtatta ctcactagac aaagattatt atcttcatct tatagttgct gccaattcaa 126540
caataaataa aaagaaatgt ttattatatt tggccttgtt ctaacagaat ataatgtgtc 126600
cggcaataat tatatccagg tcaaataaat ttaatcaaaa tttaaagtaa ttaaaaataa 126660
aacaaattaa gatggaaaca aaaaatggca ctccaaaacc taaaaggctc atcatgagga 126720
cactcatgtt tcccagaatc ccggaccaag cgtgactaat ttttctagca aatatacact 126780
ttgatagaca aacatgttta tacaagttct acagtgatta tgaggtacca actagttcct 126840
agagaagcat aattattcct gatcaagtcc agaaagacat ttttctggat gttattaatc 126900
ttaatggtta ctatatgtga ttgcttttta tgtgtgagag tttacaagag ccttgaagaa 126960
aaatggagag atggatgggt atacacagag agagagaggt gcacacacac atagatatcc 127020
acatatatct aatataattc tcaaaacgac ttcattaagg aaaattatgg acgttataaa 127080
aaagaagcag tgaggaacag caccagaaat tgatgagaac caacaagaaa acatataagg 127140
ctacaataac tagaagtgca tgacttaggg ttcgcagaca gatgaatggg caaacacgca 127200
ttgctataaa gaaatacctg agactgagca atttctaaag caaagaggtt taattggctc 127260
accattctgc aggctgtaca ggaagtgtag caccagcgtc tgctccttgt gaggcctcag 127320
caagcttcca gtcatggcag aaggtgaaag gggagcaagc ggtttcacat ggcgagaaca 127380
gaaacaaaag agagaggagg cgccaactct ttcaaacaac cagatctcct gtgaatttag 127440
agcttagagc gagagctcac tcgttatcat agacagcacc aagccatttg tgagacagct 127500
gccccgtgac ctaaacacct cccagcaggc ccccacctcca acactgggga tcacatttca 127560
acatgggatt tggaaaggac acacatgcaa accatatcac catatattca ctcaaacaac 127620
atgtaacaaa gtgtcacaat gaatgattaa aagacatatt aaacaagtta agttgggaat 127680
actaaagccc tctcatactg tgtccggaat tgttgggttc ttggtcttgc tgacttcaag 127740
aatgaagccg cagacccttg cggtgagtgt tacagttctt aaagatggtg tgtctagagt 127800
ttgttccttc tgatatttgg acgtgtccgg agtttctgcc ttctggtggg ttcctggtct 127860
cgctggcctc aggagtgaag ctgcagacct ctgtgatgag tgttacagtt cataaaggtg 127920
gcacatctgg agttgtttgt tcctcccgtc gggagttctt tgtccctcct ggtgggttcg 127980
tggtcttgct ggcttcagga gtgaagctgc agaccttcgt ggtgagtgtt acagttcata 128040

aaggtggcgc atctggagtt gttcgttcct cctgttggga gttgtttgtc cctcctggtg 128100
ggttcgttgt cttgctggct tcaggagtga cgctgcagac cttcgtggtg agtgttacag 128160
ttcataaagg cagcgcctct ggagttgttc attccttccg gtgggttcgt ggtctcgctg 128220
gcttcaggag tgaagccgca gacctttgcg gtgagtgttg cagctcataa agacagtgcg 128280
gacccaaaga gtgagcagca gcaagatgta ttgcaaagag caaaagaaca aagcttccac 128340
agcacggaag gggacccaag caggttgctg ctgctggctt gggtggtctg cttttattcc 128400
cttatctggc cccacccaca tcctgctgat tggtccattt tacagagcgc tgattggtcc 128460
attttacaga gtgctgattg gtgtgtttac aaatctttag ctagacacag agtgctgatt 128520
ggtgcattta caatcctttg gctagacaga aaagttctcc aagtccccac tcaacccaga 128580
agcccagcca gcttcatgtc tcaatactat gtatcaaaac attttcttga tgaattagag 128640
caaggatatt taaagaatga aaaatgtaga gaacaaaaag aaaataaaat cttaatcttg 128700
gatagtcaaa ggccacctaa gcctaacaga ataaaatcaa ttacaaagaa gaatgaattt 128760
gaatttttaa acaagtcaaa aataaccacc aaaaatattt tctaaacaaa atatgaaata 128820
caaccttaac ttaccatttc agtaagaaaa tgtcaaatgg acaagattta tttgctatat 128880
ttggtttttg ttatcaacaa tgcacactgc tggcaaggaa gcacagatac tgtgatatat 128940
tgcatgtggc agtgaaaatt tgtgtaactt ttctgagaat tggtttttta ttatataaaa 129000
tgacactaaa aagcatctta tcctttgatc ttctaattgc atttataaaa atctctccta 129060
nagaanagta acctgagatg catacaaaga ttgatcttca aaaaatgttt tttgttgcat 129120
tatttactgt tttgaaaatt ttgaaacagt ttttctgtcc aaccatagta gaatgactaa 129180
gtaaattttg aaatatagtg gtttatcatg caattatcaa aactatattt ttatagacta 129240
attagtgatg taagtcatga gtgagagaaa tggtgatgat aaaatatttt taaaaatttt 129300
accaaactat atttgcagct gaattcaatt ttgattcaag tacatttgta tgcctagaaa 129360
caaactgata gaatgtttaa attacaaaac aaaatatata catttttttgt tatatgtgta 129420
tatatatata tatatattta aaaaacctgt tgtttgaatt attttatgct tcatatattt 129480
tggtattttg agatttttta ctatgaaaat cttcaaaaat ggaaacaggt ggactgataa 129540
acaaatagat ataaaacctt actttatagt taaacatcag tgagaaaaaa actgtttttt 129600
aaatggcaag tacatgaaca ggtaaagttc taaaagaaag aaaatacaca cacacacgga 129660
catgctagtt atatttacaa taaaactaat ttaggttttt gacttgtaaa atattttatt 129720
aaagtaaaat aatttcaatt aagtgtttat tcataaacag ctatcaagtt tatttttaag 129780
gtattaaagt actaaataag cactattaga agtgtggctt tgaaatttat atctgcaaaa 129840
gaccacatca tttttgataa agctctttat ttgtgagctt agtgaagaga aatgagaaat 129900
caaagttgat tacacaggtt actttttcac atgataagag aaagatacag ggagggagga 129960
aggcaggaat tgnntgagat tttagaaaat ctcagattta aaacttttaa aaattaagta 130020
ttgggcatga ggaaatgagc agagaaaata gtagtcatat ttccacattt gtaaagagaa 130080
gaacaatagc caggcaaact ccatgtctga agtgtggttt attttaatat gcaaaacata 130140
agcatttctc tgaccaaagc tgctttctat ttaaagctaa atataaaaat attattttg 130200
agcatagtat tgcgagaaca tagtgaaatt tcctatgtag attctgtgct gtttagtta 130260
cggaaatatt tttaattaat cctccattta acaatggggc atacatctgt gtttttcgaa 130320
gcactgtttt gtatatatta gtaattatca ttgcactgaa atgaaaatat caatttcatg 130380
tacaatttta tagcacatat ttgattgaca atgaaatgca tttaacttat aaaaatgtaa 130440
tatggctgac attagcaaaa tctcttatta agaaataggt cataagtact ccaagaaact 130500
tgacagtctg ttctatgaag agcattaatt caaaagtccc ctatttgcat aaatctccag 130560
gtctcatttt cacagctgtt tgttgtcagc tagacggttg cagttcaaca ttattttaac 130620
ataatcggtg ggaagtatac ctttcaacta aaaataccat gctctgcaag aaagattgtc 130680
aggggtaaaa tataagttca ttcagttgat cttgatattt ttccttaaaa taaatatttc 130740
taatacagga actttaagtc tcaaattact gaaatatttt tattattaaa atatattttg 130800
taattttata ttttcataaa aataaaaagg ttatgtgttc atacataaaa atgttaatca 130860
gtagagctta ttttagcaaa gagtaaaaaa tactgaggat atctattttt cattaatcag 130920
ccaagcatta tatggagtgc aatataggtg tgctggaaaa aagaggatta aaattttag 130980
atattgctga gaatatgcat gtatgttctt tgttttatta attttaataa tggaaaggga 131040
tcagcaatta ttagatctta aatgtctttt acctgtaaca cccaagagca cagaaggaat 131100
caaagtcaag agatctcagc ctggatactg agcaacacca tttgctacct gtaagctgga 131160
gttcttgatt taaaccctat gtacttcagc tctccatctg cagtaggggc cattatatct 131220
acagtaatca cttcacaggg tcattgctaa gattatatta aataatggac aattaactac 131280
tgtgggaatt gcaaagcacc atacaaatgc tagtcctggt tcagtgcctg ttctcatatc 131340
atttcatctt agctgcaatg aaatcctagt acaaattcat gaccttgtcc agctgtcatt 131400
tatggtataa tacataaaag ttaacccaag catagccaag gaaagagaca attggacata 131460
cctacatata ttttcataac tagcaaaaag attcgctggt ttttgccaat gtgcttctgt 131520
tactttttttt ttcttttact cttgaaatta tatttaagtg tagactccac ttaaagtaat 131580
ttgctgctgg aataggcata atgttctgat gtctgctttg cttctaatta tgttcaagct 131640
cattttgaca tttaaggcca attacaaaag tctagattca gaagactttt ccttagtcat 131700
ctaaaatttg aacaataaac agttttttc tcctctctct ttattcattt aagtggtatg 131760
cttggcaaca agcatatttt gggaattgta aaagcaaaat aaataatgtg ggttcatttt 131820

```
gaaaaaagaa ctacttggtc tttcttgcca acataaaagg cagtcacaga attaactgaa 131880
accaccagtt tcaactttat ttcctcctaa atcacaagga ataaggaatt attttacaga 131940
tacagtgata ttttaaacgc ctaagttcta ttaacataaa ggtaattgac ttcaacagat 132000
ctctaattcc aaaagagttc atacatagag agatatgtat aaaaatagct acaaaatctt 132060
tatcatttaa aagtcactca taattaaact ccccagacta ggaccctaca ccaaaattca 132120
gttagtgttc cattgcgttt taacatccag tcatgtgatg acttacgaca tagagcaaag 132180
cacttaattt acctgcctcc ttattttcag tcaatttgcc aactgcaatt tttactgttg 132240
attcagtaga agaccctgat gagttgacag caccaaaagg caattcatag catctattag 132300
taagaagtca ctgtagcttc tttgtttaac ttttataggt aagatctggc ttggaaaaat 132360
agactggaaa gtatattgta aaaaaaacaa aattttccaa ttgactgtca ttatcagtta 132420
atagattata gaatagtaga cctttatagc tggaagatat gtatcgtgta cttcaaagat 132480
gaaaggaaat aatgtctttg aaaacactgt caaattttca aaattatgta tctaaaagaa 132540
attccttaaa aaatagttta tcctttataa tttttgaact ttataaaaat ggaatcacac 132600
catatatgac caaacatttt aagtcataaa gaatacacag gaattctggg ccgggcacag 132660
tggctcacac ctgtaatccc agcactttgg gaagctgagg tgggtggatc acctgaggtc 132720
aggagttcaa gaccagcctg gccaacatgg tgaaaccccg tctctactaa aaaatacaaa 132780
agtcagccag gtgtggtggt aggcacctgt aatcccagct acttaggaga ctgaggcagg 132840
agaatcgttt gaacctggaa ggtggaggtt gcagtgagcc aagatcgtgc cattgcactc 132900
cagcctgggc gacaagagcg aaactctgtc tcaaaaaaaa gaaaacatag gaattccgga 132960
atttaaccac tgtgatagaa gttgcttttc cttgggacaa tatttttaag gcagatttca 133020
aaattattat ttcaactatt ctttatataa tgctttatta tttttaaagt attcacatat 133080
atgttatttt gattttcaaa acaatcttcg agtgacaatg ggattgcata tttgagaaag 133140
gctggtctca ggacggctga atgtttttcc caaagccgtt aagtaagtcg gtgacttaga 133200
ctcagacgct gtgaaagcag aggctgagta cttggttctt gacctcactg atctgctggc 133260
ttacagagca tcctggtctg catactcagc gattcaatct ctatcttaaa cgcagtccct 133320
cagccattcc ttctagaaag gtgatctctt tagagtgaga gcagtgatgg aggtggctga 133380
agttacacca gggggagcag ctttgaaaag ctcctggctg ataggcatgt actaatggga 133440
gcaggagctt tgttggcagt gtcgaagtcc ctttggaggc tgtgtgagga gggaacccat 133500
ttccctggga tatagctctc tccacagggc agctggatgg ctaggggcag ccatggagta 133560
tcagattcac ctaggaatcg atatttttcc tgagggtttc acaaaaaaag acagagagat 133620
ggaatttgaa gaactgggaa gagtgtgatt gaattggagg gatctgaaat aaagaaatag 133680
ctatggtcaa tggctctaac caacagtact atatttatac tccaaattcc aaatccacac 133740
tatgtgtatt taggtacaat ttagtttagc catagtaagt tgtggcttct ggagctttta 133800
tttaccatag gattttaaat acatttcatg tcatcttcat tggttttcct taacccctaa 133860
ttctccacaa gatcttcatc ccgtcagtta tgactttgcc aaccaatata ctttttagaa 133920
atgtaacccc aacattaggt acgaccagca tttactgtat atcaatatta tcctaaatat 133980
tgctgtagct ttgaatagct gaattaagtt ttaaataaaa gcaaaggaat tataaaacaa 134040
aatattaacg gattatgagg tcagtgggtt tttctgaaga taaaactatt ctactaatta 134100
ttgctgtttg ggaaaaatag aaacaaggag actgagtaaa tgaatcatta aggcaattaa 134160
aaacagaaga gaagtcactt ttgcactcag tgaataccat caaggcaagt cttctatttt 134220
agggctgtct tatagtaaga tataacaggg actctctcaa taaaaaggtt gagctactat 134280
ataaatcatc aagtagattt tattttgaaa gcaacttttt ttgtagagaa agagttcttg 134340
aaaccagtta aagatttaaa aagttgatac aagttatgtt gtaaaatcaa agttatatgt 134400
ggatgatacg agttgtattc cccaccacat tttaacattt ttagtgtata tatttttaaa 134460
tgaaaaaatg tacattttac acgtatttta gtacaaacag taaaaaaaaa gtcagaagta 134520
atttatcaaa acatatgttt acacaaaaac ttgtatataa atgttcataa cagcattatt 134580
cataatagcc aaaaggtgga aacaacccaa atatcaactg ataaataggt aaaatgtggt 134640
atatccatta ataaaatatt atttggcaac aaaagggaat gaagttccga tgcatactac 134700
aacatggatg acccctggcc acatattggg tgattccatt tatagaaaat atccagagta 134760
caaaaagcca tagagacaga aagtagatgt ctttgatttc caaaggctga gggagggagg 134820
aatggagaat gacagctaac aagtatgtag cttcttttgg ggataaagag aatgttctta 134880
aattagatag tggtaatggc tgcacaacca tgtgaataaa ctaaaaccca cttaatcgta 134940
cactttaaaa ggtgaacttt atggtatgca aattgtatct caaaaaccta tttcttttaa 135000
aagtcaaaaa aaatagtata gaccccttcc atagttcctt agtcctttcc attctagtcc 135060
ttagagttac aggtttattg tatacacttt caagcattta tatgcacaca aaagcatatc 135120
tatgtgcttg ggatatagca aagggatggt actatatatt ctacttactc tactttgacc 135180
aaaacccaag atgtttaacc attctacttt ttcttaatat ttgactatgt ttgaacttaa 135240
attgatatcc acacatacta aaataccaaa atatgcattg agtcatcaaa tttcaaaggt 135300
catccatgat attttttaag ggcttaaaac tccttngcat gatcnttcag cctgtttant 135360
tcagcatagg aaaataccct tgctttgcct tcacctaact ctaaggaagt ctattagaga 135420
tatttagggt cagaaatggg ttcttggggc ttgagagaga aaaggatctt ctgctattga 135480
tgatctggct ctgccatgac cctatggcat gggtcctcag tggccctgtg ggaagcctag 135540
tgtaggcaac atggcctagc ccacctatgt tgatgtttaa ggggtgctag gaacaaaaga 135600
```

gaagctgtgg aaactttttgc ttatccccct ttcttccctc tttggtggtc ttatttttat 135660
ccgacgctct taatctcaac ttataaccgg aaaatacagc accaaagcgt gagggcattt 135720
aggggaaaaa tgttatctgt tttggaagta atatcaagga agatcaggga caaatgcaga 135780
actggaagca aggactaagg ttggatttag caggatttcc tgaaagattg gatgtaggat 135840
gtgaggaaat gagatgaacc aaagatgtct tcagggtcat tggcctgaat aacttgaaga 135900
atagatttgc cattttcttg gctgggaagg agtgcagaaa gaacaggttt gaaagtgggg 135960
catgaggagt ttcatatgac acatcaaata cttgagattc ctgttatcta acatctgtcc 136020
aagtaaaaat gtggagttga ttctggaggt ggggctggaa atataactgt ggaagtcatc 136080
agaacataca agggatgtga aggcattaca atggatgaga tcaagcaggt agtgagtatt 136140
gagatataag aaaagatctg aggtcaagta ctggagctct gtaacattta gacattagga 136200
aattgaagag aaaccagcaa aggagcatgc aaagggctgg cattggattt tgtggctctg 136260
tagtgcagtg ctctggctct tttggtgact gaaagatgct tgttcactgc ttttctctgt 136320
gttttcaccc ggagcatccc tgtgacattt ctatatatat atatttattt atactctaag 136380
ttctagggta catgtgcaca acgtgcaggt ttgtacatat gtatacatgt gccatgttag 136440
tgtgctgcac ccattaactc gtcatttaca ttagctatat ctcctaatgc tatccctccc 136500
ccctccccc accccacaac aggccccagt gtgtgatgtt ccccttcctg tgtccaagtg 136560
ttctcattgt tcaattccca cctatgagtg agaacatgcg gtgtttggtt ttttgtcctt 136620
gcgatagttt gctgagaatg atggtttcca gcttcatcca tgtccctaca aaggacatga 136680
actcatcatt ttttatggct gcatagtatt ccattgtgta tatgtgccac attttcttaa 136740
tccagtctat aattgttgga catttgggtt ggttccaagt ctttgctatt gtgaatagtg 136800
ccacagtaaa catatgtgtg catgtgtctt tatagcagca tgatttattt atttattttt 136860
tccaactctt tttttatta tactttaaat tttagggtac atgtgcacaa tgtgcaggtt 136920
tgttacatat gtatatatat gccatgttgg tgtgctgtac ccattaactt gtcatttaca 136980
ttagctatat ctcctaatgc tatacctccc acctccccc accccacaac aggccccagt 137040
gtgtgatgtt ccccttcctg tgtccatgtg ttctcattat tcagttccca cctatgagtg 137100
agaaagtgca gtgtttgact tttgtccttg tgatagtttg ctgagaatga tagtttccag 137160
cttcatccat gtccctacaa gggacatgaa ctcatcaatt ttatggctgc atagtattct 137220
atggtgtata tgtgccacat tttcttaatc cagtctatca ttgttggaca tttgggttgg 137280
ttccaagtct ttgctattgt gaatagtgcc acaataaaca tacatatgca tgtgtcttta 137340
tagcagaatg atttataatc ctgtgagtat atacccagta atgggatggc tgggtcaaat 137400
ggtaattcta gttctagatc cctgaggaat tgccacactg acttccacaa tggttgaact 137460
agtttgcagt cccaccaaca gtgtaaaagt gttcgtattt ctccacatcc tctccagcac 137520
ctgttgtttc ctgacttttt aatgatcgcc attctaactg gtgtgagata gtatctcatt 137580
atggttttga tttgcatttc tctgatggcc agtgatgatg agcatttttt catgtgtctg 137640
ttggctgcat aaatgtcttc ttttgaaaag tgtctgttca aatcctttgc ccactttttg 137700
atggggttgt ttgttttctt cttataattt gtttgagttc attgtagatt ctggatatta 137760
gccctttgtc agatgagtag attgcaaaaa ttttctccca ttctgtaagt tgcctgttca 137820
ctctgatggt agtttctttt gctgtgcaga agctctttag tttaattaga tcccatttgt 137880
caattttggg ttttgttgcc attccttttg gtgttttaga catgaagtct ttgcccatgc 137940
ctatgtcctg aatggtattg cctaggtttt cttctagggt tttcatggtt ttatgtctga 138000
catttaagtc tttattccat cttgaattaa tttttgcata agattcctca tacattcctg 138060
ttgtgctttc accctctact ctctggttct caccctgttc tcatatatcc aaggaagatg 138120
atacactcta attagaaaca acaactgaac tcagctcatt attttcaaaa tctttcttct 138180
ccattaggta aaaatcattg cagtctgttt attcaatatg ttttcttttc cacttaaatt 138240
ctgttcatgc agagactgat tcctgggaac atcaatgtgc tggcaacttt gactttgtaa 138300
tttccctttt gttgtcccac tcaactgcat ggctgatctc cattcatttg ttcacttcca 138360
gagcctactg ttggcaatct gatttttctt catagtttcc cattaaaatt cactagagat 138420
gcttttgatt tacttatttc ttccctggca gggtagagtt gcttgttcca gatcacctca 138480
tagcaaactg gaccattctc aagtaaactg ctcatccctg gtgcagttat cagcagggag 138540
gcatctgcag cattaacagt gattgacccg tatagtgatg tttaatatgt gataatgtta 138600
ctagcagcaa acaaaatagc acttgtaaac acagttgttt actcatactt acatgtatag 138660
gtttggactt gaggggtaaa atagcaaaat tacccaactc ccctgctaat aaaacctaag 138720
gaaatgaata aaatgacctg agaaaattat accagcataa ctacagtaag gaagagctac 138780
aatttcatgc cattttctcc gaatagcagc taggaggaaa gaaaagaaga gaaagatgaa 138840
ggaggaggaa aaggaaaggg tcgcggagga ggagaagaga atgagagaga acatgaatat 138900
tctaggctca tcccttaaga ctgtcttctg gaagaagtta agaagaaata aacttggaaa 138960
aacaacgaaa ccgtatactc aaattcccca atctgtgaag tctgtggcca aagggctatg 139020
cagagccctg gaaagtgata agaaccactc atgtatagaa gccacttgtt ccagaggcaa 139080
aataatccac tagatctcac catttttatt tttttcagct gaggaggctt gagagtgaag 139140
gcaaaatgtc ataccttctg aaaatgaatc atctcccatt ctgctacaaa aatgtagcat 139200
aatgaaaata tgaagagaca aaaacagaag cttcaagagt acaggaagct caggataaag 139260
aggggtgtcg aaagccccag gagttgtcct attcgcccat acagagaacc aaaaccaaca 139320
ggaggacatt gacttctccc tcacctggag tcagtggagt gggaatgatg gctggagaga 139380

ggccagtgaa tttggggaag tgaacactta ggactgatta gttctggctg caataaagat 139440
ttcctgaacc tacctgaatt gaagaaatat ctgaagcctg atatttcaag cataattgcc 139500
catacacagc ctcagctaat acttctctcc ctgtacccct ctcctcatac tgaaacagta 139560
aaacacagag aagaaaacct cgttgataaa ttgaaaaagg atgctaatga gtgggatgag 139620
atatctggaa taattctctg cccctgtatt agaaagaatg aaagatctag ataataatgt 139680
taaaagtgga aattttggca actgtggaac cagtctagaa agaaattagt tcaaattaaa 139740
acaggtagtg agaggacccc aagaataata tctatgagag taagaatcaa atggtttctg 139800
ggcaattgat ggtttaaaga aagaacatct gcaggatagt aagaagggtt tattctaaat 139860
gataagtgta atgtgtgaga atctggaagc tacttgagat agaagaaaga aagaatatat 139920
ttantttcc caatgagaca tgtgagaaat agtcagaaaa tctcagcaaa aacaaaacca 139980
aaccaaagat tgtattaaaa aaaaatggct caaataacgt gagttaaatt ggcccagatt 140040
ctgagcaatt aatggaaagt aaggaaggtt ccatttaacc ttgaccattt actgtctaat 140100
agagtcttgg agggggaagt ataattttag aacattacct ggcccaagag tgagcaatgt 140160
tttcatagga aacattgact atcaacatat tgattatcaa cctgctgaga agcacggatg 140220
acccaattat gatagaaaaa caaaatgtaa atattgtcaa acttgaggat ataaatgtat 140280
acatgacacg agatggaaaa tgaaagcaga gagaagaggt gacctgacaa gtggcaatgg 140340
taataccttc atctcacact ggaaggggtc aagtctttct agtttgatgg aaaaagaaat 140400
cgagatttac atacaaattt gggagtggtt aagggaagtg gggagtgatg ttgtgcctga 140460
atgaaatctt cacatatcat agcagaagtc tatgtctaaa gtggataaat caagaaatcc 140520
taaataggga taggcataac gccatcagaa gtgaaaacaa aaaattcctg tctcattact 140580
tataccaaaa taaatttcag atttaaactg atttaaacag ccaattcagg ttcaacatgt 140640
ttaaaataat aaagcaagga aagtgtcaaa ctactgcata aaaccatggg taaatttatg 140700
tctaatcatg cagtgagaga agtcttttta agcaaaataa aaacacttaa aaggaatgac 140760
aaattagacc acactgaaat ttaaaactga tctttgcaaa aatataagaa atgtataggg 140820
gaaaatgcac tgaacataca tggcagataa aagatgggtt ttcttatttg taaagagttg 140880
gtggaaacca ataaagaaaa taaaatggtc aacagggcag aaaaatggaa taacatgcga 140940
ataggtagtt cagagaaaca acttcctctc tgccttcttt ccactgaaac tctactcctg 141000
tgcattactg aggtcagttg cttcatttc ccaaaactgt tcttgattca cccaagagga 141060
ctaaattggc tcttctctgt tgcccctcag tactttaacc atattttgct tacacaccaa 141120
agttactatt cttccctttc tttttatttt cttttttctt ttgagacagg atctcgctct 141180
gttgtccagg ctggagtgca gtggcacaat ctcagctcac tgaaacctcc gcctcccagg 141240
ttcaagcaat tcttcttcct cagcctcccg agtagctggg attacaggtg accatcacca 141300
caactggcta atttttgtat tttagtaga gacggggttt caccatgttg gccaggctgg 141360
tctggaactc ctgacctcaa gtgatctgcc cgccttggcc tcccaaagtg ctgggattac 141420
aggcgtgagc catcatgccc agccagtatt cttcctttt gagaattgat cctgttcatc 141480
tttttcttcc cctctcaatt atacaattct taagggaaga aactatgtct tactccactc 141540
aatccccaat gcatcaaacc tttagtaacc actaataatc cttgtttgaa taaataaaga 141600
gcacaggccc tacggacaat gtaagtcagt aagtgcactt gagtcactat ctcaagcaca 141660
ctaccatttg aatttaaatt tctggataaa aatctccaca aaactgataa ggcacccttc 141720
aagcaagaca caagttaatt ttcagcctct aatagaagta tttgttggca ctacctttca 141780
ttggtttatt catttcagca gccataaaca cacagatccc attgtcacag aaagccttgt 141840
tcgcaaatat aatttagtgc atctcacaat atgagctttc aggtggttga gctgtgtgga 141900
aattcgattt ttttttctcc cgagactctg aaatgaaaag aggataaccc cagtgctctt 141960
gcccacatcg cttatctcac aacaacagat tacaatccca taactccacg ttcccaagca 142020
gctactgtgt tgatcaaatg taatgcaaca gatgagaaaa catcagctgg ttcatgccca 142080
agacagctga aaggtgcact tacattttta gtctccagat accaaaataa atctgttggg 142140
cttaatcatg agttgtcctg ctacttttgg ggaaagaaag ccatgtccag tgaaatttct 142200
tttatactgg caggcatttt gactcttcac ctattgctgt tgaatctcca ggatatgtac 142260
atgttttcac accgatacct tcattgaaga aaatacagca atctgatatt tttatgtgaa 142320
tcatctttgg aattcgtgtt cctcaccttt ggatacaata aatattcaag agaaaaaaaa 142380
tacaggaaaa aaataattgc cctcaatgat gagttctgaa atacttataa ggataagtga 142440
tgtgttgaaa aattctcaaa agcaatttta gaaggaataa tttttgcaga gagtggctgt 142500
ctcttgggca ccataacaag aaaaatagta tattgccctt ggtgatggtg cggtcaagta 142560
tcaataacaa cacgcttcat attttcatga acaaaacact tcttcataag gtaatgagat 142620
tatagaaaaa tcaactcaag gtaaaaataa ttactttgca catggacaaa ggataagtgg 142680
aagaacttat cttctagttc gtggttcatt taacaattaa ttgctccttt ttcaaatagg 142740
tccactgtgt tctgggcctt gaacagtgta cctctaagaa agtttgttat ccttactgaa 142800
ggaaattaca gttaggtcgc ttctcagcct tttggctaag atcaagtgtg aaaattatac 142860
ttagattact atgtaattag gcctggtaga tcctgggtag acttcactga atgcaggatg 142920
caatatttgg aaaagtactt actttggagt cctaatgcct gggtgctact tctattagtg 142980
ccagaaatta atagtgtgac tttgggataa tcacttaaca tctttgagcc tcattttcct 143040
tatcttcaaa aaaaaaaaaa aaagaaggaa tcagatgaaa tcatctttac cagattaggt 143100
ctatctttag gtcccccagt gtaactgggt ttctgtcttg gtctcagtcc atccttgcat 143160

tatcctggac tttatttatt ggaatcccaa tatctgagaa ttcaatttat gtctttatcg 143220
tactgaagtc tataaagatg tttttaaaga gtcactagac tccagtgggg caacttgctc 143280
cagatatcat catagtctca ttaaaaagta tttattgact actaatgtgt ataggataca 143340
tgagttggag actcacagat acacatatat ttaattagaa aaatatgtaa gaatgaaact 143400
agccaaagat attggcagaa agtgtgtgaa atttttagct tggctggcct ttgttggaat 143460
gctctatttt ttattttttt gtttgtttgt ttgttttttt ggtcttggct tcttccatga 143520
tcttgaccct gtgccttgaa acttttttta aatcatgaaa aatgtgtttg tcttcaagct 143580
gtccagtgtg gcaatatcac ttagaaatat ttcttttttta aagagtccca tgtttttaat 143640
atgatagcta aaataatatg atagaaaggt accataaaaa taaaattata tgcctttata 143700
tgccttagac aagtgtaatt cactataaaa ataagtttct cttagcagca ggaacaaaga 143760
aaaagtaatc tgttgaaaat gttagatagc ttgctgaaag gaaaaaaatt aacggcaaaa 143820
accaaagatc ttgaatttgt ttctaatttg ctcaacagca tttactgcga acaaaactaa 143880
ttttaattca gggtaattat tttcttattc cacatcaaat agttaaatat cacattttta 143940
agatctttat tcttatatct ttcctatgtg ttgaatcaag actgttcttg ccctgatcag 144000
ataaaaatca ataatattga gatcaaagtc ttcaaacatc acatgctaat ttgaagttgt 144060
atgcaagcct aaagctagtg tgtaatacct ttgttctttt cctcttaggc atttatgtcc 144120
atgttccaga tcctcaccca ggaaggatgg gtggacgtaa tggaccaaac tctaaatgct 144180
gtgggacata tgtgggcacc cgtggttgcc atctatttca ttctctatca tctttttgcc 144240
actctggtga gttcagcatt ttttttcaa ttatatcttg gaatttataa tgggatgaac 144300
atcaacagat atttggccta ttgtcaattt caaaatggga gccaaaggac taatgcagac 144360
taatctggtt ggatgagatc cctccttcca ttctaagttt ctacacatca aagaactgct 144420
tgatttgtta agttagatat tttctccatc aggtaccagt gtttgtaaga gggaaaacaa 144480
gacagaacaa aatgaaaatc tcacatttac ataccaattt aagtatcccc aagcattcac 144540
acctagatga tctcaggatc aaaccggaac tccattatta tttttttct catgtagaga 144600
cactgtcttc agtttcactg catcctcttc acagctgtaa ttaaactgaa gacagaagct 144660
atagacctca gggtgcagtc agttaattag aaatcagcac agtggttttg tgctatgtgc 144720
gactggaaaa aaacacatgg aaaaaaataa aacacattgc tgcaagtatt gtaaaagcac 144780
aagaaaactg aataccaaag tccagtaaga accgacttct gtcacacaag cagatttttc 144840
tcttcaatat acttccatta aaaacaaaga aaaaatgtta tgaaatgcag cctgtgactt 144900
actaaggaat gtaataccaa atgctcatct ctatgtaata aatttgttca tatttaagaa 144960
gcaaacctaa gttctaacat cacgttagaa gttccaagtt gaggactcaa atgtttccct 145020
ttatcaggaa tacaagaaat aaatacaact gctactattg tttttggcaa tatgtcttac 145080
agttacaaaa tatcttacaa tattattgct aatctttgga acacccgatt agatagtatt 145140
cccagttaac agatgataaa actgatactc agaagagttt agtaatttat ctgatgtcac 145200
atggaatgta aatatgacca gagtgttccc aggtctgtct gatctagatt tggtccttag 145260
tcttgcagct aattattatt gcttccctgg ataatgaagt ttaaagttgt ctgaaaaaat 145320
gtctttgtca aatcaaactt ctgccaaaga atcactcaca tcaaacaaat tacatataat 145380
ctctaaggtc agttttaatt ctaaatatct atgaatgaaa taagtccttg ataaactaga 145440
tataacttag aagttgtgtt tgctatagac agacagaggc tttcaaaact aagacatgtc 145500
caccaaaaag ttaatgagaa ttcattgtta catggtgtga agtcatgggc tcaaacagta 145560
gaactacata gacatagttt tggccttttt atacatggag ttgactttta aaagttacgc 145620
gaaagatagg aacattgtgc aataggaatg gaagcctggc ttgcaaagct gaatctggct 145680
gtgctggtgt ttgcacaaca cttagccctg gggtctttgc atcttccctt ccaggacctt 145740
taagcctgtg ccatttattc attcatccat tcatttacac atcatttatg cgttcaacta 145800
agtttattaa gctacttgtg tttaagttac tctaaaacta tggaattgct cagactgttg 145860
attgtgggtt aaatgcagga tagatttcat tgaaatcatc tatgtcaggg aatatcaatc 145920
taattatttt atagtaatca acctaatcat tttcctaata aagcaacaaa atggtttact 145980
ttcctcactg aaaatgtttc acactcagat atatgtttga ttcttaaatc agggacacaa 146040
aaaatgaaag ctggaagaaa aaactagcct attacattat ttagtaaagt aacatttttt 146100
caagtgaaaa ttctactggg gtcactaata taataaaata aaatattaaa actggtatat 146160
agccttcttg ggcaatctaa atcttcttta tgggctaaaa tatggcattg gttatagaac 146220
acttaaagtt catccaagga ctcctggggt tcacctgagg ctttaggaat ccacagagca 146280
caagatgaaa accaatctct aattctactc tttccactta ctgatttaag tatctaccca 146340
aaacatctgt tctaactttt ttattgcaat tttcaaacaa cagttataag ttaaaactga 146400
aaattgaaat aacttgttct tttattcaca ctttttaaaa gtctgtaagg caattgagaa 146460
ttcagaagtc tacatgttaa cgagaacagt ttggtaaatg tggtgtcgta caagactacg 146520
aaattggagg gcaaaaatcc agattcaggt tctagctctg ctggtcactc gatatctgat 146580
ttctgaagac tcacataact tccctgatct ctctgttgtt tgtatcggaa agtgaggata 146640
ataattcaca atacacccag tttccaaggt tgtcatgtat ctgcgagtta accctgtatt 146700
tctgaggatg ttttagagca ccagcactga gccccttttaa atgtagataa gacggtttgt 146760
gcatttaaag ccatatatta ataaatgtta aatttatatt atgttcagat atttttaatt 146820
tatgcaagta tcatttatta gggtatcatt tatgcccctt cctgaattaa tttccattct 146880
tcttctttct ctccttccct atacctcttc tcccaggcaa tgactctgat aattttaata 146940

taaattcatt ttccaatttt tagaacatat ttcactaaat ttaacattct gtcattcagt 147000
agaagactca gtttgtgtaa cactaaggag aaaaacaatt aaactataca catagaggat 147060
aaggtgcatc cctacttcag agtttttaaa aggtgaaagt aaatgtgcat cttaaaatca 147120
atgaaatgca gaggtttccc acacatatat gcatccatta aaaatatagt atttagggcc 147180
agcacggtgg ctcacgcctg taatcccagc actttgggag gctgaggcag gtggatcacc 147240
tgaggtcagg agttaaagat cagcctggcc aacatggtga aaccctatct ctactaaaaa 147300
tacaaaatat tagccaggca tggtggtgag cacctgtaag cccagctact caggatactg 147360
aggcaggnna gaatcacttg aatctgggag gtgggggttg cagtgagccg agatcatgct 147420
gttgtacccc agcctgggca acaacagtga aactccatct caaaaaaant atatatatat 147480
acacacacac agacacacac acacacnnnn gtgtgtgtgt atatatgtgt atatatgtat 147540
atatgtgtgt atatatgtat atatatgtgt gtgtatatat gtatatatat gtgtgtgtat 147600
atatatgtat acatatagta ttttaaactt aaggttacca tctaaataca ttttatgccc 147660
aaaattctga agattaatat taagttttct tagtttgcaa agcactgctc cagtcaactg 147720
atggccatcc ctaggggagt ggtctggtaa tacatacttc tctcccntat aagtccacgc 147780
tggggccaag ttatacatgg tcatgtgttt ccctccttat caattatttg tagcagcaat 147840
ttttagaaca aatggtatca aactcaacat atattctgca gttaaatttt tcactggcta 147900
tttttttctg agatcttttt atcttactcc acatgaagct aatttggtca ttgttataga 147960
gagccgtgtg gtactccgcg gactgtgaat atgccatatt tgtttatcag ttccctttct 148020
gatggctatt taggctgttt tccaaagttg tacttttata atgctgcaat acatttatgt 148080
atcgtgccca cttatgtgaa tttctctagg atatatgtcc aaaagtagaa tgcttagatc 148140
ttaggtactt cccaaattgc tctataccag ggacctgcaa gcccagatac caaatccagc 148200
ccaccacctg tattatacaa cctgtgagct aaaaatggtt tctataattt agtggttata 148260
caggtaccca cagaatatcc tcagtattgc ctcatgaccc tcaacgtcta aaatgtttac 148320
tgtctggacc tttaagaaaa cactgtggac tcctgctcta tagataaatt gtgctaattt 148380
accctcccaa gtgcaatagt tgagaattcc tgtgttctta cactctcact tagacgtcac 148440
attgtttgac atgtgaggtt ttggcaatct tatgagtgaa cactggtatc aattgggctt 148500
gcatttttgc atttccataa ttattaataa gggagcacct cttactatgt ttattgacca 148560
tctttttgt gactcaattg tttgctttac ctattttcct aaaaacaaat ttgtcttaac 148620
tcatttattg gatatttgtt ggatattaat ctcttgacta ttacatgtgt tgcgaatatc 148680
ctctcttagg cagtattttg tcttccagtt tgtctgtgat gtcttttgac attctatata 148740
tttttatttc atttttagtt aacaaataat aattgtatat atagtagtcc tcctttgtcc 148800
ccaagggaaa ctttccaaga ttcccagtag atacctgaaa ccatggatag tacagaaccc 148860
tatatatact atgtgtttta ttatacatac ataactttga taaagtttaa tttgtaaatg 148920
aggcatagta agaaattaac aacaataata acaaaataga acagttatga caatatactg 148980
taataaaagt tatgtgaata cgcattctct ctctgtctcc atctctcctt ttcaaaatgt 149040
cttaatattt tcaggctgtg gctgaccaca gggaactgaa actacagaaa gtgagacagt 149100
ggataagggg ggggcctact gtatttatgg ggtacaacgt gatgttttga tacatgcata 149160
cattgtggaa tgatcaaatt aggtcaaaac tatcggtttt tcactgatgg tctgtattta 149220
ttttgacctc atgcttaagg aattctttcc cacctgaggt aatacaaata ttctcctata 149280
tcttcttcta attgtttaca atttttgctt ttcaccttta agtctttagc tcatctctac 149340
ttactagtta gatatagcat aagctagatg aaatatactg tttaaataaa actcaaatca 149400
atttttctca gatttatatg agaaactgca aggtttagaa tatttatttg atttatctga 149460
ttatgaagtc taacacaaaa aaggtaaact gttcaaactt ttaaatttct aaattatttt 149520
ttaaatgata tataatttaa taaaaggaaa cactatataa ctaaattaaa acaaagatta 149580
tcacttctat actacccaaa aaaagtctaa tactgaagtc acttccccag ttcacaaaaa 149640
attttgctta aaacatggtc agcagttgtt tctcttttcc tttgtaccct tagactaaca 149700
gttactgtaa tgggctttat gaagtgagtt caatttgatt tgatacagtt ttactgaacc 149760
tctactacca cgtgcaggta ctaacctacg tcctggccaa agtgggaaag agcttaattc 149820
atgtcctctt aggggctaca aataattgga aattagcaat aacgaacaca gacaggctaa 149880
ccaacaaaca aataaaacca gtggttccaa ggcctaaaga tgcaaatggt aagagaggca 149940
tgtgtacatt gctacttgct atgtgatcaa taaacatctg cttcccagtt gttcccaatc 150000
tgccttatga gaaatatcaa aagtagtggc agtgaaacag ctggagatag tctgtgggct 150060
aacatataaa cacatgcgtg agggtgacag gtctgtgtta cacacacatt gcatgcccct 150120
aaaaactaaa cttcacaagt taaaatattc cataagcctg tcttcagagc tgaacttacc 150180
ttaaaatgag catttttgtt taattgtatg tttcctttta caaaatgcta caacgcattt 150240
taacccaaat gggtgagaag ggaaaaaaaa agtagtctgt gtaaacatac aagctctgat 150300
gtgtcttttg ttcacaccga acagtggtgc catacaactt gatgagttcc tgatttagtc 150360
ttcccaggga caggcatgtg ccaagcagcg aaaaataaaa tgtgtggttg cagcttgttc 150420
tggaataaag aacagcaggc tatttcatca cccctggggg caccaatttt cagcccacaa 150480
tgaaaatcaa gctgaagtat atagcaaatg catattcata acctccatgt attgtccctt 150540
tcaaacatct ctctcttccg ccaggtatgt attctgtatt tctgtcctga ctgattgcag 150600
tttattgaat gcttgttaaa ttcatctttt tccccatctt tgccaatgat ttggggatag 150660
ggactatgga agacctaaca agttgaaaga ctccattttt cctgcaatgt tttctgtct 150720

attactgttt caaaggaaag ccctgtgatg tgaatactga aaagaaagaa aatgtaatta 150780
agcgttcatt gtgggtagtc tttttgttac ctttgaagtg ctgaaattaa cacagcaatc 150840
tcttgatttt gcctcggtag tggcagcttg ctgagaatag tcatattttg ctgacacaaa 150900
tgggcattcc aaagtgtaaa ttaaataact tgattgtata gaattcattt aaaagtgcat 150960
ctgtgttgaa cgtatcccat tttacactgc gctgattaca tttgactttt tgttgaccca 151020
tataaaaagt gaaattgttt cataccacag gcggcaagac agcttaggat agggtgatta 151080
taaatggtaa acaggaaaag aaaaacacaa gagtgtggga cattgattcc tgctgtgttt 151140
ctagtgtata tttcaaaata atttaaagat aagaaattat taataaagtt cacagcaaag 151200
acgaaaataa gattaaaggt gaaatttcag ctaaagggac atctttaagc tatataaaag 151260
gaaaaaaatc ataaaattaa acttcataaa aagaaagaaa aatgaaaagg gaaagctata 151320
tagggtaagg tcatggctat ctgcagagtc agagagaatg taaaatagga tatgttgctg 151380
cagggagaga aattttcaac cagaaaatcc attgtgaatg acaaggctgc cctcttgcaa 151440
aaataaatca tacttttcag ctgtttgaat catgctaatc caaaagtctt attccgaggg 151500
aatgagaact acattatgtt ggacaaaatg atctgcatca caacaaaatt ggaagaaata 151560
attatacttg caaatgcaac tatttcagta atttattgaa tttgactgaa atttattgta 151620
gttaaataga aaaaaaataa atatatctgt ggtcacatgt ataaaactta aacagagcaa 151680
ataactacca cagncattaa ctgaggtgaa tctgggctgt ttcagaggna gaaaaaggaa 151740
gaggaggtat tagagaaaga ggagcgtcat aaagaatatt agagaaatca aacgcctgtg 151800
agaccctggt ccaggtacca gtttgaacct ggaaaattac gcttttgaaa tccactaagc 151860
tgaaaggtca aaatcctatt atatcattca taaatattct gagagagact tcccagactt 151920
cagcaggcct gataatgtgg cattttccac actgaatgca ttgtcaatgc taaacatacc 151980
ttagcaatgg gcattataat ctgagggaaa taaagaatta gatatctgaa atacattgtt 152040
attctccact ttttcattga catcactgat tcataatgta tcaatatcgg tcataaaatt 152100
aaaaagtgga tacatacatc agctgtatat tacacagtag gacaaatgct gtgttgcata 152160
ttaatatgat caaacgctat agacctgtta aaagtgccaa atagtaaatg catgaaaaaa 152220
tagatatttg gctttctttt ttacagcatt gtacaaacta aacaccactg tgttgaaagc 152280
atacatgtac ttagcagggt tcaagaggag atgaaaggtg gtgtcattag ccttttttccc 152340
ctgaaattat atcattaaaa aaaaataggg acatagtata tcctgctgca gtataaattg 152400
aaatatcggg ccaggcgtgg tggctccccc tataatccca gcactttggg aggccgaggc 152460
gggtggatca cctgaggtcg ggaattccag accagcctga tcaacatggt aaaaccctgt 152520
gtctactaaa cacacaaaaa ttagcctggt gtggtggcag gcacctaaaa tcccagctac 152580
ttcggaggct gaagcaggag aaccacttga acccgggagg tagaggttgc agtgagctga 152640
gatcgcacca ctgcactcca gcctgggga cagagagaga ctctgcctca atcaatcaat 152700
caatcactca atagaaatat cacataaatc taaatattaa gaaataaata atgcaaagca 152760
aattagttgc ataatttaga acagtcaaag cacaaaggta aaagtgtttt atacatatga 152820
tgacactcct gtgatttaat gtggcaagtc cctgtaaaat tcctttatta catcaatacc 152880
gttttaatat gaacctgtaa atcacattta gtggctatag aaaaaccttt tggcacagca 152940
acatgtggta gtgtaggtaa acattagctg aaggtaatat gcagacatcg ctgtaatggg 153000
ttctgattac taatgagcat tacctcagct ttctgtaaaa acagaataaa tgagaacact 153060
cagagaactg aaaaatggtt cttcctttca gaatgagggc ttgtcaccca ctcccatgac 153120
tttccatacc atgtgttggc aagtgtctga cacccaggtc gacctgctct ttcagtccaa 153180
atccacattg cactgggttt gtaggaaaat actgtatcat tgataacata aattaaatat 153240
gatggacacc aagctgccat tcttccttca cttctaccct ttatcgcagt gcttattgtc 153300
accttattag agtctgtgag aaaagaaccg caaagtcatt ctcacctcct ggcaatcagc 153360
tacagactca ccctgcacct cctgaatctt ctgtttccat tgcaaagaca cagccaaagc 153420
cttgggtcag acttcagtca tctctcaccc ggagtgccag ttgccacctt atcacagtgc 153480
cctacatgcc agggacagtt tgatgaggat caatctgaaa gtccacattc aaataggggct 153540
tcagcggaaa cccaagcccc ctaccacaca catacatctt gaaccagttt ctgttttgta 153600
agtttgtgct tttgtcatgc cactgtcaaa ttaattgaaa aatgatgcct aaacaaagat 153660
atttgctgaa taaaattagg tttatgctac tttcaagcaa acttcaaact ttattatatt 153720
atttttaaat ttgttgcctc cccaagagtt tgttctaatt ctttaagttt gggccttcct 153780
gttgtgaagt cacgcagcgt tttcccagag aagccaaagc tcttccttta ggagtcttgc 153840
cttctcctat ccacagcaaa tatcaaatgc atctgccttg aggagtcagt gcagctggca 153900
tgccgtggct ctctctgtcc tgctctgagg gtggcttttc tctgtttatc ccaaggaaaa 153960
agtatatacc agtttcttgt ttaaccaatg taagaaaaca ttacatatct tttttttttt 154020
tttttttttt tttgagatgg agtctcgctc tgttgcccag gctggagtgc agtggcacga 154080
tctcggctca ctgcaagctc cgcctcccgg gttcacgcca ttctcctgcc tcagcctcct 154140
gagtaactgg gactacaggc acccgccacc acgcccggct aattttttttg tacttttagt 154200
agaaacgggg tttcaccatg ttagccagga tggtctcgat ctcctgacct cgtgatctgc 154260
cggccttggc ctcccaaagt gctgggatta caggcgtgag ccactgcacc cggctagaaa 154320
acattatata tctaataaca ttttgtcaaa ctgcacttaa ccttgtctag ttactatttc 154380
agtgagccag tttatttttt tgttttgttt gtttttagtt tttggagaca gggacttgct 154440
ctgccatcca ggcctagagt gcagtggcac attcacagct cactgcagcc tcgacctccc 154500

aggctcaaga aatcctccca cctcagcctc ctcccaagta gctggggcca caggcatgcg 154560
ccaccatgcc tgactaattt ttgttttttt tttttttga gagacagggt tgcccaggct 154620
ggtctcaaat tcctggggtc aagcaatcct gcctcagctt cccaaactgc taggattaca 154680
cgcatgagtc accatgccca gctgagccag tttaaacttg ctgcattaca tgcattaaaa 154740
acaaaaacac acacacaaaa agccccagaa cttttaaaag gaaaaattta gcagaaagaa 154800
actgatgtca tcactttgtg attattccag cctccctctc ttatccgtga cctctgtggc 154860
actcaagacg cctgtccatc ctgccttcct ctccagcctc caaaccctct gcctggaagc 154920
cagactctgg ccagacttac tcaccaggcc ctcaccacac cacctggaat gcgttcctca 154980
cagctctttt ccaaaatcag tcttctcctt ggcatctgcg tggcttggga gagcatcctt 155040
ggctctctgt cattgtctca caatcttcga taagtagtgt tcggaaagtg ccccccctggc 155100
ccatgtgccc agatgggctc tccaagtcta aatgcttcaa aatggtacag aactaaatgt 155160
gtgaatttat gatggggctt catttctggt cctccttctt cacacttgcg tggccgccct 155220
tcccatccgc tctccaattt tctttcacgt ttctgctttc tgagaagaaa taagactgat 155280
ttctgggatg gagcatgaga gagacagatg cagagagaga gagagacaaa gcgtgtttcc 155340
gtgggagatg cccacacacc tcacttggga gcatcggcag tgttggtacc cacacaggtg 155400
tgttttgaca tccaagactt gaccctaatt cttcttggaa cctgatgggg agagaacctt 155460
gctgggggcc ccacatgggc caggggcagc cagggcagag ttgggcagct gcctgagagt 155520
tctcggtgga gaggcccaga atatcccaca agaacaagtc tccgttagtt ccaagtggaa 155580
tgacagccct ttctgagttt ccctgagttg agagctaata catgtgttgt gggaacaaca 155640
gcccaagcca aaggccacgc ggaggccagg cgtggtgact cacacctgta atcctagcac 155700
tttgggaggc caagatccac ggatctcatg agctcaggag ttcaagacca gcctggtcaa 155760
ccttgtctct acaaaaaata caaaaaaaaa aaaaagtagc caggcatggt ggtaggtgcc 155820
tgtagtccca gctacttggg aggctgaggt gggaggatcg cttgagccca tgaggtggag 155880
gctgcatgag ttgagatcac accactgcac tccagcctgg gtgacaatgt gaggccctgt 155940
ctcaaaaaaa aaaaaaaaaa aaagaccagg tggagagtat agtggattaa atgggcccct 156000
acaaaagata ggaccatggc ttaacccaca gaacctatga acacgacctt attggaaaaa 156060
gagtctttgc agatgtaatt aaattaagga tcttgtgatg agatcatcct ggattatctg 156120
ggtgggccct aagtccaatg acaagcgtcc ttgtaagaca caaaagagca caagacacag 156180
agagaaagcc ttgtgactgg tgttagataa aggccaagtg actggtgtta acaaagccac 156240
aagccgaggg acagaggagg ccaccagcag ctggaagaga caagggagta ttctccccccg 156300
tggcctttgg agagagtgtg gtcttgccga caactcaatt ttggactctg gcctccagaa 156360
ttgtgatcaa ataaatgtct gttgtttgaa gctaccaact ccgtgcacgg tgttacagta 156420
ctctaggaag cgcatatgga ggacatcact gggaatcaga gctcttagct ttgggagctg 156480
agatggtggt caagtcagca gaaagaaagc cccaaacccc ctgtacagcc tcatcagttc 156540
ccctgatggg ggatggagga catattaccc agcttgtagt gggtaacctt gaccttaaac 156600
taccctaaaa agaaaaggag catttattca aatgtaactg agcttgaaag gactgaatgg 156660
aactgatgta gcaagataat gtttccagcc cccaacagaa atggagggat tgtgcaccaa 156720
attcaattca tttgtagaaa aaccaagaaa tttattttag ctttgcataa ctaattttat 156780
gtctgtatat tttcagacat tgtatagcta tactacatgt cagggttgct cggggagaat 156840
taaatgaaaa aaaagctgca atttgttgag ctattattac atttgtaata ttttacaaac 156900
gagagcacca aaactcactg tgggctggca gttgccacgt ggctcatagc tggcacagcc 156960
tgtttgctac ctgagctctc tgactccaag cacagtgctc tcttctatgt ctgcttaatt 157020
cagtgataaa catcactgca gtgagtgcaa tattgtattt atgtatcaga ccaggcagtg 157080
cctcacacat ccatggagtc cagtcaactg tttgtcaaat ttaatagaat aacatagcct 157140
tgctgtcctt aagtagcctg ccctctgtcc agtttggggg atataattaa gaataaaatc 157200
tcctgccaac ccacgaaacc tctccttaaa ggtagaggag agagagccca gttttattat 157260
tgaaaaagct ctaacgcagg ctgcaatgca catcactggc aatctgctaa agagatcacg 157320
gaggcagaaa atacctccct cttttatatc accgctttcc atacgtgttc tcaagataga 157380
aataactagt cctcaagtaa gaaggcttga cagcaccact tgttaaatat agtttatcct 157440
aaattcacct ggtaagtggg gtggccatct gtgggagctg attgccttta tccaaaggaa 157500
aattaaaact tctcatattt ccgtgacaag taggtaggtt tacaacttgg agccagatgt 157560
ctaaactccc accctctacg gagaagggga gacggaggct cttgtcttcc ttgatgttta 157620
cattacttca aggagacagc cctcaaggaa aacattcttt taggttgtag aactggtgag 157680
aagcttattt ggctcttaaa agtatttata tacatctcaa acagaaaggg aaagaattta 157740
caattccaag tcttataaag gaaatgctct aagaaaaggg aggcgaacag gatcccttcc 157800
gacccccctt tttttagatt catgtttgta ctttcactag agagagagga aacatgatta 157860
aattaggaca cagaacaatg cctgctccag tgcccaggtg tgagagaagg ggtataagat 157920
atcccctcct gcagaaggaa accagcaggt gaacatctca gaattcgggg tgagaaatca 157980
agacagggga aggtagactc tgcctctgat attactttct gtcgtcgagg tattgggaaa 158040
tgggctgaac atggagcaaa ctttaaaatg tccagtttgg aatgatgtct ggttcagaat 158100
aagcctgatg cccagttacc agtggtgtac cagtcatggt gatgttaata aaaaccctct 158160
ggtctagatc attctgtatc ttcttcaagt tatggtaaga ctccaaaggg catggctgtt 158220
gaaaagaagg gtgctggcat ttgtcccaag ccaagcatat cccatcataa tttaatcatg 158280

ttgatatact cattaaaatt gaaaaagagc tagccattaa cttctgcata ataacttctc 158340
tagcactggc tgaattatct caactttctg accttcatgc ttaaccacct aatattaaca 158400
aatgggaata attcattaag aagaaaactg aaaagaaaaa tatgagttag aaatgaaaaa 158460
gatgatcaag tgaatatggg acttaaaaaa attcaaagtt gaagagggac tacagcgtaa 158520
aatattttta aaaattatac tattttgttt tattttattt acctgttcta aaagaagcat 158580
acattttta aaaactgaga aaatcataac atttttaaa agaggaaaaa ttgtattact 158640
ccaatacaca aaagcaagta ccattaatat ttctaaagta cctccttcaa gcattttctc 158700
tattactccg ttatatgcat agttgagctt ctaccatata tatatatata tatatatact 158760
cgtatttact gttataatgt gttttcaaca ccataaaaat tcaccagtgt attaggtatc 158820
tgttgctgtg taataaatta cccacacatt agaagcctac caggaaatag atgtttatta 158880
tctcaattag tttctcaagg ttgggaatct gagacaggct tagctgagtg tttctagttc 158940
aaagtgtcat gaggttgcag ttgagatgtt gtctacagct gcagtcacct tgaagtttta 159000
ctagggctgg agtgcctctt tccaagatgg gtcactccag tggctgttgg caggaggcct 159060
cagttcctca ccacatggac ctctccagag ggctgcttga gcatcctcac aacatagcac 159120
ctgcttccac agagcaggtg atctaggaga gaccaagggt aaagacacag tgtattttat 159180
tgcctagcct cataagtcac acacaattat ttctgcaacc tgctgatgac acaggccaga 159240
catattcagt gtggaaggga actacacaaa ggcatgaacc aggagtctag aacaacgctg 159300
gaggccatct tgaagagtag ctaccacatg ggaatttgta atggctgcat gtatcttatc 159360
acataaatct ctataactgt attgactatt catgcaaagt tggatgttat ttctaatatt 159420
tccttattac aaacaaccct ctaataagca cctgtgtata ttaagctttt catcaatagt 159480
tcagatcact tttttagaat attatcacac aagtgggatt gttgagttca aaaaaacctt 159540
tttcttaatt tcctttttaa gtaagttggt ttcataaagc aagttaacat attatttcaa 159600
tgaacgtatg cattttattt catgtatttg tgataaatac cttgaaatgc ctatgcaatg 159660
attcatcact gcatgtttat tttgcattta atgaattata tactcaatat attttaggta 159720
gctatattat acatataata ttttgatact tatggcatgt gggtttttc attgttattt 159780
caccatttta tagatcctcc tgagtttgtt tgttgctgtt attttggaca acttagaact 159840
tgatgaagac ctaaagaagc ttaaacaagt aagtagtgtg tgggggattt tttttaaaaa 159900
ggacaaaaaa ccaggggcaa accatttgct tataggcaat attaaaaatt tgagcttcaa 159960
agtaaaaata aaatgtaata aggctatcga aggtggtaaa atggtgttaa gtttaacttt 160020
tatctaatat taacaaggga aactttctat tactctataa atatcctagg aatagtttta 160080
ttatttttga ataaaagtaa aatgggcaga gtatagttaa ttggatttgg agactgttct 160140
ccttctgcca ttaaatgaag gttgtttgga aatgagatgc ctcaaggcca gattttgcat 160200
aattatatgt aatagtcttt atcatcatca taaaaatact acaatcaacc tgcctggtct 160260
ctagacacta aaggacaatt tagtctgggt gcccatagtg agtataagaa acagaataaa 160320
tgatcaaatg tggacccagt tatgtatgaa aacagacggg atgtcatcat ggtgaaagtt 160380
ataccccaga gaatgaaact agaaataggt gaatgctgtg ttcttaatgg caggaggatt 160440
acagctatcg tcaatcattc ctatagcttc tgttgcatgg aggatatttg gcttgaatat 160500
ttggctctat caaagagtac agaccctttg agaagaagag ctcgcaagag agtgggacaa 160560
tgggtggaac gttcttgtat caccctcccc aaaactgaga gaatagttga gatgttaact 160620
accaaactct acccaactgt tagaatgtta taatgttgac agaaccacac ctgttataat 160680
gttaacagaa ccatggctct gaaactttcc atttggaatt ataagcaggc cctccatatt 160740
cagaatttcc caaagctctc tatttttaac aaagcaaaaa gtataaccta tttaaatttt 160800
ttaattttt ttaatctatc tcatgatcag gttaggcatt tcattttatt actcttacct 160860
ttaaagccta tgcttttaac agagggtatc aacaaggaaa aaaaaaaaca gaacaaacaa 160920
acaaaaaaaa attgttatga agattcctac ttggagcata ccaagaaagg ttttgagttc 160980
tttaccatcc tacagctacc caggcaaatg ggcatcttcc cggctctgct agagatgatt 161040
tcttccccgt cttttcactc tgtggcagca gtgatagatt cttcaaatga gagttgggac 161100
attcctttat gccatcaatg aaaatacttt gtgtggcttc tagttgcaga aaaaaatatc 161160
taataggtga attcaagatt gaaagtgacc agccatataa ttagacatga tcaataaaaa 161220
tctgctgctg tagaaccact ttagtccatg ctttacaaat ctggtattga gctgtcactt 161280
ttccttcatg ccatggttat tatgcacaga tatgtactat cctctgagac agtatgttac 161340
aatgtgtatt ctatagataa aatttaaaat atccaatttt ttttagctag tgctaccgcc 161400
aggttccata tgcttttaaa taaccatata attccagccc tcatttgtaa aataattttt 161460
ctgctggaaa gcaacaaaat aaacttgtat gtagcaattt tttcaggatt gtgaattcaa 161520
acctaaggat actttgcagt gttcattttt ttcaacattt taacttattt atatatacac 161580
gtatatgcat agcctactta aatttttgt catctttata tctagtggtc acaataggca 161640
tttcatatct gacccaaagt gaagaagcaa caataaaaag tcaggacttt aattggcatt 161700
gacattcttc ctgggctata aagttttaat aaactatatg gcccttcact ttgggtaaat 161760
gtcattattt ataaaatggc ttttaatcac ttgctagata ttagtgaagc tttgtgtttc 161820
tggaaattct gtatatcaga actggttatc caattgcact agcctacaat ttggaccata 161880
tactctgggt atttgggcat tgaaaactcc aatcataacg cagcaattcc attttgtta 161940
gtactttta cactattctt tattcatggt tggactgaaa ctatgtatct attaaattct 162000
tctattaatt tcatacttga aactcgttga acctgaaatt cattatagca aatattatta 162060

```
aactaagtta aagtgttcct ggttaacttt ctctcatttt tgtatgatta tgttttgttt 162120
tcagtagttt ttaacaacgg tgtcattttt tatgcccctg atttttctta ttttcagtga 162180
tcttggcctt taaggtaggg ctcatttgca tatttttaaa aacacacaca ttctcatgca 162240
ttcactttca tatacacatg tggtcatgtg gatacctgaa actttagact gtcttttaca 162300
gattctttta aaaaacaatg aaattggtac cctaaatatt tgtttcaatt gtccatgtac 162360
aaatccagtc cttaagattt aaacttatgt actctatttt tactactccc agatagagag 162420
tagagtggag gaaatgttca aaaaaagtat ttgattttct taatgtttca agtttcagaa 162480
agcgctatgc attccaaatt ccatctttgc tccgttcaaa ctttttgctt cttttaccac 162540
atctgcttgt atgtagattc tcatagctat ggtgacattg gttgaattct tgctcagtat 162600
caagtgtttt atgttttcac ttatttttca caacgaccca ttcaatgcag gtgttattat 162660
tatgtccatc ttacagatga ggaaactaag acatagagag gttaagtaac ttgctcaagg 162720
ttacacagcc aacaaatgtc ggagctagaa tttgaaccta ggcaatttgg accgaaagcc 162780
cacactctta acctctaaac tgtattgatt caaactaaag ctatataaag gccaaacatg 162840
agtcatgcat actggggagg gcactcatag gactatgaat tatttgttta taaataatga 162900
tgttgtagat aacaaaggca tcctcatatt caagtaactg ttagaatgaa ggacagtttc 162960
tgagaatagc ggtgaatgca gcaacccaat cgttaaaact attcacatta gttctttgtt 163020
tcctctcaaa actcagagga tctaatcaga gccactggag ttacagtctt gctgcgcgat 163080
ggcaaacgcc actcaaggtg ccactttgca natgatcttc ttggagtgat gatcctcctc 163140
agacgcctac ataacaggct tgcagcatag cagcatttta gtgactttaa tgtcttttat 163200
tatcgatatg tttttaactg tgttaatatg taaaagttgg ttcatagatt tttttcaaga 163260
gtaataaaaa ccagggcctg ttcgctctaa aatgggggtg ttggccaggc gcggtggctc 163320
acacctgtaa tcccagcact tggggaggct gaggtgggcg gatcacgagg tcaggagatc 163380
gagaccatcc tggctaacac ggtgaaatcc cgtctctact aaaaatacga aaaacaaaaa 163440
aattagctgc gcgtggtggc gggcacctgt agtcccagct acttgggagg ctgaggcagg 163500
agaatggcgt aaacccggga ggcggagctt gcagtgagcc aagatggcgc cactgtactc 163560
cagcctgggt gacagagcga gactccgtct caaataataa taacaataaa tggggtgtt 163620
aacatgcaac actcagtagg gctcaacaga ggaagaggaa gcagccctca tacaggggag 163680
aggagaggcc gcccttaagc taagggtgtg agatggtggt agaaagtgaa aaggtactag 163740
tgaagaaatt ataatggaaa agaaggaaga agtgaagtag gaaaacacca gatatgaaat 163800
aaagagtatg actataactc agggataaag gtgcaggaat gtggccaaat ccagagttag 163860
gaatgtgagc aagtcagtgg ccaaaagtca aagcccacca gggcgttccc ctcagaagct 163920
ccctgagggc aggcatgggc tgcatgatgg attgtcggtg ggcccgtgtt ctcctggaag 163980
agaagcatgg cgctgtatgt cttttcccat tatttttcta tgcaacctcc gtgtgcatca 164040
gaggtggttt ctagccaagg actgctgagc tctaggcgtc ctcatgagaa gctatggtgt 164100
catttttaat acttcaggaa gctggaagag aattgtgcat tcacttaata taagcccgca 164160
cctgctaaat aaaatgtcaa gtttaatact tttagctgga aacctgaaaa ttctaagtgg 164220
cagcactgtt tccttaatac tatagttctg aagtgttttc atatatagtt tcctttaagc 164280
aaatttgttt aaagctacaa tgaatttctt attgcttaat cgaggcattt tggcaggaaa 164340
gaagtttggg ttttagaatc cagaggagaa agtaaaagga ctttttgcac agaaattttg 164400
agaggctctg gtgtgtttat tcccatcacc gcctctccaa gacatcacca gtgtgtgccg 164460
tgatggtcac ctctatggcn ttttcctttg tcacttccca caatcatgtt ccaaaaactc 164520
acctcaatca tataaccata tgaaattcct ctattgcctt ccctgtgtgt tatatatatg 164580
acacagctga ttgctaatag caatagggca gcgtaatctt ttcatagctg ccatcattta 164640
catgaccccc atattgaagt acgtagaaac catatttctc ttttcagcat ccccaaaagc 164700
caatacagag acctcggaaa aaaatatata tgtgtgtgtg tgtatatcta ttattacata 164760
tatgtaatat atgtaacata tatgtatatg tcatacatta aatacaaaaa tatattataa 164820
ttttgtatta catatatgta atatctatac atatatgtat atattaaata taaagattat 164880
atttataaat atatgtaata catgacataa attatacata atgtgtgtaa tatatgtaac 164940
agtatataaa aattatattt tttatattta gtatttatac acacatatat ataaaacatt 165000
gtttctgcat gacatttaaa aattggctaa aaatgagatt gtggtgagct gaggagattc 165060
ctgataaatc agttggtata gaggctacaa caaaaccctt gacctaaaac ttctctgaac 165120
agaagagttg atgactgtga agctcaagtg atgcaggaat gacactgggg aatgtacaca 165180
gagttttgca aggtttgaat atgaagagaa ttcttgcagc tttaatgatt tttgcaaatt 165240
tggtgaaaga cagatataaa gggttttctt ttaagtggta taaaattcat taagctttga 165300
gggaagaaaa aacccacaga aaagcctgag gggaggaatc atcgaaaagg gaagtatagg 165360
tagtctgttt agattaaatt cagagctgtg aaattgaaaa ctgtttaccc agccctgtat 165420
ccagtctcac ttgaatcctg gttccaattt aatgatgttt ggtctttgct agagtccact 165480
gcatagtgtt ggatagatat tatatattaa gaagctttcc tttcttattg actgtgactt 165540
ttttaaacaa aaaccataaa tatcttatat tctcttttgt taaagtatga gctctaggtg 165600
aatgtttttc tttagcctta agctgcatga tagttgattt tatgtgtcaa cttgactggg 165660
ctaagggatg cccagatagc tggtaaaaca ttgtttctgg gtgcatctgt cgggtggttc 165720
tggaaagatt tgcatttgaa ttggtagcct gagtaaagca gacctccttc cccaatgtcg 165780
ttaaaccact tccaaagctt tgaggtccca gatagaacaa aaaggtggag gaaagctgaa 165840
```

tttgctgtct gtctcattgc ctgcaccatg taaacaacct tctcctgccc tgaggaatcc 165900
tggttcttag gccttcaaaa tgttgccctg gcttccaaag gtctccagct tgaagagagc 165960
agatagtggg acttatcatc agcctccata attacatgag ccaatacctt aaaataaata 166020
ctgatgtata ttttattggt cctgtttctc tggagaatct tgcctaacac acctgaagct 166080
ctcattkgca tgtccatttt gtcgcctata ttgagttcat tccagattga gggcggtagc 166140
aaggtggcag ggatactgct ggaagatgcc attcggctga gcactggttg catctcctga 166200
tgttagttga atcttcaatc ttctcagcat tttgtcagtc tctcatgaat gaactcaaac 166260
taatgtaatt ttaaaagact gatataactg ctgcaggagc aaatgctatc tattgagaac 166320
taccttgtgt aggctrcttt atattaaatt atcacattta atcctaaata ttaaagacgt 166380
ggcaactaac attcagggga gtgaatttct cacccaagtc cacacaattc atatgtgcct 166440
gggtcagagc cacaaaggct gtctgaccct aaaccaatgt ccttgctgaa aaaggccatg 166500
ctgcctgcag agaaccagct ccttcattta taatgaaaag catatctagg tgtggattgg 166560
atgagatgct tatccatttt ttagatcagc atattttctc cctgtgggta ttcaaagaag 166620
aagccaaatt ataatcagac tatagcttag tgagccagga atcaggatgg agagggaggc 166680
gagaaaggtt gtgtgattta aatacataga tgatggtttg atagatgcag caaaccacca 166740
tggcacatgt aagcctgtgt aacaaacctg cacattctgc acatgtatcc cagtatttaa 166800
agtaaaatct aaaaataaat aaaaatattt ttaaaaatac ataatggcat aggctaagat 166860
aggctgagat caactgagtt atctctttta cagttgagat attagtgacc tagaaactaa 166920
atctactcat tctgtgaaac tggggtctgg acactcatct actatgctat agatattgtg 166980
ttggggctag ggacatgtga tggtcaggac actgtttcta ccctcaaaaa taactcctga 167040
gccagtggga gaaaaagaca agtaaacagg cagtttgggt tcagtggtga tgattataag 167100
gataatgttt cggaagcaca cagaaaacat ctaaagcaga ctgtgaaatc agggacgttt 167160
tctcacaagc aggtgcagac tgaaatctgg aggttgagta gtgctaatcc aggtgaagag 167220
ggttggagga agatggcatg aaggagggta gtgaagacca agggaactgc agagtctttt 167280
ttaggtggga aaaaaacaga gtggctttct cgacactaag agtttgctgt ggctaatgtg 167340
tagagcaaga gagaaaattg ttaactggaa aaataaggag ataccagcac aggaaatgct 167400
tgagcagcac taagcatttt gatattattc tgatggtatt gatgccacag ggtgtcataa 167460
tcatacctac attttggtgt cagtgtgaaa tgcactggag ggcacccaga ctggagatgg 167520
aagaatccgt tcttgaggca agtggtgatg gcagcctgaa ccaaaggaaa taggaatgag 167580
agaatatcga aagcggtgga gtacagagaa gaggatttag tgacaaattg gatgtaggaa 167640
gtgagaaaaa ccaggacaca gggatgtcac ccagatttct agatttcttt aagtgcgctg 167700
atgcagaaga aatacaagag aaagaagaaa tgagagtttt gtttatttgg gtttttttc 167760
gggggaagaa gtttggtcat attttccctg agatgcctgg gaaatatcta tgagacaatg 167820
tctatggaga gatacaaagt ttaaagctta ggtggaatat ctaggctgaa atagatactg 167880
aaaaagagca aaatcaacat gcagatggta atttaagcct ttagagagca ggaacttacc 167940
aagggaagga tggttcccta aggggtaata aataaatggt atgaaccagt gaggaggagc 168000
caaaatgtgt atgagaagtc acagcacaag aggtaagagg aaagcctaga atgtgtttgt 168060
caccaagatc aaaaggtgaa agagtttcaa gagagatcct tgttcaacga tctcaaagac 168120
ttcagagagg tcaaaaaaat agtcaccatg gaaagcaatc ccatttacag tagcaccaaa 168180
aagaataaaa tacttggaat aaacttagcc aaggaggcaa agacacatac cctgaaaact 168240
ataaaatact gaggaaagaa cttaagccac aaataaagat gaattctgac ttacgatgat 168300
tcgacttaac agtttttgtt tttgtttttt tttgacagag tctcaacctt ttccttaggc 168360
tggagtgcag tagcatgatc tttggctcac tgcaacctct gcctcccagg ttcaagtgat 168420
tctcctgcct cagtctacca agtagctggg attacaggca tgcaccacca aatccaacta 168480
atttttgtat tttcagtaga gacagggttt caccatgttg gccagggctg gtctcgaact 168540
cctgacctca agtgactcac ctgcctcagc ctcccaaagt gctgggatta caggcgtgaa 168600
ccacctaacc tggcaactta caattttctg attttatgat ggtgcaaaac agatacacgc 168660
tcagtatgct cctcaactta tgatggaatt atatccagaa acactcatag taacttgaaa 168720
atatcataag ttaaaaaatg cacttttaca gtattttcaa cttataatga atttataaac 168780
cctattgtaa gttgaggaac atctgcaagt gaaaagacat tctgtgttga agaactggaa 168840
ggcttaatat tgttaaaatg ttcataccat ccaaagcaat ctacagattc aatgcaatct 168900
ttcgtaaaat tccaatgacg ctttttgcag atatagaaaa gaaatcttaa aattcatacg 168960
aaatcttaag ggataccaaa tagccaaaac aattctgaaa aagaacaaac ttgaagatgt 169020
catacttcct gatttcaaaa catattacaa agataaaata atcaaaacag tgtgatactg 169080
gcataaaggc acacatatag accagtggaa cagaatagag aggccacaag taaaccctca 169140
catatatagt cacatgatct tcaacagtgg tgtcaagacc acactatggg gaaaggacag 169200
tctcttcaac aaatgatgtt gaaaaattgg ataccacaca caaaagaatg aagtttggcc 169260
cttaccttat atcatatata aaaattagct caaaatggat taaggacctt aacaaaagac 169320
ctaaaaccat aaagctccta gaagaaaaca aggcaaaagc ttcatgacat tggaattgtc 169380
aatgatttat tggatatggc acagacagca aaagcaaaaa taggagactg cattaaactt 169440
aaaaacactt gcacgtcaaa ggaaacaatc aatagagtga aaagccaacc tatgggattg 169500
tagaaaatat ttgcaaatcg tgtgtctgat gaggagttaa tattgagaat atgaaagagc 169560
tcctatagtc aacaagaaaa atacaaataa tctgttttaa aaataggcaa aatacttaag 169620

ctgacattta tctaaaaaag gtatacaaat ggccaagtag atgaaaagat gttcaacatc 169680
actaatcatt agagaaatgc aaatagaaac cacaatgaga taccacttta tacctattag 169740
aatggccact atcaaaaaaa aaancagttt ggcaagaatg tgaaaaaatt gcaacatttg 169800
tactttgttg atgagaatgt aaaatggtgc tgctgctgcg gaaaacagta tggcagttcc 169860
tcaaaaaatt aaaagtagca tgatcatatg ataaaatata cttctggata tgtatatcca 169920
aaagaaatga aaatagaatc tcaaagacat atttgcacac tcatgttcat tgcagtattt 169980
ttcacaataa cagaaggtgg aagcaaccct agtgtccacc agtggatgaa tagataaaca 170040
aaatgtggta tctacacgca aggaaatatc attcagcctt aaacatgaat aaaactctga 170100
cacatgctac acatggatga accttgagga catcatgcta agtgaaataa gccagtcaca 170160
aaaagacaaa ttctgtatgg tttctcttat gtgagtttat ctaaagtagt caaattcaga 170220
gaaacagaag acagaatgag agttgccagg ggctagggag gggagtaaac ccattgttgt 170280
tcaatgggta cagagtttcc actttgaaag atgaaaaagt tctggagatc tgttgtacaa 170340
caatgtgagt atgtttaaca ctgctgaact gtacacttaa aatggttaag atggggctag 170400
gcgtggtggc tcacgcctgt aatcccaaaa ctttaggagg ccgaggtaag cacattgttt 170460
gagcccagga atttgagatc agcctgcgca acgtggcaac accctgtctc tactaaaaat 170520
acaaaaaatt agcctagccg gacatggtgg cgcatgcctg tagtcacagc tacgtaggtg 170580
gccaaggtga aaggattgct tgagcccaag agatcaaggc ttcagtgagc tgtgatcatg 170640
ccattacatt tcggcctggg caagagtgag accctgtctc aaataacaac aacaacgaca 170700
aaaatgatca agatggcaaa atttatgttg tatgtttttt accacaatta atttctttta 170760
tttaaaactg atcatattaa caataaggag gtggttagtg aaggcaataa gagcaagctc 170820
agggccttcg atgatacaga gctgggttgc agaggatagg gaatcaatga cgggtaagga 170880
agtgaaggtg gatcaccaac cttttcctca aacgcctcat actctgtttc cagatagacc 170940
acctggttgc tggctgattc ttaattttgt tagatatgtt aacaccattt gctacagtgc 171000
tacccaacct tttgccattg tggtatgcat agagaatgat atttgtacaa cacgctgaag 171060
tcaataggta ggaagcaaaa gcagctggaa atatcagata tcaaggattc ctgatgctcc 171120
agggaacagc tgcccaggag cgagaggacc gtaccctgaa gaacctataa atcattagtg 171180
gctgagagtt gaaaaactct gcatttcact caaatctgtt gatgctacct aactccactt 171240
gaagccattc tttcaatctt ttggtcagag atgactcttt tgaggtcagt ttattattgc 171300
ccataacttt ccatgatgta aatctcaaaa gatcagctta tagagcattt gatctgctat 171360
ttctgaaata gctacttact tttataataa attcttactt ttataataaa tgttagactt 171420
tttataataa attctctgtc acactaagac ataaacagca ataaaattgg tttgaagaaa 171480
tgaaatggct aagacagaac agtttagatc aaggtttaag ccaagaagtc acaacagagg 171540
ctcagaattt gggaatatca attatcaaca taaaaacaac agcctaggaa tctagaagtc 171600
attcaaaaag tagaaatatg ggaggccaag gtgggaggat cgcttgagcc cagtagttgg 171660
agaccaacct gggcaatata gtgagaccct catctctaca aaaaataaaa attatcgcag 171720
tgtggtggtc catacctgtg gtcccagctg cttgtgaggc tgaggtagga gaatcacttg 171780
agcctgggag gtcaaggctg cagtgagctg tgattgtgcc actgcattct agcctgggca 171840
acagagaaag accctgtccc cccaaccccc caagaaagat aaagatagaa acagccaact 171900
ggatttttac agtcaaataa gtataggtgg gagattcaaa aagtcagttc aaaacagaag 171960
tacctgttag aattgatgtt ctcctgatat gagtggaatt ctgtccctca aataggtacg 172020
tggaagccct acgcctcagt ccctgtgaat gtaacttgat tggaaatagg gtctttgtag 172080
atgcaatcag gttaagatga ggtcattagg gtggactcaa tatgactgtt gtcctcataa 172140
aaagagaaga gacacagaca cagacacaca gagagagcac tgtgtgttga tgaagccaca 172200
gaccagagtg atgtggtcca caaggaacac ccaggattgc tggcatcaca agcagctgga 172260
aaagtcccat ggagtagacc cccctcagaa cctccagaag gagcttgcct tgctgacatc 172320
ttgatctcag acttccagcc cccagaaata tgagacaata catttctgtt ttcagccacc 172380
cagtttttg gaactttgtg gcagcggccc tagacaacta acacacctcc agagccttgt 172440
tctctggact caagaagaaa cgatcccttc taaagctggc aagtagcctg gctgagagca 172500
tggggcagca gggaacaatg gacgtaccct tggaatccta cgccagcctg cgccaccctg 172560
gagcagcttt ctcagcagtt ggagttattt tcttctgcct ttctttgatt ataggaatca 172620
aaccccatca tagtaaaatt tttatagactt gagactttct tagaggaaag gtttctgtaa 172680
gcccttccaa aatttcatgt catgtgtgat taactcaaac cagaaggaca ttgttttaga 172740
gacctttctt ttgcctcatc catgggtact cgggactgtt ttgttagcat gatctctctt 172800
accatggaac aaacatgaaa ctacacacct aagattgtcc actcgagtct acaatggtgg 172860
gttgtcggtt gtatcattct ttaattgtgc taattcagaa ttatttttcat tctagtttcc 172920
acagcactct gcaaggtaac ccttcaatta aaatatatat atatatatagc caggcatggt 172980
gactcacacc tgtaatccca gcactttggg aagccgaggc aggtggatca cctgtggtca 173040
ggagtttgag actagcctgg ccaatatggt gaaaccctat ctctactaaa aatacaaaaa 173100
attagccagg cgtagtggcg ggcgcctgta gtcctagcta cttgggagac tgagacagga 173160
gaatcacttg aacccaggag gcagaggttg cagtgagctg agatcgcgtc tgcgaatctc 173220
catctcaaca agagtgaaac tccatctcaa aaaaataaat aaaataaaat atagtgggat 173280
ttggagggaa gatgtgccca aacaggagag aagctcacac ttcaactcca actgaacacn 173340
cacccactgc ttaactgtaa ggatgctatt ttattttgtt tcctttaatn ncaagagnnn 173400

nacaaanatt gttnaagana gcatgnagct tactgcactg taccattgaa aacagcaagg 173460
atgataagga agagcaacct cgcagcactt tttctttcag atcctgtcac aggcagtggg 173520
aaactagatt gcaatggttc taagagaaaa gcatgacagt tttcagtttt atgcggtgcc 173580
tggcagttgt tgaattacaa aaaggcactc ttatagttgt gttaagattt gttgtgagga 173640
gttagaatag tgaggtttac cctgtgtgga cctacacatt aaataggaac atgctggagc 173700
tggagtcatt gccctgattc cttttggact caactttcac acagtccgtt tggatctttt 173760
ttcaagtaat tcatgtactt ttccgttctt ctggaagcat cctcacaatc tgctttatca 173820
tccattcttc aaatatttga gtacctacca tgtgtacaac gctaggccag gtacaataat 173880
aagtaagttc tttgctccca tggagccttc tggtctaata ggggagttgg atgtaaatca 173940
agtaattaaa ataattccta actgtgtaaa gattgagatg agctttcttc agaaaataac 174000
atcatttaag gagagacact cgtagaagac cccgacacaa atggtggtca ggggtgagga 174060
tgaagaaagt tacaggagag ctaagttttg caagatgagc cttgtggact tcacggaggt 174120
gtggtgggtc aggggagggg agcacaactc cagggagaag ggaaagcctc tgtgcaccca 174180
gaatgcagga agaccagggt ggagtggtat agagtaagtg tggaaaagtc agcagggcat 174240
gaaccatgca gtgccttgag aaacacggta aggtcattta ccatttgttt tttgtttgtt 174300
tgtttgtttt gagacagtct cactctgtca cccaggctgg agtgcagtgg tacagtcttg 174360
gctcactgca acctctacct ccaaggttca agcgattctc ctgcctcagc ctcccgagta 174420
gctgggatta caggcgtgcg ccaccactcc tggctaattt ttatattttt agaagagacg 174480
gggtttcacc atgttggcca ggctggtctt gaactcctga cctcaggtga tccgcctgcc 174540
ttggcctccc aaagtgctgg gattacaggc atgagccacc ataccagcca gaacatttac 174600
ctttaatgtt tacctttaac ctgagccact gaaagcttat aagatgggag gcagtggtga 174660
accagagagc tgtttggaaa ataaaatcaa gagggacttg gtgataggtt ggatttaaag 174720
acatgagtga gagagtctca aagatgattc ctggtttcag gtttctacag ttgcatgaat 174780
gaaatgtcat taactgagtn aagaaacact agcatacaac cgtatttggg ggaaacgttc 174840
atcaactcag ccttgggcat gttgagttgg tgaatctttc aagatgtctg aacngagatg 174900
cctaatagtt gtgtataggt ctggagttca ggggagaagg atggctgcct agagatataa 174960
atgtggacat tcaatgtaat tctcaattat ttattcaggt gctgttctag acatgtggcg 175020
taggtcaatg aacagaacag ncaaaaatga ctgcctcctt tgagcttcag tcctagtaaa 175080
gttctccatg ttttcataat actggaaggt gtaaatctgg atgagattgt atagggtggg 175140
aagaaaaaat atgctggagc cttgaggaag agcaacattt aataactgct ggtgtttgcc 175200
tttatggtaa ctcaacctgt ctctgttttc ctccttgtgt attttcactt tcagttccat 175260
tgcaaatagt caacattttc tgtatctcag gttcaaaact tgatcaataa gaacagattt 175320
ggtcgatcca gctaatcaat atgattcctt ttaggcagga atttcatctc tgattgcctc 175380
gcaatgctgg ccagtcttat agaaagtccc tgtctttggc ccggtttctg gctcaaccag 175440
ctttgacaag ggtgatgagt tactttgtac cataaggcaa tttatnagga agaaacacct 175500
tcataaaaaa aaacaaaaac aaaacaaaac aaaaaacaga acaggcagac atttggagaa 175560
ctcacctgca tattacagag agatttaata tgtttatcta ttgcatgcca ctgtaattta 175620
acagtctagg caaaggtaac agaaaagagg agctgagata atccagctcc tccccaacca 175680
ctcattcagg aggaagcaga tgtgagtgcc attgtagggt cctgagggag tattggttcc 175740
catttcctag cctgcccctc ctccccacgc ttggtcagtt acaccttcat atggctcctg 175800
ttctcacttt acctctctgc tttatgataa gcgcagataa aaggcaagag acacagacaa 175860
agcgaaaaag aaggaagagg gaaggaaatt tagaagggac cttgcaaaca ttttccttcc 175920
actgagtggt ccacaggagg aaacaatgaa aacttctctc tcctttggga ggccaaggcg 175980
ggcagatcac aaggtcagga gatcgagacc atcctggcta acatggtgaa accctgtctc 176040
tactaaaaat acaaaaaatt agtcaggcat ggtggtgtgt acctgtagtc ccagctactc 176100
aggaggctga ggcagtagaa tcgcttgaac ccaggaggca gaggttgcag tgagccgaga 176160
tcgcaccgct gcacttcagc ctgggcgaca gaatgagact ccgtctcaaa ataaaataaa 176220
ataaaaataa caaaaaataa caaaaaacaa aaaaanacct tctctctcac taacaaagag 176280
atgtgatttt ggagaaatgt cttccctctc tttcagtttt accaaatcta acttgggtat 176340
ctttagatcc ggtgtgccct cttttctttg atgacacgag tcctaactcc tatatattgt 176400
cttatatgat tgttttatgt catccaagct ggcacttata tcatctccct ggtctcatag 176460
gcatttaaat ctgggcccct gcactagacc ctgacaagat ctcttctaga tctaaaattc 176520
tcatccggga aagagtcgag agctgtcatt actgggacac ctgtagctgt atacttcatt 176580
atgacagatc tgttgtttat gtccagtgtc tcttctgatt ttggggtgtt catgctgcat 176640
ggttttctaa tatacgtagt tctaatatgt tctctgttca ttactgcgaa tttctgctcc 176700
gacccctggc cacctacaca aaccgcaatc cccagcatag gcaacatgtt agtaaccacc 176760
cggagggggc gactgcaagc catgatgaag agctgggtct accatgtacc tgacctgtgc 176820
tgcaagctct ggcactagag ctggggtcac agggccctag ataagtaatc tcctctgaga 176880
atagtcagat tttgtcaata catacaattt tctcttagca gaaatcttgg ttgtattaat 176940
aaatgctgtc attgacagca ttgctttgta tttgtacatg gatcaaatga aagaataaaa 177000
gtcaggaaag tgatgatatt cattccttgc catttactta atatgccatc aaactttccc 177060
actttcctat tattttttca ctctgggagt ttaggaaagg gaattatatg actacctgga 177120
ttctcatatg ccttcaacat atttttaact ggatcatttc atcttactct ggatttgtgc 177180

ctaaaatctc agtgctaagt gctctatctt ctaaattgag attccacctt ccaaagatgn 177240
actggtggtc ctcttattcc tgattatctt cccagctcac actccccag atgaaataag 177300
gagagtgctg tgggaggaaa gttgtggtag aatcatcagt agcagtgttt acggagagag 177360
attattcctc ctttgccaaa gcgcaagaga ggacttccag agaatcactt gtaggagtga 177420
ggagtaccag tcaaacagaa agagactggc tcttctatct ggaagcttgg aaacccgtag 177480
ctctactcag ttcagccact agattacagc aagctgagtt ggttgtgaaa ggaagctggc 177540
atcttccttc tgaagaatgg tgcagatggg ggtagagtga acctgccctt gcgtgacata 177600
gacagttctg aaagtcagag gccagccaga gtacagctag cagtagcttg tcaagaaagg 177660
actgtcaaga agaatatttt ctaagattaa tgctgggctg ggggaacccc acccctttct 177720
ccttctctgc cacctcagac agtgcagagg ggagctcagc ggtgcccatg acagatgtta 177780
gaggaaggtt ggggaggcag agggctgcac aggcagaggc caagccaaga catgctcatt 177840
caggagagga ttaaaggaag gacctaagga agacaagatt ctctttctct taggacgtct 177900
ttggctgcgg aagaccagag taaaactctc cttatacaaa aaagaaatca cacaaattat 177960
atgttaaccc tatcaacaaa ggacataagt gctgaatggg tgtagatcca aaccaaacta 178020
gaagggaggt cagtggtttc ctaagataat accttctgct cctaactctc cactctgacc 178080
ccaactttgg aggagctaca cccaaaagaa gaagatgtgg agcaatgaaa gaaaagacag 178140
ttggttgctc accctgtaat ctcagcactt tgggaggctg aggtgggtgg atcacaaggt 178200
caagagtttg agaccagcct ggccaacgtg gtgaaaccct gtctctacta aaaatacaaa 178260
aattagctgg gtgtggtggc gcctgcctat aatcccagct actcaggaag ctgaggcagg 178320
agaattgctt gaacccagga ggcagaggtc gtagtgagct gagatcacgc cattgcactc 178380
cagctctggg caacagagca agactccatc tcaggaaaaa aaaaaaaaaa agaccatgtc 178440
ccctccctag ctcaaaacca tatgtcatac tgtcatgtaa tggggtgagg aagaagggag 178500
gtttgaatca gatatgagac tagactgcac tgaagtaaat ttttaataac caaagttgac 178560
caaaaagtta gggaattaac tagggataac aaccagtctt ttatcctcat ttataatcca 178620
actttcctat aattgaggat gactgatttc actgttggcc ttcccttaca ttccacttgg 178680
agcattactc agtgagaggt agggcatccc caggcacagt gagaggtgag acattcctgg 178740
cattctgtca ccgtgaaatt tccagtcctc atcttgtgtt ttctgtaatt cagctctaaa 178800
gccagccatt tcttcaaaga gtgctgattt gttttattgg agaatggtat ttagggacca 178860
aaancctagc actaggtttc tcattgtttc cagtatatca gtgtttctag gctctctcag 178920
caagaagagc agggaaatac atgtatgtgt actaactcaa gtacacacat acatgtattt 178980
agctagtttt gtatatattt acctatctat gcatgtgtct tacataacca tagaaccatc 179040
tgccttgata acttgtactc aggtattaac aatatactaa ttttgttaga ttgttactgg 179100
gacatgagtg cagtatatac atgttatttt ctttaatgtt atccctattt ccacttgttt 179160
ttaataaaat atgtggatta atgtgaattt tcatagtgta tctaataagc ccttttatca 179220
acataggaga catttaaaaa ctattcattt gaccacagac atacacaaaa atgataattg 179280
gaaatgcatg attgtggcca gaaaagaaat tacttaaggg aataggacac tgctggggtc 179340
caagacccca atgttaagac tgcattccaa agtacgtatt ttccatgcct ctaatttaaa 179400
gtgtcatatt tatttgatag taaataccat tactgataca acgttacatg cacactaagt 179460
gcaacaaata agggaatttt ctatagaaaa tgagagccag agttcatacc atcagagaaa 179520
gaagttataa atgagcagag gggagatgcc acaggagaac ctgtggggct agattaggag 179580
tatcagtaag aactcagctt attttaagag agaggatagg taggtagtta catgcatata 179640
tatatatata cctgcataca tatggttgga gggttatgta agatacatac gtaggtaaat 179700
atatacaaaa ctaggtaaat gtgagagagt gtgtgtacat gagttagtat acatacatgt 179760
atttccccgc tctttctgct gagggagcct agaagtaagg atatactaga aacaatgaga 179820
aacctagtgc tgggattttg gtccctaaat aacattctcc aataaaacaa gtcggcactc 179880
tgaagaaatg gctggcttta gagctgaatt aaagagaaca caagatgagg ataggacatt 179940
ttgtggtgcc agaaaataaa gaagacctaa aaaggaaaaa acaaacaagc aaacaaacaa 180000
acaaacaaaa tagatagaag tatgtctgaa gactagagaa gccgactgaa agtgccccca 180060
gtggccaaag caggaagact ttgagcaatg aaatgaataa ggaaagtact gggttataac 180120
ccaaagaata caataaatag tcacaaatac atatttatac agatacatga ttgaataaat 180180
aaataaagga ggagggacaa ctgattctct cagaataatt ccaagtaaga aatgtagaga 180240
aatagaaata atagaataat aaatgaagta aacagaaaat tgccattagg aaactattgt 180300
tgtaattgcc acagacaaga gctgatgaat accaaaatta gtatgtgaaa ctttaaggag 180360
aaataaaaca tttgcatggc ctcaaagtgt cctctccaag tcttttttta attattgtag 180420
cagctttaac atccacccac aaattctttg atactcctcc ttcctaagag ataaagcttt 180480
aattcttcta agtgtcagtt ggagtcagtg acttgcttca agccaatgga gtaggaaaag 180540
agaaacactg aatgtaactt tataatgcag aaacctggca gacatcacct tacccaagta 180600
aggacagtta acctcaatgt catatacaca ctgatatgac gagaagggcg tatcatctcc 180660
atggtatttg tcccaaaaat gtataagctc agtctaatca tgagaaaaga tcagacaacc 180720
caaaattggg ggacactctg caaattacct gactgatact cctcgaaacc atcaaagtca 180780
ttaacaacaa gaagaattag aggaattgtc acagccaaga gaaacttcag atgtgacaat 180840
gaaatgcaat gtggtttcca ggatgagctc ctggatcaga cgaaagtcat tcgtgcaaaa 180900
acctgggaat tcccaataac ttctttagtt aaaagtattt gaccaaggtt aattttgtag 180960

ttttgataaa tgttctatgg tatataacat gctgggtcaa ggtatacagg aactctctgt 181020
actatctttg caactctcat gtgagtttta aattacttca aaatagtgtt tttgtaaaca 181080
ggatgagaat tttctatggt aagctgtaat aatgcaacat agaaattagc tcctgtgttg 181140
ccttgttcna aagnatatgt acatctgtgt agctttctgc catgaatacg ttgtatcaag 181200
tttatacttt attattcttt tttgatcttn aaaaagctgg gaacacacac tttatcaaga 181260
gccaaatgct ttttgtgatt ttgtttncta atagaaaatc tgatgtaact tgtcattact 181320
cctctttcat ctttgccagt aatataactc atctgaaatc ggcagacttg cttgtgctgg 181380
ataatataga tgattccata ttgtaaaggc tctaagaaaa caataataga atgaagagaa 181440
atattacaaa tgatcctctt ctgcctgttg atgggactcc gttagtgttg ccttcttgtt 181500
ctcatctcat caccattgga ttatcattgt attgtgtgca caatttgagg ccacagggcc 181560
acagagagtt gtgcccttaa ttaaaaatgc atcatgctag taggggaaaa gcacacgtaa 181620
ttagaaagtt atagtgcaaa cccctgagca aggtgcaaca gagggagatg tgactaaatg 181680
caaggtgcac agaggaagat gtgcctaatt tggtttagag gcaggtcctg gaagatgtgt 181740
ctaaaaatga tgcataaaat tttcttagga aaacgagaga aaatatccca tataagagga 181800
ataacaggca caagattcac aagtaaaagt gtaccaattg cttgaaagtg ttgttttggt 181860
gtggcagtat acaacagttt tagaagctca agctctggaa tcaggaaaat tgttgtcaaa 181920
ttccagttct gccacttcta gctatgtgac ttcaggcagc taattgaagc tccatggacc 181980
tgtttcctca tcgatgacat tgcataatat ggagctaatt atggtaccga catcagggag 182040
ctaattttta gggttaaata atattagcat aaagtgttca gcatcatatc cagcacgcgt 182100
gtttactgct tcacctacca gttgttattc tgagaaatat tcctggagaa gatggttttg 182160
gaaggattta taagagttaa ataagtgtat tgcaggcatg gggagcagta tcttcatgtg 182220
acatccctgt gtgagggggg cacagaccta caggttagag catagagggc aaagcactgg 182280
aagacccgct tatgccatgc taagaagatt agaatttctt ctgtacataa ttgatagtct 182340
ttgcattact ttaaggacca gagggacata aaggtatttc ttttctagat gttctaaaag 182400
attggtttaa agggaaaaaa ctaagacaga aagccagtta ggaaggtttc aggagtctca 182460
acgagatgaa atttggatga ccaattgacc attggattca gcaaaatggg aaatacgctg 182520
actttgccaa gagcagcccc actgaggcag ggggggacac ctgagacagg agaggctgga 182580
agaggggtgg gaagcgatat aatggtgctg taagcatctc tttgaagaag caaccttaaa 182640
tagcaaagga ggcatgatca ggagaaatgg tttaactgcc tattttcgtt ttcattttta 182700
caaccaagag tcttgagaaa acaatccaat caacatagca aacgagaaga aattggggaa 182760
agagggagag gttgagttgt ctcagattag ccagagggaa gggactcaag cacacatatg 182820
tgtcacccca ttcatatggg tgaagtcctc ctcctgtaga gcaagcagtc actgtggcat 182880
ggtgatagct ttggtttgta tagcacattt ccccttgagta attcaaatta caggcatatt 182940
gctatctcac ttacacgtct ggcatttata tgaaagacta aagatcctca ttttttattt 183000
gagaacactg aaatccaaag ggtgaagag attataaaac tgaacaaaag tttaaatcca 183060
tgtcttctta ctttgttttt tactgttttt acttatgcta tctctgatgt tttataaatt 183120
aaaaaaaaga gaaaaattac atgggagtgt atgtgcgtgt ttgtgtgtgt gtaagtgtgt 183180
gtgtgtgtat cctgaattcc ctttggtgtg acatttttcc ctagcactta acgaagaatc 183240
atctcacaat aagaaccaag tatctagttg acatgaaact ctaatataat ttcctctgaa 183300
gtggagcaaa aaagatatat actttttttt atcacactga gcatatttct atgactcagt 183360
aaattctctg atggaaacaa ctgtgtctca gtggagtaac aaaggacaga tataatataa 183420
aatagagatg ttntgggacc tagaagaata aacctacaaa atcaatcttt attcatcacc 183480
actgtctcta agaaattatt aattacatta atgggatatt aaaggacaat aatatgagca 183540
aaatgtgctc attcaacata attaggtatt ctggatttga atagtttttt ttttctttta 183600
ctaattcatt agagttgttg gttagcttag ccttgctgca aatattatct atgttgttgc 183660
acaaagtaac cttctgaagg acatttaaag cttgtaagat cttaagggtt gtgataacag 183720
catgaaagta aacattttat gcacacttca gggatatcat aagtggcagg aatgcaacag 183780
tctgttataa gtgctctgct gagctcccaa accaacaaac acaaaacagc cacttaattg 183840
cccagtgcat ccgcccctcc gaaaacagag cttctcttca tacttactta agggacatgc 183900
tgcttggacg aatgagtaac cagccaggat acagcacatg cagttaatca gctaatgtta 183960
tgaacatttt ggatgtcagc tatcacataa aaatgtagct tttatggccc ataggctaac 184020
caagaaatga gccctccaat ttctatctgt ggcttcagtc gatgcgtcca aataattgaa 184080
aaccccactt tgaccttgga tatactgggt gtattaaata tttttgtaaa acggggaaaa 184140
attagtgact taaacatgtt ttttgatgaa acgtttgaaa actgtacagt atcgctgtct 184200
tctatgcact aatttgactt ggttgccttt tgtccacagc agaaaagaca gtgcccaccc 184260
acctgaagaa attctgtatt ggatttcaca ggccagtgag ggatattctt tgtatttgca 184320
tattaaggtt ttataattac acagaggtat tattaaaaac caacatgtgt gatcattatt 184380
ccagatgccc catctgtttt gactgtgcga tttgggttgt aaaatcacat atacaaatta 184440
gcaagagaaa ctcccttgaa aaataatagt ttacgcctta gcaagactct atgttctgaa 184500
ttgctatgtc atatatatag atacagaaaa aatagctaaa gccactgtgt aaagaagcaa 184560
agagacttca gccttaattt attcacataa caaagaggag ggactccttt cttctgattg 184620
aagataatgt ggttattttt cctatattga acaaccaaaa aatattctac agttacatca 184680
actcacaaat ctatgcacag ttttcaaact aacacggact agatttcaaa atgagaatct 184740

cccaagtctg aatgtttttt aaattgtttt cttttgggaa atcataccaa aaaattacaa 184800
aaatttttat ttaatgtaat tcttcaattc agttattcct cacagcaatc agaaaggaac 184860
tgaaactgaa taatgtttga atattttaac agaagaaagc acaggctgaa atagggaaga 184920
tttaagcgat gaggcagaac acagtatgtg tcgtgcagag caattacacc cgaagtctct 184980
tgagaagtca gggaaagcag aggatttcag ccgcccgatg tgtaatgata tctcagccag 185040
gcagatgctg agccgtagaa tagcactgag cttagacgac ttctagtttt aaagctcaag 185100
gtttgaagtt tgtttctttg attctaattt cattcttgca aatgtgcttt tctttaccat 185160
gtgaatgtgt gacaaagcta tcatttgatc tctcggttgc agccagactt catgctcagc 185220
tgaggccatt ataaacttcc tttgtactgt tctttacatt agatttttca atatgagaaa 185280
taaaatccta ggattctcta cactgtgttc ttttccttca agaatcatga tcttaaaaac 185340
actttttaaa tggacattta caattataat tactttatgt taaaagatct tcttttcttg 185400
gtatagtata atgatcttaa aaattcataa ccacttctta taatatatat tattcattca 185460
tttgcatcaa agtaaattta atcttgcaaa atctttgctt aaatgcaaaa aaggggaaag 185520
aatttggata aaaggtagaa taccatttca ttacatttta aagaaactta atagtggctt 185580
caaaataaca tgtaatactt tgtgtatcat ggtcacctca tcccccacca ccaacacaca 185640
ccctgtctac tcccatctta ctctttcttc ctttttctgt gtctgtagct tggtcaaaat 185700
catgggttgg gaaagacgtc aaagtttggg tggctgatag tgccgaagta agaatggaat 185760
aattttgctt aactgcatca tcttcccttt tgaaaaactt ttaactgaac tctaatatac 185820
atatagaaag aggtatagta tacatttttg taaactgaac acacccagca ccctgctcag 185880
gaaatattga ctgtgcctta gaagctcctt tccatactcc ctcccgctcc ccgccacccc 185940
cacccaggac gaccactctg cagacacccg gcagcacacg tcagtgccac ctgctggttt 186000
aacttcctcc acgtggaatg gagtacatac aacagtatgt actctgttgt tgcgtctggc 186060
tcccttcact caatgttatg ttgtgaaatc cacatttctt acactgagat gtagttcact 186120
cattcaaata tttaaataaa cccaaatgta ttttcccact caactgctag tgggcatttg 186180
gacagttcac attttgggac ttttaacaac tgacactgct gtggccaagg gagggcttgt 186240
gccttgtggg tgtgcacgca gttcttgcag gcacgcacct aggagaggat ccacagggct 186300
gctggattta cctgtgctca tttttagttg atgctgcttc tctcttaata acagcaaaga 186360
gtgcatggga actgctgtcc agaagaacaa gtccttgctt ttaggaggtt tgattcagtg 186420
cctcagccat gagattcaaa atactcaccc ttcaagcagc cgctcatcct tctctgtgtt 186480
aactggccct taatgcacaa ctgtagggca atgatgccag gtttgcacaa tcgtgaagaa 186540
ccttctgagt ctccaaaaag agcttcccgt ctgttctttg tagtttcaca gcgcaatgac 186600
caagtngtgc tgagttctaa ttcatttatt tacaaagagt tctatttnct actccctaga 186660
atgtactata gaagtaaatt taaaatggta tcttgttcct aaggagcgta caagctttgg 186720
ggagtgtaaa tatctctaat accaattaga atgcgggtga aacccatttt taaaatgtgt 186780
aaacaaggtg actgttggta tcctacatat gttaaacaat atattcaagg atattcgata 186840
aaatataact tgccatttct ccaacatgtc cctttataat tttttttttt tttttttgag 186900
acagtctcgc tgtgtccccc aggctggagt gcagtggcac aatcttggct cactgtaagc 186960
tccacctccc gggttcacgc cattctcctg cctcagcctc cctagtagct gggactacag 187020
gcgcctgcca ccacgcccag ctaatttttt gtatttttag cagagacggg gtttcaccgt 187080
gttagccagg atggtctcga tctccttacc tcgtgatccg cccacctcgg cctcccaaag 187140
tactgggatt acaggcgtga gccactgcgc ccagcctaca aaatttttat atctgtactt 187200
tgtctaaaca ttcattagtt tgtcaacgac agacatcagt tcaggttaaa cgacacagga 187260
gtctaatttt attctcttct gtgaatctaa ggacacgcat actccacact aacaaattta 187320
ttcaatcaat caatagatat tttatgctta tctctctacc cttcaggata tatctaaaat 187380
cttttctttt atttcccagt tgtctgctgg agagtttagc ttagagttga gttccgccac 187440
ctgatcaata ccattattcc ttcactgaat atttgctcag ctcaccttgt gtccagtgcc 187500
atgccagcca ctgcccgtgg aataatgagc agagccctgc atggttcctg tcctcaccaa 187560
gtttacagtc tcagggaaaa gagaactgtt gtgcaagggg tcacacaata tagtgtcaaa 187620
cgacagtacg agtagggggt ttaaggcatt cccccaaaat ttttgtccag ctggatcctc 187680
agaatgagac catctttgga aattgggtct ttgcagaggt aaccagttaa gttaattgtt 187740
ctggatggtc cagtgtttgg catctataag gaaaaggaga gggggattca gattcagaga 187800
cacagaaaca cgcaggccca gaaggccatg tggtgacaga agcagggatg gggtgttaca 187860
gctacaagcc agggaactcc gagcatggct gagaatcatg agaaacaaga gaaaggcaag 187920
gaaggactct cgcctggagc cttcagagag agcatggccc tgctggcgcc ttggttttg 187980
acttctagcc tccagaatgc tgagagagta aatttctatt gtgttaagct aatgatctag 188040
tggtaatgtg tgacaggagt cctaggaaac taatgtattg tggattcaat tgacccgtaa 188100
gaaattgcag atgttcaact gctttgagcc acacaaacag caatattata ggattcatcc 188160
taccatgtta tgagcttgta agaggaaaac tttgtcagag aaagcttctg tgaggaattt 188220
ctagtggaac tgaaatctag aaaacaagta gaatttgatg gagttcaaga gaaaaagagg 188280
aagaaagggg aacccagcag agggccagca gagggccggc agaggtgggg gaggcacaga 188340
ggttctgagg cagagggtgc agatgtttcc aagcaactga aggatgtgac tggaacccgg 188400
tgtggggata gagctagctg aggctggaca ggcagggagg ggccaaaaaa aggcctctga 188460
gcctagattg aagattgaga ttttgaccag gcatggcagc tcgcacctgt agtcccggct 188520

actcaggagg ctgaggcaag aggatccctt gagcccagga gtttgaggcc agcctgggca 188580
acattgcaag atcccatatc tttaaaaaag aaaagtttat atttttgttt ttgtttgtta 188640
aaacaaaagt ttttttgttt gtttgttaaa agcagtgggt agccaggtgt ggtggtgcat 188700
gcttataatt ccagcgactc aggaaactga ggcttgaacc agaaggcaga ggttgcggtg 188760
agccaagatc gcaccactgc actccagcct gggagacaga gcaagactcc atctcaaaaa 188820
aaaaaaaaaa aaagagagag agaggaagaa aaaggcagtg gaatgccatt gaaaagtttt 188880
aagacagagc gtgagaaatg gcaataaccg cagaatgaag agcagattag aaagagctga 188940
agcagatgga gaaagcactg caggaaatgc aggaacccag tggtgcattg gccagggagg 189000
cactggtggg gatgcaggca gtgggcaggc ctagaggctt ttaggaaaga actctgtgtt 189060
ctcatttcaa tgatatgtcc caattaatat accatttgcc cttgtgtctg tttgaagaca 189120
ttgaattctc ataaaatatg cataccagtc ctgatacatc tagagccccc aagtcaactc 189180
ttctgaccac gtgtgtctat tctgctgccc gtgaagtctc gtggagtaga agtagtgcct 189240
tcattttttt tcttatttct ctctttgctc tataccttgt gtttccgagc actctgttcc 189300
tatatctgcc agagtggcca ttctatttta ttatcattgt ctctttatag aactatctac 189360
ccagttatac tgtgggctcc gtgagggcga gtgcagtgac ttatttcttt ttatatcccc 189420
tgagcttagc acagaacctg acctgtaaga tttgatttat aagtgacagt atcaatgtat 189480
aaataaatga gtcagcaaat gcatgaatta acaatacttt atttctgctc tgtcttatca 189540
cctttggcct gatatacctg ccttacactc tgatctgatc taatagatta actgcttttt 189600
ctacttctgc tctatttacc actgacaagt atttttctac tagttgtatc aacttttaga 189660
aaacaagact cttcctggta aatctcttta ctcgtgtgtg gaatcatttt tctaactgtg 189720
tacctctcac agagatgcta ctacttttttg ttttgtgtct ttgggagcta attccttacc 189780
attgtcatca tcataaccat ggaactgttg agaacgtgcc attttgcaca cattactgcg 189840
ttgagcactc cagttaggaa gggaggaagg caacgaacag cagtgactcc ttctccaaat 189900
aagattacca tctaagacag aaaaaccaaa ggctacccac aaaatcaaat cagacttaca 189960
aatggctaag gtaaatgttt atgttacaca aaacaaaaac ataagagcga tttctgcagc 190020
ctaataagaa aaccagaaat caggatgtat aatctcatca tgagctcaca accaatagga 190080
tagttgagtc atccatcata ttgaaatggg aatgtttccc caaaaagaag ccctcctgtt 190140
ctcccttagcc aatttgtatt tccacatata ggagggatgc attgccatct ctcttcctgt 190200
tttaaaagct ctcttggcct ctaattctta tctttccata attcctaaaa caaattatag 190260
agtaaaacca tacccaacaa ggaggtaaat gagtggatga tttttgaaac ccaccccccca 190320
aatccctcca aaaaggcagc ctgaattaca tgtactcttt gaaaggcatc agtgttaaaa 190380
gatgcagttt gggaagccgg gacagaagac agggcagacg tggggaaggg tgttccactc 190440
tgcagaaggt cctggagaag gggagaggta aacagaagta aagagggact ccaagagacg 190500
tcggcactgg gagagaaaaa gagagcatag gaaagtatag catgaattct ggaagccaca 190560
gtccaccttt gtagtatctt agttcagcct tgttccagtt tatgatgtta aacctgagct 190620
tccctgaaac tcaatttgta gaaatataaa tatacactct taaataaatc catttgattg 190680
tcattcctca caaagatatc atatggcatt taagttcgtc gtgtccaagc atgtttgcag 190740
ccaaatggaa aactcgctgc atggaccaaa tgtggtattt gtgttttgag cttttctgtt 190800
ttaatctgct aggcgacata accaattttc ctgccattgt ctctctttac aaatgctact 190860
acttagtatg attctgtagt taattttttt taaatttttc atggttttta aaaattttta 190920
attctagcgt taggcatgtg taaacaaatt ttattgttta aatatgcaag taatagtaaa 190980
ccattaaaaa tgtctgagat gaaaagagac ttgtctttgg gaataaaatg acataaagtt 191040
ttccttgaat ttttttctgg tttantttt ttgttgttaa gttttgtttt tgttttttct 191100
gttaactcaa aagttttagc tcatagagca atttatggcg ggcaagagag gtatacttgg 191160
taggcctttg ctgccatcta ctggcatttc atgtaattac ttattgcttc cattctaaaa 191220
ctaattttgt gtaacgtata taatagtatt ataggttccc ccttttnttc ttctttccaa 191280
tttttcttta gttaaagcaa agtgaagcaa atgcggacac caaagaaaag ctccctttac 191340
gcctgcgaat ctttgaaaaa tttccaaaca gacctcaaat ggtgaaaatc tcaaagcttc 191400
cttcagattt tacagttcct aaaatcaggt accaaaatac ttctttaatt gcatatttca 191460
catatattgt aaaagattca tcatcttaaa tctgatttaa cccttctttt aagtatatgt 191520
gggttgggtt tctttctatt ttattgtcat ctttctacta cctctacctc tatttaaaat 191580
aaataaataa ataaacaact ttgactcttt taagcaatgt attcacagaa gagtcacttt 191640
ataactgcag tagcctaagc aagaaggctg cttcccatcg cttctctctg ggatgactcc 191700
tcgggaatgg gacatcccag gaaactggag aagctgctca gtgttgcagg gatggcaggt 191760
agacatcaat ctcagacccc aaccgtatga gactaaaatt gcctctgtgg ccctttttatt 191820
ttcaagcaaa cagggaaacc acagctggcc cagtttgtgt actactacag tgtttatgga 191880
gcctaccatg tcaggttacc atatataatt tgccttccac tttttaaaag aagaaccgtg 191940
gcgtttttcc actcatactg atttgatttt ctaccacgtt tgccctccag ttaagcaaat 192000
gcataaaaga tgctttaaac acctattttt aataataaaa ggaaattctt ctagttatag 192060
ctcaagaaat tacaaacaga attacagtaa tatgttacta gctttaaaaa taattctgtg 192120
gggggttata aaagtatgag actatatatg gtgccataat atttaaaatt cgggtagaaa 192180
tatatttgca tttctaattt taaaaaacaa aattaattga atttggtaat ttgaagagac 192240
gttacttgaa ttttcgttat gctttgaaga tcagatgaac gcacttgctt ttgagtctta 192300

aaatgtaaaa ttctctctta gtcattctta tgataaatgg acatttagag agagcaacta 192360
attcttccaa gtcttagagg gggaaaaaaa gtgttttatt accacaataa gattcaaaaa 192420
actgggctgg gtgcggtggc tcacacctgt aatcccagca ctttgggagg ccgaaccggg 192480
cagatcacct gaggtcggga gttcgagacc agcctgacca acatggagaa atcccatctc 192540
tagtaaaaat acaaaattag ctgggcgtgg tggcgcatgc ctgtaatccc agctactctg 192600
gaggctgagg caggagaatt gcttaaactc gggaggcgga ggttgtggtg agctgagatt 192660
gcaccattgc actccagcct gggcaacaag agtgaaactc cgtctcaaaa aaaaaaaaga 192720
ttcaaaaaac taccatcggt tactgagtaa tattgcttga aagttatttc aaggatattg 192780
ctttttaagc aggagtcact gagtttcagg aagctgttgc taatgaaata ttttagtcat 192840
gatctagttt tctatgatct tttatcactg ctttatcttt gctcaagcaa actcatcatt 192900
gcaaaatgct gtcacttgct cccttccact aataatgcct gatgcacata tacttcattt 192960
gcaaagggag agttttatga agcagtttat tgaccgccag caacaggaca catgttgcct 193020
cctgagaagc ctcccgacca cctcttcctc ctcctgcgac cactccaaac gctcagcaat 193080
tgaggacaac aaatacatcg accaaaaagt aagatcngct gtttcgaata accaggcttc 193140
taaaaagcat ctatgtttga gaaaagaacc gcagagttct ttagggtcct ggaatccaat 193200
ttnctcaaga gaataatgaa aanaaaatga tttcatttgt aaaagatatc atgaaaccta 193260
acatttttta ctctatgcat tgaaattttg catttgcccg tgtaagcttt tgagctgcgc 193320
cacgtggcag ccatgcgact ttgtaaacat caagagcctc ccaaatctgc attttctcan 193380
gtgcggaata aagggctggg atctccaagg ctcagtccag gtgtaatgac ctgccatctc 193440
ctgcaagtac aactgcttat gtctacttat tacatcagag aggatacgag agcatgtttc 193500
tctaaaatcg gtcgaaagaa ttgccattta ttcaatgctt agtatgtggc aggaacttag 193560
gtgcaagagc ttattcatgt tctagtttac ctatttcttt atcatttgaa aagtagaagg 193620
gaaaattttt ttcagattcc acagcaaagt atcagtaggg aaccacatat aaataaagca 193680
aatttaggta ataattttaa agaaatgtac tttttcattt attcactttt aaaaaaaata 193740
atataagggc tgtgtgccgt ggctcacccc tgtaatccca gcactttggg aggccgaggc 193800
gggtggatca cttgaggtca gaagttcgag accagcttgg ccaacatggt gtaaccccat 193860
ctctactaaa aatacaaaag tcagccgggc gtggtggtgg atgcctgtaa tcccagctac 193920
tcgggaggct gaggcaggag aatcactcga acctaggagg cagaggttgc agtgagctga 193980
gattgtgcca ctgtactcca gcctgggcaa cagagtgaga ctccatctca aaaaaaaaaa 194040
aaaaaaaaaa nangacntag aatgtattat ctaggtataa taagtaaaca tatttatata 194100
tacacacata gacatataaa tgtatatgtg tgtatatatg tatgcatagt atcaaactat 194160
atatatcagc ttggtcacat aatcaatgtc agttcagtta gaaaatgaaa gattaataat 194220
tgtcctttaa tattaaagag attaaacagt agagtttgca aaaacatcta gttcacttgc 194280
aagtattcca ctttggcatt cctagagtgt tttttattcc tttacattgt tactgccctt 194340
tttcttttgc ttatagtgaa atccatattt ctctatgatc atgtgacaat aaaatgccaa 194400
ctgtttcatc ctccaggatc ttctttctct ccgctttctt tcccttccca cagggatgaa 194460
ccctttggaa ttccctcacc tttctcttta tctcgcctcc ccttttttctc ctctttcttg 194520
cctccccttt ttcctccttt ttcacctccc ccttttctcc tcctctttct cacctcccct 194580
cccctaatgt gatactactc ccgtttacct ctccccccagc tcccaggttc tctgctgtag 194640
ttcagtcatg ttgctcttgc tgaaaatgat gatgattttc tttgcaggat gtctcttctt 194700
tttcaaggat ttccatgtgt tgattttcct tcagccatct agagatgcta ttctactatc 194760
tttgcagaat ccaagattgt aaatgacaag tctgatgcca atgtcatttt tttttaagt 194820
aattagcctt ttggttggta gacttggttt gcttgtttct tcaggaaaac ttgtaagatt 194880
ttctgtgtat ccttggagtt cagaagcttt cttagaatat accttatatg tctttttttca 194940
tcagaactgc tccaactaga gtcttttcag cttgccaact aagatatttg ctcagggaaa 195000
tttccattta ttttgcgttc aattgttact cttccatctg cttctctctc ccctgggatt 195060
gccatctctc tatttttgtt ctgtgcttgg tcttccagga cactaattag atttcaactg 195120
aggtcatcct ttgcttctaa atttctcgtg aaaaattatg atatggacat atgaccctta 195180
actatcctta aaggcttgca tattaccctt agctgtcctt agaggcttta cttttatctt 195240
ttatttttat tttttagag atgagatctt gctgtgttgc ccaggctggg gtgcagtggc 195300
tattcacagg cacggccata gtgcactata gccttgaact cctgagctca agtgatcctc 195360
ctgcctcagc ctcccgagta gccgggacta taggcatggc caccatgcct ggcttttttg 195420
atttttttaa attaaactta tcacctctct tcttcacagt tataatccac tcaacactgt 195480
gcatggggca tggaagcaga aggtcatatg tcagtggata aacaagtggg aagatagtgc 195540
cttggcttca gccagtcagt ctgaaattgc agtttgcgtg gactagaaag caggttgttt 195600
tgttttgttt tgtttccaaa gcagattgta agccagaagt ctgggctgtc cttgtgacca 195660
cttatctcca ggaagaggat agagagctga tttccattat ggccaaagtc acaaaaccat 195720
gacatgcttt taaaaatcac tggtaacact accccactgg aaacacaaaa ttgaaatatg 195780
agtttatttt ctctctaaaa ggaaatgtga ttgtttctca catttcctga acacagtatt 195840
ttgatttggt gaggacaaag ataaaaagag tcttgagagg catgtgccgg gctgaggagg 195900
agtctaaaga gaatgtggcc cgcccctctg tgcgcccgcc cgaagcgaac cctccccagg 195960
tacagttgga ataacacgtt ttatcccata caacatgtct tattgctccc acttggaaag 196020
tggaatgtca aaaacatact ttttcttcat cagaggaccc tattatggga gtggcaagac 196080

aactgctcct gttactatcc acgctttctt aaagctttga ttcgatctag gttgtcagaa 196140
aagtagactt aattcttttc aggatccacc attttcagca atagttccca ccagttcttg 196200
gtgttcctca taagacctta ggatttgtca accttgttga gtgcccctgg gccagggaaa 196260
tctaaagcag aaaatccctc ttcaaaggat ttcagtgata aggatttaat ggaagcacaa 196320
attgcggaga cagcattctg gcatttgtat tatgaatacg gcaagccttt ttcattttta 196380
ttaagaaacc cgaaagccac ttaaaaacaa gagcgcataa acacagaaca aaaatccccg 196440
tcacagaccc taccaccatt agttcccctt ataaagtatt tttcagcgag cacttagaaa 196500
tttccattaa aatcccaatt gcctttggaa atcctagtca cagtaaagtg acaggcaggg 196560
ttcttcactc tacgtgnaaa aaaaaaacca cccagccctc tgcaaattgt gcaaattgag 196620
gctgtgtttc ctcagtgact ggtatgaaag aaaaaggaca ttagaggctg gtttatcggt 196680
gattctcata acatgaacaa aggaacaata aaataaaact gttctcttaa tggttatctt 196740
gtaccaggag aaacttctga cttcactatg ctagagggaa tcacctttct ctttccaggg 196800
aaaatagtga agccccaggt gcctgaagac tgcaagttca gggagaaaaa aacaacaact 196860
gacagcaacc ccagaagggg gctggggttt gatgaagcat tgcagaaaac acaaaagcac 196920
attaggcaag attttctccc agtaattacc caagctttga acagggatga gtaattccca 196980
tggcaacctc actttgcaga aaccatgttt ttaaaaatag ccctggtgga tcacctttat 197040
tatacagttt aaaatacctg tccttctccc agaaaacaac cagcaaacaa aaatctttaa 197100
aatcttgggg ctgacccaga tcctaaaagg aaaagacagg aaagagtgtt ttcctgcctg 197160
tcgtttgaga ctccatagaa acgaccttcc caagtggtca tttccccatc cctttatcct 197220
gctacctacc gttcctgtga gcaattgtga gaggttcctt ttcttcctga ttcaaggatt 197280
ttgacagggg cttttccagt ttccaagcag aaagaaatca agcaacaatt tctacttgta 197340
tttttcctcc atattattga aattaaactc tagtttcctt aagcagagtg gtgattaata 197400
aagtgagatc tgaacttgac tttccagtaa ccctgtaata aaagcagccc gctctgttct 197460
ccgaagtgag acatccagtc aaggtcaagg tctcacctgt ggttgagtct acagcgtctc 197520
actgccctgt ggcctgagga tggttctgtg ctgctgtctt ggctgatgca ccatttctta 197580
tgtcagaacc tgcaatagcc tgggaagcat gagtgaacct cctggagcca tgctaggatg 197640
agggctgtcc cagcacctcc acctcgtgcc cctgtacttc ccagcataga atcagagccc 197700
acataataat gaacagtgaa tattttctct gacagtaggg caatatctgc ccaccctctt 197760
gtcagcctcc tttctgctga agcatatgat agttctttaa taagccttca caatgaacag 197820
ctaacttatg tacagtaaag aggaaaatga tcattggaat gactatgttt tgtttaccca 197880
tttgatttta taactcatta acattcattc agtcattcaa caagcactta tagagcaccc 197940
gttattcatc tgctattctc tgggctgtgg gaattcaaca gtgagcaaga tgcgaactgt 198000
accctttaga agctcatagt ccaatgggag gaaatgcaca tacatgcatg aaagtaaaaa 198060
gtattactgg aaccatgaga cagaaccccc taagttcccc ttacagaaga agtgtgattg 198120
attgataaag gaagaaagca ccccaggcag agggggacat atatgcaaag gtatgcaaag 198180
catcaggtgc tcgatgtttg agagaaattg ggtagaagag tgattaagtg gtgcaggttc 198240
caaccctcat cagtaaagtg gagataattc tcattttcaa ggtttgttgt gagaatcaaa 198300
taatgtaata atgttaaata cctagcattg cacctgggct agcatatgtt ctgtgcctct 198360
caaaccagac aagaaaggtt actgttanta aactgtacca tgaagtcaga ctcagcaaac 198420
agatttctat tcatcagtta ggacaaaaga cttttacata aatttaacct tcagatgtaa 198480
cagtctagtt gagtgcaata tattaattac tgatcaattt ccttatggta cctaagtctg 198540
atttttttag agtttgattt ttgttgatat tttagttgct atccctattg tctacacggt 198600
gtcttcgaat ctgaagaagt aaattaggtc attctggtaa gatttactct tccgnttttt 198660
ttattgccat acagcatttt ataatgttct atatgattat agagtgcttg ataatttgtt 198720
cttgtaactc ctcacatagc aagttactca gcctgatcta taatttccag gatgaatgaa 198780
tgtttgttca ccttaaaaga gagagagaga gagagagaat gaacaaanga acgaacgaac 198840
acagcctgnt ttcattttta gngcttttgg tcttttgaaa gaattttttt tttaaataat 198900
ggcttagctt ctctgcagta ccattcaaga tgtatctcca ggaaaagagt gacagatgac 198960
atgatgctac catgtaacca tgggttgtat ctcaaaggga agaattggtg ttcctcatgt 199020
caaatatgaa aactctgtac acttctctct agcttaaaaa tgctttagta gataatggat 199080
tcccaattta gtcacaataa tggaaagaaa taaaaagatg cgtaagattt catcatcact 199140
ggccatcaga gaaatgcaaa tcaaagccac aatgagatac catctcacac cagttagaat 199200
ggcgatcatt aaaaagtcag gaaacaacag gtgctggaga ggatgtggag aaataggaac 199260
acttttacac tgttggtggg accgtaaact agttcaacca ttgtggaaga cagcgtgcta 199320
ttcctcaagg atctagaact agaaatacat ttgacccagc catcccatta ctgggtacat 199380
gcccaaagga ttataaatca tgctgctata aagacacatg cacatgtatg tttattgcag 199440
cactgttcac aatagcaaag acctggaacc aacccaaatg tccatcaatg atagactgga 199500
ttaagaaaat gtggcacata tacaccatgg aatactatgc agtcataaaa aaggatgagt 199560
tcatgtcctt tgtagggacg tggatgaagc tggaaaccat cattctcagc aaactatcac 199620
aaggacaaaa aaccaaatac tgcatgttct cactcatagg tgggaanctg aacaatgaga 199680
acacttggac acnaggaagg nggnacatca cacactggga cctgtcatgg ggcaggggga 199740
ggggggnnag ggatagtatt aggagatata cctaatgtaa atgatgagtt aatgggtgca 199800
gcacacaaac atggcacatg catacctatg taacaaacct gcacgttgtg cacatgcacc 199860

ctagaaccta aagtataata taaataaata aataaataaa taaataaata aataaataaa 199920
taaaacgatg cgtactataa aatcaaacca ttttttcttc ttactggtct ttgcagtaaa 199980
ttctcaagct aaaaccaaat atctgtgtgt gtgagcactc accctttcat ccaacaatca 200040
tttttgagga ccattatatg caaacagttt gctgggccct gagtaaacaa acacatcttt 200100
ctatattcaa ctaacattag agtaggaatt ccctctcaat gattattttt gcagaatatt 200160
taattgtgta accattttta ggttggaact atgtatcagc tgtttgtatt ccttagtaaa 200220
tgcgcattga gagtcaaaag taaatcagtc taggattaag aattttttat aggtaattta 200280
aaagattttt aatcgcattt tctgtatcat gcacaaaaga ggtatcaagg agttaaggcc 200340
gctgctaaat ctatcttgga aatttacatt ttgctcttct ttttaaactt tttcctcttc 200400
tgactacagt tttacttata aattgtctct tctatcaagt gtcatgctag tctcagtgtg 200460
ttgtatttgt tgctgatgat ggtgttgagt ccaagtcctg cttccttgtg ttgccacaga 200520
ccttaaattt cgaatttcta cacccataat cccagcactt tgggaggcca aggctggcgg 200580
atcacctgag ctcaggagtt cgagaccatc ctgggcaaca tggtgaaacc ccatctctac 200640
taaaaaaaag tagctgagca tggtggcaca caactatagt cccagctact cgggaggctg 200700
aggcatgaga attgcttgag cccagggggc ggaggttgca gtgagccaag atcatgccac 200760
tgcactccag cttgggcgat acagcgagac tccgtctcaa aacaaaaaaa aattttggaa 200820
tttctaaatt cacaaacagg ttaaaagcat ctgacattta ctctagaatc acaagcccac 200880
tcaccttctt ttgtctctgc agatttccaa tctttgcctt cctgtttata ttagatataa 200940
tcataaagtc attttgtatt ttacttttgt aattaaaatc tatctagcgt atatttatag 201000
ttttgtttgt ttgtttgttt gagacggagt ctcgctctgt cacccagcct ggggtgcagc 201060
ggcacgatct cggctcactg caagctccgc ctcccgggtt cacgccattc tcctgcntca 201120
gcctcctgag tagctgggac tacaggcgcc cgcaaccacg cccggctaat ttttttgtat 201180
ttttagtaga gacagggttt caccgtgtta gccaggatgg tctncnattt cctgacctcg 201240
tgatccaccc gcctcggcct cccaaagtgc tgggactaca ggcgtgagtc tccgcacccg 201300
ggcatttata gttncttatc attaagtact gtatattaaa cctatctaaa tttgtttcag 201360
tactcgaaga tattgctttt ccctcgattt ttgcacagtt tcattatttn atgtgtaaga 201420
tgttggagat ttgaccaagt gaccccaggc tactgtgtta gtggccgaaa cattgtcata 201480
acattcacgt gcaaaaaaaa atcagatcca ttaagtttta aattgtttag tttctaatat 201540
tacaagctgt tcctatatat caagttagtt gattcatttc aaaataagca gtccctcttt 201600
gtaattttta cgtcattcat atttcttatt ttcaaggact aactacatta aaatatataa 201660
gatagaaaaa gtaatacttt aatattatga aattaattca attttcaaat atatttcaag 201720
caccaacaat gcacagtgct agacaatgaa agaaaatgta ataaacaaag aatacattat 201780
acatatccta aggagtttac aatctattgg aatttacaga aagataaaaa gatgtttcac 201840
acagggcagg gatcaacttg tgctgggtgc ctaggagtcc aaaggaagag cgaagatttg 201900
gtgtatgcat ggcatgcaca cacgtgcaca tgcatgttta tatgaatatt ttaactccta 201960
cacatagtgt ttttacaggg ttcagaacac atgatattga aataagttac aataggaaat 202020
cttttttttt gtttgtttgt ttttttgaga cagagtctcg ctctgtggcc catgctggag 202080
tgcagtggca cgatctcagc tcactgcaag ctctgcctcc caggttcacg ccattctcct 202140
gcctcagcct cccgagcagc tgggactaca ggcgcccgcc accatgcccg gcttattttt 202200
tctatttttt agtagagacg gggtttcacc atgttagcca ggatggtctc gatctcctga 202260
cctcgtgatc cacccacctc ggcctcccaa agtgctggga ttacaggcgt gagccaccgc 202320
acctggccta caataagaaa tcttgaccca tcatctaaaa ttctcttcac aaaattcaca 202380
ttttaagata ctgttcaaag cttcataaca tgatattata atggtattca cctttatata 202440
catccataat caacatttta atcttgctga tgctcacatt aatcaccttt taaaattttt 202500
tttctaagca taatgtcaat tccnagagaa agtttatttt atagaaacat cttgccacac 202560
acatgatctg tcgtatgcac tttgggatta attatgaatt cacgggtgca tgaaaattca 202620
aatacagtca aatgagatac caccttcgac ccatgcaatt ttaagtaatt gcttattccc 202680
aattactata ttttatttgg gagacttatt caataaagtg gctcgttaca ataattacac 202740
tacaactttc tagcagaaat gacattcatc aagccatatt cattggaccg tggttttgtg 202800
tcttatggtc atatgttttt ataaccactt tcccatttat tattaacctg ttaatatttg 202860
taaagtgaga aaagaaaaat ggagccactc aaactttttc ccagttagaa acagaaatgt 202920
attgttaaca ttgtaaatca aaaaagctat tgaatattta taatacttgt ggtgtgcttt 202980
gtcaaataaa tattgatggc ttcattctga aggtggtttc tagggtcaag ctatggttct 203040
ttaggtctaa gcattgccgc atcctaaagg aatctatatt caagataggt ctgtggttac 203100
ttctgggtga ttgaattaat caaattaata ttctgattga ttgagagttt tgatggatcc 203160
cagattttaa ggaatcagta agtgtaaaat ataaaaacta tgatcatact agctttaatg 203220
agttaggaaa tgtattgatc tctttcagtg cttcagagtc attattctga aaatcaatta 203280
tgcatatgta gaacaaacaa aaagttggtt agtttcgatt ttgcattagg atgtgagaat 203340
agcaagagtt tataatatgt gtaaacaaaa tatttcaagt caagcagata atattttgtt 203400
atgatatagt ttagaaaatg ctttttttata ctaagcctag tgacatttta catagacata 203460
atgcagtgaa aagatttgaa cactgaagct gcatgatcca gagttctagt aataattttg 203520
caatttatta tctgagtgaa cttagacaac ttaattttta tgagccttag atctttaatc 203580
tggaaaaaag agataataat accttaccta cctatgagga ttagtttgaa gattaagttt 203640

aaaatatgta tatatcacct cagcacagat caaggagcca agaaatacag atgtagaagt 203700
tataaaatgt acatttttaa atgtgcaatg ggcatctata aaattcttca ttcaatgact 203760
agagaataac attctaattc aagaacacat gtatgatttt aaatgtaacc atttaccaca 203820
gacttctaaa tatatacaca tcagtgtcct agaaaagata ttatttgacc aaaatgtaac 203880
aaaatcngaa acctaataag aagaaaataa ccacaaaaaa acctacacat tcagaaattt 203940
aaaactattt tcaaataagc acagaagaaa caccaatgaa aataagaaaa tatctacaat 204000
taaaaaataa tgaacaaaag atgcatcaaa cttgtgggaa gtagctaatg gcatttttag 204060
aggaaaaaat ttagccctga atgaatatct agtaaagaaa taaaggctta aattaatcct 204120
ccaagcattc atttcaagaa tttagaaaaa ttatcaacca aggaaacact aagatagcag 204180
aaagaaagaa ataataaaga gaaaagcagt tattaatcaa atggggggaa agaggtgata 204240
aataatatca acataattga aattttgttc tttggaagca gtaataaaat tgataaatct 204300
actttaaaat tcaacaagaa aaaattaagg agccttcttc tataaagatg acagaggcat 204360
tgacattgtt ttttaacctt gaattcccac atgaanaaca aaaagaatac caagatatca 204420
aaaccccaaa tccatagtta atatttacca taaaactcgg taatccacaa accctaaata 204480
aaagcaagtg ttgacaaaca gccacatgat ttgcatgata ttagtgtctg tgggaggaaa 204540
ccaagcttag ttacagagcg tccaatgttc atggaatggt agaacatctt ctttgctcca 204600
ttctgaaata aaaagtccag gagtggaatc aaaattgaac aggccaggaa caagagtgat 204660
aaggataaaa gcagatccag acaaaatttg tggggggagg aggattggga aacagagctt 204720
ggaaattcca gaaagaaagc taccatattt gatcgatata ttaaaacaac agaagaggaa 204780
actctgaagt tagaagagaa atactaaact gtttgggaaa actaatttttg tataaaaatg 204840
agtaaggaaa attatcaaag ccaaattccc tgcaaacgtt attataagaa aaaaacagca 204900
gaatactgtc tctcactgta gacaaggaaa gcaacctana aaaacgtgct ctaaccagct 204960
caaactgtag catactattg aaaaattagt taaaagacat taaaaattac ataaggatga 205020
aatagcaaca taaataaaag ttagaaaaac tcagaaatga ggtgccagaa ctgagaaaag 205080
ttataaatta aagcaatgaa aactaaacga gaaggaacat aaagagacct cagatgattc 205140
aaaagtgaga aatagaaaat ggagaagagg gcattttttta aaatcaaaga gaaatgaaga 205200
aagagatgaa aatgatttgc aagaaaaatg acaaattgaa gaaagtcaaa aatccgacat 205260
ctgcattata ggtgtcccta aggaagaaaa accacagcaa gagaataaaa cgtatactaa 205320
acttttctga aacataaaaa gatatgaaac tatatattga cagcacacac ctaaaaatat 205380
tggagtaaat gattaaaaac aatacataca ccagacaaat gacaggtctt taaatttttt 205440
aaaaatatat tatctagccc aaataagaac aagtgattta taaaggaaag aacgttagat 205500
tatcatgaga tttttcagca gcaatgcttt atgtcagaag aaaatagagt aacatatata 205560
ggatgcccag gcaaagaata tctgagccag attttttttcc atcctgaaaa ctgactttca 205620
actttcaagg tcagagaaac tgctttcaag atctaagaat atgagtaatg taagatacaa 205680
gtaatcttgt ttcaatgaac ctgtaatcta ctaaaaaaca tgcctcagat aacctaaatg 205740
aactccaaaa gggactctta aaccatttgg cataaaaatt cacattcata tgtaacaact 205800
aactacataa ggacaacatg aagagtgaaa tatgtaataa ttatttgttc taatatggat 205860
agagtacaga tatacaaatg aggaggtgaa ngggaagaag atatgcaaac ccttaagata 205920
ttcagtagtn ttcttggttg tgtattttca ttctgagact attgtctatg aaatgtgaaa 205980
taaaactgag tcactgtnga gacaatctaa ttctggcatc cctggtgctc ttgataatca 206040
agagtgctct ttgtggagaa aaaaggagat atagatatca tatagaaaag cttaggtaaa 206100
aataaaaatc ctaaagaaaa cgtggcatgt atacaccatg gaatactatg cagccatata 206160
aaagaatgag ctcatgtcct ttgcagggac acggatgaag ctggaaacca tcattctcag 206220
caaactaaca caggaacaga aaaccaaaca cagcatgttc tcagtcataa gtaggaattg 206280
aacaatgaga acatatgggc acagggaggg gaacatcaca cactgggtgc tatcggggtg 206340
gggggcaagg ggagggatag cattaggaga aatacctaat gtagatgacg ggttgatggg 206400
tgcagccaac cactgtggga catgcatacc tacgtaacaa acctgcacgt tctgctcatg 206460
taccccagaa cttaaagtat aactaaaaaa aaaaaaaaaa aatcctgggt catgaattta 206520
aattgaaaac atcagaataa acccaggcaa aatatttgaa acagtgacca actccatagc 206580
agtgagcatc tatgggctct agatatgttc ttgaaacacc atttttccaa ttttccaata 206640
agtaaaccag ggcttcttga agaaaatggt tgattttgaa taggaagtgt acaagctgac 206700
cttggagcat cttgtcatac taggaacaag gaagctttgt caaagactac gagggtcata 206760
taaaaagaac ctacagactg accaacttga aaaactccca atgagcacag tagagataat 206820
ttgactaaca gtaagaaatg gtaattgaca tggactgaaa tccattaaac attaaataag 206880
tttaaatctt gagttcataa tgacattntt ttaaatagta attggttacc ttgagcgatg 206940
atagaaaatc aaccatgtaa aattaataag taaaagtcga taatcaagca tttatcttgg 207000
cttttccata ccagttttgc tactgggtaa ccaaataata caggagggaa agcacctcat 207060
tataaaacta tcccaactaa taaatgaaaa agaaatgaca gttttgcaaa cggaatgaag 207120
tcaagggatc taagaaatga acatcaagac ccgtaacatc acaaaaagaa acacacacac 207180
tctgttcctt tcgatggaag aatctgacac cacctatggc cttgccaaag ggatgaaacc 207240
agagtctgta tgatgaaacc tctggatcca gctgccgatt ttcagaaaat agaagacaga 207300
agaacgtgtt gaactgtacc aagagtgtgc tttcagcaaa atccagactc tctcttggct 207360
ggnngtgcgg tggcttactc ctacaatccc agcatattgg gaggccacgg caggaggatc 207420

acttgagccc aggcatttca agaccagcct gtgcaacatg aagaaacccc atctcttaaa 207480
aaaaaaaaaa tgttacttag ctatatgtga tggcacacgc ctgtagtccc agctactcag 207540
aaggctgaga caggaggatc gattcagccc aggaggtcaa gactgcagtg agtcatgata 207600
attccactgc atgcactcca gcctgggtga cagaacgaaa ccctgtcaaa aaaagaaaaa 207660
aaaaaatcca gactcaaaaa ctataggtca aatggcccag gtttctctat tttaagggga 207720
aaaaagggta acatgtagat taattagaag acatcgccaa aaaatcagca agattcatac 207780
attggaatgt ctttcaggtc caaattattc aatcacaaat aactctatgg gaattgagtt 207840
gaagcttttg aattatgtgt taaagtatgt aattctggaa acgtgtttta acttagaagt 207900
tcaagactta aaatatctct atattgttga taatttaaat ttaatttact tgttttgagt 207960
tattaattct gtcaaagaat tgttatctga ggcagttgga tgttttcagt ttccaaacaa 208020
aaccgaagaa aatcttcttc aacgaatctt tgactttttt caaagtaaac tccttgtaat 208080
tccatcattc atccccttcc tttttattcc cacatctctg atatgtttac atccagaatg 208140
agcaattact ggctcctttc atagctgtaa tttacaattt cagatctgaa attactcatc 208200
atgaaatgga tctctgtatt ctttgttttg ctaattattt caggtgattg tcagtatctt 208260
ctagagccag aagagggcac tgcagagcag tgtagctctg agctctttat gtttagcaca 208320
gtctaggaat gccctttgcc aaaaaaaatt cgcaaagctt tactatagaa gtaagaaaaa 208380
agaagggaat aactagataa tcactatagg ccttctttat ttagtagaaa aaagaaagta 208440
tataacgatc taaatacata tctaggacat caaatgtaat atcatatata tatgtataag 208500
tagatgaaag aagacatata tatgtgtgtg tgagtccttt ccttctttta agaacatccc 208560
aaaggccagg tgtggaggct cacacctgta atcccagcac tttgggaggc caaggtcggt 208620
ggatcacttg aggtcaggag tttgaggcca gcctggccaa catggtgaaa cctcgtctct 208680
actaaaaata caaaaattag ccggtgtggt agtgcatgcc tgtaatccca gctactaggg 208740
aggctgaggc aggagtattg cttgaaccca gggggcggag gttgcagtga gccgggatca 208800
caccactgcc ctccaacctg ggcaacagag tgagactctt tctcaaaaaa caaaacaaaa 208860
caaaagaaca tcccaaagct aaatctgatg tttttctatt ataatattga ttctcctgct 208920
acctctggca ttagttataa aaacagttct caagtactct cctgtatgaa ttaatactgt 208980
attttgtctt agttcaaatt caggacaaag gccattcagt agccttttag caatcttgtg 209040
cagggcagaa atcatttagg caatagtgta ccagatatta tcccaaaagt tttttcctaac 209100
gtgaaattgc aggggcagta gataagtgat tggattcaga ctctcatctt ccactgaagg 209160
aaagacaact aacacatgtc attgtccttt caagatgaaa taagagttgt ggaatctgga 209220
gtcctctctt aaaactcact gtcacaggac ctctgtgcct gggtctgtct caggactatg 209280
tgtgttctta ggtgtctaaa gagcagatgc tccacgatga tggggccacc agctgagtct 209340
tccatctcca aaagcggctc caagcagctt tccttaggag ctgagctgtc aactcaagag 209400
aggttaaatc catgtcaaaa gtcaactttt ggccgggtgt ggtggctcat gcctgtaatc 209460
tcagcacttt gggaggctga ggcaggtgga tcacaaggtc aggagttcaa gaccagcctg 209520
gccaatatgg tgaaacccca tctctactaa aaatacaaaa atcagtcggg catggtggcg 209580
ggcacctgta gtcccagcta ctcgggtggc tgaggcagga gaatcgcttg aacccaggag 209640
gtggaagttg cggtgagctg agattacgcc actgcactcc agcctggatg gcagagcaag 209700
actatcttaa aaaaaaaaaa aaaattatgc caaaagtcaa ctctttcagg aggggagaag 209760
taggaaagtt tcaagattta tcattggaaa tagcatgtat gtgctaccaa gaaaatggaa 209820
gttccaaata gttggtatcc actcttgtct ccttgtccat acattaattt ttctcctaag 209880
agttctagtg aataatgaaa ccaattccgg tttttcattt ttgagcagaa aaatgaacaa 209940
accacctttc ctcatgaggg ggaaatcacc agtatgttca gtgatgctga tctttactac 210000
atatctgctg taaaatatga aaagtggatt gctagttttg atgttatcta ggttatcatt 210060
tgtgactaaa aagacctgtt tgttactctg gttcttctcc actatggctt tatggacaca 210120
ctgactgaca ttatagcatt tgttcatagc tttgtttgta gaatggggtg ctgtgagttt 210180
gtacctcaac atacctaacc cttgttgaaa agaaactaac taggtgatct atttttactg 210240
aatacagagt tttctgtctt ctgagggttt tctttccccc ctcaactttg tttgacacac 210300
agtatgccct gtagggcttc ttgcctggtt tcaagatctg gctagcacag cacttataca 210360
ggtagtttgt accaaattaa cttgccctgt gcacacatgt ccacttcagt ccttcaccca 210420
gtaccgcctt tgcctctagg aatcttagcc ttccttcaag gcccagtata acctccactt 210480
tttcattgaa gcctttgccg actagtccag acttgctgtt ccaccgcaga gagctccagc 210540
ccctctgatc ttgtgttttc tcctttacgt atcatctagc actgtgccat ctactgcact 210600
gtacttgtca ctgatgatct catgtgtcct acccttgtct tcctatctag gctattcact 210660
tctagaaacc aagaactcca aattctattt accctaaatc tcctatggtc actaatatca 210720
tgctaagtgc tcagtaaatc taatctattg atcaaaatga acaggtctca attactatct 210780
tcagtctgac tactggagtt acactttcct gttatttatt atttattcta gttatttatt 210840
attgtgaatc tagttattta tttattatta tgagaacata gtggcccatt tgcctgtttt 210900
ctctttgccc ttcttctgcc tgctttaact ccttgtanac cttccaccaa tctaaaatct 210960
gcaactagca agccacacag aactgagctt ggggagtata tctttgaatt tacagtcagc 211020
atccaggatg gtttctcct tagttctctt ctctggagta attttatcat cgccaactag 211080
gaagagatct tgaatgccca tttcattcat tccccacacc tgcaagcaaa acaactccta 211140
aactcattgt tcagattagt gttaggttac aggccaagaa cctgcagcag acataatgtg 211200

gattagagaa cattgcgcta atcaaagaca aataaggagt ccagtatgag ggtcaacgtt 211260
aaagtctttg gaaactgaaa attttattca gacgaaggcc attatcagag tgaggcacag 211320
ccaatgcacc atccgtggct gggacgtggg gccccacaag tttgttcatg gtgctgccca 211380
aataaaagta aaaatagaca agaattgtca attgtacgat gatgctttag cgaaacatat 211440
tcatgtttca tgttttaaca atgtcatcta ttgaatgatt tgacagtata ttatttctta 211500
acattccaaa actaaaagtc atactcttat cattttcagc ttcgcaagtc tgttttcagc 211560
atcagggcaa ggaaccttct ggaaaaggag accgcagtca ctaaaatctt aaggtaacac 211620
caattaatgt tgacacatat ttaaacacaa gtgggattat attaatcgaa aaatagcttt 211680
ttgaaaaatg aattaatgtt ctgggcatca gcatatactt tctgccagaa agtagctttg 211740
agtatctatg tacttataaa caatgtaaaa gacatattga tgtcatttct aaaaaaaaat 211800
tttagtagct cagtaggcct ttctttcaag tatttaaaca taaatgataa gtataaacat 211860
tgaccaaaag gtgaaaataa cttactgtta tttttaacag ttttatatct tttgtgatat 211920
gtaaaggaga aattgcaatc taaaggataa atggtaaaag ggtaaacttt ttcattatag 211980
ttcattacac tattaagagc acatttatta aaaatccnga aataataaaa agtataccaa 212040
tcttagaatg cctatgaaaa catatgcttt taatgctact ttctagaaga tgcgctgaaa 212100
tgaagtatca aggaggaaaa ctaagcaacc gacttgagtg ttttataaga ctatatgtta 212160
tattatggag taacatgatg agtataaaaa cacataggca tttagaatat catttattgg 212220
tacatatatt acatagacat atgtagatga cacaactcaa gattgggagc tggaggtagg 212280
atcgctcgca aggcgagtgg acagattcat gccaggaagc cccctgcaaa cctgcggtga 212340
gtgataagga gttctagctc ccttctacta gatttattac taggaataga cattaatgca 212400
ttctctccag aatgcatatt actataactt ggaagactgt gtgagaaaat aaagcttagt 212460
atatatgatc tttaaaacta ttcttacttt ataatactac ctaaaatttg tgtgcaaaaa 212520
aaatttccta actcatcaag acagtatttc tctcaatatt ggctgacgtt catgcataga 212580
gttaagtatg tgttggagta gctgtcatag gatgaaaata agcatttcta gggcttgtat 212640
attatcacca caaaatgtga tgagataaac agacaaggtc tcattgaaat agacaacctg 212700
cgcccccaact taggcttact cccgactggc tggtaactcc atcctggtat taccatcaga 212760
tcatgcagtg aggctggcct tgaaccacac atgttacatc aggatcatga ttccagctgc 212820
acctacatca gacaggttca tggtagggtc ctacagcaag gctgatccac tcactaaaaa 212880
gagaaattca ccctcaagcc agaaactacc tacaaggaca tcataattct cagtctgcat 212940
cgagtatctg cctgccaagt caggaactac agggacctta ctcacctgtc cagagccact 213000
gccttgcagt gtgaatgggc cctctgtgtg aatgggttcc cagagacttt ccaaggcaga 213060
tgcagaaaca gatactggca aggaagagat ttccagacct cacttctcta ggaactcttc 213120
cttaaaactg atctatcact ttaccttttt caccctcctg tccttattta cccctctctc 213180
agtttacaaa agaaaggtga aactctcact catccggaaa ctatggcgtg tattaatctg 213240
ggatgtaaaa tcctctgggt tgccaagcaa aaggaccgtt cccacctgga tttctccaaa 213300
aagagggtcc catggggccg tgccttatta tttatccagg tgacagtttt atggataact 213360
tccttcccaa agctatccac cttagattag gccttagaaa taacatgatt gtcacaaggg 213420
catgtggtta catgttattt gaattgaaaa ataaagccag actttttctg acagattcag 213480
ctgattggtt caacaatgaa aactgattta ataagtgccc aatgtcataa aattgaatag 213540
attggatggc gaagagtctc caatattttc ttaaaattat tttcaagcac aaacacaata 213600
atgctagaaa ttacattctt ctatttaacg tggtgcaaga gggatatagt tgctgatttt 213660
taaatataca gaagtgtttt tgttttttca attatatat gtacatgagc aatattttta 213720
agactatcag ggaatataga cacctctaac ttgcctaaca atggtgtagt ttaggggata 213780
cccacagcca ataaataata ttttactgca gcccagcata ctacagaatg agaggcagca 213840
ccttaaaact acctgagcta tatcaccttt tgaaactcag gaaataaaaa gccataattt 213900
gaaaaaataa aaaaccttag aattttttaa aatacaaatt tgatcttaga aagccatttt 213960
gtttttttctt ctgtgcgaat ggtttcttca ttgcttctat gctagtttct gcatgaatta 214020
agggcaagtg caaaaaatca aactgatatt ttgggaatac ttatcttttc accactaatc 214080
gtagtaatag taatggcaat gataccatac ctgcattata atgtgatcca tcagttaaaa 214140
agtcaagaat agattgggtc catgtcaatg agtgtttgta ttgttttata ctgtttttca 214200
aggaacaata acagctaaaa ggaaaaaaag agggagaaaa agaatttttt ttnnaaaaaa 214260
aaaaaaggaa aacatttgca gtaacctcag gacaattgat tcgaataacc ttccacaaaa 214320
ttgggaagga gaaaaagctc tccaccactt tttcagtgtc tttttactgc ctctagggac 214380
tccgctctcc taggctccaa ccttgctgtc gattaaactc tgccaaggtt cctggataac 214440
ttagcagtgg ggtctcccag gaccatttgc attttgaaag aaggctttta aaaaatgaaa 214500
gggcttttaa gacaaagagg atgcagattc tgtcttttaa tagatgggtc tatcttgata 214560
taaataaaag aaatctggaa gagactgccg ttttagtgtt ctattcctct taactttcta 214620
ggtgttctta atttcataga attattgttt ttagtcttat ccaagaggtt cctcgccagg 214680
gaggtaagta acagcagtat agggaggctt gaaaaagaga cctgagctgg atcattccgg 214740
caaatatcac aggttggcat tcgtcctaat ttgtgtgcag atagagataa atcacaaaaa 214800
ccttaaatcc agagtgatat aagaatagga agtttttatg aaatattagc gtcttgagat 214860
aactttccaa aaaataacaa gtatttggga aaaaataaag caacccccttg acttaaggtt 214920
tctggaggcc tggctgtgta aacagacaat taatttcaat tctcagctct cttgtctatt 214980

ccatgggagc taggaggagg cgatcttcag ggcccctgaa gatctaatgt aggatactgg 215040
acttggtgtt aaatcccccc tggctgggat caggctctgc ttctctgtaa cattgaacac 215100
ctcactaatc tgaccctcag ttttcttatc taccttaaaa agattgtctg aataatctat 215160
tgaccaagca aaatttaata cttattccag taagggaaac tgccactttg ttttcataat 215220
gtctcagaag ggataagtga gaggtggata tatgtaaggt tttgggtttg ggtttcagtg 215280
tttgtaggat ggaggtctgt ttagactggg cagaattctt gatatgagtt gaggagtatt 215340
gaagatgcct ggtatggaca agtaatagtt actgttctga agacggcagt gctgtggttg 215400
aggaccagaa agagaaagcg acctgcctgg tgcctctcca ctgctttcca tgttctttcc 215460
cccggctttg gttcaacatt tgagtaatcc attctttaca gcgagtttgc ccttctattt 215520
tcattctcct tcttgacttg ggtgatctaa gagaagaaaa ataaatggtg accttatgtt 215580
gtcctcttca cacatgaaat ttattttttc ttttgtgcct ttcagatttt taaccagaca 215640
catgtattac ctttaacaaa caaatacaac tgacttttat agtggttttt tttttttttt 215700
aagtttggca ttgatcccca ttgggtagag tcttcacaaa aaatcctaat gaataccaac 215760
tatttgtagg tgactagctt gaccagttga gtgacctact gtctggagaa aggggttggc 215820
ccagcagtct atagttcaga taaatgaatt tgtgggaagt gcctgaaatc aacggtgaag 215880
ttgctgactg tttaacagct ttaccttcct ggacaagtca agtcatattg acgcagaggc 215940
tcagctctca gccctggaaa ttaagctgga gagctgccag gggtcttagt tctcaggggg 216000
gacccacatt tccaaatatc catcttccat ttgaaacagg atccacatta ttctagcaga 216060
aagtatgtta tatacattac tatgaacagg aaattccagc ccacagagga agaagtcttt 216120
cagagaggaa gggccctaga gcctgaggta tgtaactcac aggtagaatc acccatttct 216180
atgtataatc atctttaatt catgttcttg tacttttgaa aattaattga tgttttgaga 216240
aatataaatg aattttttga tatatttgag aactctttga tgagaaaaaa aaaagtgggc 216300
catttttaca gaacttgagg ctactggccc atgctaaaat gcagatgatt cacttggcac 216360
aaacaggcat ctgagttgtt gtttttaag agttatccga agaggatgtt ccactacctg 216420
aattataaaa tccaggattg ctattaaatt tattgttgtt ctgacttaga ctgtatcgtt 216480
cagctccact aaataaagct tcttttcaaa aggcagtgac cttttctctc atcaggcgaa 216540
ataaaaaaca aatagcaatg tactttcaaa agcatcacta cctttgttga gatttcctct 216600
aattcaaagc cctgaaatcg agcaaaaaat gcattatcgt tcctgcaagg ctgaatgaat 216660
agagaaatgt taaaataaaa caattaaaca ttttacatta gtttagaaag ttccatcctt 216720
gtgtggtttg ccatgagtac ctccttagtg aggacaatat atttataacc tttcgaatgc 216780
tcagtctcgt atgatgtttg cattctgaca tttggtttta taacgtaact ttctttgcca 216840
tgcacttata ttaataactg tttaagtctt aaggaatagt ctttagttta gtgctactta 216900
ctacagaaaa aaattaaacg gatttaataa aatacataga gatagttttt ctcttatcat 216960
tttaaaagaa tcatttaatg actataaata caacttatat actacttaca tatatatata 217020
ancactatgt atttattaat ctgcaattat gtaaatgaca ttagctggat tttttttcct 217080
gaaagaaagt gcccctaaga attcacgcat tcattcgaga aatatctctg tagtgtctca 217140
catgccaggc atttcacacc atttgctttt tcccatttgg aaagttctcc ttttataatt 217200
attgttccat aggttagcca gctattcagc aactttggat aaggtcatat tatttctctg 217260
cagctccgca ttctgggcag aactagcaag acaccctgta cactgctggg agggcctgag 217320
taccttacag cgntcccctg gacctttgag ctcagctcct cagtggaaag ggaaggtgct 217380
ggactgaccc tacagatgag agaaagaaac gacagagacc cttgcagcct agggtttgtg 217440
ccaacccctg agatagcagc aattttttttc ttagcatttg gtatgaataa ccccaagttt 217500
taagaaaata gagtatgaga atgaatgtca taaatattta tctttgcaat tctatgaagt 217560
ctgcctggtt attgttggct ttttcttaag attttcctga cacttaaaaa ctctgataag 217620
ttagttgaaa gactatttgg aaataagcta agtagataga tgaaaattaa ctgtattcag 217680
acaaccataa actaaggaga caagatgtgt agatatatgt ttttgcataa aacataaaat 217740
agtaaagatt taaaagtggc aaaatataac taaaacaaaa ataatatatt ctaatcacat 217800
ttttagatag tacctgattt gcggtaatca aaatatgtta tattttattg agatagcata 217860
tgatttatag attgttttta gttaagccca aaagtgatta taatagtgtt atggattaga 217920
agggctacta ttgagtttta ccaaacagat gtgggaagtt ttttttaata gactattttt 217980
tgagcagttt taggtttgcg gcaaaatgga gcaaaggtac agagatttcc catgtacctt 218040
ctgccccccac acaggcacag tctccccaac tatcaacatc cccatcagag tggtacattt 218100
gttacaattg atgaacctac actgacacat cactatcacg ccaagtctgt agctgacact 218160
agggttcact cctagagtac tatagtctat gggtttgaac aaatatgtga tgatgtgtat 218220
ctatcattat aacatcatac agagtaattt gagtgctcta aaaatcctct gtgctccacc 218280
tattcattcc tccctcccca caaaccctgg caaccaatga tctttttact gtttccacag 218340
acatagaaat gcacatagaa atacatagat atgtgtctcc atagacatgc attgatgttg 218400
atggcagctt tattcctaat tgccaaaact tggaagcaac caagatgtcc ttcagtagga 218460
gaatggataa gcaaactgtg gtacatccag acagtggaat aattattcag catggaaagg 218520
aaattagata tcatcatgga aagacatggt gtaaacataa atgcctgtta ctgagtaaaa 218580
gaagtcagtc tgaaaagtct acatactgta tgattctaac tatatggcat tctgggaaag 218640
atagaaccat ggagatggaa agatatctta acaaggtctt tcttttgtta ttttgtcact 218700
ggagagtaaa gtagtaaaga aaaatggaat acatatctgt tgaaaattgg ccaatgcagt 218760

tcctgcagca tttacaaaat ttcactttta ttgtagtaaa aagttcagcc tttgcattgt 218820
aatttgcatg tttcggagtc agtagttggg ttactaaatt tgaagcccag tgaatgatgc 218880
ggggttgcag acatttttta tgctgaaatt taatagatgc acgaaaggca tggatttctc 218940
atgagttaat ttttcttttt tgtttatagt atgggtataa gtgcgtaaga taaagacatt 219000
caaggtttat tttattttct gcctacnatg aagttctggt cgcccatgta tttacatgcc 219060
tttctatgaa aacattttta tgaataacac aagattggga agaactaact ggcagttgct 219120
atgaaagagc cagatccatt tactcaggat agggctgact cacaaaggag tggggtatcc 219180
actaggataa aaatgcagct ttttcctcat ctcagcaaga ggccattacc gacgagctca 219240
cccattagca tccgctgttg tctctacttg ggaagatcac attgtcactc tagtggagtc 219300
aggtaagagc catttccagg aaatactcat tatctattct cttagttata cagtcatagt 219360
tttacataat taccactgaa agactatttt cctgttgtct gctaatgtga ttacattaca 219420
ttaaccaacc ttggataata cttttaaatg tattacttgt ggagttcaag atttgagcaa 219480
aatccctcaa atgtaatgct ctgcaaagtt atactgcagt tcctttagat ggtaattacg 219540
tttaatacca gacggtattg tgtaatctaa cttttctata atgagtgaaa ataatttgaa 219600
gactcatagg cctctccctt tcattacaga atgtataggc tttttccaca cccttgttct 219660
gggacattaa gaaagaagac atggagacaa agaagaaaag aaccggggac cccctgctac 219720
tgtgtctata aacaactagt ggtgtttaaa ggctaataaa gctccaaaga tctctcttat 219780
aaccataatc tttgggccaa ttttaaaata ttttagatgg agaaatgtca agaaaaatag 219840
agacaatttc aagacatgat tctctttccc caacgtccat aaaagattat atatatatat 219900
atatatatnn nnggctgtca tataaggagc aattgaaatt ccacagcaca aaggcaatgc 219960
agggggaaaa aaaaggatca atctttcata ttattttttc tcaaaggctt ggcaagaaac 220020
caggggtaat atttttgcaa ctgggaatgt gtgtttgctg aagaatattc ccacatttga 220080
atcatcctca ggggtcctca tcttgtttta ctttcaaagg tagacagggc ataacattaa 220140
ccccaactgg gaaaaggaaa atgcctcact gtaattctcc tccagggaac aggaataaag 220200
ggattacata tgcctgcata atgaagaatt tcctcaccca gacaagacaa aagacttcat 220260
tatttttcac cagacacttg aagtcttgtg cctcccagtt gcagaagctg aggaggcagg 220320
ccagtttttt atatgaagcc actagatgga gttcctaaga aactggcctt ctagtacttg 220380
ccattgtgaa aagatctagg gaagatgtta aaaaccttgg tacctgacac tacccagtct 220440
ttcctctaag aaaagtgaga ctgtccctga agatcacagt attcttccag taaaattagc 220500
cttgccttta ctctgggaag caaataaatg agcattttct ttctgaaaat gcacctcctt 220560
tgtccttaca tgagaccaaa aggcatctca atactaatga aaaatgtaaa gtggcaaaat 220620
ataacaaaat ggaatattca agttcttctc cttctgggta ggtctgttac ttcgtaactt 220680
taaggatgta tgcatctcat ttcttatata aaatattgga agtctcaaat tttgtagtta 220740
ctcactattt cggcttgcaa gtattagatt cctgtaagag gaggtatatg gagactatgt 220800
tcccagcaac agatctgatc tccagttaaa gaaattgaac taagttcacc tgctacagcc 220860
tgctctgctc cctcttggct ttcaggcttc cctaatagct ttcacataca acatagagaa 220920
agangtatga agtttttagt cgtaagaagc aatgtctttt ttttttcatt actgtctctt 220980
ctagtgtagg tatgcagccc attattcttg agtggcatca tattctaact tgagataaaa 221040
ttaagattat ttaacttgcc cctttaaaag tatattttaa acacatgctg ttttgtccgt 221100
gattattttc atgcttcaaa agcagggagt gatcagaaaa gaaaatattt gatcttcacg 221160
gaatttcaag gttcattcat tccacaaata tttattgagc acctactgtg gataagagct 221220
gagaaataat catgaccaat tttaaagaaa accctactct cttagagctc tttggagcat 221280
ggcatatgtg acgaactaaa agaacaaaga aaatggcatg gagagaacga ggttcctcct 221340
ggtaaagatg agaccagaaa gggaggttag agggacacta aacggggctg tgcaagccat 221400
gctgctcgtt aggcttagtc tttttaaggc agtgataata accgcattcg tgatctattg 221460
ctgcaggaca aattactcca aaacttaagt gatttaaaca acacgttttt catctcacag 221520
ttttagtcag gaatctgggc atcccttagt catgtctctc tccaagctgc atgcatttgg 221580
acttgaatgg gaagagcccc actcaatcgc atggttgtta gtggattcag ttcctcgtga 221640
gctgtgatat gaaggcctca gttccatccg gtttgctgga ttcctcccca agtggaactc 221700
tccgtagagc acctcacaat atgcagcttg cttcaccagg gcaagcaagc aagatgggaa 221760
gagcaagtca gcgagagaga gagagagaga gagagagaga atgaatatgc tatgtagagt 221820
gttataacat ccaccatgac gcgggaaaag atacaacagg aaataatagc aactaaaatt 221880
caaagaaagc gttgatgttt ccaaagcact gtcacattca taatcatctt tacttcttcc 221940
agctccctgt gaagtcagaa ggaaaggcat tattgttctc tgcattttac atatgggaaa 222000
tggaggctta gagaaagcac atcacctgtc taaggtccca cagcttataa aagctgaagt 222060
cagaagtgtg tgcttttaaa gcttatgttt tctcaactca tcttctccat taggcttcca 222120
gacaaaggca gaactttcca tcagcagctg cactgcaggg tgcacttctt tgaaccatga 222180
gaggctagca agttagggat ggggtcacac agtccccacg cacctgccgc taccctccct 222240
actcacccct ccccaccttt tcacttactc attctgttca tcaggcagca tgagggccag 222300
aaactgaact tcctaggaat gggaggctga gtgataaagt ggtccctaat caactcacag 222360
cctagtaagg ggaataacta atgataacaa aatgccatga gcgttaaaca agcttggtta 222420
ataacatact gtaggaacat ggagtataaa acaagtcttt ttttgtgctt tcacataggt 222480
gaaatattca tttctcattt catgttactt ttcagagccc atttatatca gtctgtcatt 222540

atactgtgca tgttaaaaca ctatttggta tttatttcat ggtttgcctt actgtatatg 222600
cttatctata gtaacattgn tattcccttt aaaaaataat aaagcaaact tttaccatgc 222660
ttttgtatga acctgggcac attggattag agtatatgta acctcttagc aggtctgatt 222720
gtgtccaact gaagttcata gctttctgcc aacactgtta ttttccaaaa tagcaaaatt 222780
tcttacatga ccaacaaatg tgacaatggc ttaggctgta caaggctgtt tcattatgca 222840
gaaacgtcct atcatgggaa tctttctata cctgtctcaa tntccaggtg agctctaggc 222900
caaagggtca taaaacttcc ttcccattgc cttgctcctg catccaccgt ccaacctcag 222960
gtttataact tccatctgcg gtccttccct cattcaccag gaacctgtgt gtggccagtg 223020
gtctccttgt ataatggaat aagatttttc agaatgttct cgttagacat atttatatat 223080
ctgtagaaaa gttgcttcat gctgtttagt aaatgaaaaa tagtgattat ttagggtttg 223140
tttccaagaa tacatttaca ttcagtgaat ttgtatgatt tttcatagat attcaccata 223200
attatcttca tccacattaa aagcattttt aaggacttaa atatgacata tttagtaagt 223260
tcttatataa tgtattacta tataataagt tcttatatga tgtattatat aagttcttct 223320
gtaatatatg tgttatattt aaatacttaa aaatgcttct attatacatg tatcataaga 223380
gctgctattt taagtgcgct aagcacatac aaatcttttt ctcaaagatc ttaattctca 223440
gacagatggt aatcatattc aaagccattc aagaaatgca ttttaagaaa cataaatgtg 223500
aaaataatat gtaaaaaatg gcagaattgt gtgttcaggt gttgagcaaa tagagtatca 223560
ttagatttgc atatcataaa atcattgcat tttcaacctg tgagctatct ttcagagcaa 223620
ggctttctct tcattttacc aatgaggaag ttaggcccaa agatcactca actacttcat 223680
aacagtgtaa cttctggcac acaggtcttt taatcccttg tgttatttc ttgccactta 223740
tcaactatct caagggaatt cctgtcattc ccagttggag aaaattttct ggagaataat 223800
tttttttct caccaaaatc attcacacca caggtgttag gccacccaat tgtcttcact 223860
cattagcaan atcagaaatt aacacaagtc cagttattgt ccaaaaagaa catgtttta 223920
aaagtcttcg tggtttaaac aagtggtaac tggttaccag taagctgcaa gcttcagatt 223980
gaggtttctg taggtaataa ttccaaaaac tgtgtgcttt ttttttttt ttgaactgtg 224040
gttttaaaaa caacacataa catttaccat ctaaaccatt tctaagtgta cagttcagtc 224100
gtgttaagta tgtttacatt gttgtgcata attccagctt tagttacttc tctttatgac 224160
tcatttttgc ttgtaagctt atgttttcct atcttcctaa agttctacaa aatctccttg 224220
gaacatatat ttaatcattc caggagcctg agtggacaaa tccttcccaa aggggtttta 224280
ttagaaacct tgtttaaatt caaaaccgac atcatgattg cctgtgtgta ttaatctgtt 224340
ttcatgctgc tagtaaagac atacctgaga ctgggcaatt tacgaaagaa agaggtttag 224400
tgaacttaca gttccacgtg gctggagagg cctcacaatc atagcagacg gtgaaaggca 224460
cgtcttatat ggcagcagac aagagaagag agcttgtaca gggaaactcc ccttttaaa 224520
accatcagat cttatgagac taattcacaa tcacgagaaa agcacaagaa agacctgccc 224580
ccatgattca attacctccc accgggtccc tcccacgaca catgggaatt caagatgaga 224640
tttgggtggg gacaaagcca aaccatatca ctgtgggtag gctggccttc tgcttatggt 224700
gtcctgatag aagctctacc attatttttg gaaaggaggg aggaggcaga aggaggaggg 224760
gcaagagggg agctttctgt ttattttcca gcccttcttc atagctgtga tacttctgct 224820
ttgtaagtgt tcttgacgca gagcgtaaag ttcattttcc tttataatta tacgttattt 224880
gctgtatttt gtgttttcat tnnggtgttg nccntttaaa ataataaaag taatgagcac 224940
ttatgtgtta ttcatctcaa gtgtacaaac catgcattta tcttgggctc cttttttttt 225000
cctagagctt gcacccgaca gcgcatgctg agcggatcat ttgaggggca gcccgcaaag 225060
gagaggtcaa tcctcagcgt gcagcatcat atccgccaag agcgcaggta aatacttggg 225120
aagacagggc ttcagagact ctaaaaaagg ggggttttac aggaaatggt cgctgtacac 225180
agtcacggct gtcagccctg gctgccagac aggcatggct gaggaatcgc gttctatttg 225240
ctgtttgtta caataatgaa acacctaatc agatccaaag attcaaaaaa atctctactt 225300
cacaatcctt ttccttagag atggccaaat tctcaccttt aagatcagaa ggaaaaattc 225360
tgaattttgt tagatgggat caaagacatt tggagagagt ataagcttct gctatgaaaa 225420
gaaatcatag gcatttgtgg cgttgtagca gaaaaagtgt tttattttga ttttatcatg 225480
gtcactgctt gaaaggccag tttgatcttg agatgtatta accagtgcct gtggttgttt 225540
taggtcacta agacatggat caaacagcca gaggatcagc aggggaaaat ctcttgaaac 225600
tttgactcaa gatgtaagtt ttaagcaatg ctttggattt cagaaacgtg attatgaaaa 225660
tgtatttaaa tgactgctta gacaatgccc agtgtctttc tgctgcattc ccatgtctcc 225720
ttaagacttc ataccagagg gaatcaggaa actcaggata tgaattatgt agccaagtat 225780
cagaagtaac tgttaacagc agaaaactcc agtcatccca cacaaagcac atgtcttagt 225840
taaaatagta acacaaaagt gtcattatca gttggatttg acttgaaatt aaagacattt 225900
taagcattgt atataggatt gatttgcatc attgactttg aatattcact gagaatcgaa 225960
tattctagaa tctagatcaa gggtctataa tttcagggct ttatatatta ggaagtgaaa 226020
ttttatttct agaagaattt tcctgtcaag gactacctag tatttcccag gaaagctttt 226080
tgcttttaga ctctaagtca tggatttatt tactggctct ataaatagtt atcaagtgtt 226140
tgctctgtgc tgggttctgt tttaaggaac gaggagcagt ggggtaataa gatagactca 226200
gtgccctcct ggagcttaaa ttttagaata ggagacagat aataagttca taaaaatata 226260
atggaatttc tggagtgatt tctttttatt ttccatcatc tcttcattac tgtgacagat 226320

ttgctttata atgtaaagta aaatgaagtc atgtaaataa atcaaaggtg gggatttggg 226380
ggcagggaat tttagatacg atggttatcg tagacccatc tgcattatcc cggagaccca 226440
aatgaaatga aggagacatg aaaaaaatgc agagaagcca agcgttatat ggcccagcag 226500
ctataccatg caaagattac aatgaaaggt ttgggaaatg catattaaat aaccgtaatt 226560
atgaagccct ggtaaaaaga caggcgttag gttgaatctg gatgaactgg cttaaaaagt 226620
aatcttgaat gttcagagcc aaggatggca cttgagaaag gagggctagc aagagaaagt 226680
tagcaaagcc ttggggttat ttcgtttttc tctttttcac cacttatctc ttatttcttg 226740
agcttttgga gtgcttgcat actacatctg ctttcattct gtagaaactg ctgttttagt 226800
ccaagtacat ggtttttatt aagtgttccg ggcaaaatca agagcgagaa gttggcaatg 226860
agaacacctg atatcaccaa taccctatag acatggttnc tagttggcac agagnctctg 226920
tttaccacca tgatttttaa tatccatagc tctatctgat tggctaccat attgttgaca 226980
tagaattgcc atcaaatcca acatatggcc tgcgcaactt agaactgcag agtcagccat 227040
attgcacgga gaacattttc aggtgaaaag tcaaagaggc aaccctcatt caaagcgcct 227100
tcaatatgtt tcaaccgaaa tgtacaacct acttaagaat caaatcagaa catgtctttg 227160
aaaataatgt gtttaaacat agacagcaaa ggccctattt gaatagtctc agtgccggag 227220
cttacttctt cnnagagact tcctacccta tttttcatgt agaagtatag taaaatccat 227280
ctcccacctt ctccatagca aaatgccatg gggttctgat catttccaat aaattcttat 227340
aatgtctttt ctcccacaaa ggggagattt aaattagaga attttaactt tttcacttat 227400
tcggaatgat agcangacac agataattaa gtctaaggag tgctttctgt aaaatctact 227460
gagttactgg aactctggcc ttgcaccttc catggggtcc atctaaaggg ctgtgaaggg 227520
cctgcatggc ctgctcacct ctcttagctt tacccacact ggcatcctgg ctattccaca 227580
tgtagacacg ccaggcctgc tcctagctct ggaagaacac atttctgtag tcctaccaca 227640
gcctgcaccc tcacctcctt ctagtctttc ctcaaatagc accttcatat tgaggtctac 227700
cctgacctgt tcatttaaaa tcataatctt ctagcctgtc tgtgtctctc tatgcctttc 227760
acccattgtg attttttaaa aaatcggatc actaagcatc ctcttacata cttattttga 227820
attattttt atctgtctcc cactgactag aacatcctat gagaataagg atgttttgtt 227880
tccacttgcc atatcctgca cactagaata gtgcctggca cataatagga tttcaatcaa 227940
tatgtgctga atgaattact ttttagagga acaaatactc ttgctatgaa gactgtggcc 228000
attaaatatg tgcatttaat cgtatacata ttttatatac tgtaaattta tatatttaat 228060
gtaagtatat acatttactt taaattacat atttaatgta aatatatacc tttagtataa 228120
atatacataa ttataaatat atacatttca tatagatata tatttattgt aaatatatac 228180
ttagtataaa tatacatatt taatgtaaat atatacattt agtgtaaata tacatatttg 228240
ttgtaaatat atacatttag tataaatata cattgtaaaa tatacattta atataaatat 228300
ataaatatat gtttaacagt aaatatatgt aattaatatt tatactttat attgagggag 228360
atattagttt taagtttttt aactgataat atatttgcca ttcaaaaccc tcctgtttta 228420
acacaccccc tgttaattta ntttcntagc attccaatac agtgagatat agaaatgcac 228480
aaagagaaga cagtgaaata aagatgattc aggaaaaaaa ggagcaagca gagatgaaaa 228540
ggtacaggtt gctgcagtgt cccatggctc tcattcggga atggcaaaat ttcccttagc 228600
ctcattctcc catgaatttc tccttcaagg aaagtgcaag aagaggaact cagagagaac 228660
cacccatact tcgataagcc actgttcatt gtcgggcgag aacacaggtt cagaaacttt 228720
tgccgggtgg tggtccgagc acgcttcaac gcgtaagtac acttcatttc agccaggtgt 228780
ttggcctgag ccatgcaaac agcaggggtg ttgtcatatg aagaagaagg aacgtgtaat 228840
cctaatgggg tcacaaaata aactgcggac taattaatca tgctgctcct ctttgctgaa 228900
cacccgacca ggtggaaaac aaaggtcgca agtagttaac atttatagaa aactcgcatt 228960
gataaaacac aaaacagcca ttttcgtttg tgcattttaa cagtttcctg atacacttat 229020
atcggcatta ttaactccat attttatttt ggaagtactg agacaaaagg agactaaagg 229080
acatggtgaa aggaaggtca ttgtccatgt cttccatgag gcttgtagtt cctttgtggt 229140
aaaaatacct caatttgttt tttggagaat aacgtaaagt tgattagtgc tttcattacc 229200
aactgttata agaaggaaac ccactgttca ctgtctctac cctaccaaca tggaagaaat 229260
gtgtaagaag ccgtaagtag agtggacatt gaagacacta gttgtggaca cctaagccaa 229320
gtttgacagg caacaaatgg atgaaatgga tttttgtcaa gcatacctgt atnagtccat 229380
tttcatgcca ctgataaaga catacctgag actgggtaat ttataaagaa aaagagattt 229440
attggattca cagttccaag tggctgggga ngcctttcaa tcatggtgga aggcaaaagg 229500
cacatcttac atggcagcag acaagagagg gaagtgaaag cagaaacccc tcataaaatc 229560
atcagatctc atgagactta ttcaccacca caagaacagt atgggggaac cgcccccatg 229620
attcaattat ctctgactgg gtccctccta caacatgtgg gaattatggg agctaccatt 229680
caagatgaga tttgggtagg gacgcagagc caaaccctat cagtaccttt aagtaaatat 229740
tctgaagaca ttaagagtca tggaggaaat gagaaaactt tccatgttcc cattatgtag 229800
ttggagtgtc cctgatgcca tcgtttaagt aaattgattt ctttgttatt aattagtagc 229860
atatgataat cactatctgt tatggactga attgttcccc ctccacaaaa tccatacggt 229920
agagtccaaa ccctcagaac ctcagaatgt gactgtattt ggaaatagga attttaaaca 229980
ggttaattaa aatgagatca ttagggtggg ccctaatcta atatgattgg tttccttaag 230040
agaacatttg gacacagaca tatacagaga gaagaccttg cgaagacacg tagggagaag 230100

acggctctcc acaagtcaag gagacagccc tggaacaggg ttttccctca cagccctcag 230160
aaagaaccaa ctcagctggc atcttgatct tggatttcca gcctccaaaa ctatgagaaa 230220
ataaacttat gttgcttgag ccatccagtc ttaagcacca gtggtacttt gttacagcag 230280
ctcgaggaaa ctaatacaca ctattataac atttcaggga tggaagagac ctcagggacc 230340
actcggtgtc atttgtgtgc cccctccttc aacagaggag gacatttaag cgtgcagaag 230400
ttgagggctt tgctcaggga cccagctact acctggctct ctcttgattg ccagactatt 230460
gttctttctg ctgttctata aagactttca caatttaaag caactgagtc acaattatta 230520
aaatcttcca tgagttttca aagtaaagga agaaaatgcc attttggaga ttgatttgat 230580
cagttcttga agtacgtttt ttttatcttg aattatcctg ttggctgcct gtaccttaac 230640
atctcatggt taattctcca cttcacttag tgtgatgtaa tttccctttt atcagcaagg 230700
cagctgatgt tgaatacaat tgatgaagac tctgttgtaa ttggggacac atacacaaag 230760
actgtaagca atctcattga cagtgctcac aggcttcctg aaagtagaaa caacgactct 230820
cccttccaag aaattctaaa taaatcattc taccacatca cagttaaatg caggggggaag 230880
aagtctattt aaattatgga gtatttaatt aaataatctt gaataactaa cattcttgaa 230940
ctattaaggc aaagatttca aatgagtgtt tttaaatgaa agtcaaaact taattttgta 231000
ctacccatgt cagaaaattc tgaacaaagt cacaaagtct aaaatcaagt attttgagaa 231060
atataaaact ttattttcat aagcatcaac tgcaaattct ttggattttt tctttcatca 231120
atgttctctg acctccagag ttacctacag ttaacatatt ttataatgga cattgtagag 231180
aggctgtaaa agagatttta ggtagatgag atgtttgcaa atatgatttt tagcaagtta 231240
aaacatgctt gctttgttgc agaattttat atatgtgttc tcttacagat ctaaaacaga 231300
ccctgtcaca ggagctgtga aaaatacaaa gtaccatcaa ctttagtaag catcaccaac 231360
ttcattttcc atgcacattt aaagttttca tgtactattt tatgctgcat acaacgatta 231420
aaattctctt ttattttctt gctatatagt gctatgaaat agatgattgg ggaatgatgt 231480
ttttaaaata ccagtcatgt tgatagagcc acagaagcca tcatctctaa ctcttagaag 231540
cctttcctgg aacctttatg tttctcaatt ttgtttggtc tttggcagtg atttgctggg 231600
attggtcact tacctggact gggtcatgat catcgtaacc atctgctctt gcatttccat 231660
gatgtttgag tccccgtttc gaagagtcat gcatgcacct actttgcagg taccgcttac 231720
cgctgcaaaa tctcagctta caactaggag gtaaaatact gacctgctgt ttttccgttc 231780
attgtaacca aactgccttt tgcccgtttc agattgctga gtatgtgttt gtgatattca 231840
tgagcattga gcttaatctg aagattatgg cagatggctt atttttcact ccaactgctg 231900
tcatcaggga cttcggtgga gtaatggaca tatttatata tcttgtaagt ctttgcttat 231960
tgcctaaatg aaaaatgtaa tggttttgta aaatctcctg catttcttgg tataagcgca 232020
agaggtatga gttacagagg cttagattcc ctaaccgaat tcttaaatgg actctagagt 232080
agttcattat atgcaaataa gggccatgca tgtaaaatct aaattaagag catcataaat 232140
atctatggat agttatattg ttcaaataca ctaatcacaa ttttatgtat gaatgagaca 232200
tacattaata aggtctaaca tggcaaatct agtttgcatc ccacaaacct tatggcttct 232260
atactaaaaa aaaaaagttt gcctcatata aaccatgtta ttgattatga gccccttatt 232320
gctgaaagca aactaaagtg attgtgattg tatctctctc ctgattccat tgcataaaat 232380
acctggctat agcagttgcc cattcaactg tctagtaaaa ctcgctggaa aaccatagaa 232440
atgggctcag agaatttttt atttttcctg tatgtccaag gaggcatgaa atcaatgtct 232500
ttgcagagac aacaaggaga attgtgatca ttaacaagta gcatgttgca ggtcattaat 232560
aaaatagcag taaccttgat aaaaggaatt ccagccagat cgacatcagt ggagcctcca 232620
gagtagtaag ttaaaagaag aggcagaacg cactggtgta cacacacaca cacacacggt 232680
gtggacaccc ccatttcttt cctgcataca catgatgact ttcctttgag gtacctagca 232740
agtctagatg aggtttgttg tttaaagtta acagatggca cgtacaacca gtgagtggaa 232800
tctctgctaa atgaaaagtt gtggccagtc ggctgtcaga aaatgtaaat agtaggggat 232860
gaattgtata aattccagag atcatttgtt cccctttagg tgagcttgat atttctttgt 232920
tggatgcctc aaaatgtacc tgctgaatcg ggagctcagc ttctaatggt ccttcggtgc 232980
ctgagacctc tgcgcatatt caaactggtg ccccagatga ggaaagttgt tcgagaactt 233040
ttcagcggct tcaaggaaat ttttttggta tgtgagaatt atcctttgat tccagttcac 233100
agtagtccac ctctaatgca gctaatgagg gaaaagtgag tcttgcttat tgaggtcaat 233160
tcaatagttt tggttattgc cagagggtaa tggaggcact aacaaaacac aatgcacctt 233220
caaacacttt actcatgggc acttctggcg tatgagttta tttaattcac ctaaagagaa 233280
gcactctgag atttgaaaga gttcntatct gctgactctt tcggtactaa cctcttgact 233340
ctaattccta ctctgggttt tagtttcctc ccatctgaaa atgaaggata agaagattca 233400
acacngtgta ctccacaaat gggattagga attcatgctt ttgggctcct aatctcattc 233460
tgtgtgttcc tgaaataata tagcacagaa tttgtcttta gatctcagga gntgttaaca 233520
taaaacaact ttaaaaatgc aacatatagg tataacccac ttcaattaac ttcagcatta 233580
cagtaatgag tgattacaaa ngatgattga gggggcaaaa cgtactttnt agctgggcaa 233640
acattgattt agggtcttct gaaaacaagg tcagagataa aacaacaaga aagaggctca 233700
ttgttcacaa tctgttacag aaagatcttt gccttacaaa acagtgatca aaagtttttt 233760
ttattattga tgaggtctgc ttgtgcatga gaagcatgaa ttaatttaca taggcttgtt 233820
ttgtatcttg ctttctagag acttacctgt gatgagaaaa cagctgtagt tgtagttgac 233880

acaaatgtat tttaaagata gattgtgatg ttactagagg tatatatatc attactgact 233940
tttttttttct tttttttga gacagaatct tgctctgtcg cccaggctgg agtgcaatgg 234000
cgcaatctca gctcactgca acttccacct actgggttca agagattctc atgcctcagc 234060
ctcccaagta gctgagacta cacgcatgtg ccaccactcc cagttaattt tttgtatttt 234120
agtagacacg gggctgcacc atgttaccca tgctgccctt gaactcctga cctcaggaga 234180
tccacccacc tcagcctccc agttgctgaa tttctttttt aagatgggtt taaaaattct 234240
tgttttcttt atgcaaagcc ataaaacagt gactcttcac acagctggaa ttaaaagaaa 234300
atatcacctg actgctaatg tatatgaatc ttttgatcaa taataaattt tagaggagaa 234360
tttgtgcctt tccctaatgg tggtcagtgt gattttagta cacttaggta tatgtttagg 234420
ttattcaacc agtgcagttc acttgttcca tattttgccc tctgccactc gtctctgctg 234480
agaccaaaga agtcagccag cagagggagc atgagttaat taacagattt gctcttcagg 234540
actgaattct ctcctctcct ctatgcatag aacattctcc taacgcacac cttcacagtt 234600
acaagacagc taagccactg tcccgctcat ttgtgctctg ctaataaaaa tatgaatcca 234660
ggccgggcac agtggctcac gcctgtaatc ccagcacttt gagaggccca ggcgggtgga 234720
ttgcctgagg tcaggagttc aagaccagcc tgaccaatat gatgaaaccc tgtctctact 234780
aaaaatacaa aaattagctg ggcttggtgg cgtgtgcctg tagtcccagc tactcgggag 234840
gctgaggcag tagaattgct tgaacccagg aggtggaggt tgcagtgagc tgagattgca 234900
ccattgcact ccaccctggg ggacagagtg gcactccatc tcaaaaaaaa aaaaaaaaaa 234960
agaaaaaaaa ataaatatat atatgtccat ttgggaaaaa atagacaaaa tataattact 235020
taaacatttt tttcctttgt tccattaaat aaaaaagatc aaggtgttga ttttaaagtc 235080
agaaaagttc aagccatatc aaaaatacct tattccattt tcttacatga aattcatgtt 235140
tccatttaaa aaattcaaag cttgttaatt ttctcctttt gaccttccag taaggctaat 235200
tgtgtgacct atggtggaga agttaaaata tatcatcaaa aacccatgat ttttatacaa 235260
tgttatctta gaagtcctat ttatgtcact gtttaaacca aacctaatat ttagtcttta 235320
acaagagatt taacatttca tcaatttctt tcaggtctcc attcttttgc tgacattaat 235380
gctcgttttt gcaagctttg gagttcagct ttttgctgga aaactggcca agtgcaatga 235440
tcccaacatt attagaaggg taagatgtat ctttctgtct tttgaaataa aaattaaggg 235500
ccaagtgtgg tggcatgtgc ctacaatcct agcactttgg aatgccaagg agggaagatt 235560
acttgaactc aggaggtcaa ggttgcagtg agctatgatc tcaccactgt actccaacct 235620
gggcaacaga gcgagagccc tgtccctaaa aagaaaacaa aataataata aggtgtttac 235680
actcccttgg caagcatgtg gtaccagtgg ctgacaagcc tagtttaaga actccctata 235740
aaaccctaag aattctcttc ccacaaaccc ctttttgctt aagcatgtga tatgtctctt 235800
ctggctagaa aggttaataa gatgaaaagc acacctcaga gaatgtcact cttccctcct 235860
attccagtgt caacccactg ttgacaactt gagygtggtc acccaagatt ccagctgttt 235920
acagctgtaa gctttggata ctttcccttg aggtcttagc taacttgatc cttcctttgt 235980
tgtcacataa gactcatatc agcaaacagc cccatgcaaa aatgtccaag aagcagcgtt 236040
tggttctcac cactttttat tgcttcccca ctctcagctt tctccccatt agaaangcca 236100
gtgcctttac attagctcta gctgcccaga atctccctct ttatgacaac caacaaggac 236160
ctccacaaag acccaaactt gaatacttgc actacggcgt ttctttctct ttcttcagtc 236220
attggttata agggaaggct agctgctgta acaagcccaa tatttcggta gcttcacata 236280
atgaaatgta tatattgcat cttggatcag ttgcaatata tacatttgca atatatacac 236340
tatatacagc tctcctccaa atgaccactc agggactcac atgccttcta tcctatgccg 236400
ccaccctttg ccatgtcttc agagccttct tcaatgagct ggagataaca gtggagagcc 236460
atctccaact gagtgggctg tgggagaatc tgctccaccc acattcctct gtaaagagca 236520
gagatactgc aagggggggct ggaatttagt tgtgtgctcc agaaaaataa aaggggggaca 236580
cagtttggtt aataattaga caatttttgt aatatcataa agtgtcccat tctcttagga 236640
ttcttatgtt acaagtaaat tcataaagta aaccaaccac tgctgatgta taaactagaa 236700
aagcattttc tctttgatcc caataaaatg atgcctcagt tcctaaatgt atatcttcaa 236760
aaggcctact agacctgaac tacattattt atttctagca aatatggcta tctagtatag 236820
attttaaatg atacaaattg ttctctaatt gtgacaaaat acaggtaaca tctttgttca 236880
gccaaaatac attataaatt atattaataa tgagaatttg aaacaaaggc cagactggga 236940
gagacattcc aagagctttg atacacctga gaagacagaa caaggaatgt ggcctgacgg 237000
tcatgccgta tcaaattagc tgataaagaa agtgacagag acctgacgca ttcatccaca 237060
agcttgtgtg tggaaagaag agtgattttt gatccacact gaacagaggc cagaaagata 237120
ataacacttc aggatgaagc ctgctaggaa tcttggatga ctcagtatat taacagtggc 237180
ctggccgggc gcggtggctc acgcctgtaa tcccagcact ttgggaggcc gaggcgggcg 237240
gatcacgagg tcaggagatc gagaccatcc tggctaacaa ggtgaaaccc cgtctctact 237300
aaaaatacaa aaaaaaatta gccgggcgta gtggcgggcg cctgtagtcc cagctacttg 237360
ggaggctgag gcaagagaat ggcgtgaacc cgggaggcgg agcttgcagt gagccgagat 237420
cccgccactg cactccagcc tgggcgacag agcgagactc cgtctcanaa nnaaaaannn 237480
nnaaananann anaannanna aaannnnana aaaccggggg ccctaaaaac gggggggttt 237540
tactttttcc ccttatcccc ccccaaagtt aaaaattggg ggggaccgcc ccccccaaaa 237600
aaccctaacc cccattttta ggggcctttt ttcctttttc caaaaacagg ggggttttgc 237660

```
caaggtttaa atggaaaccc taagggaacc tcccctttttt aaaagccccc ccccccaacc 237720
aaaaccttta aaaaaaattt ggcccgggaa aacggggttt aaattttttt gtttttcaaa 237780
aaccaaaaaa aaaaggccgg ggccggggcc aaaaacccct taaagaaagg ggacccccgt 237840
taaatttttt ttgaaaaact tccccccttt tttctaaaaa aaaaaataat tccaaaaaag 237900
gggggaaagg taatcccccc acttttaaaa ccccccccctt tcctttaaaa aaaaaaaaaa 237960
t                                                                 237961
```

<210> 3
<211> 47841
<212> DNA
<213> Homo sapiens

<220>
<221> exon
<222> 3895..4001
<223> exon 28

<220>
<221> exon
<222> 9611..9731
<223> exon 29

<220>
<221> exon
<222> 9816..9914
<223> exon 30

<220>
<221> exon
<222> 15776..15869
<223> exon 31

<220>
<221> exon
<222> 16382..16488
<223> exon 32

<220>
<221> exon
<222> 16697..16771
<223> exon 33

<220>
<221> exon
<222> 17934..18053
<223> exon 34

<220>
<221> exon
<222> 23644..23712
<223> exon 35

<220>
<221> exon
<222> 24928..25076
<223> exon 36

<220>
<221> exon
<222> 25913..26006
<223> exon 37

<220>
<221> exon
<222> 30767..30899
<223> exon 38

<220>
<221> exon
<222> 31561..31676
<223> exon 39

<220>
<221> exon
<222> 34044..34201
<223> exon 40

<220>
<221> exon
<222> 37493..37643
<223> exon 41

<220>
<221> exon
<222> 39652..39801
<223> exon 42

<220>
<221> exon
<222> 41563..41680
<223> exon 43

<220>
<221> exon
<222> 44131..45841
<223> exon 44

<220>
<221> misc feature
<222> 45842..47841
<223> 3’regulatory region

<220>
<221> allele
<222> 31646
<223> 99-79310-29 : polymorphic base A or C

<220>
<221> allele
<222> 42373
<223> 99-79311-50 : polymorphic base G or C

<220>
<221> primer_bind
<222> 31618..31635

<223> 99-79310.pu

<220>
<221> primer_bind
<222> 32080..32100
<223> 99-79310.rp complement

<220>
<221> primer_bind
<222> 42324..42344
<223> 99-79311.pu

<220>
<221> primer_bind
<222> 42704..42723
<223> 99-79311.rp complement

<220>
<221> primer_bind
<222> 31627..31645
<223> 99-79310-29.mis

<220>
<221> primer_bind
<222> 31647..31665
<223> 99-79310-29.mis complement

<220>
<221> primer_bind
<222> 42354..42372
<223> 99-79311-50.mis

<220>
<221> primer_bind
<222> 42374..42392
<223> 99-79311-50.mis complement

<220>
<221> misc_binding
<222> 31634..31658
<223> 99-79310-29.probe

<220>
<221> misc_binding
<222> 42361..42385
<223> 99-79311-50.probe

<220>
<221> misc_feature
<222> 26,350..351,353,356..357,366..367,371,383,389,392,396,399..400 404,411..412,416,421..422,435..438,440,442,446..447,450,455 457..459,461,463..465,1805,2175,2328,2402, 2561,4048,4661,6659 6799,7509,7534,8289..8290,8294,10663,10789,11052,12083,12904 13017,13916,14116.. 14117,14680,14971..14974,17093,18448,18669 18767,18808..18811,18838,18971,18985,20714,20903,21355.. 21367 22901,24093,24112..24115,24171..24173,24868..24869,25356,25797 25879,26062,26234,26824, 27130,27254,27437,28745,28781,29278,29475 29526..29528,29617,31152,31790..31791,31796,32011,32262.. 32273 32296,32415,33656,33719,39140,39142,39343,39944,40945..40947 41948,42166..42169,43371..43372, 46709..46720
<223> n=a, g, c or t

<400> 3

```
gtttaaaaaa aaggtgaagg cctctnggct tttttttcccc aaattttttt gggaaaaaaa     60
agattttttt ttccccccctc cacaagggcc caaaaaataa aaacccttcg ggagaaaccc    120
gcttgggaat tttgggggc tccgggaatt taccaagggc ccggcccggg ggggggggtt    180
cccccctttta ttcccccccc ttttgggggc caaggggggg ggtttccagg gttgggggat    240
taaacccttt ttggttaaaa aggggaaacc cccttttttt ttaaaaaaca aaaaaaaaat    300
aacccggggg gtgggggggg gccccttttt ccccccttcct tgggggggtn ngncannaaa    360
aagggnntaa ncccgggggg ggnggtttnc antaanccnn aatnccccccc nngccnccca    420
nncgggggggg aaaannnnan ancccnntcn cccanannna nannnaaaaa aaaaaaaaaa    480
aaaaaaaaaa aaaaaaaaac agtggcctaa gagcatggtg attttacatt atccactcat    540
acccagccaa agctgaagat tgatggtgac agcagccagc agatgagcat agacacagat    600
ttcatgatgc atttatcctg tttcctagag cagtgaggtc tgtgcgaggc tcagatggaa    660
```

```
agcctaagga gactcagcca tcatagagag caccagcacc aagcagaacg attgaaaaga     720
aattggcacg gtgagacggg gcttgagtct tttgtcttc tcaaatccaa acataagagg      780
ccgaggccga ggccaaaaac gccatgagtg aaggtgacgt cggctgaagt tctgttggag     840
aacttcagcc catcttacta aaagaagaga taaatccaaa gaaaggggga aagtagatac     900
accaacattt agatcacaca cagttcctgt taaagacaac gaagcttcag ttgctgcgcc     960
catagtttcc agttgggaag atgtgttttc ttgtatgagg ctgagcactt cctccctctg    1020
ctcctttgtt tgttgtctta aaactcaaca gatgtcatga gatcaatata gatatagata    1080
tggagcctag gactttgtca ccaaaaccct gtaaacaaac gctgaggata cctttttgaca   1140
aatagtcatt aaatgtcagt tgtgtccaag gcactgtgat aggtgaaatg aaatgtggaa    1200
gggaaacatt aggaggtcct tgcttctgtt ttgtggcatt ttctgtccaa tcagaacgtg    1260
tttcatgttc aagcaacagt aatataaagt cagcagtttt caaaagccta tgagaagcag    1320
aaataacatg taatctatga tgtcagcatg ggaaattgtg cttccaactg ggagtactgt    1380
actgggaggg cttcagggag gaggtgggat ttgagatgag caggaaagga ccttccaagg    1440
agagcaagca gtgaagaagg acacggagca tacaaagaag ggtcacagtg tagaggcagg    1500
gaccgaacaa ggaggaagga gcatgggctt cgaaatcaca gcttctgaga ttggaatcct    1560
acttcaggag aggtgatggc ctggtctggt cacttaatcc ttctggaacc tatttctgcc    1620
ttgaatgctt ctcacacaat gatattatga agattagatt accttgttta tgcgaagtac    1680
ctaacctgat ctcaacagtg agtgttagtt tcattcccgc ccatgtagga aaaaactgaa    1740
gaacaaggcc agaaatgtaa gttagggaga gatcctcaag tgcctgaaat gcctggctaa    1800
ggggntttgg aatttcgttg atgagaagtg agaaaccaca aaaggttggt aagaagagga    1860
atcagacaca tcctagtgcc cacaaagtac aaagcaccat tcctagtgca gtttttgtta    1920
cccatatcct aatgaggata tccattccta atgaggaaaa caggtgtaga aagattaaat    1980
aatttgtcca aggtcaccca cctaataagt ggagaagcag ggaactagtg tccaggcacc    2040
ctggcttttc accaagatac cagactgctt catgcaatca aaggtgggct caggtgggtt    2100
caggaagaca aattggaaaa ggcagtgagg ccaaatatgt caagggacct ttatacaata    2160
cactacaaca cagcnctttc caaataatgt tctacataaa aggtatggt tatggaaaga     2220
taatcgcaat gtagttttag ctgaaaaaag tgggcgatgg aagaatataa actttatgag    2280
ctcagataca tataggaaaa aagactggaa ggatatactc caaaacgntt cattgtgttt    2340
gtctgtggca attgtgggta agtttcttc cttttatttg tccaggcttt ataaattaaa     2400
gnatgtgtta tttgcattaa aatattctta ttttaaagaa tcatgctggt aatccagaaa    2460
gaagtaacgt tggcattact aaactcatcg tagtaagaat ggagacttgg gccaagaagg    2520
acagggaaag cccagggaac cctaagcttg atgctgggct nctgctggga tgctggtcca    2580
gggacagaaa ggaggaggcc aagaggagac atggcttagg gtcaaagagg acgtgaagca    2640
gattaaaatg gaatttttgt tgaaatattc aaacagtgat caagaatttt accatctaca    2700
ccttgaaccc agaaaagaga tttaggttca aaatatgacc atcatagcca aagaaacaaa    2760
aataaccaaa gggagaatag gaaatgagtc aaaaacttcc tatagatgtt taattttttaa   2820
aaccttttta acagctttat tgagatgtaa tccacatacc acacaataca cccacttcaa    2880
gtgttcaatt caatggtttt ttagcgtatt catagagttg tatacacgac agccactttt    2940
agaacatttg tatctccttg aaaagaaacc ctatgtctct taactattac ccccatccct    3000
ttagcctcca cctcaagacc taaacaacaa ctaatattct ttttgtctct atagagttca    3060
ctgttctgga cttctatatg aataaaatca taacatatgg tcttttgtga ctttttctc     3120
aattagcata atattttcaa ggatcctcca tgttgtagca cgtatcagga cttcatccct    3180
tcctatggct gaataatatt cacttgtatg gttggaccac attttgttta tctgtttgtt    3240
caaaaatgga catctggatt gtttccacct tttggctatt gtgaataatg cttatataaa    3300
cattttatac aagtttctat agggacatat attttcatct ctcttgggta gatacctagg    3360
agtggagttg ctggctcata tggtaactct gtgttagtgg tttgaggacc tgctagaatg    3420
ttttccaaag tggttgcacc attctacatc ccaccagta tgtaagggtt ccagtatgag     3480
gattgcgatt cctccacatc ctcgccaatg cttgttgtta tctgactttt tggttatggc    3540
tttctcagtg ggtgtgaggt gatatatcat tatggttttg atttgcattt tccggatggc    3600
agatggcaca tgatactgag catcttgtcg tgtgcttttt ggccatttgc atatcttcct    3660
tggagaagtg tctactcagg tcctttgctc atttaagtaa tattctcagc atcgattttt    3720
aactgcctgt ctccaaagtc tttgttacta tttgctgggt agctgtccac atcactgttg    3780
ttagataaag ggcttttggg attccgtgaa agaagaaaga gtttcctatc ctttatcttt    3840
tctgagttca ttttctgacc aggtttcccc tctcctcttg tttttctact ttaggaagat    3900
tgcaatggca tattcagaat taatgtcagt gtgtcaaaga acttaaattt aaaattgagg    3960
cctggagaga aaaaacctgg attttgggtg ccccgtgttt ggtaagtata tcataaaaaa    4020
atattctgaa gaatggtgtg tttgtcanta taaggcttta aataagtatc atatggcatg    4080
taactaaaag tagttttgct agagatgtta aaatgatctt aagtcataca gggcgaagcg    4140
ctagattata ttggaagata gaaataaggt aaaaaataag agagcaagta gtgcaatgag    4200
ttctgtatag cttcatgaaa ctagagaaat attatctcag tatttttcta atacttcaga    4260
aatatgtatc tggattcaaa tgccttaaaa aattaggctt aacaggattt tctgtcaaat    4320
taaaactaaa cttatcctga gtttatatct ttaaaggagg atctgatgtc ctcataatag    4380
aaccctaaat cataaagcta tttccatttt aaaacccatt tacactgaag gacttccatt    4440
```

attgaaacac tagagaggaa taatcagtga cacagttgat tgcttctaac tttccctta 4500
atattccttt ttctttaaat tgagttttca aagcacttag atcaaagcta gaaataccta 4560
gtaccttctt tcttatttta ttaatgacca tgagtgaatc tctcagtctg tgtcattggt 4620
gaactgagtg ttctgtcacc ttcatttgtc ctttaaaatt nttatctgtt ttttaagttt 4680
tcttttagaa aatgtgtaag aataacaact gagtcacagc acctctgctt ttgtcacaat 4740
tgatgttctt ttctaaatgt caaaactctt taaaaggaac agcatttcac ccagctgtac 4800
cctagcactc actgttacct ctctttggca tttttctcat atgaaattcg cttttatacg 4860
taatccaata gaatgcatat tgggttttta ttttttaaaa tatccaagaa ctgagaaaaa 4920
ggattcattt ggggtatgtg gcttcagctt ggagagagag gaatgtgaga catgagaata 4980
tcctggcccc actgctgact agaaaagccg tccaatttcc attgaaataa aaattaaagg 5040
agcataaaaa ttttcttcca tgagataaaa attaattata gcataaagtt atttatgtta 5100
taatttatta atttttatta attcaaagat aatatcatgg aagtaaatgt agtcaggaat 5160
agagaacact ggggcatttc atttccttca ctatagagaa aggtagaggg tgcctcccac 5220
ctctgccaag tgaggtaccc caggggggatc acttaacgtc agagatgggc agggcaagaa 5280
gagagactct tgtggccagg ctgctcatca ccatctggag agcagggtta tctggtgttc 5340
ctggagagcc cagtagtcac tgcctagggc cacatcatct gagtcctctt gagccttgcg 5400
gcaaagggct ttgtgctggg aaagggtaac cccctccaaa gggtccgtag gtgtgtttcc 5460
aggagccaga cttgagaatc acccagagac tgcacactta caggagaaaa ggtgcaatga 5520
ggcactcctg ggctggagag ggggttcaaa ctcttgaaca cctatggcca gaatccaatt 5580
gtatcagatg cacaagactt aaactccatt cctccattcc tttcaacgac atcccacctc 5640
agtcatgtga gaatctcgtg gtggcctgaa gaggatgagt ggaagtgaag aaagtggcca 5700
acttttcttc ctttcctcaa accggggtcc ttgagctgct aaggatggga ttgttatatg 5760
agttatagga tcagttggat gtaaaactga attactgaaa ataaccaaaa agctctggga 5820
cctccccaag aattcagaca gggagctgga acacacttga aggtagtgtt tggagaaaag 5880
taaacctgcc agttgtgccc tatagcactc aacctagtcc ataaaaccag ctacatggga 5940
tttttaaaat caaagttaat ttaaatgcaa tattttatgg aaggtattat taggggtttc 6000
aaaggcaatg ccattctaga agcataagca aatagccagg cattccttga acatcatcac 6060
aagtgctctt gtccatttct ccaggataat gtgacttgga ttgtagtgaa atagacacct 6120
tcgatctaat aaatgtcatc tgtaaccatg ataacttttt acactttcct tcttaagtaa 6180
gctaatcaag agttggaagc tgaaattttg taattttata ttggctaaat tatgattaag 6240
aaaataacat atctaggaat acactagagg aatgctttgg taattataat taggacataa 6300
atatgcacta attcaggaga cagtctagtt tcaatattct ctcttcagat taataaatgt 6360
actttcttct gaatcactca gacttgatga gtcacctcag taaaaaggat taaacatatc 6420
atggattata aatacagtgt gatttatttt aagaggaggt ctggaatata ggaaaatggc 6480
atgcatggga gaaaaactaa caaagtattc aggcttataa taaatgtatt tattgagtgc 6540
ttgctcttca ccagctagta tgcttgtcac tgtggtaagg ccctgaataa gcgatggccc 6600
ctacccctta ggagcttaaa gtctagtggg aaacagaaaa taaaattaca aataatttna 6660
tataatatgc taagggcttt aatgctgcac agagaagccc ctaagcttac ctctggctca 6720
cccgaaactc atctccatgt aatgttaagc tacactgtga tatgttctag gaagaaggtg 6780
atcaggtttt atagggtcna aggcccaaac aatttggggt accaacatct cagaagggcc 6840
tgtacaaaag aggggtccta cccccttaggt ttctttggct tcatcatata cctgcctctg 6900
attcctacag aaaggctaag ggtacaatgg acacacagga gaagcaccta accccaacta 6960
tgcagggcca aagagaagga ccagatgaga agacacactg gttacctttt gaaggacaac 7020
caataggaac tagccaggtg aagggggcag ggtcagaggc aggtgaggag gaaagtctag 7080
gcataggttt ggtattacaa gttaaggcaa ctctatgccc aaagctcaag gcaaaaagcg 7140
ttggggtgct gtaggaagta cgacgtttct gatttctttt aggagagatg gttgtgtaag 7200
atacagcatg ccgtttttgg acaaagtctt tttctgcatt tccacatcca agggcagacg 7260
ctcagtcacg tcttcagggt gcccccatgt tccccaaggg aagagcagct ctccctacct 7320
tgatcttggg gggccttgga gcacctcctc tgggaggtaa aaaactgaca aaattatgtg 7380
gtcagatcta atgtcttagt tttctgaatt ggacaggcaa gattacaata tttcacaaat 7440
taaccagaaa aaatgacaac cttttcaatt caaggaaggg tagcaggtaa aaatgacaat 7500
aatagatgnc atttgggatt tattgaagga ctcngtagtt acaggtaaag tttcaaactt 7560
tgcaactttg ctagcatgaa gaaagcataa gaaaacactt tacatgatta aaactgaaaa 7620
agacttattt ttttaatgtg ataaattctt ccaactattg aagtctttga tgctacattt 7680
tgttttactt ggtttagtta gaatattttt cacatattct aacttctaga agatctagaa 7740
cataacagta gtttcagtgg ctgcaaattc agctaagaaa gcacttctac aaaagaaaat 7800
gagcaggtaa ggaagtccaa caccggctct gagaaccggt tcacttgctt gtaaaaaatc 7860
cttattcaag caaactgtgg catctgtaga atccttataa cttctgtcta ctgtaaaata 7920
tatgaaattt agtggaaaca tcccaaagga aagattgttg gaagcatcca aaagaatata 7980
tttgtacttt catacataaa aaaatataaa gcagaattcc aaaatatgca tttctgagtg 8040
gggcttttgc actatattaa agaccgtctc cactaatcag ccctaagccc ttaagaacaa 8100
atcagtttca gtttaatgtt ttcttcttat atcctcatct atttaatatt caaaaaactt 8160
atgaaggcac atcttgtgat tgtctctata gtttaaaaaa cataaaagat ggtagaaaag 8220

tacagaacca gattttccct agagcgttgc ctgtcctgag ggttaaattt tgagaaatga 8280
gctagcagnn gggnaaaaaa tgatcaaaat ataatttaaa taggctccaa ggggcctact 8340
ctagtcagtg gtccggagct cagttcttca ttaaattcaa gaaatatagt ccatgatttt 8400
ccagtgtttg gttatcaaaa tcttaattat ttaaaattag cctctaataa gaaggaaaga 8460
gggggaaaag ccactgaaag cttcttctat taacaacaaa aatgttagct aagaaccata 8520
acaattaaag aagatgcaat ctagtaaaaa ggatcacttt tataattggg aagagaaaag 8580
tataaccatg ttaaaagaac aagaaatgag acatccctgg cccccacaga agtgggagct 8640
aaacagtggg tacacctcga cataaagttg gaaactctgg acagcgggga cttcaaatgg 8700
gaggaggaag gaaggaggtc ataggttgga aaaattacct gttggattct gcgtgcacta 8760
tctgagtaat gggttcatta gaagctcaaa ccccagcatt atgcaataca cccatgtaac 8820
aagcctgcac gtgtaccccc caaatctata agaaaacaga aaatgaataa gccaatgagt 8880
ttcaggaatc aggaatcagc ggtggaggta gaaagcatac cacccataaa ggggaccaga 8940
ctaagagtcc actgagaaga ctcagagtta ataagaaaaa atccaactgg aagagagtaa 9000
tttggaaaaa tacatactac aagcaaacac ctcttgccgt ctcagtgacc tctaaatgca 9060
tcactacttt ccagccctgt gttcaccatg atgacacaaa gtgaaataaa gtgactaaat 9120
tctactctac actttctata ctaacacagg caattgtaat ttatgccttt cgaaagattt 9180
tcagttacaa aactatttca aaatccacat ccaaaatcca atgcttcttt gtgcaaatcg 9240
ataaaatcat ctgtccagtc ctcagggtaa tcaggacaag gtagaaatgt tgcagaacct 9300
ctcaagctag aactagagct gttttacatt gagatcaccc catcattacc taatgaacac 9360
aatcaattat aacacattga tgcattgtta tgatttagca aacgaatgcc atatagcgta 9420
aaaatgtgtg tgtatacata tacacacata tgtatgtgtg tgtgcacgca cacgtgtgtg 9480
tgtatatatg tgtgtgtata tacacacaca cacacatatc ccacactcat gactggaaag 9540
ggctacagat atttgccttc gggtgctgct tattggaatt tcacagaaca ttcctgtatt 9600
ggaatcgcag ggcgaatcct cggaacttta atttcgacaa tgtgggaaac gctatgctgg 9660
cgttgtttga agttctctcc ttgaaaggct gggtggaagt gagagatgtt attattcatc 9720
gtgtggggcc ggtaagcgag cacatcgaat cctttgaaca cttcaagagc agctttcatt 9780
tgttttgatg aataaccata ttttccttta tgcagatcca tggaatctat attcatgttt 9840
ttgtattcct gggttgcatg attggactga cccttttttgt tggagtagtt attgctaatt 9900
tcaatgaaaa caaggtaagg atttttggtt tggataccac agggtttagc cttttctagc 9960
cactcacttt gagctatttg tttcttttca cacgcggcgt gaagccgctt taaactgtaa 10020
tgtgactggt gtaaataatc tctataactg ttttacaagc tccctcattg ctgctttttca 10080
gacacattat ttaagagtac agttctcttg ctaaatttac ccttttggta acagtgtcat 10140
taattcaccg acaactgaga ggcgcgtctc ctattgttca aattgaaaag aaagatgggc 10200
agcaagttat taaaatacaa tagatgaaag tgattcgcta attacaggta atattacttt 10260
cactaaatgt aaattgtatg cttctgtgta actcagtagg tgaactcagg attttgatgg 10320
gggtgagggg agtctggtag aaatttcaaa atcctaattc acagaaaaaa agattttgtg 10380
gcagggcatg gtggctcacg cctgtaatcc cagcactttg ggaggccaag gcgggtggat 10440
cacttgaggt caggagttca agaccagcct ggccaacata gtgaaaccct gtctctacta 10500
aaaatacaaa aattagccgg gcgtggtggc gcatgcctgt aatcccagct actgggttgg 10560
ctgaggcatg agaatcgttt gaacctagaa ggcagaggtt gcagtgagcc gagatcacac 10620
cactgcatcc agcctgagca acagagcgag actcaaaaaa aanaaagaaa aaaaagaaaa 10680
aaagattttc taatttcata tgaacttgac atactttgtg aactcgggaa gcctgttgag 10740
tttgtaacag ctctaatgat tagaacaaag gctcacaaat gggaaaatna caatgtagtt 10800
tcctatcaaa gtgtgagttt ggggaagaac attctattag cactccttgg agaggggctt 10860
gctcatctag gcacttgagc tatgccctct attgtttgag acgcagtctt tggaatcata 10920
gtaagggaag tgcatgcagt ttttgagcac acactgtgta ctaagatttt acatattcaa 10980
tcaattctca caactctcaa atgcacggtt gcagaaaaag caatgactag tctggggctt 11040
ataaggtgtg anaaacaggg ccagaattca gaattggtgc ctattaccgc aggctctgtg 11100
attcttcgaa ggcatgagta gaataaagtc aaccttcact ctgcattaag ttgaagctag 11160
atctgcctgt aaagcaccgt gctgctcgat gttattcatg gctaggactg attcccatgc 11220
gttcacatca gagctcttca gtttgtgttg ctacacatgg gactgtatct gtcattcact 11280
gtcctcaggc tcaacgtgag tggtaccagt tagccagtca attgacacct ttgatgtgtg 11340
gctaatgagc atgtcatttc attcaggctt tcaaagggtg agatttaaaa attcctcttt 11400
tgggccccga ctctacttgg accctcagaa acatgggatg ttagagacag aaagaacagt 11460
agtgatcatg tcacccagcc ccttatttta ctcctatcac attcattaac gagatgcttt 11520
gagccctaca gtgttgagtt taataccaag cagaaagagg aagaaggatc tttttagatg 11580
cctttgagcc atgtaatgca gcagacactg agaattctag aatataagca gaagctaatc 11640
agtcaattaa caggagagtt taggcactgc ttgcccagaa caccgttaag aagccgtctg 11700
agcagacacc tgatttcagg aagattagaa tgtatcacag ctcacaattc agcaacatac 11760
atagaacaac ataaaatgtt cagtaactgg aaataaatgt ataattaatt gcaacacttg 11820
agcttagaga agtggctgag gttagcagaa taaccaggga ggtctccatc cagcactgta 11880
tttatgttct gcaggtagga aagagtaacg tggcctgttt gggggatctg ggcgtatgt 11940
tctatcagct caaatgggca cttaaagaaa ggagaaattg acaagaaatt atattaccaa 12000

gctttacaaa cttgatttgt taattggaga tatcaattgc taaattatgt gagcctttta 12060
gaaaagtttc tttctctgca agnttttttt tttttttag aaagcagctc atgagctgcc 12120
atctaacagc atttatacat tattcaatca ttcaacagat gtatttgagc atctactagg 12180
tgccagaggc tgctcttgac actgaggacc tagtgaacta acctaaacac taaacacaga 12240
aaagttattt gccctcatgg agcacacatc ttaatagtca tacccaaagg cacataggta 12300
aatttccaaa tgtagagaca acagagatag aaaatgaggg aatctttagg gtacaattca 12360
gaatcatgca aaaatatgtc ttcttttgaa aatggaaacc tctttaaaga gttccaagtc 12420
tatattagta atatagattt ttttctgatt tctctactat gtaagacaat gtgtttgatt 12480
tattaaccta acttgataat aataaacttt cagaggtttt tatatattaa tgatcagatg 12540
atatctccat gcctattatt cttttagatc ctttcagagt tcaactttcc aaaatttttt 12600
ttatgtataa caaagtaatt acattagtgt ctatggtggc cagagaacaa gtgtttgctt 12660
ataattagtg agaaaagtgg tttagcaatg tgattatcag aaaagaacaa ttttaaacaa 12720
acactattat tgggaataaa tgatgtacta aagaatgaca aaataatcac cagaaagata 12780
tagaacttcc aaatcccaaa atgtacaaag caaaaattaa acaagttgta aaaagaagct 12840
agaaaatcca aaatcatagt gaaagatatt aacatatatc tcaaaaaatc tcataattca 12900
gagnagaata aaaggaaata tgagatttga tacaataaac acaatcaagt gaatgtataa 12960
ggaaaactct aattacaaca atttgagaat gtgcattatt ttcaagcaca aaatgtntct 13020
cccagaaggc cataattacc caagaattga taatgtaaga atgttttctg accataatga 13080
aaacaaaaac aaaaaaaact ctacaatttg gaaatattaa gacaatacta taaataattt 13140
atgagttgaa gaaattataa tagaaaatca caaaatattt acagacaaaa cattttatag 13200
cgtcatatac aaaaactaat gaggtatagg taaatcagta attacaaata aatgtatcat 13260
tttaagtgta tatattaaag aagaatcaca agttaaatat taatatgtca aatatccaaa 13320
tcaataaatt ataataaagt aaaatcaaat aaagtagggg ggaagaaagt aacaaagatt 13380
aggacacaaa ttaatgatac agaaagtaag gaaacttgta tagcttctat aatattcaag 13440
aataacaaaa gctgggtttt ggtttttttt aaatcaagtc aataaacctc taggaaggtt 13500
gaacaaaaat aaaaacatga aacaagaaag taagctacaa tcattaaaag agtgtcagta 13560
ttgttatagg tatagaaaat tagacttaca gaaaaaacta gaaagaccca gcagggaccc 13620
atgtatgtag aaaacagaag cagctcaaca aattagtgca gagggtggtg agaccaacaa 13680
ggagtggtgt tgggagtatt gattgtattg atgtaaagag ctatgctgag atctctacct 13740
cactgcatac tcaaaaatga attccagatg aattaaagac ttcaatatga aaagcaaaat 13800
ttaaaccatt tttaaagcat gtcagcattt atttttattt tgaatttcag ataagcagca 13860
aataatgttt ttgcctttat tatttttaat cagcgcataa ttgtacatat ttatcnggta 13920
cagtgtgata tttcaattca tgtattataa tgtgtaatga tcaaatcagg ataattagca 13980
tatctatcac ctcaaacatt tatcactgtt ttgtgttggg aacattcata atcctctctt 14040
ctaggtattt aaaaataaac aattaattgt tgtcaattag attcaccata tattaccatg 14100
gaacactaaa atttannttc ctccattcta gctgtatttt catatccatt aatcaacatt 14160
tgactatccc tattctagtt cccctcccca cccctagtaa gcatttgttt tttgccctgg 14220
agtagttatg gatttcatcc cattagccac caagtgaaat ctataaagga aaaaaattat 14280
aaactcgatt atatttaaat taaaattttc tgaatgacaa aagacactct aagcaaaata 14340
aaaatacaag ccaagactaa gagaaaatat gtacaactta cataattgat aaaagattag 14400
tatccagcat gtataaagaa tctctacaaa ccattaaaaa gtaaatatca aaatacaaaa 14460
atgagcaaag gatatgaatg ggcaattcac ataggaagat acaagcatca ccatttacac 14520
acatgcaaat gtttcatctc gttaatatta aaacaacatt agatacattt tcacacctat 14580
caaaatggtc agattacaat gtctggcaat atcaagtatt gcaagaatat taggatatgg 14640
taacacctgt gtattgctgg taggaaattt gttttggttn ttgctctttg gaaagcaagt 14700
tggcaacatc tggtaaatgt aaagatgctt aaattcctag accccatcaa ttcttctcct 14760
agagatcaac aatggagaaa ccctcataga tatttactgt gagacagtac atggatgctt 14820
attgcagcat tggttatagt ccataaatca aaaatgaata acttcggata ttcgtacagt 14880
ggatggtgtt tgtgagcagt taaacaaatt agatttagta atctagttgt atggaaagta 14940
tactgttcaa tgcagatata aagaaacagc nnnnataatc acatgttgga gtccatttct 15000
ataaatatta aaaaattcag aacaaattct tacgtattga ttcatgcgta agcacaaaag 15060
tctgaaaaca tagtaaggag gatgcacgcc tgaagggtag tggttagctc tgggtaagga 15120
aagggagagt ggggtttttg tctagttttt aaaattacac ttcagctttt atgataaaat 15180
ataatgcagt gcagtgcaat acaatataat ataatttaa tcatatgcat tttaatcaag 15240
tactctatta tacagaatat ggaattccat ttaggcagtt atcacagcac tactttctta 15300
aaatgcaaac tctatgactt tgtatctctc aaaaacagtt ttgcaattgc acatattaga 15360
cattttttaa agaaagacaa catacctta actggaagcc acagcattga caagctataa 15420
ttttgctgct atgaaatgaa aggaaactaa ctactattag tgagtatcct tttttgaaaa 15480
tgtaattatg tagaaactcc tctttacatt ttcccaaacc tgaatattac acatctccaa 15540
agatacaagt cgaaggctgc tgaattctct ctgaaacacc tggtcatcat tttgggaact 15600
cctcccaaga ccaactttga gcattctgac catgtttctc tcaccaccac ggttggaaga 15660
acatggtcag tgcaagccct cacatcagtc ctgtttgtct cggcacccca tctgccatgt 15720
tcttggtgca agcacatgac aaaaggcctt ttcctgcttt cttttgtctg ttaaggggac 15780

ggctttgctg accgtcgatc agagaagatg ggaagacctg aagagccgac tgaagatcgc 15840
acagcctctt catcttccgc ctcgcccggg tacccaaaat gctctgattt tattcatgtt 15900
gggcactagt gtgcacgatt ccccaggagt ctcagattta aattaaaata ataatcgtga 15960
ggccgctggg aggttgtgca taaggtaata cagaaacagg ctccaaacca cagatctctc 16020
cataaggcta ttaaaatcaa ccacccaaag gctgtgcttc agtgctctca gactaagcct 16080
taatgaaaaa atgaattttt aaataaaata tatttgctct tgcctttttaa aaatataagt 16140
gaaatgtatt aaactgacag aaattgaatg atgcacttct ccttccaata tagacggcat 16200
catgcactgt cgttttgaat attcagagcc agccattgct gcaaaaacac ctcacaatag 16260
acggaactgt ttggggagaa aaatgtaatg ggttttaaga taagcattta agaaaataaa 16320
cgacaagaaa gtaaatgtga cctgtgtcta aaatgccctt gcccaaacct ttcttttcta 16380
gataatgatg gttttagagc taaaatgtat gacataaccc agcatccatt ttttaagagg 16440
acaatcgcat tactcgtcct ggcccagtcg gtgttgctct ctgtcaaggt acttcgtttc 16500
accctaagac tccgggaatg aattttgtat tcactgctac atccgagagt atcacaagct 16560
gctatatgca ttcacggtta tgttcttggg tgtaaaaatt aagattcact gtcaatggca 16620
aagtgggcag aagaggggca agcctgtatg tgtccgatgg acgtgggcaa cgactcgtgt 16680
gctgtttctg ttgcagtggg acgtcgagga cccggtgacc gtacctttgg caacaatgtc 16740
agttgttttc accttcatct ttgttctgga ggtactgtaa tgagtcagct agctctgggg 16800
gaatctgtgc acagttttaa agtaaatcag cctccctctt tgatgccact tactctcctc 16860
tctgtgttcc attgcctttg aagtttgccc ttaggatatt tcctacttaa gcggttctgc 16920
ttaactcgaa caaagacatc actttatcca tcagattttt ctaactacag aaatgctgct 16980
gtcgattgtt gtgtctgaac attggcctgt ctagttatgc gagttctgat atttagaggg 17040
aatggtgcaa gccactataa ttcattgact caaattgcaa tagagacgca aancaagttg 17100
gcccagacaa ggttatttta acatgtgatt ctcataatat tgctttccag ggcagtcttc 17160
ctgaaataca agcagattct tattttgccc tacactggtt gatgtttccc aaaatctatg 17220
gccttttgct taaggaagaa aaatgtccca tgatcagaac tagtcataaa acgaagagga 17280
aagagtgtag agaacacaga tttgtaagct acttagcttc aaaaataaaa tcatatatct 17340
tggtaataaa ctcttatttt tagttactac atgtttacaa ttattcttta aatagatagt 17400
ataaccgagc atgctggctc actcctgtaa tcccagaact ttgggaagcc gacgtgggtg 17460
gatcacctga ggccaggagt ttgagaccag cctggccaac gtggtgaaac cccatctcta 17520
ctaaaaatac aaaaattagc caggagtggt tgcacgtgcc tataatctca gctactcggg 17580
aggctgaggc aggagaattg cttgaacccg ggaggcagag gctacagtga gccaagatca 17640
tgccactgca ctccagcctg ggtgacagag cgagactcag tctcaaaaaa taataataaa 17700
taaatagaca gacagacaga tagatagtgt aggcacgtgg cctaaaagga aacctcaaag 17760
ggcattaaag tgtttgtctc atttcaatg gaaaagatac tgtgtccaca tcaaataatt 17820
taaaattttc ttcatttgaa aaaaatacat acacataatg catctcaagt taatacattt 17880
aaatatgtac taaacactct ctctgtttat tttcttcttt gctttttctc caggttacca 17940
tgaagatcat agcaatgtcg cctgctggct tctggcaaag cagaagaaac cgatacgatc 18000
tcctggtgac gtcgcttggc gttgtatggg tggtgcttca ctttgccctc ctggtaagca 18060
tagaagtcaa gttgatttca gctgattatt tcagtttggt tcagttctgc tcagtcctgt 18120
ttccagtctc attttactcc acatcaacct gctctgaggt gttagatgga cctccctaaa 18180
gcccagcttt gactgtgatg ggctctcaga catgcttgtg tctacatgta cacacccccca 18240
cactcttcac acaggcaaaa cctggcccct caaagaccta gacgatgcaa gctagaaaga 18300
gaaggacctt caagatcttc tggtttagcc cctcatttta ccaaagggga aaacaagaaa 18360
gagagaggaa gactgcaaca gagactgaga actgtccaaa ttcacacaag ctagtatcag 18420
aagactatag tcctgttttc ctgttttnct cagctgaaat atagcatttg ttccactttg 18480
ttataatcag tatttcacaa tgtgattctc aagtgcttcc actgtttcat tttctataag 18540
acatttagcc tccacttctc taaagcatat gattctccat ctctatatct gtgttctcac 18600
tattttacct aaagtgcctt caccctccat tcagtctgag atcccactca aaatctgcag 18660
ctatgggtnt actgaactgt aatctagggc catattgtcc aatgaaaata aaatgtggcc 18720
acagatatca cttaacattt tctagaagcc acaataaaaa agtagtngtt tagaaaatat 18780
aactattaaa ttgttaataa ttattaannn nataaataat aattaattat ttaattangt 18840
aatatatttt attatttaaa atatgtatta attaaaagat gcattatgtg tatgtgtatg 18900
tatatatttt tattatttat atagttatat attatatatt aatatattta attaaataat 18960
taatttaatt nattatataa ttaanttaat aattatttaa ttatataatt atttggttat 19020
aattatttat gtataattat tatttatata cttataacta ttatgtatgt aattatactt 19080
atgtaattat aattatttat ataatttaat tattaacaat ttaataatta tattttctaa 19140
actatatcca aagtattatt attttcatgt gtaagcaata taaaattatt gagataagta 19200
gtatccttgt atctgtgtgt gaagtctttg gctgagtatg tatcttacat ttacagcata 19260
tcttcattta aattgccaca ttttaagtgc tcagtaacca catgtggttc catgtggctg 19320
ctggttacca tgttggatag cacagctctg gaagatatgg gtcctagtct aggttccacc 19380
attttccagc cttgactaat ttatttgcct tcaatgatcc tttgtttcct gctctgaatg 19440
gccaatataa cttcataatc cacctattcc ctctggcatt atgaagatca agtgagataa 19500
tatatttgag gctttcttga actagaaaga ttatacaagc ctgtaggcat tattataatt 19560

accttctttt tgttagtcat acttgatctc atcccaagga atattatcgg tttttccctg 19620
tttccttcta tccattctcc catcccctta gtgttagaga catgctggga aagactcatt 19680
tacacatcct aaagggcttg ccttaatacg agacgctgtg ctcatgaaca tagtgagcgt 19740
ccttcttaaa ccagtggtta ccttcagcat caggggctgt atgaagccta ggtgagctct 19800
cagtcatcct gaagtatttt ctcagcaata aatggttctc aattcctagt acaagacaca 19860
gtactagtaa ctgagaagaa tgtaataagc aaaacaaata tagttgctgc tcttgtgaac 19920
cttatatcct agaaaagtag atagatagtc atcaattgct cacaaatcta tgagtataat 19980
gactatgatg agggagatga aggagagcct gtgttcctgt gacaacatag ataatagtgg 20040
gagatggctt catcagagag gtcttggaag acatccccaa gggagtgatg gctccatctg 20100
taggataagg ggagggcatt tcaggtagtt gtccctgtga tggaaagatg acagagctgg 20160
aagaaggata gatacatagt actagcatag tacaagattt ggatgggtaa gaaggtatca 20220
gacctcttag gaccttgcag tccaggtgaa gaattcagat tctcatccta aaaactattg 20280
gttgtcatta cttccaaaac ataaagaggg aaggtatctg caatagatct ttagctaaca 20340
tgggaagaaa aggcagcaag gcccatgttt ttcctttctc cagataaaat ttgtattagt 20400
cccttctcac actgttatga agaaataccc aaaactgggt aatttataaa ggaaagatat 20460
ttaattgact caaagttcca catggctggg aaggccccag gcaacttaca atcatggtgg 20520
aaagggaagc aaacacatcc ttcttcacaa gggaacagga gagagaagaa tgagtgccca 20580
gcaaaggggg aagtccctta taaaaccatc agatcttgtg agaactaact caatatcatg 20640
agaacaggat gggggaaact gcctccctta attcaattat ctccacctgg tccctcccac 20700
aacatgtgga gatnatggga actacaattc aagatgagat ttgggtgggg acacagttaa 20760
accatatcat tctgcctgga cccctcccaa atctcatgtc ttcacaattc aaaatacaat 20820
cataccctcc caacagtccc cgaaagtctt aattcattcc agcattaact taaaagtcca 20880
agcccaaagt cttatctgag aanaggaaag tttctgctgc ctatgagcct gtaaaatcaa 20940
aagcaagtta gttacttcct agatacaatg ggggtacagg cattgggtaa atatacccat 21000
tccaaatggg agaaattggc caaaacaaag gggctatagg caccatgcaa gtccaaaatc 21060
caatagggca gtcattaaac attaaagttc caaaataatc tcctttgact ctgtcttata 21120
tatatccagg gcacactgat gcaagaggtg ggctcccatg gccttgggca gctccaccat 21180
tgtggctttg cagggtacag accccctcct ggactggtat tgagtgtctg tggcttttcc 21240
aggcacacag tgcaagctgt cagtggatct accattctgg agtctggagg atggtggccc 21300
tcttctcaca gctccactag gcagtgctcc agtggggact ctgtgtgtgg actcnnnnnn 21360
nnnnnnncaa ccacacattt tccttccgca ctgccccagc agaggttctc catgagggct 21420
ctacccccac agcaaacttc tgcctggaca tccagttgtt tctatacatc ctgtgaaatc 21480
taggcagagg ttcccaaacc tcagttcttg acttctgttc acccacaggc ccaaaaccat 21540
gtgtaagcca ccaagtcgtg gggcttgcac tctttgaagc atcagcctga gctgtatatt 21600
ggcacctttt agccatggct ggagcagaag ctgctggaag gcagggcacc atgtcccaaa 21660
gctgcataga gcagagggag cctggggccc acccacaaaa ccattgttcc cttctaggct 21720
tccaggcctg tgatggaagg ggctgctgtg aaggtctctg gcatgccctt ggggacattt 21780
tccccattgt cctggtgatt agcattcagt ccctcgttac ttatgtaaat ttctgcaact 21840
ggcttcaatt tctccctagt aaataaggtt ttcttttcta taaaaatcat cagactgcac 21900
attttccaaa tatttatact ctgcttcctc ttgaacactg tgtcacttag aaatttattc 21960
taccagatac cctaaatcat ctctcttaaa ttcaaagttc cacagatctc tagggcaggg 22020
gcaaaatgct gccagtctct ttgctaaagc ataacaagag tcacctttgc cctagttccc 22080
aataagttcc tcatctccac ctgagaccac ctcagcctgg acttcactgt ccatatcact 22140
atcagtattt tggtcaaagc tatccaacaa gtaggaagtt ccaaactttc ccatatcttc 22200
ctgtcttctt ctgagtcctc caaattgttc caacctctgc ctgttaccca gttccaaagt 22260
cgcttccaca ttttcaggta tccttatagt agccccccac tctaagtacg aatttactgt 22320
gttagtcctt tttcatgctg ctatgaagaa atacccaaaa ctgggtaatt tataaaggaa 22380
cgaggtttaa ttgactcaca gttctgcatg gctgggaagg cctcagaaaa cttatgatca 22440
tggtggaagg ggaagcaaac acatctttct ccacatggtg gcaggaagaa gtatgagtga 22500
ccagtgaaag gggaagcccc ttataaaact atcagacctg gtgagactta atcatcacaa 22560
gaacaggatg agggaaacca ccccagtgat tcaattatct ccacctggtc cctcccagga 22620
tacatgggga ttatgggaac tacaatccaa aataaggttt gggtgggaac atagccaaac 22680
catatcgaaa tgcaacctaa agctactgag tccaagcaaa agtcaagtag actctctaat 22740
cccagataac ctcagagagg aaatcttaaa cccaaagacc aacttccctt ttctgcctaa 22800
ttgcctttgt tcttctccaa gaacctcttt ggtcttggaa ggatgctcat tctccaccgt 22860
atttgcagt ggactattgg gaaggaaagt tccccatcca nggatgctat gtgcacacat 22920
tcatgtgctt ggacctcacc aaggatctca ctggaccccc ttcgcggggc ccatctgtga 22980
ctgtcatcat ttggctaggc aagcctcctt gagagtaatc acactctggc atcggctgaa 23040
tgtcaaggtt gttattgcag ttatagctgg tgggatcttt gtttctcaaa tgaagctttc 23100
ttattcataa agtgcagtca tctcttcctg ctaatcacac ccttgaaaaa agagctggat 23160
gtgttcctga gcagggagga cctctctgaa atcagcttag aggaatgagt gtatgtttgt 23220
tcttcagtgg tgtctagaat ctgacttctg gcaatctttt ccaccatcag ccctttctct 23280
ttgccaggat tcctttacct cctatgtcag ttgaccttcc tatgttgcac tgttaccctg 23340

tgtttttcac accctgtatg caaaagagct ggtcaccatt ttatgtaaaa taaatttcaa 23400
aatacagaat ttagctgagc aaaacagaac ccgaagtttc atttgagact gaattttaaa 23460
tctaatattt attcatgagt ttttgcagtg accttttcag caccttttttt agatcacttt 23520
cttataataa tttacacata ttaagctaat tattcagtat acttcacaga aggcttaaaa 23580
aatggaactg taacaagatc ttttgtgcaa attatgagaa gctgtcttct gtttaaattt 23640
cagaatgcat atacttacat gatgggcgct tgtgtgattg tatttaggtt tttctccatc 23700
tgtggaaaac atgtaagtac attgttaaga atcttatctt aaaggtcata tatgtgttaa 23760
tgaaagaatg gtgattaatt gattacagcc ttacctcggt tatttaggga tgtaatcaca 23820
aattactaga catcataagt caagattaac aaatattaaa cattgttaaa tgaatcagta 23880
gcttagttat cattcgatgt gtttcctgcc atttatgttg cttattcttt atattcaata 23940
attaagtaaa ttatttaaat ttattaattt aattaattaa agataaccag tcatgaaaac 24000
aaaaaccaat attctttctt aatgcatttt tatttttatt ttcaataaag ttaagtaact 24060
tcaaagaaaa ataatcttta atgaattttt atngtttcca tgattccaga tnnnnttttt 24120
tttttttttt tttgagatgg agtcttgctc tgtcaccagg ctggagtgca nnngtggcag 24180
gatctcggct cactgttttc tccacctccc aggttcaagt gattctcctg cctcagcctc 24240
ccgagtagct gagattacag gcatgtgcca ccacacccag ctaatttttg tatttttagt 24300
agagacaggg tttcaccacg ttagccagga tgagattcca gatttttttaa tattatttgt 24360
taatgtccat gggtatattc cacatatatt tttcattttt tacaatgctg tttccttttt 24420
tattacaaac gttatttcat atatacattc catatataca cataaccaat atacttaata 24480
tttgatagtt atttagtatt ataccattgc ttaggtttgt ttttgtgaaa gtctgtttgt 24540
ggaagaaacc tgagtttact tgctgtcagt caaaaatgtg tagcccttat catagagaat 24600
tgaggagcag aagagaaaat ggaaggtgaa aatgcttagt gttactctta ttaatttgtg 24660
tgaagaggct gaaatgtttg aagtaaaaat aaaattcgcc caacgcctgg tgggttttga 24720
agaatatttt gatgtaatac cttatagaaa gcaactttca aatgatacac ctaagagtga 24780
ttttgagcca atctgcttaa agattagttg ctacccaggt cttccatagc ttaatagatt 24840
gaatattctc tctctctctc tctctctnng tctctgtctc tctctctctc tctctctgtc 24900
tccccgcttc ccacccctgg tccccaggta acgctaaaga tgctcctctt gacagtggtc 24960
gtcagcatgt acaagagctt ctttatcata gtaggcatgt ttctcttgct gctgtgttac 25020
gcttttgctg gagttgtttt atttggtact gtgaaatatg gggagaatat taacaggttg 25080
gtatatactt atctttctat tcatttatca agcaaaccct ttggtaataa aattttaaat 25140
aaacaaatgt tttttgtcta ttaaagtcta ggagagggga aagggaagta atcagttgga 25200
aattaaaata aaatagccaa gttttaaatg atatttttcc agttgaaaaa ggttattcaa 25260
ctgaaaaggt ctactttcaa aaccttagaa aaattagaaa taaaagaaaa caaaaagaat 25320
ctataattcc accaacattc agtaatcatc actccnaatt ccagcacttt ttctatctac 25380
atattgccaa aaaatgatat attattttta gatggaacaa ttttaagata actctgggtt 25440
tctttctcag caaaatatgg gaatttagag ccccccaaat ggttgtaata tatgaaaagc 25500
agttttcaac gatttgccta agttttatag atgcatcatt ttttttcaag tcctgataca 25560
acaaagtata taccgatatg tcaagttgta ggaattagat acatggctgc cccacctttt 25620
atagcatcgt ccaagtgaaa gaaggaatca cttagttgcc aaagcctgcc tacttagtca 25680
ttcaagatga atggtaataa attagtggcc atgctagagt tgggtgttca gacggctata 25740
cttggaggca agtataaatg acaaattaat aaaaaggtat aacctttgga aaaggtnact 25800
tagcaaccaa atttgctaca acctccactt gttttggtgt tggcaaacca tgctatttca 25860
tgacaaaccc ttctataant cacattctgt taattttttt ttcttaaatt aggcatgcaa 25920
atttttcttc ggctggaaaa gctattaccg tactgttccg aattgtcaca ggtgaagact 25980
ggaacaagat tatgcatgac tgtatggtaa atatctctca tcatgaacaa ggcttagact 26040
tagaatcagc atgaaactct tncaacacaa tgttggcagg atcatccttg ttagcgttaa 26100
gactttatgt atgaaatata aatcctgaca cttcagtcca taaaattgca ttattataca 26160
ttccaatgtg tgaatattta gggcaaaata ctcaattaac ccaacttgag ctattgtagt 26220
tccattcaac aaantatgca ttttgtgagt gttgttcttt ccctcccatc tttaatattt 26280
tggtaatgaa agtcaaacag gaagcccttt aatttaatgt gttcagtgga tagttcactg 26340
cccctcagct atatgctagg ccctaggtct tcaacaactc caaggcacac tcaaggagct 26400
tacctcaagg gctctgagtt actcttcctt tatactataa agtatacttt atacttcctt 26460
cttactatat cctgtcttcc ccaagcatca tccttctggt ctcagtttaa acaggtcttt 26520
tgccttaagc taagacagcc agctacatgt tcttatgctg ctctatagtt ctcctttgta 26580
ataaggagct gtagtgaatt actggcttac attcatttat tttatctctt tctcaccgta 26640
ggacagacca atgctgtgtt aaaaaatgtc tgcagtgatg gaaatctgca ctgtccaatt 26700
cgggagccac tagccatatg tggttatgga gcatttaaaa tgtggctaac acaactgagg 26760
aactgaattt tagttttaat tttatattat tacattttaa ttaacataaa tttaaattta 26820
catngctgca tggagctagt ggtcaccata ttggactgca cagcactcaa ctgtcagtag 26880
gaagaagaga tagggccgcc tatgtctttc ttaccagtct ctccccaaac ttggcacaaa 26940
cctagcacac agtaggagtg ctgggacttc atgcatgaat taacaaggag aatttgccaa 27000
atcaaataga atacaaggca aaatttaatg aatcttaaat aaaaatacaa gcagaatgct 27060
gggattatag aagagagagc tagtttatct ggctgagaaa aaccagaaag ttctctcaga 27120

ggtgtcggcn ttttatagag cctgtaaaca atgagagagt ttttgatgac ttaagtcttg 27180
tgctattcca tagcatccca taacagtgtt ccaagaatgg ggcttactcc tcacacaatt 27240
tgaatgtgaa tttnctatgt gcaggcacat cttgttttac tgtgcttccc ttattgcctt 27300
ttgcagacat tgcattttta caagttgaag gtttatggca atcttgtgtg gagcaggtct 27360
atcagggcca tttttccaat agcatgtttg ctcactttct ggttctgtat cacattttgg 27420
taattctcac tatttcngac tttttcatta ttatctgttt tggtgatctg tgatcagtga 27480
tcttttgatg tttcttttgt agtttttttt gaggggtgcc atgaaccatg ctcaataaga 27540
tggagaattt aattgataaa tgtcatatgt cttctgattg ctgcaccgaa catctgtttc 27600
tccaactctt ctctcttttc ttgggcctcc ctatgtcctg agacacaaca atattgacat 27660
taggccgatt cacaacccta caatggccta taagtgttca agtgaataga agattcacac 27720
atcagtcact tcaaatcaaa agctagaaat gattaagctt atggaggaag gcatgtcaaa 27780
agctgagtta gcccaaaagc taggtccctt gcaccaaata gttactcaag ttgtaaattc 27840
aaaggaaaag ttcttgaagg gaattaaaat gctactccag tgaacacatg actgataaga 27900
aatagcctta tggctgatat ggagaaaaaa ttttaccggt ctcagtagaa gatcagacta 27960
gccacaacat gcccttaagc taaagccaaa tgcagaagaa ggcccttact ctcttcaaat 28020
ttatgaaggg tgagagaggt gaggacgctg tagaaaaaaa tgtttgacgc tagcaaaggt 28080
tggttcatta tgtttaagga aagaaagaag ccatctccat aatatgaaag tgtaagtgct 28140
gctgatataa aagctgtggc aagttatcta gaagatctag ctcaaataat taaggtggct 28200
acactaaaca atggattttc aatgtagaca aaacagcctt ctattggaag aagatgccct 28260
ctagaacttt catagctaga aaggagaagt caatgcctgg attcaaagct tcaaataaca 28320
gacttactct gttgttaggg actaatgcag ctggtgactt tcagtggaag ccaatgctca 28380
tttaccattc caaaaatcct aggaccctgc aatggtttga ctgtgtcccc acccaaatct 28440
catcttgaat tgtagttccc ttaatctcca tgtgtcgtgg gagggaccag gcagagataa 28500
ttgaatcatg ggggtggttt cttccatcct gttcttgtga tactgagtga gttctcacta 28560
tagccgtcat gagatctgat ggttttataa gaggtttttc cccccatttt cttggcactt 28620
cttgctgcca ccatgtgaag aagaacatat ttgcttcccc ttccgccgtg attgtaaatc 28680
tcctgaggcc tcccaatccc tgctgaactg tgagtcaatt aaatctcttt cctttataaa 28740
ttacncagtc tcaggtatgt ctttattagc agtgtgagaa nggactaata cagtctctta 28800
agaattatgc taaatctacc ctgcctgtgc tctataaatg gaacaacaaa gcctggatga 28860
cagaatagct gtttgcagca tggtttgctg gatatttaag cccactcttg agacctactg 28920
ctcagaaaaa aaagattgct ttcaaagtag tactgctcat taacaatgca tctacctggt 28980
cacccaagag ctgtatcagg agatgtataa ggagattaac attgttttca tgcctgctaa 29040
cacaacatcc attctgtcca tggatcaaga agtcatttca actttcaagt cttattattt 29100
cagaaataga tttcataagg ctaatagctg ccatagatag tgattgctct gatggatctg 29160
ggcaaagtat attgaaaacc ttctggaaag gattcactat tctacatgcc attaagaaca 29220
ttcatggcca ggcgcagtgg ctcacgcctg taatcctagc actttgggag gtcaaggngg 29280
gtggatcacg aggtcaggag atcgagacca tcctggctaa catggtgaaa ccttgtctgt 29340
actaaagata caaaaaatta gccgggcatg gtggtgggcg cctgtagact cagctactcg 29400
ggaggctgag gcaggagaat ggcgtaagcc caggaggcgg agcttgcagt gagcggagat 29460
tgcaccactg cactncagcc tgggcgacag agtgagactc cgtctcaaaa aaaaaaaaaa 29520
aaaaannnaa cattcatgat tcatggaagg aggtcaaaat atcatcaaca ttaacaggaa 29580
tttggaagaa gttgattcca gccattatgg atgactntga ggggttcaag acttcagtga 29640
aggaggtcgc tgcacatgtg gttggtagaa atagcaagag aactagaatt agaaatggaa 29700
cctgaagacg cgactgaatt gctgcagtct cctgatcaaa cttgaaggaa tgaagattgc 29760
tccttatgga tgatcagagt ggtttcttga aatggaatct actcttgcag aagatgctat 29820
gaacatagtc ataatgacaa caaaggattt ataatgttac ataaaattac tcgataaacc 29880
agcagcaggg tttgagaggg ttgactccaa attcgaaaga agttctattg tgggtaaaat 29940
gctagcaaac agcatcacat gctgcagaaa aatctttcat gaaaggaaga gtcaattgat 30000
atgataaact tccatgttga cttgttttaa gaaatcgcca cagccacccc aaccttcagc 30060
agccaccacc ctgctcagtc agcagctgtc aacattgagg caagaccctc caccaccagc 30120
agaagattac aactcactga aggctcacat gattgttagc ttttcttagc aataaaagta 30180
ctttttatta aggtctgtac ctttttggac ataatgccat tgtgcactta ataagccaca 30240
gtatggtata aacatgagtt gcatgtgcac tgggaaacca aaacatgcat aggactttat 30300
tgcaatattc actttatcac agcagtctgg aactgaaact gcaatgtctc cgaggtatac 30360
ctgtagagtc tatattccca aatcttaacc cattacttca gtcagagaaa aacttgcaaa 30420
tgtcttttta tttgattttc tgcagtttga tcacagtggc tacttatcac ttggggctgg 30480
tggtggtggc agaaagaata tctagtggag atcagttcat tttcattgtg cttttaaaaa 30540
tggcaataac cttgggattc tacaagataa cagcatagca taagtgaaat attcttcttt 30600
atttctgcta cagatgatgt acccaatgta tgtggctttt gaaagcttac tttgaagtta 30660
attatatgct atatgtttag gaatctattc attaactata gtcagctaaa agaaatttgt 30720
attctctact cttatcctct ccttcttcca ccccaatgcc ccaaaggttc agcctccgtt 30780
ttgtactcca gatgaattta catactgggc aacagactgt ggaaattatg ctggggcact 30840
tatgtatttc tgttcatttt atgtcatcat tgcctacatc atgctaaatc tgcttgtagg 30900

```
taagtgatgt tagttgaata ctttttactc tttaaactca ggtacttaga taattatgta 30960
ttattttcaa gcaattaccc tggatttctt ggggtgggtg tttcatggca ttaattataa 31020
attgttgtct ctcaatccag aagctgaaaa gacctttgaa aggcacgtaa atgacagttg 31080
actgcaattt taagagttgt agcatttatt atgatcatta atttcaaaag ggctcttctc 31140
atggattgag tngaacaaat atcagagagg actatctttc tagccttgaa atatcacatt 31200
tatgtttttg aaaggtacac cttatttacc cagatgtggg cccacccaga actcaatttg 31260
acctttaaaa atggccctgg aactctcaac taagagttaa agccagctgg catgactatc 31320
tctatacaca gaatgtgtgt ttgtgcttac gaccctcatg ttttggaaag gcaggagctg 31380
gagaaatagc ttagtccaag tgtaaacatg ccaatttgga aaccatacta aaagcacttt 31440
aaaaatttgg cttatgatat aggtgataga atccattatt aataatagat gttttactct 31500
ttctaaaata tgacagatta gcataatggt ctaacttctg aatttttctt tttttaaaag 31560
ccataattgt ggagaatttc tccttgtttt attccactga ggaggaccag cttttaagtt 31620
acaatgatct tcgccacttt caaatmatat ggaacatggt ggatgataaa agagaggtat 31680
gggagtgggt tgttgtttca tgcctcacct caaagagact tatcaacatg tctcaaacac 31740
ttaaatggtt ttcaggttgg caaatgacac agattgaatt tttaaatggn ntgaanttgc 31800
agaaagccat catttacagt tcagcagaat cctctaggaa gggagacagc atcgttcctc 31860
cttacatgat cttcaaccat gaacatcctc ttcttcagac tttccgaagg ccagcaggga 31920
aaggaagcca aagggactct gtaggaatca gccttgggct caggccagaa aaagaggtga 31980
cactgaaact accaaaggga cagtatatta ncaaattcat gatcacctca aactcaagta 32040
gcaatcccaa ttaaaagtac aaaggagtta tttaagtggc atctgaaggt gtgtattgga 32100
ttatttttga gttactactt gaatgtttta accataaagt ttacccaacg tttcttaaaa 32160
ttagccaaag tagctgcagt ctgctcttcc tcttcttgtc attgtcgtca tcaacctcca 32220
cctccttccc ttcctcctac tcctcctcct ctacccccctc tnnnnnnnnn nnntcttccc 32280
cctcctcttc cccctnctct tccccctcct cttccccctc ctcctcgtcc acctcctctt 32340
ccacctcatc ttccccccta cctcctcctc ctctacccct tcctctacct tctcctcccc 32400
cacctcctcc ttctncactt cctcctgttc ttcctccctc tcctcttctc tcttcctcat 32460
cctagcaaac actcaggcac agttctaggc actgaggata tagcagtgaa caagatggaa 32520
cagctcctgt actcacagag cttacagtct aataggtgga gacaaataat aaaaagtgaa 32580
ataataataa atagtgcagt atttccagaa gttaaaacta tagacagtag ttggggttgg 32640
ggctgcgccg gcaggaatga ccatgaagga ggcctgaggg gtgctgtggg acctcatgtc 32700
tgaacaacaa ggaaagttct gccagggtat ttggggcgag atctggaaat agttcaagaa 32760
gagttgatga agggcacaaa gaccaagaag ccagcctggg catcataaat gaggggctcc 32820
ggcagtgcac tcaccccaat ttgctccctc ttcttggtac cctgcccgc ccaggcttca 32880
gtcttgaggg ctttcctttc aaggtagtga gagccctgcg tgcagggcat ctgagctcac 32940
tctcagctcc tcaggatcca cctgtctctc tgtaactgtc acccacggga acctgctttc 33000
atgcaataca cacatgaaga cccactgccc tgcacaccga gatgtcagta actggcaggc 33060
agtcatggtc gccagtgttc cgcccagcac tttcattcaa agaaagatac agccaggggg 33120
catggtggtt tgtgttgcat ggaaggtagc aacacatctt agattctcag tgtctggatc 33180
agttctgacc ccagaagagg gcagagctga aggcctaatt ctcttgtata tagtttttct 33240
aatcctgaag aagacatgaa aaggagttag ttagctcttt tttttttttt tttttgaga 33300
tggagtccca ctctgtcccc aggctggaat gcagtggtgc aacctctgct tcctgggttc 33360
aagtgattcc ccagcctcag cctcccaaat agctgggact acaggcatgt accactatgt 33420
ccgggtaatt tttttatttt tagtagagac tgggcttcat catgctggcc aggctggtct 33480
cgaactcctg acctcaggtg atccacccgc ctcggcctcc caaagtgctg agattacagg 33540
catgatccaa cacgagttat cttttaatgg tagaaaaaca tgtaataaac attgagacgt 33600
tcctagcatc ccgattgagt gaagaggagg attacacgtg tgtgtcttgt ccattncctt 33660
gtgtgagtac attatggccc actcagtgca tgtgttggag gtggcgtctg tgggacagnc 33720
tatgcaagca tcagcctggt gagagcaaga gtagggtcct ggggggctga aggggagctt 33780
cccatgcctc actccaaatg ctgcaatgtg gtgacatccc agaaagagaa gcaccagcag 33840
aaatttatca ctcagttttc tatcctctgc tcttcttagt gcacttaatt ctaccaggct 33900
ccaaggaggt gaccagcttt cttcacatct ggtgctaatt atgctagctt ttgagaaata 33960
gcaccttttt ttttttcttt tgagaaaccc aagtgaatca tcgaggcctg atgatttgct 34020
acttcttgtg ctctgccccg caggggtga tccccacgtt ccgcgtcaag ttcctgctgc 34080
ggctactgcg tgggaggctg gaggtggacc tggacaagga caagctcctg tttaagcaca 34140
tgtgctacga aatggagagg ctccacaatg gcggcgacgt caccttccat gatgtcctga 34200
ggtacaaggc aggctttcgc agggctgaat gggggccaga aaccacagct gtcagccttc 34260
aaggcctctg cagtacaatt aacaagtgtg tttccaaccc aaaatgccac atctgctgtg 34320
gtcgtgaagt ggggtcacat gtcccaggcg ctaagtgccc tttcctgccg tcctccagtg 34380
aacatttcca agtaggtgcc tcgcagggga gggctgggcg gctgtgccct gatgtcccgt 34440
agggacccct gcccactgag gtgcaccccg aggggagagg tggaagggat ctgggagagc 34500
acagtcccac cccttccctg tgatgcggga actgcccaga agagctgagt gacttgcttg 34560
cggttacagg gctctggcaa agctgggact aacatgcaga gcacctcact tgcatgggtc 34620
tgatgagaat ttgcctggcc tgctctgtga tggttgcccc gttggctaaa acaccagatt 34680
```

ttccagacat ttcatgagcg cccagacagg acagagggtc aaatggagtc tctgcctttt 34740
cttcttcctg ggagacttct gtatttgttt ccttgggcta ctgtaactta gcaccttaag 34800
ctgagggatt taaacaacag aaaggtatgg tctcgaagtc ctggatgctg gaagcccgag 34860
atggaggtat tgcggggctg attccttctg aggcctgtga gggagaatct agcctagcct 34920
ctcccctggc tgctggaggt gcgctgccaa tgttggtcat tccttggctt gtagaagcat 34980
cagcttggtc tcccttcatc ttcacatggc attctgtttg tgtgtgtgtg tgtgtgtgtg 35040
tgtgtgtgtg tatctatgtt caaattcctc cttttataag aacatctgtc gtattacatt 35100
aggagtccat tctactccac tgtgatctca ttttaactta attactaatt acatctgcaa 35160
tgttctattt ccaaataacc ttacgttctg aggtactaga ggttaggact ccaacatatc 35220
tttttttggg aggaaacatt taacccataa cagcttctta agcaggagtc catggataaa 35280
acagaggccc atggacttgc aagggggaaa caattacatc tttattttca ctaatcactc 35340
tttgaaatgt gtcattctct tccatgatga acgtaggcaa caaaccacag tcgcattagc 35400
aatatccatg tctttgttac caggagaaat catagatatt ttcagatgac ataactgact 35460
gccttgactg acatcttaaa atatcattta tgctcaccag tgcttctgag ttatggtagt 35520
catttgagct ctgctagatg gtattattta atggaataat aaaggaagca cttacttatg 35580
ttactttatc actgccgttt taaagtaaat ttgagaattg tattttaata taattggttt 35640
cctttgtaat acggtgtatt tcattccgtg catttaaagc attatttcaa gaaatgtccc 35700
ataagctcca ctgccacagg ggtccatgcc acagaaaaaa ataagagcga ctgactagca 35760
gtagctattt taatcacata gcagtaccta caaggagtgt gtgggatatt gtgttgtgtc 35820
tcaaatttca ggagaactca ggtgtacaga aatatgcttg tggccagaag ctgaaaaaaa 35880
aaaaggatca gatcagcaca ggaagaatct gggaaaagtt ctctcaagtt atcaatttca 35940
cctcccacta tccagttctt ccaccaacaa gctaaaaagc agaaaaacat gggacggaga 36000
aatgcatgtg aatcacaaag gaggtcatct ctgtcagaat gtttctgagt ctttcctcct 36060
agtggcaaat ttttttggtt gacatgaggc agataggcat actgttgtca ttaataatta 36120
atattcagcc tctttggaac acaccgatgt atctaataaa aatagaccca ggcaaaatct 36180
gtgatggggc tgtctctccc cttcctattt attcccctag cccagtatat gataaaaaca 36240
aacagtgaag ataaaagcaa tgaagctttg atgggattct tgattgagtca gggaagagag 36300
gaagagaaaa ttcatttttt ttccctctgt ggtgggaaga gatttagcaa agagaagccc 36360
aatgtaccag tgctctcaag agcctccagg ctgtgatttg ggctctagga tggaactgag 36420
ctcacagaag tgggtagctc acaggtgcac cagggtgcat gggtctgagc cataacctgg 36480
aatcctgtgg ctacacctgg gtgcatcaca ctgccaagag gggctgtctt ctcctctggt 36540
ggtggactgc tcagtccagg catcctggga gctgccttat tgccccatga actgtaatgc 36600
tgtgttggct ttacctacaa ctgcatgggc ctgctgtgtg tcaagggtta aatgggagtg 36660
gatattcaac cctgggtttg tttggtagcc tctgaaatta ataaggaaga ccaggcatgg 36720
tggctcacac ctgtaatccc agctctttgg gaggctgagg caggtggatc atttgaggtt 36780
aggagtttaa gaccagtttg accaacatgg tgaaaactcg tctctactaa aaatacaaaa 36840
ctagctgggc atggtggcac acacctgtaa tcccagctac ttgggaggct gaggcagaag 36900
aaatgcttga acccaggagg cggaggttgc agtgagctga cagcgcgcca ttacactcca 36960
gcctggtcaa caagagtgaa gctccatctc aaaaaaagaa aaaattaatt aataaggaag 37020
atacagaagg atgaatgaga gaaacagaga gggaagtggg atgcagggag tagggatgtg 37080
tgacgcacag ccgagcatat taaccatcac ctcggggggc taagtgtgaa gaaagtatgg 37140
gaggtgcggt cttccttctg cagagaaagt cgcgagcctt ttcctagcct ggatcttttg 37200
ctttacaggc acttagatgg cacttactac tataagagcc tcacagatat taactcactt 37260
agtcattaaa taaaggagtt accaggacta tctatcatct ttgttttaca gacgagggaa 37320
gggagacaca gagaggtgac aggaccagcc caagacatgc gggtggcgag gggtggcgtc 37380
aaaacactag acttattaga ctgagtgatg tcctctctgg attttgacgt atctaagggt 37440
gtaaatctca ggtgaatcag agctgcgatt gcaggtgtgt ttctcccacc agcatgcttt 37500
cataccggtc cgtggacatc cggaagagct tgcagctgga ggaactcctg gcgagggagc 37560
agctggagta caccatagag gaggaggtgg ccaagcagac catccgcatg tggctcaaga 37620
agtgcctgaa gcgcatcaga gctgtgagtg aactgcacac atgcaggttg ggtccccgcc 37680
ccgccccgcc ctgccccgca ggaggatgaa agaaaagagc ttcgtgaaca tgggattagc 37740
tccttctgtg atcagccttc tatcccttta aactatgtcc actacctcca cctacagtgg 37800
tcagatcatg atgcagactt tcactctagt ccacagtcag ccctgccctc tttagcagag 37860
tccattagga gtttccatgg aatcccattt ttataatatg acatgaagac ttaaaaagca 37920
gtttattggc caggtgtggt ggctcatgcc tgtaatccca gcactatgag aggtcaaggg 37980
gggagctcaa ctcacttgag gccaggagtt ggagaccagc ctgccaacat ggcaaaaccc 38040
catctctact aaaaatacaa aaattaacca ggcgtggcac acatgcctgt agtcccagct 38100
actcaggagg ctgaggcatg agaatcactt gaacctggga ggtagaagtt gcagtgagct 38160
gagatcatgc cactgcactc cagcctgggt gacacagtga gaatctgtct caaaaaaaaa 38220
aaaaaagttt attgatatta aaatatgatg tccatttatt aaactcaact gttcaaaatt 38280
ctagaatttt ccacatattg aatttgcaaa actactttaa attgctgcta aaaatttcaa 38340
caaactgtga taatgtcatt tattctgaaa agaccacctc tcaagattaa tgatgtttcc 38400
tgcttcctga cttataagcc tataagattc tctaactcat aaattaatga atgtatactg 38460

cattgacata tactgtattt acagtgaatg tttctatgag ataattactt ttatgaatgc 38520
ttttggcatg tttcacagac agggaaaaaa cccaaagcaa tcactaaaca tttatatata 38580
acagagattg tttcaatcct cttcctcaga gcaacaacgt caattgcaga aacatgtatt 38640
tctattggag gagaaaaggt tcccagagaa aagatacaac tcaacctcct atagcaaggc 38700
cagctgccca gtgatgtccc atcactgctg gccactgtag ctatgttagc tccagggatc 38760
aatctgtcct cctctggctc tgagagggca caaggagtcc tggctttagt atatgccgag 38820
acagactggt ctcctggatt gacctgaaaa cttcagaacc aagacaagca caaaggtttt 38880
ctcatccacc caaggctctt tgcctcttac aacttgcagg aaagtgggaa aggggttggg 38940
attgtattgg cttaatgagc ctagaataaa ataagaggag aaaaaataga atagccacat 39000
tagtaatttt aaataagaca taagctgggc acagtgtctc atgcctgtaa tcccagcaca 39060
ttgggaaact gaggcgggag gatcgcttaa gaccagaagt ttgaaactag cttgggcaac 39120
agagtgagac ctcctctctn gnaaaaaaaa aaaaaaaaaa aaaattaatt agccagccac 39180
agtcatgcac acttttagtc tcagcttctc aggaggctga ggcaggagca tagcttgagc 39240
ctaggagttt aaggttacag tgagctataa taatcgcacc actgaactct agtctacggg 39300
acatagtgag atcctatcta aaaaaaaaaa aaaaaaaaaa aancagaaaa caccaacaac 39360
aaaaagaaaa acaaataaat aaaatacaag catcaatgac cgtcactatt ctgattgtta 39420
cacaaatgaa aatggactgg gaagtcaatg catacctctc taagcccacc tttgcatcag 39480
cctaccttaa ttgaacattg aaattagttc aggatcctta cttggaaatt cctaagaggt 39540
cacccgtcat gcaggtctta ggagagggga gaagaaaata agatttgtgt ggcggtgggg 39600
caggcaggat gctgggctta ttttactgaa caaacaatcg catttccata gaaacagcag 39660
cagtcgtgca gtatcatcca cagcctgaga gagagtcagc agcaagagct gagccggttt 39720
ctgaacccgc ccagcatcga gaccacccag cccagtgagg acacgaatgc caacagtcag 39780
gacaacagca tgcaacctga ggtatgggca cgaggcgtcc tctgtcccag gacactctgt 39840
gggcttcttt cttttaataa aaataaataa aatgggtgct ggtcagatca ttcaaacaag 39900
gtgacaatat ttctaatagg aatattttca ttaaaaaaaa aaanggcaac ggaaatccat 39960
tgatccagat tatgtctatg tctttgccat cttggatgca gttgaggtta tttaaaaaga 40020
gtctgactca tatgaatgcc aaaaaaagtg aatgccctga acttcataat tctgtagttt 40080
tatttttgtc taaagagtta acatcagctc taagggctgc atttgaatct gggtatatta 40140
catggagtat ggtttacagg aagctctgcc ctaaatattt tcctcccttc tctaaagtca 40200
tagaaatttg cagtaaaatc agtgaaaatg tttgtaatta gctttcaacc tccttaaata 40260
atcagactca ttgaagatat atcctttcct ggttgggtat cagaagaaca tatggaattc 40320
aggacaattg gcttgaggag tttctattta ttaagtcaca gcatatttc attttcaaac 40380
tctatgtatg tgtctatata aaaaagtaca aggagttatc agcgtcctgg agagtcctgt 40440
gctgtgagaa gcagctggct gggaggacct gccccttat gaggcagcaa ttacgatttg 40500
aggttaacaa cgtggagctg atcccgtgct ctgtgcagag cccccagcag caccaggctc 40560
catggtgtgc agcaacgcag ccaggggcca gagtaccttt ggaagcctaa ggctccacgg 40620
cccagcaggg tgctccagga ggcccactgg tgccatagct cagttttcag tagatctcat 40680
gttttgggct ccccagcctt tagaaatcat ctgccaccga ctgcagggac ttctgcggag 40740
ttagtcaacc aaggggcaag gggaggcatc tgcagtggcc ttgccactgg tcaccctgaa 40800
gatggttggc aatgtctcgc atgggagagg gaggcaggct gaagtcagga cagacagtga 40860
gacagggcta atgaggttgg agaaagtgat gatttcagag ccctctgttt ttagatgaaa 40920
aagcctaaga aagaaagagg tgacnnnaaa aaaaaaaaaa ggatactaaa gagatgtgaa 40980
gaccaaggct tttgttagga aagggagagg tgacaggtga acctgagaac aggtgaaatg 41040
tgtgggaagt cttggcatct ctctttcttt cactgtagct tagattgtta tcagctccat 41100
cctccaccag ggccatcat acagctttgc ctgcagagaa ctctttccct gaagctctaa 41160
gtgtggatct ccaggctggt gacagtagac tgccccgcc cagccggtcc ccactgcccc 41220
accttccttc ctgcagttcc ccttccttac cccctggttt gccagcgaag atatgaaggg 41280
aaacaatgac attctcaggc atggagggga acgaagcagt gggggttacc caggacaaga 41340
tgtcccagga agagtggtgt ctccttcagc agggcgtgca gtggtggctc tttttcttct 41400
gtgacccttt atacccattt tcacttttcc ccattgtgga cactctgagt ccatgttgtt 41460
cttcccactt gtcctcctgc catgtgtttt cctttcttat agtaaaagga ggagagcgca 41520
gggttaaaaa gacggtaact gtgtgcttct cctcccatgc agacaagcag ccagcagcag 41580
ctcctgagcc ccacgctgtc ggatcgagga ggaagtcggc aagatgcagc cgacgcaggg 41640
aaaccccaga ggaaatttgg gcagtggcgt ctgccctcag gtaggtccat ccaggcattt 41700
ctccagcgat cgaggcattt taaaggtatt tactctgttt gtgtgttttg ggtcttgcct 41760
ttcaaaatgc aagtctgcat ctacagttgt ttacagacag caacataatg aaaaatgtag 41820
tcttgtcaaa aacattgtcc cccaaaataa cttctctaaa tatgacttac attagccccc 41880
attttccggg tacatttcag gctatcatgg ttgagaagcc agcacctatg aaaaagacaa 41940
gattcagnaa gagggagaaa tttccaaggg ctttctgtgt gcctcaaggc atgctcaaac 42000
atgggcaaaa gtattcaact gaagagggag tgggcagatg caatcattca gaaaatgcca 42060
cgaagttctc aaaaaaggga caggcccccg atttctctca gcacactttg cagctgagcc 42120
ggtcactggc tttaggcaag ctgctttacc tccatacaat aacaannnnc aacaacaaac 42180
acttacgtag tactcactgt atgccagtca ctcttctaag tgcattactt gtgttctctc 42240

attcaatcct ttaataaaca gtcccattat tttccatatt ttactgattc agtaagagaa 42300
ttttcttcta tgagcttctt ctctataggg ttttggaaag ccaggataat cccattaaga 42360
gcactcagga atsgagggag agcaatgctc cccacagccc agtgtccttg tacatgtttt 42420
tttctataga taactaagtt catgctaaag caggagcttt ataatgctct ttaactgtgc 42480
cccaacttca gcccaattga aaggagaaga tgtgtagcat atgtgttcca caaagcagat 42540
gacagcacag cttacatttt gaggctgacg atgttcagtg ggtcttcact gggactacca 42600
ccaaggaaag tatccccttt catatccagg aacttatttt tcagagatca gagaagatct 42660
aggttcctcc tgattcaaac acagcagaga atgacagcat caagacaacg tagatggtgg 42720
tgccaggtca taaattacaa gctgagtcgg ttcaatttta tcctgtaggc aattaggagc 42780
tagcaaagat ttctgagcag tatgtgactt ttggaatctg tgctttagga agttgacttg 42840
gcagcaaagg agggaattgt ctatgacaga gcctggaggc aggttacaag ctggaggaag 42900
ttaccgtggt gtgagcacaa ggcaacaaag gcatggacag agctgggggc cagatactgc 42960
agggacagat aggagaggtg acctggggag aatgcacagt ccttggtatt catttaaatg 43020
gacaaaagaa atgatgctgt caaaagtact tccattgggc caggcgcagt ggctcacccc 43080
tgtaatccca gcactttggg aggccgaggc aggtggatca tttgaggcct ggagttcaag 43140
accagcctgg tcaacatggt gaaaccctgt ctctactaaa aatacaaaaa ttagctgacc 43200
ggtagtggtg tgcacctgta atcccagctg cttgggaggc tgaggcagga gaatcacttg 43260
agcctgggaa gcagaggttg tggtgaacca agattgtgcc gctgcactcc tgtctgggtg 43320
agagagtgag aacctgtctc ccaaaaaaaa aaaaaaaaaa aaaaaaaaaa nngtacttcc 43380
actggtccat gcctaggtgg tccccaccaa aaacggtgat cgtgaaatag acatgctctt 43440
ggggtcaggc tgagtttgag gaggcagagg agaagaaaaa tagtattttg aggggcagc 43500
aacaatgtca caggtgtcat gctatgcact ttcctaagtc atctcatgta acctgtttta 43560
atcctgacaa tagtctaatt aggacatttt cattgtcttc atcttacaat ggaggaaact 43620
tgggttcaga gacgtgaagt tgcccatagc tacatatcca caggttgcag aggtgggtcc 43680
caacaccatg actgtgaggc cccagaacca cacctttcct cccatacccct cactgcatcc 43740
cccctcgcct ttactgtggg tcacactctc cttccttttt gtttttttta ttgcatatgc 43800
taatgaatgc acaggtttct gtgacactta atagttcccg aattggaatt tttttagtga 43860
ttttctagtt tttttaatta tttcaaaatg aaaggggtga gcagcacgtt tttttcccaa 43920
atgacatcat tagttgtcca tgagcacaat taaaaaaaaa atctatcgtg aacataaaga 43980
atcaagtcct gaaaaggatg tttacttagc atctgtggct aggacacgtt agccaaaagt 44040
atcactgatg ggaatatcat gggtttttag gagtgattac aatagggtga agcaatacca 44100
agtggctcat aggactcatt aattccacag ccccaaaacc aataagccat tcagtgtcct 44160
cagtcaactt acggtttgga ggaaggacaa ccatgaaatc tgtcgtgtgc aaaatgaacc 44220
ccatgactga cgcggcttcc tgcggttctg aagttaagaa gtggtggacc cggcagctga 44280
ctgtggagag cgacgaaagt ggggatgacc ttctggatat ttaggtggat gtcaatgtag 44340
atgaatttct agtggtggaa accgttttct aataatgtcc ttgattgtcc agtgagcaat 44400
ctgtaattga tctataactg aattccagct tgtcacaaga tgtttataaa ttgattttca 44460
tcctgccaca gaaaggcata agctgcatgt atgatgggtt actatcaatc attgctcaaa 44520
aaaatttttg tataatgaca gtactgataa tattagaaat gataccgcaa gcaaatgtat 44580
atcacttaaa aatgtcatat attctgtctg cgtaaactaa ggtatatatt catatgtgct 44640
ctaatgcagt attatcaccg ccccgcaaaa gagtgctaag cccaaagtgg ctgatattta 44700
gggtacaggg gttatagctt tagttcacat ctttcccatt tccactagaa atatttctct 44760
tgagagaatt tattatttat gattgatctg aaaaggtcag cactgaactt atgctaaaat 44820
gatagtagtt ttacaaacta cagattctga attttaaaaa gtatcttctt tttctcgtgt 44880
tatattttta aatatacaca agacatttgg tgaccagaac aagttgattt ctgtcctcag 44940
ttatgttaat gaaactgttg cctccttcta agaaaattgt gtgtgcaagc accaggcaaa 45000
gaaatggact caggatgctt agcggtttaa aacaaacctg tagataaatc acttgagtga 45060
catagttgcg caaagatgtt aagtttctta agaaaccttt taataactga gtttagcaaa 45120
aagaataaaa ctatatagct caatttattt aaaaaaatct ttgcatgtgt gatgttatca 45180
ttggcttcat ttcttaccca aggtatgtct gttttgccat aaatcagcag agtcatttca 45240
ttctgggtga tcctaacaca ccattgctat gttagatttg aaatgacatc tctgttaaaa 45300
gaatcttcta tggaaataat ggtgccctgc aaaatcttcc tctgaactca caggttaggg 45360
atcacacaac ttacttaatc gtttttgtt tttgtttttt ttccttatat gtcaatggcc 45420
catgtcctcc gggaaaatta gaaaagcaaa atgattacaa agtgctgtta gatttcttgt 45480
gctgggccag ccaagtagaa gtggacttga cttggacctt taactatttt attacagatt 45540
ggacatttgc tgttcagatg ttttttaaca gagggattat ctcagaatcc tgtgacctcc 45600
aggttgtttt ataatctatt tttctctatt taacattcct cagatagata ggcaaatagg 45660
acattccttc tgtgtcacag aagtatcgtg gtagtggcag tctacagttt atatgattca 45720
ttgtaactat gagataaaga acaaccagtc atgtggccaa aaggattaga tttgatttga 45780
tgttcacttg gagtttactt tttgtacata caagataaaa taaatattgg atttgtaaaa 45840
taatttactc tgagttgacc atttttaaac ccttttcttg gtggggcagg cactcggtag 45900
cagcacattg caaacctgga ttgaatattc agtccataat tagttggcaa aagctgtgtc 45960
cctgatactc atttcaactc aattctctga gccagaatgt tcctttcaca cttgtgctga 46020

```
taaggctgag tgaatacccct gtgccctcta agcctacaat gaacagaaaa ggcacttaca  46080
ggtccagggc atgggtctgt actagtgctg cataaaaaca agatgttttt ggctcattac  46140
acttgagtaa tacagcttca ttctcacgaa actgctgagc cctttctaaa acgtctggaa  46200
ttgatgttca gcagaacatc attgttgttc attctgcagt gaagccaggg gcagaatacc  46260
aatgtattta aagccatagc accctatatt ttcaatgaag cgaaacagtc acacacttcc  46320
tgtaggactc ctctctacga ttttcctgaa acaggtagag catatgcaag ccagagaaat  46380
taagcgccac ccccatttag attttacccc tagatatgta aacatatgaa aaatcccatt  46440
acacagatgg gaaaacttgg gtcacagtgt tccctggtcc gtagggaagc cagacactga  46500
tactaatcct gtcatcacgc cacccactgc ctttaattag agggtgctgt cagttccaaa  46560
ggcaccagaa agcatatggg gtggaatact tacttgactc agagcccctg agagagcagg  46620
ccacacatgt gtgacctgta tggcacttca aggagactca aagggaacaa ggatggacag  46680
aatggaaggg ccaatgctat tcactctann nnnnnnnnnn tttttttttt tttttgaga  46740
cggagtcttg ctctgtcacc caggctggag tgcagtggca cgatctcggc tcactgcaag  46800
ctccacctcc cgggttcaca ccattctcct gcctcagcct cccaattagc tgggactaca  46860
ggtgcccgcc accatgccca gctaattttt ttgtattttt agtagagacg gggtttcacc  46920
atgttagcca ggatggtctc gatctcctga cctcgtgatc cacccgcctc ggcctcccaa  46980
agtgctggga ttacaggcgt gagccaccgt gcccggccca ctctattctt aatggtactt  47040
ccagaccaac atacatcagc agtgtctggc tggctgtgcc ttggccaagc tgaatgagat  47100
aagggtacat agaagaaaaa ccagtgttat aaagccttac aggaaagcag ctttaagcca  47160
gtggaatgat gtgtttgcct gtttccttgt aggaagtggc attctataca tgggtggttt  47220
tcagtatatc agtaagcagt ggaactgttt ctaaggcctt caacagatcc caaaatttta  47280
accagccaaa agccaggcag ccctcgttga agttgaggtg aagatgaaaa cggcactcat  47340
gtggtctctt ctcacccact attatgggtt gaagtgtgac ccccagaagg ataggttgaa  47400
gtcctaaccc ttagtacctg tgaatatgac cttattagga aatagggtct tcgcagatat  47460
aattacttat gaggaggtca tactagagga gggtaatccc ttactccaac atacctggta  47520
tcctctgaag acacaggcat gtggggagga tggccatgag acggtgaagg cagaggcatc  47580
ggagctatgc tgcccagagc caagggacac ctgggctacc agaagctgca agaggcaagg  47640
aaggcgcctc ccagaggctt ctaagggaga atggccccct gtcaacacct tgattctgga  47700
cttctagcca gaagaactgt gagaacatac atttctgttg ttttaagctg cccagtttgt  47760
ctgctttttt atcattttat catttagtt ttttagttta gtctctgtct tctactaact  47820
ccctttccaa ttctacttta g                                               47841
```

<210> 4
<211> 6799
<212> DNA
<213> Homo sapiens

<220>
<221> 5'UTR
<222> 1..65

<220>
<221> CDS
<222> 66..5282

<220>
<221> 3'UTR
<222> 5283..6799

<220>
<221> polyA_signal
<222> 6081..6086

<220>
<221> polyA_signal
<222> 6777..6782

<220>
<221> allele

<222> 1658
<223> 99-79316-158 : polymorphic base C or T

<220>
<221> allele
<222> 4481
<223> 99-79310-29 : polymorphic base A or C

<400> 4

```
gacagctctg agcgctgggg ttacagactg tggttttgtg cttgctcacc aaagctaacc    60
tcagc atg ctc aaa agg aag cag agt tcc agg gtg gaa gcc cag cca gtc   110
      Met Leu Lys Arg Lys Gln Ser Ser Arg Val Glu Ala Gln Pro Val
      1               5                   10                  15
act gac ttt ggt cct gat gag tct ctg tcg gat aat gct gac atc ctc     158
Thr Asp Phe Gly Pro Asp Glu Ser Leu Ser Asp Asn Ala Asp Ile Leu
                20                  25                  30
tgg att aac aaa cca tgg gtt cac tct ttg ctg cgc atc tgt gcc atc     206
Trp Ile Asn Lys Pro Trp Val His Ser Leu Leu Arg Ile Cys Ala Ile
            35                  40                  45
atc agc gtc att tct gtt tgt atg aat acg cca atg acc ttc gag cac     254
Ile Ser Val Ile Ser Val Cys Met Asn Thr Pro Met Thr Phe Glu His
        50                  55                  60
tat cct cca ctt cag tat gtg acc ttc act ttg gat aca tta ttg atg     302
Tyr Pro Pro Leu Gln Tyr Val Thr Phe Thr Leu Asp Thr Leu Leu Met
    65                  70                  75
ttt ctc tac acg gca gag atg ata gca aaa atg cac atc cgg ggc att     350
Phe Leu Tyr Thr Ala Glu Met Ile Ala Lys Met His Ile Arg Gly Ile
80                  85                  90                  95
gtc aag ggg gat agt tcc tat gtg aaa gat cgc tgg tgt gtt ttt gat     398
Val Lys Gly Asp Ser Ser Tyr Val Lys Asp Arg Trp Cys Val Phe Asp
                100                 105                 110
gga ttt atg gtc ttt tgc ctt tgg gtt tct ttg gtg cta cag gtg ttt     446
Gly Phe Met Val Phe Cys Leu Trp Val Ser Leu Val Leu Gln Val Phe
            115                 120                 125
gaa att gct gat ata gtt gat cag atg tca cct tgg ggc atg ttg cgg     494
Glu Ile Ala Asp Ile Val Asp Gln Met Ser Pro Trp Gly Met Leu Arg
        130                 135                 140
att cca cgg cca ctg att atg atc cga gca ttc cgg att tat ttc cga     542
Ile Pro Arg Pro Leu Ile Met Ile Arg Ala Phe Arg Ile Tyr Phe Arg
    145                 150                 155
ttt gaa ctg cca agg acc aga att aca aat att tta aag cga tcg gga     590
Phe Glu Leu Pro Arg Thr Arg Ile Thr Asn Ile Leu Lys Arg Ser Gly
160                 165                 170                 175
gaa caa ata tgg agt gtt tcc att ttt cta ctt ttc ttt cta ctt ctt     638
Glu Gln Ile Trp Ser Val Ser Ile Phe Leu Leu Phe Phe Leu Leu Leu
                180                 185                 190
tat gga att tta gga gtt cag atg ttt gga aca ttt act tat cac tgt     686
Tyr Gly Ile Leu Gly Val Gln Met Phe Gly Thr Phe Thr Tyr His Cys
            195                 200                 205
gtt gta aat gac aca aag cca ggg aat gta acc tgg aat agt tta gct     734
Val Val Asn Asp Thr Lys Pro Gly Asn Val Thr Trp Asn Ser Leu Ala
        210                 215                 220
att cca gac aca cac tgc tca cca gag cta gaa gaa ggc tac cag tgc     782
Ile Pro Asp Thr His Cys Ser Pro Glu Leu Glu Glu Gly Tyr Gln Cys
    225                 230                 235
cca cct gga ttt aaa tgc atg gac ctt gaa gat ctg gga ctt agc agg     830
Pro Pro Gly Phe Lys Cys Met Asp Leu Glu Asp Leu Gly Leu Ser Arg
240                 245                 250                 255
caa gag ctg ggc tac agt ggc ttt aat gag ata gga act agt ata ttc     878
Gln Glu Leu Gly Tyr Ser Gly Phe Asn Glu Ile Gly Thr Ser Ile Phe
                260                 265                 270
acc gtc tat gag gcc gcc tca cag gaa ggc tgg gtg ttc ctc atg tac     926
Thr Val Tyr Glu Ala Ala Ser Gln Glu Gly Trp Val Phe Leu Met Tyr
            275                 280                 285
aga gca att gac agc ttt ccc cgt tgg cgt tcc tac ttc tat ttc atc     974
Arg Ala Ile Asp Ser Phe Pro Arg Trp Arg Ser Tyr Phe Tyr Phe Ile
        290                 295                 300
```

```
act ctc att ttc ttc ctc gcc tgg ctt gtg aag aac gtg ttt att gct      1022
Thr Leu Ile Phe Phe Leu Ala Trp Leu Val Lys Asn Val Phe Ile Ala
    305                 310                 315
gtt atc att gaa aca ttt gca gaa atc aga gta cag ttt caa caa atg      1070
Val Ile Ile Glu Thr Phe Ala Glu Ile Arg Val Gln Phe Gln Gln Met
320                 325                 330                 335
tgg gga tcg aga agc agc act acc tca aca gcc acc acc cag atg ttt      1118
Trp Gly Ser Arg Ser Ser Thr Thr Ser Thr Ala Thr Thr Gln Met Phe
                340                 345                 350
cat gaa gat gct gct gga ggt tgg cag ctg gta gct gtg gat gtc aac      1166
His Glu Asp Ala Ala Gly Gly Trp Gln Leu Val Ala Val Asp Val Asn
            355                 360                 365
aag ccc cag gga cgc gcc cca gcc tgc ctc cag aaa atg atg cgg tca      1214
Lys Pro Gln Gly Arg Ala Pro Ala Cys Leu Gln Lys Met Met Arg Ser
        370                 375                 380
tcc gtt ttc cac atg ttc atc ctg agc atg gtg acc gtg gac gtg atc      1262
Ser Val Phe His Met Phe Ile Leu Ser Met Val Thr Val Asp Val Ile
    385                 390                 395
gtg gcg gct agc aac tac tac aaa gga gaa aac ttc agg agg cag tac      1310
Val Ala Ala Ser Asn Tyr Tyr Lys Gly Glu Asn Phe Arg Arg Gln Tyr
400                 405                 410                 415
gac gag ttc tac ctg gcg gag gtg gct ttt aca gta ctt ttt gat ttg      1358
Asp Glu Phe Tyr Leu Ala Glu Val Ala Phe Thr Val Leu Phe Asp Leu
                420                 425                 430
gaa gca ctt ctg aag ata tgg tgt ttg gga ttt act gga tat att agc      1406
Glu Ala Leu Leu Lys Ile Trp Cys Leu Gly Phe Thr Gly Tyr Ile Ser
            435                 440                 445
tca tct ctc cac aaa ttc gaa cta cta ctc gta att gga act act ctt      1454
Ser Ser Leu His Lys Phe Glu Leu Leu Leu Val Ile Gly Thr Thr Leu
        450                 455                 460
cat gta tac cca gat ctt tat cat tca caa ttc acg tac ttt cag gtt      1502
His Val Tyr Pro Asp Leu Tyr His Ser Gln Phe Thr Tyr Phe Gln Val
    465                 470                 475
ctc cga gta gtt cgg ctg att aag att tca cct gca tta gaa gac ttt      1550
Leu Arg Val Val Arg Leu Ile Lys Ile Ser Pro Ala Leu Glu Asp Phe
480                 485                 490                 495
gtg tac aag ata ttt ggt cct gga aaa aag ctt ggg agt ttg gtt gta      1598
Val Tyr Lys Ile Phe Gly Pro Gly Lys Lys Leu Gly Ser Leu Val Val
                500                 505                 510
ttt act gcc agc ctc ttg att gtt atg tca gca att agt ttg cag atg      1646
Phe Thr Ala Ser Leu Leu Ile Val Met Ser Ala Ile Ser Leu Gln Met
            515                 520                 525
ttc tgc ttt gty gaa gaa ctg gac aga ttt act acg ttt ccg agg gca      1694
Phe Cys Phe Val Glu Glu Leu Asp Arg Phe Thr Thr Phe Pro Arg Ala
        530                 535                 540
ttt atg tcc atg ttc cag atc ctc acc cag gaa gga tgg gtg gac gta      1742
Phe Met Ser Met Phe Gln Ile Leu Thr Gln Glu Gly Trp Val Asp Val
    545                 550                 555
atg gac caa act cta aat gct gtg gga cat atg tgg gca ccc gtg gtt      1790
Met Asp Gln Thr Leu Asn Ala Val Gly His Met Trp Ala Pro Val Val
560                 565                 570                 575
gcc atc tat ttc att ctc tat cat ctt ttt gcc act ctg atc ctc ctg      1838
Ala Ile Tyr Phe Ile Leu Tyr His Leu Phe Ala Thr Leu Ile Leu Leu
                580                 585                 590
agt ttg ttt gtt gct gtt att ttg gac aac tta gaa ctt gat gaa gac      1886
Ser Leu Phe Val Ala Val Ile Leu Asp Asn Leu Glu Leu Asp Glu Asp
            595                 600                 605
cta aag aag ctt aaa caa tta aag caa agt gaa gca aat gcg gac acc      1934
Leu Lys Lys Leu Lys Gln Leu Lys Gln Ser Glu Ala Asn Ala Asp Thr
        610                 615                 620
aaa gaa aag ctc cct tta cgc ctg cga atc ttt gaa aaa ttt cca aac      1982
Lys Glu Lys Leu Pro Leu Arg Leu Arg Ile Phe Glu Lys Phe Pro Asn
    625                 630                 635
```

```
aga cct caa atg gtg aaa atc tca aag ctt cct tca gat ttt aca gtt      2030
Arg Pro Gln Met Val Lys Ile Ser Lys Leu Pro Ser Asp Phe Thr Val
640                 645                 650                 655
cct aaa atc agg gag agt ttt atg aag cag ttt att gac cgc cag caa      2078
Pro Lys Ile Arg Glu Ser Phe Met Lys Gln Phe Ile Asp Arg Gln Gln
                660                 665                 670
cag gac aca tgt tgc ctc ctg aga agc ctc ccg acc acc tct tcc tcc      2126
Gln Asp Thr Cys Cys Leu Leu Arg Ser Leu Pro Thr Thr Ser Ser Ser
            675                 680                 685
tcc tgc gac cac tcc aaa cgc tca gca att gag gac aac aaa tac atc      2174
Ser Cys Asp His Ser Lys Arg Ser Ala Ile Glu Asp Asn Lys Tyr Ile
        690                 695                 700
gac caa aaa ctt cgc aag tct gtt ttc agc atc agg gca agg aac ctt      2222
Asp Gln Lys Leu Arg Lys Ser Val Phe Ser Ile Arg Ala Arg Asn Leu
    705                 710                 715
ctg gaa aag gag acc gca gtc act aaa atc tta aga gct tgc acc cga      2270
Leu Glu Lys Glu Thr Ala Val Thr Lys Ile Leu Arg Ala Cys Thr Arg
720                 725                 730                 735
cag cgc atg ctg agc gga tca ttt gag ggg cag ccc gca aag gag agg      2318
Gln Arg Met Leu Ser Gly Ser Phe Glu Gly Gln Pro Ala Lys Glu Arg
                740                 745                 750
tca atc ctc agc gtg cag cat cat atc cgc caa gag cgc agg tca cta      2366
Ser Ile Leu Ser Val Gln His His Ile Arg Gln Glu Arg Arg Ser Leu
            755                 760                 765
aga cat gga tca aac agc cag agg atc agc agg gga aaa tct ctt gaa      2414
Arg His Gly Ser Asn Ser Gln Arg Ile Ser Arg Gly Lys Ser Leu Glu
        770                 775                 780
act ttg act caa gat cat tcc aat aca gtg aga tat aga aat gca caa      2462
Thr Leu Thr Gln Asp His Ser Asn Thr Val Arg Tyr Arg Asn Ala Gln
    785                 790                 795
aga gaa gac agt gaa ata aag atg att cag gaa aaa aag gag caa gca      2510
Arg Glu Asp Ser Glu Ile Lys Met Ile Gln Glu Lys Lys Glu Gln Ala
800                 805                 810                 815
gag atg aaa agg aaa gtg caa gaa gag gaa ctc aga gag aac cac cca      2558
Glu Met Lys Arg Lys Val Gln Glu Glu Glu Leu Arg Glu Asn His Pro
                820                 825                 830
tac ttc gat aag cca ctg ttc att gtc ggg cga gaa cac agg ttc aga      2606
Tyr Phe Asp Lys Pro Leu Phe Ile Val Gly Arg Glu His Arg Phe Arg
            835                 840                 845
aac ttt tgc cgg gtg gtg gtc cga gca cgc ttc aac gca tct aaa aca      2654
Asn Phe Cys Arg Val Val Val Arg Ala Arg Phe Asn Ala Ser Lys Thr
        850                 855                 860
gac cct gtc aca gga gct gtg aaa aat aca aag tac cat caa ctt tat      2702
Asp Pro Val Thr Gly Ala Val Lys Asn Thr Lys Tyr His Gln Leu Tyr
    865                 870                 875
gat ttg ctg gga ttg gtc act tac ctg gac tgg gtc atg atc atc gta      2750
Asp Leu Leu Gly Leu Val Thr Tyr Leu Asp Trp Val Met Ile Ile Val
880                 885                 890                 895
acc atc tgc tct tgc att tcc atg atg ttt gag tcc ccg ttt cga aga      2798
Thr Ile Cys Ser Cys Ile Ser Met Met Phe Glu Ser Pro Phe Arg Arg
                900                 905                 910
gtc atg cat gca cct act ttg cag att gct gag tat gtg ttt gtg ata      2846
Val Met His Ala Pro Thr Leu Gln Ile Ala Glu Tyr Val Phe Val Ile
            915                 920                 925
ttc atg agc att gag ctt aat ctg aag att atg gca gat ggc tta ttt      2894
Phe Met Ser Ile Glu Leu Asn Leu Lys Ile Met Ala Asp Gly Leu Phe
        930                 935                 940
ttc act cca act gct gtc atc agg gac ttc ggt gga gta atg gac ata      2942
Phe Thr Pro Thr Ala Val Ile Arg Asp Phe Gly Gly Val Met Asp Ile
    945                 950                 955
ttt ata tat ctt gtg agc ttg ata ttt ctt tgt tgg atg cct caa aat      2990
Phe Ile Tyr Leu Val Ser Leu Ile Phe Leu Cys Trp Met Pro Gln Asn
960                 965                 970                 975
```

```
gta cct gct gaa tcg gga gct cag ctt cta atg gtc ctt cgg tgc ctg      3038
Val Pro Ala Glu Ser Gly Ala Gln Leu Leu Met Val Leu Arg Cys Leu
                980                 985                 990
aga cct ctg cgc ata ttc aaa ctg gtg ccc cag atg agg aaa gtt gtt      3086
Arg Pro Leu Arg Ile Phe Lys Leu Val Pro Gln Met Arg Lys Val Val
            995                 1000                1005
cga gaa ctt ttc agc ggc ttc aag gaa att ttt ttg gtc tcc att ctt      3134
Arg Glu Leu Phe Ser Gly Phe Lys Glu Ile Phe Leu Val Ser Ile Leu
        1010                1015                1020
ttg ctg aca tta atg ctc gtt ttt gca agc ttt gga gtt cag ctt ttt      3182
Leu Leu Thr Leu Met Leu Val Phe Ala Ser Phe Gly Val Gln Leu Phe
    1025                1030                1035
gct gga aaa ctg gcc aag tgc aat gat ccc aac att att aga agg gaa      3230
Ala Gly Lys Leu Ala Lys Cys Asn Asp Pro Asn Ile Ile Arg Arg Glu
1040                1045                1050                1055
gat tgc aat ggc ata ttc aga att aat gtc agt gtg tca aag aac tta      3278
Asp Cys Asn Gly Ile Phe Arg Ile Asn Val Ser Val Ser Lys Asn Leu
                1060                1065                1070
aat tta aaa ttg agg cct gga gag aaa aaa cct gga ttt tgg gtg ccc      3326
Asn Leu Lys Leu Arg Pro Gly Glu Lys Lys Pro Gly Phe Trp Val Pro
            1075                1080                1085
cgt gtt tgg gcg aat cct cgg aac ttt aat ttc gac aat gtg gga aac      3374
Arg Val Trp Ala Asn Pro Arg Asn Phe Asn Phe Asp Asn Val Gly Asn
        1090                1095                1100
gct atg ctg gcg ttg ttt gaa gtt ctc tcc ttg aaa ggc tgg gtg gaa      3422
Ala Met Leu Ala Leu Phe Glu Val Leu Ser Leu Lys Gly Trp Val Glu
    1105                1110                1115
gtg aga gat gtt att att cat cgt gtg ggg ccg atc cat gga atc tat      3470
Val Arg Asp Val Ile Ile His Arg Val Gly Pro Ile His Gly Ile Tyr
1120                1125                1130                1135
att cat gtt ttt gta ttc ctg ggt tgc atg att gga ctg acc ctt ttt      3518
Ile His Val Phe Val Phe Leu Gly Cys Met Ile Gly Leu Thr Leu Phe
                1140                1145                1150
gtt gga gta gtt att gct aat ttc aat gaa aac aag ggg acg gct ttg      3566
Val Gly Val Val Ile Ala Asn Phe Asn Glu Asn Lys Gly Thr Ala Leu
            1155                1160                1165
ctg acc gtc gat cag aga aga tgg gaa gac ctg aag agc cga ctg aag      3614
Leu Thr Val Asp Gln Arg Arg Trp Glu Asp Leu Lys Ser Arg Leu Lys
        1170                1175                1180
atc gca cag cct ctt cat ctc ccg cct cgc ccg gat aat gat ggt ttt      3662
Ile Ala Gln Pro Leu His Leu Pro Pro Arg Pro Asp Asn Asp Gly Phe
    1185                1190                1195
aga gct aaa atg tat gac ata acc cag cat cca ttt ttt aag agg aca      3710
Arg Ala Lys Met Tyr Asp Ile Thr Gln His Pro Phe Phe Lys Arg Thr
1200                1205                1210                1215
atc gca tta ctc gtc ctg gcc cag tcg gtg ttg ctc tct gtc aag tgg      3758
Ile Ala Leu Leu Val Leu Ala Gln Ser Val Leu Leu Ser Val Lys Trp
                1220                1225                1230
gac gtc gag gac ccg gtg acc gta cct ttg gca aca atg tca gtt gtt      3806
Asp Val Glu Asp Pro Val Thr Val Pro Leu Ala Thr Met Ser Val Val
            1235                1240                1245
ttc acc ttc atc ttt gtt ctg gag gtt acc atg aag atc ata gca atg      3854
Phe Thr Phe Ile Phe Val Leu Glu Val Thr Met Lys Ile Ile Ala Met
        1250                1255                1260
tcg cct gct ggc ttc tgg caa agc aga aga aac cga tac gat ctc ctg      3902
Ser Pro Ala Gly Phe Trp Gln Ser Arg Arg Asn Arg Tyr Asp Leu Leu
    1265                1270                1275
gtg acg tcg ctt ggc gtt gta tgg gtg gtg ctt cac ttt gcc ctc ctg      3950
Val Thr Ser Leu Gly Val Val Trp Val Val Leu His Phe Ala Leu Leu
1280                1285                1290                1295
aat gca tat act tac atg atg ggc gct tgt gtg att gta ttt agg ttt      3998
Asn Ala Tyr Thr Tyr Met Met Gly Ala Cys Val Ile Val Phe Arg Phe
                1300                1305                1310
```

```
ttc tcc atc tgt gga aaa cat gta acg cta aag atg ctc ctc ttg aca      4046
Phe Ser Ile Cys Gly Lys His Val Thr Leu Lys Met Leu Leu Leu Thr
            1315                1320                1325
gtg gtc gtc agc atg tac aag agc ttc ttt atc ata gta ggc atg ttt      4094
Val Val Val Ser Met Tyr Lys Ser Phe Phe Ile Ile Val Gly Met Phe
        1330                1335                1340
ctc ttg ctg ctg tgt tac gct ttt gct gga gtt gtt tta ttt ggt act      4142
Leu Leu Leu Leu Cys Tyr Ala Phe Ala Gly Val Val Leu Phe Gly Thr
    1345                1350                1355
gtg aaa tat ggg gag aat att aac agg cat gca aat ttt tct tcg gct      4190
Val Lys Tyr Gly Glu Asn Ile Asn Arg His Ala Asn Phe Ser Ser Ala
1360                1365                1370                1375
gga aaa gct att acc gta ctg ttc cga att gtc aca ggt gaa gac tgg      4238
Gly Lys Ala Ile Thr Val Leu Phe Arg Ile Val Thr Gly Glu Asp Trp
                1380                1385                1390
aac aag att atg cat gac tgt atg gtt cag cct ccg ttt tgt act cca      4286
Asn Lys Ile Met His Asp Cys Met Val Gln Pro Pro Phe Cys Thr Pro
            1395                1400                1405
gat gaa ttt aca tac tgg gca aca gac tgt gga aat tat gct ggg gca      4334
Asp Glu Phe Thr Tyr Trp Ala Thr Asp Cys Gly Asn Tyr Ala Gly Ala
        1410                1415                1420
ctt atg tat ttc tgt tca ttt tat gtc atc att gcc tac atc atg cta      4382
Leu Met Tyr Phe Cys Ser Phe Tyr Val Ile Ile Ala Tyr Ile Met Leu
    1425                1430                1435
aat ctg ctt gta gcc ata att gtg gag aat ttc tcc ttg ttt tat tcc      4430
Asn Leu Leu Val Ala Ile Ile Val Glu Asn Phe Ser Leu Phe Tyr Ser
1440                1445                1450                1455
act gag gag gac cag ctt tta agt tac aat gat ctt cgc cac ttt caa      4478
Thr Glu Glu Asp Gln Leu Leu Ser Tyr Asn Asp Leu Arg His Phe Gln
                1460                1465                1470
atm ata tgg aac atg gtg gat gat aaa aga gag ggg gtg atc ccc acg      4526
Ile Ile Trp Asn Met Val Asp Asp Lys Arg Glu Gly Val Ile Pro Thr
            1475                1480                1485
ttc cgc gtc aag ttc ctg ctg cgg cta ctg cgt ggg agg ctg gag gtg      4574
Phe Arg Val Lys Phe Leu Leu Arg Leu Leu Arg Gly Arg Leu Glu Val
        1490                1495                1500
gac ctg gac aag gac aag ctc ctg ttt aag cac atg tgc tac gaa atg      4622
Asp Leu Asp Lys Asp Lys Leu Leu Phe Lys His Met Cys Tyr Glu Met
    1505                1510                1515
gag agg ctc cac aat ggc ggc gac gtc acc ttc cat gat gtc ctg agc      4670
Glu Arg Leu His Asn Gly Gly Asp Val Thr Phe His Asp Val Leu Ser
1520                1525                1530                1535
atg ctt tca tac cgg tcc gtg gac atc cgg aag agc ttg cag ctg gag      4718
Met Leu Ser Tyr Arg Ser Val Asp Ile Arg Lys Ser Leu Gln Leu Glu
                1540                1545                1550
gaa ctc ctg gcg agg gag cag ctg gag tac acc ata gag gag gag gtg      4766
Glu Leu Leu Ala Arg Glu Gln Leu Glu Tyr Thr Ile Glu Glu Glu Val
            1555                1560                1565
gcc aag cag acc atc cgc atg tgg ctc aag aag tgc ctg aag cgc atc      4814
Ala Lys Gln Thr Ile Arg Met Trp Leu Lys Lys Cys Leu Lys Arg Ile
        1570                1575                1580
aga gct aaa cag cag cag tcg tgc agt atc atc cac agc ctg aga gag      4862
Arg Ala Lys Gln Gln Gln Ser Cys Ser Ile Ile His Ser Leu Arg Glu
    1585                1590                1595
agt cag cag caa gag ctg agc cgg ttt ctg aac ccg ccc agc atc gag      4910
Ser Gln Gln Gln Glu Leu Ser Arg Phe Leu Asn Pro Pro Ser Ile Glu
1600                1605                1610                1615
acc acc cag ccc agt gag gac acg aat gcc aac agt cag gac aac agc      4958
Thr Thr Gln Pro Ser Glu Asp Thr Asn Ala Asn Ser Gln Asp Asn Ser
                1620                1625                1630
atg caa cct gag aca agc agc cag cag cag ctc ctg agc ccc acg ctg      5006
Met Gln Pro Glu Thr Ser Ser Gln Gln Gln Leu Leu Ser Pro Thr Leu
            1635                1640                1645
```

```
tcg gat cga gga gga agt cgg caa gat gca gcc gac gca ggg aaa ccc      5054
Ser Asp Arg Gly Gly Ser Arg Gln Asp Ala Ala Asp Ala Gly Lys Pro
        1650                1655                1660
cag agg aaa ttt ggg cag tgg cgt ctg ccc tca gcc cca aaa cca ata      5102
Gln Arg Lys Phe Gly Gln Trp Arg Leu Pro Ser Ala Pro Lys Pro Ile
    1665                1670                1675
agc cat tca gtg tcc tca gtc aac tta cgg ttt gga gga agg aca acc      5150
Ser His Ser Val Ser Ser Val Asn Leu Arg Phe Gly Gly Arg Thr Thr
1680                1685                1690                1695
atg aaa tct gtc gtg tgc aaa atg aac ccc atg act gac gcg gct tcc      5198
Met Lys Ser Val Val Cys Lys Met Asn Pro Met Thr Asp Ala Ala Ser
                1700                1705                1710
tgc ggt tct gaa gtt aag aag tgg tgg acc cgg cag ctg act gtg gag      5246
Cys Gly Ser Glu Val Lys Lys Trp Trp Thr Arg Gln Leu Thr Val Glu
            1715                1720                1725
agc gac gaa agt ggg gat gac ctt ctg gat att tag gtggatgtca           5292
Ser Asp Glu Ser Gly Asp Asp Leu Leu Asp Ile  *
        1730                1735
atgtagatga atttctagtg gtggaaaccg ttttctaata atgtccttga ttgtccagtg   5352
agcaatctgt aattgatcta taactgaatt ccagcttgtc acaagatgtt tataaattga   5412
ttttcatcct gccacagaaa ggcataagct gcatgtatga tgggttacta tcaatcattg   5472
ctcaaaaaaa tttttgtata atgacagtac tgataatatt agaaatgata ccgcaagcaa   5532
atgtatatca cttaaaaatg tcatatattc tgtctgcgta aactaaggta tatattcata   5592
tgtgctctaa tgcagtatta tcaccgcccc gcaaaagagt gctaagccca aagtggctga   5652
tatttagggt acaggggtta tagctttagt tcacatcttt cccatttcca ctagaaatat   5712
ttctcttgag agaatttatt atttatgatt gatctgaaaa ggtcagcact gaacttatgc   5772
taaaatgata gtagttttac aaactacaga ttctgaattt taaaaagtat cttctttttc   5832
tcgtgttata tttttaaata tacacaagac atttggtgac cagaacaagt tgatttctgt   5892
cctcagttat gttaatgaaa ctgttgcctc cttctaagaa aattgtgtgt gcaagcacca   5952
ggcaaagaaa tggactcagg atgcttagcg gtttaaaaca aacctgtaga taaatcactt   6012
gagtgacata gttgcgcaaa gatgttaagt ttcttaagaa acctttttaat aactgagttt   6072
agcaaaaaga ataaaactat atagctcaat ttatttaaaa aaatctttgc atgtgtgatg   6132
ttatcattgg cttcatttct tacccaaggt atgtctgttt tgccataaat cagcagagtc   6192
atttcattct gggtgatcct aacacaccat tgctatgtta gatttgaaat gacatctctg   6252
ttaaaagaat cttctatgga aataatggtg ccctgcaaaa tcttcctctg aactcacagg   6312
ttagggatca cacaacttac ttaatcgttt tttgttttttg tttttttttcc ttatatgtca   6372
atggcccatg tcctccggga aaattagaaa agcaaaatga ttacaaagtg ctgttagatt   6432
tcttgtgctg ggccagccaa gtagaagtgg acttgacttg gacctttaac tattttatta   6492
cagattggac atttgctgtt cagatgtttt ttaacagagg gattatctca gaatcctgtg   6552
acctccaggt tgttttataa tctatttttc tctatttaac attcctcaga tagataggca   6612
aataggacat tccttctgtg tcacagaagt atcgtggtag tggcagtcta cagtttatat   6672
gattcattgt aactatgaga taaagaacaa ccagtcatgt ggccaaaagg attagatttg   6732
atttgatgtt cacttggagt ttactttttg tacatacaag ataaaataaa tattggattt   6792
gtaaaat                                                              6799
```

<210> 5
<211> 1738
<212> PRT
<213> Homo sapiens

<400> 5

```
Met Leu Lys Arg Lys Gln Ser Ser Arg Val Glu Ala Gln Pro Val Thr
1               5                   10                  15
Asp Phe Gly Pro Asp Glu Ser Leu Ser Asp Asn Ala Asp Ile Leu Trp
            20                  25                  30
Ile Asn Lys Pro Trp Val His Ser Leu Leu Arg Ile Cys Ala Ile Ile
        35                  40                  45
Ser Val Ile Ser Val Cys Met Asn Thr Pro Met Thr Phe Glu His Tyr
    50                  55                  60
Pro Pro Leu Gln Tyr Val Thr Phe Thr Leu Asp Thr Leu Leu Met Phe
65                  70                  75                  80
Leu Tyr Thr Ala Glu Met Ile Ala Lys Met His Ile Arg Gly Ile Val
                85                  90                  95
```

Lys Gly Asp Ser Ser Tyr Val Lys Asp Arg Trp Cys Val Phe Asp Gly
100 105 110
Phe Met Val Phe Cys Leu Trp Val Ser Leu Val Leu Gln Val Phe Glu
115 120 125
Ile Ala Asp Ile Val Asp Gln Met Ser Pro Trp Gly Met Leu Arg Ile
130 135 140
Pro Arg Pro Leu Ile Met Ile Arg Ala Phe Arg Ile Tyr Phe Arg Phe
145 150 155 160
Glu Leu Pro Arg Thr Arg Ile Thr Asn Ile Leu Lys Arg Ser Gly Glu
165 170 175
Gln Ile Trp Ser Val Ser Ile Phe Leu Leu Phe Phe Leu Leu Leu Tyr
180 185 190
Gly Ile Leu Gly Val Gln Met Phe Gly Thr Phe Thr Tyr His Cys Val
195 200 205
Val Asn Asp Thr Lys Pro Gly Asn Val Thr Trp Asn Ser Leu Ala Ile
210 215 220
Pro Asp Thr His Cys Ser Pro Glu Leu Glu Glu Gly Tyr Gln Cys Pro
225 230 235 240
Pro Gly Phe Lys Cys Met Asp Leu Glu Asp Leu Gly Leu Ser Arg Gln
245 250 255
Glu Leu Gly Tyr Ser Gly Phe Asn Glu Ile Gly Thr Ser Ile Phe Thr
260 265 270
Val Tyr Glu Ala Ala Ser Gln Glu Gly Trp Val Phe Leu Met Tyr Arg
275 280 285
Ala Ile Asp Ser Phe Pro Arg Trp Arg Ser Tyr Phe Tyr Phe Ile Thr
290 295 300
Leu Ile Phe Phe Leu Ala Trp Leu Val Lys Asn Val Phe Ile Ala Val
305 310 315 320
Ile Ile Glu Thr Phe Ala Glu Ile Arg Val Gln Phe Gln Gln Met Trp
325 330 335
Gly Ser Arg Ser Ser Thr Thr Ser Thr Ala Thr Thr Gln Met Phe His
340 345 350
Glu Asp Ala Ala Gly Gly Trp Gln Leu Val Ala Val Asp Val Asn Lys
355 360 365
Pro Gln Gly Arg Ala Pro Ala Cys Leu Gln Lys Met Met Arg Ser Ser
370 375 380
Val Phe His Met Phe Ile Leu Ser Met Val Thr Val Asp Val Ile Val
385 390 395 400
Ala Ala Ser Asn Tyr Tyr Lys Gly Glu Asn Phe Arg Arg Gln Tyr Asp
405 410 415
Glu Phe Tyr Leu Ala Glu Val Ala Phe Thr Val Leu Phe Asp Leu Glu
420 425 430
Ala Leu Leu Lys Ile Trp Cys Leu Gly Phe Thr Gly Tyr Ile Ser Ser
435 440 445
Ser Leu His Lys Phe Glu Leu Leu Val Ile Gly Thr Thr Leu His
450 455 460
Val Tyr Pro Asp Leu Tyr His Ser Gln Phe Thr Tyr Phe Gln Val Leu
465 470 475 480
Arg Val Val Arg Leu Ile Lys Ile Ser Pro Ala Leu Glu Asp Phe Val
485 490 495
Tyr Lys Ile Phe Gly Pro Gly Lys Lys Leu Gly Ser Leu Val Val Phe
500 505 510
Thr Ala Ser Leu Leu Ile Val Met Ser Ala Ile Ser Leu Gln Met Phe
515 520 525
Cys Phe Val Glu Glu Leu Asp Arg Phe Thr Thr Phe Pro Arg Ala Phe
530 535 540
Met Ser Met Phe Gln Ile Leu Thr Gln Glu Gly Trp Val Asp Val Met
545 550 555 560
Asp Gln Thr Leu Asn Ala Val Gly His Met Trp Ala Pro Val Val Ala
565 570 575
Ile Tyr Phe Ile Leu Tyr His Leu Phe Ala Thr Leu Ile Leu Leu Ser
580 585 590
Leu Phe Val Ala Val Ile Leu Asp Asn Leu Glu Leu Asp Glu Asp Leu

```
        595                 600                 605
Lys Lys Leu Lys Gln Leu Lys Gln Ser Glu Ala Asn Ala Asp Thr Lys
    610                 615                 620
Glu Lys Leu Pro Leu Arg Leu Arg Ile Phe Glu Lys Phe Pro Asn Arg
625                 630                 635                 640
Pro Gln Met Val Lys Ile Ser Lys Leu Pro Ser Asp Phe Thr Val Pro
                645                 650                 655
Lys Ile Arg Glu Ser Phe Met Lys Gln Phe Ile Asp Arg Gln Gln Gln
            660                 665                 670
Asp Thr Cys Cys Leu Leu Arg Ser Leu Pro Thr Thr Ser Ser Ser Ser
        675                 680                 685
Cys Asp His Ser Lys Arg Ser Ala Ile Glu Asp Asn Lys Tyr Ile Asp
    690                 695                 700
Gln Lys Leu Arg Lys Ser Val Phe Ser Ile Arg Ala Arg Asn Leu Leu
705                 710                 715                 720
Glu Lys Glu Thr Ala Val Thr Lys Ile Leu Arg Ala Cys Thr Arg Gln
                725                 730                 735
Arg Met Leu Ser Gly Ser Phe Glu Gly Gln Pro Ala Lys Glu Arg Ser
            740                 745                 750
Ile Leu Ser Val Gln His His Ile Arg Gln Glu Arg Arg Ser Leu Arg
        755                 760                 765
His Gly Ser Asn Ser Gln Arg Ile Ser Arg Gly Lys Ser Leu Glu Thr
    770                 775                 780
Leu Thr Gln Asp His Ser Asn Thr Val Arg Tyr Arg Asn Ala Gln Arg
785                 790                 795                 800
Glu Asp Ser Glu Ile Lys Met Ile Gln Glu Lys Lys Glu Gln Ala Glu
                805                 810                 815
Met Lys Arg Lys Val Gln Glu Glu Glu Leu Arg Glu Asn His Pro Tyr
            820                 825                 830
Phe Asp Lys Pro Leu Phe Ile Val Gly Arg Glu His Arg Phe Arg Asn
        835                 840                 845
Phe Cys Arg Val Val Val Arg Ala Arg Phe Asn Ala Ser Lys Thr Asp
    850                 855                 860
Pro Val Thr Gly Ala Val Lys Asn Thr Lys Tyr His Gln Leu Tyr Asp
865                 870                 875                 880
Leu Leu Gly Leu Val Thr Tyr Leu Asp Trp Val Met Ile Ile Val Thr
                885                 890                 895
Ile Cys Ser Cys Ile Ser Met Met Phe Glu Ser Pro Phe Arg Arg Val
            900                 905                 910
Met His Ala Pro Thr Leu Gln Ile Ala Glu Tyr Val Phe Val Ile Phe
        915                 920                 925
Met Ser Ile Glu Leu Asn Leu Lys Ile Met Ala Asp Gly Leu Phe Phe
    930                 935                 940
Thr Pro Thr Ala Val Ile Arg Asp Phe Gly Gly Val Met Asp Ile Phe
945                 950                 955                 960
Ile Tyr Leu Val Ser Leu Ile Phe Leu Cys Trp Met Pro Gln Asn Val
                965                 970                 975
Pro Ala Glu Ser Gly Ala Gln Leu Leu Met Val Leu Arg Cys Leu Arg
            980                 985                 990
Pro Leu Arg Ile Phe Lys Leu Val Pro Gln Met Arg Lys Val Val Arg
        995                 1000                1005
Glu Leu Phe Ser Gly Phe Lys Glu Ile Phe Leu Val Ser Ile Leu Leu
    1010                1015                1020
Leu Thr Leu Met Leu Val Phe Ala Ser Phe Gly Val Gln Leu Phe Ala
1025                1030                1035                1040
Gly Lys Leu Ala Lys Cys Asn Asp Pro Asn Ile Ile Arg Arg Glu Asp
                1045                1050                1055
Cys Asn Gly Ile Phe Arg Ile Asn Val Ser Val Ser Lys Asn Leu Asn
            1060                1065                1070
Leu Lys Leu Arg Pro Gly Glu Lys Lys Pro Gly Phe Trp Val Pro Arg
        1075                1080                1085
Val Trp Ala Asn Pro Arg Asn Phe Asn Phe Asp Asn Val Gly Asn Ala
    1090                1095                1100
```

```
Met Leu Ala Leu Phe Glu Val Leu Ser Leu Lys Gly Trp Val Glu Val
1105                1110                1115                1120
Arg Asp Val Ile Ile His Arg Val Gly Pro Ile His Gly Ile Tyr Ile
                1125                1130                1135
His Val Phe Val Phe Leu Gly Cys Met Ile Gly Leu Thr Leu Phe Val
            1140                1145                1150
Gly Val Val Ile Ala Asn Phe Asn Glu Asn Lys Gly Thr Ala Leu Leu
        1155                1160                1165
Thr Val Asp Gln Arg Arg Trp Glu Asp Leu Lys Ser Arg Leu Lys Ile
    1170                1175                1180
Ala Gln Pro Leu His Leu Pro Pro Arg Pro Asp Asn Asp Gly Phe Arg
1185                1190                1195                1200
Ala Lys Met Tyr Asp Ile Thr Gln His Pro Phe Phe Lys Arg Thr Ile
                1205                1210                1215
Ala Leu Leu Val Leu Ala Gln Ser Val Leu Leu Ser Val Lys Trp Asp
            1220                1225                1230
Val Glu Asp Pro Val Thr Val Pro Leu Ala Thr Met Ser Val Val Phe
        1235                1240                1245
Thr Phe Ile Phe Val Leu Glu Val Thr Met Lys Ile Ile Ala Met Ser
    1250                1255                1260
Pro Ala Gly Phe Trp Gln Ser Arg Arg Asn Arg Tyr Asp Leu Leu Val
1265                1270                1275                1280
Thr Ser Leu Gly Val Val Trp Val Val Leu His Phe Ala Leu Leu Asn
                1285                1290                1295
Ala Tyr Thr Tyr Met Met Gly Ala Cys Val Ile Val Phe Arg Phe Phe
            1300                1305                1310
Ser Ile Cys Gly Lys His Val Thr Leu Lys Met Leu Leu Leu Thr Val
        1315                1320                1325
Val Val Ser Met Tyr Lys Ser Phe Phe Ile Ile Val Gly Met Phe Leu
    1330                1335                1340
Leu Leu Leu Cys Tyr Ala Phe Ala Gly Val Val Leu Phe Gly Thr Val
1345                1350                1355                1360
Lys Tyr Gly Glu Asn Ile Asn Arg His Ala Asn Phe Ser Ser Ala Gly
                1365                1370                1375
Lys Ala Ile Thr Val Leu Phe Arg Ile Val Thr Gly Glu Asp Trp Asn
            1380                1385                1390
Lys Ile Met His Asp Cys Met Val Gln Pro Pro Phe Cys Thr Pro Asp
        1395                1400                1405
Glu Phe Thr Tyr Trp Ala Thr Asp Cys Gly Asn Tyr Ala Gly Ala Leu
    1410                1415                1420
Met Tyr Phe Cys Ser Phe Tyr Val Ile Ile Ala Tyr Ile Met Leu Asn
1425                1430                1435                1440
Leu Leu Val Ala Ile Ile Val Glu Asn Phe Ser Leu Phe Tyr Ser Thr
                1445                1450                1455
Glu Glu Asp Gln Leu Leu Ser Tyr Asn Asp Leu Arg His Phe Gln Ile
            1460                1465                1470
Ile Trp Asn Met Val Asp Asp Lys Arg Glu Gly Val Ile Pro Thr Phe
        1475                1480                1485
Arg Val Lys Phe Leu Leu Arg Leu Leu Arg Gly Arg Leu Glu Val Asp
    1490                1495                1500
Leu Asp Lys Asp Lys Leu Leu Phe Lys His Met Cys Tyr Glu Met Glu
1505                1510                1515                1520
Arg Leu His Asn Gly Gly Asp Val Thr Phe His Asp Val Leu Ser Met
                1525                1530                1535
Leu Ser Tyr Arg Ser Val Asp Ile Arg Lys Ser Leu Gln Leu Glu Glu
            1540                1545                1550
Leu Leu Ala Arg Glu Gln Leu Glu Tyr Thr Ile Glu Glu Val Ala
        1555                1560                1565
Lys Gln Thr Ile Arg Met Trp Leu Lys Lys Cys Leu Lys Arg Ile Arg
    1570                1575                1580
Ala Lys Gln Gln Gln Ser Cys Ser Ile Ile His Ser Leu Arg Glu Ser
1585                1590                1595                1600
Gln Gln Gln Glu Leu Ser Arg Phe Leu Asn Pro Pro Ser Ile Glu Thr
```

```
                    1605                      1610                      1615
Thr Gln Pro Ser Glu Asp Thr Asn Ala Asn Ser Gln Asp Asn Ser Met
                1620                      1625                      1630
Gln Pro Glu Thr Ser Ser Gln Gln Gln Leu Leu Ser Pro Thr Leu Ser
            1635                      1640                      1645
Asp Arg Gly Gly Ser Arg Gln Asp Ala Ala Asp Ala Gly Lys Pro Gln
        1650                      1655                      1660
Arg Lys Phe Gly Gln Trp Arg Leu Pro Ser Ala Pro Lys Pro Ile Ser
1665                      1670                      1675                      1680
His Ser Val Ser Ser Val Asn Leu Arg Phe Gly Gly Arg Thr Thr Met
                    1685                      1690                      1695
Lys Ser Val Val Cys Lys Met Asn Pro Met Thr Asp Ala Ala Ser Cys
                1700                      1705                      1710
Gly Ser Glu Val Lys Lys Trp Trp Thr Arg Gln Leu Thr Val Glu Ser
            1715                      1720                      1725
Asp Glu Ser Gly Asp Asp Leu Leu Asp Ile
        1730                      1735
```

<210> 6
<211> 453
<212> DNA
<213> Homo sapiens

<220>
<221> allele
<222> 105
<223> 99-62617-105 : polymorphic base G or C

<220>
<221> misc_binding
<222> 93..117
<223> 99-62617-105.probe

<220>
<221> primer_bind
<222> 86..104
<223> 99-62617-105.mis

<220>
<221> primer_bind
<222> 106..124
<223> 99-62617-105.mis complement

<220>
<221> primer_bind
<222> 1..20
<223> 99-62617.pu

<220>
<221> primer_bind
<222> 435..453
<223> 99-62617.rp complement

<400> 6

```
tgaggatgag gggcaaacac agaggaagaa tctgggcccc atgaggtcaa cagtgagctg    60
ctaaaattaa cctgaaattt ccctgctggg agacattgtg ttgtsaaata gttttatacc   120
atcttttact aggttttcaa ttaccagcag ttggatgcac cctgtgatcc aatggataca   180
aatgagattt agaagtgcac tttaacttaa ataaaataaa tagccttgtc taatgtatta   240
acgagaaaga catttttgtc ttatttatat gtcttatgtt ctttgaaatg aaataaatga   300
ccatatatgc ttgtaaagag agggagtcta cagatatttg gagaaaggac attctgggtg   360
gagagaacac agagtgcaaa ggacttgaag ttaaagtgca tccagcagtc tcaaggaaga   420
gtaaaggggt ccatgtgagt ggaataaggt gag                                453
```

<210> 7
<211> 18
<212> DNA
<213> Artificial Sequence

<220>
<223> sequencing oligonucleotide PrimerPU

<400> 7
tgtaaaacga cggccagt 18

<210> 8
<211> 18
<212> DNA
<213> Artificial Sequence

<220>
<223> sequencing oligonucleotide PrimerRP

<400> 8
caggaaacag ctatgacc 18